(19) Europäisches Patentamt
European Patent Office
Office européen des brevets

(11) EP 4 722 219 A1

(12) # EUROPEAN PATENT APPLICATION
published in accordance with Art. 153(4) EPC

(43) Date of publication:
**08.04.2026 Bulletin 2026/15**

(21) Application number: **24810503.3**

(22) Date of filing: **27.05.2024**

(51) International Patent Classification (IPC):
***C07D 491/20*** (2006.01) ***C07D 498/04*** (2006.01)
***C07D 471/04*** (2006.01) ***C07D 471/14*** (2006.01)
***C07D 471/10*** (2006.01) ***C07D 487/10*** (2006.01)
***C07D 471/20*** (2006.01) ***C07D 498/14*** (2006.01)
***C07D 487/04*** (2006.01) ***A61K 31/435*** (2006.01)
***A61K 31/438*** (2006.01) ***A61K 31/437*** (2006.01)
***A61K 31/407*** (2006.01) ***A61P 35/00*** (2006.01)

(52) Cooperative Patent Classification (CPC):
**A61K 31/407; A61K 31/435; A61K 31/437; A61K 31/438; A61P 35/00; C07D 471/04; C07D 471/10; C07D 471/14; C07D 471/20; C07D 487/04; C07D 487/10; C07D 491/20; C07D 498/04; C07D 498/14**

(86) International application number:
**PCT/CN2024/095621**

(87) International publication number:
**WO 2024/240268 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.05.2023 CN 202310597246**
**16.06.2023 CN 202310717359**
**03.04.2024 CN 202410400297**
**03.04.2024 CN 202410398157**

(71) Applicant: **Gan & Lee Pharmaceuticals Co., Ltd.**
**Beijing 101109 (CN)**

(72) Inventors:
- **XU, Fuming**
  **Beijing 101109 (CN)**
- **CHEN, Wenmin**
  **Beijing 101109 (CN)**
- **ZHOU, Guan**
  **Beijing 101109 (CN)**
- **LIU, Xiaobing**
  **Beijing 101109 (CN)**
- **DING, Yongzheng**
  **Beijing 101109 (CN)**
- **LI, Xiaoguang**
  **Beijing 101109 (CN)**

(74) Representative: **Kraus & Lederer PartGmbB**
**Thomas-Wimmer-Ring 15**
**80539 München (DE)**

(54) **CEREBELLAR PROTEIN E3 UBIQUITIN LIGASE INHIBITOR AND CHIMERIC COMPOUND TARGETING PROTEIN DEGRADATION**

(57) The present invention provides a novel human cerebellar protein (CRBN) E3 ubiquitin ligase inhibitor, capable of being used to prepare a PROTAC molecule. The CRBNE3 ubiquitin ligase inhibitor and the PROTAC molecule can be used to treat cancer and other diseases.

EP 4 722 219 A1

## Description

### Technical Field

The present invention relates to a cerebellar protein E3 ubiquitin ligase inhibitor and a chimeric compound targeting protein degradation (Proteolysis Targeting Chimeras, PROTAC) comprising the inhibitor, and a use thereof in medicine.

### Background Art

Cereblon (CRBN) is a brain-associated protein with ionic protease activity that may form a functional E3 ubiquitin ligase complex (CRBN-CRL4) through interactions with DNA damage-binding protein 1 (DDB 1), Cullin 4 (Cul4A or Cul4B), and Regulator of Cullins 1 (RoC1). Upon substrate binding, this complex mediates proteolysis through the ubiquitin-proteasome pathway. Immune modulating drugs (IMiDs) such as thalidomide, lenalidomide, and pomalidomide recruit novel substrates when bound to CRBN, causing them to bind to CRBN-CRL4 complexes. This leads to increased ubiquitination and proteasome-dependent degradation, thereby treating cancer and other diseases.

Building upon this foundation, CRBN ligands have been extensively applied in protein degradation, leading to the development of a series of CRBN-based protein degradation-targeting chimeras (PROTACs). Due to the inherent effects of CRBN ligands on their target sites, which may induce additional degradation of domain proteins, the design and synthesis of novel CRBN ligands hold significant importance for the further advancement of PROTACs molecules.

The present invention discloses a series of novel CRBN ligands and further enables the synthesis of corresponding PROTACs molecules.

### SUMMARY

A first aspect of the present invention provides a compound represented by Formula I, or an isomer, isotopic derivative, polymorph, prodrug, or a pharmaceutically acceptable salt or a solvate thereof:

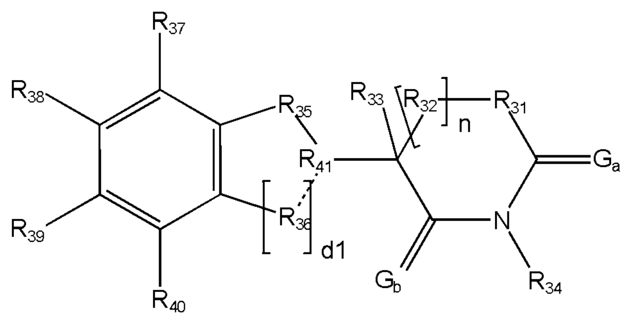

Formula I

wherein, $G_a$ and $G_b$ are independently selected from O, S, and Se; in one embodiment, $G_a$ and $G_b$ are independently O;

each occurrence of $R_{31}$ and $R_{32}$ is independently $CR^aR^b$;

each occurrence of $R_{33}$, $R_{34}$, $R^a$, and $R^b$ is independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, each occurrence of $R_{33}$, $R_{34}$, $R^a$, and $R^b$ is independently selected from H, deuterium, F, Cl, Br, I, $C_1$-$C_6$ alkyl, hydroxyl, $C_1$-$C_6$ haloalkyl, $C_1$-$C_6$ hydroxyalkyl, $C_1$-$C_6$ alkoxy, $C_3$-$C_6$ cycloalkyl, and $C_3$-$C_6$ heterocyclyl containing 1, 2, 3, or 4 heteroatoms independently selected from N, O, P, and S; wherein the alkyl, haloalkyl, hydroxyalkyl, alkoxy, cycloalkyl, and heterocyclyl are each independently optionally substituted by one or more substituents selected from halogen, alkyl, heteroalkyl, alkoxy, haloalkyl, hydroxyl, hydroxyalkyl, cycloalkyl, and heterocyclyl; in one embodiment, each occurrence of $R_{33}$, $R_{34}$, $R^a$, and $R^b$ is independently selected from H, deuterium, F, Cl, Br, I, $C_1$-$C_6$ alkyl, hydroxyl, $C_1$-$C_6$ haloalkyl, $C_1$-$C_6$ hydroxyalkyl, and $C_1$-$C_6$ alkoxy, wherein the alkyl, haloalkyl, hydroxyalkyl, and alkoxy are each independently optionally substituted

by one or more substituents selected from halogen, alkyl, heteroalkyl, alkoxy, haloalkyl, and hydroxyl; in one embodiment, each occurrence of $R_{33}$, $R_{34}$, $R^a$, and $R^b$ is independently selected from H, deuterium, F, Cl, Br, I, $C_1$-$C_3$ alkyl, hydroxyl, $C_1$-$C_3$ haloalkyl, $C_1$-$C_3$ hydroxyalkyl, and $C_1$-$C_3$ alkoxy;

n is 0, 1, 2, or 3;

when $R_{35}$ and $R_{36}$, together with the carbon atoms to which they are attached and $R_{41}$, form a ring, and d1 is 1: $R_{35}$ and $R_{36}$ are each independently selected from $CR^aR^b$, C(=S), C(=O), $NR_a$, or $SO_2$, and at least one of $R_{35}$ and $R_{36}$ is C(=O);

$R_{41}$ is N;

$R_{37}$ and $R_{38}$, together with the carbon atoms to which they are attached, form

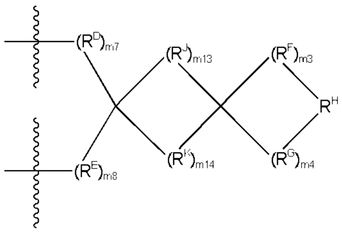
,
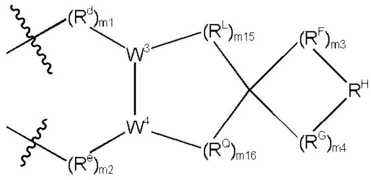
,
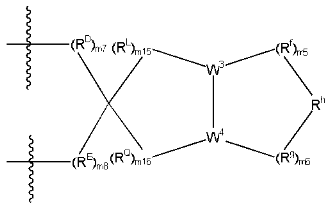
,

or

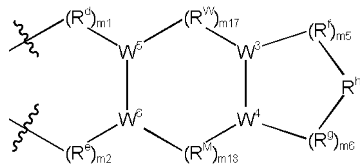
,

and $R_{39}$ and $R_{40}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R_{39}$ and $R_{40}$ are each independently selected from H, deuterium, halogen, alkyl, deuterated alkyl, haloalkyl, alkoxy, and hydroxyl;

or $R_{38}$ and $R_{39}$, together with the carbon atoms to which they are attached, form

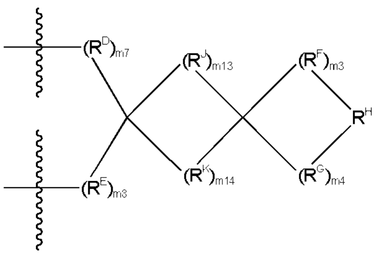
,
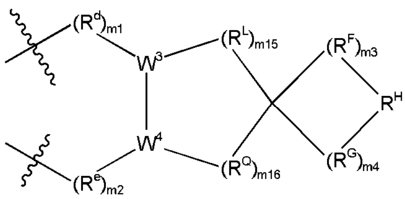
,

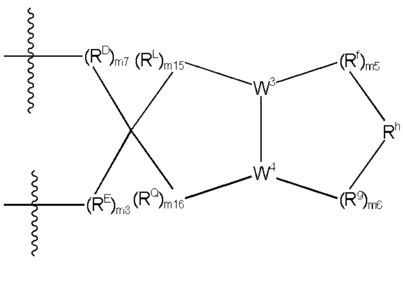
, or
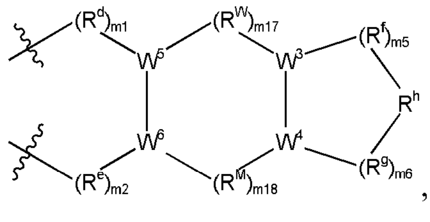
,

and $R_{37}$ and $R_{40}$ are each independently selected from H, deuterium, halogen, alkyl, deuterated alkyl, heteroalkyl,

alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl;

or $R_{39}$ and $R_{40}$, together with the carbon atoms to which they are attached, form

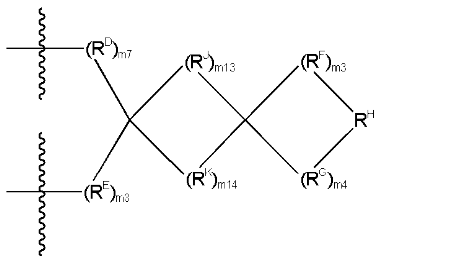

,

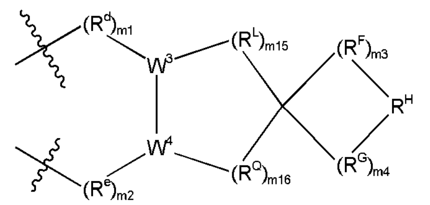

,

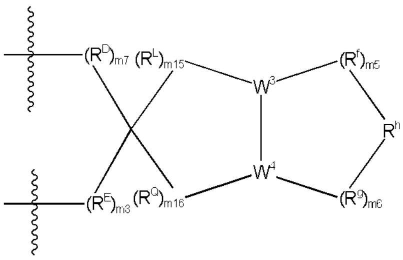

, or

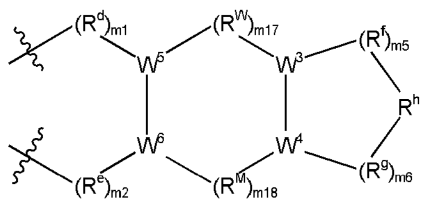

,

and $R_{37}$ and $R_{38}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl;

each occurrence of $R^d$, $R^e$, $R^f$, $R^g$, $R^D$, $R^E$, $R^F$, $R^J$, $R^K$, $R^L$, $R^Q$, $R^W$, $R^M$, and $R^G$ is independently $C(R^m)_2$, $NR^m$, C(=O), O, or S; in one embodiment, for each occurrence of $R^d$ and $R^e$, at least one occurrence exists and at least one of them is O; and for each occurrence of $R^D$ and $R^E$, at least one occurrence exists and at least one of them is O; in one embodiment, for each occurrence of $R^d$ and $R^e$, one occurrence exists and at least one of them is O, and for each occurrence of $R^D$ and $R^E$, one occurrence exists and at least one of them is O; in one embodiment, for each occurrence of $R^d$ and $R^e$, one occurrence exists and one of them is O, and for each occurrence of $R^D$ and $R^E$, one occurrence exists and one of them is O;

each occurrence of $W^3$, $W^4$, $W^5$, and $W^6$ is independently $CR^m$ or N; in one embodiment, each occurrence of $W^3$, $W^4$, $W^5$, and $W^6$ is independently CH or N; and

each occurrence of $R^h$ and $R^H$ is independently $NR^{1h}$, C(=O), $SO_2$, or $CR^{2h}R^{3h}$; in one embodiment, each occurrence of $R^h$ and $R^H$ is independently NH, C(=O), C(Cl)H, or C(OH)H; in one embodiment, each occurrence of $R^h$ and $R^H$ is independently NH;

when d1 is 0, $R_{35}$ and $R_{41}$ form a ring with the carbon atoms to which they are attached:

$R_{35}$ is $-N(R^a)-W^{11}-$, $W^{11}$ is selected from $CR^aR^b$, C(=O), C(=S), $NR^a$, and $SO_2$; in one embodiment, $W^{11}$ is C(=O); in one embodiment, $R_{35}$ is $-N(CH_3)-C(=O)-$;

$R_{41}$ is N;

$R_{37}$ and $R_{38}$, together with the carbon atoms to which they are attached, form

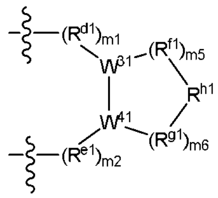

or

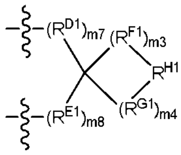

,

and $R_{39}$ and $R_{40}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R_{39}$ and $R_{40}$ are each independently selected from H, deuterium, halogen, alkyl, deuterated alkyl, haloalkyl, and hydroxyl;

or $R_{38}$ and $R_{39}$, together with the carbon atoms to which they are attached, form

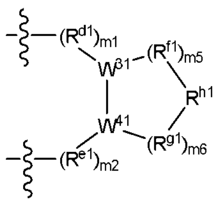

or

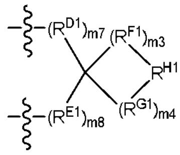

,

and $R_{37}$ and $R_{40}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R_{37}$ and $R_{40}$ are each independently selected from H, deuterium, halogen, alkyl, deuterated alkyl, haloalkyl, and hydroxyl;

or $R_{39}$ and $R_{40}$, together with the carbon atoms to which they are attached, form

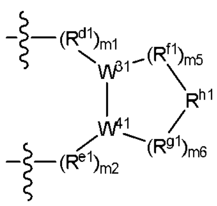

or

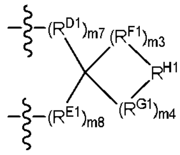

,

and $R_{37}$ and $R_{38}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl;

each occurrence of $R^{d1}$, $R^{e1}$, $R^{f1}$, $R^{g1}$, $R^{D1}$, $R^{E1}$, $R^{F1}$, and $R^{G1}$ is independently $C(R^m)_2$, $NR^m$, C(=O), O , or S; in one embodiment, for each occurrence of $R^{d1}$ and $R^{e1}$, at least one occurrence exists and at least one of them is O, and for each occurrence of $R^{D1}$ and $R^{E1}$, at least one occurrence exists and at least one of them is O;
each occurrence of $W^{31}$ and $W^{41}$ is independently $CR^m$ or N; in one embodiment, each occurrence of $W^{31}$ and $W^{41}$ is independently CH or N; and
each occurrence of $R^{h1}$ and $R^{H1}$ is independently $NR^{1h}$, C(=O), $SO_2$, or $CR^{2h}R^{3h}$; in one embodiment, each occurrence of $R^{h1}$ and $R^{H1}$ is independently NH, C=O, CHCl, or CHOH;
when no chemical bond exists between $R_{36}$ and $R_{41}$, $R_{35}$ and $R_{36}$ do not form a ring together with the carbon atoms to which they are attached and $R_{41}$, and d1 is 1:

$R_{35}$ is a single bond;
$R_{41}$ is selected from $-NR^a-$, $-NR^aCO-$, $C_1$-$C_6$ alkylene, and $C_1$-$C_6$ haloalkylene; in one embodiment, $R_{41}$ is selected from -NH-CO-, -NH-, and $-N(CH_3)-$;
$R_{37}$ and $R_{38}$, together with the carbon atoms to which they are attached, form

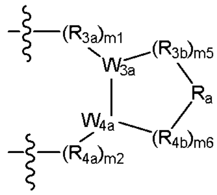

or

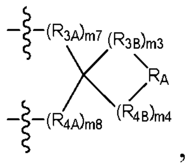

,

and $R_{39}$, $R_{40}$, and $R_{36}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R_{39}$, $R_{40}$, and $R_{36}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; in one embodiment, $R_{39}$, $R_{40}$, and $R_{36}$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy, and hydroxyl;
or $R_{38}$ and $R_{39}$, together with the carbon atoms to which they are attached, form

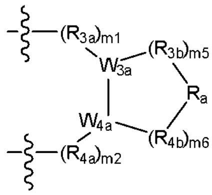

or

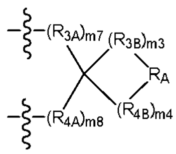

,

and $R_{37}$, $R_{40}$, and $R_{36}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R_{37}$,

$R_{40}$, and $R_{36}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; in one embodiment, $R_{37}$, $R_{40}$, and $R_{36}$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy, and hydroxyl;
or $R_{39}$ and $R_{40}$, together with the carbon atoms to which they are attached, form

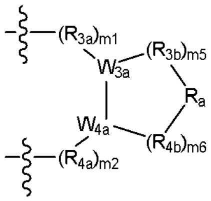 or 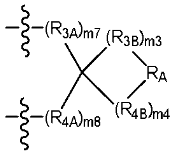 ,

and $R_{36}$, $R_{37}$, and $R_{38}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R_{36}$, $R_{37}$, and $R_{38}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; in one embodiment, $R_{36}$, $R_{37}$, and $R_{38}$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy, and hydroxyl;
or $R_{40}$ and $R_{36}$, together with the carbon atoms to which they are attached, form

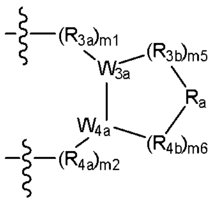 or 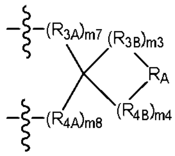 ,

deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R_{37}$, $R_{38}$, and $R_{39}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; in one embodiment, $R_{37}$, $R_{38}$, and $R_{39}$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy, and hydroxyl;
each occurrence of $R_{3a}$, $R_{3b}$, $R_{4a}$, $R_{4b}$, $R_{3A}$, $R_{3B}$, $R_{4A}$, and $R_{4B}$ is independently selected from $C(R^a)_2$, $NR^a$, C(O), O, and S, and for each occurrence of $R_{3A}$ or $R_{4A}$, at least one occurrence exists and at least one of them is O, for each occurrence of $R_{3a}$ or $R_{4a}$, at least one occurrence exists and at least one of them is O or $N(CH_3)$; in one embodiment, for each occurrence of $R_{3A}$ or $R_{4A}$, at least one occurrence exists and at least one of them is O, for each occurrence of $R_{3a}$ or $R_{4a}$, at least one occurrence exists and at least one of them is O;
each occurrence of $W_{3a}$ and $W_{4a}$ is independently $CR^a$ or N, and at least one of $W_{3a}$ and $W_{4a}$ is N;
each occurrence of $R_a$ and $R_A$ is independently $NR^{4h}$, C(=O), $SO_2$, or $CR^{2h}R^{3h}$; in one embodiment, each occurrence of $R_a$ and $R_A$ is independently $NR^{4h}$, $SO_2$, or $CR^{2h}R^{3h}$; in one embodiment, $R_A$ or $R_a$ is -NH or $-NC(O)CH_3$; and
$R^{4h}$ is selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkylaminocarbonyl, cycloalkyl, heterocyclyl, aryl, and heteroaryl; wherein the alkyl, heteroalkyl, alkoxy, cycloalkyl, heterocyclyl,

aryl, and heteroaryl are each independently and $R_{37}$, $R_{38}$, and $R_{39}$ are each independently selected from H, optionally substituted by one or more substituents selected from halogen, alkyl, heteroalkyl, alkenyl, alkynyl, alkoxy, haloalkyl, hydroxyl, hydroxyalkyl, cyano, amino, nitro, cycloalkyl, heterocyclyl, alkylamino, alkylacyl, alkylaminocarbonyl, aryl, and heteroaryl; and

when $R_{41}$ is selected from -$NR^aCO$-, and $R_{38}$ and $R_{39}$, together with the carbon atoms to which they are attached, form

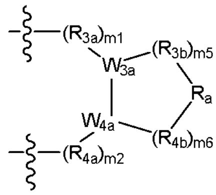

,

or $R_{39}$ and $R_{40}$, together with the carbon atoms to which they are attached, form

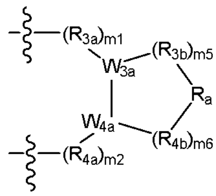

,

m1 is 2, the two occurrences of $R_{3a}$ are independently O and $CH_2$, and m2 is 0, at least one of $R_{37}$ and $R_{36}$ is not H, and $R_{36}$ is not F;

each occurrence of $R^m$, $R^{1h}$, $R^{2h}$, and $R^{3h}$ is selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkoxy, cycloalkyl, heterocyclyl, aryl, and heteroaryl are each independently optionally substituted by one or more substituents selected from halogen, alkyl, heteroalkyl, alkenyl, alkynyl, alkoxy, haloalkyl, hydroxyl, hydroxyalkyl, cyano, amino, nitro, cycloalkyl, heterocyclyl, alkylamino, alkylacyl, alkoxycarbonyl, alkylaminocarbonyl, aryl, and heteroaryl; in one embodiment, each occurrence of $R^m$, $R^{1h}$, $R^{2h}$, and $R^{3h}$ is independently selected from H, deuterium, halogen, alkyl, deuterated alkyl, haloalkyl, alkoxy, hydroxyl, and alkylacyl; in one embodiment, each occurrence of $R^m$, $R^{1h}$, $R^{2h}$, and $R^{3h}$ is independently selected from H, deuterium, F, Cl, Br, I, $C_1$-$C_3$ alkyl, $C_1$-$C_3$ deuterated alkyl, $C_1$-$C_3$ haloalkyl, hydroxyl, $C_1$-$C_3$ alkylacyl, and $C_1$-$C_3$ alkoxy;

each occurrence of m1 and m2 is independently an integer of 0, 1, 2, 3, 4, 5, or 6, and $m1 + m2 \leq 6$;

each occurrence of m3 is independently an integer of 0, 1, 2, 3, 4, 5, 6, or 7, m4 is an integer of 1, 2, 3, 4, 5, 6, 7, or 8, and $m3 + m4 \leq 8$;

each occurrence of m5 and m6 is independently an integer of 0, 1, 2, 3, 4, 5, 6, or 7, and $m5 + m6 \leq 7$;

each occurrence of m7 and m8 is independently an integer of 0, 1, 2, 3, 4, 5, 6, or 7, and $m7 + m8 \leq 7$;

each occurrence of m13 and m14 is independently an integer of 0, 1, 2, 3, 4, 5, 6, 7, or 8, and m13 and m14 are not 0 at the same time, $m13 + m14 \leq 8$;

each occurrence of m15 and m16 is independently an integer of 0, 1, 2, 3, 4, 5, 6, or 7, and $m15 + m16 \leq 7$;

each occurrence of m17 and m18 is independently an integer of 0, 1, 2, 3, 4, 5, or 6, and $m17 + m18 \leq 6$; and the compound is not

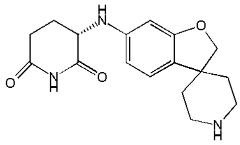, 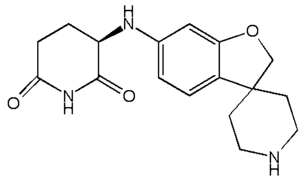, 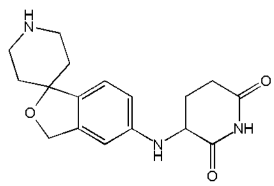,

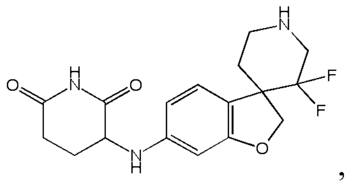, 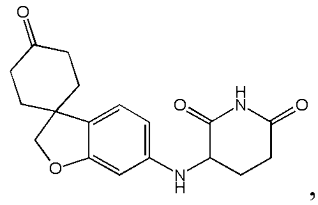, 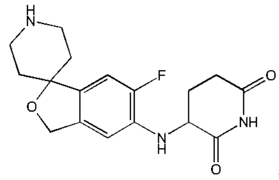,

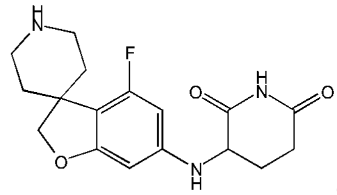, or 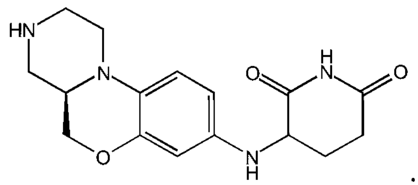.

In one embodiment, the compound is selected from:

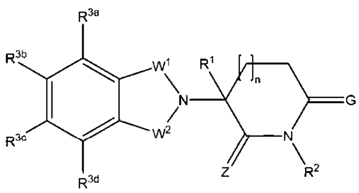

Formula IA,

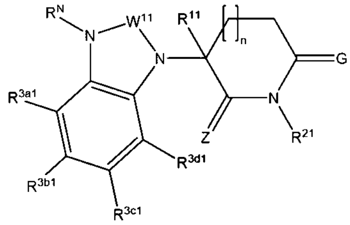

Formula 1B, and

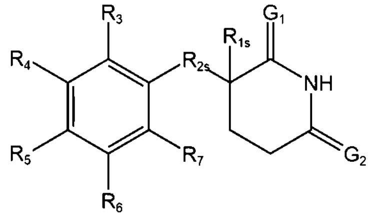

Formula 1C;

when the compound has the structure of Formula IA:

$W^1$ and $W^2$ are identical or different, and are independently $CR^aR^b$, C(=S), C(=O), $NR^a$, or $SO_2$, and at least one of $W^1$ and $W^2$ is C(=O);
G and Z are identical or different, independently selected from O, S, and Se; in one embodiment, G and Z are independently O;
$R^{3b}$ and $R^{3c}$, together with the carbon atoms to which they are attached, form

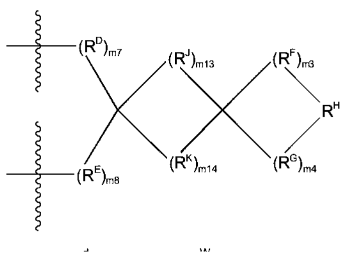, 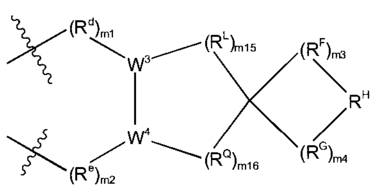, 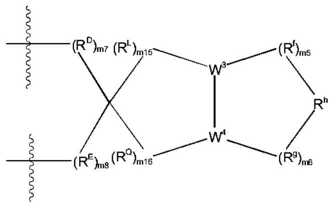,

or

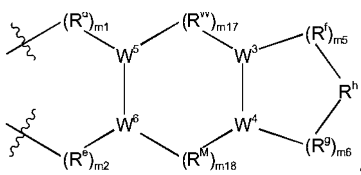,

and $R^{3a}$ and $R^{3d}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano,

amino, nitro, cycloalkyl, heterocyclyl, alkenyl, alkynyl, alkylamino, alkylacyl, alkoxycarbonyl, alkylaminocarbonyl, aryl, and heteroaryl;

or $R^{3c}$ and $R^{3d}$, together with the carbon atoms to which they are attached, form

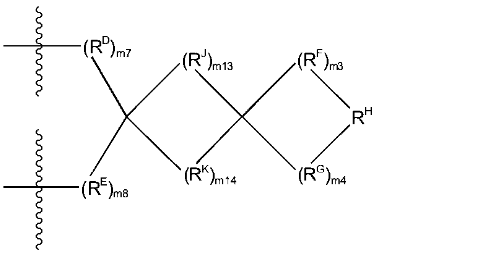

,

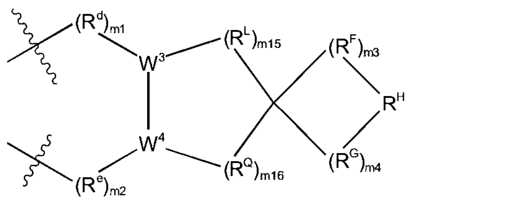

,

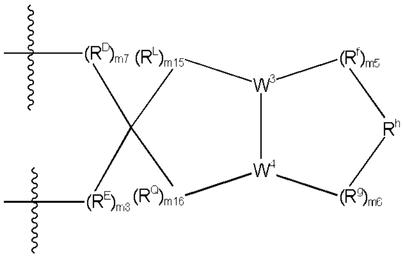

, or

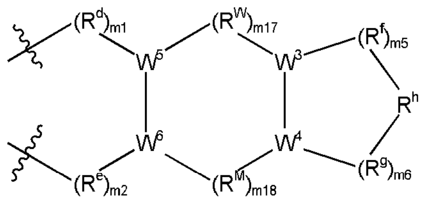

,

and $R^{3a}$ and $R^{3b}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl;

or $R^{3a}$ and $R^{3b}$, together with the carbon atoms to which they are attached, form

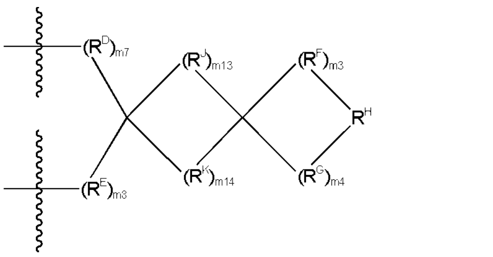

,

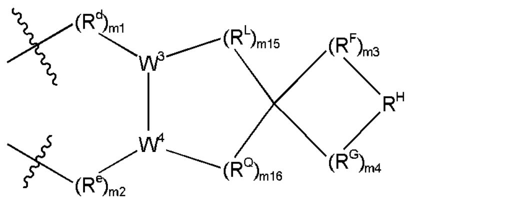

,

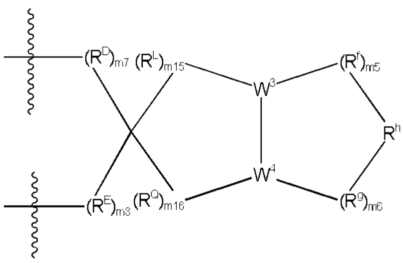

, or

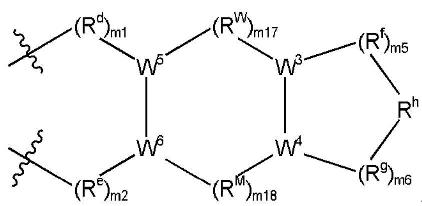

,

and $R^{3c}$ and $R^{3d}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano,

amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R^{3c}$ and $R^{3d}$ are each independently selected from H, deuterium, halogen, alkyl, deuterated alkyl, haloalkyl, alkoxy, and hydroxyl;

each occurrence of $R^d$, $R^e$, $R^f$, $R^g$, $R^D$, $R^E$, $R^F$, $R^J$, $R^K$, $R^L$, $R^Q$, $R^W$, $R^M$, and $R^G$ is independently $C(R^m)_2$, $NR^m$, C(=O), O, or S; in one embodiment, for each occurrence of $R^d$ and $R^e$, at least one occurrence exists and at least one of them is O, and for each occurrence of $R^D$ and $R^E$, at least one occurrence exists and at least one of them is O;

each occurrence of $W^3$, $W^4$, $W^5$ , and $W^6$ is independently $CR^m$ or N; in one embodiment, each occurrence of $W^3$, $W^4$, $W^5$, and $W^6$ is independently CH or N;

each occurrence of $R^h$ and $R^H$ is independently $NR^{1h}$, C(=O), $SO_2$, or $CR^{2h}R^{3h}$; in one embodiment, each occurrence of $R^h$ and $R^H$ is independently NH, C(=O), C(Cl)H, or C(OH)H;

each occurrence of m1 and m2 is independently an integer of 0, 1, 2, 3, 4, 5, or 6, and $m1 + m2 \leq 6$;

each occurrence of m3 is independently an integer of 0, 1, 2, 3, 4, 5, 6, or 7, m4 is an integer of 1, 2, 3, 4, 5, 6, 7, or 8, and $m3 + m4 \leq 8$;

each occurrence of m5 and m6 is independently an integer of 0, 1, 2, 3, 4, 5, 6, or 7, and $m5 + m6 \leq 7$;

each occurrence of m7 and m8 is independently an integer of 0, 1, 2, 3, 4, 5, 6, or 7, and $m7 + m8 \leq 7$;

each occurrence of m13 and m14 is independently an integer of 0, 1, 2, 3, 4, 5, 6, 7, or 8, and m13 and m14 are not 0 at the same time, and $m13 + m14 \leq 8$;

each occurrence of m15 and m16 is independently an integer of 0, 1, 2, 3, 4, 5, 6, or 7, and $m15 + m16 \leq 7$;

each occurrence of m17 and m18 is independently an integer of 0, 1, 2, 3, 4, 5, or 6, and $m17 + m18 \leq 6$;

each occurrence of $R^m$ is selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, each occurrence of $R^m$ is independently selected from H, deuterium, halogen, alkyl, deuterated alkyl, haloalkyl, and hydroxyl; in one embodiment, each occurrence of $R^m$ is independently selected from H, deuterium, F, Cl, Br, I, $C_1$-$C_3$ alkyl, $C_1$-$C_3$ deuterated alkyl, $C_1$-$C_3$ haloalkyl, and hydroxyl;

$R^{1h}$, $R^{2h}$, and $R^{3h}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkoxy, cycloalkyl, heterocyclyl, aryl, and heteroaryl are each independently optionally substituted by one or more substituents selected from halogen, alkyl, heteroalkyl, alkenyl, alkynyl, alkoxy, haloalkyl, hydroxyl, hydroxyalkyl, cyano, amino, nitro, cycloalkyl, heterocyclyl, alkylamino, alkylacyl, alkoxycarbonyl, alkylaminocarbonyl, aryl, and heteroaryl; in one embodiment, $R^{1h}$, $R^{2h}$, and $R^{3h}$ are each independently selected from H, deuterium, halogen, alkyl, deuterated alkyl, haloalkyl, hydroxyl, and alkylacyl; in one embodiment, each occurrence of $R^{1h}$, $R^{2h}$, and $R^{3h}$ is independently selected from H, deuterium, F, Cl, Br, I, $C_1$-$C_3$ alkyl, $C_1$-$C_3$ deuterated alkyl, $C_1$-$C_3$ haloalkyl, hydroxyl, and $C_1$-$C_3$ alkoxy;

$R^1$ is selected from H, halogen, deuterium, $C_1$-$C_6$ alkyl, $C_1$-$C_6$ alkoxy, hydroxyl, cycloalkyl, $C_1$-$C_6$ haloalkyl, and hydroxyalkyl, and in one embodiment, $R^1$ is selected from H, F, Cl, Br, I, $C_1$-$C_3$ alkyl, $C_1$-$C_3$ alkoxy, $C_3$-$C_6$ cycloalkyl, $C_1$-$C_3$ haloalkyl, hydroxyl, and $C_1$-$C_6$ hydroxyalkyl;

$R^2$, $R^a$, and $R^b$ are each independently selected from H, deuterium, F, Cl, Br, I, $C_1$-$C_6$ alkyl, hydroxyl, $C_1$-$C_6$ haloalkyl, $C_1$-$C_6$ hydroxyalkyl, and $C_1$-$C_6$ alkoxy; and

n is 0, 1, 2, or 3;

when the compound is selected from the structure of Formula 1B:

$W^{11}$ is selected from $CR^aR^b$, C(=O), C(=S), $NR^a$, or $SO_2$; in one embodiment, $W^{11}$ is C(=O);

G and Z are identical or different, and are each independently selected from O, S, and Se;

$R^{3b1}$ and $R^{3c1}$, together with the carbon atoms to which they are attached, form

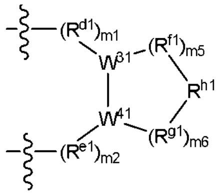

or

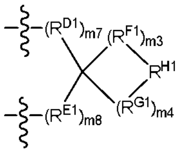

,

and $R^N$, $R^{3a1}$, and $R^{3d1}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R^N$, $R^{3a1}$, and $R^{3d1}$ are each independently selected from H, deuterium, halogen, alkyl, alkoxy, deuterated alkyl, haloalkyl, and hydroxyl;
or $R^{3c1}$ and $R^{3d1}$ together with the carbon atoms to which they are attached, form

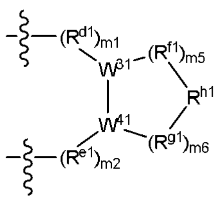
or
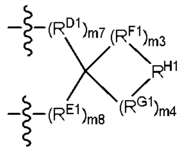
,

and $R^N$, $R^{3a1}$, and $R^{3b1}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R^N$, $R^{3a1}$, and $R^{3d1}$ are each independently selected from H, deuterium, halogen, alkyl, alkoxy, deuterated alkyl, haloalkyl, and hydroxyl;
or $R^{3a1}$ and $R^{3b1}$, together with the carbon atoms to which they are attached, form

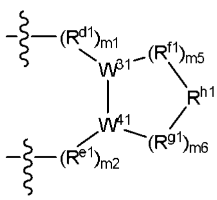
or
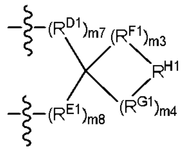
,

and $R^N$, $R^{3c1}$, and $R^{3d1}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R^N$, $R^{3c1}$, and $R^{3d1}$ are each independently selected from H, deuterium, halogen, alkyl, alkoxy, deuterated alkyl, haloalkyl, and hydroxyl;
each occurrence of $R^{d1}$, $R^{e1}$, $R^{f1}$, $R^{g1}$, $R^{D1}$, $R^{E1}$, $R^{F1}$, and $R^{G1}$ is independently $C(R^m)_2$, $NR^m$, C(=O), O, or S; in one embodiment, for each occurrence of $R^{d1}$ and $R^{e1}$, at least one occurrence exists and at least one of them is O, and for each occurrence of $R^{D1}$ and $R^{E1}$, at least one occurrence exists and at least one of them is O;
each occurrence of $W^{31}$ and $W^{41}$ is independently $CR^m$ or N; in one embodiment, each occurrence of $W^{31}$ and $W^{41}$ is independently CH or N;
each occurrence of $R^{h1}$ and $R^{H1}$ is independently $NR^{1h}$, C(=O), $SO_2$, or $CR^{2h}R^{3h}$; in one embodiment, each occurrence of $R^{h1}$ and $R^{H1}$ is independently NH, C=O, CHCl, or CHOH;
each occurrence of m1 and m2 is independently an integer of 0, 1, 2, 3, 4, 5, or 6, and $m1 + m2 \leq 6$;
each occurrence of m3 is independently an integer of 0, 1, 2, 3, 4, 5, 6, or 7, m4 is an integer of 1, 2, 3, 4, 5, 6, 7, or 8, and $m3 + m4 \leq 8$;
each occurrence of m5 and m6 is independently an integer of 0, 1, 2, 3, 4, 5, 6, or 7, and $m5 + m6 \leq 7$;
each occurrence of m7 and m8 is independently an integer of 0, 1, 2, 3, 4, 5, 6, or 7, and $m7 + m8 \leq 7$;
each occurrence of $R^m$ is independently selected from H, deuterium, halogen, alkyl, deuterated alkyl, heteroalkyl, alkenyl, alkynyl, alkoxy, haloalkyl, haloalkoxy, hydroxy, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl,

alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, each occurrence of $R^m$ is independently selected from H, deuterium, halogen, alkyl, deuterated alkyl, haloalkyl, and hydroxyl; in one embodiment, each occurrence of $R^m$ is independently selected from H, deuterium, F, Cl, Br, I, $C_1$-$C_3$ alkyl, hydroxyl, and $C_1$-$C_3$ alkoxy;

$R^{1h}$, $R^{2h}$, and $R^{3h}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkoxy, cycloalkyl, heterocyclyl, aryl, and heteroaryl are each independently optionally substituted by one or more substituents selected from halogen, alkyl, heteroalkyl, alkenyl, alkynyl, alkoxy, haloalkyl, hydroxyl, hydroxyalkyl, cyano, amino, nitro, cycloalkyl, heterocyclyl, alkylamino, alkylacyl, alkoxycarbonyl, alkylaminocarbonyl, aryl, and heteroaryl; in one embodiment, each occurrence of $R^{1h}$, $R^{2h}$, and $R^{3h}$ is independently selected from H, deuterium, halogen, alkyl, deuterated alkyl, haloalkyl, hydroxyl, and alkylacyl; in one embodiment, each occurrence of $R^{1h}$, $R^{2h}$, and $R^{3h}$ is independently selected from H, deuterium, F, Cl, Br, I, $C_1$-$C_3$ alkyl, hydroxyl, and $C_1$-$C_3$ alkoxy;

$R^{11}$ is selected from H, halogen, deuterium, $C_1$-$C_6$ alkyl, $C_1$-$C_6$ alkoxy, hydroxyl, cycloalkyl, $C_1$-$C_6$ haloalkyl, and hydroxyalkyl, and in one embodiment, $R^1$ is selected from H, F, Cl, Br, I, $C_1$-$C_3$ alkyl, $C_1$-$C_3$ alkoxy, $C_3$-$C_6$ cycloalkyl, $C_1$-$C_3$ haloalkyl, hydroxyl, and $C_1$-$C_6$ hydroxyalkyl;

$R^{21}$, $R^a$, and $R^b$ are each independently selected from H, deuterium, F, Cl, Br, I, $C_1$-$C_6$ alkyl, hydroxyl, $C_1$-$C_6$ haloalkyl, $C_1$-$C_6$ hydroxyalkyl, and $C_1$-$C_6$ alkoxy; and

n is 0, 1, 2, or 3; in one embodiment, n is 0, 1, or 2;

when the compound is selected from the structure of Formula 1C:

$G_1$ and $G_2$ are each independently selected from O, S, and Se; in one embodiment, $G_1$ and $G_2$ are each independently O;

$R_{2s}$ is selected from -$NR_{2A}$, -$NR_{2A}$CO-, $C_1$-$C_6$ alkylene, and $C_1$-$C_6$ haloalkylene; in one embodiment, $R_{2s}$ is selected from -NH-CO-, -NH-, and -N($CH_3$)-;

$R_3$ and $R_4$, together with the carbon atoms to which they are attached, form

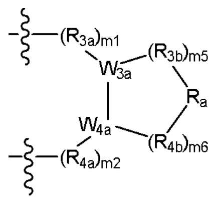

or

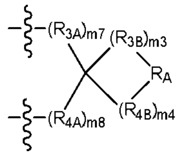

,

and $R_5$, $R_6$, and $R_7$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R_5$, $R_6$, and $R_7$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; in one embodiment, $R_5$, $R_6$, and $R_7$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy, and hydroxyl;

or $R_4$ and $R_5$, together with the carbon atoms to which they are attached, form

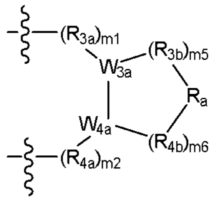 or 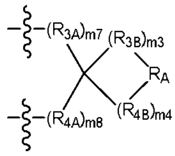 ,

and $R_3$, $R_6$, and $R_7$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R_3$, $R_6$, and $R_7$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; in one embodiment, $R_3$, $R_6$, and $R_7$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy, and hydroxyl;

or $R_5$ and $R_6$, together with the carbon atoms to which they are attached, form

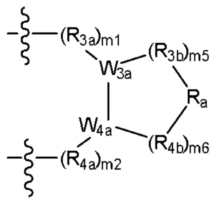 or 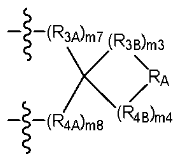 ,

and $R_3$, $R_4$, and $R_7$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R_3$, $R_4$, and $R_7$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; in one embodiment, $R_3$, $R_4$, and $R_7$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy, and hydroxyl;

or $R_6$ and $R_7$, together with the carbon atoms to which they are attached, form

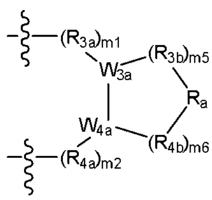 or 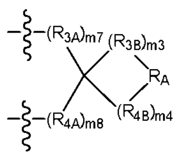 ,

and $R_3$, $R_4$, and $R_5$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; in one embodiment, $R_3$, $R_4$, and $R_5$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; in one embodiment, $R_3$, $R_4$,

and $R_5$ are each independently selected from H, deuterium,F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy, and hydroxyl;
each occurrence of $R_{3a}$, $R_{3b}$, $R_{4a}$, $R_{4b}$, $R_{3A}$, $R_{3B}$, $R_{4A}$, and $R_{4B}$ is independently selected from $C(R_{2A})_2$, $NR_{2A}$, C(O), O, and S; in one embodiment, for each occurrence of $R_{3A}$ or $R_{4A}$, at least one occurrence exists and at least one of them is O or N, and for each occurrence of $R_{3a}$ or $R_{4a}$, at least one occurrence exists and at least one of them is O;
each occurrence of $W_{3a}$ and $W_{4a}$ is independently $CR_{2A}$ or N, and at least one of $W_{3a}$ and $W_{4a}$ is N, and

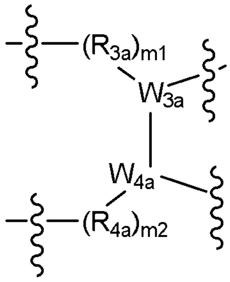
or
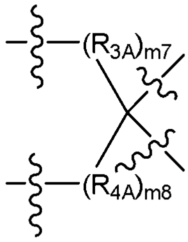

contains at least one of O or N;
each occurrence of $R_a$ and $R_A$ is independently $C(R_{2A})_2$, $NR_{2A}$, or C(O); in one embodiment, each occurrence of $R_a$ and $R_A$ is independently -NH or $-NC(O)CH_3$;
each occurrence of m1 and m2 is independently an integer of 0, 1, 2, 3, 4, 5, or 6, and $m1 + m2 \leq 6$;
each occurrence of m3 and m4 is independently an integer of 0, 1, 2, 3, 4, 5, 6, 7, or 8, and $m3 + m4 \leq 8$, and m3 and m4 are not 0 at the same time;
each occurrence of m5 and m6 is independently an integer of 0, 1, 2, 3, 4, 5, 6, or 7, and $m5 + m6 \leq 7$;
each occurrence of m7 and m8 is independently an integer of 0, 1, 2, 3, 4, 5, 6, or 7, and $m7 + m8 \leq 7$;
each occurrence of $R_{2A}$ and $R_{1s}$ is independently selected from H, halogen, deuterium, $C_1$-$C_6$ alkyl, $C_1$-$C_6$ alkoxy, hydroxyl, cycloalkyl, $C_1$-$C_6$ haloalkyl, $-C(O)-CH_3$, and hydroxyalkyl; in one embodiment, each occurrence of $R_{2a}$ and $R_{1s}$ is independently selected from H, F, Cl, Br, I, $C_1$-$C_3$ alkyl, $C_1$-$C_3$ alkoxy, $C_3$-$C_6$ cycloalkyl, $C_1$-$C_3$ haloalkyl, hydroxyl, $-C(O)-CH_3$, and $C_1$-$C_6$ hydroxyalkyl;
when $R_{2s}$ is selected from $-NR_{2A}CO-$, and $R_4$ and $R_5$ , together with the carbon atoms to which they are attached, form

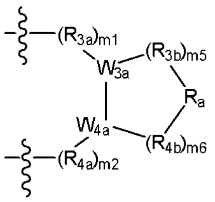
,

or $R_5$ and $R_6$, together with the carbon atoms to which they are attached, form

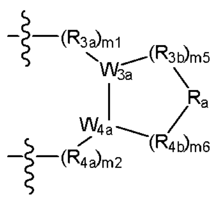
,

m1+m2=2, at least one of $R_3$ and $R_7$ is not H, and $R_7$ is not F; and
the compound is not

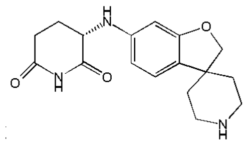, 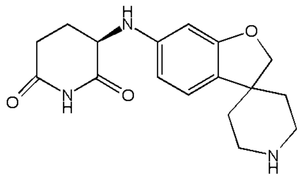, 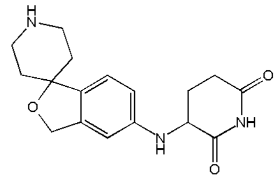,

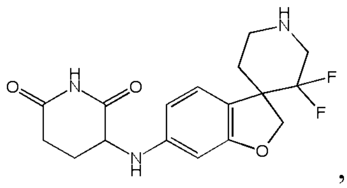, 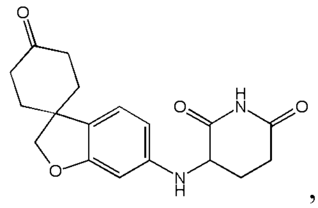, 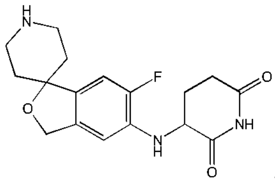,

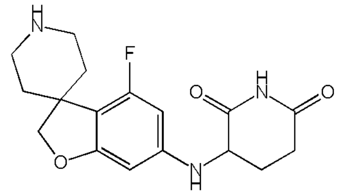, or 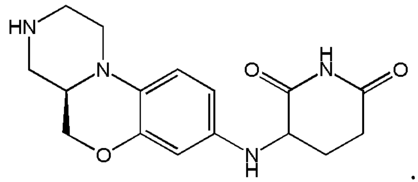.

In one embodiment, wherein:

when the compound is of the structure of Formula IA, $W^1$ and $W^2$ are identical or different, and are each independently C(=S), $CH_2$, or C(=O), and at least one of $W^1$ and $W^2$ is C(=O); in one embodiment, when $W^1$ is $CH_2$, and when $W^2$ is C(=O) or $W^1$ is C(=O), $W^2$ is $CH_2$ or C(=O); in one embodiment, when $W^1$ is $CH_2$, and when $W^2$ is C(=O) or $W^1$ 为 C(=O), $W^2$ is $CH_2$;

in one embodiment, G and Z are O;

in one embodiment, $R^{3b}$ and $R^{3c}$, together with the carbon atoms to which they are attached, form

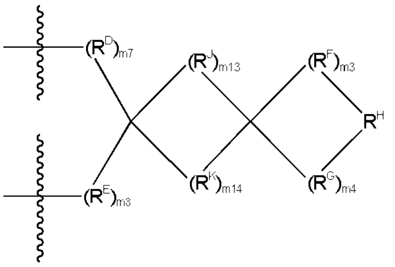, 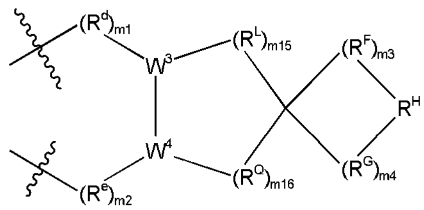,

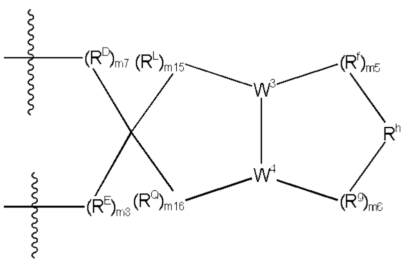, or 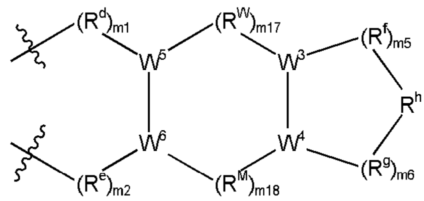,

and $R^{3a}$ and $R^{3d}$ are each independently selected from H, deuterium, halogen, $C_1$-$C_3$ alkyl, and $C_1$-$C_3$ alkoxy; in one embodiment, $R^{3a}$ and $R^{3d}$ are each independently selected from H, deuterium, F, Cl, Br, I, $C_1$-$C_3$ alkyl, and $C_1$-$C_3$ alkoxy;

or $R^{3a}$ and $R^{3b}$, together with the carbon atoms to which they are attached, form

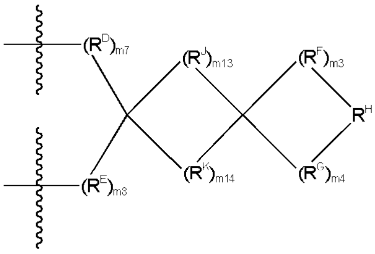
,
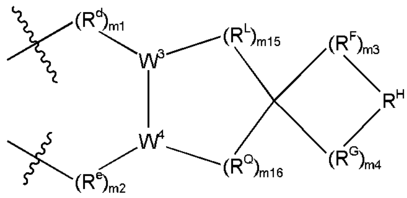
,

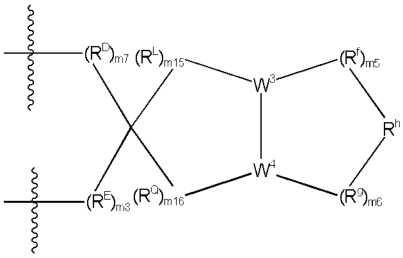
, or
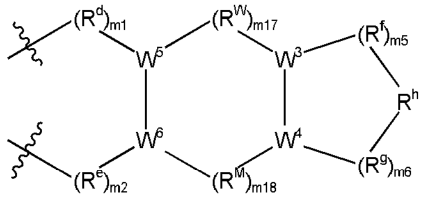
,

and $R^{3c}$ and $R^{3d}$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, $R^{3c}$ and $R^{3d}$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, $R^{3c}$ and $R^{3d}$ are each independently selected from H, deuterium, halogen, $C_1$-$C_3$ alkyl, and $C_1$-$C_3$ alkoxy; in one embodiment, $R^{3c}$ and $R^{3d}$ are each independently selected from H, deuterium, F, Cl, Br, I, $C_1$-$C_3$ alkyl, and $C_1$-$C_3$ alkoxy;

in one embodiment, each occurrence of $R^d$, $R^e$, $R^f$, $R^g$, $R^D$, $R^E$, $R^F$, $R^J$, $R^K$, $R^L$, $R^Q$, $R^W$, $R^M$, and $R^G$ is independently $C(R^m)_2$ or O; in one embodiment, each occurrence of $R^d$, $R^e$, $R^f$, $R^g$, $R^D$, $R^E$, $R^F$, $R^J$, $R^K$, $R^L$, $R^Q$, $R^W$, $R^M$, and $R^G$ is independently $CF_2$, $CH_2$, or O; in one embodiment, for each occurrence of $R^d$ and $R^e$, at least one occurrence exists and at least one of them is O, and for each occurrence of $R^D$ and $R^E$, at least one occurrence exists and at least one of them is O; in one embodiment, for each occurrence of $R^d$, and $R^e$, one occurrence exists and at least one of them is O, and for each occurrence of $R^D$ and $R^E$, one occurrence exists and at least one of them is O; in one embodiment, $R^d$ and $R^e$, one occurrence exists and one of them is O, and for each occurrence of $R^D$ and $R^E$, one occurrence exists and one of them is O;

in one embodiment, $R^h$ and $R^H$ are $NR^{1h}$, C(=O), or $CR^{2h}R^{3h}$; $R^{1h}$, $R^{2h}$, and $R^{3h}$ are each independently selected from H, deuterium, halogen, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, $R^{1h}$, $R^{2h}$, and $R^{3h}$ are each independently selected from H, deuterium, F, Cl, Br, I, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$

heteroaryl; in one embodiment, $R^{1h}$, $R^{2h}$, and $R^{3h}$ are each independently selected from H, deuterium, halogen, $C_1$-$C_3$ alkyl, hydroxyl, and $C_1$-$C_3$ alkoxy; in one embodiment, $R^{1h}$, $R^{2h}$, and $R^{3h}$ are each independently selected from H, deuterium, F, Cl, Br, I, $C_1$-$C_3$ alkyl, hydroxyl, and $C_1$-$C_3$ alkoxy; in one embodiment, $R^h$ and $R^H$ are selected from NH, C(=O), C(Cl)H, and C(OH)H;

in one embodiment, each occurrence of m1 and m2 is independently an integer of 0, 1, 2, or 3, and $m1 + m2 \leq 3$; in one embodiment, m1 + m2 = 1, m1 + m2 = 2, or m1 + m2 = 3;

in one embodiment, each occurrence of m3 is independently an integer of 0, 1, 2, 3, or 4, m4 is an integer of 1, 2, 3, 4, or 5, and $m3 + m4 \leq 5$; in one embodiment, m3 + m4 = 1, m3 + m4 = 2, m3 + m4 = 3, or m3 + m4 = 4;

in one embodiment, each occurrence of m5 and m6 is independently an integer of 0, 1, 2, 3, or 4, and $m5 + m6 \leq 4$; in one embodiment, m5 + m6 = 2 or m5 + m6 = 3;

in one embodiment, each occurrence of m7 and m8 is independently an integer of 0, 1, 2, 3, or 4, and $m7 + m8 \leq 4$; In one embodiment, m7 + m8 = 2 or m7 + m8 = 3;

in one embodiment, each occurrence of m13 and m14 is independently an integer of 0, 1, 2, 3, or 4, m13 and m14 are not 0 at the same time, and $m13 + m14 \leq 5$; in one embodiment, m13 + m14 = 3 or m13 + m14 = 4; In one embodiment, both m13 and m14 are 2;

in one embodiment, each occurrence of m15 and m16 is independently an integer of 0, 1, 2, 3, or 4, and $m15 + m16 \leq 4$; in one embodiment, m15 + m16 = 2 or m15 + m16 = 3;

in one embodiment, each occurrence of m17 and m18 is independently an integer of 0, 1, 2, 3, or 4, and $m17 + m18 \leq 3$; in one embodiment, m17 + m18 = 1 or m17 + m18 = 2;

in one embodiment, each occurrence of $R^m$ is independently selected from H, deuterium, halogen, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, each occurrence of $R^m$ is independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, each occurrence of $R^m$ is independently selected from H, deuterium, halogen, $C_1$-$C_3$ alkyl, and $C_1$-$C_3$ alkoxy; in one embodiment, each occurrence of $R^m$ is independently selected from H, deuterium, F, Cl, Br, I, $C_1$-$C_3$ alkyl, and $C_1$-$C_3$ alkoxy; in one embodiment, $R^1$ is selected from H, halogen, $C_1$-$C_3$ alkyl, and hydroxyl;

in one embodiment, $R^1$ is selected from H, F, Cl, Br, I, $C_1$-$C_3$ alkyl, and hydroxyl;

in one embodiment, $R^2$ is selected from H and $C_1$-$C_3$ alkyl;

in one embodiment, n is 0 or 1; or

when the compound is of the structure of Formula 1B,

$W^{11}$ is selected from $CH_2$, C(=S), or C(=O); in one embodiment, $W^{11}$ is C(=O), or C(=S); in one embodiment, $W^{11}$ is C(=O);

in one embodiment, G and Z are O;

in one embodiment, $R^N$ is selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, aryl, and heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, aryl, and heteroaryl; in one embodiment, $R^N$ is selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ haloalkyl, $C_1$-$C_6$ hydroxyalkyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, $R^N$ is selected from H, deuterium, F, Cl, Br, I, $C_1$-$C_6$ alkyl, hydroxyl, $C_1$-$C_6$ haloalkyl, $C_1$-$C_6$ hydroxyalkyl, $C_1$-$C_6$ alkoxy, $C_3$-$C_8$ cycloalkyl, and $C_4$-$C_8$ heterocyclyl containing 1, 2, 3, or 4 heteroatoms independently selected from N, O, P, and S, wherein the $C_1$-$C_6$ alkyl, $C_1$-$C_6$ haloalkyl, $C_1$-$C_6$ hydroxyalkyl, $C_1$-$C_6$ alkoxy, $C_3$-$C_8$ cycloalkyl, and $C_4$-$C_8$ heterocyclyl containing 1, 2, 3, or 4

heteroatoms independently selected from N, O, P, and S, are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, $R^{3b1}$ and $R^{3c1}$, together with the carbon atoms to which they are attached, form

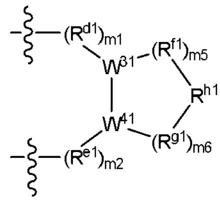
or
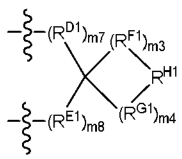
,

and $R^{3a1}$ and $R^{3d1}$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, aryl, and heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, aryl, and heteroaryl; in one embodiment, $R^{3a1}$ and $R^{3d1}$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, , $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, $R^{3a1}$ and $R^{3a1}$ are each independently selected from H, deuterium, halogen, $C_1$-$C_3$ alkyl, and $C_1$-$C_3$ alkoxy; or $R^{3c1}$ and $R^{3d1}$, together with the carbon atoms to which they are attached, form

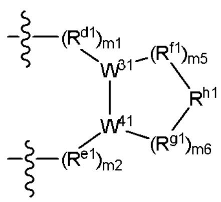

or

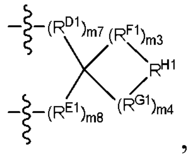
,

and $R^{3a1}$ and $R^{3b1}$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, $R^{3a1}$ and $R^{3b1}$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, $R^{3a1}$ and $R^{3b1}$ are each independently selected from H, deuterium, halogen, $C_1$-$C_3$ alkyl, and $C_1$-$C_3$

alkoxy; or $R^{3a1}$ and $R^{3b1}$, together with the carbon atoms to which they are attached, form

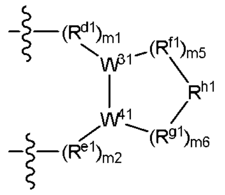 or 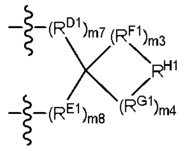 ,

and $R^{3c1}$ and $R^{3d1}$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, $R^{3c1}$ and $R^{3d1}$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, $R^{3c1}$ and $R^{3a1}$ are each independently selected from H, deuterium, halogen, $C_1$-$C_3$ alkyl, and $C_1$-$C_3$ alkoxy;

in one embodiment, each occurrence of $R^{d1}$, $R^{e1}$, $R^{f1}$, $R^{g1}$, $R^{D1}$, $R^{E1}$, $R^{F1}$, and $R^{G1}$ is independently selected from $C(R^m)_2$ or O; each occurrence of $R^m$ is independently selected from H, deuterium, F, Cl, Br, I, $C_1$-$C_3$ alkyl, and $C_1$-$C_3$ alkoxy;

in one embodiment, $W^{31}$ and $W^{41}$ are CH or N;

in one embodiment, each occurrence of m1 and m2 is independently an integer of 0, 1, 2, or 3, and m1 + m2 ≤ 3; in one embodiment, m1 + m2 = 1, m1 + m2 = 2, or m1 + m2 = 3;

in one embodiment, each occurrence of m3 is independently an integer of 0, 1, 2, 3, or 4, m4 is an integer of 1, 2, 3, 4, or 5, and m3 + m4 ≤ 5; in one embodiment, m3 + m4 = 1, m3 + m4 = 2, m3 + m4 = 3, or m3 + m4 = 4; in one embodiment, each occurrence of m5 and m6 is independently an integer of 0, 1, 2, 3, or 4, and m5 + m6 ≤ 4; in one embodiment, m5 + m6 = 2 or m5 + m6 = 3;

in one embodiment, each occurrence of m7 and m8 is independently an integer of 0, 1, 2, 3, or 4, and m7 + m8 ≤ 4; in one embodiment, m7 + m8 = 2 or m7 + m8 = 3;

in one embodiment, $R^{11}$ is selected from H, halogen, $C_1$-$C_3$ alkyl, and hydroxyl; $R^{11}$ is selected from H, F, Cl, Br, I, $C_1$-$C_3$ alkyl, and hydroxyl;

in one embodiment, $R^{21}$ is selected from H and $C_1$-$C_3$ alkyl; or

when the compound is of the structure of Formula 1C,

$G_1$ and $G_2$ are each independently selected from O;

in one embodiment, $R_{2s}$ is selected from $-NR_{2A}-$, $-NR_{2A}CO-$; each occurrence of $R_{2A}$ is each independently selected from H and $C_1$-$C_6$ alkyl; in one embodiment, $R_{2s}$ is selected from -NH-CO-, -NH-, and $-N(CH_3)-$;

in one embodiment, $R_3$ and $R_4$, together with the carbon atoms to which they are attached, form

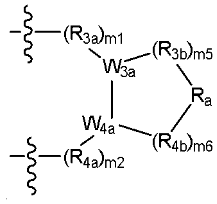 or 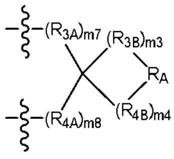 ,

and $R_5$, $R_6$, and $R_7$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, aryl, and heteroaryl are each independently optionally substituted by one or more substituents selected from halogen, $C_1$-$C_6$ alkyl, $C_1$-$C_6$ heteroalkyl, $C_1$-$C_6$

alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, aryl, and heteroaryl; in one embodiment, $R_5$, $R_6$, and $R_7$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, $R_5$, $R_6$, and $R_7$ are each independently selected from H, deuterium, halogen, $C_1$-$C_3$ alkyl, and $C_1$-$C_3$ alkoxy; in one embodiment, $R_5$, $R_6$, and $R_7$ are each independently selected from H, deuterium, F, Cl, Br, I, $C_1$-$C_3$ alkyl, and $C_1$-$C_3$ alkoxy; or $R_4$ and $R_5$, together with the carbon atoms to which they are attached, form

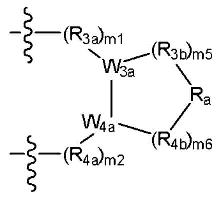
or
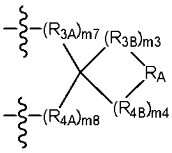
,

and $R_3$, $R_6$, and $R_7$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, aryl, and heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, aryl, and heteroaryl; in one embodiment, $R_3$, $R_6$, and $R_7$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, $R_3$, $R_6$, and $R_7$ are each independently selected from H, deuterium, halogen, $C_1$-$C_3$ alkyl, and $C_1$-$C_3$ alkoxy; in one embodiment, $R_3$, $R_6$, and $R_7$ are each independently selected from H, deuterium, F, Cl, Br, I, $C_1$-$C_3$ alkyl, and $C_1$-$C_3$ alkoxy; or $R_5$ and $R_6$, together with the carbon atoms to which they are attached, form

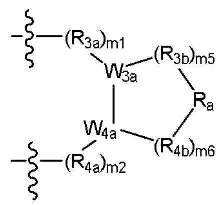
or
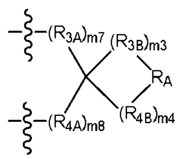
,

and $R_3$, $R_4$, and $R_7$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, aryl, and heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, aryl, and heteroaryl; in one embodiment, $R_3$, $R_4$, and $R_7$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, $R_3$, $R_4$, and $R_7$ are each independently selected from H, deuterium, F, Cl, Br, I, $C_1$-$C_3$ alkyl, and $C_1$-$C_3$ alkoxy; or $R_6$ and $R_7$, together with the carbon atoms to

which they are attached, form

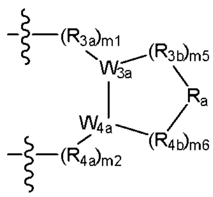 or 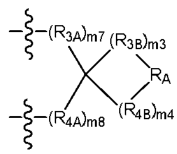 ,

and $R_3$, $R_4$, and $R_5$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, aryl, and heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, aryl, and heteroaryl; in one embodiment, $R_3$, $R_4$, and $R_5$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; in one embodiment, $R_3$, $R_4$, and $R_5$ are each independently selected from H, deuterium, halogen, $C_1$-$C_3$ alkyl, and $C_1$-$C_3$ alkoxy; in one embodiment, $R_3$, $R_4$, and $R_5$ are each independently selected from H, deuterium, F, Cl, Br, I, $C_1$-$C_3$ alkyl, and $C_1$-$C_3$ alkoxy;

in one embodiment, each occurrence of $R_{3a}$, $R_{3b}$, $R_{4a}$, $R_{4b}$, $R_{3A}$, $R_{3B}$, $R_{4A}$, and $R_{4B}$ is independently selected from $C(R_{2A})_2$, $NR_{2A}$, and O;

in one embodiment, each occurrence of $W_{3a}$ and $W_{4a}$ is independently $C(R_{2A})_2$ or N;

in one embodiment, each occurrence of $R_a$ and $R_A$ is independently $NR_{2A}$ or $CR^{2h}R^{3h}$; in one embodiment, each occurrence of $R_a$ and $R_a$ is -NH or $-NC(O)CH_3$;

in one embodiment, each occurrence of $R_{2A}$ and $R_{1s}$ is independently selected from H, halogen, deuterium, $C_1$-$C_6$ alkyl, $C_1$-$C_6$ alkoxy, hydroxyl, cycloalkyl, $C_1$-$C_6$ haloalkyl, and hydroxyalkyl; in one embodiment, each occurrence of $R_{2A}$ and $R_{1s}$ is independently selected from H, F, Cl, Br, I, $C_1$-$C_3$ alkyl, $C_1$-$C_3$ alkoxy, $C_3$-$C_6$ cycloalkyl, $C_1$-$C_3$ haloalkyl, hydroxyl, and $C_1$-$C_6$ hydroxyalkyl.

In one embodiment, the compound is selected from the following structures:

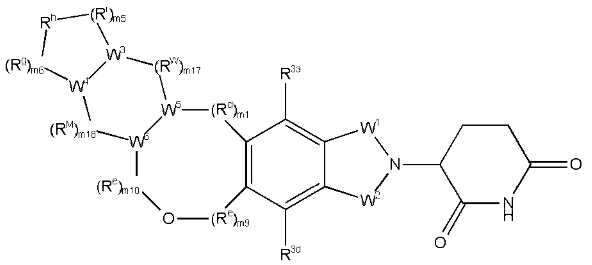

,

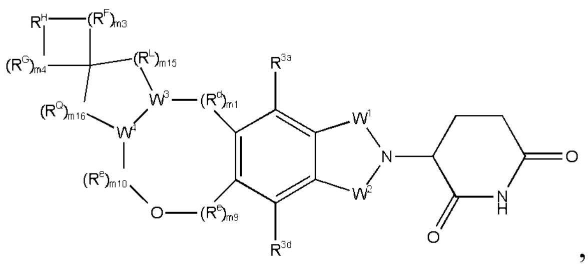

,

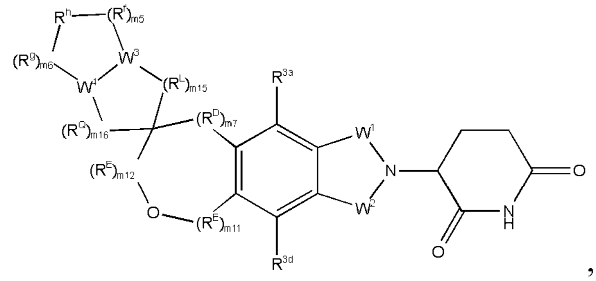,
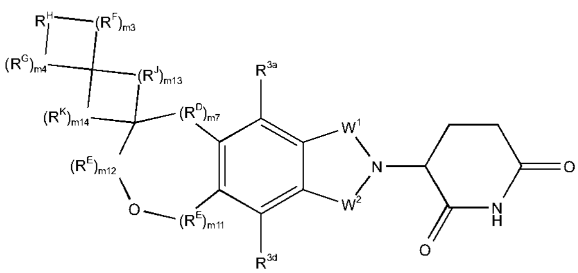

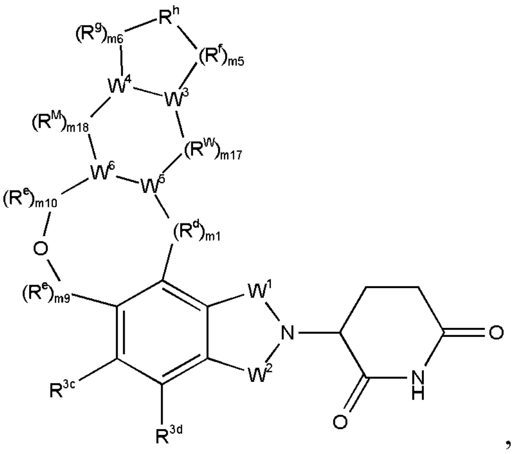,
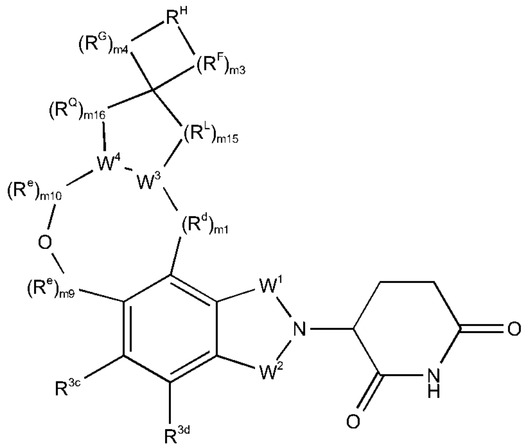,

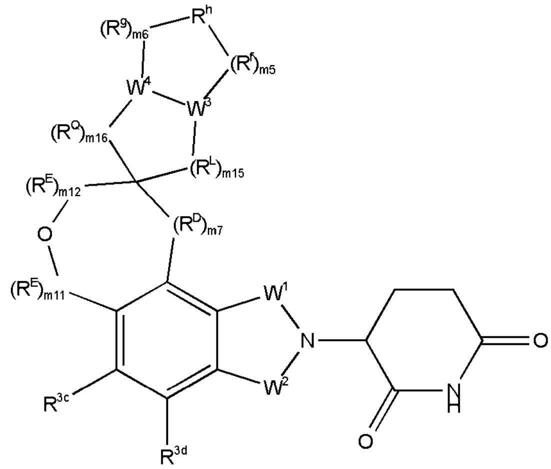,
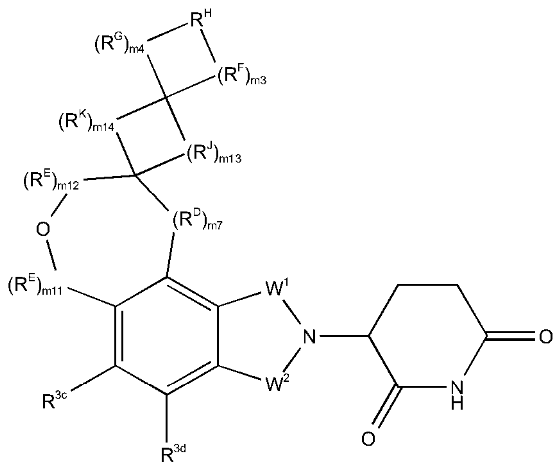

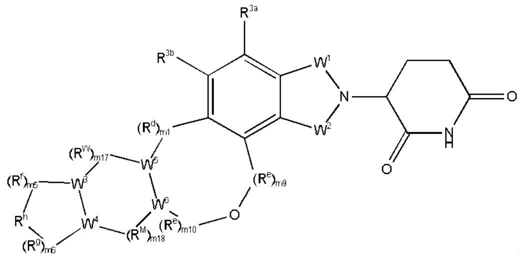,
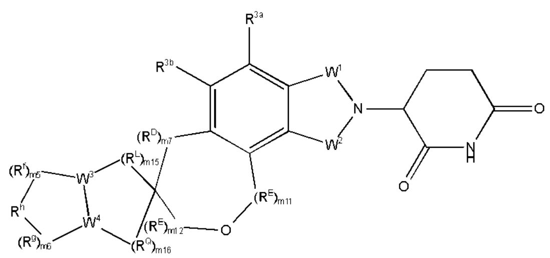

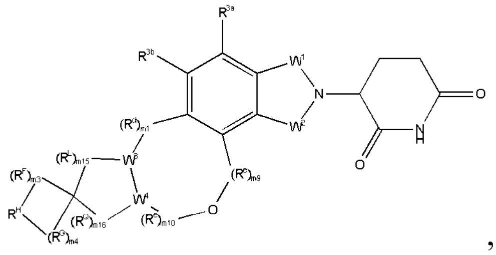,
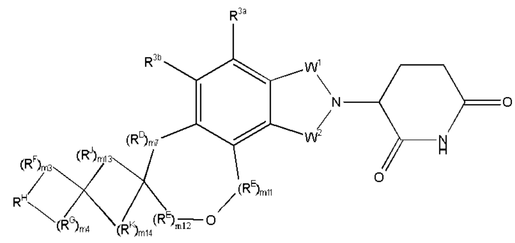,

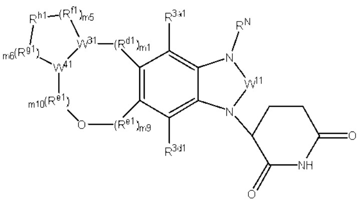,
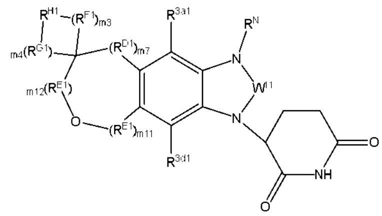,
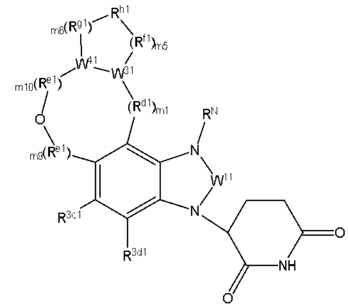,

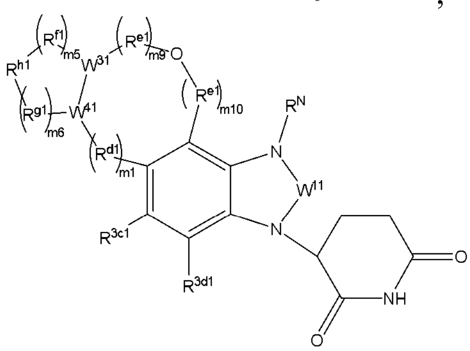,
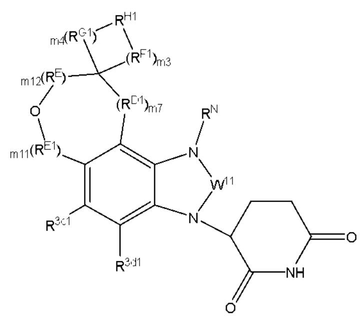,

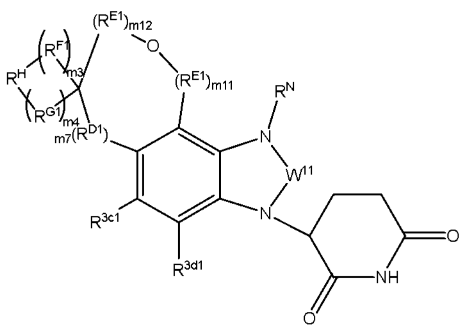,
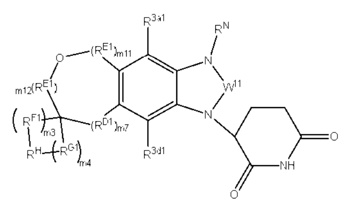,
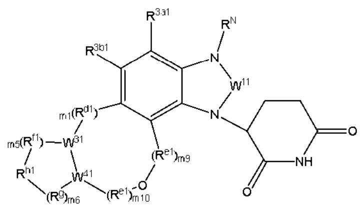,

and

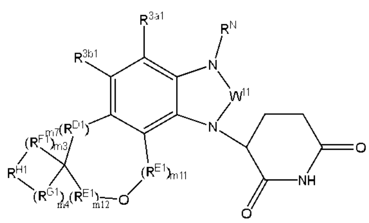;

wherein:

$R^{H1}$, $R^H$, $R^h$, $R^f$, $R^g$, $R^W$, $R^M$, $W^3$, $W^4$, $W^5$, $W^6$, $R^d$, $R^e$, $R^{3a}$, $R^{3b}$, R3c, $R^{3d}$, $W^1$, $W^2$, $R^F$, $R^G$, $R^J$, $R^K$, $R^L$, $R^Q$, $R^{f1}$, $R^{g1}$, $W^{31}$, $W^{41}$, $R^{d1}$, $R^{e1}$, $R^{3a1}$, $R^{3b1}$, $R^{3c1}$, $R^{3d1}$, $W^{11}$, m3, m4, $R^{E1}$, $R^{D1}$, $R^{G1}$, $R^{F1}$, m2, m5, m6, m8, m13, m14, m15, m16, m17,

m18, $W^{11}$, $W^{31}$, $W^{41}$, $R^N$, and $R^{h1}$ are respectively as defined above;
each occurrence of m1, m9, and m10 is independently an integer of 0, 1, 2, 3, 4, or 5, and $m1 + m9 + m10 \leq 5$; in one embodiment, each occurrence of m1, m9, and m10 is independently an integer of 0, 1, or 2, and $m1 + m9 + m10 \leq 2$; in one embodiment, $m1 + m9 + m10 = 1$ or $m1 + m9 + m10 = 0$; and
each occurrence of m7, m11, and m12 is independently an integer of 0, 1, 2, 3, 4, 5, or 6, and $m7 + m11 + m12 \leq 6$; in one embodiment, each occurrence of m7, m11, and m12 is independently an integer of 0, 1, 2, or 3, and $m7 + m11 + m12 \leq 3$; in one embodiment, $m7 + m11 + m12 = 2$ or $m7 + m11 + m12 = 1$.

In one embodiment, the compound is selected from the following structures:

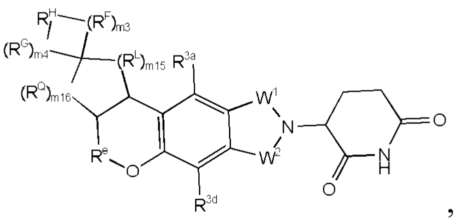
,
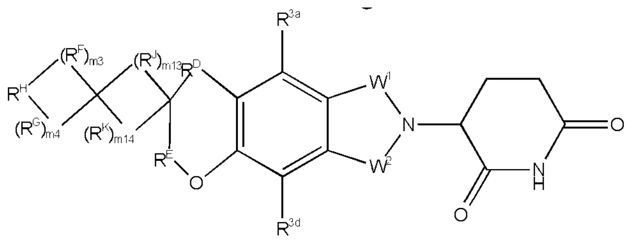
,
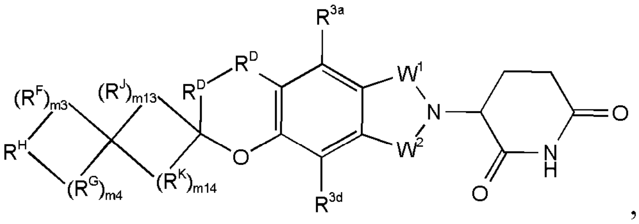
,
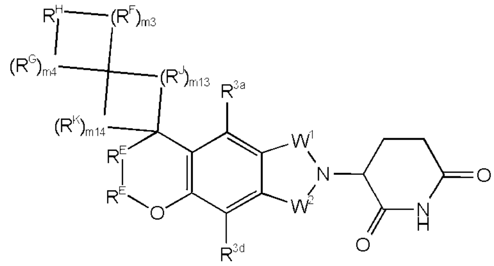
,
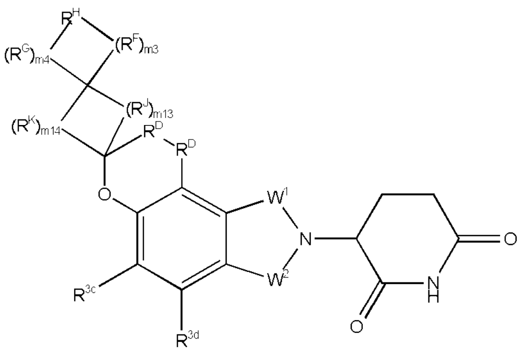
,
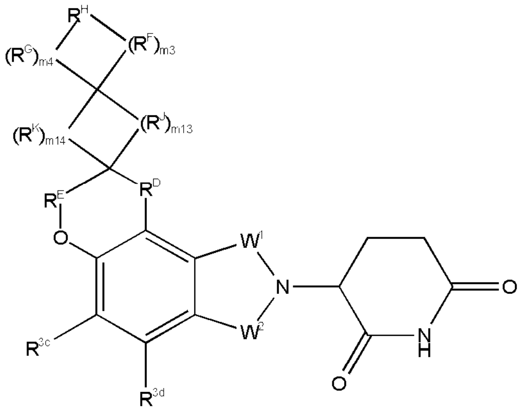
,
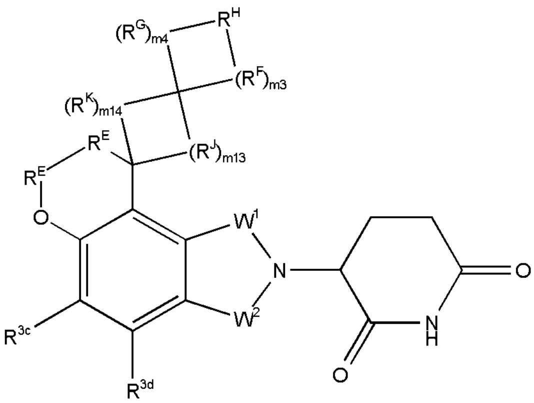
,
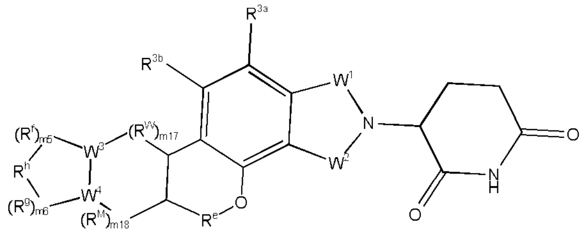
,

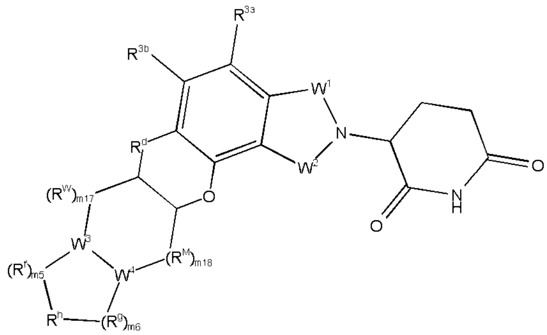

,

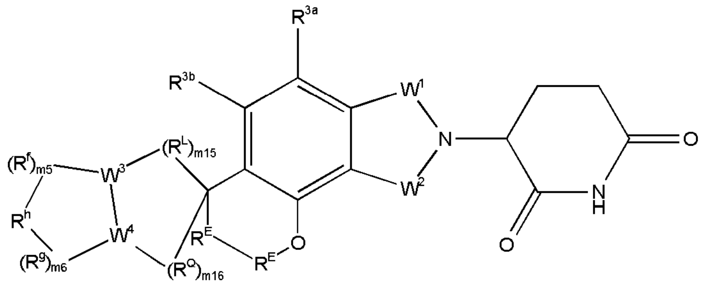

,

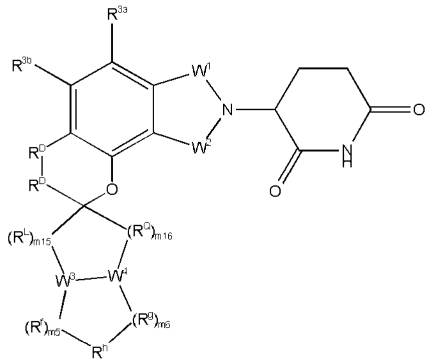

,

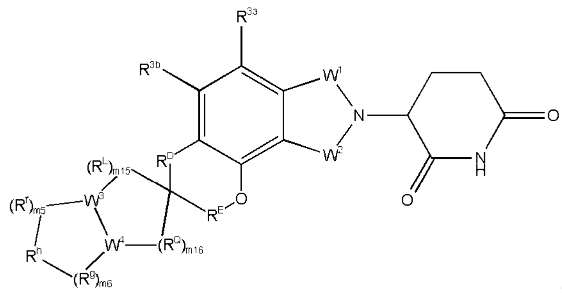

,

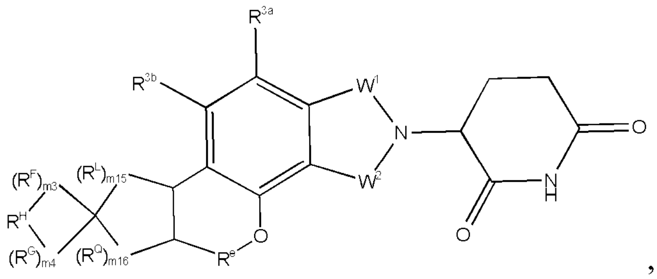

,

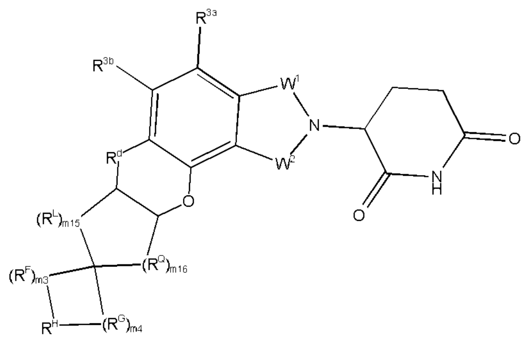

,

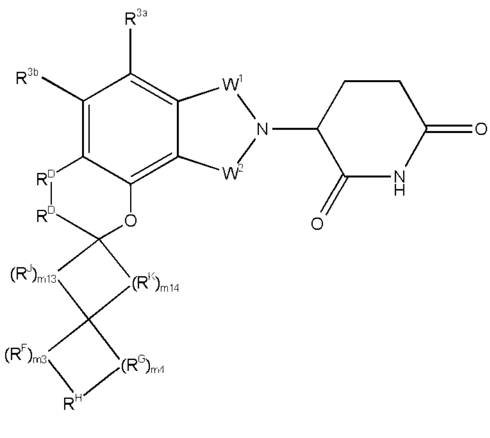

,

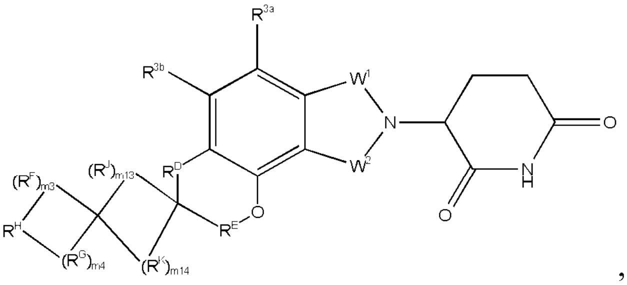

,

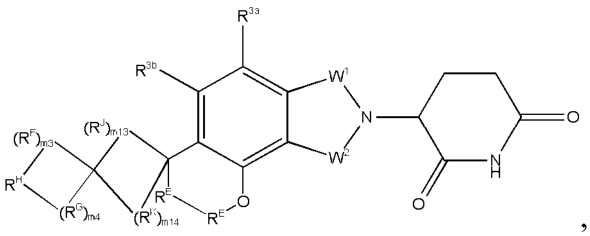

,

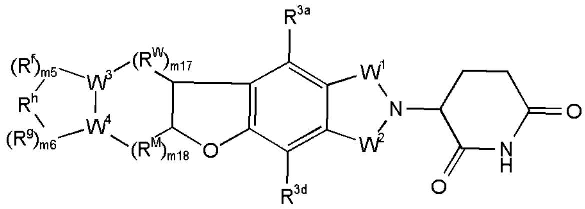

,

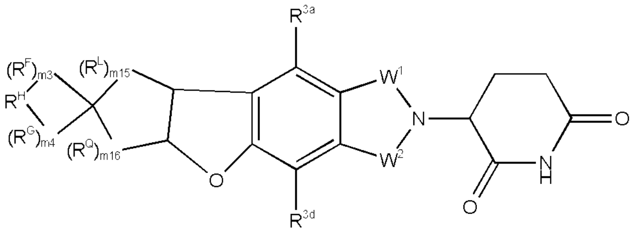

,

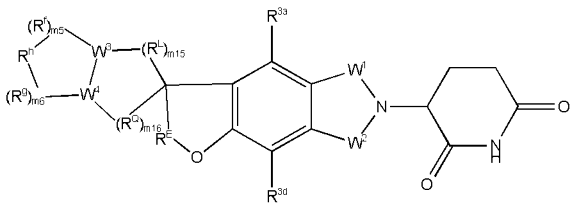

,

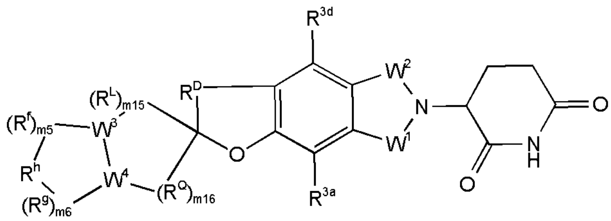

,

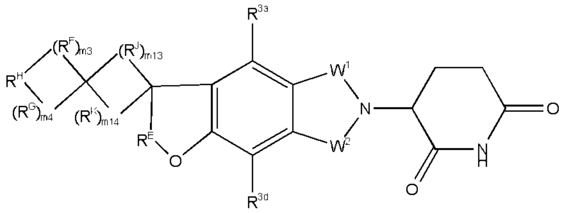

,

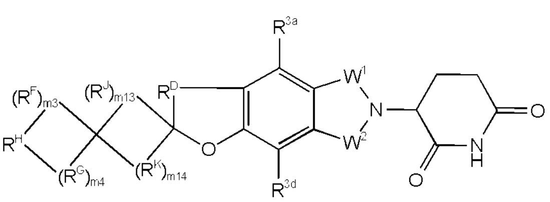

,

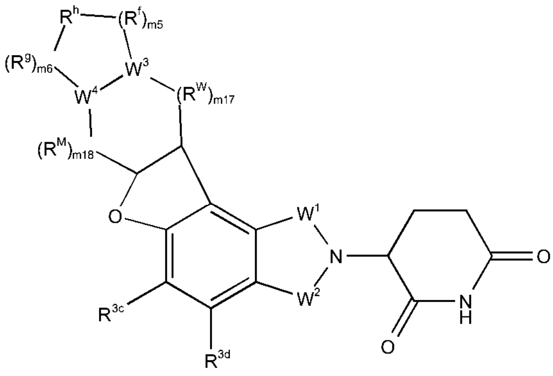,

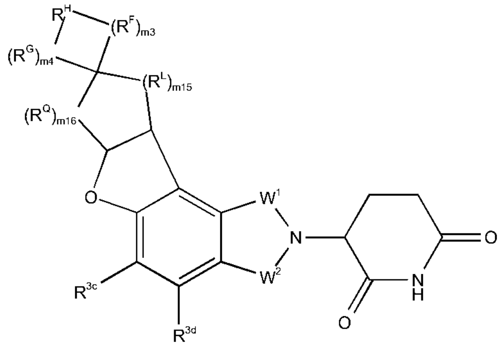,

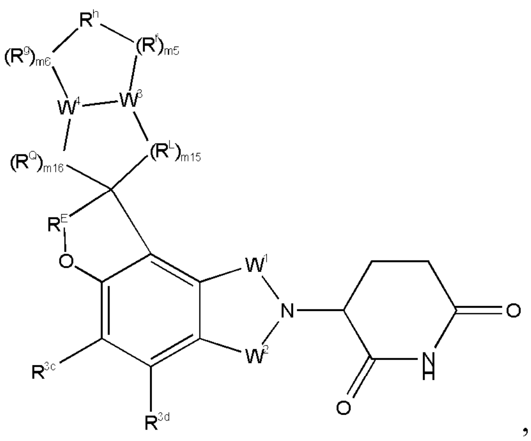,

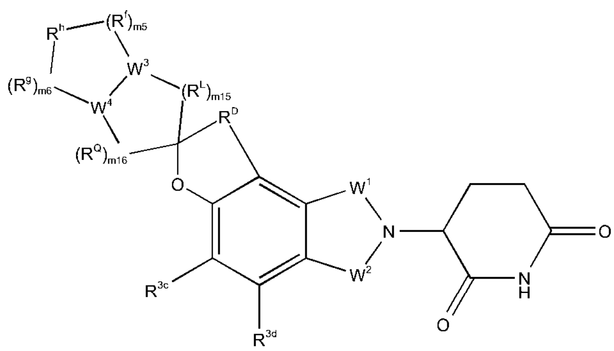,

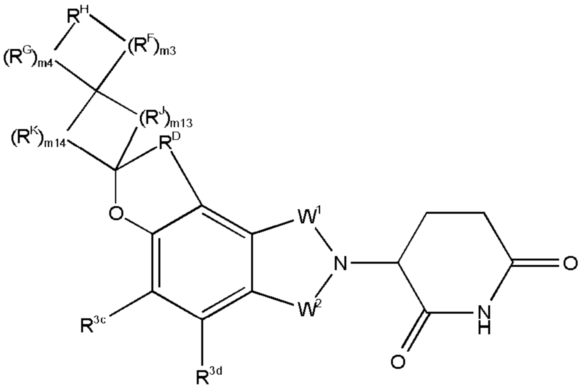,

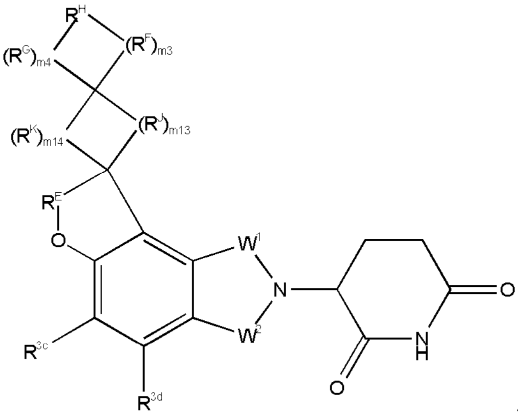,

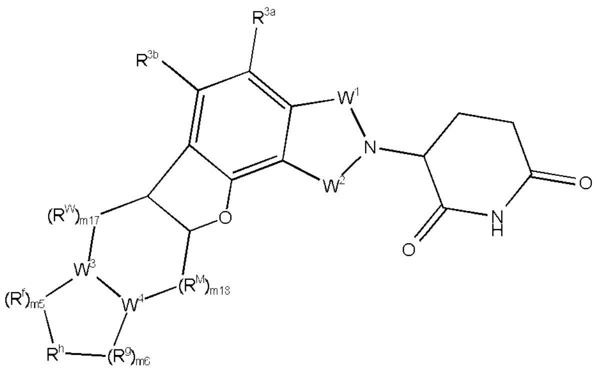,

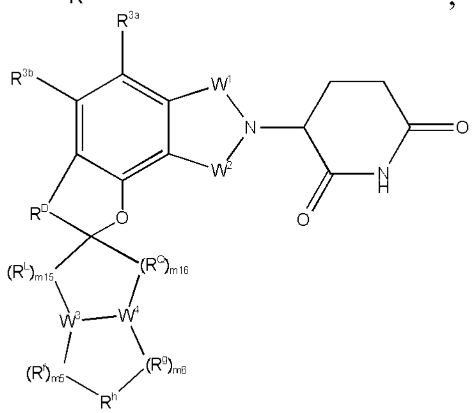,

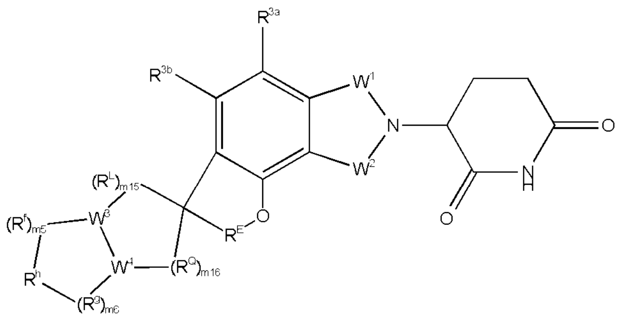,

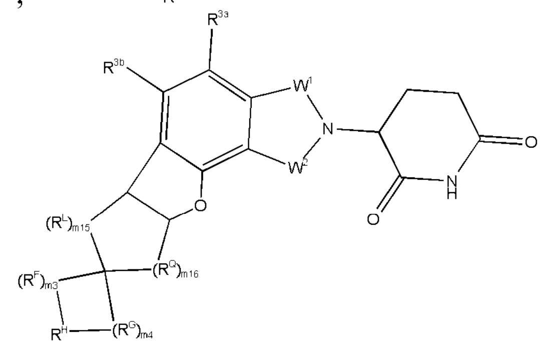,

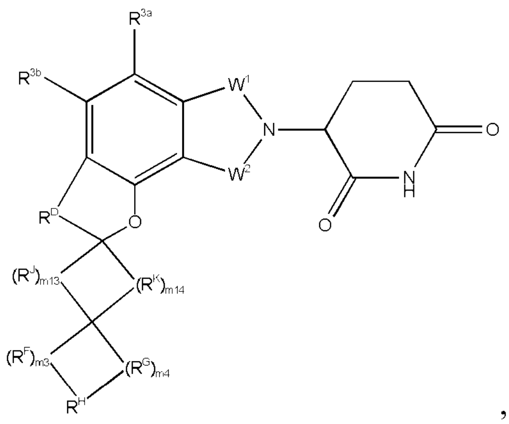,

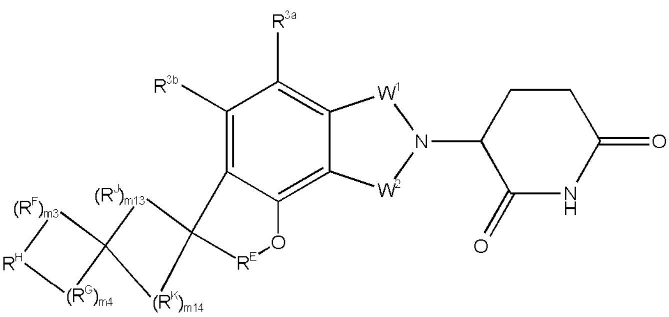;

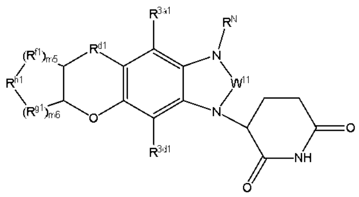 , 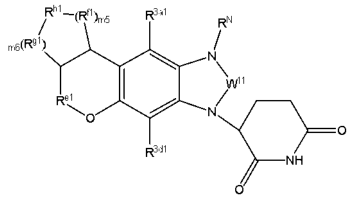 , 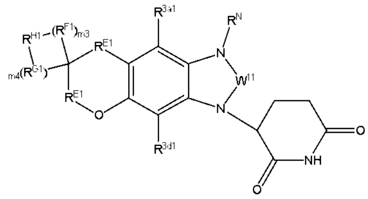 ,

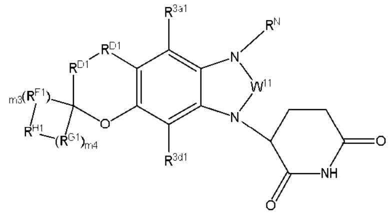,
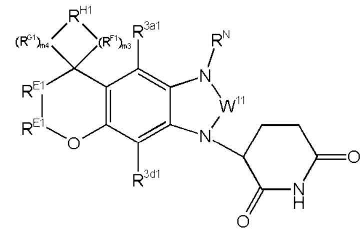,
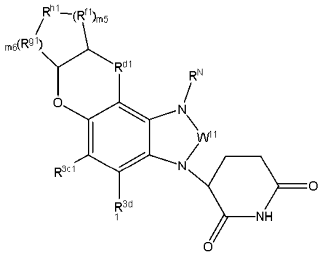,
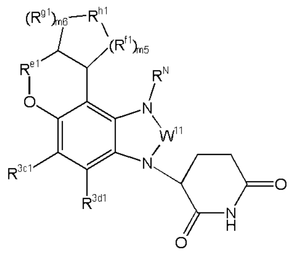,
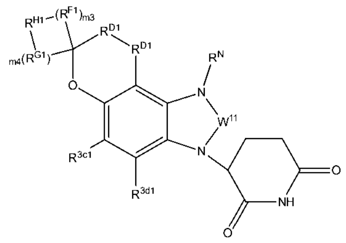,
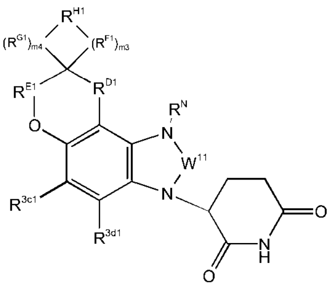,
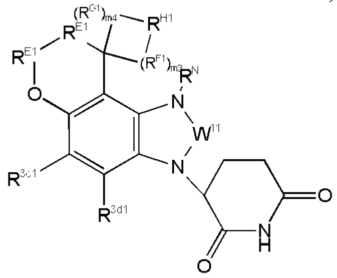,
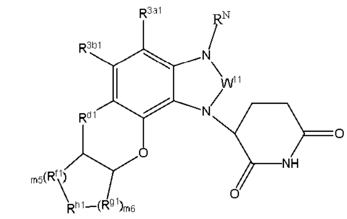,
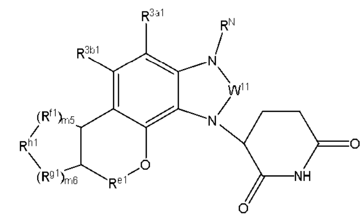,
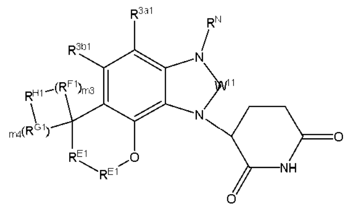,
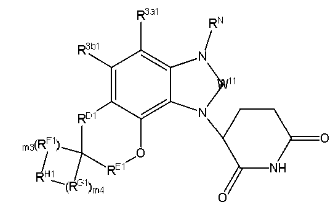,
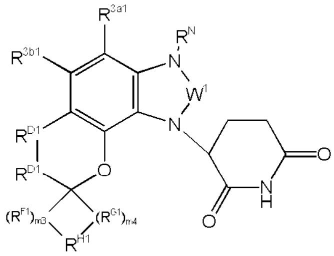,
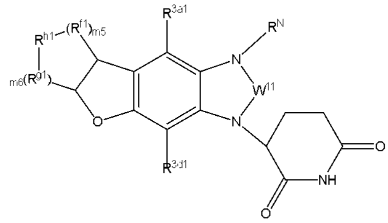,
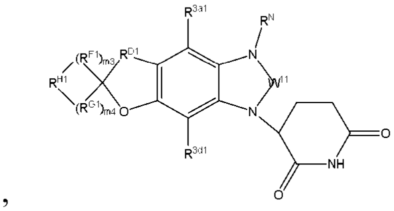,
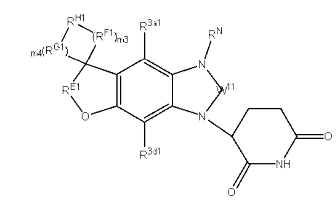,
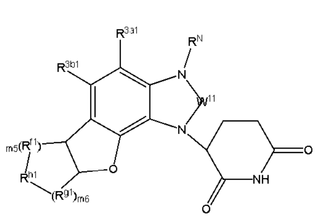,
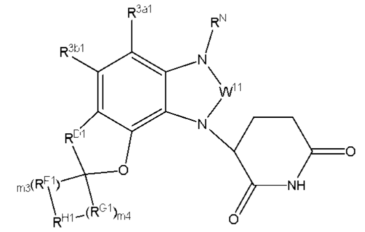,
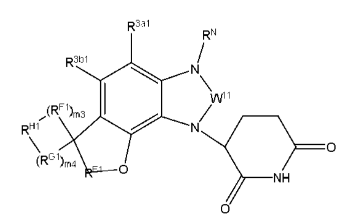,
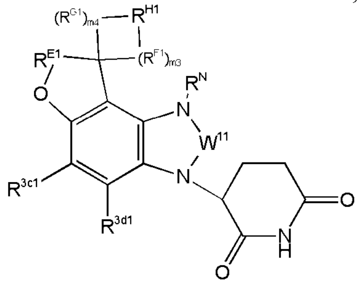,
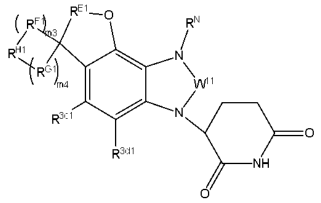,
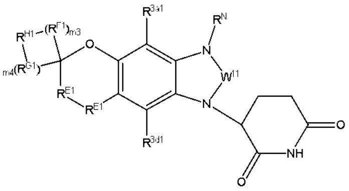,

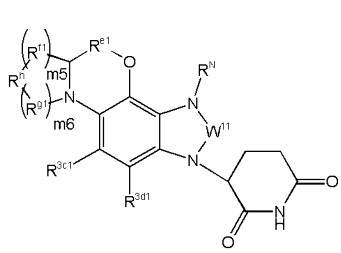,
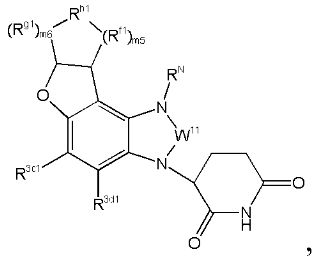, and
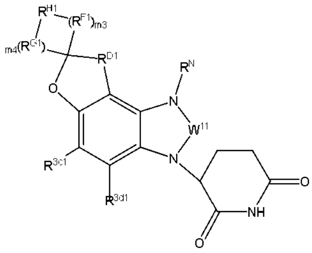;

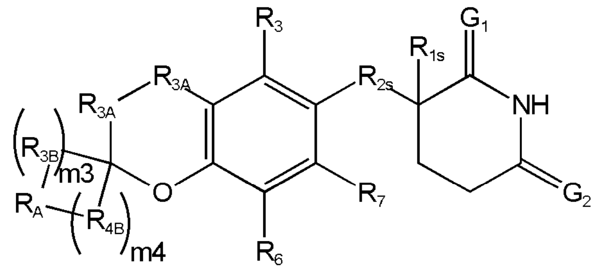,

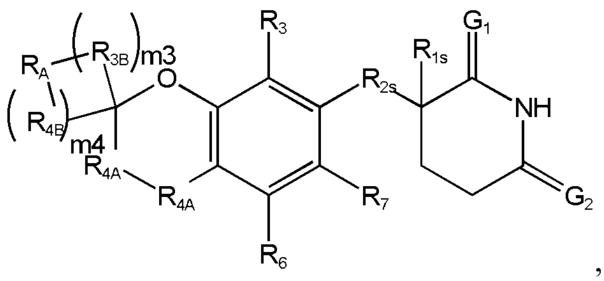,

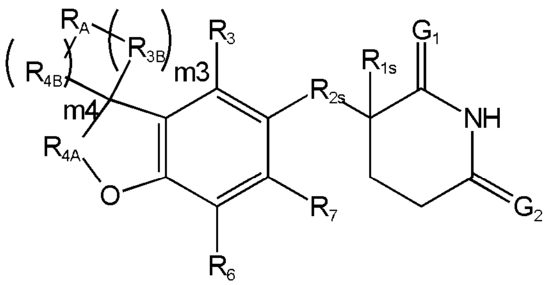,
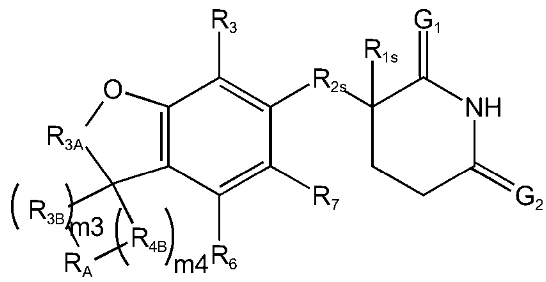,

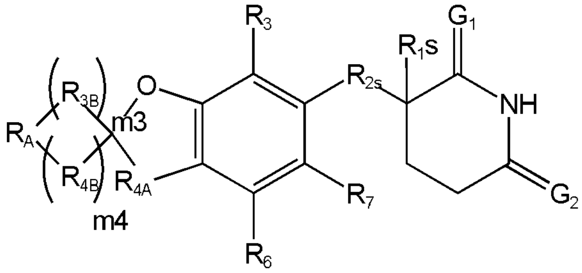,
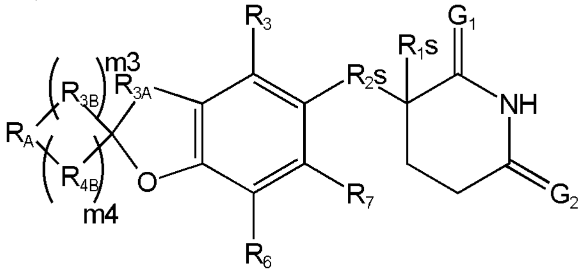,

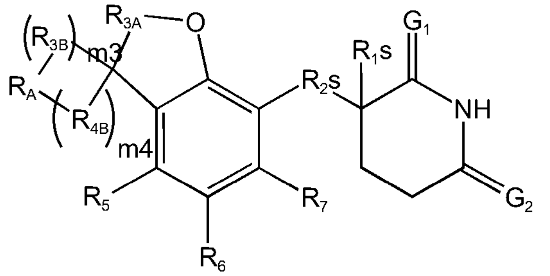
,
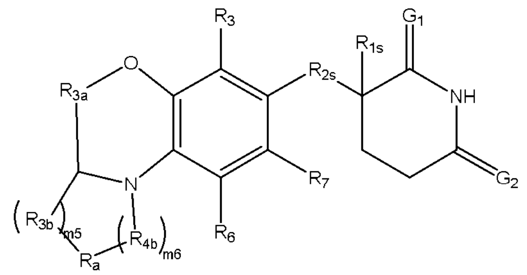
,
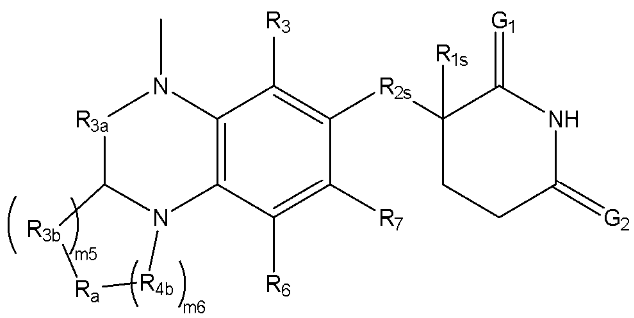
,
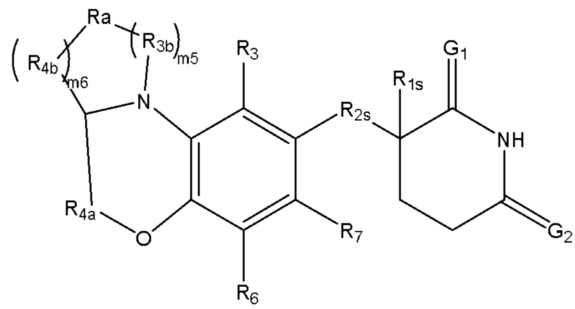
,
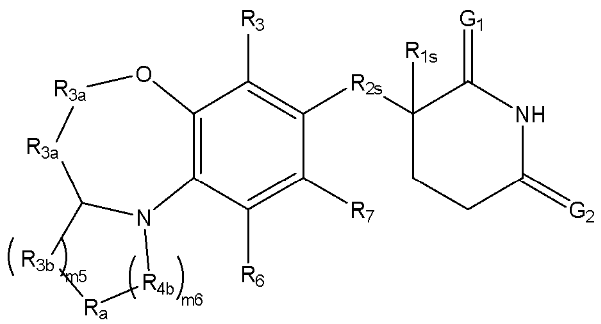
,
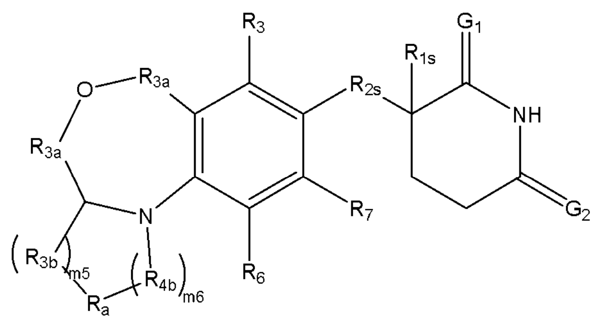
,
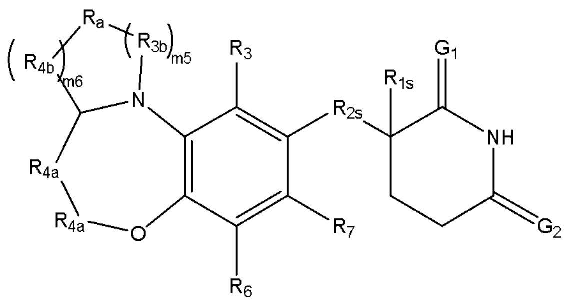
,and
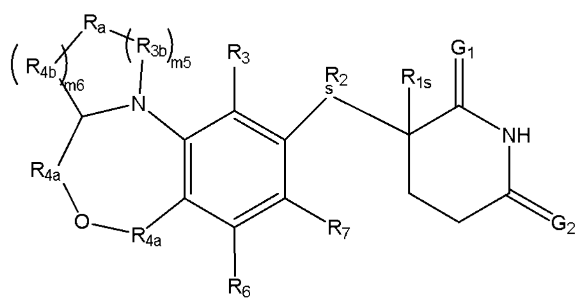
;

$R^{H1}$, $R^H$, $R^h$, $R^f$, $R^g$, $R^W$, $R^M$, $W^3$, $W^4$, $W^5$, $W^6$, $R^d$, $R^e$, $R^{3a}$, $R^{3b}$, $R^{3c}$, $R^{3d}$, $W^1$, $W^2$, $R^F$, $R^G$, $R^J$, $R^K$, $R^L$, $R^Q$, $R^{f1}$, $R^{g1}$, $W^{31}$, $W^{41}$, $R^{d1}$, $R^{e1}$, $R^{3a1}$ , $R^{3b1}$, $R^{3c1}$, $R^{3d1}$, $W^{11}$, $R^N$, $G_1$, $G_2$, $R_{1s}$, $R_{2s}$, $R_3$, $R_4$, $R_5$, $R_6$, $R_{3A}$, $R4_A$, $R_{3B}$, $R_{4B}$, $R_{3a}$, $R_{4a}$, $R_{4b}$, $R_{4a}$, $R_a$, $R_b$, m3, m4, m5, m6, m13, m14, m15, m16, m17, m18, and $R^{h1}$ are respectively as defined above.

A second aspect of the present invention provides a compound represented by of Formula II, or an isomer, isotopic derivative, polymorph, prodrug, or a pharmaceutically acceptable salt or a solvate thereof:

CLM-L-PTM (Formula II),

wherein:

the PTM is a moiety that binds to a target protein;
the L is a bond or a chemical linking moiety covalently connecting the CLM and the PTM; and
the CLM is a cereblon E3 ubiquitin ligase binding moiety, selected from the following structures:

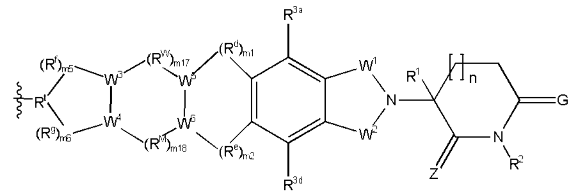

,

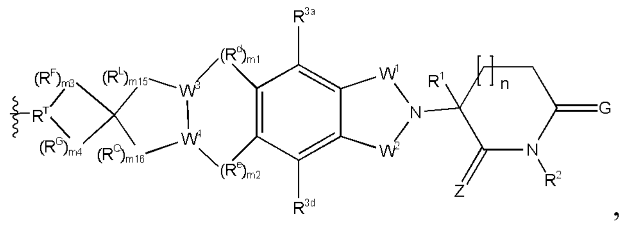

,

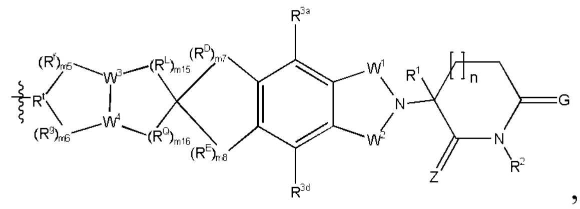

,

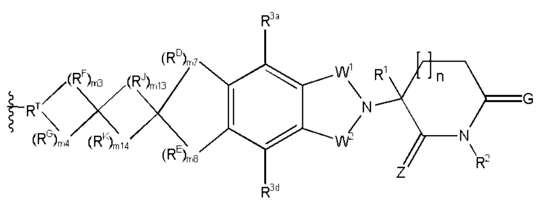

,

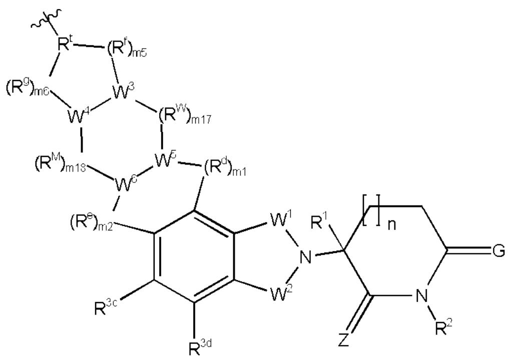

,

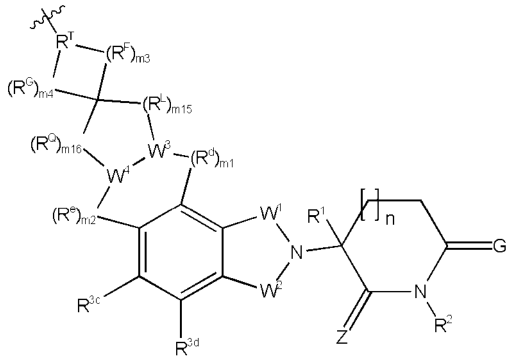

,

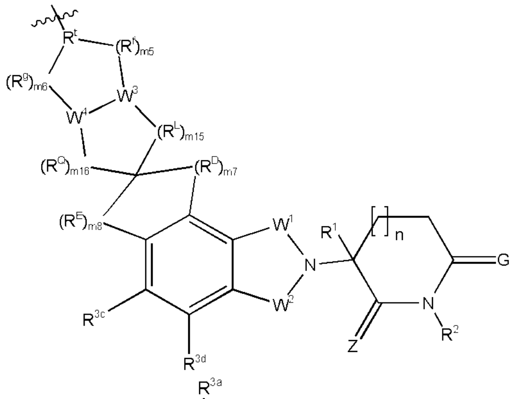

,

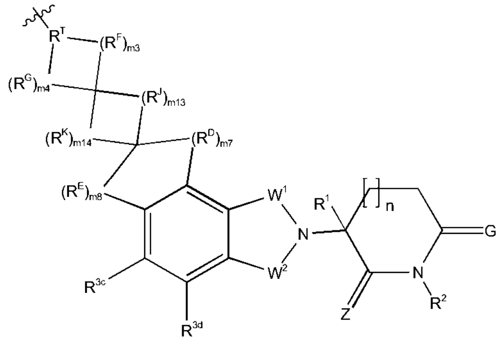

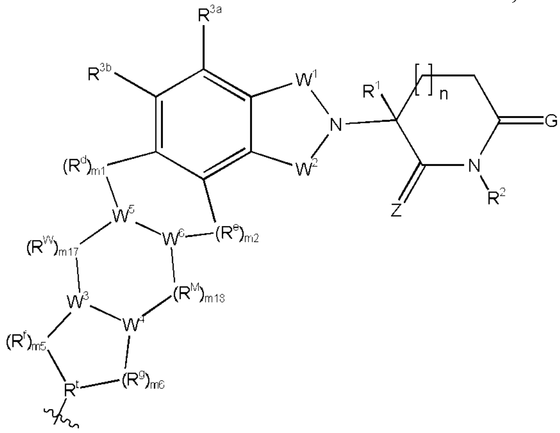

,

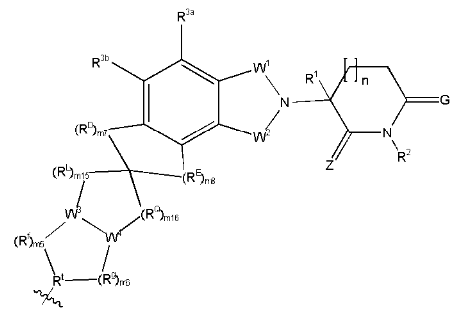

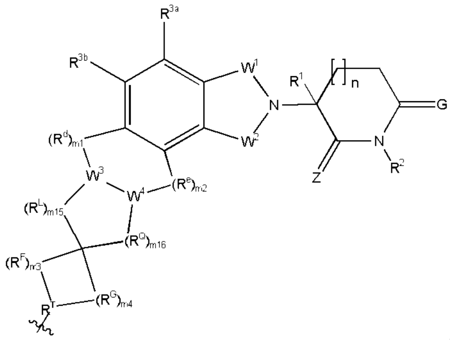

,

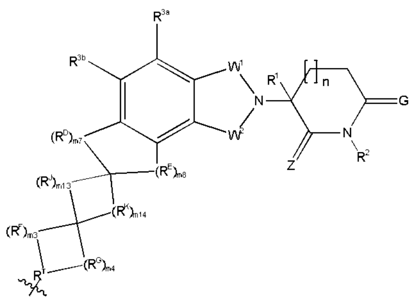

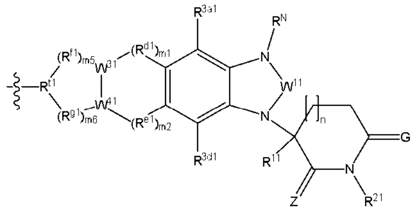

,

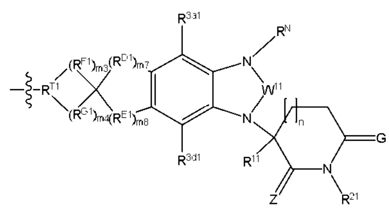

,

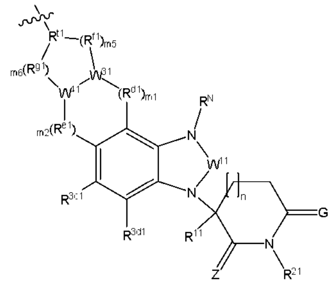

,

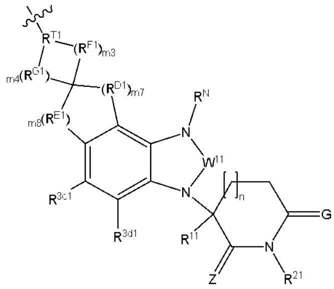,
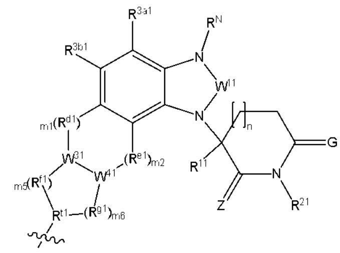,
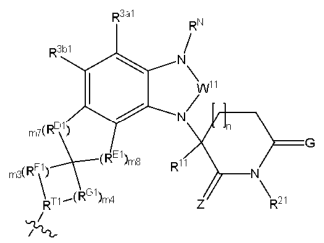,

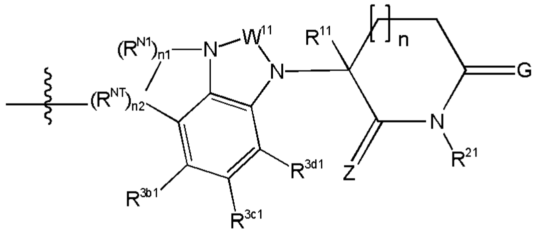,

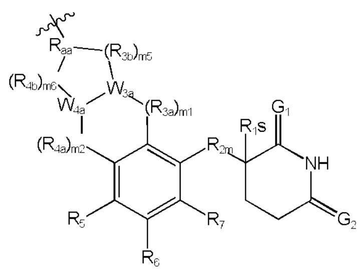,
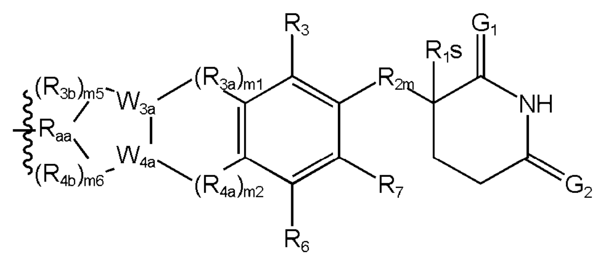,

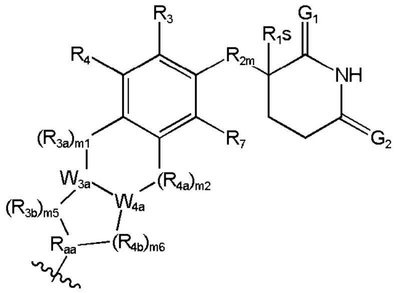,
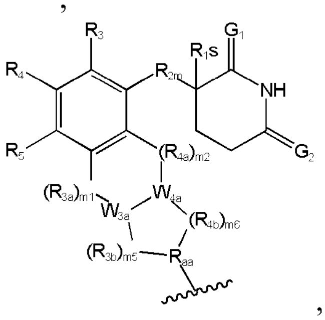,

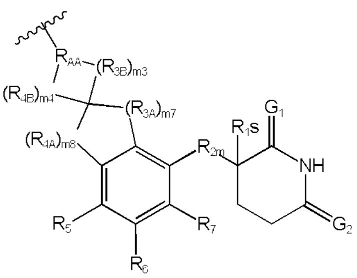,
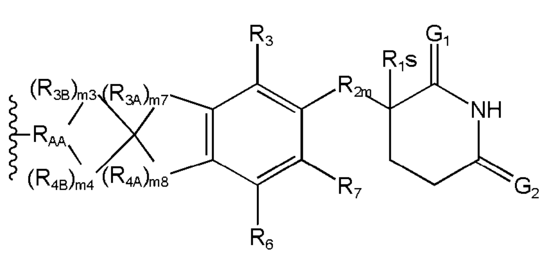

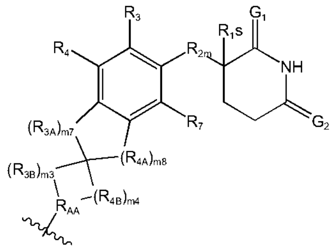

, and

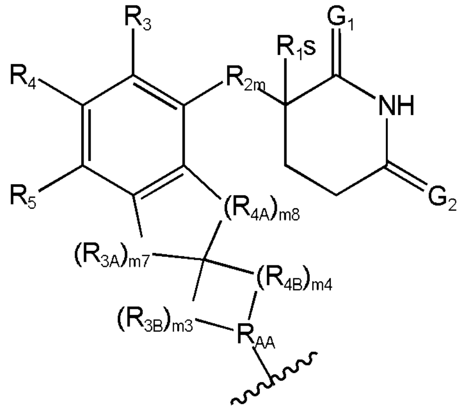

;

wherein:

$W^{11}$, $W^1$, $W^2$, G, Z, $R^{3a}$, $R^{3b}$, $R^{3c}$, $R^{3d}$, $R^N$, $R^{3a1}$, $R^{3b1}$, $R^{3c1}$, $R^{3d1}$, $R^d$, $R^e$, $R^f$, $R^g$, $R^D$, $R^E$, $R^F$, $R^G$, $R^J$, $R^K$, $R^L$, $R^Q$, $R^W$, $R^M$, $R^{d1}$, $R^{e1}$, $R^{f1}$, $R^{g1}$, $R^{D1}$, $R^{E1}$, $R^{F1}$, $R^{G1}$, $W^{31}$, $W^{41}$, $W^3$, $W^4$, $W^5$, $W^6$, m1, m2, m3, m4, m5, m6, m7, m8, m13, m14, m15, m16, m17, m18, $R^m$, $R^1$, $R^{11}$, $R^2$, $R^{21}$, n, $R^{2h}$, $G_1$, $G_2$, $R_{1s}$, $R_3$, $R_4$, $R_5$, $R_6$, $R_7$, $R_{3a}$, $R_{4a}$, $R_{3b}$, $R_{4b}$, $R_{3A}$, $R_{4A}$, $R_{3B}$, $R_{4B}$, $W_{3a}$, $W_{4a}$, and $R_{2A}$ are as defined above;
each occurrence of $R^{N1}$ is independently $C(R^m)_2$, $NR^m$, C(=O), O, or S;
each occurrence of $R^{T1}$, $R^{t1}$, $R^{NT}$, $R^t$, and $R^T$ is independently N or $CR^{2h}$;
each occurrence of n1 and n2 is independently an integer of 0, 1, 2, 3, 4, or 5, and $n1 + n2 \leq 5$; in one embodiment, n1 + n2 = 2, or n1 + n2 = 3; in one embodiment, n1 is 1 or 2, and n2 is 1;
$R_{2m}$ is selected from $-NR_{2A}-$, $-NR_{2A}CO-$, $C_1$-$C_6$ alkylene, and $C_1$-$C_6$ haloalkylene;
each occurrence of $R_{aa}$, $R_{AA}$, $R_{BB}$, and $R_{bb}$ is independently selected from $CR^m$ or N;
when the PTM is

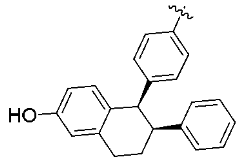

,

the CLM is

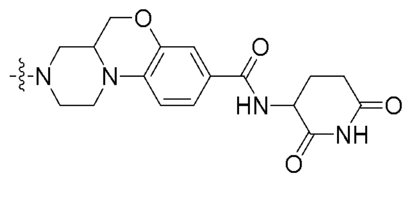

or

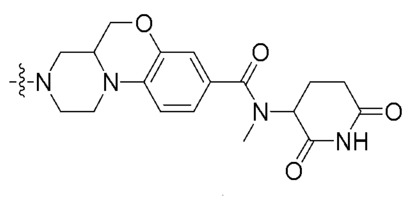

,

and the L is not

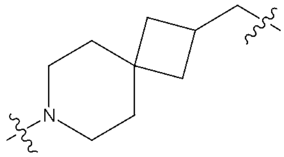,
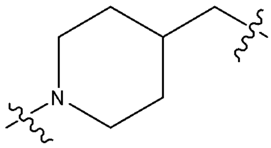,

or

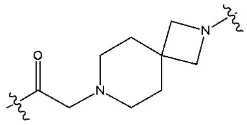;

and
when the PTM is

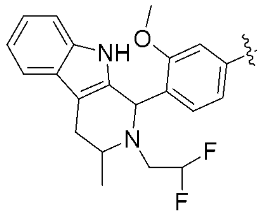,

and the CLM is

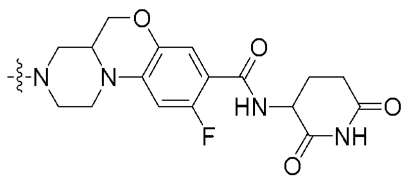,

the L is not

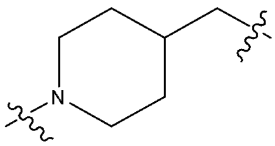.

In one embodiment, wherein the CLM is selected from the following structures:

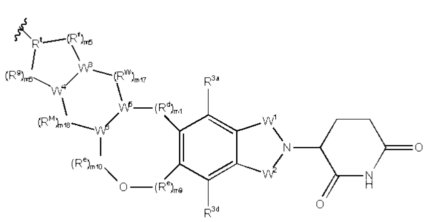,
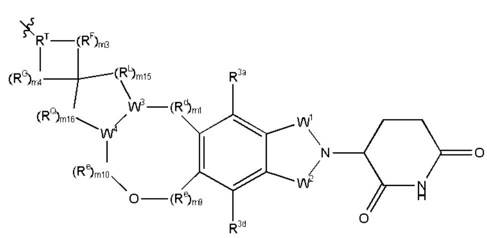,

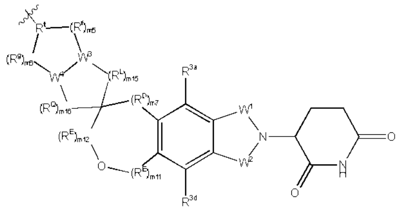
,

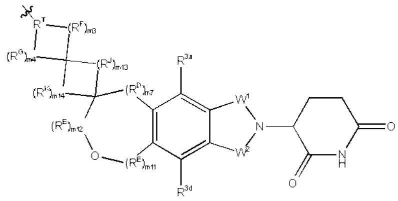

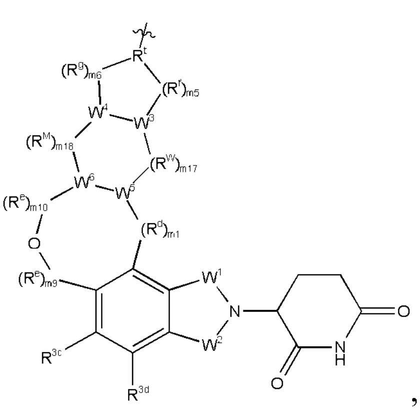
,
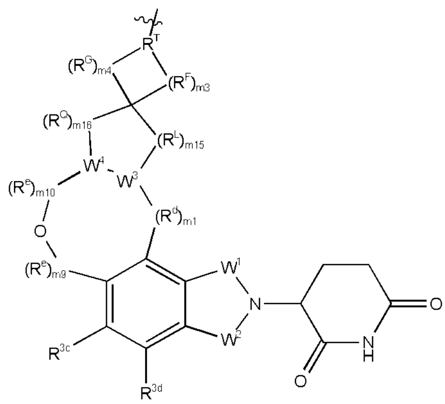
,

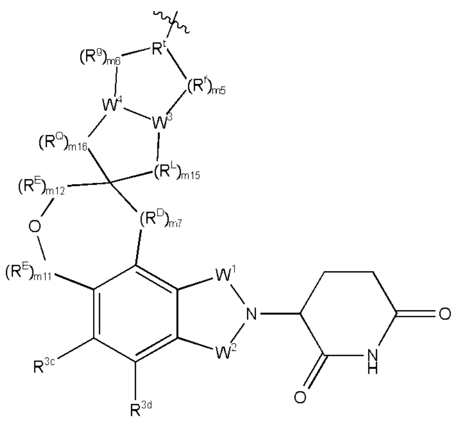
,
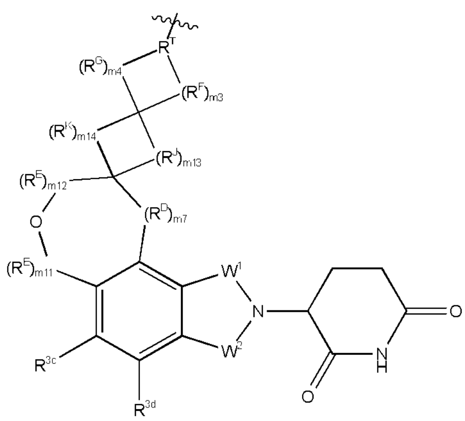

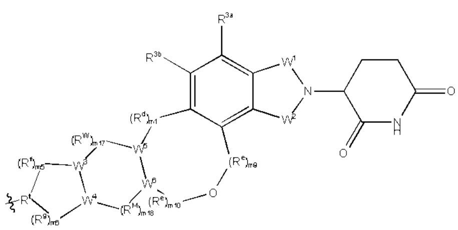

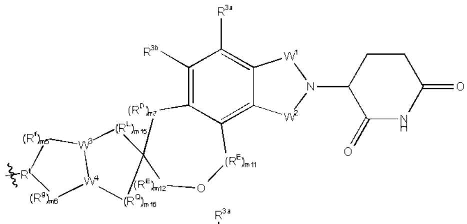

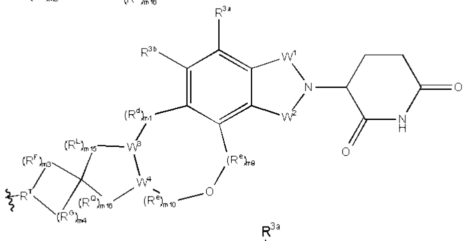

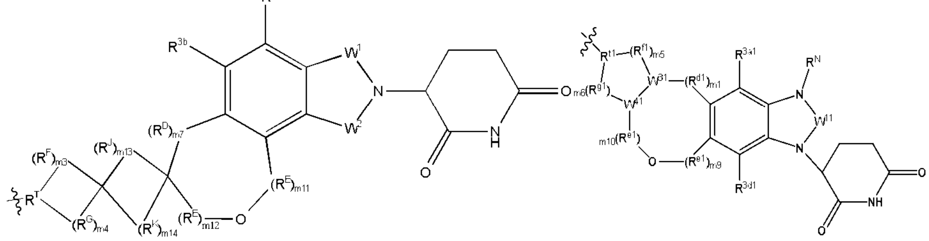

,

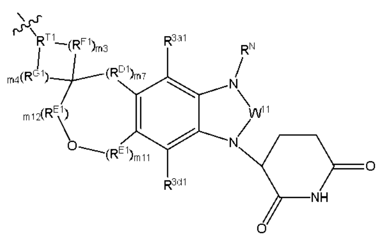

,

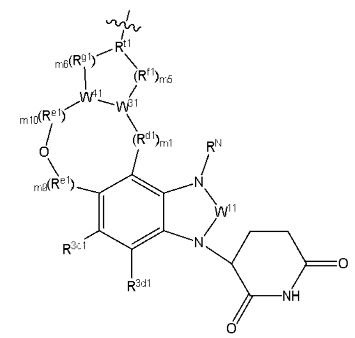

,

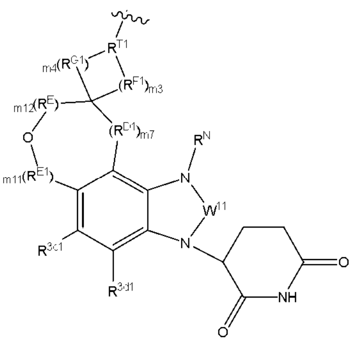

,

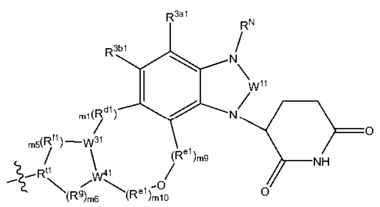

,

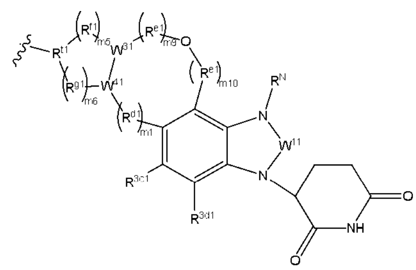

,

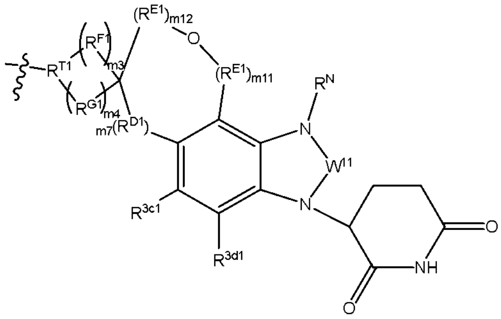 , 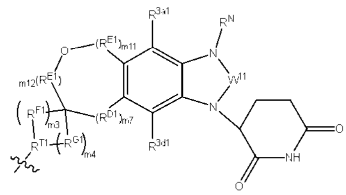 , 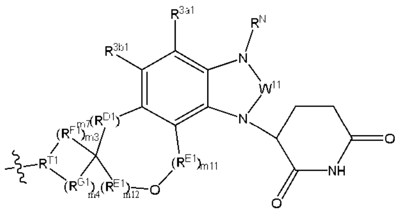 , and 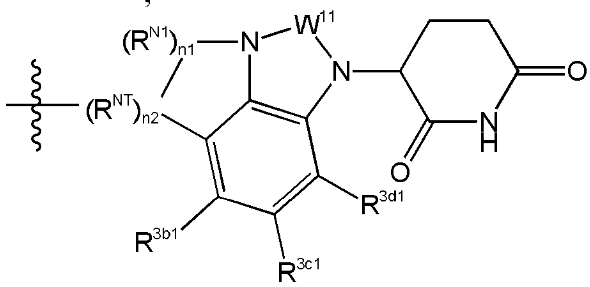 ;

$W^{11}$, $W^1$, $W^2$, $R^{3a}$, $R^{3b}$, $R^{3c}$, $R^{3d}$, $R^N$, $R^{3a1}$, $R^{3b1}$, $R^{3c1}$, $R^{3d1}$, $R^d$, $R^e$, $R^f$, $R^g$, $R^D$, $R^E$, $R^F$, $R^G$, $R^J$, $R^K$, $R^L$, $R^Q$, $R^W$, $R^M$, $R^{d1}$, $R^{e1}$, $R^{f1}$, $R^{g1}$, $R^{D1}$, $R^{E1}$, $R^{F1}$, $R^{G1}$, $W^{31}$, $W^{41}$, $W^3$, $W^4$, $W^5$, $W^6$, m1, m2, m3, m4, m5, m6, m7, m8, m13, m14, m15, m16, m17, m18, n1, n2, $R^{N1}$, $R^{NT}$, $R^t$, $R^T$, $R^{t1}$, and $R^{T1}$ are as defined above.

In one embodiment, wherein the L is a bond or $-(B^L)_q-$;

each occurrence of $B^L$ is identical or different and is independently selected from $CR^{L1}R^{L2}$, O, S, SO, $SO_2$, $NR^{L3}$, $SO_2NR^{L3}$, $SONR^{L3}$, $CONR^{L3}$, $NR^{L3}CONR^{L4}$, $NR^{L3}SO_2NR^{L4}$, CO, $CR^{L1}=CR^{L2}$, C≡C, $SiR^{L1}R^{L2}$, $P(O)R^{L1}$, $P(O)OR^{L1}$, $NR^{L3}C(=NCN)NR^{L4}$, $NR^{L3}C(=NCN)$, $NR^{L3}C(=CNO_2)NR^{L4}$, cycloalkylene, heterocyclylene, arylene, or heteroarylene, wherein the cycloalkylene, heterocyclylene, arylene, and heteroarylene are optionally substituted by 0 to 6 $R^{L1}$ in one embodiment $R^{L2}$;

each occurrence of $R^{L1}$, $R^{L2}$, $R^{L3}$, and $R^{L4}$ is independently selected from H, halogen, $C_{1-8}$ alkyl, O-$C_{1-8}$ alkyl, S-$C_{1-8}$ alkyl, NH-$C_{1-8}$ alkyl, N($C_{1-8}$ alkyl)$_2$, $C_{3-11}$ cycloalkyl, aryl, heteroaryl, $C_{3-11}$ heterocyclyl, O-$C_{3-8}$ cycloalkyl, O-$C_{3-11}$ heterocyclyl, O-aryl, O-heteroaryl, S-$C_{3-8}$ cycloalkyl, NH-$C_{3-8}$ cycloalkyl, N ($C_{3-8}$ cycloalkyl)$_2$, N($C_{3-8}$ cycloalkyl)($C_{1-8}$ alkyl), NH-$C_{3-8}$ heterocyclyl, N($C_{3-8}$ heterocyclyl)$_2$, N($C_{3-8}$ heterocyclyl)($C_{1-8}$ alkyl), NH- aryl, N( aryl)($C_{1-8}$ alkyl), NH-heteroaryl, N( heteroaryl)($C_{1-8}$ alkyl), OH, $NH_2$, SH, $SO_2P(O)(O-C_{1-8}$ alkyl)($C_{1-8}$ alkyl), P(O)(O-$C_{1-8}$ alkyl)$_2$, C≡C-$C_{1-8}$ alkyl, C≡CH, CH = CH-($C_{1-8}$ alkyl), C($C_{1-8}$ alkyl) = CH-($C_{1-8}$ alkyl), C($C_{1-8}$ alkyl)=C($C_{1-8}$ alkyl)$_2$, $Si(OH)_3$, Si($C_{1-8}$ alkyl)$_3$, Si(OH)($C_{1-8}$ alkyl)$_2$, CO-$C_{1-8}$ alkyl, $CO_2H$, CN, $CF_3$, $CHF_2$, $CH_2F$, $NO_2$, $SF_5$, $SO_2NH$-$C_{1-8}$ alkyl, $SO_2N$($C_{1-8}$ alkyl)$_2$, SONH-$C_{1-8}$ alkyl, SON($C_{1-8}$ alkyl)$_2$, CONH-$C_{1-8}$ alkyl, CON($C_{1-8}$ alkyl)$_2$, N($C_{1-8}$ alkyl)CONH($C_{1-8}$ alkyl), N($C_{1-8}$ alkyl)CON($C_{1-8}$ alkyl)$_2$, NHCONH($C_{1-8}$ alkyl), NHCON($C_{1-8}$ alkyl)$_2$, $NHCONH_2$, N($C_{1-8}$ alkyl)$SO_2NH$($C_{1-8}$ alkyl), N($C_{1-8}$ alkyl)$SO_2N$($C_{1-8}$ alkyl)$_2$, $NHSO_2NH$($C_{1-8}$ alkyl), NH $SO_2$ N($C_{1-8}$ alkyl)$_2$, and NH $SO_2NH_2$, optionally, the $C_{1-8}$ alkyl, $C_{3-11}$ cycloalkyl, $C_{3-11}$ heterocyclyl, $C_{6-10}$ aryl, and $C_{5-10}$ heteroaryl are each independently optionally substituted by one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, halocycloalkyl, haloheteroalkyl, alkylamino, aryl, heteroaryl, haloaryl, and haloheteroaryl; and

q is an integer greater than or equal to 1; in one embodiment, q is 1, 2, 3, 4, 5, 6, 7, 8, 9, 10, 11, 12, 13, 14, 15, 16, 17, 18, 19, or 20.

In one embodiment, wherein the $B^L$ is selected from one or more of the following structures: -O-, -S-, -SO-, $-SO_2-$, $-CH_2-$, -CO-, -NH-, $-N(CH_3)-$

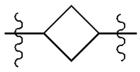 , 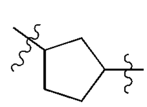 , 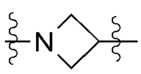 ,

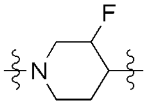, 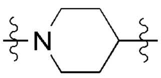, 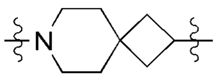, 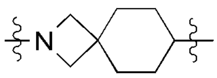, 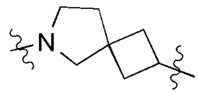,

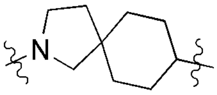, 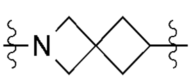, 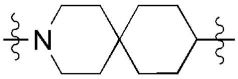, 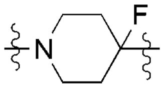, 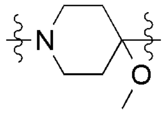,

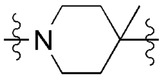, 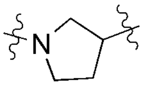, 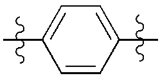, 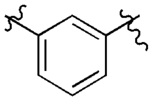, 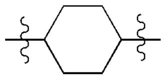, 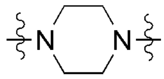,

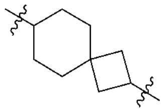, 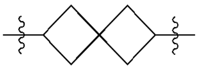, 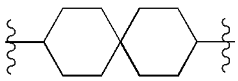, 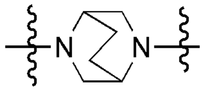, 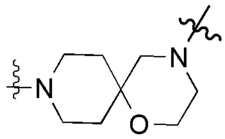,

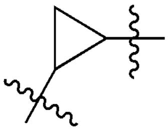, 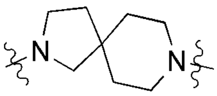, 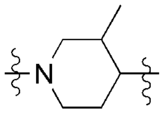, and 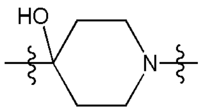;

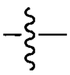

is a point of attachment.

In one embodiment, the L is selected from the following structures:

a covalent bond, $-(CH_2)_j-$, $-(CH_2)_p-NH-(CH_2)_s-$, $-(CH_2)_y-NH-(CH_2)_j-NH-(CH_2)_s-$, $-(CH_2)_p-CO-(CH_2)_s-$, $-(CH_2)_p-O-(CH_2)_s-$, $-(CH_2)_y-CO-(CH_2)_j-CO-(CH_2)_s-$, $-(CH_2)_y-O-(CH_2)_j-O-(CH_2)_s-$, - $(CH_2)_y-O-(CH_2)-CO-(CH_2)_s-$, $-(CH_2)_y-CO-(CH_2)_j-O-(CH_2)_s-$, $-(CH_2)_p-NH-(CH_2)_y-O-(CH_2)_j-CO-(CH_2)_s-$, $-(CH_2)_y-CO-(CH_2)_j-O-(CH_2),-NH-(CH_2)_p-$,

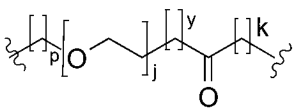, 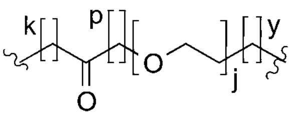, 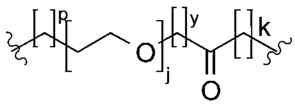,

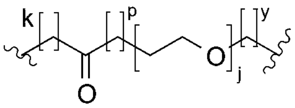, 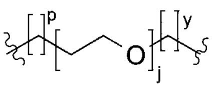, 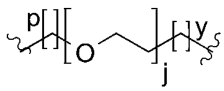,

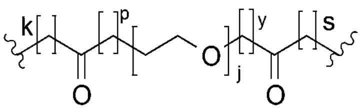, 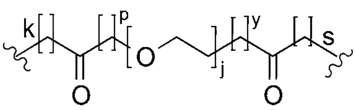, 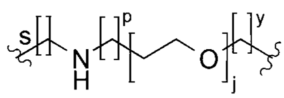,

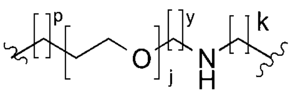, 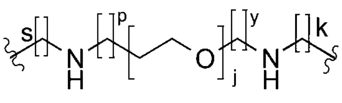, 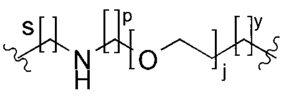,

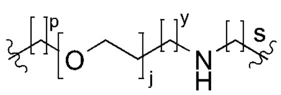,
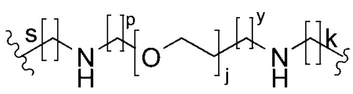,
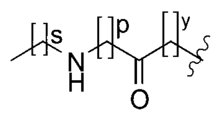,
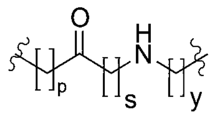,

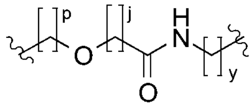,
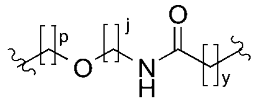,
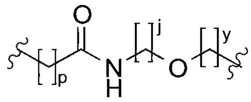,
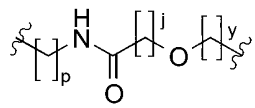,

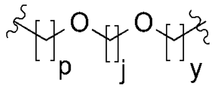,
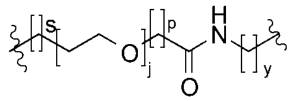,
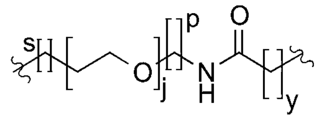,

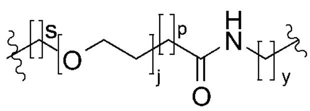,
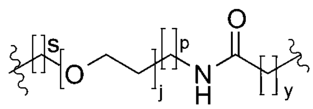,
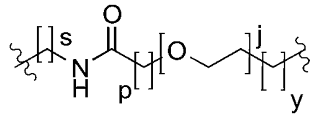,

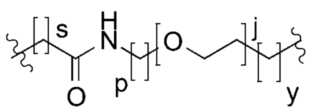,
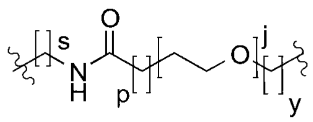,
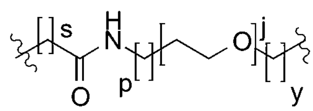,

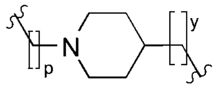,
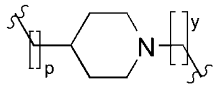,
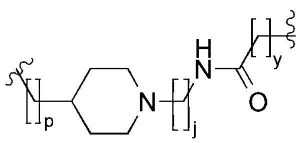,
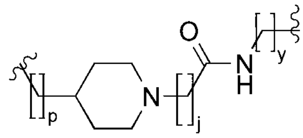,

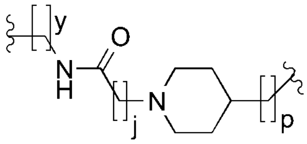,
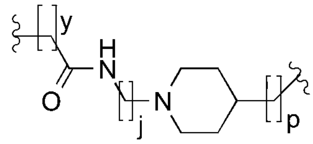,
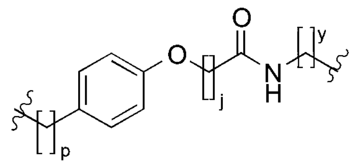,

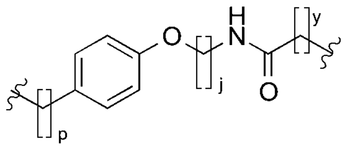,
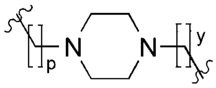,
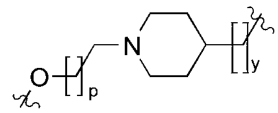,

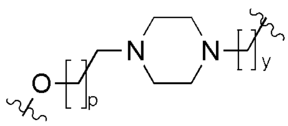,
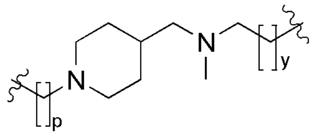,
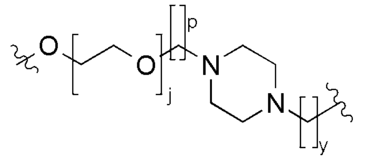,

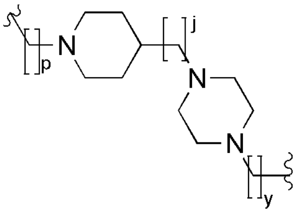 , 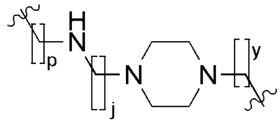 , 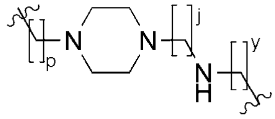 ,

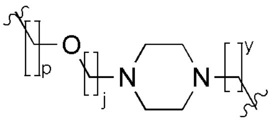 , 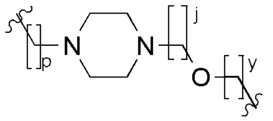 , 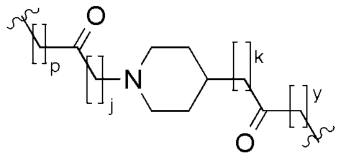 ,

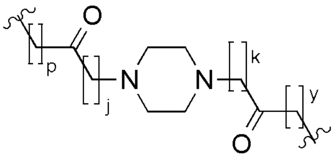 , 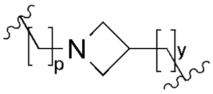 , 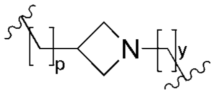 , 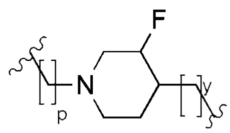 ,

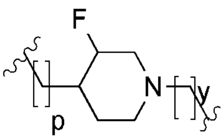 , 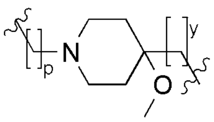 , 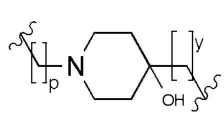 , 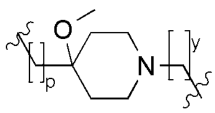 ,

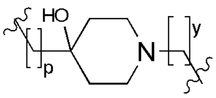 , 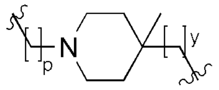 , 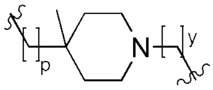 , 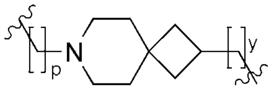 ,

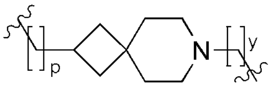 , 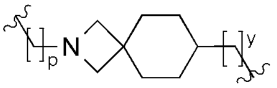 , 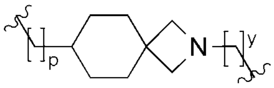 ,

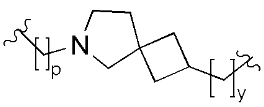 , 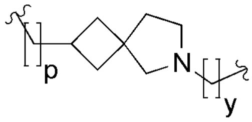 , 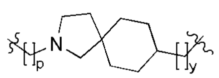 , 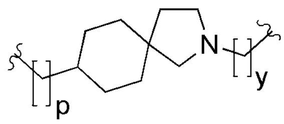 ,

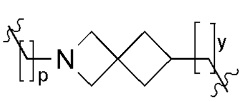 , 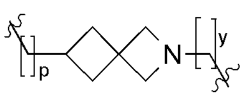 , 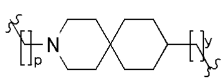 , 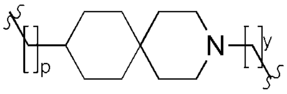 ,

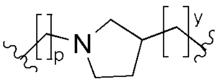 , 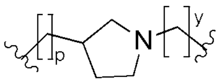 , 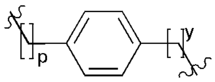 , 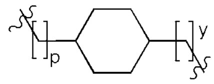 ,

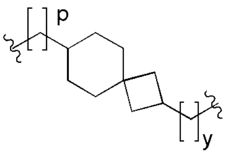 , 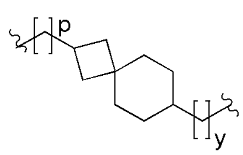 , 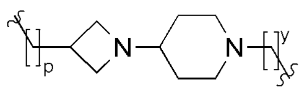 , 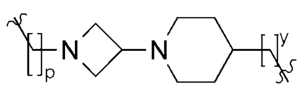 ,

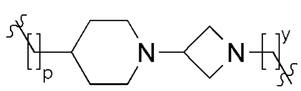 , 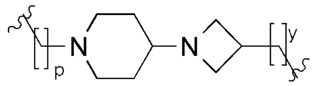 , 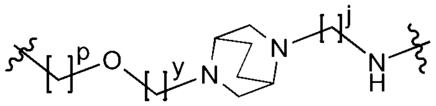 ,

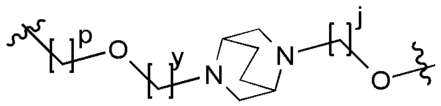 , 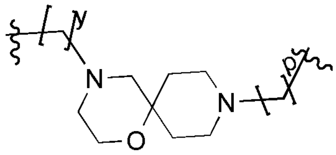 , 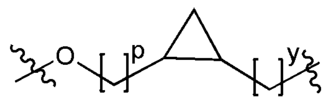 ,

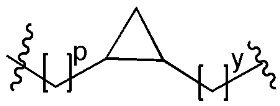 , 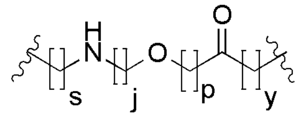 , 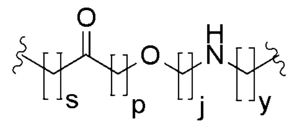 ,

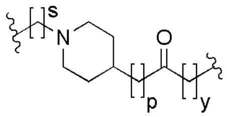 , 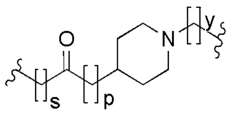 , 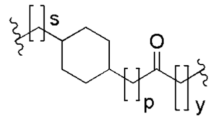 , 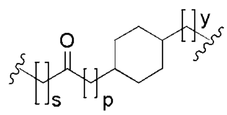 ,

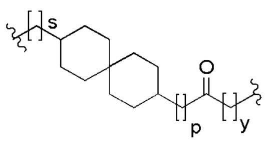 , 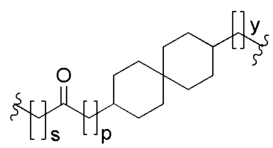 , 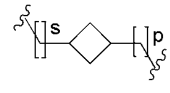 , 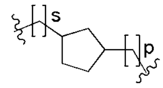 ,

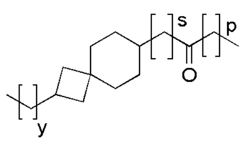 , 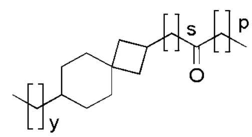 , 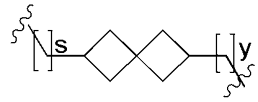 , 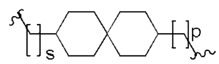 ,

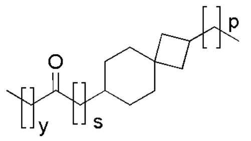 , 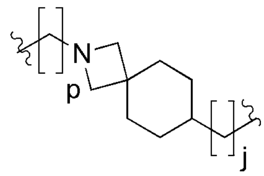 , 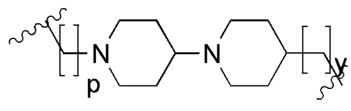 ,

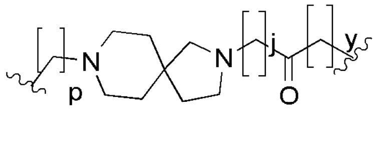 , 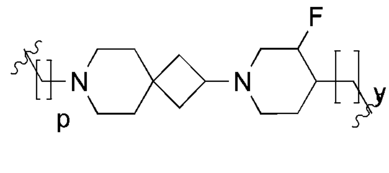 , 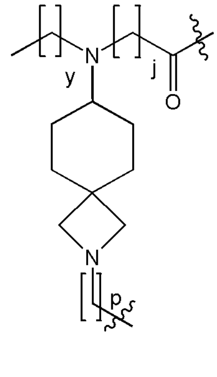 ,

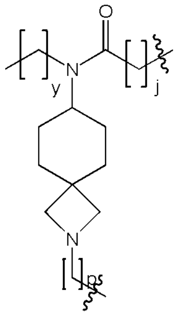, 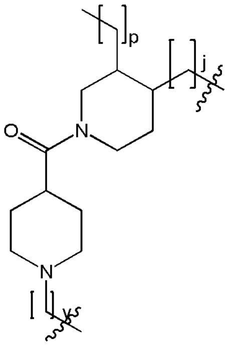, 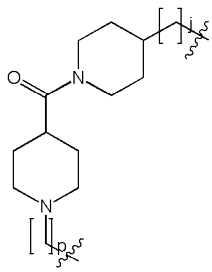, 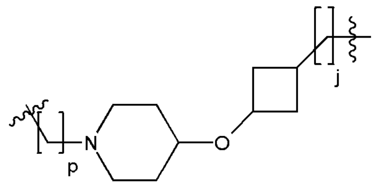,

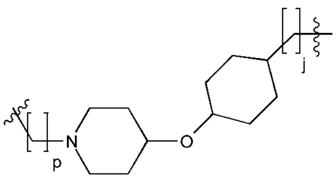, 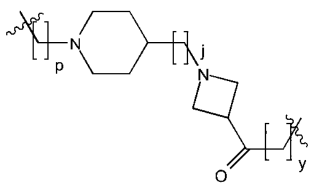, 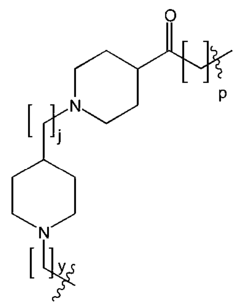, 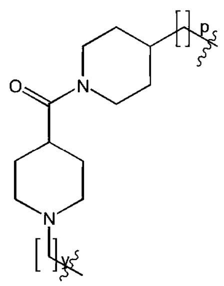,

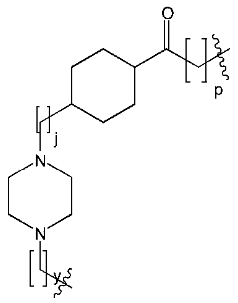, 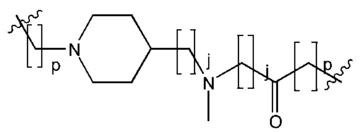, 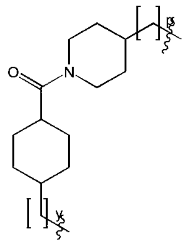, 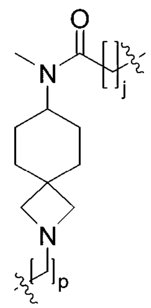,

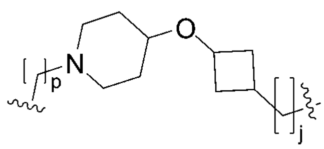, and 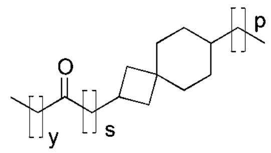;

wherein, j is 1, 2, 3, 4, 5, 6, 7, 8, 9, or 10;
k, s, p, and y are 0, 1, 2, 3, 4, 5, 6, 7, 8, 9, or 10;

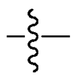

is the point of attachment to the CLM and the PTM.

In one embodiment, the PTM is a moiety that binds to a target protein or polypeptide, wherein the target protein is selected from: structural proteins, receptors, enzymes, cell surface proteins; proteins related to cellular integration functions, including those involved in catalytic activity, aromatase activity, motor activity, helicase activity, metabolic processes, antioxidant activity, proteolysis, biosynthesis; proteins having kinase activity, oxidoreductase activity, transferase activity, hydrolase activity, lyase activity, isomerase activity, ligase activity, enzyme regulatory activity, signal transduction activity, structural molecular activity, binding activity, receptor activity, cell motility, membrane fusion, cell communication, regulation of biological processes, development, cell differentiation, response to stimulus; behavioral proteins; cell adhesion proteins; proteins involved in cell necrosis; and proteins involved in transport, including proteins

with protein transport activity, nuclear transport activity, ion transport activity, channel transport activity, carrier activity, permease activity, secretory activity, electron transport activity, pathogenesis, chaperone regulator activity, nucleic acid binding activity, transcription regulator activity, activity in extracellular organization and biogenesis, and translation regulator activity.

In one embodiment, the PTM is a moiety that binds to a target protein or polypeptide, wherein the target protein is selected from B7.1 and B7, TNFR2, NADPH oxidase, BclI/Bax and other partners in the apoptosis pathway, C5a receptor, HMG-CoA reductase, PDE V phosphodiesterase type, PDEIV phosphodiesterase type 4, PDEI I, PDEI II, PDE III, squalene epoxidase, CXCR1, CXCR2, nitric oxide (NO) synthase, cyclooxygenase 1, cyclooxygenase 2, 5HT receptor, dopamine receptor, G proteins (i.e., Gq), histamine receptor, 5-lipoxygenase, tryptase serine protease, thymidylate synthase, purine nucleoside phosphorylase, GAPDH trypanosome, glycogen phosphorylase, carbonic anhydrase, chemokine receptor, JAW STAT, RXR and analogs thereof, HIV1 protease, HIV1 integrase, influenza neuraminidase, hepatitis B reverse transcriptase, sodium channel, multidrug-resistant bacteria, protein P-glycoprotein, tyrosine kinase, CD23, CD124, tyrosine kinase p56lck, CD4, CD5, IL-2 receptor, IL-1 receptor, TNF-$\alpha$R, ICAM1, $Ca^{2+}$ channel, VCAM, VLA-4 integrin, selectin, CD40/CD40L, inosine monophosphate dehydrogenase, p38 MAP kinase, Ras/Raf/MEK/ERK pathway, interleukin-1 converting enzyme, caspase, HCV, NS3 protease, HCV NS3 RNA helicase, glycinamide ribonucleotide formyltransferase, rhinovirus, 3C protease, herpes simplex virus-I (HSV-I), protease, cytomegalovirus (CMV) protease, poly(ADP-ribose) polymerase, cyclin-dependent kinase 4/6, vascular endothelial growth factor, oxytocin receptor, microsomal transfer protein inhibitor, bile acid transporter inhibitor, 5$\alpha$ reductase inhibitor, angiotensin II, glycine receptor, norepinephrine reuptake receptor, endothelin receptor, neuropeptide Y and receptor, adenosine receptor, adenosine kinase and AMP deaminase, purinergic receptors (P2Y 1, P2Y2, P2Y4, P2Y6, P2X1-7), farnesyltransferase, geranylgeranyltransferase, TrkA receptor for NGF, $\beta$-amyloid, tyrosine kinase Flk-II KDR, vitronectin receptor, integrin receptor, Her-2/neu, telomerase inhibition, cytosolic phospholipase A2, EGF receptor tyrosine kinase, ecdysone 20-monooxygenase, ion channel of the GABA-gated chloride channel, acetylcholinesterase, voltage-sensitive sodium channel protein, calcium release channel and chloride channel, acetyl-CoA carboxylase, adenylosuccinate synthase, protoporphyrinogen oxidase, enolpyruvylshikimate phosphate synthase, MYC protein, androgen receptor, estrogen receptor, interleukin-1 receptor-associated kinase 4 (IRAK4), Aurora kinase A, Bcr-Abl protein, epidermal growth factor receptor, anaplastic lymphoma kinase, Bruton's tyrosine kinase, BRM/BRG1 protein, bromodomain-containing protein 4.

In one embodiment, the PTM is a moiety of the following compounds: a Hsp90 inhibitor, a kinase inhibitor, a phosphatase inhibitor, a MDM2 inhibitor, a compound that binds to a protein comprising the human BET bromodomain, a HDAC inhibitor, a human lysine methyltransferase inhibitor, a compound that binds to RAF receptor, a compound that binds to FKBP, an angiogenesis inhibitor, an immunosuppressive compound, an compound that binds to aryl hydrocarbon receptor, a compound that binds to androgen receptor, a compound that binds to estrogen receptor, a compound that binds to IRAK4, a compound that binds to thyroid hormone receptor, a compound that binds to HIV protease, a compound that binds to HIV integrase, a compound that binds to HCV protease, a compound that binds to acyl protein thioesterase 1 and/or 2, a compound that binds to c-MYC protein, a compound that binds to laser kinase A, a compound that binds to Bcr-Abl protein, a compound that binds to bromodomain protein 4 (BRD4), a compound that binds to anaplastic lymphoma kinase (ALK), a compound that binds to Bruton's tyrosine kinase (BTK), a compound that binds to cyclin-dependent kinase 4/6 (CDK4/6), a compound that binds to SMARCA2/4 (BRM/BRG1),or a compound that binds to epidermal growth factor receptor.

In one embodiment, the PTM is a moiety that binds to an estrogen receptor. in one embodiment, the PTM is selected from the following structures:

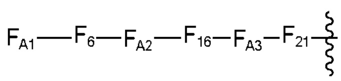

and

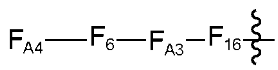

;

wherein, each occurrence of $F_6$, $F_{16}$, and $F_{21}$ is independently selected from one, or a combination of multiple, selected from a single bond, NH, SO, S, O, $SO_2$, alkylene, haloalkylene, heteroalkylene, alkyleneoxy, heteroalkyleneoxy, alkenylene, alkynylene, C(=O), OC(=O), C(=O)O, C(=O)NH, and NHC(=O); wherein the alkylene, alkyleneoxy, and alkenylene are optionally substituted by 0, 1, 2, 3, 4, 5, or 6 Rc; in one embodiment, each occurrence of $F_6$, $F_{16}$, and $F_{21}$ is independently selected from a single bond, NH, SO, S, O, $SO_2$, C(=O), OC(=O), and NHC(=O); $F_{A1}$ and $F_{A3}$ are each independently 6-10 membered aryl, or 6-10 membered heteroaryl; the aryl or heteroaryl is optionally substituted by 0, 1, 2, 3, 4, 5, or 6 $R_{da}$; in one embodiment, $F_{A1}$ is phenyl, and is optionally substituted by 0, 1, 2, 3, 4, 5, or 6 $R_{da}$; $F_{A3}$ is phenyl, or 6 membered heteroaryl containing 1, 2, or 3 heteroatoms selected from N, O, and P, and is optionally substituted by 0, 1, 2, 3, 4, 5, or 6 $R_{da}$;

$F_{A2}$ is 3-15 membered cycloalkylene, 5-15 membered spirocycloalkylene, 3-15 membered heterocycloalkylene, or 5-15 membered spiroheterocycloalkylene, and is optionally substituted with 0, 1, 2, 3, 4, 5, or 6 $R_{ca}$; in one

embodiment, FA2 is 4-6 membered cycloalkylene, 7-11 membered spirocycloalkylene, 4-6 membered heterocycloalkylene, 7-11 membered spiroheterocycloalkylene, and is optionally substituted with 0, 1, 2, 3, 4, 5, or 6 $R_{ca}$; in one embodiment, $F_{A2}$ is 4, 5, or 6 membered cycloalkylene, 10 membered spiroheterocycloalkylene containing 1, 2, or 3 nitrogen atoms, and is optionally substituted with 0, 1, 2, 3, 4, 5, or 6 $R_{ca}$;

$F_{A4}$ is a 6-10 membered aryl-fused 7-15 membered heterospirocyclic group, optionally substituted with 0, 1, 2, 3, 4, 5, or 6 $R_{da}$ substituents; in one embodiment, $F_{A4}$ is a phenyl-fused 7-15 membered heterospirocyclic group containing 1, 2, 3, or 4 heteroatoms selected from N, O, S, optionally substituted with 0, 1, 2, 3, 4, 5, or 6 $R_{da}$ substituents;

each occurrence of $R_{da}$ is independently selected from H, halogen, $C_{1-6}$ alkyl, $C_{1-6}$ haloalkyl, $C_{1-6}$ heteroalkyl, $C_{1-6}$ alkoxy, $C_{1-6}$ heteroalkoxy, $C_{3-7}$ cycloalkyl, $C_{3-7}$ heterocyclyl, $C_{6-8}$ aryl, $C_{5-8}$ heteroaryl, OH, $NH_2$, CN, and $NO_2$; in one embodiment, each occurrence of $R_{da}$ is independently selected from H, F, Cl, Br, I, $CH_3$, $OCH_3$, $CF_3$, OH, $NH_2$, CN, and $NO_2$;

each occurrence of $R_{ca}$ is independently selected from H, halogen, $C_{1-6}$ alkyl, $C_{1-6}$ haloalkyl, $C_{1-6}$ heteroalkyl, $C_{1-6}$ alkoxy, $C_{1-6}$ heteroalkoxy, $C_{3-7}$ cycloalkyl, $C_{3-7}$ heterocyclyl, $C_{6-8}$ aryl, $C_{5-8}$ heteroaryl, oxo (=O), thioxo (=S), OH, $NH_2$, CN, and $NO_2$; in one embodiment, $R_{ca}$ is H, F, Cl, Br, I, $CH_3$, $OCH_3$, $CF_3$, OH, $NH_2$, CN, and $NO_2$.

In one embodiment, the PTM is selected from the following structures:

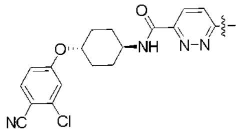,

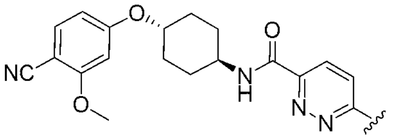, 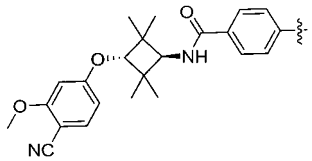, 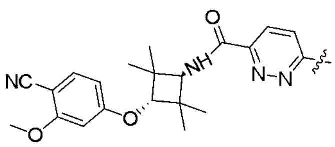,

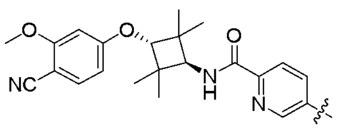, 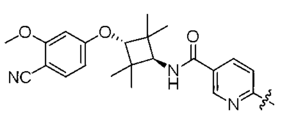, 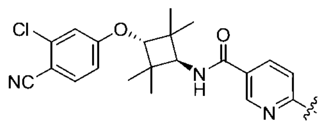,

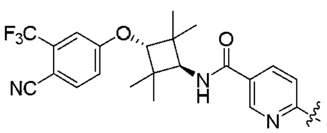, 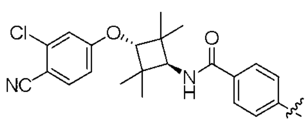, 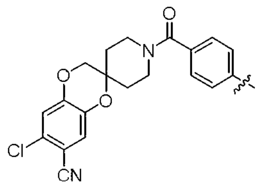, 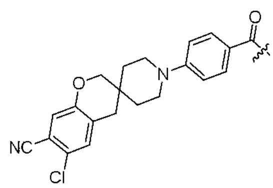,

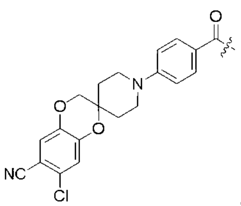, 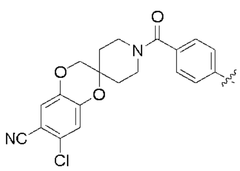 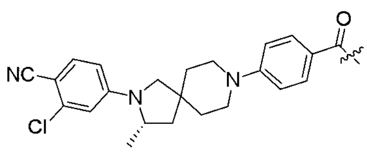 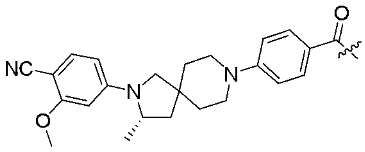,

and

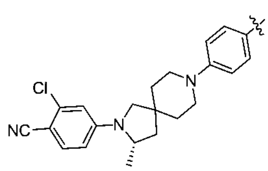;

in one embodiment, the PTM is selected from the following structures:

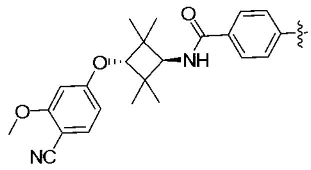, 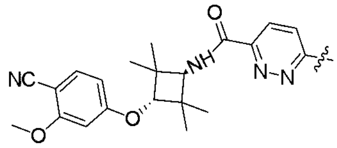, 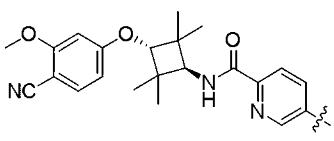,

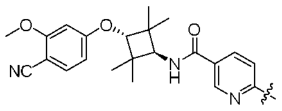, 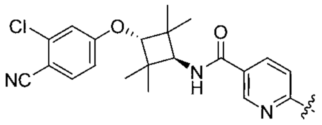, 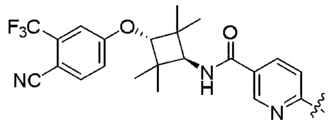,

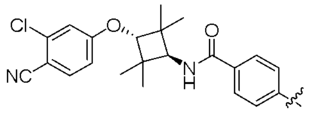, 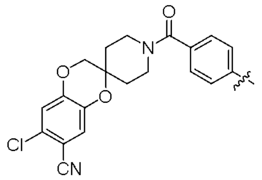, 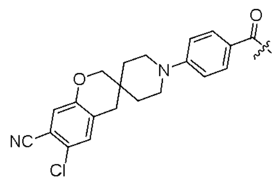, 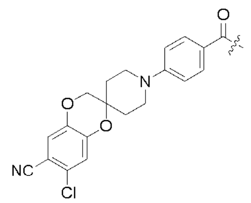,

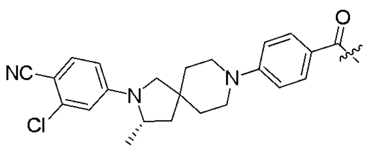, 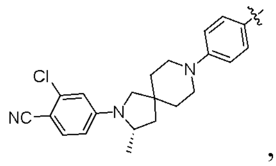, and 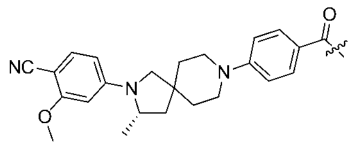.

In one embodiment, the PTM is a moiety that binds to an estrogen receptor, in one embodiment, the PTM is selected from the following structure:

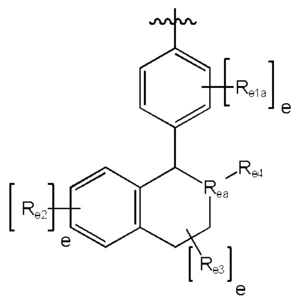;

$R_{ea}$ is selected from $CR_{e1a}$ and N;

each occurrence of $R_{e1a}$, $R_{e2}$, and $R_{e3}$ is independently selected from H, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, and amino, in one embodiment, each occurrence of $R_{e1a}$, and $R_{e2}$ is independently selected from hydroxyl, H, F, Cl, Br, I, and $C_1$-$C_3$ alkyl;

each occurrence of e is independently selected from 0, 1, 2, 3, and 4;

$R_{e4}$ is selected from 6-10 membered aryl, 6-10 membered heteroaryl, $C_1$-$C_6$ alkyl, $C_1$-$C_6$ haloalkyl, the 6-10 membered aryl, or 6-10 membered heteroaryl is optionally substituted with 0, 1, 2, 3, 4, or 5 $R_{e1a}$; in one embodiment, $R_{e4}$ is selected from phenyl and difluoroethyl, the phenyl is optionally substituted with 0, 1, 2, 3, 4, or 5 $R_{e1}$;

In one embodiment, the PTM is selected from the following structures:

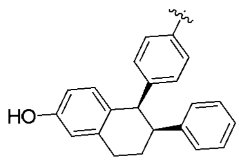 and 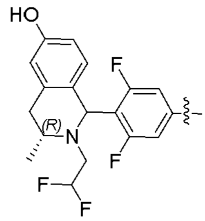 .

In one embodiment, the PTM is a moiety that binds to an estrogen receptor, in one embodiment, the PTM is selected from the following structure:

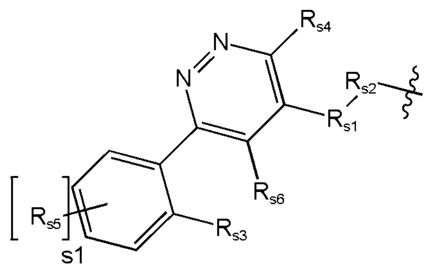

$R_{s1}$ is selected from one, or a combination of multiple, selected from a single bond, NH, SO, S, O, $SO_2$, alkylene, haloalkylene, heteroalkylene, alkyleneoxy, heteroalkyleneoxy, alkenylene, alkynylene, C(=O), OC(=O), C(=O)O, C(=O)NH, and NHC(=O); in one embodiment, $R_{s1}$ is selected from a single bond, $C_{2-6}$ alkenylene, and $C_{2-6}$ alkynylene;

$R_{s2}$ is selected from 6-10 membered arylene, 6-10 membered heteroarylene, 3-15 membered cycloalkylene, 3-15 membered heterocyclylene, and 5-15 membered spirocycloalkylene, wherein the 6-10 membered arylene, 6-10 membered heteroarylene, 3-15 membered cycloalkylene, and 5-15 membered spirocycloalkylene are each optionally substituted with 0, 1, 2, 3, 4, 5, or 6 $R_{s5}$; in one embodiment, $R_{s2}$ is selected from 5-8 membered heteroarylene containing 1, 2, or 3 heteroatoms selected from N, O, or S, 4-6 membered heterocyclylene containing 1, 2, or 3 heteroatoms selected from N, O, or S, and 7-11 membered spirocycloalkylene, wherein the 5-8 membered heteroarylene containing 1, 2, or 3 heteroatoms selected from N, O, or S, the 4-6 membered heterocyclylene containing 1, 2, or 3 heteroatoms selected from N, O, or S, and the 7-11 membered spirocycloalkylene are each optionally substituted with 0, 1, 2, 3, 4, 5, or 6 $R_{s5}$; in one embodiment, $R_{s2}$ is selected from pyrazolylene, 4-membered heterocyclylene containing one nitrogen atom, and 8-membered spirocycloalkylene;

each occurrence of $R_{s3}$, $R_{s4}$, $R_{s5}$, and $R_{s6}$ is independently selected from H, halogen, $C_{1-6}$ alkyl, $C_{1-6}$ haloalkyl, $C_{1-6}$ heteroalkyl, $C_{1-6}$ alkoxy, $C_{1-6}$ heteroalkoxy, $C_{3-7}$ cycloalkyl, $C_{3-7}$ heterocyclyl, $C_{6-8}$ aryl, $C_{5-8}$ heteroaryl, OH, $NH_2$, CN, and $NO_2$, in one embodiment, each occurrence of $R_{s3}$, $R_{s4}$, $R_{s5}$, and $R_{s6}$ is independently selected from H, F, Cl, Br, I, $CH_3$, $OCH_3$, $CF_3$, OH, $NH_2$, CN, and $NO_2$; in one embodiment, $R_{s3}$ is OH, $R_{s4}$ is $NH_2$; and

s1 is selected from 0, 1, 2, 3, and 4;

In one embodiment, the PTM is selected from:

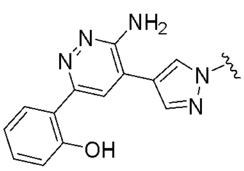 , 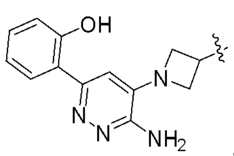 , and 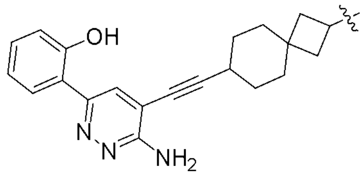 .

A third aspect of the present invention provides a pharmaceutical composition, wherein the pharmaceutical composition comprises the compound as defined above and a pharmaceutically acceptable excipient.

A fourth aspect of the present invention provides a use of the compound or the pharmaceutical composition as defined above in the manufacture of a medicament for treating or preventing a condition treated by degradation of a target protein bound to a target protein ligand, wherein in one embodiment, the target protein is cyclin-dependent kinase 4/6, androgen receptor, estrogen receptor, Aurora kinase A, IRAK4, Bcr-Abl protein, epidermal growth factor receptor, anaplastic lymphoma kinase, Bruton's tyrosine kinase, BRM/BRG1 protein, or bromodomain-containing protein 4.

A fifth aspect of the present invention provides a use of the compound or the pharmaceutical composition as

defined above in the manufacture of a medicament for treating or preventing a condition treated by binding to a cereblon protein in vivo.

A sixth aspect of the present invention provides a use of the compound or the pharmaceutical composition as defined above in treating or preventing a condition associated with accumulation, wherein in one embodiment aggregation, of a target protein, wherein in one embodiment, the target protein is cyclin-dependent kinase 4/6, androgen receptor, estrogen receptor, Aurora kinase A, Bcr-Abl protein, IRAK4, epidermal growth factor receptor, anaplastic lymphoma kinase, Bruton's tyrosine kinase, BRM/BRG1 protein, or bromodomain-containing protein 4.

A seventh aspect of the present invention provides the compound or the pharmaceutical composition as defined above for use in treating or preventing a condition treated by degradation of a target protein bound to a target protein ligand, wherein in one embodiment, the target protein is cyclin-dependent kinase 4/6, androgen receptor, estrogen receptor, Aurora kinase A, Bcr-Abl protein, IRAK4, epidermal growth factor receptor, anaplastic lymphoma kinase, Bruton's tyrosine kinase, BRM/BRG1 protein, or bromodomain-containing protein 4.

An eighth aspect of the present invention provides the compound or the pharmaceutical composition as defined above for use in treating or preventing a condition treated by binding to a cereblon protein in vivo.

A ninth aspect of the present invention provides the compound or the pharmaceutical composition as defined above for use in treating or preventing a condition associated with accumulation, wherein in one embodiment aggregation, of a target protein, wherein in one embodiment, the target protein is cyclin-dependent kinase 4/6, androgen receptor, estrogen receptor, Aurora kinase A, Bcr-Abl protein, IRAK4, epidermal growth factor receptor, anaplastic lymphoma kinase, Bruton's tyrosine kinase, BRM/BRG1 protein, or bromodomain-containing protein 4.

A tenth aspect of the present invention provides a method for treating or preventing a condition treated by degradation of a target protein bound to a target protein ligand, comprising administering a therapeutically effective amount of the compound or the pharmaceutical composition as defined above to a subject in need thereof, wherein in one embodiment, the target protein is cyclin-dependent kinase 4/6, androgen receptor, estrogen receptor, Aurora kinase A, IRAK4, Bcr-Abl protein, epidermal growth factor receptor, anaplastic lymphoma kinase, Bruton's tyrosine kinase, BRM/BRG1 protein, or bromodomain-containing protein 4.

A method for treating or preventing a condition treated by binding to a cereblon protein in vivo, comprising administering a therapeutically effective amount of the compound or the pharmaceutical composition as defined above to a subject in need thereof.

A method for treating or preventing a condition associated with accumulation, wherein in one embodiment aggregation, of a target protein, comprising administering the compound or the pharmaceutical composition as defined above to a subject in need thereof, wherein in one embodiment, the target protein is cyclin-dependent kinase 4/6, androgen receptor, estrogen receptor, Aurora kinase A, Bcr-Abl protein, IRAK4, epidermal growth factor receptor, anaplastic lymphoma kinase, Bruton's tyrosine kinase, BRM/BRG1 protein, or bromodomain-containing protein 4.

In one embodiment, the condition is a tumor or cancer.

The present invention further provides a method for inducing degradation of a target protein in a cell, the method comprising administering an effective amount of the compound or the pharmaceutical composition as defined above, wherein in one embodiment, the target protein is cyclin-dependent kinase 4/6, androgen receptor, estrogen receptor, Aurora kinase A, Bcr-Abl protein, IRAK4, epidermal growth factor receptor, anaplastic lymphoma kinase, Bruton's tyrosine kinase, BRM/BRG1 protein, or bromodomain-containing protein 4.

Detailed Description

### Definitions

The term "alkyl" as considered herein refers to a saturated aliphatic hydrocarbon group, which is a straight-chain or branched group containing 1 to 20 carbon atoms, wherein in one embodiment it contains an alkyl group having 1 to 12 (e.g., 1, 2, 3, 4, 5, 6, 7, 8, 9, 10, 11, and 12) carbon atoms, and in one further embodiment, an alkyl group containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and their various branched-chain isomers. In one embodiment, the alkyl group comprises 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl,

1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl groups may be substituted or unsubstituted. When substituted, substituents may be attached at any available bonding site. Wherein the substituent is independently and optionally selected in one embodiment from one or more substituents selected from H, D, halogen, alkyl, alkoxy, haloalkyl, hydroxyl, hydroxyalkyl, cyano, amino, nitro, cycloalkyl, heterocyclyl, aryl, or heteroaryl groups.

The term "heteroalkyl" refers to an alkyl group in which one or more $-CH_2-$ moieties are substituted by a heteroatom selected from NH, O, and S, or one or more -CH- moieties are substituted by an N atom; wherein said alkyl group is as defined above; The heteralkyl group may be substituted or unsubstituted. When substituted, the substituent may be attached at any available bonding site. Wherein the substituent is independently and optionally selected in one embodiment from one or more substituents selected from H, D, halogen, alkyl, alkoxy, haloalkyl, hydroxyl, hydroxyalkyl, cyano, amino, nitro, cycloalkyl, heterocyclyl , aryl, or heteroaryl. The term "alkoxy" refers to -O-(alkyl) and -O-(unsubstituted cycloalkyl), where alkyl or cycloalkyl is defined herein. Non-limiting examples of alkoxy groups include: methoxy, ethoxy, propoxy, butoxy, cyclopropoxy, cyclobutoxy, cyclopentoxy, and cyclohexyloxy. The alkoxy group may be optionally substituted or unsubstituted. When substituted, the substituent in one embodiment comprises one or more of the following groups independently selected from H, D, halogen, alkyl, alkoxy, haloalkyl, hydroxyl, hydroxyalkyl, cyano, amino, nitro, cycloalkyl, heterocyclyl, aryl or heteroaryl.

The term "alkene" refers to an alkyl compound containing a carbon-carbon double bond, wherein the alkyl is defined as described above. The alkene may be substituted or unsubstituted. When substituted, the substituent in one embodiment comprises one or more of the following groups independently selected from hydrogen, alkyl, alkoxy, halogen, haloalkyl, hydroxyl, hydroxyalkyl, cyano, amino, nitro, cycloalkyl, heterocyclyl, aryl, and heteroaryl.

The term "alkyne" refers to an alkyl compound containing a carbon-carbon triple bond in their molecules, where the definition of alkyl is as described above. The alkyne group may be substituted or unsubstituted. When substituted, the substituent in one embodiment comprises one or more of the following groups independently selected from hydrogen, alkyl, alkoxy, halogen, haloalkyl, hydroxyl, hydroxyalkyl, cyano, amino, nitro, cycloalkyl, heterocyclyl, aryl, and heteroaryl.

The term "cycloalkyl" refers to a saturated or partially unsaturated monocyclic cyclic hydrocarbon substituent, wherein the cycloalkyl ring contains 3 to 20 carbon atoms, in one embodiment contains 3 to 12 carbon atoms, in another embodiment contains 3 to 8 (e.g., 3, 4, 5, 6, 7, and 8) carbon atoms, and in yet another embodiment contains 4 to 7 carbon atoms. Non-limiting examples of single-ring cycloalkyl groups include cyclopropyl, cyclobutyl, cyclopentyl, cyclopentenyl, cyclohexyl, cyclohexenyl, cyclohexadienyl, cycloheptyl, cycloheptatriene, cyclooctyl, etc.

Cycloalkyl may be substituted or unsubstituted. When substituted, substituents may be attached at any available connection point, wherein the substituent is independently and optionally selected from hydrogen, halogen, alkyl, alkoxy, haloalkyl, hydroxyl, hydroxyalkyl, cyano, amino, nitro, cycloalkyl, heterocyclyl, aryl, or heteroaryl in one embodiment.

The term "heterocyclyl" refers to a saturated or partially unsaturated monocyclic cyclic hydrocarbon substituent containing 3 to 20 ring atoms, wherein one or more ring atoms are heteroatoms selected from nitrogen, oxygen, or S(O)m (where m is an integer from 0 to 2), but excluding ring segments consisting of -O-O-, -O-S-, or -S-S-, with the remaining ring atoms being carbon. In one embodiment, it comprises 3 to 12 (e.g., 3, 4, 5, 6, 7, 8, 9, 10, 11, and 12) ring atoms, wherein 1 to 4 (e.g., 1, 2, 3, and 4) are heteroatoms; In one further embodiment, the heterocyclyl comprises 3 to 8 ring atoms, wherein 1 to 3 are heteroatoms; in one further embodiment, the heterocyclyl comprises 3 to 6 ring atoms, wherein 1 to 3 are heteroatoms; and in one final embodiment, the heterocyclyl comprises 5 or 6 ring atoms, wherein 1 to 3 are heteroatoms. Non-limiting examples of single-ring heterocyclyl include pyrrolidine, tetrahydropyran, 1,2,3,6-tetrahydropyridine, piperidine, piperazine, morpholine, thiomorpholine, and homopiperazine, etc.

The heterocyclyl group may be substituted or unsubstituted. When substituted, substituents may be attached at any available connection point. Wherein the substituent is independently and optionally substituted in one embodiment by one or more substituents selected from hydrogen, halogen, alkyl, alkoxy, haloalkyl, hydroxyl, hydroxyalkyl, cyano, amino, nitro, cycloalkyl, heterocyclyl, aryl, or heteroaryl groups. The term "aryl" refers to a 6- to 14-membered monocyclic or fused polycyclic (where fused polycyclic rings share adjacent carbon atom pairs) group possessing a conjugated $\pi$-electron system, with 6 to 10 members in one embodiment, such as phenyl and naphthyl. The aromatic ring may be fused to a heteroaromatic, heterocyclic, or cycloalkyl ring as described above, provided that the ring connected to the parent structure is an aromatic ring. The aryl may be substituted or unsubstituted. When substituted, the substituent may be attached at any available connection point. Wherein the substituent is independently and optionally substituted in one embodiment by one or more substituents selected from hydrogen, halogen, alkyl, alkoxy, haloalkyl, hydroxyl, hydroxyalkyl, cyano, amino, nitro, cycloalkyl, heterocyclyl, aryl, or heteroaryl.

The term "heteroaryl" refers to a heteroaromatic system containing 1 to 4 heteroatoms (e.g., 1, 2, 3, and 4) and 5 to 14 ring atoms, wherein the heteroatoms are selected from oxygen, sulfur, and nitrogen. In one embodiment, the heteroaryl comprises 5 to 10 members (e.g., 5, 6, 7, 8, 9, or 10 members), and in another embodiment comprises 5 or 6 members, such as furan, thiophene, pyridine, pyrrole, N-alkylpyrrole, pyrimidine, pyrazine, pyridazine, imidazole, pyrazole, triazole, or tetrazole. The heteroaryl ring described herein includes heteroaryl rings fused to aryl, heterocyclyl, or cycloalkyl rings as described above, wherein the ring connected to the parent structure is the heteroaryl ring. The

heteroaryl may be substituted or unsubstituted. When substituted, the substituent may be attached at any available connection point. Wherein the substituent is independently and optionally substituted in one embodiment by one or more substituents selected from hydrogen, halogen, alkyl, alkoxy, haloalkyl, hydroxyl, hydroxyalkyl, cyano, amino, nitro, cycloalkyl, heterocyclyl, aryl, or heteroaryl. The term "haloalkyl" refers to an alkyl group substituted by one or more halogens, where the alkyl group is defined as above.

The term "hydroxyalkyl" refers to an alkyl group, as defined above, substituted by one or more hydroxyl.

The term "halogen" refers to fluorine, chlorine, bromine, or iodine.

The term "amino" refers to $-NH_2$.

The term "cyano" refers to -CN.

The term "nitro" refers to $-NO_2$.

The term "ubiquitin ligase" refers to a family of proteins that catalyze the transfer of ubiquitin to specific substrate proteins, thereby targeting these substrate proteins for degradation. For example, cerebellum is an E3 ubiquitin ligase protein that, either alone or in combination with E2 ubiquitin ligases, causes ubiquitination of lysine residues on target proteins. This subsequently targets specific protein substrates for degradation by the proteasome. Therefore, E3 ubiquitin ligases-either acting independently or as part of a complex with E2 ubiquitin ligases-are responsible for transferring ubiquitin to target proteins. Generally speaking, ubiquitin ligases participate in polyubiquitination, whereby the second ubiquitin is attached to the first ubiquitin, the third ubiquitin is attached to the second ubiquitin, and so on. Polyubiquitinated proteins are targeted for degradation by the proteasome. However, there exist some ubiquitination events restricted to monoubiquitination, in which only a single ubiquitin is added to the substrate molecule by the ubiquitin ligase. Unconjugated ubiquitinated proteins are not targeted to the proteasome for degradation, but may instead undergo changes in their cellular localization or function, such as through binding to other proteins possessing ubiquitin-binding domains. To complicate matters further, different lysine residues on ubiquitin can be targeted by E3 ligases to form chains. The most common lysine is Lys48 on the ubiquitin chain. This lysine is used to form polyubiquitin, which is recognized by the proteasome.

The term "target protein" refers to proteins and peptides possessing any biological function or activity, including structural, regulatory, hormonal, enzymatic, genetic, immune, contractile, storage, transport, and signal transduction functions. In certain implementation schemes, target proteins include structural proteins, receptors, enzymes, cell surface proteins, and proteins involved in cellular integration functions, encompassing those associated with the following activities: catalytic activity, aromatase activity, motility activity, helicase activity, metabolic processes (anabolism and catabolism), antioxidant activity, proteolysis, biosynthesis, proteins with kinase activity, oxidoreductase activity, transferase activity, hydrolase activity, lyase activity, isomerase activity, ligase activity, enzyme regulatory factor activity, signal transduction factor activity, structural molecule activity, binding activity (proteins, lipids-carbohydrates), receptor activity, cell motility, membrane fusion, cell communication, biological process regulation, development, cell differentiation, stimulus response, behavioral proteins, cell adhesion proteins, proteins involved in cell death, proteins involved in transport (including protein transport activity, nuclear transport, ion transport activity, channel transport activity, carrier activity), permease activity, secretory activity, electron transport activity, pathogens, chaperone regulatory factor activity, nucleic acid binding activity, transcription regulatory factor activity, extracellular structure and biological origin activity, translational regulatory factor activity. The proteins described include those derived from eukaryotes and prokaryotes, with eukaryotes and prokaryotes encompassing microorganisms, viruses, fungi, parasites, and numerous others. This includes humans, microorganisms, viruses, fungi, and parasites serving as targets for drug therapies, other animals such as domesticated animals, microorganisms used to determine targets for antibiotics and other antimicrobial agents, plants, viruses, and numerous other entities.

The term "isomer" refers to that the compounds of the present invention may possess an asymmetric center and may exist as racemates, racemic mixtures, or individual diastereomers. All such isomers, including stereoisomers and geometric isomers, are encompassed within the scope of the present invention. In the present invention, when a compound or its salt exists in stereoisomeric form (e.g., containing one or more asymmetric carbon atoms), the individual stereoisomers (enantiomers and diastereomers) as well as mixtures thereof are included within the scope of the invention. The invention also encompasses individual isomers of the compound or salt, as well as mixtures of isomers with one or more of their chiral centers reversed. The scope of the present invention includes mixtures of stereoisomers, as well as purified enantiomers or mixtures enriched in enantiomers or enantiomer/diastereomer mixtures. The present invention encompasses mixtures of stereoisomers comprising all possible combinations of enantiomers and diastereomers. The present invention encompasses all combinations and subsets of stereoisomers of all specific groups defined above. The present invention also includes geometric isomers of the compound or its salts, wherein the geometric isomers include cis-trans isomers.

The term "isotope derivative" refers to a compound differing structurally only in the presence of one or more isotopically enriched atoms. For example, structures according to the present disclosure encompass compounds wherein hydrogen atoms are substituted with deuterium or tritium, fluorine atoms are substituted with $^{18}F$-fluorine ($^{18}F$ isotope), or carbon atoms are substituted with $^{11}C$-, $^{13}C$-, or $^{14}C$-enriched carbon ($^{11}C$-, $^{13}C$-, or $^{14}C$-carbon labeling; $^{11}C$-, $^{13}C$-, or

$^{14}C$-isotopes) instead of carbon atoms. Such compounds can serve as analytical tools or probes in biological assays, or as in vivo diagnostic imaging tracers for disease detection, or as tracers for pharmacodynamic, pharmacokinetic, or receptor studies.

"Optional" or "optionally" means that the event or circumstance described subsequently may or may not occur; the description encompasses both instances where the event or circumstance occurs and those where it does not. For example, "the cyclopropyl group may optionally be substituted" means that the cyclopropyl group may be substituted but need not necessarily be present. This description encompasses both cases where the cyclopropyl group is substituted and where it is not substituted.

"Substituted" refers to one or more hydrogen atoms in the group, with up to five hydrogen atoms in one embodiment and one to three hydrogen atoms in another embodiment, each independently replaced by the corresponding number of substituents. It goes without saying that substituents occupy only their possible chemical positions, and those skilled in the art can readily determine (through experimentation or theory) which substituents are possible or impossible without undue effort.

"Pharmaceutically acceptable salts" or "pharmaceutically acceptable salt" refers to salts of the compounds disclosed herein that are safe and effective for use in mammals and possess the desired biological activity.

## Examples

The following examples are provided by way of illustration and not limitation.

The abbreviations used hereinare as follows:

MeOH is methanol;
$H_2SO_2$ is concentrated sulfuric acid;
NIS is N-iodinated succinimide;
TFA is trifluoroacetic acid;
Pd is palladium;
Sn is tin;
Pyrrolidine is tetrahydropyrole;
OH is hydroxyl;
Boc is tert-butoxycarbonyl;
$NaBH_4$ is sodium borohydride;
$Et_3SiH$ is triethylsilane;
$H_2$ is hydrogen;
LiOH is lithium hydroxide;
NaOAc is sodium acetate;
AcOH is acetic acid;
PE is petroleum ether;
EA is ethyl acetate;
$CDCl_3$ is deuterated chloroform;
$HNO_3$ is nitric acid;
$HBF_4$ is tetrafluoroboric acid;
$NaNO_2$ is sodium nitrite;
NBS is N-bromosuccinimide;
KOtBu is potassium tert-butylate;
NaH is sodium hydride;
$B_2pin_2$ is boric acid pinacol ester;
Oxone is potassium peroxymonosulfate;
Cbz is benzyloxycarbonyl;
$PPh_3$ is triphenylphosphine;
$CBr_4$ is carbon tetrabromide;
Zn is zinc;
$NH_4Cl$ is ammonium chloride;
B is boron;
Br is bromine;
$(TMS)_3SiH$ is tris(trimethylsilyl)silane;
AIBN is azobisisobutyronitrile;
DMF is N,N-dimethylformamide;
$K_2CO_3$ is potassium carbonate;

TBAB is tetrabutylammonium bromide;
Bn is benzyl;
$LiAlH_4$ is lithium aluminum hydride;
$BH_3$ is borane;
THF is tetrahydrofuran;
$H_2O_2$ is hydrogen peroxide;
DMP is the Dais-Martin oxidizer;
$PBr_3$ is phosphorus tribromide;
$Pd(OAc)_2$ is palladium acetate;
$BF_3Et_2O$ is boron trifluoride etherate;
S is sulfur;
MsCl is methanesulfonyl chloride;
TEA is triethylamine;
DBU is 1,8-diazabicyclo[5.4.0]undec-7-ene;
$NaHCO_3$ is sodium bicarbonate;
Malonic acid is malonate;
PPA is polyphosphoric acid;
HBr is hydrogen bromide;
$Tf_2O$ is trifluoromethanesulfonic anhydride;
Pd/C is palladium on carbon;
DMSO is dimethyl sulfoxide;
UPLC stands for ultra-performance liquid chromatography;
$MgCl_2$ is magnesium chloride;
NADPH is nicotinamide adenine dinucleotide phosphate;
$NaS_2O_3$ is sodium thiosulfate;
$Pd(dppf)Cl_2$ or $PdCl_2(dppf)$ is [1,1'-Bis(diphenylphosphino) ferrocene] dichloropalladium;
$Boc_2O$ is di-tert-butyl carbonate;
AIBN is azobisisobutyronitrile;
DIPEA or DIEA is N,N-diisopropylethylamine;
TsOH is p-toluenesulfonic acid;
$CCl_4$ is carbon tetrachloride;
CAN is ammonium cerium nitrate;
IBX is 2-iodobenzoylbenzoic acid;
HATU is 2-(7-azabenzotriazole)-N,N,N',N'-tetramethylurea hexafluorophosphate.

The following examples pertain to the intermediate compounds and final products identified in the specification and synthesis scheme. The following examples provide detailed descriptions of the preparation of the compounds of the present invention, but the chemical reactions described are disclosed based on their general applicability to the preparation of the compounds of the present invention. At times, the reactions described may not be applicable to every compound within the scope of the present invention as described. Those skilled in the art can readily identify compounds where this situation occurs. In such cases, the reaction can be successfully carried out through conventional modifications known to those skilled in the art. In all preparation methods, all starting materials are known or can be readily prepared from known starting materials.

The starting materials, chemical reagents, and solvents used in this disclosure are commercially available and were purchased from companies including Anajie Chemical, Shanghai Bide Pharmaceutical, Beijing Innochem, Jiangsu Aikang, Sinopharm Group, Beijing Bailingwei, and Yunnan Xinlanjing.

The structure of the compound was determined by nuclear magnetic resonance (NMR) and mass spectrometry (MS) in one of the embodiments. Nuclear magnetic resonance (NMR) measurements were performed using a Bruker AVANCE-400/600 NMR spectrometer. Deuterated solvents employed included deuterated dimethyl sulfoxide (DMSO-$d_6$), deuterated chloroform ($CDCl_3$), and deuterated methanol ($CD_3OD$). Tetramethylsilane (TMS) served as the internal standard.

Mass spectrometry (MS) measurements were performed using a Waters Acquity UPLC® Plus system. High-performance liquid chromatography preparations were conducted using a Waters 2489 system. The medium-pressure rapid preparation chromatograph utilizes the COMBIFLASH NEXTGEN 300+ instrument. Thin-layer chromatography silica gel plates use Silica gel 60 thin-layer chromatography silica gel plates (aluminum plates, fluorescent). The silica gel (100-200 mesh, 200-300 mesh) used in silica gel thin-layer chromatography was purchased from Innochem.

The reaction progress in the examples was monitored using thin-layer chromatography (TLC). The systems employed for the developing solvent in the reaction and the eluent in the column chromatography purification of the

compound included: petroleum ether/ethyl acetate system and dichloromethane/methanol system.

**Example 1**

Synthesis of Compound A1

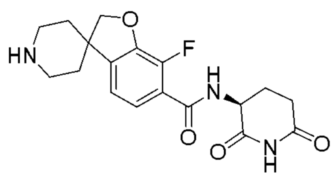

(S)-N-(2,6-dioxopiperidin-3-yl)-7-fluoro-2H-spiro[benzofuran-3,4'-piperidine]-6-carboxamide

Synthesis Scheme

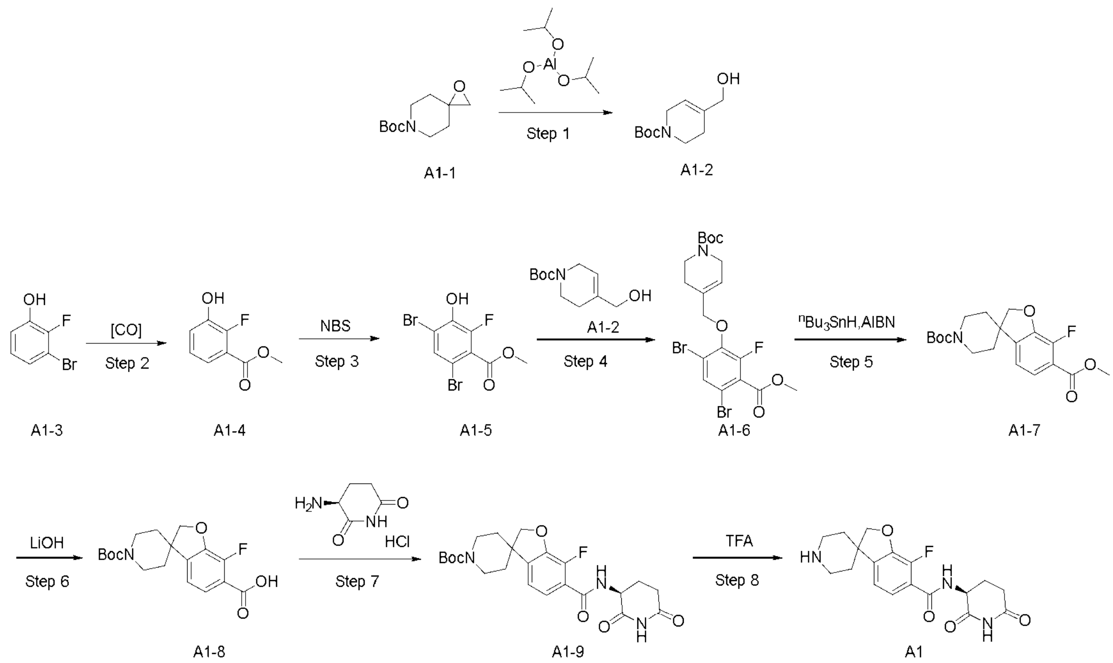


Step 1: 4-(hydroxymethyl)-3,6-dihydropyridine-1(2H)-carboxylic acid tert-butyl ester (compound A1-2)

The compound A1-1 (10.0 g, 46.9 mmol, 1.0 eq) was dissolved in toluene (100 mL), and aluminum isopropoxide (12.5 g, 61.0 mmol, 1.3 eq) was added. The mixture was reacted at 120°C for 24 hours. The reaction system was cooled to room temperature. The dilute hydrochloric acid (1.0 mol/L, 200 mL) was added to the system. The system was extracted three times with ethyl acetate and the organic phase was collected. The crude product obtained from the concentrated organic phase was purified by column chromatography to afford the colorless oily compound A1-2 (4.7 g, 47%).

$^1$H NMR (600 MHz, Chloroform-d) δ 5.67 (s, 1H), 4.07 (s, 2H), 3.98 - 3.92 (m, 2H), 3.54 (dd, *J* = 8.2, 4.0 Hz, 2H), 2.15 (d, *J*= 6.8 Hz, 2H), 1.49 (s, 9H).
LCMS (ESI): [M-tBu+H] $^+$ = 158.20

Step 2: Methyl 2-fluoro-3-hydroxybenzoate (compound A1-4)

The compound A1-3 (5.0 g, 26.2 mmol, 1.0 eq) and triethylamine (8.0 g, 78.5 mmol, 3.0 eq) was dissolved in

methanol (50 mL), followed by addition of 1,1-bis (diphenylphosphino) dimethanedio-iron(II) dichloropalladium (2.9 g, 3.9 mmol, 0.15 eq). The mixture was reacted at 70°C under pressurized carbon monoxide gas for 24 hours. The reaction mixture was concentrated, and the crude product was purified by column chromatography to afford the white solid compound A1-4 (2.4 g, 54%).

$^1$H NMR (600 MHz, DMSO-$d_6$) δ 10.24 (s, 1H), 7.27 - 7.23 (m, 1H), 7.22 - 7.18 (m, 1H), 7.12 - 7.07 (m, 1H), 3.84 (s, 3H).

LCMS (ESI): $[M+H]^-$ = 169.23.

Step 3: Methyl 4,6-dibromo-2-fluoro-3-hydroxybenzoate (compound A1-5)

The compound A1-4 (2.0 g, 11.8 mmol, 1.0 eq) was dissolved in acetonitrile (20 mL). After stirring at room temperature, trifluoroacetic acid (10 mL) was added, followed by adding the N-bromo succinimide (4.2 g, 23.5 mmol, 2.0 eq). The reaction mixture was maintained at 50°C for 12 hours. The crude product obtained from the concentrated organic phase was purified by column chromatography to afford a yellow oily compound A1-5 (2.5 g, 65%).

$^1$H NMR (600 MHz, DMSO-$d_6$) δ 11.37 (s, 1H), 7.78 (d, $J$ = 2.0 Hz, 1H), 3.90 (s, 3H).

LCMS (ESI): $[M+H]^-$ = 327.06.

Step 4: 4-((4,6-dibromo-2-fluoro-3-(methoxycarbonyl)phenoxy) methyl)-3,6-dihydropyridine-1(2H)-carboxylic acid tert-butyl ester (compound A1-6)

The compound A1-5 (2.0 g, 6.1 mmol, 1.0 eq), compound A1-2 (1.69 g, 7.9 mmol, 1.3 eq), and triphenylphosphine (3.2 g, 12.2 mmol, 2.0 eq) were dissolved in tetrahydrofuran (20 mL) and stirred at 0°C under nitrogen for 15 minutes. Diethyl azodicarboxylate (2.1 g, 12.2 mmol, 2.0 eq) was slowly added dropwise to the reaction mixture, which was then allowed to react at room temperature for 15 hours. The reaction mixture was concentrated, and the crude product was purified by column chromatography to afford a colorless oily compound A1-6 (2.8 g, 88%).

$^1$H NMR (600 MHz, DMSO-$d_6$) δ 7.94 (d, $J$ = 1.7 Hz, 1H), 5.82 (s, 1H), 5.75 - 5.73 (m, 2H), 4.54 (s, 2H), 3.92 (s, 3H), 3.89 - 3.80 (m, 2H), 2.25 - 2.19 (m, 2H), 1.40 (s, 9H).

LCMS (ESI): $[M\text{-}Boc+H]^+$ = 424.09.

Step 5: 1'-(tert-Butyl) 6-methyl-7-fluoro-2H-spiro [benzofuran-3,4'-piperidine]-1',6-dicarboxylate (compound A1-7)

The compound A1-6 (2.0 g, 3.8 mmol, 1.0 eq) was dissolved in toluene (20 mL). Azobisisobutyronitrile (952 mg, 5.7 mmol, 1.5 eq) and tributyltin hydride (3.3 g, 11.5 mmol, 3.0 eq) were added at room temperature, and were reacted at 110°C for 2 hours. The reaction mixture was concentrated, and the crude product was purified by column chromatography to afford a colorless oily compound A1-7 (1.1 g, 79%).

$^1$H NMR (600 MHz, DMSO-$d_6$) δ 7.37 (ddd, $J$ = 7.5, 5.7, 1.4 Hz, 1H), 7.22 (dd, $J$ = 7.9, 1.3 Hz, 1H), 4.03 (qd, $J$ = 7.2, 1.3 Hz, 2H), 3.96 - 3.88 (m, 2H), 3.83 (s, 3H), 2.99 - 2.81 (m, 2H), 1.80 - 1.67 (m, 4H), 1.42 (s, 9H).

LCMS (ESI): $[M\text{-}tBu+H]^+$ = 310.28.

Step 6: 1'-(tert-butoxycarbonyl)-7-fluoro-2H-spiro [benzofuran-3,4'-piperidine]-6-carboxylic acid (compound A1-8)

The compound A1-7 (500 mg, 1.4 mmol, 1.0 eq) was dissolved in tetrahydrofuran: water = 3:1 (8 mL). Lithium hydroxide (287 mg, 6.8 mmol, 5.0 eq) was added, and the reaction mixture was allowed to react at room temperature for 4 hours. 1 N dilute hydrochloric acid was added to the reaction system to adjust the pH to 5-6, then the mixture was extracted three times with ethyl acetate and the organic phase was collected. The organic phases were combined and dried using anhydrous sodium sulfate, and was concentrated to obtain a crude white solid compound A1-8 (300 mg).

LCMS (ESI): $[M\text{-}Boc+H]^+$ = 252.27.

Step 7: (S)-6-((2,6-dioxopiperidin-3-yl) carbamoyl) -7-fluoro-2H-spiro [benzofuran-3,4'-piperidine]-1'-carboxylic acid tert-butyl ester (compound A1-9)

The compound A1-8 (300 mg, 0.9 mmol, 1.0 eq) was dissolved in N,N-dimethylformamide (5 mL). Under stirring at room temperature, (S)-3-aminopiperidine-2,6-dione hydrochloride (211 mg, 1.3 mmol, 1.5 eq), N,N-diisopropylethylamine (552 mg, 4.5 mmol, 5.0 eq), and N,N, N', N'-tetramethyl-O-(7-azabenzotriazol-1-yl)hexafluorophosphorylurea (390 mg, 1.0 mmol, 1.1 eq) were added. The mixture was allowed to react at room temperature for 1 hour. The reaction mixture was purified by reverse-phase column chromatography to afford the blue solid compound A1-9 (300 mg, 76%).

$^1$H NMR (600 MHz, DMSO-$d_6$) δ 10.86 (s, 1H), 8.55 (dd, $J$= 8.3, 1.8 Hz, 1H), 7.20 (d, $J$ = 7.7 Hz, 1H), 7.11 (dd, $J$ = 7.8, 5.6 Hz, 1H), 4.74 (ddd, $J$ = 12.3, 8.3, 5.4 Hz, 1H), 4.61 (s, 2H), 3.97 - 3.88 (m, 2H), 3.02 - 2.85 (m, 2H), 2.78 (ddd, $J$ =

17.3, 13.2, 5.6 Hz, 1H), 2.56 - 2.52 (m, 1H), 2.07 (qd, *J*= 12.9, 4.5 Hz, 1H), 2.03 - 1.97 (m, 1H), 1.77 (td, *J*= 12.8, 4.5 Hz, 2H), 1.73 - 1.67 (m, 2H), 1.43 (s, 9H).

LCMS (ESI): $[M\text{-}Boc+H]^+$ = 362.29.

Step 8: (S)-N-(2,6-dioxopiperidin-3-yl)-7-fluoro-2H-spiro [benzofuran-3,4'-piperidine]-6-carboxamide (compound A1)

The compound A1-9 (200 mg, 0.4 mmol, 1.0 eq) was dissolved in dichloromethane: trifluoroacetic acid = 10:1 (1 mL) and reacted at room temperature for 2 hours. The reaction mixture was concentrated and the crude product was purified by reverse-phase column chromatography to afford the white solid compound A1 (100 mg, 64%).

$^1$H NMR (600 MHz, DMSO-$d_6$) δ 10.87 (s, 1H), 8.78 - 8.76 (m, 1H), 8.57 (d, *J* = 8.3 Hz, 1H), 7.18 (ddd, *J*= 7.4, 5.6, 1.4 Hz, 1H), 7.08 (dd, *J* = 7.8, 1.5 Hz, 1H), 4.74 (dt, *J*= 13.1, 6.8 Hz, 1H), 4.68 (s, 2H), 3.38 - 3.32 (m, 2H), 3.03 (q, *J* = 12.1 Hz, 2H), 2.79 (ddd, *J* = 17.9, 13.0, 5.6 Hz, 1H), 2.57 - 2.52 (m, 1H), 2.13 - 1.97 (m, 4H), 1.94 - 1.88 (m, 2H). LCMS (ESI): $[M+H]^+$ = 362.39

Example 2

Synthesis of Compound A2

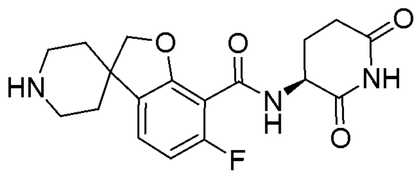

(S)-N-(2,6-dioxopiperidin-3-yl)-6-fluoro-2H-spiro[benzofuran-3,4'-piperidine]-7-carboxamide

Synthesis Scheme

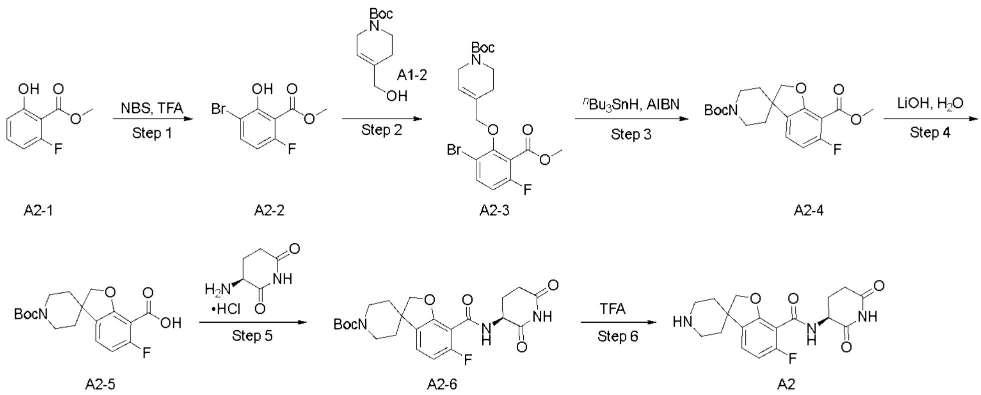


Step 1: Methyl 3-bromo-6-fluoro-2-hydroxybenzoate (compound A2-2)

The compound A2-1 (2.0 g, 11.8 mmol, 1.0 eq) was dissolved in 50 mL of methanol. N-bromosuccinimide (2.2 g, 12.3 mmol, 1.04 eq) was added at room temperature. 10 mL of trifluoroacetic acid was add to the reaction mixture,the temperature was raised to 40°C, and reacted for 12 hours at this temperature. The reaction mixture was concentrated under reduced pressure and purified by reverse-phase column chromatography to afford a white solid A2-2 (1.78 g, 61%).

$^1$H NMR (400 MHz, DMSO-$d_6$) δ 11.08 (s, 1H), 7.77 (dd, *J* = 8.9, 5.9 Hz, 1H), 6.84 - 6.80 (m, 1H), 3.89 (s, 3H).

LCMS (ESI): $[M\text{-}H]^-$ = 247.06.

Step 2: 4-((6-bromo-3-fluoro-2-(methoxycarbonyl) phenoxy) methyl)-3,6-dihydropyridine-1(2H)-carboxylic acid tert-butyl ester (compound A2-3)

The compound A2-2 (1.68 g, 6.8 mmol, 1.0 eq), compound A1-2 (1.73 g, 8.1 mmol, 1.2 eq), and triphenylphosphine (3.54 g, 13.5 mmol, 2.0 eq) were dissolved in tetrahydrofuran (50 mL) and stirred for 15 minutes under nitrogen at 0°C. Diethyl azodicarboxylate (2.3 g, 13.5 mmol, 2.0 eq) was slowly added dropwise to the reaction system, which was then allowed to react at room temperature for 16 hours. The reaction mixture was concentrated, and the crude product was purified by column chromatography to afford a colorless oily compound A2-3 (809 mg, 27%).

LCMS (ESI): $[M\text{-}Boc+H]^+$ = 344.19.

Step 3: 1'-(tert-Butyl) 7-methyl-6-fluoro-2H-spiro [benzofuran-3,4'-piperidine]-1',7-dicarboxylate (compound A2-4)

The compound A2-3 (809 mg, 1.8 mmol, 1.0 eq) was dissolved in toluene (20 mL). Azobisisobutyronitrile (448 mg, 2.7 mmol, 2.0 eq) and tributyltin hydride (1.59 g, 5.5 mmol, 3.0 eq) were added at room temperature and reacted at 110°C for 2 hours. The reaction mixture was concentrated, and the crude product was purified by column chromatography to afford a colorless oily compound A2-4 (588 mg, 54%).

$^1$H NMR (400 MHz, DMSO-$d_6$) δ 7.44 (dd, *J* = 8.3, 5.4 Hz, 1H), 6.76 (dd, *J* = 10.8, 8.3 Hz, 1H), 4.58 (s, 2H), 3.94 (m, 2H), 3.81 (s, 3H), 2.89 (s, 2H), 1.76-1.73 (m, 2H), 1.67 - 1.62 (m, 2H), 1.41 (s, 9H).

LCMS (ESI): $[M\text{-}Boc+H]^+$ = 266.37.

Step 4: 1'-(tert-butoxycarbonyl)-6-fluoro-2H-spiro [benzofuran-3,4'-piperidine]-7-carboxylic acid (compound A2-5)

The compound A2-4 (588 mg, 1.6 mmol, 1.0 eq) was dissolved in tetrahydrofuran: water = 3:1 (18 mL). Lithium hydroxide (338 mg, 8.0 mmol, 5.0 eq) was added, and the reaction mixture was allowed to react at 50°C for 7 hours. 1 N dilute hydrochloric acid was added to the reaction system and the pH was adjusted to 5-6, then the mixture was extracted three times with ethyl acetate and the organic phase was collected. The organic phases were Combined and dried using anhydrous sodium sulfate, and was concentrated to obtain the crude white solid compound A2-5 (393 mg, 70%).

$^1$H NMR (400 MHz, DMSO-$d_6$) δ 7.36 (dd, *J* = 8.2, 5.4 Hz, 1H), 6.71 (dd, *J*= 10.5, 8.3 Hz, 1H), 4.55 (s, 2H), 3.90 (s, 2H), 2.87 (s, 2H), 1.72 (dd, *J*= 12.6, 3.7 Hz, 2H), 1.64 (d, *J* = 12.8 Hz, 2H), 1.41 (s, 9H).

LCMS (ESI): $[M\text{-}Boc+H]^+$ = 252.27.

Step 5: (S)-7-((2,6-dioxopiperidin-3-yl) carbamoyl)-6-fluoro-2H-spiro [benzofuran-3,4'-piperidine]-1'-carboxylic acid tert-butyl ester (compound A2-6)

The compound A2-5 (393 mg, 1.1 mmol, 1.0 eq) was dissolved in N,N-dimethylformamide (15 mL). Under stirring at room temperature, (S)-3-aminopiperidine-2,6-dione hydrochloride (276 mg, 1.7 mmol, 1.5 eq), N,N-diisopropylethylamine (723 mg, 5.6 mmol, 5.0 eq), and N,N,N',N'-tetramethyl-O-(7-azabenzotriazol-1-yl) hexafluorophosphorylurea (512 mg, 1.3 mmol, 1.2 eq) were added to the mixture. The reaction was allowed to proceed at room temperature for 1 hour. The reaction system was quenched with water, extracted three times with ethyl acetate, and the organic phase was washed four times with saturated sodium chloride solution. The organic phases were combined and dried using anhydrous sodium sulfate. The crude product was purified by column chromatography to afford the white solid compound A2-6 (355.0 mg, 69%).

$^1$H NMR (400 MHz, DMSO-$d_6$) δ 10.83 (s, 1H), 8.63 (d, *J*= 8.1 Hz, 1H), 7.33 (dd, *J*= 8.2, 5.4 Hz, 1H), 6.72 (dd, *J* = 10.1, 8.3 Hz, 1H), 4.72 (dd, *J* = 17.4, 8.1 Hz, 1H), 4.55 (s, 2H), 3.90 (s, 2H), 2.88 (s, 2H), 2.79 - 2.71 (m, 1H), 2.56 - 2.51 (m, 1H), 2.04 - 1.96 (m, 2H), 1.74 (t, *J* = 12.6 Hz, 2H), 1.65 (d, *J* = 13.2 Hz, 2H), 1.42 (s, 9H).

LCMS (ESI): $[M\text{-}Boc+H]^+$ = 362.37.

Step 6: (S)-N-(2,6-dioxopiperidin-3-yl)-6-fluoro-2H-spiro [benzofuran-3,4'-piperidine]-7-carboxamide (compound A2)

The compound A2-6 (353 mg, 0.8 mmol, 1.0 eq) was dissolved in dichloromethane: trifluoroacetic acid = 5:1 (18 mL) and reacted at room temperature for 1 hour. The reaction mixture was concentrated, and the crude product was purified by reverse-phase column chromatography to afford the white solid compound A2 (257.0 mg, 93%).

$^1$H NMR (400 MHz, DMSO-$d_6$) δ 10.85 (s, 1H), 8.66 (d, *J*= 8.1 Hz, 2H), 8.44 (d, *J*= 9.4 Hz, 1H), 7.21 (dd, *J* = 8.3, 5.3 Hz, 1H), 6.79 (dd, *J* = 10.0, 8.3 Hz, 1H), 4.73 (dd, *J* = 17.4, 8.1 Hz, 1H), 4.61 (s, 2H), 3.33 (d, *J* = 13.0 Hz, 2H), 3.00 (dd, *J* = 23.4, 10.9 Hz, 2H), 2.80 - 2.71 (m, 1H), 2.54 (t, *J* = 3.7 Hz, 1H), 2.03 - 1.93 (m, 4H), 1.85 (d, *J*= 14.0 Hz, 2H).
LCMS (ESI): $[M+H]^+$ = 362.25

Example 3

Synthesis of Compound A3

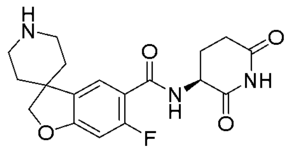

(S)-N-(2,6-dioxopiperidin-3-yl)-6-fluoro-2H-spiro[benzofuran-3,4'-piperidine]-5-carboxamide

Synthesis Scheme

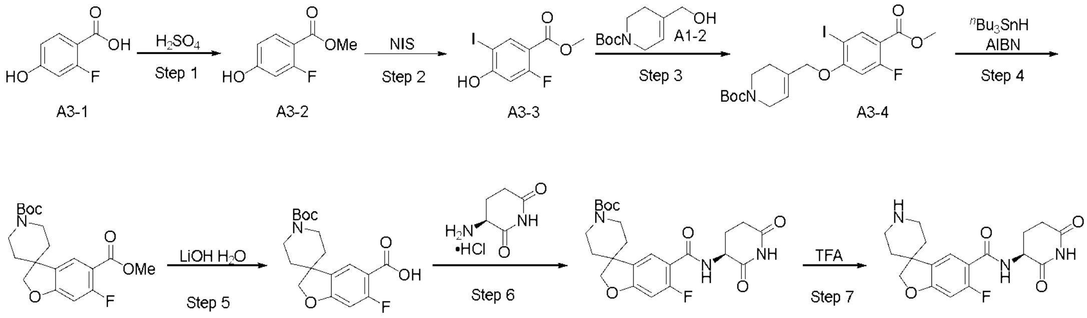


Step 1: Methyl 2-fluoro-4-hydroxybenzoate (compound A3-2)

The compound A3-1 (5.0 g, 32.0 mmol, 1.0 eq) was dissolved in 80 mL of methanol. 6 mL of concentrated sulfuric acid was added dropwise to the reaction mixture. The reaction mixture was heated to 70°C and reacted at this temperature for 24 hours. The reaction mixture was concentrated under reduced pressure. The crude product obtained was slurried with an appropriate amount of water and filtered to yield a white crystal A3-2 (5.38 g, 99%).
LCMS (ESI): $[M-H]^-$ = 169.23.

Step 2: Methyl 2-fluoro-4-hydroxy-5-iodobenzoate (compound A3-3)

The compound A3-2 (2.5 g, 14.7 mmol, 1.0 eq) and N-iodobis (acetoimidoyl) imide (3.47 g, 15.4 mmol, 1.04 eq) were dissolved in 70 mL of methanol. The reaction was carried out at room temperature for 19 hours. The reaction mixture was concentrated under reduced pressure and purified by reverse-phase column chromatography to afford the white solid compound A3-3 (566 mg, 13%).
$^1H$ NMR (400 MHz, DMSO-$d_6$) δ 11.79 (s, 1H), 8.16 (d, *J*= 8.3 Hz, 1H), 6.73 (d, *J*= 12.6 Hz, 1H), 3.80 (s, 3H).
LCMS (ESI): $[M-H]^-$ = 295.08.

Step 3: 4-((5-fluoro-2-iodo-4-(methoxycarbonyl) phenoxy) methyl)-3,6-dihydropyridine-1(2H)-carboxylic acid tert-butyl ester (compound A3-4)

The compounds A3-3 (566 mg, 1.9 mmol, 1.0 eq), A1-2 (734 mg, 3.4 mmol, 1.8 eq), and triphenylphosphine (1.5 g, 5.7 mmol, 3.0 eq) were dissolved in 30 mL of dry tetrahydrofuran. Under nitrogen at 0°C, diethyl azodicarboxylate (999 mg, 5.7 mmol, 3.0 eq) was slowly added dropwise to the system. After completion of the addition, the mixture was recovered to room temperature and reacted for 10 hours. The reaction mixture was concentrated under reduced pressure and then purified by column chromatography to afford the colorless, transparent oily liquid A3-4 (876 mg, 93%).
$^1H$ NMR (600 MHz, DMSO-$d_6$) δ 8.22 (d, *J*= 8.2 Hz, 1H), 7.10 (d, *J*= 13.0 Hz, 1H), 5.89 (s, 1H), 4.66 (s, 2H), 3.88 (s, 2H), 3.82 (s, 3H), 3.46 (s, 2H), 2.16 (s, 2H), 1.41 (s, 9H).
LCMS (ESI): $[M-Boc+H]^+$ = 392.19.

Step 4: 1'-(tert-butyl) 5-methyl-6-fluoro-2H-spiro [benzofuran-3,4'-piperidine]-1',5-dicarboxylate (compound A3-5)

The Compound A3-4 (876 mg, 1.8 mmol, 1.0 eq), tri-n-butyltin hydride (1.56 g, 5.4 mmol, 3.0 eq) and azobis(isobutyronitrile) (439 mg, 2.7 mmol, 1.5 eq) were dissolved in 30 mL of toluene. The reaction mixture was reacted at 110°C for 11 hours. After cooling the reaction system to room temperature, the mixture was concentrated under reduced pressure and purified by column chromatography to afford a colorless, transparent, oily liquid A3-5 (569 mg, 87%).

$^1$H NMR (400 MHz, DMSO-$d_6$) δ 7.73 (d, *J* = 7.4 Hz, 1H), 6.84 (d, *J* = 11.6 Hz, 1H), 4.61 (s, 2H), 3.92 (s, 2H), 3.80 (s, 3H), 2.86 (s, 2H), 1.77 (td, *J*= 12.9, 4.4 Hz, 2H), 1.66 (d, *J*= 13.0 Hz, 2H), 1.43 (s, 9H).

Step 5: 1'-(tert-butoxycarbonyl)-6-fluoro-2H-spiro [benzofuran-3,4'-piperidine]-5-carboxylic acid (compound A3-6)

The compound A3-5 (569 mg, 1.6 mmol, 1.0 eq) was dissolved in 25 mL of a mixed solution of tetrahydrofuran and water (v/v = 4/1). Lithium hydroxide monohydrate (327 mg, 7.8 mmol, 5.0 eq) was added to the mixture, and the system reacted at 50°C for 7 hours. The reaction mixture was concentrated under reduced pressure, then diluted with 50 mL of water. The pH of the system was adjusted to 5-6 using 1 M HCl. The aqueous solution was extracted three times with ethyl acetate. The combined organic phases were dried over anhydrous sodium sulfate. The filtrate was concentrated under reduced pressure to afford a pale yellow solid crude product A3-6 (617 mg). The crude product is used directly in the next reaction without further purification.

$^1$H NMR (600 MHz, Methanol-$d_4$) δ 7.68 (d, *J*= 7.2 Hz, 1H), 6.56 (d, *J*= 11.2 Hz, 1H), 4.57 (s, 2H), 4.03 (d, *J*= 13.8 Hz, 2H), 3.01 (s, 2H), 1.84 - 1.78 (m, 2H), 1.74 (d, *J* = 13.0 Hz, 2H), 1.49 (s, 9H).

LCMS (ESI): $[M-H]^-$= 350.38.

Step 6: (S)-5-((2,6-dioxopiperidin-3-yl) carbamoyl)-6-fluoro-2H-spiro [benzofuran-3,4'-piperidine]-1'-carboxylic acid tert-butyl ester (compound A3-7)

The compound A3-6 (617 mg, 1.7 mmol, 1.0 eq), (S)-3-amino-2, 6-piperidinedione hydrochloride (434 mg, 2.6 mmol, 1.5 eq) and N,N,N',N'-tetramethyl-O-(7-azabenzotriazol-1-yl) hexafluorophosphorylurea (801 mg, 2.1 mmol, 1.2 eq) were dissolved in N,N-dimethylformamide (20 mL). N,N-diisopropylethylamine (1.53 mL, 8.8 mmol, 5.0 eq) was then added, and the mixture reacted at room temperature for 15 hours. The reaction mixture was diluted with 100 mL of ethyl acetate, washed five times with saturated sodium chloride solution, and the organic phase was dried over anhydrous sodium sulfate. The filtrate was concentrated under reduced pressure, and the crude product obtained was purified by column chromatography to afford a pale blue solid A3-7 (630 mg, 78%).

$^1$H NMR (400 MHz, DMSO-$d_6$) δ 10.86 (s, 1H), 8.29 (dd, *J*= 7.9, 3.9 Hz, 1H), 7.56 (d, *J* = 7.5 Hz, 1H), 6.81 (d, *J*= 11.5 Hz, 1H), 4.80 - 4.70 (m, 1H), 4.58 (s, 2H), 3.92 (s, 2H), 2.89 (s, 2H), 2.77 - 2.73 (m, 1H), 2.56 - 2.52 (m, 1H), 2.10 (tt, *J*= 12.8, 6.5 Hz, 1H), 2.04 - 2.00 (m, 1H), 1.76 (td, *J* = 12.9, 4.3 Hz, 2H), 1.67 (d, *J*= 13.0 Hz, 2H), 1.43 (s, 9H).

LCMS (ESI): $[M-H]^-$ = 460.39.

Step 7: (S)-N-(2,6-dioxopiperidin-3-yl)-6-fluoro-2H-spiro [benzofuran-3,4'-piperidine]-5-carboxamide (compound A3)

The compound A3-7 (625 mg, 1.4 mmol, 1.0 eq) was dissolved in 10 mL of dichloromethane. 2.5 mL of trifluoroacetic acid was added to the system. The reaction proceeded at room temperature for 12 hours. The crude product obtained after concentrating the reaction system under reduced pressure was purified by reverse-phase column chromatography to afford the white solid compound 3 (65.3 mg, 13%).

$^1$H NMR (400 MHz, DMSO-$d_6$) δ 10.87 (s, 1H), 8.75 (s, 1H), 8.34 (dd, *J* = 8.0, 4.8 Hz, 1H), 7.50 (d, *J* = 7.4 Hz, 1H), 6.88 (d, *J* = 11.5 Hz, 1H), 4.80 - 4.72 (m, 1H), 4.65 (s, 2H), 3.35 (s, 2H), 3.01 (s, 2H), 2.79 (ddd, *J* = 18.6, 13.5, 5.5 Hz, 1H), 2.56 - 2.52 (m, 1H), 2.12 (qd, *J* = 12.9, 4.4 Hz, 1H), 2.06 - 1.98 (m, 3H), 1.88 (d, *J*= 14.1 Hz, 2H).
LCMS (ESI): $[M+H]^+$ = 362.39

Example 4

Synthesis of Compound A4

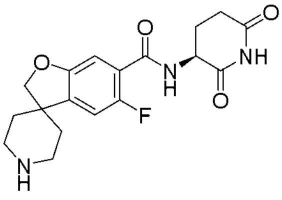

(S)-N-(2,6-dioxopiperidin-3-yl)-5-fluoro-2H-spiro[benzofuran-3,4'-piperidine]-6-carboxamide

Synthesis Scheme

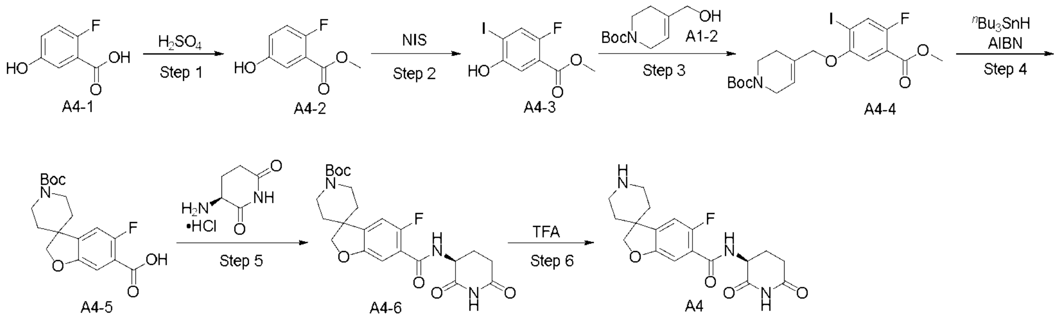


Step 1: Methyl 2-fluoro-5-hydroxybenzoate (compound A4-2)

The compound A4-1 (1.0 g, 6.4 mmol, 1.0 eq) was dissolved in 15 mL of methanol. 1 mL of concentrated sulfuric acid was added dropwise to the reaction mixture. The reaction mixture was heated to 70°C and reacted at this temperature for 17 hours. The reaction system was concentrated under reduced pressure. The crude product obtained was purified by column chromatography to afford a white solid A4-2 (992 mg, 91%).
LCMS (ESI): $[M-H]^-$ = 169.33.

Step 2: Methyl 2-fluoro-5-hydroxy-4-iodobenzoate (compound A4-3)

The compound A4-2 (955 mg, 5.6 mmol, 1.0 eq) and N-iodobis(acetoimidoyl)imide (1.33 g, 5.9 mmol, 1.05 eq) were dissolved in 30 mL of methanol. The reaction mixture was allowed to react at room temperature for 7 hours. The reaction mixture was concentrated under reduced pressure and purified by column chromatography to afford the pale yellow solid compound A4-3 (205 mg, 12%).
$^1H$ NMR (400 MHz, DMSO-$d_6$) $\delta$ 10.66 (s, 1H), 7.73 (d, *J*= 10.0 Hz, 1H), 7.31 (d, *J* = 6.3 Hz, 1H), 3.83 (s, 3H).
LCMS (ESI): $[M-H]^-$ = 294.98.

Step 3: 4-((4-fluoro-2-iodo-5-(methoxycarbonyl) phenoxy)methyl)-3,6-dihydropyridine-1(2H)-carboxylic acid tert-butyl ester (compound A4-4)

The compounds A4-3 (155 mg, 0.5 mmol, 1.0 eq), A1-2 (134 mg, 0.3 mmol, 0.6 eq), and triphenylphosphine (274 mg, 1.0 mmol, 2.0 eq) were dissolved in 15 mL of dry tetrahydrofuran. Under nitrogen at 0°C, diethyl azodicarboxylate (182 mg, 1.0 mmol, 2.0 eq) was slowly added dropwise to the system. After completion of the addition, the mixture was recovered to room temperature and reacted for 10 hours. The reaction mixture was concentrated under reduced pressure and then purified by column chromatography to afford the colorless, transparent oily liquid A4-4 (133 mg, 52%).
$^1H$ NMR (400 MHz, Methanol-$d_4$) $\delta$ 7.69 (d, *J*= 9.6 Hz, 1H), 7.36 (d, *J* = 6.0 Hz, 1H), 5.91 (s, 1H), 4.55 (s, 2H), 3.95 (s, 2H), 3.90 (s, 3H), 3.56 (t, *J*= 5.4 Hz, 2H), 2.26 (d, *J*= 1.6 Hz, 2H), 1.47 (s, 9H).
LCMS (ESI): $[M-Boc+H]^+$ = 392.19.

Step 4: 1'-(tert-butoxycarbonyl)-5-fluoro-2H-spiro [benzofuran-3,4'-piperidine]-6-carboxylic acid (compound A4-5)

The compound A4-4 (183 mg, 0.4 mmol, 1.0 eq), tri-n-butyltin hydride (325 mg, 1.1 mmol, 2.75 eq) and azobis(isobutyronitrile) (92 mg, 0.6 mmol, 1.5 eq) were dissolved in 6 mL of toluene. The reaction mixture was reacted

at 110°C for 11 hours. The reaction mixture was concentrated under reduced pressure, then diluted with 50 mL of water. The pH of the system was adjusted to 5-6 using 1 N hydrochloric acid. The resulting aqueous solution was extracted three times with ethyl acetate. The combined organic phases were dried over anhydrous sodium sulfate. The filtrate was concentrated under reduced pressure to afford a pale yellow oily liquid A4-5 (172.3 mg). The crude product is used directly in the next reaction without further purification.

$^1$H NMR (600 MHz, DMSO-$d_6$) δ 7.13 (d, *J*= 9.8 Hz, 1H), 6.95 (d, *J*= 5.6 Hz, 1H), 4.44 (s, 2H), 3.90 (s, 2H), 2.96 - 2.77 (m, 2H), 1.75 (td, *J*= 13.0, 4.4 Hz, 2H), 1.69 - 1.63 (m, 2H), 1.42 (s, 9H).

LCMS (ESI): $[M\text{-}Boc+H]^+$ = 252.27.

Step 5: (S)-6-((2,6-dioxopiperidin-3-yl)carbamoyl)-5-fluoro-2H-spiro[benzofuran-3,4'-piperidine]-1'-carboxylic acid tert-butyl ester (compound A4-6)

The compound A4-5 (172 mg, 0.5 mmol, 1.0 eq), (S)-3-amino-2,6-piperidinedione hydrochloride (121 mg, 0.7 mmol, 1.4 eq), and N,N,N',N'-tetramethyl-O-(7-azabenzotriazol-1-yl) hexafluorophosphate urea (224 mg, 0.6 mmol, 1.2 eq) were dissolved in 6 mL of N,N-dimethylformamide, followed by addition of N,N-diisopropylethylamine (0.43 mL, 2.5 mmol, 5.0 eq). The mixture reacted at room temperature for 12 hours. The reaction mixture was diluted with 50 mL ethyl acetate, washed five times with saturated sodium chloride solution, and the organic phase was dried over anhydrous sodium sulfate. The filtrate was concentrated under reduced pressure, and the crude product obtained was purified by column chromatography to afford a pale blue solid A4-6 (74 mg, 33%).

$^1$H NMR (600 MHz, DMSO-$d_6$) δ 10.85 (s, 1H), 8.46 (dd, *J*= 8.1, 3.3 Hz, 1H), 7.34 (d, *J* = 10.0 Hz, 1H), 6.97 (d, *J* = 5.4 Hz, 1H), 4.79 - 4.68 (m, 1H), 4.50 (s, 2H), 3.92 (d, *J*= 12.0 Hz, 2H), 2.89 (s, 2H), 2.83 - 2.70 (m, 1H), 2.55 (t, *J* = 3.3 Hz, 1H), 2.14 - 2.01 (m, 2H), 1.85 (ddd, *J*= 48.3, 16.6, 4.1 Hz, 2H), 1.66 (d, *J*= 13.0 Hz, 2H), 1.43 (s, 9H).

LCMS (ESI): $[M\text{-}H]^-$ = 459.79.

Step 6: (S)-N-(2,6-dioxopiperidin-3-yl)-5-fluoro-2H-spiro [benzofuran-3,4'-piperidine]-6-carboxamide (compound A4)

The compound A4-6 (74 mg, 0.16 mmol, 1.0 eq) was dissolved in 3.0 mL of dichloromethane. 1 mL of trifluoroacetic acid was added to the system, which reacted at room temperature for 4 hours. The crude product obtained after concentrating the reaction system under reduced pressure was purified by reverse-phase column chromatography to afford the white solid A4 (33.8 mg, 58%).

$^1$H NMR (400 MHz, DMSO-$d_6$) δ 10.87 (s, 1H), 8.49 (dd, *J*= 8.0, 2.3 Hz, 1H), 7.19 (d, *J* = 9.5 Hz, 1H), 7.00 (d, *J* = 5.3 Hz, 1H), 4.76 - 4.70 (m, 1H), 4.54 (s, 2H), 3.25 (d, *J* = 11.8 Hz, 2H), 2.94 - 2.88 (m, 2H), 2.82 - 2.73 (m, 1H), 2.57 - 2.52 (m, 1H), 2.08 (ddd, *J* = 25.6, 12.8, 4.4 Hz, 1H), 2.03 - 1.90 (m, 3H), 1.81 (d, *J*= 13.0 Hz, 2H).

LCMS (ESI): $[M+H]^+$ = 362.29.

Example 5

Synthesis of Compound A5

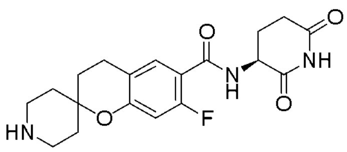

(S)-N-(2,6-dioxopiperidin-3-yl)-7-fluoro-spiro[2,4'-piperidine]-6-carboxamide

Synthesis Scheme

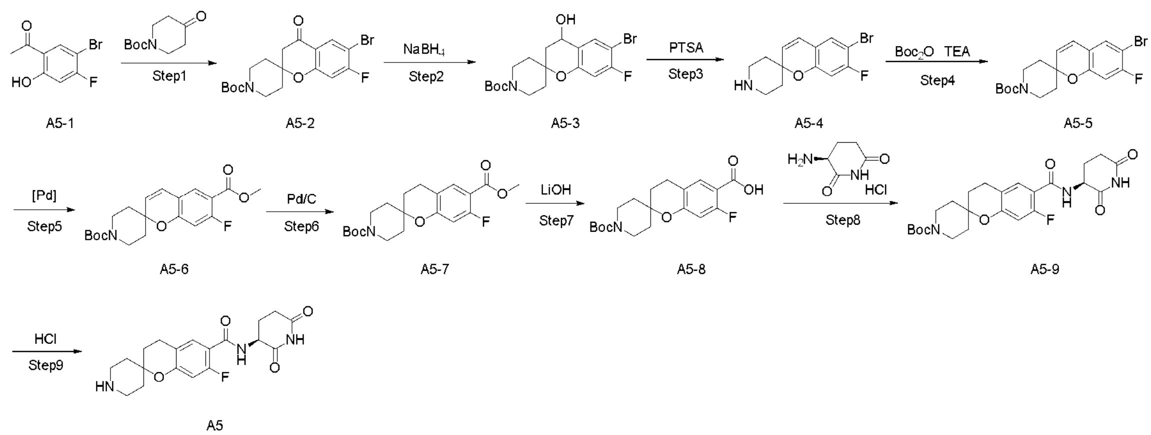


Step 1: 6-bromo-7-fluoro-4-oxo-spiro[thia-2,4'-piperidine]-1'-carboxylic acid tert-butyl ester (compound A5-2)

The compound A5-1 (10.0 g, 42.9 mmol, 1.0 eq) was dissolved in methanol (100 mL). N-tert-butoxycarbonyl-4-piperidone (8.6 g, 42.9 mmol, 1.0 eq) and tetrahydropyrole (3.1 g, 42.9 mmol, 1.0 eq) were added, and reacted at room temperature for 2 hours. The reaction mixture was concentrated under reduced pressure. The crude product obtained was purified by column chromatography to afford the pale yellow solid compound A5-2 (15.8 g, 88.8%).

$^1$H NMR (600 MHz, DMSO-$d_6$) δ 7.94 (dt, *J* = 8.0, 1.7 Hz, 1H), 7.23 (dt, *J* = 9.9, 2.3 Hz, 1H), 3.72 (d, *J* = 13.1 Hz, 2H), 3.19 - 3.09 (m, 2H), 2.88 (s, 2H), 1.90 - 1.87 (m, 2H), 1.69 - 1.58 (m, 2H), 1.40 (d, *J* = 1.6 Hz, 9H).
LCMS (ESI): $[M-Boc+H]^+$ =316.18

Step 2: 6-bromo-7-fluoro-4-hydroxy-spiro[xeno [2,4'-piperidine]-1'-carboxylic acid tert-butyl ester (compound A5-3)

The cmpound A5-2 (13.2 g, 31.9 mmol, 1.0 eq) was dissolved in methanol (150 mL). The mixture was cooled to 0°C, and sodium borohydride (1.5 g, 38.2 mmol, 1.2 eq) was slowly added. After addition, the mixture was warmed to room temperature and reacted for 2 hours. Water was added to the reaction mixture. The mixture was concentrated under reduced pressure, then it was extracted three times with ethyl acetate, washed with saturated sodium chloride solution, combined the organic phases, and drid over anhydrous sodium sulfate. The crude product A5-3 (13.0 g) was obtained from the concentrated organic phase. This crude product was used directly in the next reaction without further purification.
LCMS (ESI): $[M-Boc+H]^+$ =316.28

Step 3: 6-bromo-7-fluoro-spiro[dichloro-2,4'-piperidine] (compound A5-4)

The compound A5-3 (13.0 g, 31.2 mmol, 1.0 eq) was dissolved in toluene (150 mL). p-Toluenesulfonic acid (6.5 g, 34.4 mmol, 1.1 eq) was added, and the reaction mixture was heated to 110°C and reacted for 12 hours. After cooling the reaction system to room temperature, the mixture was concentrated under reduced pressure to afford crude product A5-4 (11.0 g). This crude product was used directly in the subsequent reaction without further purification.
LCMS (ESI): $[M+H]^+$ = 298.18.

Step 4: 6-bromo-7-fluoro-spiro [diphenylstyrene-2,4'-piperidine]-1'-carboxylic acid tert-butyl ester (compound A5-5)

The compound A5-4 (11.0 g, 34.8 mmol, 1.0 eq) was dissolved in dichloromethane (150 mL), and di-tert-butyl dicarbonate (9.1 g, 41.8 mmol, 1.2 eq) and triethylamine (10.6 g, 104.4 mmol, 3.0 eq) were added to the reaction system. The reaction mixture was allowed to react at room temperature for 2 hours. The resulting reaction system was concentrated under reduced pressure. The crude product obtained was purified by reverse-phase column chromatography to afford yellow oily compound A5-5 (10.0 g, 72.2%).

$^1$H NMR (600 MHz, DMSO-$d_6$) δ 7.45 (d, *J* = 8.0 Hz, 1H), 6.95 (d, *J* = 9.8 Hz, 1H), 6.48 (d, *J* = 9.9 Hz, 1H), 5.81 (d, *J* = 9.9 Hz, 1H), 3.69 (d, *J* = 13.0 Hz, 2H), 3.19 (brs, 2H), 1.92 - 1.73 (m, 2H), 1.63 (ddd, *J* = 13.7, 11.3, 4.7 Hz, 2H), 1.40 (s, 9H).
LCMS (ESI): $[M-Boc+H]^+$=298.08

Step 5: 1'-tert-butyl-6-methyl-7-fluoro-spiro[diphenylstyrene-2,4'-piperidine]-1',6-dicarboxylate (compound A5-6)

The compound A5-5 (10.0 g, 25.1 mmol, 1.0 eq) was dissolved in methanol (150 mL), then 1,1-bis(diphenylphosphino) dimethanediyl iron(II) dichloropalladium(II) (2.7 g, 3.8 mmol, 0.15 eq) and triethylamine (7.6 g, 75.3 mmol, 3.0 eq) were added in the system. After three exchanges of carbon monoxide gas, the reaction system was heated to 110°C and reacted for 12 hours. After cooling the reaction mixture to room temperature, it was filtered. The filter cake was washed three times with methanol (30 mL). The resulting filtrate was concentrated under reduced pressure. The crude product was purified by column chromatography to afford the transparent oily compound A5-6 (7.6 g, 80.2%).

$^1$H NMR (400 MHz, DMSO-$d_6$) δ 7.67 (d, *J*= 8.3 Hz, 1H), 6.84 (d, *J*= 12.1 Hz, 1H), 6.58 (d, *J* = 10.0 Hz, 1H), 5.83 (d, *J* = 9.9 Hz, 1H), 3.81 (s, 3H), 3.75 - 3.63 (m, 2H), 3.22 (brs, 2H), 1.86 - 1.74 (m, 2H), 1.70 - 1.53 (m, 2H), 1.41 (s, 9H).
LCMS (ESI): [M-Boc+H]$^+$ = 278.25

Step 6: 1'-tert-butyl-6-methyl-7-fluoro-spiro [pyrrole-2,4'-bipyridine]-1',6-dicarboxylate (compound A5-7)

The compound A5-6 (7.5 g, 19.9 mmol, 1.0 eq) was dissolved in methanol (100 mL). Palladium on carbon (394 mg) was added to the system. After three exchanges of hydrogen gas, the reaction was allowed to proceed at room temperature for 12 hours. The reaction mixture was filtered. The filter cake was washed three times with methanol. The filtrate was concentrated under reduced pressure. The crude product was purified by reverse-phase column chromatography to afford the transparent oily compound A5-7 (6.7 g, 88.9%).

$^1$H NMR (600 MHz, DMSO-$d_6$) δ 7.67 (d, *J*= 8.3 Hz, 1H), 6.74 (d, *J* = 12.2 Hz, 1H), 3.80 (s, 3H), 3.70 (d, *J*= 13.0 Hz, 2H), 3.13 (brs, 2H), 2.75 (t, *J*= 6.8 Hz, 2H), 1.83 (t, *J* = 6.8 Hz, 2H), 1.71 - 1.62 (m, 2H), 1.59 - 1.54 (m, 2H), 1.41 (s, 9H).
LCMS (ESI): [M-Boc+H]$^+$ = 280.28

Step 7: 1'-tert-butyl-6-methyl-7-fluoro-spiro [chromane-2,4'-bipyridine]-1',6-dicarboxylate (compound A5-8)

The compound A5-7 (6.6 g, 17.4 mmol, 1.0 eq) was dissolved in tetrahydrofuran (60 mL). Lithium hydroxide (3.65 g, 86.9 mmol, 5.0 eq) and water (60 mL) were added to the reaction mixture, which was allowed to react at room temperature for 10 hours. 1 N hydrochloric acid solution was added to the reaction mixture to adjust the pH to 5. The mixture was extracted three times with ethyl acetate, washed with saturated sodium chloride solution, combined the organic phases, and dried over anhydrous sodium sulfate. The organic phase was concentrated to yield the crude white solid compound A5-8 (6.0 g, 94.4%). This crude product was used directly in the next reaction without further purification.
LCMS (ESI): [M-Boc+H]$^+$ = 266.27

Step 8: (S)-6-(2,6-dioxopiperidin-3-yl) carbamoyl)-7-fluoro-spiro[chromane[2,4'-piperidine]-1'-carboxylic acid tert-butyl ester (compound A5-9)

The compound A5-8 (6.0 g, 16.4 mmol, 1.0 eq) was dissolved in N,N-dimethylformamide (60 mL). The following reagents were added sequentially to the system: (S)-3-amino-3-methylpiperidine-2,6-dione hydrochloride (4.1 g, 22.9 mmol, 1.4 eq), N,N-diisopropylethylamine (8.5 g, 65.7 mmol, 4.0 eq), and N,N,N',N'-tetramethyl-O-(7-azabenzotriazol-1-yl) hexafluorophosphorylurea (7.5 g, 19.7 mmol, 1.5 eq) to the system. The reaction was allowed to proceed at room temperature for 2 hours. The water was added to the reaction system, extracted three times with ethyl acetate, washed with saturated saline solution, combined the organic phases, and dried over anhydrous sodium sulfate.the organic phase was concentrated to obtain the crude product. The product was purified by reverse-phase column chromatography to yield the blue solid compound A5-9 (7.0 g, 89.7%).

$^1$H NMR (600 MHz, DMSO-$d_6$) δ 10.85 (s, 1H), 8.24 (dd, *J* = 8.1, 4.8 Hz, 1H), 7.96 (s, 1H), 7.52 (d, *J* = 8.5 Hz, 1H), 6.73 (d, *J* = 12.2 Hz, 1H), 4.75 (ddd, *J*= 12.9, 8.0, 5.4 Hz, 1H), 3.71 (d, *J* = 13.2 Hz, 2H), 3.15 (dd, *J* = 7.2, 4.2 Hz, 2H), 2.82 - 2.74 (m, 3H), 2.11 (qd, *J* = 12.9, 4.5 Hz, 1H), 2.01 (ddt, *J*= 12.4, 5.5, 2.8 Hz, 1H), 1.83 (t, *J*= 6.8 Hz, 2H), 1.74 - 1.62 (m, 2H), 1.56 (ddt, *J*= 13.7, 11.3, 4.6 Hz, 2H), 1.41 (s, 9H).
LCMS (ESI): [M-Boc+H]$^+$ = 376.29

Step 9: (S)-N-(2,6-dioxopiperidin-3-yl)-7-fluoro-spiro[2,4'-piperidine]-6-carboxamide (compound A5)

The compound A5-9 (7.0 g, 14.7 mmol, 1.0 eq) was added to a dioxane hydrochloride solution (50 mL) and reacted at room temperature for 10 hours. The reaction mixture was filtered, and the filter cake was dried to afford the white solid compound A5 (5.0 g, 82.5%).

$^1H$ NMR (600 MHz, DMSO-$d_6$) δ 10.85 (s, 1H), 9.03 - 9.00 (m, 1H), 8.28 (dd, *J*= 8.1, 4.5 Hz, 1H), 7.53 (d, *J* = 8.4 Hz, 1H), 6.81 (d, *J*= 12.1 Hz, 1H), 4.74 (ddd, *J* = 12.8, 8.0, 5.3 Hz, 1H), 3.20 (dt, *J*= 13.2, 3.5 Hz, 2H), 3.08 (dq, *J* = 15.9, 7.0, 3.9 Hz, 2H), 2.84 - 2.72 (m, 3H), 2.54 (q, *J*= 3.2, 2.7 Hz, 1H), 2.11 (qd, *J*= 12.9, 4.5 Hz, 1H), 2.00 (dtd, *J*= 13.0, 5.5, 2.7 Hz, 1H), 1.95 - 1.82 (m, 6H).
LCMS (ESI): $[M+H]^+$ = 376.30

Example 6

Synthesis of Compound A6

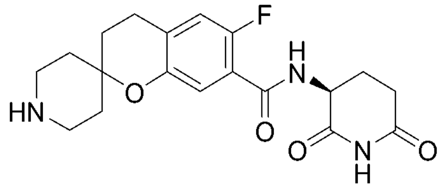

(S)-N-(2,6-dioxopiperidin-3-yl)-6-fluorospiro[chroman -2,4'-piperidine]-7-carboxamide

Synthesis Scheme

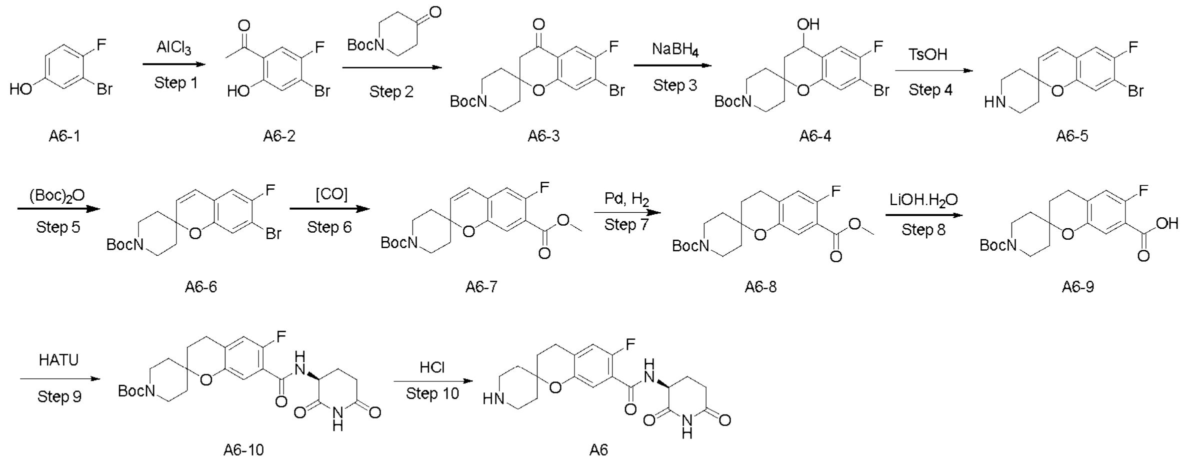


Step 1: 1-(4-bromo-5-fluoro-2-hydroxyphenyl)ethan-1-one (compound A6-2)

The compound A6-1 (20.0 g, 104.7 mmol, 1.0 eq) was dissolved in acyl chloride (41.4 g, 523.6 mmol, 5.0 eq). The reaction mixture was heated to 60°C and reacted at this temperature for 1.5 hours. After the reaction system was cooled to 0°C, aluminum trichloride (21 g, 157.1 mmol, 1.5 eq) was added to the system. The system was heated to 120°C and reacted for 4 hours. After the reaction system was cooled to room temperature, water was added and extracted three times with ethyl acetate. The system was washed with saturated saline solution, combined organic phases, and dried over anhydrous sodium sulfate. The organic phase was concentrated to obtain the crude product. The crude product was purified by column chromatography to afford yellow solid compound A6-2 (20.0 g, 91.9%).
$^1H$ NMR (600 MHz, Chloroform-d) δ 12.02 (d, *J*= 1.2 Hz, 1H), 7.48 - 7.42 (m, 1H), 7.25 - 7.20 (m, 1H), 2.65 - 2.59 (m, 3H).
LCMS (ESI): $[M+Na]^+$ = 255.27.

Step 2: 7-bromo-6-fluoro-4-oxo-spiro [chroman -2,4'-piperidine]-1'-carboxylic acid tert-butyl ester (compound A6-3)

The compound A6-2 (10 g, 42.9 mmol, 1.0 eq), 4-oxopiperidine-1-carboxylic acid tert-butyl ester (8.55 g, 42.9 mmol, 1.0 eq), and tetrahydropyrole (3.05 g, 42.9 mmol, 1.0 eq) were dissolved in methanol (150 mL). The mixture was

heated to 50°C and reacted for 2 hours. After the reaction system was cooled to room temperature and concentrated under reduced pressure, the crude product obtained was purified by column chromatography to yield yellow solid compound A6-3 (14.0 g, 78.75%).

$^1$H NMR (400 MHz, Chloroform-d) δ 7.58 (d, *J* = 7.9 Hz, 1H), 7.29 (d, *J* = 5.4 Hz, 1H), 3.90 (s, 2H), 3.17 - 3.12 (t, *J* = 12.4 Hz, 2H), 2.73 (s, 2H), 2.09 - 1.97 (m, 2H), 1.69 - 1.57 (m, 2H), 1.48 (s, 9H).

LCMS (ESI): $[M\text{-}Boc+H]^+$ = 314.08.

Step 3: 7-bromo-6-fluoro-4-hydroxy-spiro [chroman -2,4'-piperidine]-1'-carboxylic acid tert-butyl ester (compound A6-4)

The compound A6-3 (400 mg, 1.0 mmol, 1.0 eq) was dissolved in methanol (4 mL). The reaction mixture was cooled to 0°C, and sodium borohydride (54 mg, 1.5 mmol, 1.5 eq) was added to the system. The system reacted overnight at room temperature. After concentration under reduced pressure, water was added, and the mixture was extracted three times with ethyl acetate, washed with saturated saline solution, combined organic phases, and dried over anhydrous sodium sulfate. The organic phase was concentrated to obtain the crude white solid compound A6-4.

LCMS (ESI): $[M\text{-}Boc+H]^+$ = 316.18.

Step 4: 7-bromo-6-fluoro-spiro[diphenyl-2,4'-piperidine] (compound A6-5)

The compound A6-4 (45.0 g, 108.1 mmol, 1.0 eq) and p-toluenesulfonic acid (20.5 g, 118.9 mmol, 1.1 eq) were dissolved in toluene (400 mL). The mixture was heated to 110°C and reacted overnight. After the reaction mixture was cooled to room temperature, it was concentrated under reduced pressure to afford the crude compound A6-5 (5.0 g).

LCMS (ESI): $[M+H]^+$ = 298.18.

Step 5: 7-bromo-6-fluoro-spiro [diphenylstyrene-2,4'-piperidine]-1'-carboxylic acid tert-butyl ester (compound A6-6)

The compound A6-5 (5.0 g, 16.7 mmol, 1.0 eq) was dissolved in dichloromethane (50 mL). The reaction mixture was cooled to 0°C, and triethylamine (5.06 g, 50.0 mmol, 3.0 eq) and di-tert-butyl dicarbonate (4.36 g, 20.0 mmol, 1.2 eq) were added to the system. The reaction mixture was allowed to react overnight at room temperature. After concentrating the reaction mixture under reduced pressure, the crude product obtained was purified by column chromatography to afford the white solid compound A6-6 (1.8 g, 27.0%).

LCMS (ESI): $[M\text{-}Boc+H]^+$ = 300.18.

Step 6: 1'-tert-butyl-7-methyl-6-fluoro-spiro [diphenylstyrene-2,4'-piperidine]-1',7-dicarboxylate (compound A6-7)

The compound A6-6 (1.8 g, 4.5 mmol, 1.0 eq), 1,1-Bis(diphenylphosphino) dimethanedioic acid iron(II) dichloropalladium(II) (493 mg, 0.7 mmol, 0.15 eq), and triethylamine (1.37 g, 13.5 mmol, 3.0 eq) were dissolved in methanol (30 mL). After three displacements of carbon monoxide, the system was heated to 70°C and reacted overnight. The system was cooled to room temperature, the reaction mixture was concentrated under reduced pressure. The crude product obtained was purified by column chromatography to yield yellow oily compound A6-7 (900 mg, 52.75%).

$^1$H NMR (600 MHz, Chloroform-*d*) δ 7.35 - 7.40 (m, 1H), 6.75 - 6.81 (m, 1H), 6.40 - 6.34 (m, 1H), 5.77 - 5.71 (m, 1H), 3.93 - 3.91 (m, 5H), 3.32 - 3.28 (m, 2H), 1.98 (d, *J*= 13.8 Hz, 2H), 1.66 - 1.60 (m, 2H), 1.49 (s, 9H).

LCMS (ESI): $[M\text{-}Boc+H]^+$ = 278.18.

Step 7: 1'-tert-butyl-7-methyl-6-fluoro-spiro [chroman -2,4'-piperidine]-1',7-dicarboxylate (compounds A6-8)

The compound A6-7 (4.1 g, 10.9 mmol, 1.0 eq) was dissolved in methanol (40 mL). Palladium on carbon (900 mg) was added to the system. After three displacements of hydrogen gas, the reaction was allowed to proceed at room temperature for 12 hours. The reaction mixture was filtered. The filter cake was washed with methanol (10 mL) three times, and it was concentrated under reduced pressure to obtain the crude compound A6-8 (4 g).

$^1$H NMR (600 MHz, Chloroform-*d*) δ 7.39 (d, *J* = 6.2 Hz, 1H), 6.85 (d, *J*= 10.7 Hz, 1H), 3.91 (s, 4H), 3.20 (s, 2H), 2.85 - 2.77 (m, 2H), 1.86 - 1.70 (m, 5H), 1.58 - 1.50 (m, 2H), 1.48 (s, 9H).

LCMS (ESI): $[M\text{-}Boc+H]^+$ = 280.38.

Step 8: 1-tert-butoxycarbonyl-6-fluoro-spiro [chroman -2,4'-piperidine]-7-carboxylic acid (compound A6-9)

The compound A6-8 (800 mg, 2.1 mmol, 1.0 eq) and lithium hydroxide monohydrate (442 mg, 10.5 mmol, 5.0 eq) were dissolved in methanol (10 mL), tetrahydrofuran (2 mL), and water (2 mL). The system was reacted overnight at room

temperature. After concentration under reduced pressure, water was added, followed by extraction with ethyl acetate three times. The extract was washed with saturated saline solution, and the combined organic phases were dried over anhydrous sodium sulfate. The organic phase was concentrated to yield the crude white solid compound A6-9 (770 mg).
LCMS (ESI): $[M-Boc+H]^+$ = 266.27.

Step 9: (S)-7-(2,6-dioxopiperidin-3-yl) carbamoyl)-6-fluoro-spiro [chroman-2,4'-piperidine]-1'-carboxylic acid tert-butyl ester (compound A6-10)

The compound A6-9 (770 mg, 3.1 mmol, 1.0 eq), 2-(7-azobenzotriazole)-N,N,N',N'-tetramethylurea hexafluorophosphate (451 mg, 2.5 mmol, 0.8 eq), and N,N-diisopropyl ethylamine (462 mg, 6.3 mmol, 2.0 eq) were dissolved in N,N-dimethylformamide (10 mL). The reaction mixture was allowed to react at room temperature for 15 minutes. (S)-3-Aminopiperidine-2,6-dione hydrochloride (881 mg, 2.3 mmol, 0.7 eq) was added to the system. The mixture was allowed to react overnight at room temperature, then water was added and extracted three times with ethyl acetate. The mixture was washed with saturated saline solution, combined organic phases, and dried over anhydrous sodium sulfate. The organic phase was concentrated to obtain the crude product. The crude product was purified by column chromatography to afford the blue solid compound A6-10 (850 mg, 84.83%).
LCMS (ESI): $[M-Boc+H]^+$= 376.49.

Step 10: (S)-N-(2,6-dioxopiperidin-3-yl)-6-fluorohelix[chroman-2,4'-piperidine]-7-carboxamide (compound A6)

The compound A6-10 (3.0 g, 6.3 mmol, 1.0 eq) was dissolved in a hydrochloric acid solution of 1,4-dioxane (10 mL). The reaction mixture was allowed to react overnight at room temperature. The reaction mixture was filtered. The filter cake was washed three times with acetonitrile (10 mL) to obtain the white solid compound A6 (1.75 g, 73.89%).
$^1H$ NMR (600 MHz, DMSO-*d*6) δ 10.86 (s, 1H), 8.52 - 8.40 (m, 1H), 7.16 - 7.12 (m, 1H), 7.11 - 7.07 (m, 1H), 4.78 - 4.70 (m, 1H), 3.20 - 3.12 (m, 2H), 3.11 - 3.04 (m, 2H), 2.83 - 2.72 (m, 3H), 2.57 - 2.51 (m, 1H), 2.16 - 2.05 (m, 1H), 2.03 - 1.96 (m, 1H), 1.94 - 1.86 (m, 5H), 1.85 - 1.80 (m, 2H).
LCMS (ESI): $[M+H]^+$ = 376.39.

Example 7

Synthesis of Compound A7

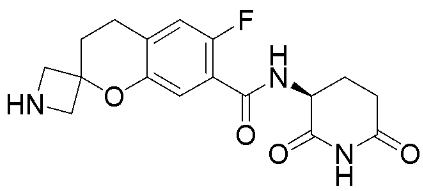

(S)-N-(2,6-dioxopiperidin-3-yl)-6-fluoro-spiroazetidine-3,2-dihydropyridine-7-carboxamide

Synthesis Scheme

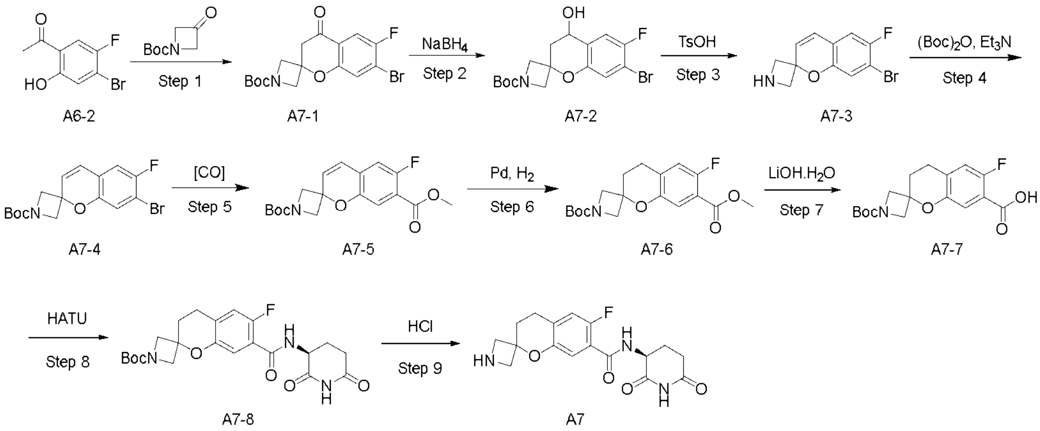


Step 1: 7'-bromo-6'-fluoro-4'-oxo-spiro [azetidine-3,2'-chromane]-1-carboxylic acid tert-butyl ester (compound A7-1)

The compound A6-2 (10.0 g, 42.9 mmol, 1.0 eq), tert-butyl 3-oxoazetidine-1-carboxylate (7.35 g, 42.9 mmol, 1.0 eq), and tetrahydropyrole (3.05 g, 42.9 mmol, 1.0 eq) were dissolved in methanol (150 mL). The mixture was heated to 70°C and reacted for 2 hours. After the reaction system was cooled to room temperature, the mixture was concentrated under reduced pressure. The crude product obtained was purified by column chromatography to afford yellow solid compound A7-1 (8.0 g, 48.27%).

$^1$H NMR (400 MHz, Chloroform-d) δ 7.59 (d, *J* = 7.8 Hz, 1H), 7.36 (d, *J*= 5.3 Hz, 1H), 4.08 (d, *J* = 9.6 Hz, 2H), 3.97 (d, *J*= 9.6 Hz, 2H), 3.05 (s, 2H), 1.46 (s, 9H).

LCMS (ESI): $[M\text{-}Boc+H]^+$ = 286.08.

Step 2: 7'-bromo-6'-fluoro-4'-hydroxy-spiro [azetidine-3,2'-chroman]-1-carboxylic acid tert-butyl ester (compound A7-2)

The compound A7-1 (5.0 g, 12.1 mmol, 1.0 eq) was dissolved in methanol (50 mL). The reaction mixture was cooled to 0°C, and sodium borohydride (654 mg, 18.1 mmol, 1.5 eq) was added to the system. The system reacted overnight at room temperature. After concentration under reduced pressure, water was added, followed by extraction with ethyl acetate three times. The extract was washed with saturated saline solution, and the combined organic phases were dried over anhydrous sodium sulfate. The organic phase was concentrated to yield the crude white solid compound A7-2 (4.5 g).

LCMS (ESI): $[M\text{-}Boc+H]^+$ = 288.28.

Step 3: 7-bromo-6-fluoro-spiro[azetidine-3,2'-diphenylstyrene] (compound A7-3)

The compound A7-2 (4.5 g, 10.8 mmol, 1.0 eq) and p-toluenesulfonic acid (2.05 g, 11.9 mmol, 1.1 eq) were dissolved in toluene (40 mL). The mixture was heated to 110°C and reacted overnight. After the reaction system was cooled to room temperature, the mixture was concentrated under reduced pressure to afford the crude compound A7-3 (4.0 g).

LCMS (ESI): $[M+H]^+$ = 270.17.

Step 4: 7'-bromo-6'-fluoro-spiro [azetidine-3,2'-benzopyridine]-1-carboxylic acid tert-butyl ester (compound A7-4)

The compound A7-3 (4.0 g, 13.3 mmol, 1.0 eq) was dissolved in dichloromethane (40 mL). The reaction mixture was cooled to 0°C, and triethylamine (4.05 g, 39.9 mmol, 3.0 eq) and di-tert-butyl dicarbonate (3.49 g, 16.0 mmol, 1.2 eq) were added to the system. The reaction mixture was reacted overnight at room temperature. After concentrating the reaction mixture under reduced pressure, the crude product obtained was purified by column chromatography to yield the white solid compound A7-4 (2.7 g, 69.36%).

LCMS (ESI): $[M\text{-}Boc+H]^+$ = 270.17.

Step 5: 1-tert-butyl 1'-methyl-6-fluoro-spiro [azetidine-3,2'-dimethylbenzene]-1,7'-dicarboxylate (compound A7-5)

The compound A7-4 (3.7 g, 9.2 mmol, 1.0 eq), 1,1-Bis(diphenylphosphino) dimethanedioic acid iron(II) dichloropalladium(II) (1.01 g, 1.4 mmol, 0.15 eq), and triethylamine (2.81 g, 27.7 mmol, 3.0 eq) were dissolved in

methanol (40 mL). After three displacements of carbon monoxide, the system was heated to 70°C and reacted overnight. After cooling the reaction system to room temperature, the mixture was concentrated under reduced pressure. The crude product obtained was purified by column chromatography to yield yellow oily compound A7-5 (1.8 g, 51.32%).

$^1H$ NMR (600 MHz, Chloroform-d) δ 7.42 - 7.38 (m, 1H), 6.79 (d, *J* = 10.2 Hz, 1H), 6.45 (d, *J* = 9.8 Hz, 1H), 6.13 (d, *J* = 9.8 Hz, 1H), 4.26- 4.21 (m, 2H), 4.03 - 3.98 (m, 2H), 3.92 (s, 3H), 1.47 (s, 9H).

LCMS (ESI): $[M\text{-}tBu+H]^+$ = 294.28.

Step 6: 1-tert-butyl-1'-methyl-6'-fluoro-spiro [azetidine-3,2'-chromane]-1,7'-dicarboxylate (compound A7-6)

The compound A7-5 (1.8 g, 4.8 mmol, 1.0 eq) was dissolved in methanol (20 mL). Palladium on carbon (360 mg) was added to the system. After three displacements of hydrogen gas, the reaction was allowed to proceed at room temperature for 12 hours. The reaction mixture was filtered. The filter cake was washed with methanol (10 mL) three times, and it was concentrated under reduced pressure to obtain the crude compound A7-6 (1.8 g).

$^1H$ NMR (600 MHz, Chloroform-d) δ 7.42 (d, *J*= 6.2 Hz, 1H), 6.91 - 6.80 (m, 1H), 4.04 (d, *J* = 9.4 Hz, 2H), 3.96 - 3.95 (m, 5H), 2.90 - 2.82 (m, 2H), 2.18 - 2.09 (m, 2H), 1.47 (s, 9H).

LCMS (ESI): $[M\text{-}Boc+H]^+$ = 252.37.

Step 7: 1-tert-butoxycarbonyl-6'-fluoro-spiro [azetidine-3,2'-dione]-7'-carboxylic acid (compound A7-7)

The compound A7-6 (1.8 g, 4.7 mmol, 1.0 eq) and lithium hydroxide monohydrate (995 mg, 23.7 mmol, 5.0 eq) were dissolved in methanol (20 mL) and water (4 mL). The system was reacted overnight at room temperature. After concentration under reduced pressure, water was added, and the mixture was extracted three times with ethyl acetate. The system was washed with saturated saline solution, combined organic phases, and dried over anhydrous sodium sulfate. The organic phase was concentrated to obtain the crude white solid compound A7-7 (1.7 g).

$^1H$ NMR (600 MHz, Chloroform-d) δ 7.49 (d, *J* = 6.3 Hz, 1H), 6.87 (d, *J*= 10.6 Hz, 1H), 4.05 (d, *J= 9.3* Hz, 2H), 3.91 (d, *J* = 9.3 Hz, 2H), 2.91 - 2.81 (m, 2H), 2.14 (d, *J* = 7.0 Hz, 2H), 1.47 (s, 9H).

LCMS (ESI): $[M\text{-}Boc+H]^+$ = 238.26.

Step 8: (S)-7'-((2,6-dioxopiperidin-3-yl) carbamoyl)-6-fluoro-spiro[azetidin-3,2'-pyrrolo[1,2-a]pyrrole]-1-carboxylic acid tert-butyl ester (compound A7-8)

The compound A7-7 (1.7 g, 4.7 mmol, 1.0 eq), 2-(7-azobenzotriazole)-N,N,N',N'-tetramethylurea hexafluorophosphate (1.95 g, 5.1 mmol, 1.1 eq) and N,N-diisopropylethylamine (1.02 g, 14.0 mmol, 3.0 eq) were dissolved in N,N-dimethylformamide (10 mL). The reaction mixture was allowed to react at room temperature for 15 minutes. (S)-3-Aminopiperidine-2,6-dione hydrochloride (997 mg, 5.6 mmol, 1.2 eq) was added to the system. The system reacted overnight at room temperature, was added to water, and extracted three times with ethyl acetate. It was washed with saturated brine (20 mL), the organic phases were combined, and dried over anhydrous sodium sulfate. The crude product obtained from the concentrated organic phase was purified by column chromatography to afford the blue solid compound A7-8 (1.5 g, 67.80%).

$^1H$ NMR (600 MHz, Chloroform-d) δ 10.86 (s, 1H), 8.48 - 8.42 (m, 1H), 7.96 (s, 1H), 7.12 - 7.07 (m, 2H), 4.78 - 4.69 (m, 1H), 3.97 - 3.92 (m, 2H), 3.83 (d, *J* = 9.2 Hz, 2H), 2.75 - 2.86 (m, 2H), 2.57 - 2.50 (m, 1H), 2.14 - 2.04 (m, 3H), 2.04 - 1.97 (m, 1H), 1.40 (s, 9H).

LCMS (ESI): $[M\text{-}Boc+H]^+$= 348.39.

Step 9: (S)-N-(2,6-dioxopiperidin-3-yl)-6-fluoro-spiroazetidine-3,2-dihydropyridine-7-carboxamide (compound A7)

The compound A7-8 (50 mg, 0.1 mmol, 1.0 eq) was dissolved in hydrochloric acid solution of 1,4-dioxane (2 mL, 4 N). The reaction mixture was allowed to react overnight at room temperature. The reaction mixture was filtered. The filter cake was washed three times with acetonitrile (2 mL) to obtain white solid compound A7 (13.0 mg, 33.49%).

$^1H$ NMR (600 MHz, DMSO-*d*6) δ 10.87 (s, 1H), 9.64 (brs, 1H), 8.52 - 8.46 (m, 1H), 7.16 - 7.10 (m, 2H), 4.79 - 4.69 (m, 1H), 4.06 (d, *J*= 11.4 Hz, 2H), 4.02 (d, *J* = 11.3 Hz, 2H), 2.88 - 2.82 (m, 2H), 2.82 - 2.73 (m, 1H), 2.57 - 2.50 (m, 1H), 2.21 (t, *J* = 6.5 Hz, 2H), 2.15 - 2.05 (m, 1H), 2.03 - 1.96 (m, 1H).

LCMS (ESI): $[M+H]^+$ = 348.39.

Example 8

Synthesis of Compound A8

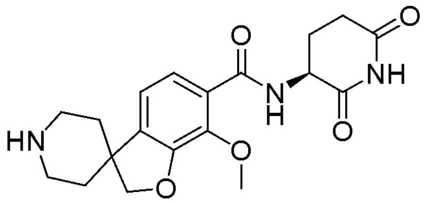

(S)-N-(2,6-dioxohesperidin-3-yl)-7-methoxy-2H-spiro[benzofuran-3,4'-piperidine]-6-carboxamide

Synthesis Scheme

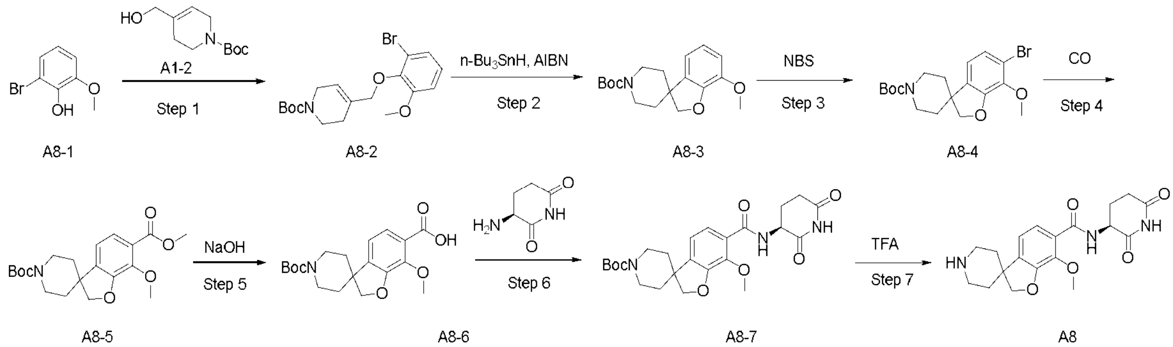


Step 1: 4-((2-bromo-6-methoxyphenoxy)methyl) -3,6-dihydropyridine-1(2H)-carboxylic acid tert-butyl ester (compound A8-2)

The compound A8-1 (3.0 g, 14.78 mmol, 1.0 eq), A1-2 (3.78 g, 17.73 mmol, 1.2 eq), and triphenylphosphine (7.75 g, 29.55 mmol, 2 eq) were dissolved in tetrahydrofuran (60 mL). The mixture was cooled to 0°C before adding diethyl azodicarboxylate (5.56 g, 28.38 mmol, 2.5 eq). After completing the dropwise addition, the mixture was warmed to room temperature and reacted for 12 hours. The reaction mixture was concentrated under reduced pressure. Water (50 mL) was added to the system, extracted with ethyl acetate (100 mL × 3 times), washed with saturated sodium chloride solution (100 mL), combined the organic layers, and dried over anhydrous sodium sulfate. The crude product obtained from the concentratedorganic phase was purified by reverse-phase column chromatography to afford the white solid compound A8-2 (5.50 g, 93.46%).

LCMS (ESI): $[M\text{-}Boc+H]^+$ = 298.38.

Step 2: 7-methoxy-2H-spiro[benzofuran-3,4'-piperidine]-1'-carboxylic acid tert-butyl ester (compound A8-3)

The compound A8-2 (1.0 g, 2.51 mmol, 1.0 eq), tri-n-butyltin hydride (1.10 g, 3.77 mmol, 1.5 eq), and azobisisobutyronitrile (412 mg, 2.51 mmol, 1.0 eq) were dissolved in toluene (200 mL) and reacted at 110°C for 12 hours under nitrogen protection. After cooling the reaction system to room temperature, concentrated under reduced pressure, then water (100 mL) was added . The system was extracted with ethyl acetate (100 mL × 3 times), washed with saturated saline solution (100 mL), combined the organic phases, and dried with anhydrous sodium sulfate. The crude product obtained from the concentrated organic phase was purified by column chromatography to afford the yellow solid compound A8-3 (800 mg, 99.7%).

LCMS (ESI): $[M\text{-}Boc+H]^+$ = 220.36.

Step 3: 6-bromo-7-methoxy-2H-spiro [benzofuran-3,4'-piperidine]-1'-carboxylic acid tert-butyl ester (compound A8-4)

The compound A8-3 (500 mg, 1.57 mmol, 1.0 eq) was dissolved in acetonitrile (50 mL). N-bromosuccinimide (306.49 g, 1.72 mmol, 1.1 eq) and thiourea (11.92 mg, 156.54 μmol, 0.1 eq) were added. The reaction mixture was reacted

at room temperature for 1 hour. The resulting reaction system was concentrated under reduced pressure. The crude product obtained was purified by column chromatography to afford the pale yellow solid compound A8-4 (600 mg, 96.23%).
LCMS (ESI): $[M\text{-}Boc+H]^+$ = 298.28

Step 4: 1'-(tert-Butyl)6-methyl-7-methoxy-2H-spiro [benzofuran-3,4'-piperidine]-1',6-dicarboxylate (compound A8-5)

The compound A8-4 (500 mg, 1.26 mmol, 1.0 eq) was dissolved in methanol (10 mL). [1,1'-Bis(diphenylphosphino)ferrocene]palladium(II) dichloride (91.8 mg, 0.13 mmol, 0.1 eq), triethylamine (381 mg, 3.77 mmol, 3 eq) were added in sequence. The mixture was reacted at 80°C under a carbon monoxide atmosphere for 16 hours. The reaction mixture was concentrated under reduced pressure. The crude product obtained was purified by column chromatography to afford the white solid compound A8-5 (220 mg, 46.43%).
LCMS (ESI) $[M\text{-}Boc+H]^+$ = 278.37.

Step 5: 1'-(tert-Butoxycarbonyl)-7-methoxy-2H-spiro [benzofuran-3,4'-piperidine]-6-carboxylic acid (compound A8-6)

The compound A8-5 (200 mg, 529.89 μmol, 1.0 eq) was dissolved in a 3:1:1 mixture of tetrahydrofuran, methanol, and water (2 mL). Sodium hydroxide (63.89 mg, 1.59 mmol, 3 eq) was added to the mixture, and the system reacted at room temperature for 2 hours. Acetic acid was added to adjust the pH to 7. The reaction mixture was concentrated under reduced pressure. The crude product obtained was purified by reverse-phase column chromatography to afford the white solid compound A8-6 (170 mg, 88.28%).
LCMS (ESI) $[M\text{-}Boc+H]^+$ = 264.37.

Step 6: (S)-6-((2,6-dioxohesperidin-3-yl) carbamoyl)-7-methoxy-2H-spiro[benzofuran-3,4'-piperidine]-1'-carboxylic acid tert-butyl ester (compound A8-7)

The compound A8-6 (160 mg, 440.27 μmol, 1.0 eq), (S)-3-aminopiperidine-2,6-dione (440.27 μmol, 1.0 eq), and EDCI (162 mg, 880.55 μmol, 2.0 eq) and HOBT (119 mg, 880.55 μmol, 2.0 eq) were dissolved in DMF (5 mL). DIEA (227.62 mg, 1.76 μmol, 4.0 eq) was added to the reaction system and reacted at room temperature for 2 hours. The reaction mixture was concentrated under reduced pressure. The crude product obtained was purified by reverse-phase column chromatography to afford the white solid compound A8-7 (200 mg, 95.93%).
LCMS (ESI) $[M-56]^+$ = 418.39

Step 7: (S)-N-(2,6-dioxohesperidin-3-yl)-7-methoxy-2H-spiro [benzofuran-3,4'-piperidine]-6-carboxamide (compound A8)

The compound A8-7 (500 mg, 1.12 mmol, 1.0 eq) was dissolved in dichloromethane (25 mL). Trifluoroacetic acid (5 mL) was added to the system. The reaction proceeded at room temperature for 1 hour. The reaction mixture was concentrated under reduced pressure. The crude product obtained was purified by reverse-phase column chromatography to afford the white solid compound A8 (268 mg, 67.97%).

> $^1H$ NMR (400 MHz, $CD_3OD$) δ 7.48 - 7.43 (m, 2H), 4.87 (dd, *J*= 10.0, 8.1 Hz, 1H), 4.65 (s, 2H), 3.93 (s, 3H), 3.54 - 3.44 (m, 2H), 3.18 (td, *J*= 13.0, 3.4 Hz, 2H), 2.86 (ddd, *J* = 17.6, 10.6, 8.8 Hz, 1H), 2.74 (dt, *J*= 17.6, 3.8 Hz, 1H), 2.28 - 2.10 (m, 4H), 2.08 - 1.98 (m, 2H).
> LCMS (ESI) $[M+H]^+$= 356.39

Example 9

Synthesis of Compound A9

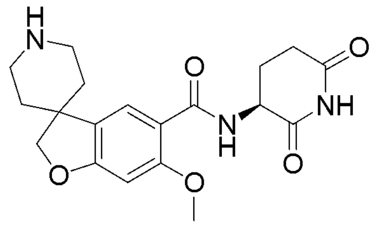

(S)-N-(2,6-dioxopiperidin-3-yl)-6-methoxy-2H-spiro[benzofuran-3,4'-piperidine]-5-carboxamide

Synthesis Scheme

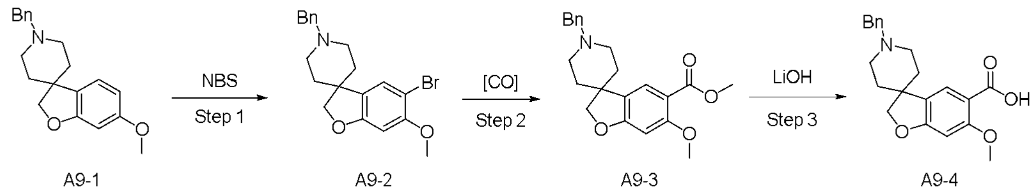


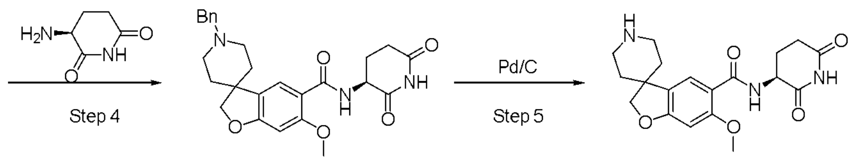


A9-5 A9

Step 1: 1'-benzyl-5-bromo-6-methoxy-2H-spiro [benzofuran-3,4'-piperidine] (compound A9-2)

The compound A9-1 (2.9 g, 9.370 mmol, 1.0 eq) was dissolved in acetonitrile (30 mL). N-bromosuccinimide (1.67 g, 9.370 mmol, 1.0 eq) and thiourea (71.34 mg, 937.3 μmol, 0.1 eq) were added. The mixture was reacted at room temperature for 2 hours. The resulting reaction mixture was concentrated under reduced pressure. The crude product was purified by forward column chromatography to afford pale yellow oily compound A9-2 (2.58 g, 70.89%).

1H NMR (600 MHz, Methanol-d4) δ 7.40 - 7.34 (m, 5H), 7.32 - 7.28 (m, 1H), 7.26 (s, 1H), 6.52 (s, 1H), 4.42 (s, 2H), 3.82 (s, 3H), 3.60 (s, 2H), 2.92 (dt, J = 12.5, 3.7 Hz, 2H), 2.15 (t, J = 12.1 Hz, 2H), 1.94 (td, J = 13.0, 4.0 Hz, 2H), 1.74 - 1.69 (m, 2H).

LCMS (ESI): $[M+H]^+$ = 389.39.

Step 2: methyl 1'-benzyl-6-methoxy-2H-spiro [benzofuran-3,4'-piperidine]-5-carboxylate (compound A9-3)

The compound A9-2 (2.58 g, 6.64 mmol, 1.0 eq), 1,1-Bis(diphenylphosphino)dimethanedioic acid iron(II) dichloropalladium(II) (538.58 mg, 664.43 μmol, 0.1 eq), and triethylamine (2.02 g, 19.93 mmol, 3.0 eq) were dissolved in methanol (30 mL), heated to 70°C under a carbon monoxide atmosphere, and reacted for 12 hours. The reaction mixture was concentrated under reduced pressure. Water (50 mL) was added to the system, extracted with ethyl acetate (100 mL × 3 times), and washed with saturated sodium chloride solution (100 mL). The organic layers was combined and dried over anhydrous sodium sulfate. The crude product obtained from the concentrated organic phase was purified by reverse-phase column chromatography to afford the yellow oily compound A9-3 (1.69 g, 69.22%).

LCMS (ESI): $[M+H]^+$ = 368.39.

Step 3: 1'-benzyl-6-methoxy-2H-spiro [benzofuran-3,4'-piperidine]-5-carboxylic acid (compound A9-4)

The compound A9-3 (1.20 g, 3.27 mmol, 1.0 eq) and lithium hydroxide (782.06 mg, 32.66 mmol, 10.0 eq) were dissolved in methanol/tetrahydrofuran/water (8 mL/8 mL/2 mL) and reacted at 50°C for 2 hours under nitrogen protection. After the reaction system was cooled to room temperature, it was concentrated under reduced pressure, then water (100

mL) was added, and was extracted with ethyl acetate (100 mL × 3 times). The resuling mixture was washed with saturated saline solution (100 mL), combined the organic phases, and dried over anhydrous sodium sulfate. The organic phase was concentrated to obtain the crude product. The crude product was purified by column chromatography to afford yellow solid compound A9-4 (1.01 g, 87.51%).

LCMS (ESI): $[M+H]^+$ = 354.29.

Step 4: (S)-1'-benzyl-N-(2,6-dioxopiperidin-3-yl)-6-methoxy-2H-spiro [benzofuran-3,4'-piperidine]-5-carboxamide (compound A9-5)

The compound A9-4 (1.01 g, 2.86 mmol, 1 eq), N,N,N',N'-tetramethyl-O-(7-azabenzotriazol-1-yl) hexafluorophosphorylurea (2.17 g, 5.72 mmol, 2.0 eq), and N, N-diisopropyl ethylamine (1.48 g, 11.43 mmol, 4.0 eq). The mixture was reacted at room temperature for 30 minutes, then (S)-3-aminopiperidine-2,6-dione (549.26 mg, 4.29 mmol, 1.5 eq) was added to the reaction mixture. After completion of addition, the mixture was reacted at room temperature for 2 hours. Water (50 mL) was added to the reaction system and extracted with dichloromethane (50 mL × 3 times). The system was washed with saturated saline solution (100 mL), combined the organic phases, and dried over anhydrous sodium sulfate. The organic phase was concentrated to obtain the crude product, and it was purified by column chromatography to yield reddish-brown oily compound A9-5 (749 mg, 56.62%).

LCMS (ESI) $[M+H]^+$ = 464.49.

Step 5: (S)-N-(2,6-dioxopiperidin-3-yl)-6-methoxy-2H-spiro [benzofuran-3,4'-piperidine]-5-carboxamide (compound A9)

LCMS (ESI) $[M+H]^+$ = 407.69.

The compound A9-5 (750 mg, 1.62 mmol, 1.0 eq) was dissolved in methanol (15 mL). Palladium(II)/carbon (10%) (172.19 mg, 161.80 μmol, 0.1 eq) was added to the system. After three displacements of hydrogen gas, the reaction was proceeded at room temperature for 12 hours. The reaction mixture was filtered. The filter cake was washed three times with methanol (15 mL each). The resulting filtrate was concentrated under reduced pressure. The crude product was purified by reverse-phase column chromatography to afford the white solid compound A9 (474 mg, 78.45%).

1H NMR (600 MHz, DMSO-d6) δ 10.90 (s, 1H), 8.53 (d, 1H), 7.74 (s, 1H), 6.66 (s, 1H), 4.64 (dd, J = 11.7, 6.2 Hz, 1H), 4.55 (s, 2H), 3.87 (s, 4H), 3.30 (d, J = 12.9 Hz, 2H), 2.95 (td, J = 13.4, 3.1 Hz, 2H), 2.73 (ddd, J = 17.3, 12.7, 6.5 Hz, 1H), 2.57 - 2.53 (m, 1H), 2.09 (dt, J = 9.4, 3.4 Hz, 2H), 2.02 - 1.94 (m, 2H), 1.83 (d, J = 14.2 Hz, 2H).
LCMS (ESI) $[M+H]^+$ = 374.39

Example 10

Synthesis of Compound A10

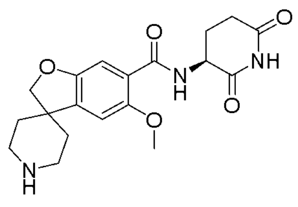

(S)-N-(2,6-dioxohesperidin-3-yl)-5-methoxy-2H-spiro[benzofuran-3,4'-piperidine]-6-carboxamide

Synthesis Scheme

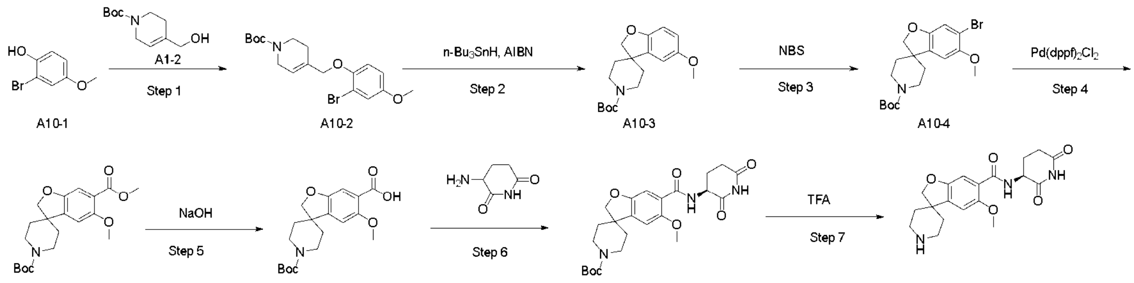

A10-1 A10-2 A10-3 A10-4 A10-5 A10-6 A10-7 A10

Step 1: 4-((2-bromo-4-methoxyphenoxy) methyl)-3,6-dihydropyridine-1(2H)-carboxylic acid tert-butyl ester (compound A10-2)

The compound A10-1 (5.0 g, 24.63 mmol, 1.0 eq), compound A1-2 (6.3 g, 29.55 mmol, 1.2 eq), and triphenylphosphine (12.9 g, 49.25 mmol, 2.50 eq) were dissolved in tetrahydrofuran (200 mL). The mixture was cooled to 0°C before adding diethyl azodicarboxylate (9.3 g, 49.25 mmol, 2.0 eq). After completion of the dropwise addition, the mixture was warmed to room temperature and reacted for 12 hours. The reaction mixture was concentrated under reduced pressure. Water (50 mL) was added to the system, extracted with ethyl acetate (100 mL × 3 times), washed with saturated sodium chloride solution (100 mL), combined the organic layers, and dried over anhydrous sodium sulfate. The crude product obtained from the concentrated organic phase was purified by forward column chromatography to afford the white solid compound A10-2 (7.3 g, 74.42%).
LCMS (ESI): $[M-100+H]^+$ = 398.0;400.0.

Step 2: 5-methoxy-2H-spiro[benzofuran-3,4'-piperidine]-1'-carboxylic acid tert-butyl ester (compound A10-3)

The compound A10-2 (7.3 g, 18.33 mmol, 1.0 eq), tri-n-butyltin hydride (11.9 g, 36.66 mmol, 2.0 eq) and azobis(isobutyronitrile) (7.5 g, 45.82 mmol, 2.5 eq) were disssolved in toluene (100 mL) and reacted at 110°C for 12 hours under nitrogen atmosphere. After the reaction system was cooled to room temperature, concentrated under reduced pressure, then water (100 mL) was added and was extracted with ethyl acetate (100 mL × 3 times). The system was washed with saturated saline solution (100 mL), combined the organic phases, and dried over anhydrous sodium sulfate. The organic phase was concentrated to obtain the crude product. The crude product was purified by column chromatography to afford yellow solid compound A10-3 (4.8 g, 82.2%).
LCMS (ESI): $[M-56+H]^+$ = 264.27.

Step 3: 6-bromo-5-methoxy-2H-pyro [benzofuran-3,4'-piperidine]-1'-carboxylic acid tert-butyl ester (compound A10-4)

The compound A10-3 (4.0 g, 12.52 mmol, 1.0 eq) was dissolved in acetonitrile (40 mL), followed by addition of N-bromosuccinimide (1.8 g, 10.02 mmol, 0.8 eq) and thiourea (95.3 mg, 1.25 mmol, 0.1 eq). The reaction mixture was reacted at room temperature for 12 hours. The resulting reaction system was concentrated under reduced pressure. The crude product obtained was purified by column chromatography to afford the pale yellow solid compound A10-4 (3.0 g, 60.14%).
1H NMR (600 MHz, DMSO-d6) δ 7.13 (s, 1H), 7.02 (s, 1H), 4.43 (s, 2H), 3.92 (d, J = 27.6 Hz, 2H), 3.78 (s, 3H), 1.80 (tt, J = 20.1, 10.2 Hz, 2H), 1.69 - 1.57 (m, 2H), 1.42 (d, J = 2.2 Hz, 11H).
LCMS (ESI) $[M+H]^+$ = 298.18;300.18.

Step 4: 1'-(tert-Butyl)6-methyl-5-methoxy-2H-spiro[benzofuran-3,4'-piperidine]-1',6-dicarboxylate (compound A10-5)

The compound A10-4 (2.6 g, 6.53 mmol, 1.0 eq) was dissolved in N,N-dimethylformamide: methanol (20:10 mL), and then the [1,1'-Bis(diphenylphosphino)ferrocene]palladium(II) dichloride (477.6 mg, 0.65 mmol, 0.1 eq) and triethylamine (2.0 g, 19.58 mmol, 3.0 eq) were added to the solution. The mixture was reacted overnight at 80°C in a carbon monoxide atmosphere. The reaction mixture was filtered, and the filtrate was concentrated under reduced pressure. The crude product obtained was purified by column chromatography to afford the pale yellow solid compound A10-5 (1.5 g, 60.88%).
LCMS (ESI) $[M-56+H]^+$ =322.28.

Step 5: 1'-(tert-butoxycarbonyl)-5-methoxy-2H-spiro[benzofuran-3,4'-piperidine]-6-carboxylic acid (compound A10-6)

The compound A10-5 (1.5 g, 3.97 mmol, 1.0 eq) was dissolved in tetrahydrofuran:methanol:water = (12 mL + 12 mL + 3 mL), and sodium hydroxide (795 mg, 19.89 mmol, 5.0 eq) was added. The reaction was carried out at room temperature for 2 hours. The reaction mixture was concentrated under reduced pressure. The crude product obtained was purified by column chromatography to afford the white solid compound A10-6 (1.2 g, 83.0%).
LCMS (ESI) $[M+H]^+$ = 364.35.

Step 6: (S)-6-((2,6-dioxyhesperidin-3-yl)aminocarbonyl)-5-methoxy-2H-spiro[benzofuran-3,4'-piperidine]-1'-carboxylic acid tert-butyl ester (compound A10-7)

The compound A10-6 (1.2 g, 3.30 mmol, 1.0 eq) was dissolved in N,N-dimethylformamide (10 mL). N,N-diisopropylethylamine (2.1 g, 16.5 mmol, 5.0 eq) was added to the system. Then 3-aminopiperidine-2,6-dione (634.0 mg, 4.95 mmol, 1.5 eq), 1-(3-dimethylaminopropyl)-3-ethylcarbodiimide hydrochloride (1.2 g, 6.6 mmol, 2.0 eq), and 1-hydroxybenzotriazole (893 mg, 6.6 mmol, 2.0 eq) were added, and the mixture was reacted at room temperature for 2 hours. The reaction mixture was concentrated under reduced pressure. The crude product obtained was purified by column chromatography to afford the white solid compound A10-7 (900 mg, 57.6%).
LCMS (ESI) $[M+H]^+$=474.49

Step 7: (S)-N-(2,6-dioxohesperidin-3-yl)-5-methoxy-2H-spiro[benzofuran-3,4'-piperidine]-6-carboxamide (compound A10)

The compound A10-7 (900 mg, 1.90 mmol, 1.0 eq) was dissolved in dichloromethane (10 mL). Trifluoroacetic acid (5 mL) was added to the system, and the mixture was reacted at room temperature for 2 hours. The reaction mixture was concentrated under reduced pressure. The crude product obtained was purified by reverse-phase column chromatography to afford the white solid compound A10 (580 mg, 81.7%).
1H NMR (600 MHz, DMSO-d6) δ 10.91 (s, 1H), 8.67 (d, J = 7.3 Hz, 1H), 7.23 (s, 1H), 6.97 (s, 1H), 4.80 - 4.68 (m, 1H), 4.51 (s, 2H), 3.90 (s, 3H), 3.35 (d, J = 12.9 Hz, 2H), 3.12 - 2.98 (m, 2H), 2.77 (dtd, J = 12.1, 9.1, 7.2 Hz, 1H), 2.54 (d, J = 3.7 Hz, 2H), 2.09 (qd, J = 9.8, 8.4, 4.2 Hz, 4H), 1.93 - 1.76 (m, 2H).
LCMS (ESI) $[M+H]^+$ =374.30

Example 11

Synthesis of Compound A11

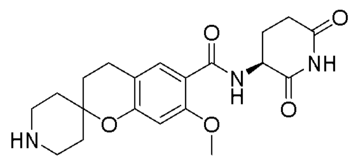

(S)-N-(2,6-dioxyhesperidin-3-yl)-7-methoxyspiro[chroman-2,4'-piperidine]-6-carboxamide

Synthesis Scheme

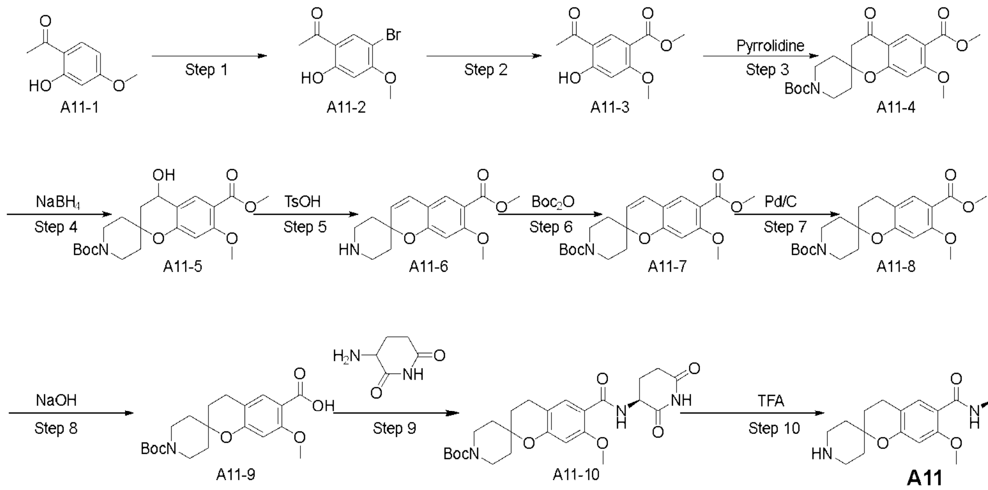


Step 1: 1-(5-bromo-2-hydroxy-4-methoxyphenyl)ethan-1-one (compound A11-2)

The compound A11-1 (10.0 g, 60.18 mmol, 1.0 eq) was dissolved in acetonitrile (300 mL), followed by addition of N-bromosuccinimide (10.65 g, 60.18 mmol, 1.0 eq) and thiourea (458.05 mg, 6.02 mmol, 0.1 eq). The reaction was carried out at room temperature for 0.5 hours. The resulting reaction mixture was concentrated under reduced pressure. The crude product obtained was washed with methanol to afford the white solid compound A11-2 (11.3 g, 76.62%).
$^1H$ NMR (600 MHz, DMSO-$d_6$) $\delta$ 12.54 (s, 1H), 8.06 (s, 1H), 6.65 (s, 1H), 3.90 (s, 3H), 2.58 (s, 3H).
LCMS (ESI): $[M+H]^+$ = 245.17.

Step 2: Methyl 5-acetyl-4-hydroxy-2-methoxybenzoate (compound A11-3)

The compound A11-2 (11.30 g, 46.11 mmol, 1.0 eq), DPPF-Pb(II) (3.37 g, 4.61 mmol, 0.3 eq), and triethylamine (14.00 g, 138.33 mmol, 3 eq) were dissolved in methanol (100 mL) and N,N-dimethylmorpholine (10.00 g, 1.0 eq). The mixture was reacted at 70°C for 12 hours. The reaction mixture was concentrated under reduced pressure. Water (50 mL) was added to the system, extracted with ethyl acetate (100 mL × 3 times, and washed with saturated sodium chloride solution (100 mL). the organic layers were combined and dried over anhydrous sodium sulfate. The crude product obtained from the concentrated organic phase was purified by forward column chromatography to afford the white solid compound A11-3 (10 g, 96.73%).
LCMS (ESI): $[M+H]^+$ = 225.26.

Step 3: 1'-(tert-butyl)6-methyl-7-methoxy-4-oxo-spiro[chroman-2,4'-piperidine]-1',6-dicarboxylate (compound A11-4)

The compound A11-3 (10.0 g, 44.60 mmol, 1.0 eq), 4-oxopiperidine-1-carboxylic acid tert-butyl ester (10.66 g, 53.52 mmol, 1.2 eq), and pyrrolidine (3.81 g, 53.52 mmol, 1.2 eq) were dissolved in methanol (200 mL) and reacted at 70°C for 4 hours. After the reaction system was cooled to room temperature, the crude product was purified by column chromatography purification to afford the white solid compound A11-4 (12.0 g, 66.36%).
$^1H$ NMR (600 MHz, DMSO-$d_6$) $\delta$ 8.12 (s, 1H), 6.80 (s, 1H), 3.89 (s, 3H), 3.77 (s, 5H), 3.29 - 3.05 (m, 2H), 2.83 (s, 2H), 1.92 - 1.85 (m, 2H), 1.65 (ddd, J = 13.8, 11.6, 4.7 Hz, 2H), 1.40 (s, 9H).
LCMS (ESI): $[M+H]^+$ = 406.39.

Step 4: 1'-(tert-butyl)6-methyl-4-hydroxy-7-methoxyspiro[chroman-2,4'-piperidine]-1',6-dicarboxylate (compound A11-5)

The compound A11-4 (5.0 g, 12.33 mmol, 1.0 eq) was dissolved in methanol (100 mL). The reaction mixture was cooled to 0°C, and sodium borohydride (933.09 mg, 24.66 mmol, 2.0 eq) was slowly added to the system. The mixture was reacted at room temperature for 2 hours. Aqueous solution (50 mL) was added to the reaction system, extracted with ethyl acetate (50 mL × 3 times), and washed with saturated saline solution (100 mL) Tthe organic phases was combined and dried over anhydrous sodium sulfate. The crude product obtained from the concentrated organic phase was purified by column chromatography to afford the white solid compound A11-5 (5 g, 99.51%).
LCMS (ESI) $[M+H]^+$ = 408.49.

Step 5: 7-methoxyspiro[chromene-2,4'-piperidine]-6-carboxylic acid methyl ester (compound A11-6)

The compound A11-5 (7.0 g, 17.18 mmol, 1.0 eq) was dissolved in toluene (100 mL). p-Toluenesulfonic acid (3.25 g, 18.90 mmol, 1.1 eq) was added to the mixture, which was reacted at 110°C for 2 hours. The reaction mixture was concentrated under reduced pressure to afford crude product A11-6 (7 g). This crude product was used directly in the subsequent reaction without further purification.
LCMS (ESI) $[M+H]^+$=290.28.

Step 6: 1'-(tert-butyl)6-methyl-7-methoxyspiro[chromene-2,4'-piperidine]-1',6-dicarboxylate (compound A 11-7)

The compound A11-6 (7.0 g, 24.19 mmol, 1.0 eq), di-tert-butyl dicarbonate (7.92 g, 36.29 mmol, 1.5 eq), and triethylamine (7.34 g, 72.58 mmol, 3 eq) were dissolved in dichloromethane (100 mL) and reacted at room temperature for 2 hours. The reaction mixture was concentrated under reduced pressure to afford crude product A11-7 (10 g). This crude product was used directly in the subsequent reaction without further purification.
LCMS (ESI) $[M+H]^+$=390.39.

Step 7: 1'-(tert-butyl)6-methyl-7-methoxyspiro[chroman-2,4'-piperidine]-1',6-dicarboxylate (compound A11-8)

The compound A11-7 (10 g, 25.68 mmol, 1.0 eq) was dissolved in tetrahydrofuran (100 mL). Palladium/carbon (5 g) was added to the system. After three displacements of hydrogen gas, the reaction was carried out at 50°C for 12 hours. The reaction mixture was filtered, and . The filtrate was concentrated under reduced pressure to obtain crude product A11-8 (10 g). This crude product is used directly in the next reaction without further purification.
LCMS (ESI) $[M+H]^+$ = 392.49

Step 8: 1'-(tert-butoxycarbonyl)-7-methoxyspiro[chroman-2,4'-piperidine]-6-carboxylic acid (compound A11-9)

The compound A11-8 (10 g, 25.55 mmol, 1.0 eq) was dissolved in methanol (80 mL), tetrahydrofuran (80 mL), and water (40 mL). Sodium hydroxide (5.11 g, 127.73 mmol, 5.0 eq) was added to the mixture and reacted at 50°C for 2 hours. The crude product obtained by concentrating the reaction mixture was purified by reverse-phase column chromatography to afford the white solid compound A11-9 (4 g, 41.49%).
LCMS (ESI) $[M+H]^+$ = 378.38

Step 9: (S)-6-((2,6-dioxopiperidin-3-yl)carbamoyl)-7-methoxyspiro[chroman-2,4'-piperidine]-1'-carboxylic acid tert-butyl ester (compound A11-10)

The compound A11-9 (3.0 g, 7.95 mmol, 1.0eq), (S)-3-Aminopiperidine-2,6-dione (1.22 g, 9.54 mmol, 1.2eq), 1-(3-dimethylaminopropyl)-3-ethylcarbodiimide hydrochloride (2.28 g, 11.92 mmol, 1.5eq), and 1-hydroxybenzotriazole (1.61 g, 11.92 mmol, 1.5eq) were dissolved in N,N-dimethylformamide (50 mL). N,N-diisopropylethylamine (3.08 g, 23.85 mmol, 3.0eq) was added to the system and reacted at room temperature for 2 hours. The crude product obtained by concentrating the reaction mixture was purified by reverse-phase column chromatography to afford the white solid compound A11-10 (2 g, 51.61%).
LCMS (ESI) $[M+H]^+$ = 488.39

Step 10: (S)-N-(2,6-dioxyhesperidin-3-yl)-7-methoxyspiro[chroman-2,4'-piperidine]-6-carboxamide (compound A11)

The compound A11-10 (7.0 g, 14.36 mmol, 1.0 eq) was dissolved in dichloromethane (50 mL). Trifluoroacetic acid (20 mL) was added to the system, and the mixture was reacted at room temperature for 2 hours. The crude product obtained by concentrating the reaction mixture was purified by reverse-phase column chromatography to afford the white solid compound A11 (5.1 g, 91.68%).

$^1$H NMR (400 MHz, DMSO-d6) δ 10.90 (s, 1H), 9.06 (d, J = 10.9 Hz, 1H), 8.49 (d, J = 7.2 Hz, 1H), 7.69 (s, 1H), 6.61 (s, 1H), 4.72 (dt, J = 11.1, 7.0 Hz, 1H), 3.87 (s, 3H), 3.24 (d, J = 12.5 Hz, 2H), 3.20 - 3.07 (m, 2H), 2.74 (dt, J = 25.5, 6.2 Hz, 3H), 2.52 - 2.44 (m, 1H), 2.08 (ddt, J = 12.3, 8.9, 3.9 Hz, 2H), 1.90 (d, J = 14.4 Hz, 2H), 1.83 (q, J = 12.1, 9.2 Hz, 4H).
LCMS (ESI) $[M+H]^+$ = 388.49

Example 12

Synthesis of Compound A12

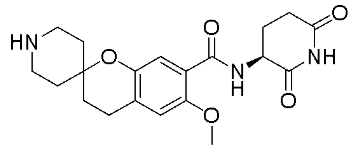

(S)-N-(2,6-dioxohesperidin-3-yl)-6-methoxyspiro[chroman-2,4'-pyridine]-7-carboxamide

Synthesis Scheme

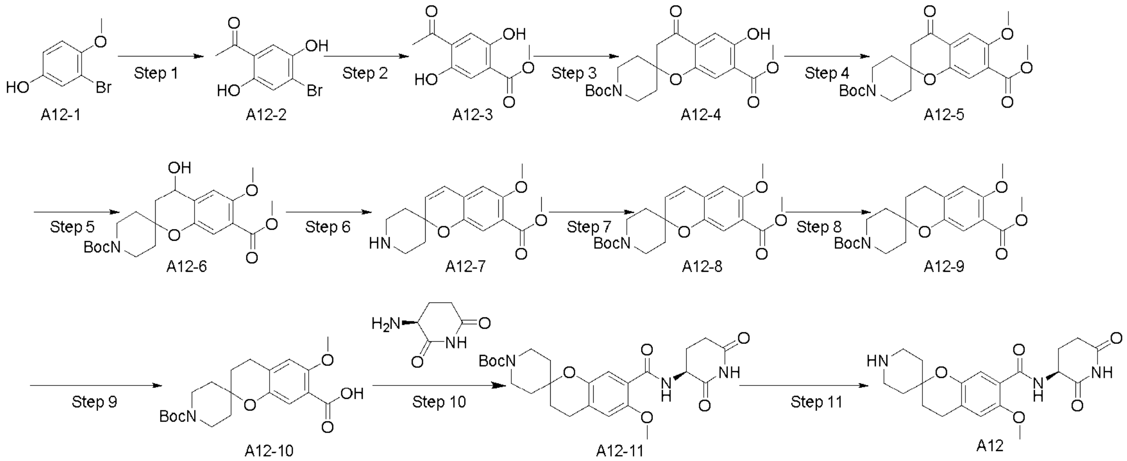


Step 1: 1-(4-bromo-2,5-dihydroxyphenyl)ethan-1-one (Compound A12-2)

The compound A12-1 (1.0 g, 4.93 mmol, 1.0 eq) and acyl chloride (1.93 g, 24.63 mmol, 5.0 eq) were reacted at 60°C for 0.5 hours. The mixture was cooled to room temperature, and aluminum trichloride (1.31 g, 9.85 mmol, 2.0 eq) was added to the reaction mixture and reacted at 160°C for 6 hours. After the mixture was cooled to room temperature, water (50 mL) was added to the system. It was extracted with ethyl acetate (100 mL × 3 times), washed with saturated sodium chloride solution (100 mL), combined the organic layers, and dried over anhydrous sodium sulfate. The crude product obtained from the concentrated organic phase was purified by forward column chromatography to afford the white solid compound A12-2 (230 mg, 20.21%).
$^1H$ NMR (600 MHz, DMSO-$d_6$) δ 11.16 (s, 1H), 9.97 (s, 1H), 7.32 (s, 1H), 7.16 (s, 1H), 2.56 (s, 3H).
LCMS (ESI): $[M+H]^+$ = 231.16.

Step 2: Methyl 4-acetyl-2,5-dihydroxybenzoate (Compound A12-3)

The compound A12-2 (300 mg, 1.30 mmol, 1.0 eq), DPPF palladium dichloride (89.64 mg, 0.13 mmol, 0.1 eq), and triethylamine (394.18 mg, 3.90 mmol, 3 eq) were dissolved in methanol (5 mL) and reacted at 70°C for 12 hours. The reaction mixture was concentrated under reduced pressure. Water (50 mL) was added to the system. It was then extracted with ethyl acetate (100 mL × 3 times), washed with saturated sodium chloride solution (100 mL), combined the organic layers and dried over anhydrous sodium sulfate. The crude product obtained from the concentrated organic phase was purified by forward column chromatography to afford the yellow solid compound A12-3 (150 mg, 50.96%).
$^1H$ NMR (600 MHz, DMSO-$d_6$) δ 10.74 (s, 1H), 9.81 (s, 1H), 7.30 (s, 2H), 3.88 (s, 3H), 2.61 (s, 3H).
LCMS (ESI): $[M+H]^-$ = 209.35.

Step 3: 1'-(tert-butyl)7-methyl-6-hydroxy-4-oxo-spiro[chroman-2,4'-piperidine]-1',7-dicarboxylate (compound A12-4)

The compound A12-3 (150.0 mg, 713.66 μmol, 1.0 eq), 4-oxopiperidine-1-carboxylic acid tert-butyl ester (170.64

g, 856.39 μmol, 1.2 eq), and pyrrolidine (60.91 g, 856.39 μmol, 1.2 eq) were dissolved in methanol (5 mL) and reacted at 70°C for 4 hours. After the reaction system was cooled to room temperature, it was purified by column chromatography to affored yellow solid compound A12-4 (220 mg, 78.76%).

LCMS (ESI): $[M+H]^+$ = 392.38.

Step 4: 1'-(tert-butyl)7-methyl-6-methoxy-4-oxo-spiro[chroman-2,4'-piperidine]-1',7-dicarboxylate (compound A12-5)

The compound A12-4 (220.0 mg, 562.06 μmol, 1.0 eq), iodomethane (239.33 mg, 1.69 mmol, 3.0 eq), and potassium carbonate (233.04 g, 1.69 mmol, 3.0 eq) were dissolved in N,N-dimethylformamide (5 mL) and reacted at room temperature for 4 hours. After the reaction system was cooled to room temperature, it was purified by column chromatography to afford yellow solid compound A12-5 (220 mg, 96.54%).

LCMS (ESI): $[M+H]^+$ = 406.38.

Step 5: 1'-(tert-butyl)7-methyl-4-hydroxy-6-methoxyspiro[chroman-2,4'-piperidine]-1',7-dicarboxylate (compound A12-6)

The compound A12-5 (220 mg, 542.61 μmol, 1.0 eq) was dissolved in methanol (3 mL). The reaction mixture was cooled to 0°C, and sodium borohydride (41.06 mg, 1.09 mmol, 2.0 eq) was slowly added to the system. The mixture was reacted at room temperature for 2 hours. Aqueous solution (5 mL) was added to the reaction system. It was then extracted with ethyl acetate (5 mL × 3 times), washed with saturated saline solution (10 mL), combined the organic phases and dried over anhydrous sodium sulfate. The crude product obtained from the concentrated organic phase was purified by column chromatography to afford the white solid compound A12-6 (170.0 mg, 76.89%).

LCMS (ESI) $[M+H]^+$ = 408.38.

Step 6: Methyl 6-methoxyspiro[chromene-2,4'-piperidine]-7-carboxylate (compound A12-7)

The compound A12-6 (170 mg, 417.22 mmol, 1.0 eq) was dissolved in toluene (5 mL). p-Toluenesulfonic acid (93.40 mg, 542.38 mmol, 1.3 eq) was added to the mixture, which was reacted at 110°C for 2 hours. The reaction mixture was concentrated under reduced pressure and purified on a reversed-phase column to afford colorless oily compound A12-7 (120 mg, 99.41%).

LCMS (ESI) $[M+H]^+$ =290.38.

Step 7: 1'-(tert-butyl)7-methyl-6-methoxyspiro[chromene-2,4'-piperidine]-1',7-dicarboxylate (compound A12-8)

The compound A12-7 (120 mg, 414.75 μmol, 1.0 eq), di-tert-butyl dicarbonate (135.7 mg, 622.12 μmol, 1.5 eq), and triethylamine (125.91 mg, 1.24 mmol, 3 eq) were dissolved in dichloromethane (2 mL) and reacted at room temperature for 2 hours. The reaction mixture was concentrated under reduced pressure and purified on a reversed-phase column to afford yellow oily compound A12-8 (150 mg, 92.87%).

LCMS (ESI) $[M+H]^+$ =390.38.

Step 8: 1'-(tert-butyl)7-methyl-6-methoxyspiro[chroman-2,4'-piperidine]-1',7-dicarboxylate (compound A12-9)

The compound A12-8 (150 mg, 385.16 μmol, 1.0 eq) was dissolved in tetrahydrofuran (5 mL), and palladium/-carbon (20 mg) was added to the system. After three hydrogen exchanges, the mixture reacted for 12 hours at 50°C. The reaction mixture was filtered, and the filtrate was concentrated under reduced pressure to afford crude product A12-9 (120 mg). This crude product was used directly in the next reaction without further purification.

LCMS (ESI) $[M+H]^+$ = 392.28

Step 9: 1'-(tert-butyl)7-methyl-6-hydroxy-spiro[chroman-2,4'-piperidine]-1',7-dicarboxylate (compound A12-10)

The compound A12-9 (120 mg, 306.54 mmol, 1.0 eq) was dissolved in methanol (4 mL), tetrahydrofuran (4 mL), and water (2 mL). Sodium hydroxide (61.30 mg, 1.53 mmol, 5.0 eq) was added to the mixture, which was reacted at 50°C for 2 hours. The crude product obtained by concentrating the reaction mixture was purified by reverse-phase column chromatography to afford the white solid compound A12-10 (50 mg, 43.22%).

LCMS (ESI) $[M+H]^+$ = 378.38

Step 10: ((S)-7-((2,6-dioxohesperidin-3-yl)carbamoyl)-6-methoxyspiro[chroman-2,4'-piperidine]-1'-carboxylic acid tert-butyl ester (compound A12-11)

The compound A12-10 (40 mg, 105.98 μmol, 1.0 eq), (S)-3-Aminopiperidine-2,6-dione (16.29 mg, 127.17 μmol, 1.2 eq), 1-(3-dimethylaminopropyl)-3-ethylcarbodiimide hydrochloride (30.48 mg, 158.97 μmol, 1.5 eq), and 1-hydroxybenzotriazole (21.44 mg, 158.97 μmol, 1.5eq) were dissolved in N,N-dimethylformamide (1 mL). N,N-diisopropylethylamine (41.09 mg, 317.93 mmol, 3.0eq) was added to the system and reacted at room temperature for 2 hours. The crude product obtained by concentrating the reaction mixture was purified by reverse-phase column chromatography to afford the white solid compound A12-11 (30 mg, 58.06%).
LCMS (ESI) $[M+H]^+$ = 488.39

Step 11: (S0 -N-(2,6-dioxohesperidin -3-yl)-6-methoxyspiro[chroman-2,4'-piperidine]-7-carboxamide (compound A12)

The compound A12-11 (20 mg, 41.02 μmol, 1.0 eq) was dissolved in dichloromethane (2 mL). Trifluoroacetic acid (1 mL) was added to the system, and the mixture was reacted at room temperature for 2 hours. The crude product obtained by concentrating the reaction mixture was purified by reverse-phase column chromatography to afford the white solid compound A12 (15 mg, 94.38%).

$^1H$ NMR (400 MHz, DMSO-d6) δ 10.91 (s, 1H), 8.64 (d, J = 7.2 Hz, 2H), 7.37 (s, 1H), 6.95 (s, 1H), 4.74 (dt, J = 11.1, 7.0 Hz, 1H), 3.86 (s, 3H), 3.20 (d, J = 12.4 Hz, 2H), 3.13 (q, J = 11.8 Hz, 2H), 2.82 (t, J = 6.7 Hz, 2H), 2.80 - 2.72 (m, 1H), 2.54 (t, J = 3.6 Hz, 1H), 2.09 (tq, J = 12.5, 4.7 Hz, 2H), 1.92 - 1.80 (m, 4H), 1.80 - 1.71 (m, 2H).
LCMS (ESI) $[M+H]^+$ = 388.39

Example 13

Synthesis of Compound B1

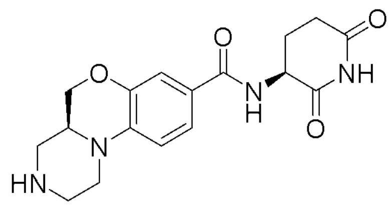

(S)-N-((S)-2,6-dioxopiperidin-3-yl)-1,2,3,4,4a,5-hexahydrobenzo[b]pyrazino[1,2-d][1,4]oxazine-8-yl)formamide

Synthesis Scheme

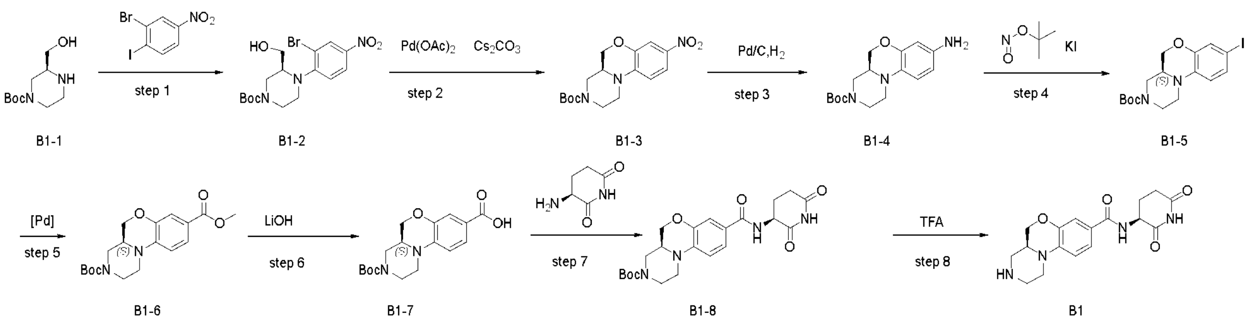


Step 1: (S)-4-(2-bromo-4-nitrophenyl)-3-(hydroxymethyl)piperidine-1-carboxylic acid tert-butyl ester (compound B1-2)

The compound B1-1 (2.1 g, 9.71 mmol, 1.0 eq), 2-bromo-1-iodo-4-nitrobenzene (3.34 g, 10.20 mmol, 1.05 eq), ethylene glycol (1.21 g, 19.42 mmol, 2 eq), cuprous iodide (462.30 mg, 2.43 mmol, 0.25 eq), and potassium phosphate

(4.12 g, 19.42 mmol, 2 eq) were dissolved in isopropanol (30 mL) and reacted at 100°C for 12 h under nitrogen atmosphere. The reaction mixture was filtered and concentrated to obtain a crude product. The crude product was purified by column chromatography to afford yellow oily compound B1-2 (1.25 g, 30.9%).

$^1$H NMR (600 MHz, DMSO-$d_6$) δ 8.37 (d, J = 2.7 Hz, 1H), 8.17 (dd, J = 9.0, 2.7 Hz, 1H), 7.32 (d, J = 9.0 Hz, 1H), 4.62 (t, J = 5.2 Hz, 1H), 3.92-3.84 (m, 2H), 3.84-3.81 (m, 1H), 3.48-3.40 (m, 2H), 3.39-3.34 (m, 1H), 3.10 (brs, 1H), 3.03 - 2.96 (m, 1H).

LCMS (ESI): $[M\text{-}Boc+H]^+$ = 360.29.

Step 2: (S)-8-nitro-1,2,4a,5-tetrahydrobenzo[b]pyrazine[1,2-d][1,4]oxazine-3(4H)-formic acid tert-butyl ester (compound B1-3)

The compound B1-2 (1.24 g, 2.98 mmol, 1.0 mol/L), 2-(di-tert-butylphosphino)-1,1'-binaphthyl (237.43 mg, 0.59 mmol, 0.2 mol/L), palladium acetate (66.9 mg, 0.29 mmol, 0.1 eq), and cesium carbonate (1.94 g, 5.96 mmol, 2 eq) were dissolved in toluene. The mixture was reacted at 100°C for 12h under nitrogen protection. The crude product obtained by concentrating the reaction mixture was purified by column chromatography to afford yellow solid B1-3 (0.74 g, 74.1%).

$^1$H NMR (600 MHz, DMSO-$d_6$) δ 7.77 (dd, J = 9.1, 2.6 Hz, 1H), 7.52 (d, J = 2.6 Hz, 1H), 7.05 (d, J = 9.2 Hz, 1H), 4.42 (dd, J = 11.2, 3.2 Hz, 1H), 4.03-3.93 (m, 4H), 3.40-3.35 (m, 1H), 2.99 (brs, 1H), 2.95-2.88 (m, 1H), 2.67 (brs, 1H).

LCMS (ESI): $[M\text{-}Boc+H]^+$ = 279.98.

Step 3: (S)-8-amino-1 ,2,4a,5-tetrahydrobenzo[b]pyrazine[1,2-d][1,4]oxazine-3(4H)-formic acid tert-butyl ester (compound B1-4)

The compound B1-3 (0.74 g, 2.21mmol, 1.0 eq) was dissolved in methanol (20 mL). Palladium(II)/carbon (150 mg, 10%) was added to the system. The mixture was purged with hydrogen for three times and reacted at room temperature for 12 h. The mixture was filtered and the organic phase was concentrated to obtain purple solid compound B1-4 (0.63 g, 93.5%). The crude product is used directly in the next reaction without further purification.

LCMS (ESI): $[M+H]^+$ = 306.28.

Step 4: (S)-8-iodo-1,2,4a,5-tetrahydrobenzo[b]pyrazine[1,2-d][1,4]oxazine-3(4H)-formic acid tert-butyl ester (compound B1-5)

The compound B1-4 (2.1 g, 6.88 mmol, 1.0 eq) was dissolved in diiodomethane (45 mL). Isoamyl nitrite (1.61 g, 13.75 mmol, 2 eq) and potassium iodide (3.42 g, 20.63 mmol, 3 eq) were added, and reacted under nitrogen protection at 80°C for 12 h. The system was filtered, followed by pressure reduction, and the organic phase was concentrated . The crude product obtained was purified by column chromatography to afford yellow oily compound B1-5 (1.53 g, 53.4%).

$^1$H NMR (600 MHz, Chloroform-*d*) δ 7.15 (dd, J = 8.5, 2.1 Hz, 1H), 7.11 (d, J = 2.0 Hz, 1H), 6.55 (d, J = 8.6 Hz, 1H), 4.23 (dd, J = 10.8, 2.8 Hz, 1H), 4.20 - 4.08 (m, 2H), 3.97 (dd, J = 10.8, 8.5 Hz, 1H), 3.64 (d, J = 12.0 Hz, 1H), 3.13-3.07 (m, 1H), 3.03 (brs, 1H), 2.72 (td, J = 12.1, 3.5 Hz, 1H), 2.62 (brs, 1H), 1.50 (s, 9H).

LCMS (ESI): $[M\text{-}tBu+H]^+$ = 361.19.

Step 5: 3-(tert-butyl)-8-methyl-(S)-1,2,4a,5-tetrahydrobenzo[b]pyrazine[1,2-d][1,4]oxazine-3(4H)-diformate (compound B1-6)

The compound B1-5 (0.12 g, 0.29 mmol, 1 eq), [1,1'-Bis(diphenylphosphino)ferrocene]palladium(II) dichloromethane complex (35.3 mg, 43.2 μmol, 0.15 eq), 4,5-Bis(diphenylphosphino)-9,9-dimethyloxanthene (19.7 mg, 86.5 μmol, 0.3 eq), and triethylamine (35.3 mg, 0.86 mmol, 3 eq) were dissolved in a mixture of ultradry N,N-dimethylformamide (6 mL) and methanol (3 mL). The system was purged with carbon monoxide gas three times and reacted at 90°C for 12 h. After removing methanol by concentrating under reduced pressure, the reaction solution was extracted with ethyl acetate and water. The organic phase was dried over anhydrous sodium sulfate, concentrated, and the resulting crude product was purified by column chromatography to afford the yellow solid compound B1-6 (45 mg, 44.8%).

$^1$H NMR (600 MHz, Chloroform-*d*) δ 7.59 (dd, J = 8.6, 2.0 Hz, 1H), 7.47 (d, J = 2.0 Hz, 1H), 6.80 (d, J = 8.6 Hz, 1H), 4.28 (dd, J = 10.9, 2.9 Hz, 1H), 4.24 - 4.06 (m, 2H), 3.99 (dd, J = 10.9, 8.2 Hz, 1H), 3.88 (s, 3H), 3.78 (d, J = 12.1 Hz, 1H), 3.28-3.22 (m, 1H), 3.04 (brs, 1H), 2.87 (td, J = 12.1, 3.5 Hz, 1H), 2.66 (brs, 1H), 1.51 (s, 9H).

LCMS (ESI) $[M\text{-}tBu+H]^+$ = 293.28.

Step 6: (S)-3-(tert-butoxycarbonyl)-1,2,4a,5-tetrahydrobenzo[b]pyrazine[1,2-d][1,4]oxazine-8-formic acid (compound B1-7)

The compound B1-6 (40 mg, 0.11 mmol, 1.0 eq) was dissolved in tetrahydrofuran (1 mL). An aqueous solution of lithium hydroxide (27.5 mg, 1.15 mmol, 10 eq) (1 mL) was added to the mixture. The reaction was carried out at 50°C for 10 hours. The reaction mixture was adjusted to pH 2 with dilute hydrochloric acid, then extracted with ethyl acetate and water. The organic phase was dried over anhydrous sodium sulfate and concentrated under reduced pressure to afford the white solid compound B1-7 (32 mg, 83.4%). This crude product was used directly in the next step without further purification. LCMS (ESI) $[M\text{-}tBu +H]^+$ = 279.38

Step 7: (S)-8-(((S)-2,6-dioxopiperidin-3-yl)carbamoyl)-1,2,4a,5-tetrahydrobenzo[b]pyrazine[1,2-d] [1,4]oxazine-3(4H)-formic acid tert-butyl ester (compound B1-8)

The compound B1-7 (32 mg, 95.7 μmol, 1.0 eq) was dissolved in ultradry N,N-dimethylformamide (1 mL), followed by sequential addition of 2-(7-azabenzotriazole)-N,N,N',N'-tetramethyluronium hexafluorophosphate (43.7 mg, 114.8 μmol, 1.2 eq), (S)-3-amino-2,6-piperidinedione hydrochloride (18.9 mg, 114.8 μmol, 1.2 eq), and N, N-diisopropyl ethylamine (37.1 mg, 287.1 μmol, 3 eq). The system was reacted at room temperature for 1 h. The reaction mixture was filtered and purified by preparative liquid chromatography to afford the white solid compound B1-8 (35 mg, 82.3%). LCMS (ESI) $[M\text{-}Boc +H]^+$ = 345.39

Step 8: (S)-N-((S)-2,6-dioxopiperidin-3-yl)-1,2,3,4,4a,5-hexahydrobenzo[b]pyrazinazino[1,2-d][1,4]oxazin-8-yl)formamide (compound B1)

The compound B1-8 (25 mg, 56.2 μmol, 1.0 eq) was dissolved in dichloromethane (1 mL), and trifluoroacetic acid (1 mL) was added. The mixture reacted at room temperature for 1 h. The reaction mixture was concentrated under reduced pressure and purified by preparative liquid chromatography to afford the white solid compound B1 (15 mg, 77.4%).

$^1$H NMR (600 MHz, DMSO-*d*6) δ 10.83 (s, 1H), 9.12-8.95 (m, 2H), 8.51 (d, J = 8.3 Hz, 1H), 7.44 (dd, J = 8.5, 2.1 Hz, 1H), 7.32 (d, J = 2.1 Hz, 1H), 7.04 (d, J = 8.7 Hz, 1H), 4.76-4.70 (m, 1H), 4.34 (dd, J = 11.1, 2.8 Hz, 1H), 4.14-4.09 (m, 1H), 4.02 (dd, J = 11.1, 7.5 Hz, 1H), 3.50-3.45 (m, 1H), 3.43 (d, J = 12.2 Hz, 1H), 3.39 (d, J = 11.9 Hz, 1H), 3.12-3.03 (m, 1H), 3.00 (td, J = 12.9, 2.7 Hz, 1H), 2.88 - 2.75 (m, 2H), 2.57 - 2.52 (m, 1H), 2.17 - 2.06 (m, 1H), 1.97-1.93 (m, 1H). LCMS (ESI) $[M+H]^+$ = 345.39

Example 14

Synthesis of Compound B2

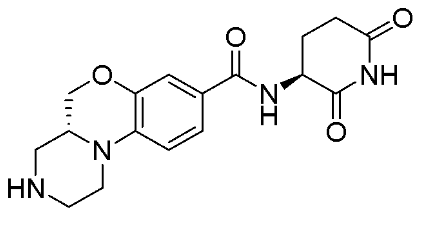

(R)-N-((S)-2,6-dioxopiperidin-3-yl)-1,2,3,4,4a,5-hexahydrobenzo[b]pyrazino[1,2-d][1,4]oxazine-8-yl)formamide

Synthesis Scheme

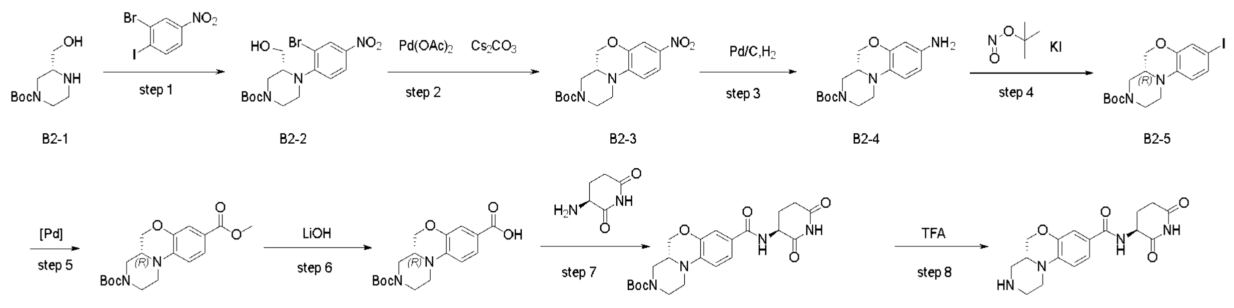


B2-1 B2-2 B2-3 B2-4 B2-5 B2-6 B2-7 B2-8 B2

The synthesis method of compound B2 is the same as that of compound B1.

$^1$H NMR (600 MHz, DMSO-*d*6) δ 10.84 (s, 1H), 9.02 (s, 1H), 8.96 (s, 1H), 8.52 (d, J = 8.4 Hz, 1H), 7.44 (dd, J = 8.6, 2.1 Hz, 1H), 7.32 (d, J = 2.1 Hz, 1H), 7.04 (d, J = 8.7 Hz, 1H), 4.76-4.70 (m, 1H), 4.34 (dd, J = 11.1, 2.8 Hz, 1H), 4.14 - 4.09 (m, 1H), 4.01 (dd, J = 11.2, 7.5 Hz, 1H), 3.49 - 3.43 (m, 3H), 3.13 - 3.03 (m, 1H), 2.99 (td, J = 13.0, 2.7 Hz, 1H), 2.88 - 2.74 (m, 2H), 2.58 - 2.52 (m, 1H), 2.15-2.06 (m, 1H), 1.97-1.91 (m, 1H).
LCMS (ESI) $[M+H]^+$ = 345.30

Example 15

Synthesis of Compound B3

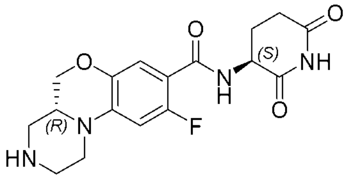

(R)-N-((S)-2,6-dioxyhesperidin-3-yl)-9-fluoro-1,2,3,4,4,4a,5-hexahydrobenzo[b]pyrazino[1,2-d][1,4]oxazine-8-carboxamide

Synthesis Scheme

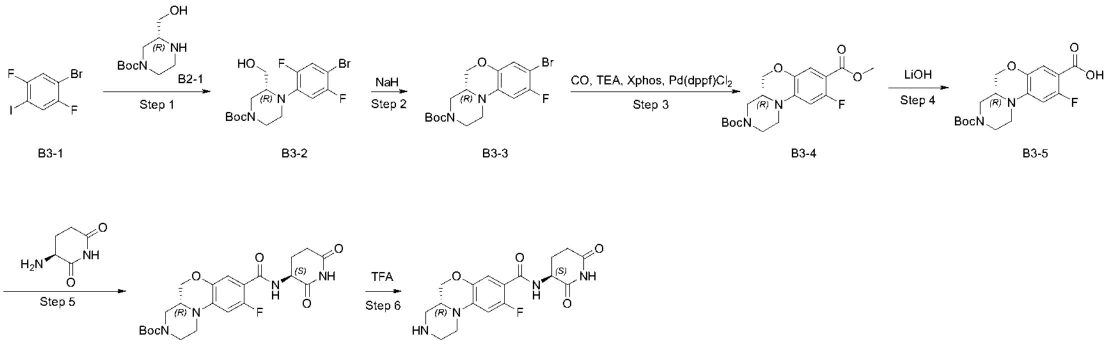


B3-1 B3-2 B3-3 B3-4 B3-5 B3-6 B3

Step 1: (R)-4-(4-bromo-2,5-difluorophenyl)-3-(hydroxymethyl)piperazine-1-carboxylic acid tert-butyl ester (compound B3-2)

The compound B3-1 (3.0 g, 13.87 mmol, 1.0 eq) was dissolved in isopropanol (30 mL). Compound B2-1 (4.87 g, 15.26 mmol, 1.2 eq), ethylene glycol (1.72 g, 27.74 mmol, 2.0 eq), cuprous iodide (660 mg, 3.47 mmol, 0.25 eq), and potassium phosphate (5.9 g, 27.74 mmol, 2.0 eq) were added. The reaction mixture was heated to 100°C under nitrogen

protection and reacted at this temperature for 12 hours. The resulting reaction system was concentrated under reduced pressure. The crude product obtained was purified by reverse-phase column chromatography to afford the pale yellow solid compound B3-2 (1 g, 17.7%).

LCMS (ESI): $[M+H]^+$ = 351.29.

Step 2: (R)-8-bromo-9-fluoro-1,2,4a,5-tetrahydrobenzo[b]pyrazino[1,2-d][1,4]oxazine-3(4H)-carboxylic acid tert-butyl ester (compound B3-3)

The compound B3-2 (1.0 g, 2.46 mmol, 1.0 eq) was dissolved in N,N-dimethylformamide (10 mL). The mixture was cooled to 0°C before adding sodium hydride (89 mg, 3.68 mmol, 1.5 eq). After complete addition in portions, the temperature was gradually raised to 110°C and reacted for 3 hours. The reaction mixture was concentrated under reduced pressure. Water (50 mL) was added to the system, and the system was extracted with ethyl acetate (100 mL × 3 times), and washed with saturated sodium chloride solution (100 mL). The organic phases were combined and drid over anhydrous sodium sulfate. The crude product obtained from the concentrated organic phase was purified by reverse-phase column chromatography to afford the yellow oily compound B3-3 (880 mg, 84.13%).

LCMS (ESI): $[M+H]^+$ = 331.19.

Step 3: 3-(tert-butyl)-8-methyl-(R)-9-fluoro-1,2,4a,5-tetrahydrobenzo[b]pyrazino[1,2-d][1,4]oxazine-3,8(4H)-dicarboxylate (compound B3-4)

The compound B3-3 (600 mg, 1.55 mmol, 1.0 eq), 2-dicyclohexylphosphino-2',4',6'-triisopropylbiphenyl (222 mg, 0.465 mmol, 0.30 eq), [1,1'-bis(diphenylphosphino)ferrocene]palladium(II) dichloromethane complex (253 mg, 3.31 mmol, 0.2 eq), and triethylamine (782 mg, 7.75 mmol, 5 eq) were dissolved in a mixed solvent of methanol and N,N-dimethylformamide (30 mL) and reacted at 90°C for 12 hours under an atmosphere of carbon monoxide. After the reaction system was cooled to room temperature, and concentrated under reduced pressure, then water (100 mL) was add. The system was extracted with ethyl acetate (100 mL × 3 times), washed with saturated saline solution (100 mL), and the organic phases were combined, and dried over anhydrous sodium sulfate. The organic phase was concentrated to obtain a crude product. The crude product was purified by column chromatography to afford yellow solid compound B3-4 (422 mg, 74.34%).

LCMS (ESI): $[M+H]^+$ = 311.18.

Step 4: (R)-3-(tert-butoxycarbonyl)-9-fluoro-1,2,3,4,4a,5-hexahydrobenzo[b]pyrazino[1,2-d][1,4]oxazine-8-formic acid (compound B3-5)

The compound B3-4 (412 mg, 1.12 mmol, 1.0 eq) was dissolved in a mixed solvent of methanol, tetrahydrofuran, and water (9 mL). The reaction mixture was heated to 50°C and reacted at this temperature for 3 hours. The resulting reaction mixture was cooled to 0°C. Acetic acid was added dropwise until the reaction mixture reaches pH 6. The mixture was extracted with ethyl acetate (50 mL × 3 times), washed with saturated saline solution (100 mL), the organic phases were combined, and dried over anhydrous sodium sulfate. The organic phase was concentrated to afford brown solid compound B3-5 (335 mg, 84.55%).

LCMS (ESI) $[M+H]^+$ = 388.29.

Step 5: (R)-8-(((S)-2,6-dioxyhesperidin-3-yl)aminocarbonyl)-9-fluoro-1,2,4a,5-tetrahydrobenzo[b]pyrazine[1,2-d] [1,4] oxazine-3(4H)-carboxylic acid tert-butyl ester (compound B3-6)

The compound B3-5 (325 mg, 0.922 mmol, 1.0 eq) was dissolved in N,N-dimethylformamide (3 mL). (S)-3-aminopiperidine-2,6-dione (227.71 mg, 1.38 mmol, 1.5 eq), 2-(7-azobenzotriazole)-N,N,N',N'-tetramethylurea hexafluorophosphate (526.06 mg, 1.38 mmol, 1.5 eq), and N,N-diisopropylethylamine (357.63 mg, 2.77 mmol, 3.0 eq) were added to the solution and the mixture was reacted at room temperature for 2 hours. Water (50 mL) was added to the system, extracted with ethyl acetate (100 mL × 3 times), washed with saturated saline solution (100 mL), the organic phases were combined, and dried over anhydrous sodium sulfate. The organic phase was concentrated to afford a crude product, and was purified by reverse-phase column chromatography to afford the white solid compound B3-6 (322 mg, 75.49%).

LCMS (ESI) $[M+H]^+$ = 407.39.

Step 6: (R)-N-((S)-2,6-dioxyhesperidin-3-yl)-9-fluoro-1,2,3,4,4,4a,5-hexahydrobenzo[b]pyrazino[1,2-d][1,4]oxazine-8-carboxamide (compound B3)

The compound B3-6 (322 mg, 0.696 mmol, 1.0 eq) and trifluoroacetic acid (0.5 mL) were dissolved in

dichloromethane (1.5 mL). The mixture was reacted for 2 hours at room temperature. The reaction mixture was concentrated, and the crude product obtained was purified by reverse-phase column chromatography to afford the white solid compound B3 (240 mg, 95.31 %).

$^1H$ NMR (600 MHz, DMSO) δ 10.86 (s, 1H), 7.95 (s, 1H), 7.10 (d, J = 7.2 Hz, 1H), 6.96 (d, J = 13.7 Hz, 1H), 4.75 - 4.67 (m, 2H), 4.31 (dd, J = 11.2, 2.7 Hz, 1H), 4.09 (d, J = 10.2 Hz, 1H), 3.99 (dd, J = 11.2, 7.2 Hz, 1H), 3.55 - 3.47 (m, 1H), 3.44 - 3.35 (m, 2H), 3.09 - 2.99 (m, 2H), 2.86 - 2.75 (m, 2H), 2.56 - 2.52 (m, 1H), 2.15 - 2.05 (m, 1H), 2.02 - 1.94 (m, 1H).

LCMS (ESI) $[M+H]^+$ = 363.49.

Example 16

Synthesis of Compound B4

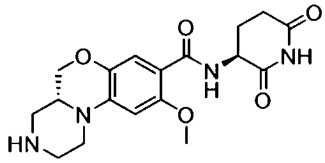

(R)-N-((S)-2,6-Dioxyhesperidin-3-yl)-O-9-methoxy-1,2,3,4,4a,5-hexahydrobenzo[b]pyrazino[1,2-d][1,4]oxazine-8-carboxamide

Synthetic Scheme:

(R)

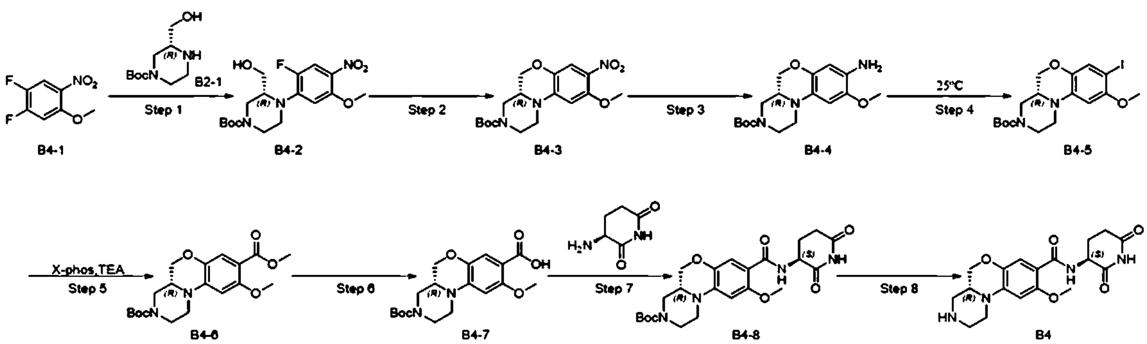


Step 1: R-4-(2-Fluoro-5-methoxy-4-nitrophenyl)-3-(hydroxymethyl) piperazine-1-carboxylicacid tert-butyl ester (Compound B4-2)

The compound B4-1 (3.90 g, 18.03 mmol, 1.0 eq) and compound B2-1 (3.41 g, 18.03 mmol, 1.0 eq) was dissolved in dimethyl sulfoxide (20 mL). The reaction mixture was heated to 100°C and reacted for 12 hours at this temperature. Water (50 mL) was added to the system, extracted with ethyl acetate (100 mL × 3 times), washed with saturated saline solution (100 mL), combined with the organic layers, and dried over anhydrous sodium sulfate. The organic phase was concentrated to afford a crude product, and was purified by normal-phase column chromatography to afford the yellow solid compound B4-2. (4.20 g, 60.44%).

$^1H$ NMR (600 MHz, DMSO-$d_6$) δ 7.82 (d, J = 13.7 Hz, 1H), 6.69 (d, J = 7.5 Hz, 1H), 4.76 (t, J = 5.3 Hz, 1H), 4.02 - 3.85 (m, 6H), 3.50 (hept, J = 6.3, 5.8 Hz, 2H), 3.37 (dt, J = 12.1, 3.1 Hz, 1H), 3.32 (dd, J = 12.6, 3.4 Hz, 1H), 3.27 - 2.92 (m, 2H), 1.42 (s, 9H).

LCMS (ESI): $[M+H]^+$ = 386.28.

Step 2: (R) -9-Methoxy-8-nitro-1,2,4a,5-tetrahydrobenzo[b]pyrazino[1,2-d][1,4] oxazine-3(4H)-carboxylic acid tert-butyl ester (Compound B4-3)

The compound B4-2 (3.0 g, 7.78 mmol, 1.0 eq) was dissolved in N,N-dimethylformamide (10 mL).The sodium hydride (373.61 mg, 9.34 mmol, 1.2 eq) was added in three portions. The reaction mixture was heated to 110°C and reacted at this temperature for 2 hours. Water (50 mL) was added to the system. The mixture was extracted with ethyl acetate (100 mL × 3 times), washed with saturated saline solution (100 mL), and the combined organic layers were dried over anhydrous sodium sulfate. The crude product obtained from the concentrated organic phase was purified by reverse-phase column chromatography to afford the yellow solid compound B4-3 (2.50 g, 87.90%).
LCMS (ESI): $[M+H]^+$ = 366.28.

Step 3: (R)-8-Amino-9-methoxy-1,2,4a,5-tetrahydrobenzo [b]pyrazino[1,2-d][1,4] oxazine-3(4H)-carboxylic acid tert-butyl ester (Compound B4-4)

The compound B4-3 (2.50 g, 6.84 mmol, 1.0 eq) was dissolved in methanol (60 mL). Palladium/carbon (500 mg) was added to the system. After three hydrogen exchanges, the mixture reacted at room temperature for 12 hours. The reaction mixture was filtered, and the filtrate was concentrated under reduced pressure to afford crude product B4-4 (1.9 g, 82.79%). This crude product was used directly in the next step without further purification.
LCMS (ESI): $[M+H]^+$ = 335.39.

Step 4: (R)-8-Iodo-9-methoxy-1,2,4a,5-tetrahydrobenzo[b]pyrazino[1,2-d][1,4] oxazine-3(4H)-carboxylic acid tert-butyl ester (Compound B4-5)

The compound B4-4 (1.30 g, 3.88 mmol, 1.0eq) was dissolved in diiodomethane (10 mL). Isopentyl nitrite (908.11 mg, 7.75 mmol, 2.0eq) andpotassium iodide (1.93 g, 11.63 mmol, 3.0eq) were added dropwise, and reacted at room temperature for 2 hours. The crude product obtained by concentrating the reaction solution was purified by normal-phase column chromatography to obtain a yellow oily compound B4-5 (750 mg, 43.36%).
LCMS (ESI) $[M+H]^+$ = 446.29.

Step 5:

3-(tert-Butyl)-8-methyl-(R)-9-methoxy-1,2,4a,5-tetrahydrobenzo[b]pyrazino[1,2-d][1,4]oxazine-3,8(4H)-dicarboxylate (Compound B4-6)

The compound B4-5 (500 mg, 1.12 mmol, 1.0 eq), DPPF palladium dichloride (81.90 mg, 0.11 mmol, 0.1 eq), 2-Dicyclohexylphosphine-2',4',6'-triisopropylbiphenyl (106.82 mg, 0.22 mmol, 0.2 eq), and triethylamine (566.86 mg, 5.60 mmol, 5 eq) were dissolved in methanol (10 mL) and N,N-dimethylformamide (5 mL) and reacted at 80°C for 12 hours. The reaction mixture was concentrated under reduced pressure. Water (20 mL) was added to the system, followed by extraction with ethyl acetate (40 mL × 3 times), washed with saturated saline solution (40 mL), and combined the organic layers. The combined organic layers were dried over anhydrous sodium sulfate. The crude product obtained from the concentrated organic phase was purified by reverse-phase column chromatography to obtain a brown solid compound B4-6 (350 mg, 82.55%)
LCMS (ESI) $[M+H]^+$ = 379.39.

Step 6:

(R)-3-(tert-Butoxycarbonyl)-9-methoxy-1,2,3,4,4a,5-hexahydrobenzo[b]pyrazino[1,2-d][1,4]oxazine-8-carboxylic acid (Compound B4-7)

The compound B4-6 (300 mg, 792.76μmol, 1.0 eq) and sodium hydroxide (158.54 mg, 3.96 mmol, 5.0 eq) were dissolved in methanol (2 mL), tetrahydrofuran (2 mL), and water (1 mL), and reacted at room temperature for 2 hours. The reaction mixture was concentrated, and the crude product was purified by reverse-phase column chromatography to obtain a brown solid compound B4-7 (250 mg, 86.54%).
LCMS (ESI) $[M+H]^+$= 365.49.

Step 7:

(R)-8-(((S)-2,6-Dioxyhesperidin-3-yl)aminocarbonyl)-9-methoxy-1,2,4a,5-tetrahydrobenzo[b]pyrazine[1,2-d][1,4]oxazine-3(4H)-carboxylic acid tert-butyl ester (Compound B4-8)

The compound B4-7 (200 mg, 548.85μmol, 1.0 eq), (S)-3-Aminopiperidine-2,6-dione (84.39 mg, 658.62μmol, 1.2 eq), 1-(3-dimethylaminopropyl)-3-ethylcarbodiimide hydrochloride (157.82 mg, 828.27μmol, 1.5 eq), and 1-hydroxybenzotriazole (111.20 mg, 828.27μmol, 1.5 eq) were dissolved in N,N-dimethylformamide (2 mL). N,N-diisopropylethylamine (212.81 mg, 1.65 mmol, 3.0 eq) was added to the system, and the mixture reacted at room temperature for 2 hours. The reaction mixture was concentrated, and the crude product was purified by reverse-phase column chromatography to obtain a white solid compound B4-8 (250 mg, 95.99%).
LCMS (ESI) [M+H]$^+$= 475.49

Step 8:

(R)-4-(2-Fluoro-5-methoxy-4-nitrophenyl)-3-(hydroxymethyl)piperazine-carboxylic acid tert-butyl ester (Compound B4)

The compound B4-8 (250 mg, 526.85μmol, 1.0 eq) was dissolved in dichloromethane (4 mL). Trifluoroacetic acid (2 mL) was added to the system, and the mixture reacted at room temperature for 2 hours. The crude product obtained by concentrating the reaction solution was purified by reverse-phase column chromatography to obtain a white solid compound B4 (180 mg, 91.25%).

$^1$H NMR (400 MHz, DMSO-d6) δ 10.90 (s, 1H), 9.10 (s, 1H), 8.55 - 8.48 (m, 1H), 7.29 (s, 1H), 6.68 (s, 1H), 4.70 (dt, J = 12.5, 6.3 Hz, 1H), 4.26 (ddd, J = 20.9, 10.7, 2.3 Hz, 2H), 3.95 (dd, J = 11.2, 7.3 Hz, 1H), 3.90 (s, 3H), 3.53 - 3.48 (m, 1H), 3.45 - 3.36 (m, 2H), 3.11 - 3.02 (m, 2H), 2.86 - 2.73 (m, 2H), 2.53 (s, 1H), 2.14 - 2.03 (m, 2H).
LCMS (ESI) [M+H]$^+$= 375.39

Example 17:

Synthesis of Compound B5

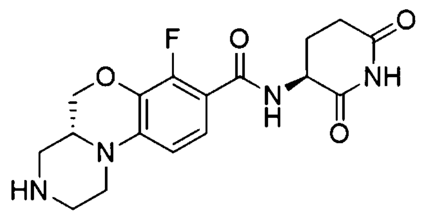

(R)-N-((S)-2,6-Dioxyhesperidin-3-yl)-7-fluoro-1,2,3,4,4,4a,5-hexahydrobenzo[b]pyrazino[1,2-d][1,4]oxazine-8-carboxamide

Synthetic Scheme:

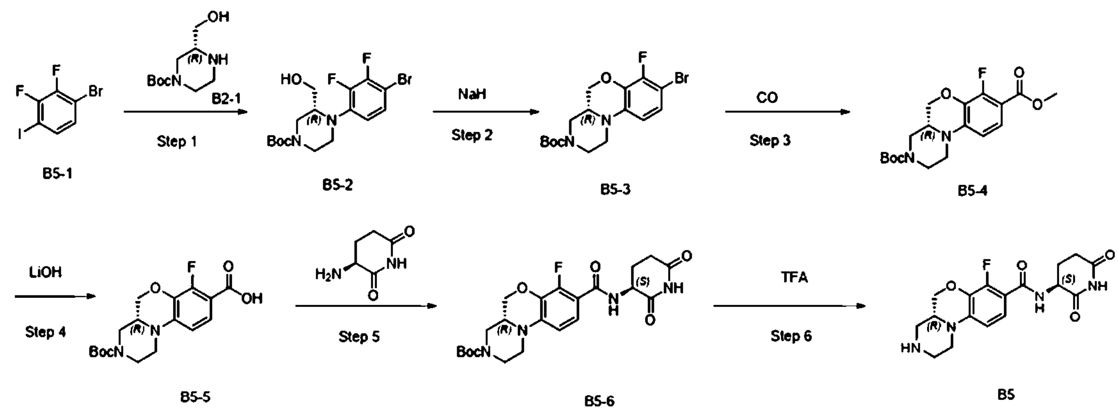

B5-1 B5-2 B5-3 B5-4 B5-5 B5-6 B5

The synthesis method of compound B5 is the same as that of compound B3.

$^1$H NMR (600 MHz, $CD_3OD$) δ 7.40 (dd, *J* = 8.9, 7.6 Hz, 1H), 6.88 (dd, *J*= 9.1, 1.4 Hz, 1H), 4.82 (dd, *J*= 12.7, 5.3 Hz, 1H), 4.45 (dd, *J* = 11.2, 2.9 Hz, 1H), 4.2-4.19 (m, 1H), 4.13 (dd, *J* = 11.2, 7.3 Hz, 1H), 3.63 - 3.58 (m, 1H), 3.56 - 3.47 (m, 2H), 3.29 - 3.20 (m, 1H), 3.16 (td, *J* = 13.2, 2.9 Hz, 1H), 3.02 (t, *J* = 12.2 Hz, 1H), 2.87-2.81 (m, 1H), 2.74-2.70 (m, 1H), 2.32-2.28 (m, 1H), 2.22-2.15 (m, 1H).
LCMS (ESI) $[M+H]^+$ = 363.36

Example 18:

Synthesis of Compound B6

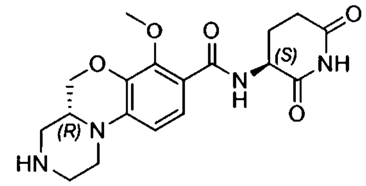

(R)-N-((S)-2,6-Dioxyhesperidin-3-yl)-7-methoxy-1,2,3,4,4a,5-hexahydrobenzo[b]pyrazino[1,2-d][1,4]oxazine-8-carboxamide

Synthetic Scheme:

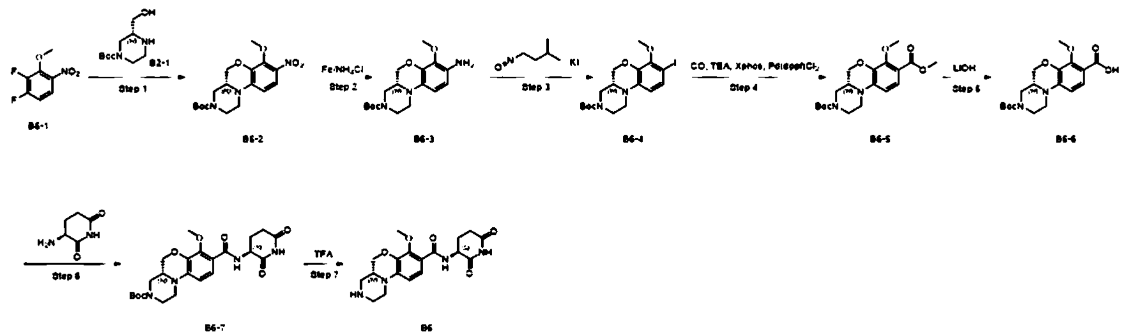


Step 1:

(R)-7-Methoxy-8-nitro-1,2,4a,5-tetrahydrobenzo[b]pyrazino[1,2-d][1,4]oxazine-3(4H)-carboxylic acid tert-butyl ester (Compound B6-2)

The compound B6-1 (2.0 g, 13.87 mmol, 1.0 eq) was dissolved in dimethyl sulfoxide (20 mL). The compound B2-1 (1.75 g, 9.25 mmol, 1.0 eq) and cesium carbonate (9.0 g, 27.6 mmol, 3.0 eq) were added to the reaction system. The reaction mixture was heated to 110°C under nitrogen protection and reacted at this temperature for 3 hours. Water (50 mL) was added to the system, extracted with ethyl acetate (100mL×3 times), washed with saturated saline solution (100mL), combined the organic layers, and dried over anhydrous sodium sulfate. The crude product obtained by concentrating the organic phase was purified by column chromatography to obtain a yellow oily compound B6-2(1.47 g, 43.51%).
LCMS (ESI): $[M+H]^+$= 310.28.

Step 2:

(R)-8-Amino-7-methoxy-1,2,4a,5-tetrahydrobenzo[b]pyrazino[1,2-d][1,4]oxazine-3(4H)-carboxylic acid tert-butyl ester (Compound B6-3)

The compound B6-2 (1.4 g, 3.83 mmol, 1.0 eq) was dissolved in a mixed solvent of isopropanol and water (13 mL). Iron powder (1.07 g, 19.15 mmol, 5.0 eq) and ammonium chloride (2.0 g, 38.3 mmol, 10.0 eq) were added to the reaction system. The mixture was heated to 80°C and reacted for 3 hours. The reaction mixture was filtered, and the filter cake was washed three times with ethyl acetate. The organic layer was concentrated, and the crude product was purified by reverse-phase column chromatography to obtain a yellow solid compound B6-3 (565 mg, 43.96%).
LCMS (ESI): $[M+H]^+$= 336.39.

Step 3:

(R)-8-Iodo-7-methoxy-1,2,4a,5-tetrahydrobenzo[b]pyrazino[1,2-d][1,4]oxazine-3(4H)-carboxylic acid tert-butyl ester (Compound B6-4)

The compound B6-3 (400 mg, 1.19 mmol, 1.0 eq), isopentyl nitrite (279 mg, 2.38 mmol, 2.0 eq), and potassium iodide (594 mg, 3.58 mmol, 3 eq) were dissovled in diiodomethane (5 mL) and reacted at 80°C under a nitrogen atmosphere for 3 hours. The reaction mixture was cooled to room temperature, concentrated under reduced pressure, and purified by column chromatography to obtain a yellow solid compound B6-4 (282 mg, 52.98%).
LCMS (ESI): $[M+H]^+$= 391.19.

Step 4:

3-(tert-Butyl)-8-methyl-(R)-7-methoxy-1,2,4a,5-tetrahydrobenzo[b]pyrazino[1,2-d][1,4]oxazine-3,8(4H)-dicarboxylate (Compound B6-5)

The compound B6-4 (262 mg, 0.587 mmol, 1.0 eq) was dissolved in a mixed solvent of methanol and N,N-dimethylformamide (6 mL). 2-dicyclohexylphosphino-2',4',6'-triisopropylbiphenyl (84 mg, 0.176 mmol, 0.3 eq), [1,1'-Bis(diphenylphosphino)ferrocene]palladium(II) dichloromethane complex (95.8 mg, 0.12 mmol, 0.2 eq), and triethylamine (296.46 mg, 2.94 mmol, 5 eq) were added. The reaction mixture was heated to 90°C and reacted for 12 hours at 90°C under an atmosphere of carbon monoxide. After the mixture was cooled to room temperature, it was concentrated under reduced pressure, then water (100 mL) was added. The residue was extracted with ethyl acetate (100 mL×3), washed with saturated brine (100 mL), combined organic layers, and dried over anhydrous sodium sulfate. The crude product obtained from the concentrated organic phase was purified by column chromatography to obtain a yellow solid compound B6-5(130 mg, 58.52%).
LCMS (ESI) $[M+H]^+$ = 323.28.

Step 5:

(R)-3-(tert-Butoxycarbonyl)-7-methoxy-1,2,3,4,4a,5-hexahydrobenzo[b]pyrazino[1,2-d][1,4]oxazine-8-carboxylic acid (Compound B6-6)

The compound B6-5 (120 mg, 0.317 mmol, 1.0 eq) was dissolved in a mixed solvent of methanol, tetrahydrofuran, and water (9 mL). The reaction mixture was heated to 50°C and reacted at this temperature for 3 hours. The reaction mixture was cooled to 0°C. Acetic acid was added dropwise and the reaction mixture was adjusted to pH 6. The mixture was extracted with ethyl acetate (50 mL×3 times), washed with saturated saline solution (100 mL), combined organic layers, and dried over anhydrous sodium sulfate. The organic layer was concentrated to obtain a brown solid compound B6-6 (100 mg, 86.54%).
LCMS (ESI) $[M+H]^+$= 309.20.

Step 6:

(R)-8-(((S)-2,6-Dioxo-3-hydroxyflavone-3-yl)aminocarbonyl)-7-methoxy-1,2,4a,5-tetrahydrobenzo[b]pyrazine[1,2-d][1,4]oxazine-3(4H)-carboxylic acid tert-butyl ester (Compound B6-7)

The compound B6-6 (90 mg, 0.246 mmol, 1.0 eq) was dissolved in N,N-dimethylformamide (3 mL). (S)-3-Aminopiperidine-2,6-dione (60.98 mg, 0.37 mmol, 1.5 eq) , 2-(7-azobenzotriazole)-N,N,N',N'-tetramethyluronium hexafluorophosphate (187.8 mg, 0.49 mmol, 1.5 eq), and N, N-diisopropyl ethylamine (159.3 mg, 1.235 mmol, 5.0 eq) were

reacted at room temperature for 2 hours. Water (50 mL) was added to the reaction mixture. The mixture was extracted with ethyl acetate (100 mL×3), washed with saturated sodium chloride solution (100 mL), and the combined organic layers were dried over anhydrous sodium sulfate. The crude product obtained from the concentrated organic phase was purified by reverse-phase column chromatography to obtain a white solid compound B6-7 (60 mg, 51.2%).
LCMS (ESI) $[M+H]^+$= 475.33

Step 7:

(R)-N-((S)-2,6-Dioxyhesperidin-3-yl)-7-methoxy-1,2,3,4,4a,5-hexahydrobenzo[b]pyrazino[1,2-d][1,4]oxazine-8-carboxamide (Compound B6)

The compound B6-7 (60 mg, 0.126 mmol, 1.0 eq) and trifluoroacetic acid (0.5 mL) were dissolved in dichloromethane (1.5 mL) and reacted for 2 hours at room temperature. The reaction mixture was concentrated, and the crude product was purified by reverse-phase column chromatography to obtain a white solid compound B6 (18 mg, 38.02%).
$^1$H NMR (600 MHz, DMSO) δ 10.91 (s, 1H), 8.54 (d, J = 7.3 Hz, 1H), 7.34 (d, J = 8.9 Hz, 1H), 6.77 (d, J = 9.0 Hz, 1H), 4.77 - 4.72 (m, 1H), 4.35 (dd, J = 10.9, 2.6 Hz, 1H), 3.94 (t, 1H), 3.87 - 3.79 (m, 4H), 3.23 (s, 2H), 3.17 - 3.06 (m, 3H), 2.84 - 2.73 (m, 3H), 2.15 - 2.03 (m, 3H).
LCMS (ESI) $[M+H]^+$ = 375.39.

Example 19:

Synthesis of Compound B7

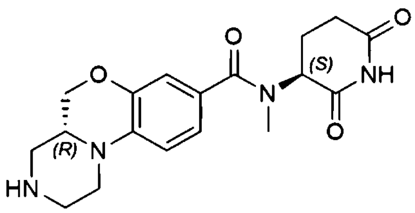

(R)-N-((S)-2,6-Dioxyhesperidin-3-yl)-N-methyl-1,2,3,4,4a,5-hexahydrobenzo[b]pyrazino[1,2-d][1,4]oxazine-8-carboxamide

Synthetic Scheme:

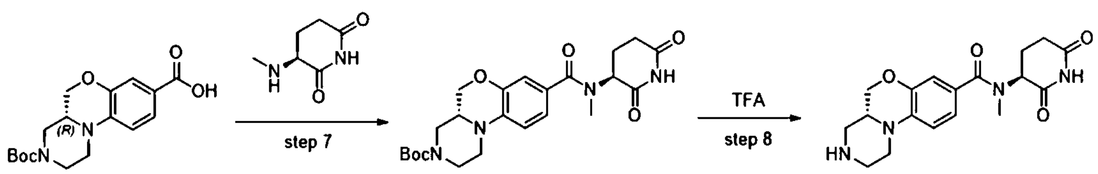


B2-7 B7-1 B7

The synthesis method of compound B7 is the same as that of compound B1.

$^1$H NMR (400 MHz, DMSO-$d_6$) δ 10.90 (d, *J* = 19.7 Hz, 1H), 7.07 - 6.72 (m, 3H), 4.33 (d, *J*= *11.0* Hz, 1H), 3.51 (d, *J* = 10.3 Hz, 1H), 3.39 (dd, *J*= 18.3, 10.7 Hz, 2H), 3.04 (d, *J* = 9.2 Hz, 2H), 2.88 (d, *J* = 6.2 Hz, 2H), 2.74 (d, *J* = 8.6 Hz, 3H), 2.43 - 2.32 (m, 1H), 2.01 - 1.86 (m, 1H).
LCMS (ESI) $[M+H]^+$ = 358.30

Example 20

Synthesis of Compound B8

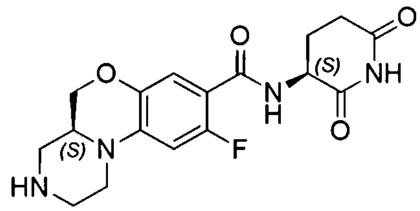

(S)-N-((S)-2,6-Dioxyhesperidin-3-yl)-9-fluoro-1,2,3,4,4,4a,5-hexahydrobenzo[b]pyrazino[1,2-d][1,4]oxazine-8-carboxamide

SyntheticScheme

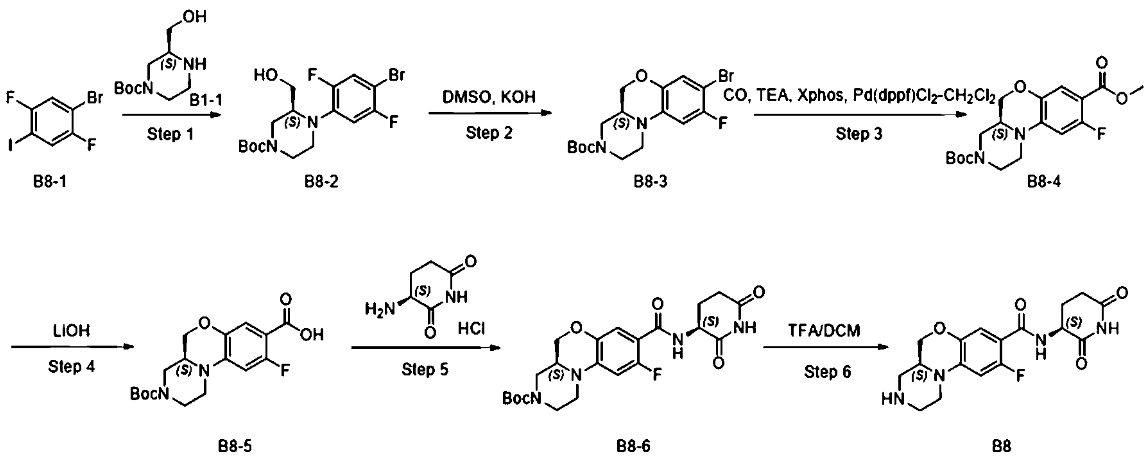


B8-1 B8-2 B8-3 B8-4

B8-5 B8-6 B8

Step 1:

(S)-4-(4-Bromo-2,5-difluorophenyl)-3-(hydroxymethyl)piperazine-1-carboxylic acid tert-butyl ester (Compound B8-2)

The synthesis method follows that of compound B3-2.

Step 2:

(S)-8-Bromo-9-fluoro-1,2,4a,5-tetrahydrobenzo[b]pyrazino[1,2-d][1,4]oxazine-3(4H)-carboxylic acid tert-butyl ester (Compound B8-3)

The compound B8-2 (2.1 g, 5.16 mmol, 1.0 eq) and KOH (0.868 g, 15.47 mmol, 3.0 eq) were dissolved in DMSO (21 mL) and heated to 40°C for 2 hours. Water (30 mL) was added to the mixture, extracted with ethyl acetate (60 mL×3 times), washed with saturated saline solution (60 mL), combined organic layers, and dried over anhydrous sodium sulfate. The organic layer was concentrated to obtain the crude B8-3 (1.49 g, 74.62%). This crude product was used directly in the next reaction without further purification.

LCMS (ESI): $[M\text{-}Bu+H]^+ = 331.29$.

Step 3:

3-(tert-Butyl)-8-methyl-(S)-9-fluoro-1,2,4a,5-tetrahydrobenzo[b]pyrazino[1,2-d][1,4]oxazine-3,8(4H)-dicarboxylate (Compound B8-4)

The compound B8-3 (955 mg, 2.47 mmol, 1.0 eq), 2-Dicyclohexylphosphine-2' ,4' ,6' -triisopropylbiphenyl (352.7 mg, 0.74 mmol, 0.3 eq), [1,1'-Bis(diphenylphosphino)ferrocene]palladium(II) dichloride dichloromethane complex (302 mg, 0.37 mmol, 0.15 eq), and triethylamine (748.6 mg, 7.40 mmol, 3 eq) were dissolved in a mixed solvent of methanol and N,N-dimethylformamide (10 mL) , reacted at 90°C for 12 hours under an atmosphere of carbon monoxide. After the reaction mixture was cooled to room temperature, it was concentrated under reduced pressure, then 100 mL of water was added. The mixture was extracted with ethyl acetate (100 mL×3 times),washed with saturated saline solution (100 mL), combined organic layers, and dried over anhydrous sodium sulfate. The crude product obtained from the concentrated organic phase was purified by column chromatography to obtain a yellow solid compound (425 mg, 47.04%).

LCMS (ESI): $[M-Bu+H]^+$ = 311.38.

Step 4:

(S)-3-(tert-Butoxycarbonyl)-9-fluoro-1,2,3,4,4a,5-hexahydrobenzo[b]pyrazino[1,2-d][1,4]oxazine-8-carboxylic acid (Compound B8-5)

The compound B8-4 (400 mg, 1.09 mmol, 1.0 eq) was dissolved in a mixed solvent of methanol, tetrahydrofuran, and water (5 mL). The reaction mixture was heated to 50°C and reacted at this temperature for 2 hours. The reaction mixture was cooled to 0°C. 1N HCl was added dropwise until the reaction mixture reached pH 3. The mixture was extracted with ethyl acetate (50 mL×3 times), washed with saturated saline solution (100 mL), combined organic layers, and dried over anhydrous sodium sulfate. The organic layer was concentrated, then purified by reverse-phase chromatography to obtain a brown solid compound B8-5 (252 mg, 65.51%).

LCMS (ESI) $[M-Bu+H]^+$ = 297.28.

Step 5:

(S)-8-(((S)-2,6-Dioxyhesperidin-3-yl)aminocarbonyl)-9-fluoro-1,2,4a,5-tetrahydrobenzo[b]pyrazine[1,2-d][1,4]oxazine-3(4H)-carboxylic acid tert-butyl ester (Compound B8-6)

The compound B8-5 (252 mg, 0.715 mmol, 1.0 eq) was dissolved in N,N-dimethylformamide (2 mL), then (S)-3-aminopiperidine-2,6-dione (141.3 mg, 0.858 mmol, 1.2 eq), 1-ethyl-(3-dimethylaminopropyl)carbodiimide hydrochloride (205.6 mg, 1.07 mmol, 1.5 eq), 1-hydroxybenzotriazole (145.0 mg, 1.07 mmol, 1.5 eq), and N,N-diisopropyl ethylamine (277 mg, 2.15 mmol, 3.0 eq) were added. The system was reacted at room temperature for 3 hours. The mixture was concentrated and purified by reverse-phase column chromatography to obtain a white solid compound B8-6 (306 mg, 92.52%).

LCMS (ESI) $[M-Bu+H]^+$ = 407.39.

Step 6:

(S)-N-((S)-2,6-Dioxyhesperidin-3-yl)-9-fluoro-1,2,3,4,4,4a,5-hexahydrobenzo[b]pyrazino[1,2-d][1,4]oxazine-8-carboxamide (Compound B8)

The compound B8-6 (400 mg, 0.865 mmol, 1.0 eq) and trifluoroacetic acid (0.3 mL) were dissolved in dichloromethane (1.5 mL), andreacted for 2 hours at room temperature. The reaction mixture was concentrated, and the crude product was purified by reverse-phase column chromatography to obtain a white solid compound 2 (300 mg, 95.72%).

$^1$H NMR (600 MHz, $CD_3OD$) δ 7.64 (d, *J* = 8.2 Hz, 1H), 7.49 - 7.34 (m, 5H), 6.93 (d, *J*= 8.2 Hz, 1H), 5.15 (dd, *J*= 13.4, 5.2 Hz, 1H), 4.65 (dd, *J* = 12.8, 5.2 Hz, 2H), 4.60 (d, *J*= 5.0 Hz, 2H), 3.86 - 3.78 (m, 2H), 3.18 - 3.13 (m, 2H), 2.99 - 2.87 (m, 1H), 2.82 - 2.75 (m, 2H), 2.70 - 2.65 (m, 2H), 2.47 - 2.40 (m, 2H), 2.31 - 2.23 (m, 2H), 2.18 - 2.12 (m, 2H).

LCMS (ESI) $[M+H]^+$ = 363.39.

Example 21:

Synthesis of Compound B9

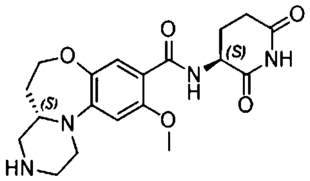

(S)-N-((S)-2,6-Dioxopiperidin-3-yl)-10-methoxy-2,3,4,4a,5,6-hexahydro-1H-benzo[b]pyrazine[1,2-d][1,4]oxazepine-9-carboxamide

Synthetic Scheme

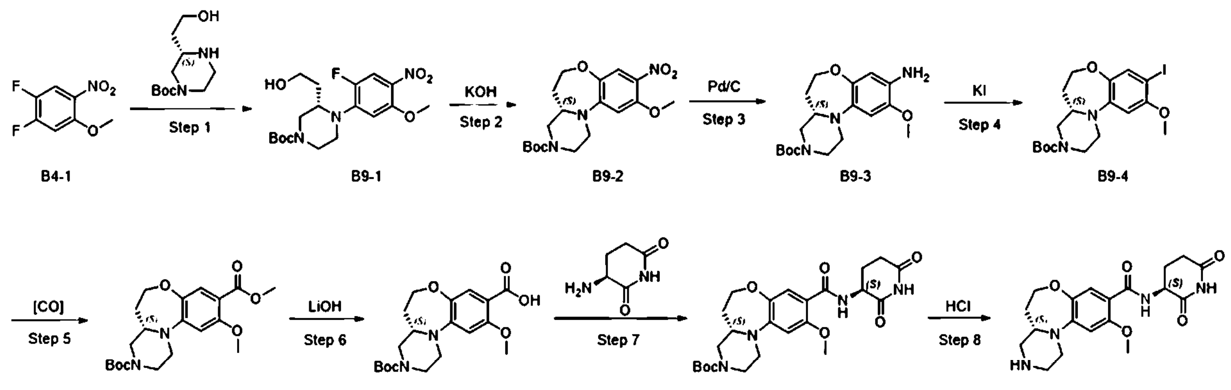


B4-1 B9-1 B9-2 B9-3 B9-4

B9-5 B9-6 B9-7 B9

Step 1:

(*S*)-4-(2-Fluoro-5-methoxy-4-nitrophenyl)-3-(2-hydroxyethyl)piperazine-1-carboxylic acid tert-butyl ester (Compound B9-1)

The compound B4-1 (5.0 g, 26.4 mmol, 1.0 eq) was dissolved in dimethyl sulfoxide (50 mL), followed by addition of (3S)-3-(2-hydroxyethyl)-1-piperazinecarboxylic acid tert-butyl ester (6.1 g, 26.4 mmol, 1.0 eq). The reaction mixture was heated at 100°C for 12 hours. After the reaction mixture was cooled to room temperature, water was added. The mixture was extracted with ethyl acetate, washed with saturated saline solution, and the combined organic layers were dried over anhydrous sodium sulfate. The crude product obtained from the concentrated organic phase was purified by column chromatography to obtain a yellow oily compound B9-1(5.9 g, 56.3%).

$^1$H NMR (400 MHz, DMSO-$d_6$) δ 7.84 (d, $J$ = 13.7 Hz, 1H), 6.77 (d, $J$ = 7.5 Hz, 1H), 4.69 - 4.61 (m, 1H), 4.09 - 3.98 (m, 2H), 3.95 - 3.86 (m, 4H), 3.48 - 3.37 (m, 2H), 3.32 - 2.92 (m, 4H), 1.76 - 1.50 (m, 2H), 1.42 (s, 9H).

LCMS (ESI): [M-tBu+H]$^+$ = 344.39.

Step 2:

(S)-10-methoxy-9-nitro-1,2,4,4a,5,6-hexahydro-3H-benzo[b]pyrazino[1,2-d][1,4]oxazepine-3-carboxylate (Compound B9-2)

The compound B9-1 (5.0 g, 12.5 mmol, 1.0 eq) was dissolved in dimethyl sulfoxide (50 mL), and potassium hydroxide (1.4 g, 25.0 mmol, 2.0 eq) was added at room temperature. The reaction mixture was stirred at 60°C for 2 hours. After the reaction mixture was cooled to room temperature, water was added, and the mixture was extracted with ethyl acetate. The organic layer was washed with saturated brine, combined, and dried over anhydrous sodium sulfate. The crude product obtained from the concentrated organic phase was purified by column chromatography to obtain a yellow solid compound B9-2 (3.5 g, 73.7%).

$^1$H NMR (400 MHz, DMSO-$d_6$) δ 7.43 (s, 1H), 6.59 (s, 1H), 4.25 - 4.16 (m, 1H), 4.16 - 4.09 (m, 1H), 3.88 (s, 3H), 3.79 - 3.72 (m, 1H), 3.67 - 3.58 (m, 2H), 3.56 - 3.38 (m, 4H), 2.07 - 1.98 (m, 1H), 1.98 - 1.87 (m, 1H), 1.43 (s, 9H).

LCMS (ESI): [M+H]$^+$ = 380.35.

Step 3:

(S)-9-Amino-10-methoxy-1,2,4,4a,5,6-hexahydro-3H-benzo[b]pyrazino[1,2-d][1,4]oxazepine-3-carboxylic acid tert-butyl ester (Compound B9-3)

The compound B9-2 (3.0 g, 7.9 mmol, 1.0 eq) was dissolved in methanol (30 mL) and palladium/carbon (600 mg) was added. It was reacted at room temperature under hydrogen gas for 12 hours. The reaction mixture was filtered through diatomaceous earth and concentrated the filtrate under reduced pressure to obtain a crude compound B9-3 (2.2 g).
LCMS (ESI): $[M+H]^+$= 350.39.

Step 4:

(S)-9-Iodo-10-methoxy-1,2,4,4a,5,6-hexahydro-3H-benzo[b]pyrazino[1,2-d][1,4]oxazepine-3-carboxylic acid tert-butyl ester (Compound B9-4)

The compound B9-3 (2.0 g, 5.7 mmol, 1.0 eq) was dissolved in diiodomethane (20 mL). Isoamyl nitrite (1.3 g, 11.5 mmol, 2.0 eq) and potassium iodide (2.9 g, 17.2 mmol, 3.0 eq) was added at room temperature for 15 hours. The reaction mixture was concentrated, and the crude product was purified by column chromatography to obtain a yellow oily compound B9-4 (1.4 g, 53.1%).
$^1$H NMR (600 MHz, DMSO-$d_6$) δ 7.13 (s, 1H), 6.57 (s, 1H), 4.19 - 4.05 (m, 2H), 3.75 (s, 3H), 3.66 - 3.45 (m, 3H), 3.30 - 3.14 (m, 4H), 2.02 - 1.85 (m, 2H), 1.42 (s, 9H).
LCMS (ESI) $[M-tBu+H]^+$= 405.29.

Step 5:

3-(tert-Butyl)-9-methyl-(S)-10-methoxy-1,2,4,4a,5,6-hexahydro-3H-benzo[b]pyrazino[1,2-d][1,4]oxazepine-3,9-dicarboxylate (Compound B9-5)

The compound B9-4 (1.1 g, 2.5 mmol, 1.0 eq) was dissolved in N,N-dimethylformamide: methanol = 2:1 (20 mL). [1,1'-Bis(diphenylphosphino)ferrocene]palladium(II) dichloromethane complex (309 mg, 0.38 mmol, 0.15 eq), 4,5-Bis(diphenylphosphino)-9,9-dimethyloxanthene (437 mg, 0.76 mmol, 0.30 eq), and triethylamine (765 mg, 7.6 mmol, 3.0 eq) were added under pressurized carbon monoxide at 80°C for 12 hours. The reaction mixture was cooled to room temperature, then was concentrated. The resulting crude product was purified by reverse-phase column chromatography to obtain a colorless oily compound B9-5(370 mg, 37.5%).
$^1$H NMR (600 MHz, DMSO-$d_6$) δ 7.15 (s, 1H), 6.55 (s, 1H), 4.18 - 4.05 (m, 2H), 3.76 (s, 3H), 3.70 (s, 3H), 3.67 - 3.50 (m, 3H), 3.42 - 3.37 (m, 2H), 3.30 - 3.18 (m, 2H), 2.01 - 1.85 (m, 2H), 1.43 (s, 9H).
LCMS (ESI) $[M-tBu+H]^+$= 337.19.

Step 6:

(S)-3-(tert-Butoxycarbonyl)-10-methoxy-2,3,4,4a,5,6-hexahydro-1H-benzo[b]pyrazino[1,2-d][1,4]oxazepine-9-carboxylic acid (Compound B9-6)

The compound B9-5 (300 mg, 0.76 mmol, 1.0 eq) was dissolved in tetrahydrofuran:methanol:water = 1:1:2 (4 mL). Lithium hydroxide (321 mg, 7.64 mmol, 10.0 eq) was added, and the mixture was reacted at room temperature for 12 hours. Add dilute Dilute hydrochloric acid (1 N) was added to the reaction mixture and adjusted the pH to ≤5. The reaction mixture was extracted with ethyl acetate, washed with saturated saline solution, combined organic layers, and concentrated under reduced pressure to obtain a crude white solid compound B9-6 (280 mg).
LCMS (ESI) $[M+H]^+$= 379.35.

Step 7:

(S)-9-(((S)-2,6-Dioxyhesperidin-3-yl)aminocarbonyl)-10-methoxy-1,2,4,4a,5,6-hexahydro-3H-benzo[b]pyrazino[1,2-d][1,4]oxazepine-3-carboxylic acid tert-butyl ester (Compound B9-7)

The compound B9-6 (200 mg, 0.53 mmol, 1.0 eq) was dissolved in N,N-dimethylformamide (4 mL). To this solution, (S)-3-Aminopiperidine-2,6-dione hydrochloride (130 mg, 0.79 mmol, 1.5 eq), N,N-diisopropylethylamine (342 mg, 2.6 mmol, 5.0 eq), and 2-(7-azobenzotriazole)-N,N,N',N'-tetramethylurea hexafluorophosphate (301 mg, 0.79 mmol,

1.5 eq) were added and reacted at room temperature for 1 hour. Water was added to the reaction mixture, extracted with ethyl acetate, washed with saturated saline solution, and the combined organic phase was dried over anhydrous sodium sulfate. The crude product obtained from the concentrated organic phase was purified by column chromatography to obtain a white solid compound B9-7(220 mg, 85.2%).

$^1H$ NMR (600 MHz, DMSO-$d_6$) δ 10.90 (s, 1H), 8.51 (d, *J* = 7.1 Hz, 1H), 7.32 (s, 1H), 6.61 (s, 1H), 4.70 (dt, *J* = 12.5, 6.4 Hz, 1H), 4.19 - 4.06 (m, 2H), 3.89 (s, 3H), 3.70 - 3.52 (m, 3H), 3.52 - 3.43 (m, 2H), 3.25 - 3.15 (m, 2H), 2.81 - 2.74 (m, 1H), 2.14 - 2.03 (m, 2H), 1.99 - 1.83 (m, 3H), 1.43 (s, 9H).

LCMS (ESI) $[M+H]^+$= 489.45.

Step 8:

(S)-N-((S)-2,6-Dioxopiperidin-3-yl)-10-methoxy-2,3,4,4a,5,6-hexahydro-1H-benzo[b]pyrazino[1,2-d][1,4]oxazepin-9-carboxamide (Compound B9)

The compound B9-7 (100 mg, 0.20 mmol, 1.0 eq) was dissolved in dichloromethane (1 mL). A solution of hydrochloric acid in 1,4-dioxane (1 mL) was added, and the mixture was reacted at room temperature for 2 hours. The reaction system was concentrated, and the crude product was purified by reverse-phase column chromatography to obtain a white solid compound B9 (50 mg, 62.89%).

$^1H$ NMR (600 MHz, DMSO-$d_6$) δ 10.91 (s, 1H), 8.53 (d, *J* = 7.2 Hz, 1H), 7.37 (s, 1H), 6.72 (s, 1H), 4.71 (dt, *J* = 11.1, 6.9 Hz, 1H), 4.18 - 4.12 (m, 1H), 4.09 (dt, *J* = 11.3, 5.8 Hz, 1H), 3.90 (s, 3H), 3.66 - 3.56 (m, 2H), 3.49 - 3.41 (m, 1H), 3.34 - 3.27 (m, 1H), 3.27 - 3.21 (m, 1H), 3.21 - 3.11 (m, 1H), 3.03 - 2.96 (m, 1H), 2.77 (ddd, *J* = 17.6, 12.1, 7.1 Hz, 1H), 2.55 - 2.52 (m, 1H), 2.13 - 1.92 (m, 4H).

LCMS (ESI) $[M+H]^+$= 389.35.

Example 22

Synthesis of Compound B10

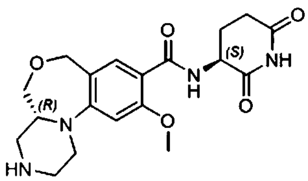

(*R*)-N-((S)-2,6-Dioxopiperidin-3-yl)-10-methoxy-1,2,3,4,4a,5-hexahydro-7H-benzo[e]pyrazino[2,1-c][1,4]oxacyclopropane-9-amide

Synthetic Scheme:

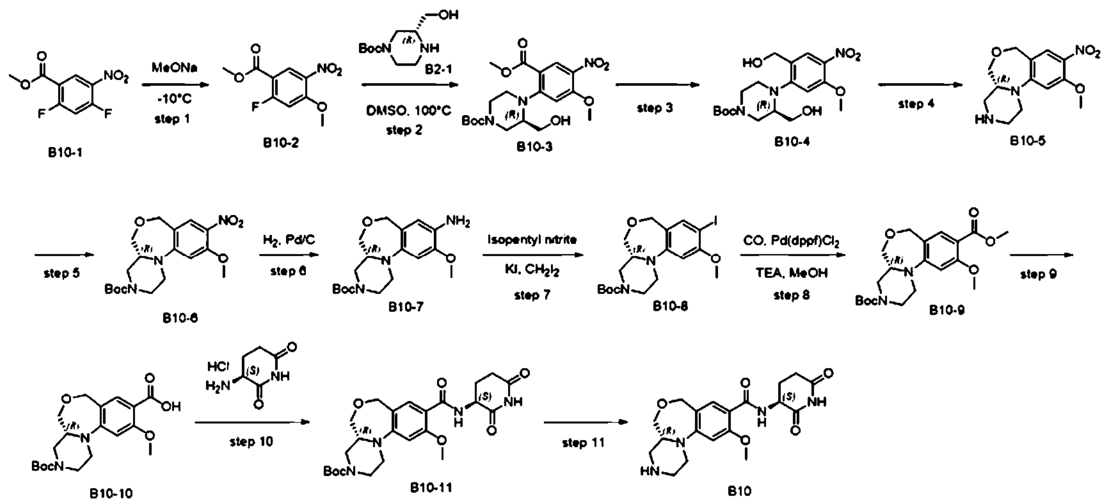


Step 1: Methyl 2-fluoro-4-methoxy-5-nitrobenzoate (Compound B10-2)

The compound B10-1 (10.0 g, 46.06 mmol, 1.0 eq) was dissolved in methanol (200 mL). A solution of sodium methoxide in methanol (5.4 M, 9.4 mL, 50.66 mmol, 1.1 eq) was added to the reaction mixture at -20°C and reacted at this temperature for 2 hours. The reaction mixture was filtered, and the filter cake was washed with carbon tetrachloride to obtain a yellow solid compound B10-2 (10.0 g, 94.75%).

$^1$H NMR (600 MHz, DMSO-$d_6$) δ 8.45 (d, J = 7.5 Hz), 7.47 (d, J = 12.7 Hz), 4.02 (s, 3H), 3.87 (s, 3H).

LCMS (ESI): $[M+H]^+$ = 230.25.

Step 2:

(R)-3-(Hydroxymethyl)-4-(5-methoxy-2-(methoxycarbonyl)-4-nitrophenyl)piperazine-1-carboxylic acid tert-butyl ester (Compound B10-3)

The compound B10-2 (10.0 g, 43.64 mmol, 1.0 eq) and compound B2-1 (11.33 g, 52.36 mmol, 1.2 eq) were dissolved in dimethyl sulfoxide (100 mL) and reacted at 100°C for 12 hours. Water was added to the system. The mixture was extracted with ethyl acetate, washed with saturated saline solution, and the combined organic layers were dried over anhydrous sodium sulfate. The crude product obtained from the concentrated organic phase was purified by reverse phase column chromatography to obtain a yellow solid compound B10-3 (10.0 g, 53.87%).

$^1$H NMR (600 MHz, DMSO-$d_6$) δ 8.32 (s, 1H), 6.69 (s, 1H), 4.65 (t, J = 5.3 Hz, 1H), 4.02-3.96 (m, 5H), 3.82 (s, 3H), 3.61 (s, 1H), 3.48-3.43 (m, 2H), 3.34-3.31 (m, 1H), 3.21 (d, J = 13.1 Hz, 2H), 3.00 (s, 1H), 1.42 (s, 9H).

LCMS (ESI): $[M+H]^+$ = 426.35.

Step 3:

(R)-3-(Hydroxymethyl)-4-(2-(hydroxymethyl)-5-methoxy-4-nitrophenyl)piperazine-1-carboxylic acid tert-butyl ester (Compound B10-4)

The compound B10-3 (10.0 g, 23.51 mmol, 1.0 eq) was dissolved in methanol (200 mL). Sodium borohydride (4.45 g, 117.53 mmol, 5.0 eq) was added at 0°C. The mixture reacted for 5 hours at 22°C under nitrogen protection. The reaction mixture was added to water and extracted with ethyl acetate. The organic layer was washed with saturated saline solution, combined, and dried over anhydrous sodium sulfate. The crude product obtained from the concentrated organic phase was purified by column chromatography to obtain a yellow solid compound B10-4 (7.0 g, 74.93%).

LCMS (ESI): $[M+H]^+$ = 398.35.

Step 4:

(R)-10-Methoxy-9-nitro-1,2,3,4,4a,5-hexahydro-7H-benzo[e]pyrazino[2,1-c][1,4]oxazepine (Compound B10-5)

The B10-4 (10.0 g, 25.16 mmol, 1.0 eq) was dissolved in toluene (100 mL), and (R)-camphorsulfonic acid (11.69 g, 50.32 mmol, 2.0 eq) was added. Reacted at 100°C for 3 hours. The crude product B10-5 (7.0 g) was obtained by vacuum

concentration and used directly in the next step.

LCMS (ESI) $[M+H]^+$ = 280.25.

Step 5:

(R)-10-Methoxy-9-nitro-1,2,4a,5-tetrahydro-7H-benzo[e]pyrazino[2,1-c][1,4]oxazepine-3(4H)-carboxylic acid tert-butyl ester (Compound B10-6)

The compound B10-5 (7.0 g, 25.06 mmol, 1.0 eq) was dissolved in dichloromethane (100 mL). Triethylamine (7.61 g, 75.19 mmol, 3.0 eq) and di-tert-butyl dicarbonate (8.2 g, 37.59 mmol, 1.5 eq) were added. The mixture was reacted at room temperature for 2 hours. The crude product obtained by vacuum concentration was purified by column chromatography to obtain a yellow oily compound B10-6 (8.0 g, 84.13%).

$^1H$ NMR (600 MHz, DMSO-$d_6$) δ 7.87 (s, 1H), 6.62 (s, 1H), 4.77 (d, J = 12.9 Hz, 1H), 4.56 (d, J = 12.9 Hz, 1H), 3.94 (s, 3H), 3.80 (d, J = 13.2 Hz, 1H), 3.74-3.69 (m, 2H), 3.64 (d, J = 11.9 Hz, 1H), 3.59-3.43 (m, 3H), 3.31 (s, 2H), 1.43 (s, 9H).

LCMS (ESI) $[M+H]^+$ = 380.30.

Step 6:

(R)-9-Amino-10-methoxy-1,2,4a,5-tetrahydro-7H-benzo[e]pyrazino[2,1-c][1,4]oxazepine-3(4H)-carboxylic acid tert-butyl ester (Compound B10-7)

The compound B10-6 (8.0 g, 21.09 mmol, 1.0 eq) was dissolved in methanol (100 mL). Palladium carbon (1 g) was added to the reaction mixture, which was reacted at room temperature under hydrogen atmosphere for 12 hours. The reaction mixture was filtered, and the filtrate was concentrated under reduced pressure to obtain a pale gray solid compound B10-7 (6.8 g, 92.09%).

LCMS (ESI) $[M+H]^+$ = 350.35.

Step 7:

(R)-9-Iodo-10-methoxy-1,2,4a,5-tetrahydro-7H-benzo[e]pyrazino[2,1-c][1,4]oxazepine-3(4H)-carboxylic acid tert-butyl ester (Compound B10-8)

The compound B10-7 (1.0 g, 3.44 mmol, 1.0 eq) was dissolved in diiodomethane (10 mL). Isoamyl nitrite (671 mg, 5.72 mmol, 2.0 eq) and potassium iodide (1.43 g, 8.59 mmol, 3.0 eq) were added. The reaction was recovered to room temperature for 16 hours. The reaction mixture was concentrated, and the crude product was purified by column chromatography to afford compound B10-8 (860 mg, 65.28%) as a white solid.

$^1H$ NMR (400 MHz, DMSO-$d_6$) δ 7.53 (s, 1H), 6.57 (s, 1H), 4.56 (d, J = 3.2 Hz, 2H), 3.83 (s, 3H), 3.69 (qd, J = 11.8, 11.3, 3.9 Hz, 3H), 3.53 (dd, J = 13.3, 5.5 Hz, 1H), 3.34-3.22 (m, 4H), 2.98 (s, 1H), 1.42 (s, 9H).

LCMS (ESI) $[M+H]^+$ = 461.30.

Step 8:

3-(tert-butyl)-9-methyl-(R)-10-methoxy-1,2,4a,5-tetrahydro-7H-benzo[e]pyrazino[2,1-c][1,4]oxazepine-3,9(4H)-dicarboxylate (Compound B10-9)

The compound B10-8 (860 mg, 1.87 mmol, 1.0 eq), 1,1-Bis(diphenylphosphino)dimethanedioic acid di(dichloropalladyl) (137 mg, 0.19 mmol, 0.1 eq), and triethylamine (567 mg, 5.6 mmol, 3.0 eq) were dissolved in methanol (10 mL). The mixture was reacted under carbon monoxide gas at 70°C for 12 hours. The reaction mixture was concentrated under reduced pressure. The crude product was purified by column chromatography to obtain a yellow solid compound B10-9 (700 mg, 95.47%).

LCMS (ESI) $[M+H]^+$ = 393.40.

Step 9:

(R)-3-(tert-Butoxycarbonyl)-10-methoxy-1,2,3,4,4a,5-hexahydro-7H-benzo[e]pyrazino[2,1-c][1,4]oxazepine-9-carboxylic acid (Compound B10-10)

The compound B10-9 (700 mg, 1.78 mmol, 1.0 eq) was dissolved in tetrahydrofuran (5 mL) and water (1 mL), and

lithium hydroxide monohydrate (150 mg, 3.57 mmol, 2.0 eq) was added. The mixture was reacted at room temperature for 12 hours. 1 M hydrochloric acid was adjusted to pH 6 and extracted with ethyl acetate,washed with saturated saline solution, combined organic layers, and dried over anhydrous sodium sulfate. The organic phase was concentrated to obtain a crude yellow solid compound B10-10 (670 mg), which was directly used in the next step.

LCMS (ESI) $[M+H]^+$ = 379.35.

Step 10:

(R)-9-(((S)-2,6-Dioxo-3-hydroxyflavone-3-yl)aminocarbonyl)-10-methoxy-1,2,4a,5-tetrahydro-7H-benzo[e]pyrazino [2,1-c] [1,4]oxazepine-3(4H)-carboxylic acid tert-butyl ester (Compound B10-11)

The compound B10-10 (670 mg, 1.77 mmol, 1.0 eq) was dissolved in N,N-dimethylformamide (10 mL), (S)-3-Aminopiperidine-2,6-dione hydrochloride (321 mg, 1.95 mmol, 1.0 eq), N,N-Diisopropylethylamine (687 mg, 5.31 mmol, 3.0 eq), and N,N,N',N'-tetramethyl-O-(7-azabenzotriazol-1-yl) hexafluorophosphorylurea (1.01 g, 2.66 mmol, 1.5 eq) were reacted at room temperature for 1 hour. The reaction mixture was purified by reverse-phase column chromatography to obtain a yellow solid compound B10-11 (500 mg, 57.81%).

LCMS (ESI) $[M+H]^+$ = 489.40.

Step 11:

(R)-N-((S)-2,6-Dioxopiperidin-3-yl)-10-methoxy-1,2,3,4,4a,5-hexahydro-7H-benzo[e]pyrazino[2,1-c][1,4]oxazepine-9-amide (Compound B10)

The compound B10-11 (500 mg, 1.02 mmol, 1.0 eq) was dissolved in dichloromethane: trifluoroacetic acid = 10:1 (5 mL) and was reacted at room temperature for 2 hours. The reaction mixture was concentrated, and the crude product was purified by reverse-phase column chromatography to obtain a white solid compound B10 (297 mg, 68.30%).

$^1$H NMR (600 MHz, DMSO-$d_6$) δ 10.90 (s, 1H), 8.52 (d, *J* = 7.2 Hz, 1H), 7.76 (s, 1H), 6.68 (s, 1H), 4.82 (d, *J* = 11.5 Hz, 1H), 4.73 (dt, *J* = 11.4, 6.9 Hz, 1H), 4.61 (d, *J* = 11.6 Hz, 1H), 3.97 (s, 3H), 3.71 (dd, *J* = 13.7, 2.7 Hz, 1H), 3.61 (dt, *J* = 13.2, 3.2 Hz, 2H), 3.49 (t, *J* = 12.3 Hz, 1H), 3.35 - 3.33 (m, 4H), 3.17 (q, *J* = 11.9, 11.1 Hz, 2H), 2.78 (ddd, *J* = 17.2, 12.4, 6.8 Hz, 1H), 2.53 (s, 1H), 2.16 - 2.05 (m, 2H).

LCMS (ESI) $[M+H]^+$= 389.30.

Example 23

Synthesis of Compound B11

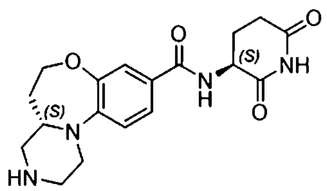

(*S*)-N-((S)-2,6-Dioxopiperidin-3-yl)-2,3,4,4a,5,6-hexahydro-1H-benzo[b]pyrazino[1,2-d][1,4]oxazepine-9-carboxamide

Synthetic scheme

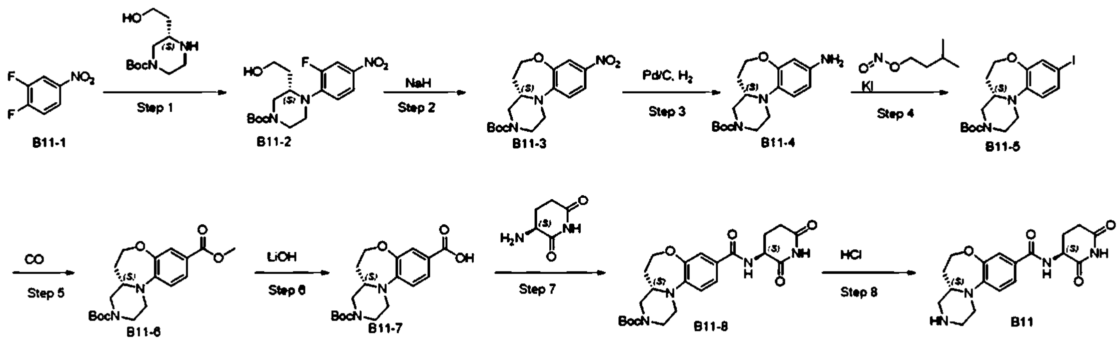


Step 1:

(S)-4-(2-Fluoro-4-nitrophenyl)-3-(2-hydroxyethyl)piperazine-1-carboxylic acid tert-butyl ester (Compound B11-2)

The compound B11-1 (8.69 g, 37.71 mmol, 1.0 eq) was dissolved in dimethyl sulfoxide (20 mL), then (3S)-3-(2-hydroxyethyl)-1-piperazinecarboxylic acid tert-butyl ester (6.0 g, 37.71 mmol, 1.0 eq) was added. The reaction mixture was heated to 100°C and reacted at this temperature for 8 hours. After cooling the reaction system to room temperature, it was added to water and extracted with ethyl acetate. The mixture was washed with saturated saline solution, combineb organic phases, and dried over anhydrous sodium sulfate. The crude product obtained from the concentrated organic phase was purified by column chromatography to obtain a yellow solid compound B11-2(12.0 g, 86.1%).
LCMS (ESI): $[M\text{-}Boc+H]^+$ = 270.27.

Step 2:

(S)-9-Nitro-1,2,4,4a,5,6-hexahydro-3H-benzo[b]pyrazino[1,2-d][1,4]oxazepine-3-carboxylic acid tert-butyl ester (Compound B11-3)

The compound B11-2 (12.0 g, 32.49 mmol, 1.0 eq) was dissolved in N,N-dimethylformamide (50 mL). Sodium hydride (1.69 g, 42.23 mmol, 1.3 eq) was added, and the mixture was reacted at 100°C for 8 hours. After cooling the reaction mixture to room temperature, the reaction was quenched with water and extracted with ethyl acetate. The mixture was washed with saturated saline solution, combined organic layers, and dried over anhydrous sodium sulfate. The crude product obtained from the concentrated organic phase was purified by column chromatography to obtain a yellow solid compound B11-3 (9.0 g, 79.2%).
LCMS (ESI): $[M\text{-}Boc+H]^+$ = 250.37.

Step 3:

(S)-9-Amino-1,2,4,4a,5,6-hexahydro-3H-benzo[b]pyrazino[1,2-d][1,4]oxazepine-3-carboxylic acid tert-butyl ester (Compound B11-4)

The compound B11-3 (9.0 g, 25.76 mmol, 1.0 eq) was dissolved in methanol (120 mL), and palladium/carbon (1.9 g) was added to the system. After three hydrogen purges, reacted for 12 hours at room temperature. The reaction mixture was filtered, and the filter cake was washed three times with methanol (100 mL), and it was concentrated under reduced pressure to obtain a crude compound B11-4 (4.7 g).
LCMS (ESI) $[M\text{-}Boc+H]^+$= 320.38.

Step 4:

(S)-9-Iodo-1,2,4,4a,5,6-hexahydro-3H-benzo[b]pyrazino[1,2-d][1,4]oxazepine-3-carboxylic acid tert-butyl ester (Compound B11-5)

The compound B11-4 (4.7 g, 14.71 mmol, 1.0 eq) was dissolved in diiodomethane (50 mL). Isoamyl nitrite (3.45 g, 29.43 mmol, 2.0 eq) and potassium iodide (7.33 g, 44.14 mmol, 3.0 eq) were added into the solution. The mixture was heated to 80°C and reacted overnight under a nitrogen atmosphere. After cooling to room temperature, the organic layer was concentrated, and the crude product was purified by column chromatography to obtain a yellow oily compound B11-5 (3.5 g, 55.2%).

$^{1}$H NMR (400 MHz, DMSO-*d*6) δ 7.25 - 7.19 (m, 1H), 7.11 - 7.05 (m, 1H), 6.78 - 6.70 (m, 1H), 4.35 - 4.24 (m, 1H), 4.18 - 4.09 (m, 1H), 3.64 - 3.56 (m, 1H), 3.55 - 3.48 (m, 1H), 3.34 - 3.24 (m, 1H), 3.19 - 3.11 (m, 2H), 3.11 - 3.04 (m, 2H), 2.07 - 1.94 (m, 1H), 1.89 - 1.80 (m, 1H), 1.41 (s, 9H).

Step 5:

(S)-1,2,4,4a,5,6-Hexahydro-3H-benzo[b]pyrazino[1,2-d][1,4]oxazepine-3,9-dicarboxylic acid 3-tert-butyl ester (Compound B11-6)

The compound B11-5 (2.1 g, 5.0 mmol, 1.0 eq), [1,1'-Bis(diphenylphosphino)ferrocene]palladium(II) dichloromethane complex (612 mg, 0.749 mmol, 0.15 eq), 4,5-Bis(diphenylphosphino)-9,9-dimethyloxanthene (867 mg, 1.5 mmol, 0.3 eq), and triethylamine (1.52 g, 14.99 mmol, 3.0 eq) were dissolved in methanol (10 mL) and N,N-dimethylformamide (20 mL). After three carbon monoxide displacements, the system was heated to 90°C and reacted overnight. Following cooling to room temperature, the reaction mixture was concentrated under reduced pressure. The crude product obtained was purified by column chromatography to obtain a yellow oily compound B11-6(380 mg, 20.9%).

$^{1}$H NMR (600 MHz, DMSO-*d*6) δ 7.54 - 7.48 (m, 1H), 7.30 (d, *J* = 2.1 Hz, 1H), 6.98 (d, *J* = 8.5 Hz, 1H), 4.23 - 4.19 (m, 2H), 3.78 (s, 3H), 3.58 - 3.51 (m, 2H), 3.53 - 3.47 (m, 1H), 3.43 - 3.36 (m, 1H), 3.32 - 3.25 (m, 3H), 2.06 - 1.97 (m, 1H), 1.93 - 1.84 (m, 1H), 1.42 (s, 9H). LCMS (ESI) $[M\text{-}Boc+H]^+$= 263.37.

Step 6:

(S)-3-(tert-Butoxycarbonyl)-2,3,4,4a,5,6-hexahydro-1H-benzo[b]pyrazino[1,2-d][1,4]oxazepine-9-carboxylic acid (Compound B11-7)

The compound B11-6 (380 mg, 1.05 mmol, 1.0 eq) and lithium hydroxide monohydrate (0.307 g, 7.34 mmol, 7.0 eq) were dissolved in methanol (2 mL), tetrahydrofuran (2 mL), and water (1 mL). The system reacted overnight at room temperature. After vacuum concentration, the pH of the reaction mixture was adjusted to 7 using dilute hydrochloric acid. The mixture was filtered, and the filter cake was washed three times with water (5 mL). The crude white solid compound B11-7 (300 mg) was obtained.

LCMS (ESI) $[M\text{-}Boc+H]^+$= 249.17.

Step 7:

((S)-9-(((S)-2,6-Dioxopiperidin-3-yl)aminocarbonyl)-1,2,4,4a,5,6-hexahydro-3H-benzo[b]pyrazino[1,2-d] [1,4]oxazepine-3-carboxylic acid tert-butyl ester (Compound B11-8)

The compound B11-7 (300 mg, 0.861 mmol, 1.0 eq), 2-(7-azobenzotriazole)-N,N,N',N'-tetramethylurea hexafluorophosphate (360 mg, 0.947 mmol, 1.1 eq) and N,N-diisopropylethylamine (333 mg, 2.58 mmol, 3.0 eq) were dissolved in N,N-dimethylformamide (10 mL). The reaction mixture was reacted at room temperature for 15 minutes. (S)-3-Aminopiperidine-2,6-dione hydrochloride (170 mg, 1.03 mmol, 1.2 eq) was added to the system. The mixture reacted overnight at room temperature, was added to water, and extracted with ethyl acetate. The extract was washed with saturated saline solution, the organic phases were combined, and dried over anhydrous sodium sulfate. The crude product obtained from the concentrated organic phase was purified by column chromatography to obtain a blue solid compound B11-8 (350 mg, 88.6%).

$^{1}$H NMR (600 MHz, DMSO-*d*6) δ 10.84 (s, 1H), 8.55 (d, *J* = 8.4 Hz, 1H), 7.50 - 7.45 (m, 1H), 7.34 (d, *J* = 2.1 Hz, 1H), 7.00 (d, *J* = 8.4 Hz, 1H), 4.77 - 4.70 (m, 1H), 4.30 - 4.23 (m, 1H), 4.22 - 4.16 (m, 1H), 3.66 - 3.59 (m, 1H), 3.58 - 3.53 (m, 1H), 3.43 - 3.53 (m, 2H), 3.26 - 3.20 (m, 2H), 2.83 - 2.75 (m, 1H), 2.57 - 2.50 (m, 1H), 2.14 - 1.98 (m, 2H), 1.95 - 1.84 (m, 2H), 1.43 (s, 9H).

LCMS (ESI) $[M+H]^+$= 459.40.

Step 8:

(S)-N-((S)-2,6-Dioxopiperidin-3-yl)-2,3,4,4a,5,6-hexahydro-1H-benzo[b]pyrazino[1,2-d][1,4]oxazepine-9-carboxamide (Compound B11)

The compound B11-8 (100 mg, 0.218 mmol, 1.0 eq) was dissolved in a solution of hydrochloric acid (1 mL, 4 N) in 1,4-dioxane and dichloromethane (1 mL). The reaction mixture reacted overnight at room temperature. The crude product obtained from the concentrated reaction system was purified by reverse phase column chromatography to obtain a white

solid compound B11 (30 mg, 38.38%).

$^1H$ NMR (400 MHz, DMSO-*d*6) δ 10.86 (s, 1H), 8.62 (d, *J* = 8.4 Hz, 1H), 7.57 - 7.49 (m, 1H), 7.38 (d, *J* = 2.1 Hz, 1H), 7.09 (d, *J* = 8.4 Hz, 1H), 4.79 - 4.68 (m, 1H), 4.41 - 4.31 (m, 1H), 4.19 - 4.09 (m, 1H), 3.47 - 3.40 (m, 1H), 3.41 - 3.30 (m, 3H), 3.27 - 3.18 (m, 1H), 3.16 (s, 1H), 3.11 - 2.98 (m, 1H), 2.85 - 2.71 (m, 1H), 2.57 - 2.53 (m, 1H), 2.17 - 2.00 (m, 2H), 1.98 - 1.86 (m, 2H).

LCMS (ESI) $[M+H]^+$= 359.38.

Example 24

Synthesis of Compound B12

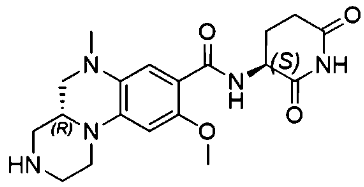

(R) -N-((S)-2,6-Dioxyhesperidin-3-yl)-9-methoxy-6-methyl-2,3,4,4a,5,6-hexahydro-1H-pyrazino[1,2-a]quinoline-8-carboxamide

Synthetic scheme

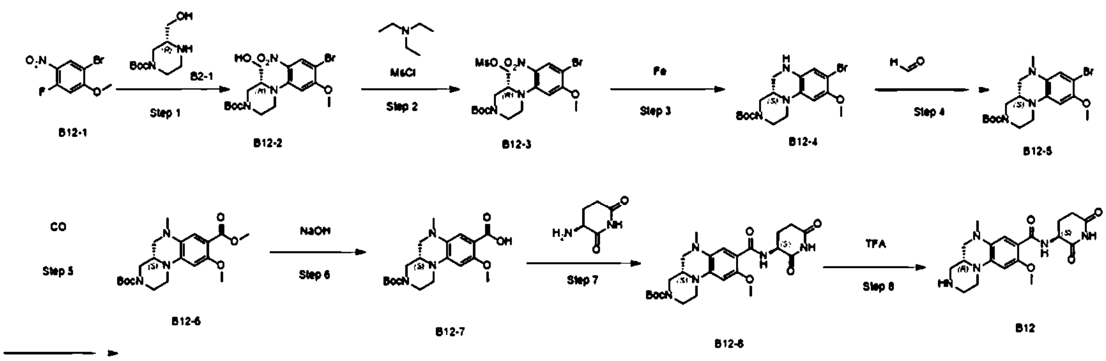


Step 1:

(R)-4-(4-Bromo-5-methoxy-2-nitrophenyl)-3-(hydroxymethyl)piperazine-1-carboxylic acid tert-butyl ester (Compound B12-2) was prepared according to Step 1 of Compound B5.

LCMS (ESI): $[M-Boc]^+$ = 390.30.

Step 2:

(R)-4-(4-Bromo-5-methoxy-2-nitrophenyl)-3-(((methylsulfonyl)oxy)methyl)piperazine-1-carboxylic acid tert-butyl ester (Compound B12-3)

The compound B12-2 (2.0 g, 4.48 mmol, 1.0 eq) and triethylamine (907 mg, 8.96 mmol, 2.0 eq) were dissolved in dichloromethane (30 mL). The mixture was cooled to 0°C and methyl sulfonyl chloride (770 mg, 6.72 mmol, 1.5 eq) was added dropwise into the mixture. The reaction was carried out at room temperature under nitrogen for 2 hours. A saturated aqueous ammonium chloride solution (50 mL) was added, and the mixture was extracted with dichloromethane (100 mL×3). The organic layers were combined and dried over anhydrous sodium sulfate. The crude product obtained from the concentrated organic phase was purified by column chromatography to obtain a yellow solid compound B5-3 (2.3 g,

97.88%).

LCMS (ESI): $[M\text{-}Boc]^+$ = 468.38.

Step 3:

(S)-8-Bromo-9-methoxy-1,2,4,4a,5,6-hexahydro-3H-pyrazino[1,2-a]quinoline-3-carboxylic acid tert-butyl ester (Compound B12-4)

The compound B12-3 (2.3 g, 4.39 mmol, 1.0 eq) and ammonium chloride (2.35 g, 43.86 mmol, 10 eq) were dissolved in ethanol/water 4:1 (50 mL). Iron powder (1.47 g, 26.32 mmol, 6 eq) was added at room temperature. The mixture was reacted overnight at 80°C under nitrogen, was cooled to room temperature, and water (150 mL) was added, then the mixture was extracted with ethyl acetate (100 mL×3), combined organic layers, and dried over anhydrous sodium sulfate. The crude product obtained from the concentrated organic phase was purified by column chromatography to afford the yellow solid compound B5-3 (800 mg, 45.79%).

LCMS (ESI): $[M\text{-}Boc]^+$ = 342.30

Step 4:

(S)-8-Bromo-9-methoxy-6-methyl-1,2,4,4a,5,6-hexahydro-3H-pyrazino[1,2-a]quinoxaline-3-carboxylic acid tert-butyl ester (Compound B12-5)

The compound B12-4 (800 mg, 2.01 mmol, 1.0 eq) and 37% formaldehyde aqueous solution (245 g, 3.01 mmol, 1.5 eq) were dissolved in methanol (20 mL). Sodium cyano borohydride (252 mg, 4.02 mmol, 2 eq) was added at room temperature. The reaction was allowed to proceed for 2 hours. Water (100 mL) was added to the reaction mixture, which was extracted with ethyl acetate (100 mL×3 times). The combined organic layers were dried over anhydrous sodium sulfate. The crude product obtained from the concentrated organic phase was purified by column chromatography to obtain a white solid compound B5-3 (500 mg, 60.37%).

LCMS (ESI): $[M\text{-}Boc]^+$ = 356.33.

Step 5:

3-(tert-Butyl)-8-methyl-(S)-9-methoxy-6-methyl-1,2,4,4a,5,6-hexahydro-3H-pyrazino[1,2-a]quinoline-3,8-dicarboxylate (Compound B12-6) was prepared according to Step 4 of Compound A8.

LCMS (ESI): $[M\text{-}Boc]^+$ = 335.47.

Step 6:

(S)-3-(tert-Butoxycarbonyl)-9-methoxy-6-methyl-2,3,4,4a,5,6-hexahydro-1H-pyrazino[1,2-a]quinoline-8-carboxylic acid (Compound B12-7) was prepared according to Step 5 of Compound A8.

LCMS (ESI) $[M\text{-}Boc]^+$ = 321.44.

Step 7:

(S)-8-(((S)-2,6-Dioxyhesperidin-3-yl)aminocarbonyl)-9-methoxy-6-methyl-1,2,4,4a, 5,6-Hexahydro-3H-pyrazino[1,2-a] quinoxaline-3-carboxylic acid tert-butyl ester (Compound B12-68)was prepared according to Step 6 of Compound A8.

LCMS (ESI) $[M\text{-}Boc]^+$ = 431.56.

Step 8:

(R)-N-((S)-2,6-Dioxyhesperidin-3-yl)-9-methoxy-6-methyl-2,3,4,4a,5,6-hexahydro-1H-pyrazino[1,2-a]quinoline-8-carboxamide (Compound B12) was prepared according to Step 7 of Compound A8.

$^1$H NMR (400 MHz, Methanol-$d_4$) δ 7.31 (s, 1H), 6.59 (s, 1H), 4.77 (dd, *J* = 12.7, 5.2 Hz, 1H), 4.30 (s, 1H), 3.98 (s, 3H), 3.70 (s, 1H), 3.53 - 3.40 (m, 2H), 3.45-3.31 (m, 2H)3.23 (s, 2H), 3.05 *(t, J* = 12.0 Hz, 2H), 2.92-2.79 (m, 3H), 2.75-2.69 (m, 1H), 2.48-2.41 (m, 1H), 2.13-2.02 (m, 1H).

LCMS (ESI) $[M+H]^+$= 332.44

Example 25:

Synthesis of Compound C1

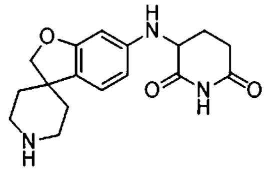

3-(2H-Spiro[benzofuran-3,4'-piperidine]-6-yl)amino)piperidine-2,6-dione

Synthetic scheme:

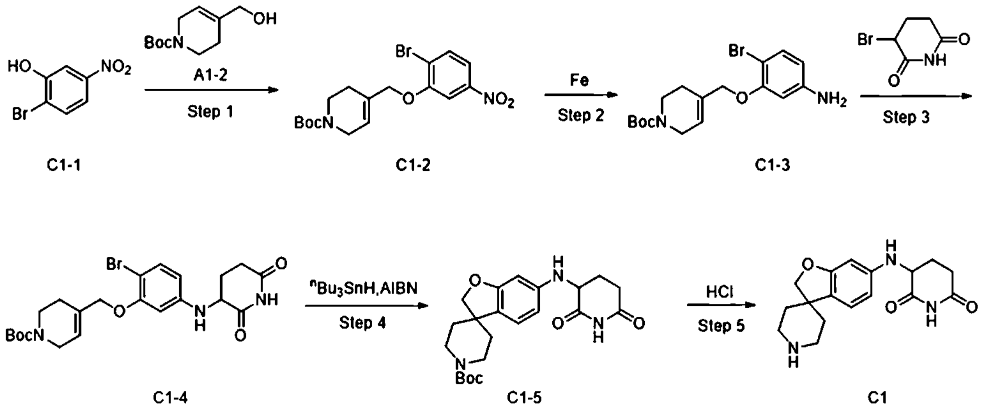


Step 1:

4-((2-Bromo-5-nitrophenoxy)methyl)-3,6-dihydropyridine-1(2H)-carboxylicacidtert-butyl ester (Compound C1-2)

The compound C1-1 (5.0 g, 22.9 mmol, 1.0 eq), compound A1-2 (6.4 g, 29.8 mmol, 1.3 eq), and triphenylphosphine (12.0 g, 45.9 mmol, 2.0 eq) were dissolved in tetrahydrofuran (50 mL) and stirred at 0°C under nitrogen for 15min. Diethyl azodicarboxylate (8.0 g, 45.9 mmol, 2.0 eq) was slowly added dropwise to the reaction mixture, which was then allowed to react at room temperature for 12 h. The reaction mixture was concentrated, and the crude product was purified by column chromatography to obtain a colorless oily compound C1-2 (8.0 g, 84.4%).

$^1$H NMR (600 MHz, DMSO-$d_6$) δ 7.92 (d, *J* = 8.6 Hz, 1H), 7.87 (d, *J* = 2.5 Hz, 1H), 7.77 (dd, *J* = 8.7, 2.5 Hz, 1H), 5.91 (s, 1H), 4.74 (d, *J* = 2.1 Hz, 2H), 3.45 (d, *J* = 5.9 Hz, 2H), 3.34 (s, 2H), 2.15 (tt, *J* = 4.8, 2.5 Hz, 2H), 1.41 (s, 9H).

LCMS (ESI): $[M\text{-}Boc+H]^+$ = 313.16.

Step 2:

4-((5-Amino-2-bromophenoxy)methyl)-3,6-dihydropyridine-1(2H)-carboxylicacidtert-butyl ester (Compound C1-3)

The compound C1-2 (5.0 g, 12.1 mmol, 1.0 eq) was dissolved in ethanol:ammonium chloride solution = 5:1 (60 mL). Iron powder (6.8 g, 121.0 mmol, 10.0 eq) was added, and the reaction mixture was stirred at 80°C for 2 hours. Water was added to the reaction mixture, and the mixture was extracted three times with ethyl acetate, combined the organic layers, dried over anhydrous sodium sulfate, and concentrated to obtain a crude compound C1-3 (4.0 g).

$^1$H NMR (600 MHz, DMSO-$d_6$) δ 7.10 (d, *J* = 8.5 Hz, 1H), 6.31 (d, *J* = 2.4 Hz, 1H), 6.11 (dd, *J* = 8.5, 2.4 Hz, 1H), 5.82 (s, 1H), 5.28 (s, 2H), 4.40 (s, 2H), 3.51 - 3.43 (m, 2H), 2.17 - 2.10 (m, 2H), 1.41 (s, 9H).

LCMS (ESI): $[M-Boc+H]^+$ = 283.15.

Step 3:

4-((2-Bromo-5-((2,6-dioxopiperidin-3-yl)amino)phenoxy)methyl)-3,6-dihydropyridine-1(2H)-carboxylic acid tert-butyl ester (Compound C1-4)

The compound C1-5 (800 mg, 2.1 mmol, 1.0 eq) was dissolved in N,N-dimethylformamide (5 mL). 3-Bromopiperidine-2,6-dione (481 mg, 2.5 mmol, 1.2 eq) and sodium bicarbonate (350 mg, 4.2 mmol, 2.0 eq) were added and the mixture was reacted at 80°C for 12 hours. After cooling the reaction mixture to room temperature, water was added. The mixture was extracted with ethyl acetate, washed with saturated saline solution, and the combined organic phase was dried over anhydrous sodium sulfate. The crude product obtained from the concentrated organic phase was purified by column chromatography to obtain a light green solid compound C1-4 (565 mg, 54.7%).

$^1H$ NMR (600 MHz, DMSO-$d_6$) δ 10.82 (s, 1H), 7.18 (d, *J* = 8.7 Hz, 1H), 6.23 (dd, *J* = 8.7, 2.5 Hz, 1H), 6.08 (d, *J* = 7.9 Hz, 1H), 5.84 (s, 1H), 4.45 (s, 2H), 4.37 (ddd, *J* = 12.2, 7.9, 4.9 Hz, 1H), 3.86 (s, 2H), 3.45 (s, 2H), 2.78 - 2.70 (m, 1H), 2.63 - 2.55 (m, 1H), 2.18 - 2.05 (m, 3H), 1.87 (qd, *J* = 12.4, 4.7 Hz, 1H), 1.41 (s, 9H).

LCMS (ESI): $[M-Boc+H]^+$ = 396.29.

Step 4:

6-((2,6-Dioxopiperidin-3-yl)amino)-2H-spiro[benzofuran-3,4'-piperidine]-1'-carboxylic acid tert-butyl ester (Compound C1-5)

The compound C1-4 (565 mg, 1.1 mmol, 1.0 eq) was dissolved in toluene (5 mL). Azobis(isobutyronitrile) (375 mg, 2.3 mmol, 2.0 eq) and tributylstannyl hydride (998 mg, 3.4 mmol, 3.0 eq) were added at room temperature, and the mixture was reacted at 110°C for 15 hours. The reaction mixture was concentrated, and the crude product was purified by column chromatography to obtain a white oily compound C1-5 (220 mg, 46.3%).

$^1H$ NMR (600 MHz, DMSO-$d_6$) δ 10.78 (s, 1H), 6.91 (d, *J*= 8.1 Hz, 1H), 6.18 (dd, *J* = 8.1, 2.0 Hz, 1H), 6.14 (d, *J* = 2.0 Hz, 1H), 5.77 (d, *J* = 7.6 Hz, 1H), 4.32 (s, 2H), 4.26 (ddd, *J* = 12.0, 7.6, 4.8 Hz, 1H), 3.91 - 3.79 (m, 2H), 2.74 (ddd, *J* = 17.5, 12.1, 5.4 Hz, 1H), 2.56 (dt, *J* = 17.4, 4.2 Hz, 1H), 2.08 (dq, *J* = 13.6, 4.8 Hz, 1H), 1.84 (qd, *J* = 12.2, 4.6 Hz, 1H), 1.69 - 1.60 (m, 2H), 1.60 - 1.53 (m, 2H), 1.42 (s, 9H).

LCMS (ESI) $[M+H]^+$= 416.35.

Step 5:

3-(2H-Spiro[benzofuran-3,4'-piperidine]-6-yl)amino)piperidine-2,6-dione (Compound C1)

The compound C1-6 (110 mg, 0.26 mmol, 1.0 eq) was dissolved in dichloromethane (1 mL). A solution of hydrochloric acid in 1,4-dioxane (1 mL) was added, and the mixture was reacted at room temperature for 2 h. The reaction mixture was concentrated, and the crude product was purified by reverse-phase column chromatography to obtain a white solid compound C1 (90 mg, 96.6%).

$^1H$ NMR (600 MHz, DMSO-$d_6$) δ 10.83 (s, 1H), 6.86 (dd, *J* = 8.1, 1.5 Hz, 1H), 6.32 - 6.26 (m, 1H), 6.27 - 6.21 (m, 1H), 4.39 (s, 2H), 4.37 - 4.29 (m, 1H), 3.30 - 3.21 (m, 2H), 3.01 - 2.88 (m, 2H), 2.73 (ddd, *J* = 17.7, 12.2, 5.4 Hz, 1H), 2.57 (dt, *J* = 17.5, 4.2 Hz, 1H), 2.13 - 2.00 (m, 3H), 1.93 - 1.82 (m, 1H), 1.79 - 1.71 (m, 2H).

LCMS (ESI) $[M+H]^+$= 316.35.

Example 26

Synthesis of Compound C2

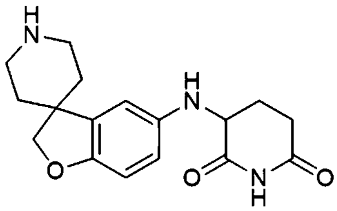

3-((2H-spiro[benzofuran-3,4'-piperidine]-5-yl)amino)piperidine-2,6-dione

Synthetic scheme

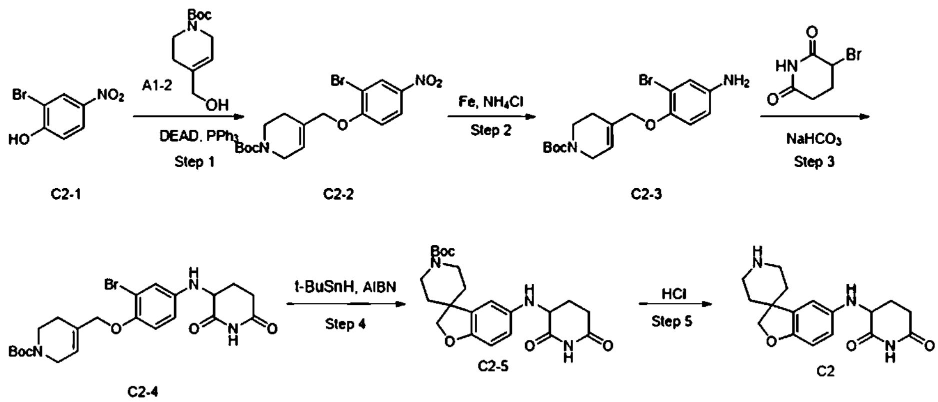


Step 1:

4-((2-Bromo-4-nitrophenoxy)methyl)-3,6-dihydropyridine-1(2H)-carboxylic acid tert-butyl ester (Compound C2-2)

The compound C2-1 (10.0 g, 45.87 mmol, 1.0 eq), A1-2 (12.72 g, 59.63 mmol, 1.3 eq), and triphenylphosphine (24.06 g, 91.74 mmol, 2.0 eq) were dissolved in tetrahydrofuran (250 mL). The mixture was cooled to 0°C, and diethyl azodicarboxylate (15.98 g, 91.74 mmol, 2.0 eq) was added. After completion of the addition, the mixture was allowed to warm to room temperature and reacted for 12 hours. The reaction mixture was concentrated under reduced pressure. Water was added to the system, followed by extraction with ethyl acetate. The extract was washed with saturated saline solution. The combined organic phases were dried over anhydrous sodium sulfate. The crude product obtained from the concentrated organic phase was purified by column chromatography to obtain a yellow solid compound C2-2 (18.0 g, 94.95%).

$^1$H NMR (600 MHz, DMSO-$d_6$) δ 8.41- 8.46 (m, 1H), 8.29 - 8.24 (m, 1H), 7.35 (d, *J* = 9.2 Hz, 1H), 5.89 (s, 1H), 4.75 (s, 2H), 3.87 (s, 2H), 3.45 (d, *J* = 5.9 Hz, 2H), 2.17 - 2.12 (m, 2H), 1.40 (s, 9H).

LCMS (ESI): $[M\text{-}Boc+H]^+$ = 313.08.

Step 2:

4-((4-Amino-2-bromophenoxy)methyl)-3,6-dihydropyridine-1(2H)-carboxylic acid tert-butyl ester (Compound C2-3)

The compound C2-2 (18.0 g, 43.56 mmol, 1.0 eq), ammonium chloride (11.65 g, 217.78 mmol, 5.0 eq), and iron powder (12.16 g, 217.78 mmol, 5.0 eq) were dissolved in ethanol (100 mL) and water (20 mL). The reaction mixture was heated to 60°C and reacted for 1 h. The reaction mixture was filtered, concentrated under reduced pressure, and water was added. The solution was extracted with ethyl acetate. The extract was washed with saturated brine, the organic phases were combined, and the mixture was dried over anhydrous sodium sulfate. The crude product obtained from the concentrated organic phase was purified by column chromatography to obtain a yellow oily compound C2-3 (16.0 g, 95.54%).

$^1$H NMR (600 MHz, DMSO-$d_6$) δ 6.83 (d, *J* = 8.7 Hz, 1H), 6.80 (d, *J* = 2.7 Hz, 1H), 6.53 - 6.48 (m, 1H), 5.77 (s, 1H), 4.91 (s, 2H), 4.35 (d, *J* = 2.1 Hz, 2H), 3.84 (s, 2H), 3.43 *(t, J* = 5.7 Hz, 2H), 2.15 - 2.10 (m, 2H), 1.41 (s, 9H).

LCMS (ESI): $[M\text{-}Boc+H]^+$ = 283.18.

Step 3:

4-((2-Bromo-4-((2,6-dioxopiperidin-3-yl)amino)phenoxy)methyl)-3,6-dihydropyridine-1(2H)-carboxylic acid tert-butyl ester (Compound C2-4)

Compound C2-3 (10.0 g, 26.09 mmol, 1.0 eq), 3-bromopiperidine-2,6-dione (6.01 g, 31.31 mmol, 1.2 eq), and sodium bicarbonate (4.38 g, 52.18 mmol, 2.0 eq) were dissolved in N,N-dimethylformamide (100 mL) and reacted at 80°C for 16 h under nitrogen protection. After cooling the reaction mixture to room temperature, the solution was concentrated under reduced pressure, then water was added and extracted with ethyl acetate. The extract was washed with saturated saline solution, the organic phases were combined, and the mixture was dried over anhydrous sodium sulfate. The crude product obtained from the concentrated organic phase was purified by column chromatography to obtain a blue oily compound C2-4 (10.0 g, 77.5%).

$^1$H NMR (400 MHz, DMSO-$d_6$) δ 10.78 (s, 1H), 6.94 - 6.89 (m, 2H), 6.67 - 6.60 (m, 1H), 5.79 (s, 1H), 5.77 - 5.74 (m, 1H), 4.39 (s, 2H), 4.32 - 4.22 (m, 1H), 3.84 (s, 2H), 3.47 - 3.40 (m, 2H), 2.79 - 2.66 (m, 1H), 2.62 - 2.51 (m, 1H), 2.14 (s, 2H), 2.10 - 2.01 (m, 1H), 1.92 - 1.77 (m, 1H), 1.41 (s, 9H).

LCMS (ESI): $[M\text{-}Boc+H]^+$ = 394.39.

Step 4:

5-((2,6-Dioxopiperidin-3-yl)amino)-2H-spiro[benzofuran-3,4'-piperidine]-1'-carboxylic acid tert-butyl ester (Compound C2-5)

The compound C2-4 (10.0 g, 20.23 mmol, 1.0 eq), tri-n-butylstannyl hydride (17.66 g, 60.68 mmol, 3.0 eq), and azobis(isobutyronitrile) (6.64 g, 40.45 mmol, 2.0 eq) were dissolved in toluene (100 mL) and reacted at 110°C for 12 h under nitrogen protection. After cooling the reaction mixture to room temperature, the solution was concentrated under reduced pressure, then water was added and extracted with ethyl acetate. The extract was washed with saturated saline solution, the organic phases were combined, and the mixture was dried over anhydrous sodium sulfate. The crude product obtained from the concentrated organic phase was purified by column chromatography to obtain a yellow solid compound C2-5 (4.0 g, 47.6%).

$^1$H NMR (600 MHz, DMSO-$d_6$) δ 10.76 (s, 1H), 6.64 - 6.59 (m, 1H), 6.56 - 6.51 (m, 1H), 6.47 - 6.43 (m, 1H), 5.37 (d, *J*= 7.2 Hz, 1H), 4.30 (s, 2H), 4.25 - 4.17 (m, 1H), 3.89 (s, 2H), 3.34 (s, 2H), 2.77 - 2.68 (m, 1H), 2.61 - 2.54 (m, 1H), 2.15 - 2.07 (m, 1H), 1.87 - 1.77 (m, 1H), 1.72 - 1.64 (m, 2H), 1.63 - 1.57 (m, 2H), 1.42 (s, 9H).

LCMS (ESI) $[M\text{-}Boc+H]^+$= 316.28.

Step 5:

3-((2H-spiro[benzofuran-3,4'-piperidine]-5-yl)amino)piperidine-2,6-dione (Compound C2)

The compound C2-5 (100 mg, 0.24 mmol, 1.0 eq) was dissolved in a solution of hydrochloric acid (4N, 1 mL) and 1,4-dioxane and dichloromethane (1 mL). The reaction mixture was reacted overnight at room temperature. The crude product obtained by concentrating the reaction system was purified by reverse-phase column chromatography to obtain a white solid compound C2 (0.07 g, 92.22%).

$^1$H NMR (600 MHz, DMSO-$d_6$) δ 10.87 (s, 1H), 6.66 - 6.61 (m, 2H), 6.60 - 6.57 (m, 1H), 5.40 (s, 1H),4.39 (s, 2H), 4.27 - 4.21 (m, 1H), 3.35 - 3.29 (m, 2H), 3.04 - 2.95 (m, 2H), 2.75 - 2.66 (m, 1H), 2.63 - 2.56 (m, 1H), 2.12 - 2.04 (m, 1H), 2.01 - 1.92 (m, 2H), 1.91 - 1.83 (m, 1H), 1.82 - 1.77 (m, 2H).
LCMS (ESI) $[M+H]^+$= 316.30

Example 27

Synthesis of Compound C3

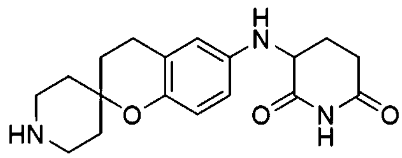

3-(Spiro[benzopyran-2,4'-piperidine]-6-ylamino)piperidine-2,6-dione

Synthetic Scheme

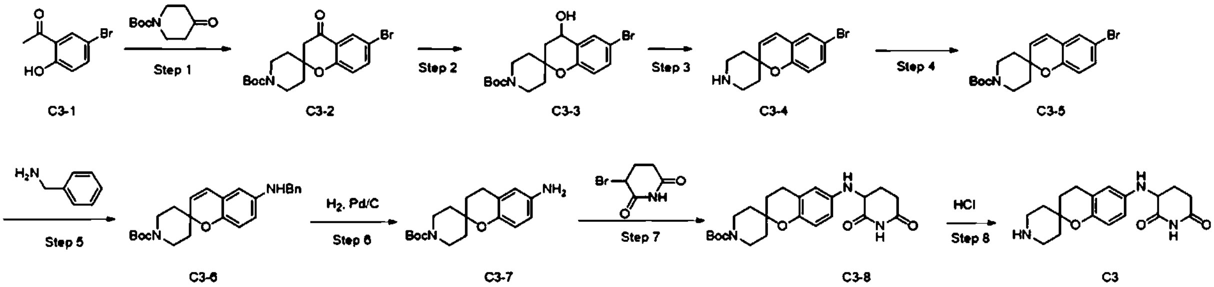


Step 1: 6-Bromo-4-oxo-spiro[semen-2,4'-piperidine]-1'-carboxylic acid tert-butyl ester (Compound C3-2)

Refer to Step 1 of Compound A5 for preparation.

Step 2: 6-Bromo-4-hydroxy-spiro[semen-2,4'-piperidine]-1'-carboxylic acid tert-butyl ester (Compound C3-3)

Refer to Step 2 of Compound A5 for preparation.

Step 3: 6-Bromo-spiro[chromene-2,4'-piperidine] (Compound C3-4)

Refer to Step 3 of Compound A5 for preparation.

Step 4: 6-Bromo-spiro[chromene-2,4'-piperidine]-1'-carboxylic acid tert-butyl ester (Compound C3-5)

Refer to Step 4 of Compound A5 for preparation.

Step 5: 6-(Benzylamino)spiro[chromene-2,4'-piperidine]-1'-carboxylic acid tert-butyl ester (Compound C3-6)

The compound C3-5 (300 mg, 0.78 mmol, 1.0 eq) was dissolved in 1,4-dioxane (10 mL). Benzylamine (168 mg, 1.57 mmol, 2 eq), BrettPhos (84 mg, 0.16 mmol, 0.2 eq), tri(dibenzylideneacetone)dipalladium(II) (144 mg, 0.14 mmol, 0.2 eq), and cesium carbonate (767 mg, 2.35 mmol, 3 eq) were added in the reaction system. The mixture was reacted at 100°C for 12 hours under nitrogen. The reaction mixture was concentrated under reduced pressure. The crude product was purified by forward column chromatography to obtain a pale yellow liquid compound C3-6 (178 mg, 55.5%).
LCMS (ESI): $[M+H]^+$= 409.54

Step 6: 6-Amino-spiro[benzopyran-2,4'-piperidine]-1'-carboxylic acid tert-butyl ester (Compound C3-7)

The compound C3-6 (178 mg, 0.44 mmol, 1.0 eq) was dissolved in methanol (5 mL). Palladium(II) carbon (20%)/carbon (36 mg, 20%) was added to the system. After three displacements of hydrogen gas, the reaction was allowed to proceed at room temperature for 12 h. The reaction mixture was filtered, and the filter cake was washed three times with methanol (5 mL). The filtrate was concentrated under reduced pressure to obtain a yellow liquid crude compound C3-7 (121 mg, 87.2%).
LCMS (ESI): $[M+H]^+$= 319.42

Step 7:

6-((2,6-Dioxopiperidin-3-yl)amino)spiro[scythadiene-2,4'-piperidine]-1'-carboxylic acid tert-butyl ester (Compound C3-8)

The crude compound C3-7 (120 mg, 0.37 mmol, 1.0 eq) was dissolved in DMF (5 mL). 3-Bromopiperidine-2,6-dione (88 mg, 0.45 mmol, 1.2 eq) and sodium bicarbonate (63 mg, 0.75 mmol, 2 eq) were added to the system. The mixture was reacted at 80°C for 3 h under nitrogen protection. After cooling the reaction system to room temperature, the mixture was purified by preparative liquid chromatography separation phase to obtain the gray solid compound C3-8 (72 mg, 44.5%).
LCMS (ESI): $[M+H]^+$= 430.52

Step 8: 3-(Spiro[benzopyran-2,4'-piperidine]-6-ylamino)piperidine-2,6-dione (Compound C3)

The compound C3-8 (72 mg, 0.17 mmol, 1.0 eq) was dissolved in dichloromethane (20 mL), then a solution of hydrochloric acid was added in 1,4-dioxane (12 mg, 0.33 mmol, 2 eq). After reacting at room temperature for 2 hours, the mixture was purified by preparative HPLC to obtain a white solid compound C3 (5 mg, 9.1%).

LCMS (ESI): $[M+H]^+$ = 330.38
$^1$H NMR (600 MHz, DMSO-*d*6) δ 10.89 (s, 1H), 8.91 - 8.82 (m, 2H), 6.74 - 6.52 (m, 3H), 4.31 - 4.23 (m, 1H), 3.22 - 3.12 (m, 2H), 3.08-3.02 (m, 2H), 2.76 - 2.64 (m, 3H), 2.60-2.56 (m, 1H), 2.10 - 2.00 (m, 1H), 1.90-1.85 (m,3H), 1.82 - 1.72 (m, 4H).

Example 28

Synthesis of Compound C4

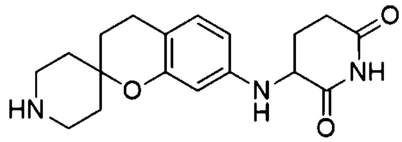

3-(Spiro[2,4'-piperidino]-7-ylamino)piperidine-2,6-dione

Synthetic Scheme

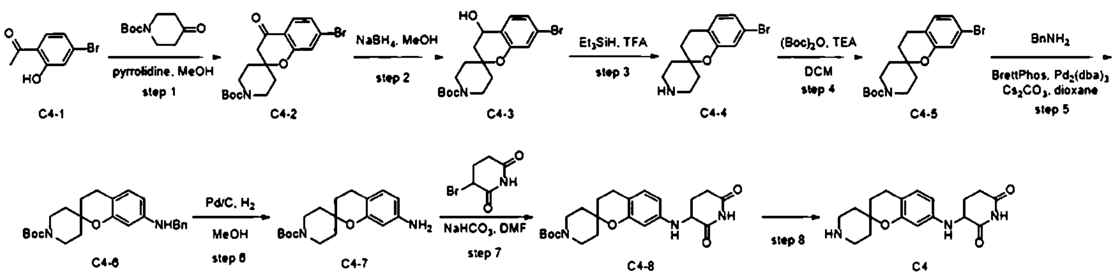


Step 1: 7-Bromo-4-oxo-spiro[semen-2,4'-piperidine]-1'-carboxylic acid tert-butyl ester (Compound C4-2)

The compound C4-1 (25 g, 116.25 mmol, 1.0 eq.), 4-oxopiperidine-1-carboxylic acid tert-butyl ester (23.16 g, 116.25 mmol, 1.0 eq.), and tetrahydropyrole (4.13 g, 58.13 mmol, 0.5 eq.) were dissolved in methanol (250 mL). The mixture was heated to 70°C and reacted for 16 hours. After cooling to room temperature, the mixture was filtrated to obtain a yellow solid compound C4-2 (40 g, 86.83%).
$^1$H NMR (400 MHz, Chloroform-d) δ 7.73 (d, *J* = 8.4 Hz, 1H), 7.23 (d, *J* = 1.8 Hz, 1H), 7.17 (dd, *J* = 8.4, 1.8 Hz, 1H), 3.90 (s, 2H), 3.21 (t, *J* = 12.6 Hz, 2H), 2.73 (s, 2H), 2.02 (dd, *J* = 14.4, 2.8 Hz, 2H), 1.66 - 1.58 (m, 2H), 1.48 (s, 9H).
LCMS (ESI): $[M-tBu+H]^+$ = 340.20.

Step 2: 7-Bromo-4-hydroxy-spiro[chroman-2,4'-piperidine]-1'-carboxylic acid tert-butyl ester (Compound C4-3)

The compound C4-2 (40 g, 100.94 mmol, 1.0 eq.) was dissolved in methanol (400 mL). The reaction mixture was cooled to 0°C, and sodium borohydride (5.73 g, 151.41 mmol, 1.5 eq.) was added. The mixture was reacted overnight at room temperature. After vacuum concentration, water was added and the mixture was extracted three times with ethyl acetate. The extract was washed with saturated saline solution, the organic phases were combined, and the mixture was dried over anhydrous sodium sulfate. The organic phase was concentrated to obtain a crude white solid compound C4-3. (40 g, 99.49%).

LCMS (ESI): $[M-Boc+H]^+$ = 298.20.

Step 3: 7-Bromospiro[chromen-2,4'-piperidine] (Compound C4-4)

The compound C4-3 (40.0 g, 100.43 mmol, 1.0 eq.) was dissolved in trifluoroacetic acid (200 mL) and triethylsilyl hydride (17.52 g, 150.64 mmol, 1.5 eq.) was added. The mixture was heated to 50°C and reacted overnight. After cooling the reaction mixture to room temperature, the solution was concentrated under reduced pressure to obtain a crude compound C4-4 (28.0 g), which was directly used in the next step.

LCMS (ESI): $[M+H]^+$= 282.20.

Step 4: 7-Bromo-spiro[chromen-2,4'-piperidine]-1'-carboxylic acid tert-butyl ester (Compound C4-5)

The compound C4-4 (28.0 g, 99.23 mmol, 1.0 eq.) was dissolved in dichloromethane (300 mL). The reaction mixture was cooled to 0°C, and then triethylamine (30.12 g, 297.68 mmol, 3.0 eq.) and di-tert-butyl dicarbonate (32.48 g, 148.84 mmol, 1.5 eq.) were added. The mixture was reacted overnight at room temperature. The reaction mixture was concentrated under reduced pressure. The crude product was purified by column chromatography to obtain a white solid compound C4-5 (32 g, 84.36%).

$^1$H NMR (400 MHz, Chloroform-*d*) δ 7.03 (d, *J* = 2.0 Hz, 1H), 7.00 - 6.91 (m, 2H), 3.90 (s, 2H), 3.21 (s, 2H), 2.74 (t, *J* = 6.8 Hz, 2H), 1.80 (q, *J* = 7.7, 7.3 Hz, 4H), 1.59 - 1.50 (m, 2H), 1.49 (s, 9H).

Step 5: 7-(Benzylamino)spiro[selman-2,4'-piperidine]-1'-carboxylic acid tert-butyl ester (Compound C4-6)

To a solution of compound C4-5 (500 mg, 1.31 mmol, 1.0 eq.), benzylamine (350 mg, 3.27 mmol, 2.5 eq.), tris(dibenzylideneacetone)dipalladium (120 mg, 0.13 mmol, 0.1 eq.), 2-(dicyclohexylphosphino)-3,6-dimethoxy-2',4',6'-tri-i-propyl-1,1'-biphenyl (140 mg, 0.26 mmol, 0.2 eq.), and cesium carbonate (1.28 g, 3.92 mmol, 3.0 eq.), anhydrous 1,4-dioxane (10 mL) were added . The reaction mixture was stirred at 80°C under nitrogen atmosphere for 12 hours. After cooling to room temperature, the mixture was concentrated under reduced pressure. The crude product was purified by column chromatography to afford a yellow solid compound C4-6 (500 mg, 93.58%).

$^1$H NMR (400 MHz, DMSO-$d_6$) δ 7.41 - 7.27 (m, 5H), 6.72 (d, *J* = 8.2 Hz, 1H), 6.14 (dd, *J* = 8.2, 2.3 Hz, 1H), 5.96 (d, *J* = 2.3 Hz, 1H), 4.19 (d, *J* = 6.1 Hz, 2H), 3.65 (d, *J* = 13.0 Hz, 2H), 3.10 (s, 2H), 2.54 (d, *J* = 6.7 Hz, 2H), 1.73 - 1.58 (m, 4H), 1.51 - 1.42 (m, 2H), 1.40 (s, 9H).

LCMS (ESI) $[M+H]^+$= 409.45.

Step 6: 7-Amino-spiro[selman-2,4'-piperidine]-1'-carboxylic acid tert-butyl ester (Compound C4-7)

The compound C4-6 (500 mg, 1.22 mmol, 1.0 eq.) was dissolved in methanol (5 mL), and palladium carbon (50 mg) was added. The mixture was reacted at room temperature under hydrogen for 16 hours. The reaction mixture was filtered through diatomaceous earth, and the filtrate was concentrated under reduced pressure to obtain a yellow solid C4-7 (350 mg, 89.81%).

$^1$H NMR (400 MHz, DMSO-$d_6$) δ 6.68 (d, *J* = 8.1 Hz, 1H), 6.08 (dd, *J* = 8.0, 2.2 Hz, 1H), 5.99 (d, *J* = 2.2 Hz, 1H), 4.81 (s, 2H), 3.69 (d, *J* = 13.0 Hz, 2H), 3.10 (s, 2H), 2.54 (d, *J* = 6.8 Hz, 2H), 1.73 - 1.60 (m, 4H), 1.40 (s, 11H).

LCMS (ESI) $[M+H]^+$= 319.35.

Step 7:

7-((2,6-Dioxopiperidin-3-yl)amino)spiro[cinnam-2,4'-piperidine]-1'-carboxylic acid tert-butyl ester (Compound C4-8)

The compound C4-7 (350 mg, 1.10 mmol, 1.0 eq.) was dissolved in N,N-dimethylformamide (3.5 mL). 3-Bromopiperidine-2,6-dione (422 mg, 2.20 mmol, 2.0 eq.) and sodium bicarbonate (185 mg, 2.20 mmol, 2.0 eq.) were added, and the mixture was reacted at 80°C for 12 hours. After cooling the reaction mixture to room temperature, water was

added. The mixture was extracted with ethyl acetate, washed with saturated saline solution, and the combined organic phase was dried over anhydrous sodium sulfate. The crude product obtained from the concentrated organic phase was purified by column chromatography to obtain a light blue solid compound C4-8 (400 mg, 84.72%).

$^1$H NMR (600 MHz, DMSO-$d_6$) δ 10.76 (s, 1H), 7.96 (s, 1H), 6.76 (d, *J* = 8.3 Hz, 1H), 6.21 (dd, *J* = 8.2, 2.3 Hz, 1H), 6.10 (d, *J* = 2.3 Hz, 1H), 5.62 (d, *J* = 7.6 Hz, 1H), 4.25 (ddd, *J* = 12.0, 7.6, 4.8 Hz, 1H), 3.68 (d, *J* = 12.8 Hz, 2H), 3.14 (d, *J* = 30.8 Hz, 2H), 2.57 (t, *J* = 6.8 Hz, 3H), 2.10 - 2.05 (m, 1H), 1.83 (dd, *J* = 12.3, 4.4 Hz, 1H), 1.72 (t, *J* = 6.8 Hz, 2H), 1.66 (d, *J* = 13.6 Hz, 2H), 1.49 (dq, *J* = 13.4, 5.9, 5.4 Hz, 2H), 1.41 (s, 9H).

LCMS (ESI) [M-Boc +H]$^+$ = 374.35.

Step 8:3-(Spiro[chromen-2,4'-piperidine]-7-ylamino)piperidine-2,6-dione (Compound C4)

The compound C4-8 (300 mg, 0.70 mmol, 1.0 eq.) was dissolved in dichloromethane (1 mL), and a solution of hydrochloric acid in 1,4-dioxane (1 mL) was added. The mixture was reacted at room temperature for 2 hours. The reaction mixture was concentrated, and the crude product was purified by reverse-phase column chromatography to obtain a blue solid compound C5 (223 mg, 87.27%).

$^1$H NMR (600 MHz, DMSO-$d_6$) δ 10.85 (d, *J* = 4.5 Hz, 1H), 9.37 (d, *J* = 12.9 Hz, 1H), 9.14 (d, *J* = 13.1 Hz, 1H), 6.85 (dd, *J* = 8.4, 3.8 Hz, 1H), 6.37 (s, 1H), 6.28 (d, *J* = 7.3 Hz, 1H), 4.34 (dt, *J* = 11.0, 4.9 Hz, 1H), 3.16 (d, *J* = 12.5 Hz, 2H), 3.04 (t, *J* = 12.3 Hz, 2H), 2.74 (ddd, *J* = 17.6, 12.4, 5.4 Hz, 1H), 2.66 - 2.53 (m, 3H), 2.05 (dt, *J* = 13.4, 4.6 Hz, 1H), 1.94 - 1.71 (m, 7H).

LCMS (ESI) [M+H]$^+$= 330.30

Example 29

Synthesis of Compound C5

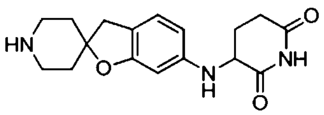

3-((3H-spiro[benzofuran-2,4'-piperidine]-6-yl)amino)piperidine-2,6-dione

Synthetic Scheme

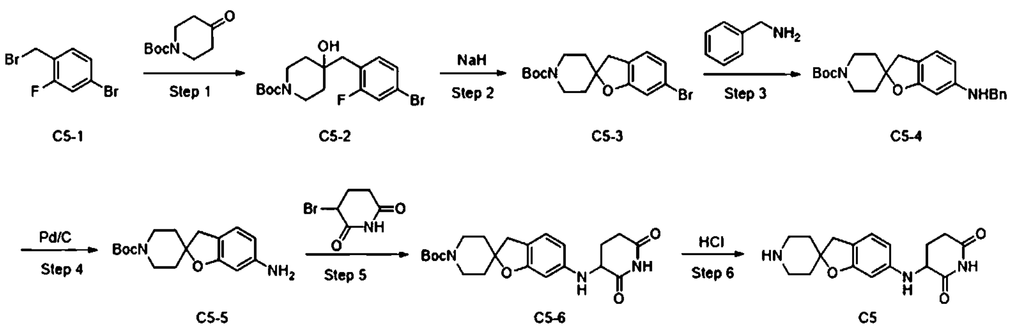


Step 1: 4-(4-Bromo-2-fluorobenzyl)-4-hydroxypiperidine-1-carboxylic acid tert-butyl ester (Compound C5-2)

The compound C5-1 (5.0 g, 18.7 mmol, 1.0 eq), magnesium rod (680 mg, 28.0 mmol, 1.5 eq), and elemental iodine (95 mg, 0.37 mmol, 0.02 eq) were added to diethyl ether (30 mL) and stirred for 1h under nitrogen at reflux temperature. N-tert-Butoxycarbonyl-4-piperidone (3.7 g, 18.7 mmol, 1.0 eq) was dissolved in diethyl ether (30 mL). The Grignard reagent was added at - 78°C, and the mixture was stirred at room temperature for 12 h. The resulting reaction mixture was concentrated under reduced pressure. The crude product obtained was purified by column chromatography

to obtain a colorless oily compound C5-2 (1.25 g, 17.3%).

$^1$H NMR (600 MHz, DMSO-$d_6$) δ 7.46 (dd, *J* = 9.5, 2.0 Hz, 1H), 7.34 (dd, *J* = 8.2, 2.0 Hz, 1H), 7.27 *(t, J*= 8.1 Hz, 1H), 4.52 (s, 1H), 3.70 - 3.60 (m, 2H), 3.10 - 2.92 (m, 2H), 2.68 (s, 2H), 1.45 - 1.30 (m, 13H).

LCMS (ESI): [M-tBu-OH+H]$^+$ = 316.18.

Step 2: 6-Bromo-3H-spiro[benzofuran-2,4'-piperidine]-1'-carboxylic acid tert-butyl ester (Compound C5-3)

Compound C5-2 (1.0 g, 2.6 mmol, 1.0 eq) was dissolved in N,N-dimethylformamide (20 mL). Sodium hydride (206 mg, 5.2 mmol, 2.0 eq) was added at 0°C. The mixture was reacted at 110°C for 2 hours under nitrogen protection. After cooling the reaction mixture to room temperature, saturated ammonium chloride aqueous solution was added. The mixture was extracted with ethyl acetate, washed with saturated saline solution, and the combined organic phase was dried over anhydrous sodium sulfate. The crude product obtained from the concentrated organic phase was purified by column chromatography to obtain a white solid compound C5-3(500 mg, 52.7%).

$^1$H NMR (600 MHz, DMSO-$d_6$) δ 7.16 (dd, *J* = 7.8, 1.1 Hz, 1H), 7.01 - 6.97 (m, 2H), 3.53 - 3.48 (m, 2H), 3.42 - 3.36 (m, 2H), 2.99 (s, 2H), 1.79 - 1.74 (m, 2H), 1.73 - 1.67 (m, 2H), 1.41 (s, 9H).

LCMS (ESI): [M-Boc+H]$^+$ = 270.27.

Step 3: 6-(Benzylamino)-3H-spiro[benzofuran-2,4'-piperidine]-1'-carboxylic acid tert-butyl ester (Compound C5-4)

The ultradry 1,4-dioxane (10 mL) was added into a solution of compound C5-3 (500 mg, 1.4 mmol, 1.0 eq), benzylamine (363 mg, 3.4 mmol, 2.5 eq), tri(dibenzylideneacetone)dipalladium(II) (124 mg, 0.14 mmol, 0.1 eq), 2-(Dicyclohexylphosphino)-3,6-dimethoxy-2'-4'-6'-tri-I-propyl-11'-biphenyl (146 mg, 0.27 mmol, 0.2 eq), and cesium carbonate (668 mg, 4.1 mmol, 3.0 eq), and the mixture was reacted at 80°C under nitrogen for 12 hours. The reaction mixture was cooled to room temperature, concentrated under reduced pressure, and purified by column chromatography to obtain a yellow solid compound C5-4 (300 mg, 56.0%).

$^1$H NMR (600 MHz, DMSO-$d_6$) δ 7.35 - 7.29 (m, 4H), 7.23 - 7.20 (m, 1H), 6.82 (d, *J* = 8.0 Hz, 1H), 6.13 (t, *J* = 6.0 Hz, 1H), 6.07 (dt, *J* = 8.1, 1.6 Hz, 1H), 5.97 - 5.95 (m, 1H), 4.23 - 4.19 (m, 2H), 3.52 - 3.43 (m, 2H), 3.40 - 3.36 (m, 2H), 2.81 (s, 2H), 1.73 - 1.65 (m, 2H), 1.65 - 1.58 (m, 2H), 1.40 (d, *J* = 1.2 Hz, 9H).

LCMS (ESI): [M+H]$^+$ = 395.40.

Step 4: 6-Amino-3H-spiro[benzofuran-2,4'-piperidine]-1'-carboxylic acid tert-butyl ester (Compound C5-5)

The compound C5-4 (300 mg, 0.76 mmol, 1.0 eq) was dissolved in methanol (10 mL), add palladium/carbon (60 mg) was added. The mixture was reacted at room temperature under hydrogen pressure for 12 hours. The reaction mixture was filtered through diatomaceous earth, the filtrate was concentrated under reduced pressure to obtain a crude compound C5-5 (200 mg).

LCMS (ESI) [M+H]$^+$ = 305.30.

Step 5:

6-((2,6-Dioxopiperidin-3-yl)amino)-3H-spiro[benzofuran-2,4'-piperidine]-1'-carboxylic acid tert-butyl ester (Compound C5-6)

The compound C5-5 (100 mg, 0.33 mmol, 1.0 eq) was dissolved in N, N,N-dimethylformamide (5 mL), to which 3-bromopiperidine-2,6-dione (75 mg, 0.39 mmol, 1.5 eq) and sodium bicarbonate (55 mg, 0.66 mmol, 2.0 eq) were added. The mixture was reacted at 80°C for 12 hours. After cooling the reaction mixture to room temperature, water was added, and the mixture was extracted with ethyl acetate, washed with saturated saline solution, combined the organic layers, and dried over anhydrous sodium sulfate. The crude product obtained from the concentrated organic phase was purified by column chromatography to obtain a light green solid compound C5-6 (80 mg, 58.6%).

$^1$H NMR (400 MHz, DMSO-$d_6$) δ 10.78 (s, 1H), 6.86 (d, *J* = 7.9 Hz, 1H), 6.18 - 6.13 (m, 2H), 5.70 (d, *J* = 7.5 Hz, 1H), 4.25 (ddd, *J* = 11.9, 7.5, 4.8 Hz, 1H), 3.56 - 3.48 (m, 2H), 3.40 - 3.32 (m, 2H), 2.85 (s, 2H), 2.79 - 2.67 (m, 1H), 2.60 - 2.52 (m, 1H), 2.13 - 2.05 (m, 1H), 1.89 - 1.59 (m, 5H), 1.41 (s, 9H).

LCMS (ESI) [M+H]$^+$= 416.25.

Step 6:

3-((3H-spiro[benzofuran-2,4'-piperidin]-6-yl)amino)piperidine-2,6-dione (Compound C5)

The compound C5-6 (75 mg, 0.18 mmol, 1.0 eq) was dissolved in dichloromethane (1 mL), followed by the addition of a hydrochloric acid/1,4-dioxane solution (1 mL). The reaction was carried out at room temperature for 2 hours. The mixture was concentrated, and the crude product was purified by reverse-phase column chromatography to obtain a white solid compound C5 (50 mg, 78.7%).

$^1H$ NMR (600 MHz, DMSO-$d_6$) δ 10.82 (s, 1H), 6.90 (d, $J$ = 8.0 Hz, 1H), 6.26 - 6.19 (m, 2H), 4.31 - 4.25 (m, 1H), 3.22 - 3.09 (m, 4H), 2.93 (s, 2H), 2.73 (ddd, $J$ = 17.5, 12.2, 5.4 Hz, 1H), 2.61 - 2.54 (m, 1H), 2.11 - 2.04 (m, 1H), 2.02 - 1.91 (m, 4H), 1.90 - 1.80 (m, 1H).

LCMS (ESI) $[M+H]^+$= 316.40.

Example 30

Synthesis of Compound C6

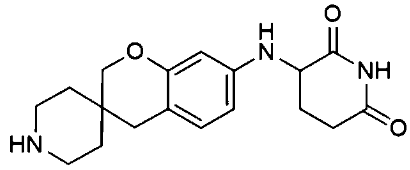

3-(Spiro[benzopyran-3,4'-piperidine]-7-ylamino)piperidine-2,6-dione

Synthetic Scheme

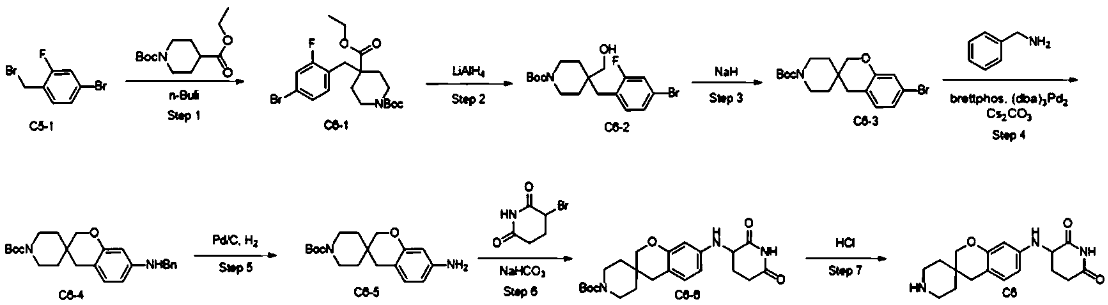


Step 1: 4-(4-Bromo-2-fluorobenzyl)piperidine-1,4-dicarboxylic acid 1-tert-butyl-4-ethyl ester (Compound C6-1)

The diisopropylamine (2.91 g, 28.74 mmol, 1.1 eq) was dissolved in ultradry tetrahydrofuran (50 mL). n-butyllithium (1.84 g, 11.0 mmol, 1.1 eq) was added to the reaction mixture and reacted under nitrogen protection at -30°C for 30 minutes. After cooling to -70°C, a tetrahydrofuran solution of 1-tert-butyl-4-ethylpiperidine-1,4-dicarboxylate (6.72 g, 26.13 mmol, 1 eq) was added dropwise to the reaction mixture. After completion of the addition, the system reacted at -70°C for 1 hour. A solution of C6-1 (7.0 g, 26.13 mmol, 1.0 eq) in tetrahydrofuran was added to the reaction system. After completion of the addition, the system was reacted at -70°C for 2 hours. The saturated ammonium chloride aqueous solution was added to the reaction mixture and extracted with ethyl acetate, then it was washed with saturated saline solution, combined organic layers, and dried over anhydrous sodium sulfate. The crude product obtained from the concentrated organic phase was purified by column chromatography to obtain a yellow oily compound C6-1 (8.5 g, 75.9%).

$^1H$ NMR (600 MHz, DMSO-$d_6$) δ 7.50 - 7.45 (m, 1H), 7.36 - 7.31 (m, 1H), 7.17 - 7.11 (m, 1H), 4.10 - 4.00 (m, 3H), 3.83 - 3.79 (m, 2H), 2.81 (s, 2H), 2.71 - 2.68 (m, 1H), 2.65 - 2.62 (m, 1H), 1.93 - 1.87 (m, 2H), 1.38 (s, 9H), 1.19 - 1.15 (m, 2H), 1.14 - 1.11 (m, 2H).

LCMS (ESI): $[M-Boc+H]^+$ = 344.29.

Step 2:

4-(4-Bromo-2-fluorobenzyl)-4-hydroxymethylpiperidine-1-carboxylic acid tert-butyl ester (Compound C6-2)

The compound C6-1 (8.5 g, 19.13 mmol, 1.0 eq) was dissolved in tetrahydrofuran (200 mL). The mixture was cooled to 0°C, and lithium aluminum hydride (798 mg, 11.04 mmol, 1.1 eq) was added in portions. The reaction was allowed to proceed at room temperature for 8 hours. The mixture was quenched with water, extracted with ethyl acetate, washed with saturated brine, combined organic layers, and dried over anhydrous sodium sulfate. The crude product obtained from the concentrated organic phase was purified by column chromatography to obtain a white solid compound C6-2 (4.26 g, 55.35%).

$^1$H NMR (600 MHz, DMSO-$d_6$) δ 7.49 - 7.44 (m, 1H), 7.37 - 7.31 (m, 1H), 7.29 - 7.24 (m, 1H), 4.75 - 4.71 m, 1H), 3.45 - 3.40 (m, 1H), 3.42 - 3.38 (m, 1H), 3.27 - 3.17 (m, 4H), 2.67 - 2.60 (m, 2H), 1.38 - 1.32 (m, 11H), 1.21 - 1.13 (m, 2H).

LCMS (ESI): $[M\text{-}Boc+H]^+$ = 302.18.

Step 3: 7-Bromospiro[benzopyran-3,4'-piperidine]-1'-carboxylic acid tert-butyl ester (Compound C6-3)

The compound C6-2 (4.25 g, 10.59 mmol, 1.0 eq) was dissolved in N,N-dimethylformamide (20 mL). Sodium hydride (508 mg, 12.71 mmol, 1.2 eq) was added to the mixture on an ice bath. The system was reacted at 100°C for 8 hours. After cooling the reaction mixture to room temperature, it was quenched with water and extracted with ethyl acetate. The mixture was washed with saturated sodium chloride solution, combined organic layers, and dried over anhydrous sodium sulfate. The crude product obtained from the concentrated organic phase was purified by column chromatography to obtain a yellow solid compound C6-3 (2.2 g, 54.3%).

$^1$H NMR (400 MHz, DMSO-$d_6$) δ 7.01 - 6.95 (m, 3H), 3.91 (s, 2H), 3.50 - 3.39 (m, 2H), 3.30 - 3.21 (m, 2H), 2.64 (s, 2H), 1.40 (s, 9H), 1.38 - 1.32 (m, 2H), 1.35 - 1.29 (m, 1H), 1.32 - 1.14 (m, 1H).

LCMS (ESI): $[M\text{-}Boc+H]^+$ = 282.38.

Step 4: 7-Benzylaminospiro[benzopyran-3,4'-piperidine]-1'-carboxylic acid tert-butyl ester (Compound C6-4)

The compound C6-3 (1.34 g, 3.51 mmol, 1.0 eq) was dissolved in 1,4-dioxane (10 mL), and benzylamine (8.89 g, 49.95 mmol, 1.5 eq), cesium carbonate (3.43 g, 10.52 mmol, 3.0 eq), 2-(dicyclohexylphosphino)-3,6-dimethoxy -2'-4'-6'-tri-I-propyl-11'- biphenyl (376 mg, 0.701 mmol, 0.2 eq), and (dibenzylideneacetone)dipalladium(II) (320 mg, 0.35 mmol, 0.1 eq) were added. The reaction was carried out at 80°C for 16 hours under nitrogen. The reaction mixture was concentrated under reduced pressure. Water was added to the system, extracted with ethyl acetate, washed with saturated saline solution, and the combined organic phase was dried over anhydrous sodium sulfate. The crude product obtained by concentrating the organic phase was purified by column chromatography to obtain a yellow solid compound C6-4 (1.3 g, 90.7%).

$^1$H NMR (400 MHz, DMSO-$d_6$) δ 7.37 - 7.27 (m, 3H), 7.26 - 7.17 (m, 1H), 6.69 (d, *J* = 8.2 Hz, 1H), 6.18 - 6.11 (m, 1H), 6.10 - 6.03 (m, 1H), 5.90 - 5.89 (m, 1H), 4.18 (d, *J* = 6.0 Hz, 2H), 3.75 (s, 2H), 3.41 - 3.52 (m, 1H), 3.44 - 3.37 (m, 1H), 3.28 - 3.17 (m, 2H), 2.46 (s, 2H), 1.39 (s, 9H), 1.33 - 1.20 (m, 4H).

LCMS (ESI): $[M+H]^+$ = 409.69.

Step 5: 7-Amino-spiro[benzopentane-3,4'-piperidine]-1'-carboxylic acid tert-butyl ester (Compound C6-5)

The compound C6-4 (1.2 g, 2.94 mmol, 1.0 eq) was dissolved in methanol (50 mL) and ethyl acetate (50 mL). Ppalladium(II) hydroxide/carbon (170 mg) was added to the mixture. After three displacements of hydrogen gas, the mixture was reacted at room temperature for 12 hours. The reaction mixture was filtered, and the filter cake was washed three times with methanol (15 mL). The filtrate was concentrated under reduced pressure to obtain a crude product C6-5 (900 mg, 96.2%). This crude product was used directly in the next step without further purification.

LCMS (ESI): $[M+H]^+$= 319.60.

Step 6:

7-((2,6-Dioxopiperidin-3-yl)amino)spiro[benzopyran-3,4'-piperidine]-1'-carboxylic acid tert-butyl ester (Compound C6-6)

The compound C6-5 (900 mg, 2.83 mmol, 1.0 eq), 3-bromopiperidine-2,6-dione (650 mg, 3.39 mmol, 1.2 eq), and sodium hydrogen carbonate (474 mg, 5.65 mmol, 2.0 eq) were dissolved in N,N-dimethylformamide (5 mL) and reacted at 80°C for 16 hours under nitrogen protection. After cooling the reaction mixture to room temperature, the solution was concentrated under reduced pressure, then water was added and extracted with ethyl acetate. The extract was washed with saturated saline solution, the organic phases were combined, and the mixture was dried over anhydrous sodium

sulfate. The crude product obtained from the concentrated organic phase was purified by reverse-phase column chromatography to obtain a green solid compound C6-6 (810 mg, 66.7%).

$^1H$ NMR (600 MHz, DMSO-$d_6$) δ 10.77 (s, 1H), 6.75 (d, *J* = 8.2 Hz, 1H), 6.25 - 6.20 (m, 1H), 6.09 (d, *J* = 2.3 Hz, 1H), 5.64 (d, *J* = 7.5 Hz, 1H), 4.26 - 4.20 (m, 1H), 3.80 (s, 2H), 3.48 - 3.41 (m, 2H), 3.26 (s, 2H), 2.79 - 2.69 (m, 1H), 2.62 - 2.52 (m, 1H), 2.50 (s, 2H), 2.12 - 2.04 (m, 1H), 1.87 - 1.78 (m, 1H), 1.40 (s, 9H), 1.35 - 1.25 (m, 4H).

LCMS (ESI): $[M+H]^+$ = 430.29.

Step 7:

3-(Spiro[benzopyran-3,4'-piperidine]-7-ylamino)piperidine-2,6-dione (Compound C6)

The compound C6-6 (800 mg, 1.86 mmol, 1.0 eq) was dissolved in a hydrochloric acid solution of 1,4-dioxane (3 mL) and dichloromethane (8 mL). The reaction mixture was allowed to react overnight at room temperature. The reaction mixture was filtered, and the filter cake was washed three times with acetonitrile (10 mL) to obtain a white solid compound C6 (500 mg, 81.3%).

$^1H$ NMR (600 MHz, DMSO-$d_6$) δ 10.89 (s, 1H), 9.11 (s, 1H), 9.06 (s, 1H), 6.86 - 6.82 m, 1H), 6.42 - 6.37 (m, 1H), 6.29 - 6.25 (m, 1H), 4.36 - 4.30 (m, 1H), 3.87 (s, 2H), 3.12 - 3.05 (m, 2H), 3.08 - 3.00 (m, 2H), 2.75 - 2.67 (m, 1H), 2.58 (s, 2H), 2.61 - 2.54 (m, 1H), 2.07 - 1.99 (m, 1H), 1.93 - 1.83 (m, 1H), 1.66 - 1.53 (m, 4H).

LCMS (ESI) $[M+H]^+$= 330.30.

Example 31

Synthesis of Compound C7

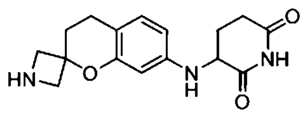

3-(Spiro[azetidin-3,2'-chroman]-7'-ylamino)piperidine-2,6-dione

Synthetic Scheme

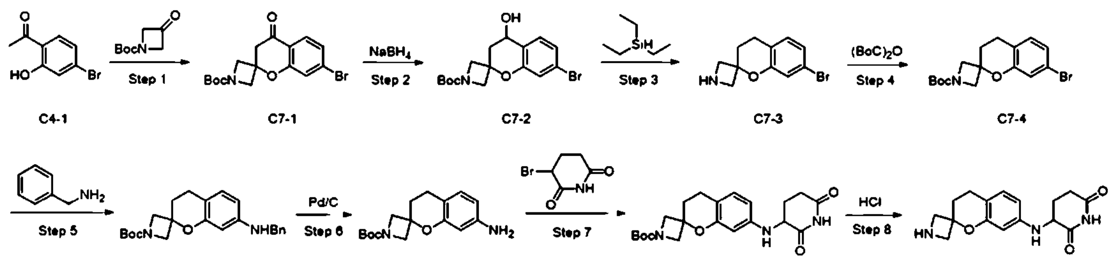


Step 1: 7'-Bromo-4'-oxo-spiro[azetidine-3,2'-chromane]-1-carboxylic acid tert-butyl ester (Compound C7-1)

The compound C4-1 (10.0 g, 46.5 mmol, 1 eq) was dissolved in ethanol (100 mL). N-tert-Butoxycarbonyl-3-azetidinone (8.8 g, 51.2 mmol, 1.1 eq) and tetrahydropyrole (3.6 g, 51.2 mmol, 1.1 eq) were added and stirred at 50°C for 12 hours under nitrogen. After cooling the reaction mixture to room temperature, it was extracted with ethyl acetate, washed with saturated saline solution, and the combined organic phase was dried over anhydrous sodium sulfate. The crude product obtained from the concentrated organic phase was purified by column chromatography to obtain a yellow solid compound C7-1 (5.5 g, 32.1%).

$^1H$ NMR (600 MHz, DMSO-$d_6$) δ 7.67 (d, *J* = 8.4 Hz, 1H), 7.48 (d, *J* = 1.8 Hz, 1H), 7.33 (dd, *J* = 8.4, 1.8 Hz, 1H), 3.99 (d, *J* = 9.6 Hz, 2H), 3.90 (d, *J* = 9.6 Hz, 2H), 3.19 (s, 2H), 1.38 (s, 9H).

LCMS (ESI): $[M-Boc+H]^+$ = 268.17.

Step 2: 7'-Bromo-4'-hydroxy-spiro[azetidine-3,2'-chromane]-1-carboxylic acid tert-butyl ester (Compound C7-2)

The compound C7-1 (5.5 g, 14.9 mmol, 1 eq) was dissolved in methanol (50 mL). Sodium borohydride (969 mg, 25.6 mmol, 1.5 eq) was added at 0°C, and the mixture reacted at room temperature for 12 hours. The reaction mixture was concentrated under reduced pressure, then water was added. The product was extracted with ethyl acetate, washed with saturated saline solution, and the combined organic phase was dried over anhydrous sodium sulfate. Concentrated under reduced pressure to obtain a crude product compound C7-2 (5.5 g).
$^1$H NMR (400 MHz, DMSO-$d_6$) δ 7.31 - 7.25 (m, 1H), 7.12 (dd, *J* = 8.2, 2.0 Hz, 1H), 7.07 (d, *J* = 2.0 Hz, 1H), 5.56 (d, *J* = 5.1 Hz, 1H), 4.75 - 4.62 (m, 1H), 4.15 (d, *J* = 9.6 Hz, 1H), 4.00 (d, *J* = 9.7 Hz, 1H), 3.82 (dd, *J* = 19.0, 9.5 Hz, 2H), 2.30 - 2.10 (m, 2H), 1.38 (s, 9H).
LCMS (ESI): $[M-Boc+H]^+$ = 272.17.

Step 3: 7'-Bromospiro[azetidine-3,2'-chromane] (Compound C7-3)

The compound C7-2 (5.5 g, 14.8 mmol, 1.0 eq) was dissolved in trifluoroacetic acid (20 mL). Triethylsilane (3.5 g, 30.0 mmol, 2.0 eq) was added and reacted at 50°C for 1 hour. The reaction mixture was cooled to room temperature, and the solution was concentrated under reduced pressure to obtain a crude product C7-3 (3.8 g).
LCMS (ESI) $[M+H]^+$= 252.17.

Step 4: 7'-Bromo-spiro[azetidine-3,2'-chromane]-1-carboxylic acid tert-butyl ester (Compound C7-4)

The compound C7-3 (3.8 g, 14.8 mmol, 1.0 eq) was dissolved in dichloromethane (40 mL). Triethylamine (4.5 g, 44.5 mmol, 3.0 eq) and di-tert-butyl dicarbonate (4.9 g, 22.3 mmol, 1.5 eq) were added at 0°C. The mixture was reacted at room temperature for 12 hours. Water was added to the reaction mixture, extracted with dichloromethane, washed with saturated saline solution, and the combined organic phase was dried over anhydrous sodium sulfate. The crude product obtained by concentrating the organic phase was purified by column chromatography to obtain a white solid compound C7-4 (2.3 g, 43.2%).
$^1$H NMR (400 MHz, DMSO-$d_6$) δ 7.09 - 7.02 (m, 3H), 3.93 (d, *J* = 9.3 Hz, 2H), 3.82 (d, *J* = 9.3 Hz, 2H), 2.74 (t, *J* = 6.5 Hz, 2H), 2.06 (t, *J* = 6.5 Hz, 2H), 1.39 (s, 9H).
LCMS (ESI) $[M-Boc+H]^+$= 254.17.

Step 5: 7'-(Benzylamino)spiro[azetidine-3,2'-chromane]-1-carboxylic acid tert-butyl ester (Compound C7-5)

The ultradry 1,4-dioxane (40 mL) was added in the solution of compound C7-4 (3.5 g, 9.8 mmol, 1.0 eq), benzylamine (2.6 g, 24.6 mmol, 2.5 eq), tri(dibenzylideneacetone)dipalladium(II) (901 mg, 0.89 mmol, 0.1 eq), 2-(Dicyclohexylphosphino)-3,6-dimethoxy-2'-4'-6'-tri-I-propyl-11'-biphenyl (1.05 g, 2.0 mmol, 0.2 eq), and cesium carbonate (9.6 g, 29.5 mmol, 3.0 eq), and the mixture was reacted at 80°C under nitrogen for 12 hours. The reaction mixture was cooled to room temperature, concentrated under reduced pressure, and purified by column chromatography to obtain a white solid compound C7-5 (2.0 g, 52.2%).
$^1$H NMR (400 MHz, DMSO-$d_6$) δ 7.35 - 7.28 (m, 4H), 7.26 - 7.18 (m, 1H), 6.72 (d, *J* = 8.3 Hz, 1H), 6.23 - 6.09 (m, 2H), 5.96 (d, *J* = 2.2 Hz, 1H), 4.20 (s, 2H), 3.84 (d, *J* = 9.1 Hz, 2H), 3.74 (d, *J* = 9.1 Hz, 2H), 2.58 (t, *J* = 6.5 Hz, 2H), 2.01 - 1.92 (m, 2H), 1.38 (s, 9H).
LCMS (ESI): $[M+H]^+$ = 381.49.

Step 6: 7'-Amino-spiro[azetidine-3,2'-chromane]-1-carboxylic acid tert-butyl ester (Compound C7-6)

The compound C7-5 (2.0 g, 5.1 mmol, 1.0 eq) was dissolved in methanol (20 mL) and treated with Pd/C (400 mg). The mixture was reacted at room temperature under hydrogen pressure for 12 hours. The reaction mixture was filtered through diatomaceous earth, and the filtrate was concentrated under reduced pressure to obtain a crude product compound C7-6 (1.0 g).
LCMS (ESI) $[M+H]^+$ = 291.37.

Step 7:

7'-((2,6-Piperidinedione-3-yl)amino)spiro[azetidine-3,2'-chromane]-1-carboxylic acid tert-butyl ester (Compound C7-7)

The compound C7-6 (1.0 g, 3.5 mmol, 1.0 eq) was dissolved in N,N-dimethylformamide (15 mL). 3-Bromopiperidine-2,6-dione (735 mg, 3.8 mmol, 1.1 eq) and sodium bicarbonate (584 mg, 7.0 mmol, 2.0 eq) were added and

reacted at 80°C for 12 hours. The reaction mixture was cooled to room temperature, water was added, and the mixture was extracted with ethyl acetate. The extract was washed with saturated saline solution, the organic phases were combined, and the mixture was dried over anhydrous sodium sulfate. The crude product obtained by concentrating the organic layer was purified by column chromatography to obtain a white solid compound C7-7 (277 mg, 19.8%).

$^1$H NMR (600 MHz, DMSO-$d_6$) δ 10.78 (s, 1H), 6.77 (d, $J$ = 8.3 Hz, 1H), 6.26 (dd, $J$ = 8.3, 2.3 Hz, 1H), 6.14 (d, $J$ = 2.2 Hz, 1H), 5.71 (d, $J$ = 7.7 Hz, 1H), 4.28 - 4.24 (m, 1H), 3.88 (s, 2H), 3.79 (s, 2H), 2.82 - 2.68 (m, 1H), 2.62 (t, $J$ = 6.5 Hz, 2H), 2.59 - 2.53 (m, 1H), 2.12 - 2.06 (m, 1H), 2.00 (t, $J$ = 6.5 Hz, 2H), 1.87 - 1.80 (m, 1H), 1.39 (s, 9H).

LCMS (ESI) [M-Boc+H]$^+$= 302.36.

Step 8: 3-(Spiro[azetidin-3,2'-chroman]-7'-ylamino)piperidine-2,6-dione (Compound C7)

The compound C7-7 (69 mg, 0.17 mmol, 1.0 eq) was dissolved in dichloromethane (3 mL), and a solution of hydrochloric acid in 1,4-dioxane (1 mL) was added. The mixture was reacted at room temperature for 2 hours. The reaction mixture was concentrated, and the crude product was purified by reverse-phase column chromatography to obtain a white solid compound C7 (10 mg, 19.3%).

$^1$H NMR (600 MHz, DMSO-$d_6$) δ 10.79 (s, 1H), 8.95 (s, 2H), 6.79 (d, $J$ = 8.3 Hz, 1H), 6.29 (dd, $J$ = 8.3, 2.3 Hz, 1H), 6.16 (d, $J$ = 2.2 Hz, 1H), 4.26 (dd, $J$ = 11.5, 4.8 Hz, 1H), 4.09 - 3.97 (m, 4H), 2.80 - 2.70 (m, 1H), 2.66 - 2.55 (m, 3H), 2.08 (t, $J$ = 6.6 Hz, 3H), 1.89 - 1.80 (m, 1H).

LCMS (ESI) [M+H]$^+$= 302.30.

Example 32

Synthesis of Compound C8

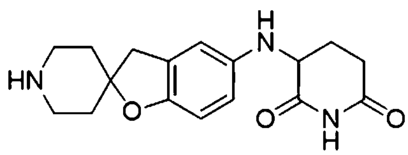

3-((3H-spiro[benzofuran-2,4'-piperidine]-5-yl)amino)piperidine-2,6-dione

Synthetic Scheme

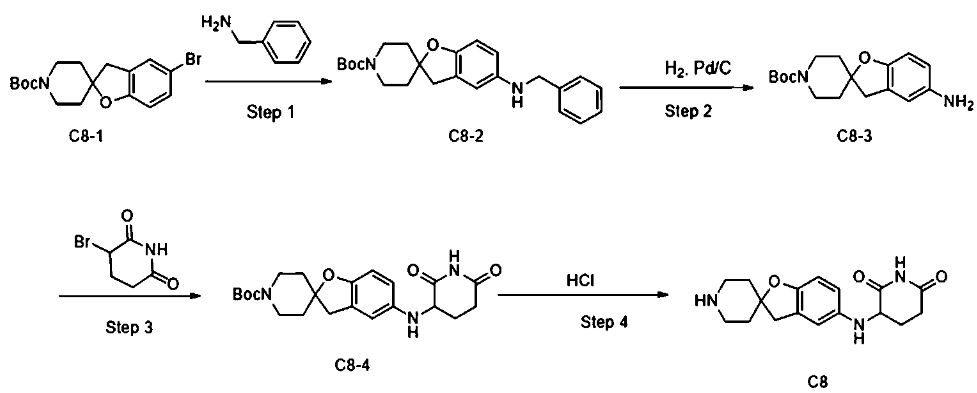


Step 1: 5-Benzylamino-3H-spiro[benzofuran-2,4'-piperidine]-1'-carboxylic acid tert-butyl ester (Compound C8-2)

The compound C8-1 (500 mg, 1.36 mmol, 1.0 eq) was dissolved in 1,4-dioxane (10 mL). Benzylamine (291 mg, 2.72 mmol, 2 eq), BrettPhos (1456 mg, 0.27 mmol, 0.2 eq), tri(dibenzylideneacetyl)dipalladium(II) (248 mg, 0.27 mmol, 0.2 eq), and cesium carbonate (1.33 g, 4.07 mmol, 3 eq) were added to the reaction mixture. The reaction was conducted under nitrogen protection at 100°C for 12 hours. The resulting reaction mixture was concentrated under reduced pressure.

The crude product obtained was purified by forward column chromatography to obtain a pale yellow liquid compound C8-2 (330 mg, 61.6%).
LCMS (ESI): $[M+H]^+$ = 395.23

Step 2: 5-Amino-3H-spiro[benzofuran-2,4'-piperidine]-1'-carboxylic acid tert-butyl ester (Compound C8-3)

The compound C8-2 (300 mg, 0.76 mmol, 1.0 eq) was dissolved in methanol (8 mL). Palladium(II) carbon (20%)/carbon (60 mg, 20%) was added to the system. After three displacements of hydrogen gas, reacted at room temperature for 12 hours. The reaction mixture was filtered, and the filter cake was washed three times with methanol (5 mL). The filtrate was concentrated under reduced pressure to obtain a crude yellow liquid compound C8-3 (119 mg, 51.4%).
LCMS (ESI): $[M+H]^+$ = 305.39

Step 3:

5-((2,6-Dioxopiperidin-3-yl)amino)-3H-spiro[benzofuran-2,4'-piperidine]-1'-carboxylic acid tert-butyl ester (Compound C8-4)

The crude compound C8-4 (120 mg, 0.39 mmol, 1.0 eq) was dissolved in DMF (5 mL). 3-Bromopiperidine-2,6-dione (90 mg, 0.46 mmol, 1.2 eq) and sodium bicarbonate (66 mg, 0.78 mmol, 2 eq) were added to the system. The mixture was reacted at 80°C for 3 hours under nitrogen protection. After cooling the reaction mixture to room temperature, the mixture was purified by preparing liquid chromatography to obtain a green oily compound C8-4 (80 mg, 49.3%).
LCMS (ESI): $[M+H]^+$ = 416.49

Step 4:

3-((3H-spiro[benzofuran-2,4'-piperidine]-5-yl)amino)piperidine-2,6-dione (Compound C8)

The compound C8-4 (80 mg, 0.19 mmol, 1.0 eq) was dissolved in dichloromethane (10 mL). A solution of hydrochloric acid in 1,4-dioxane (7 mg, 0.19 mmol, 1 eq) was added. The mixture was reacted at room temperature for 2 hours. The system, after reacting at room temperature for 2 hours, was purified by preparative liquid chromatography to obtain a white solid compound C8 (8 mg, 13.2%).

$^1H$ NMR (600 MHz, DMSO-*d*6 -$D_2O$) δ 6.99 - 6.94 (m, 1H), 6.84 (dd, J = 8.5, 2.5 Hz, 1H), 6.73 (d, J = 8.6 Hz, 1H), 4.39 (dd, J = 12.1, 5.2 Hz, 1H),4.21-3.12 (m, 4H), 3.05 (s, 2H), 2.69 - 2.55 (m, 2H), 2.00-1.86 (m, 6H)
LCMS (ESI): $[M+H]^+$ = 316.37

Example 33

Synthesis of Compound C9

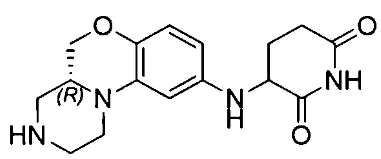

3-(((R)-1,2,3,4,4a,5-Hexahydrobenzo[b]pyrazine[1,2-d][1,4]oxazine-9-yl)amino)piperidine-2,6-dione

Synthetic Scheme

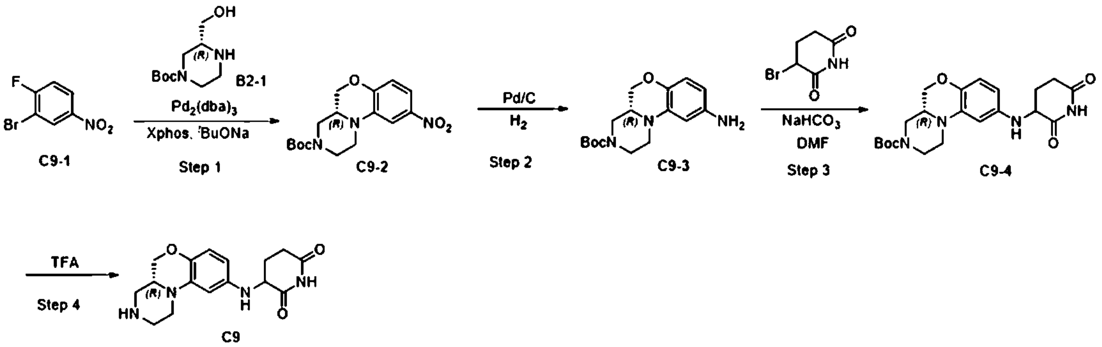


Step 1:

(R)-9-Nitro-1,2,4a,5-tetrahydrobenzo[b]pyrazino[1,2-d][1,4]oxazine-3(4H)-carboxylic acid tert-butyl ester (Compound C9-2)

The compound C9-1 (500 mg, 2.27 mmol, 1.0 equiv), compound B2-1 (737.3 mg, 3.41 mmol, 1.5 equiv), $Pd_2(dba)_3$ (416.3 mg, 0.45 mmol, 0.2 equiv), Xphos (216.7 mg, 0.45 mmol, 0.2 equiv), and sodium tert-butylate (309.5 mg, 3.22 mmol, 1.5 equiv) were dissolved in 15 mL 1, 4-dioxane. The system was purged with nitrogen gas three times, then stirred overnight at 100 °C under nitrogen. After the reaction was complete, the system was concentrated under reduced pressure and then purified by normal-phase column chromatography to obtain a yellow solid C9-2 (643.8 mg, 84%).

$^1$H NMR (600 MHz, $CDCl_3$) $\delta$ 7.70 - 7.65 (m, 2H), 6.83 (d, $J$ = 8.5 Hz, 1H), 4.33 (dd, $J$ = 10.8, 2.8 Hz, 1H), 4.19 (s, 2H), 4.04 (dd, $J$ = 10.7, 8.9 Hz, 1H), 3.71 (d, $J$ = 11.3 Hz, 1H), 3.13 (tt, $J$ = 8.9, 3.0 Hz, 1H), 3.06 (s, 1H), 2.80 (td, $J$ = 12.0, 3.3 Hz, 1H), 2.59 (s, 1H), 1.48 (s, 9H).
LCMS (ESI) $[M\text{-}Boc+H]^+$= 236.36

Step 2:

(R)-9-Amino-1,2,4a,5-tetrahydrobenzo[b]pyrazino[1,2-d][1,4]oxazine-3(4H)-carboxylic acid tert-butyl ester (Compound C9-3)

The compound C9-2 (643.8 mg, 2.27 mmol, 1.0 equiv) was dissolved in 20 mL methanol. Pd/C (320 mg) was added, and the system was purged with hydrogen gas five times. The mixture was stirred overnight at room temperature under a hydrogen atmosphere. After reaction completion, the reaction mixture was filtered through diatomaceous earth. The filter cake was washed with 100mL methanol. The filtrate was concentrated under reduced pressure and purified by normal-phase column chromatography to obtain a yellow solid C9-3 (437.5 mg, 73%).

$^1$H NMR (600 MHz, $CDCl_3$) $\delta$ 6.60 (d, $J$ = 8.3 Hz, 1H), 6.20 (s, 1H), 6.10 (dd, $J$ = 8.3, 2.1 Hz, 1H), 4.15 (dd, $J$ = 10.6, 2.5 Hz, 1H), 4.04 (s, 1H), 3.93 - 3.88 (m, 1H), 3.61 (d, $J$ = 9.7 Hz, 1H), 3.38 (s, 1H), 3.12 - 3.06 (m, 1H), 3.00 (s, 1H), 2.72 (t, $J$ = 10.6 Hz, 1H), 2.60 (s, 1H), 1.48 (s, 9H).
LCMS (ESI) $[M\text{-}tBu +H]^+$ = 250.37

Step 3:

(4aR)-9-((2,6-Dioxyhesperidin-3-yl)amino)-1,2,4-$\alpha$,5-tetrahydrobenzo[b]pyrazino[1,2-d][1,4]oxazine-3(4H)-carboxylic acid tert-butyl ester (Compound C9-4)

The compound C9-3 (357.5 mg, 1.0 eq.), 3-bromopiperidine-2,6-dione (278.1 mg, 1.2 eq.), and sodium bicarbonate (199 mg, 2.0 eq.) were dissolved in 12 mL N, N,N-dimethylformamide. The mixture was stirred overnight at 80°C. Upon completion of the reaction, the system was diluted with 50 mL water and extracted three times with ethyl acetate (50 mL$\times$3). The organic phases were combined, washed five times with saturated brine, dried over sodium sulfate, and concentrated under reduced pressure to obtain the crude product. The crude product was purified by normal-phase column chromatography to obtain a brown-green solid C9-4 (461.2 mg, 82%).

$^1$H NMR (400 MHz, $CDCl_3$) $\delta$ 8.26 (s, 1H), 6.67 (d, $J$ = 8.5 Hz, 1H), 6.23 (s, 1H), 6.09 - 6.02 (m, 1H), 4.43 (s, 1H), 4.16 (dd, $J$ = 11.0, 3.1 Hz, 1H), 4.02 - 3.86 (m, 3H), 3.61 (d, $J$ = 11.4 Hz, 1H), 3.09 (ddd, $J$ = 8.3, 7.1, 3.0 Hz, 1H), 3.00 (s, 1H),

2.88 - 2.79 (m, 1H), 2.78 - 2.69 (m, 2H), 2.60 (s, 1H), 2.54 - 2.45 (m, 1H), 1.96 - 1.82 (m, 1H), 1.47 (s, 9H). LCMS (ESI) [M-tBu+H]$^+$= 417.49

Step 4:

3-(((R)-1,2,3,4,4a,5-Hexahydrobenzo[b]pyrazine[1,2-d][1,4]oxazine-9-yl)amino)piperidine-2,6-dione (Compound C9)

The compound C9-4 (100 mg, 1.0 eq.) was dissolved in 3mL of dichloromethane, 1mL of trifluoroacetic acid was added, and the mixture was stirred at room temperature for 2 hours. After reaction completion, the reaction system was concentrated, and the crude product was purified by reverse-phase column chromatography to obtain a white solid compound C9 (37.26 mg, 49%).

$^1$H NMR (600 MHz, DMSO-$d_6$) δ 10.80 (s, 1H), 8.97 (d, *J* = 43.7 Hz, 2H), 6.52 (d, *J* = 8.5 Hz, 1H), 6.33 (s, 1H), 6.10 (d, *J* = 8.5 Hz, 1H), 4.23 (dd, *J* = 11.1, 4.4 Hz, 1H), 4.17 (d, *J* = 9.5 Hz, 1H), 3.86 (ddd, *J* = 16.0, 13.8, 8.0 Hz, 2H), 3.41 (d, *J* = 12.3 Hz, 1H), 3.32 (t, *J* = 12.4 Hz, 2H), 3.10 - 3.00 (m, 1H), 2.88 (dd, *J* = 18.3, 7.8 Hz, 1H), 2.77 (dd, J = 22.1, 10.6 Hz, 1H), 2.70 (ddd, J = 17.3, 12.2, 5.2 Hz, 1H), 2.58 (dt, *J* = 17.5, 4.1 Hz, 1H), 2.12 - 2.03 (m, 1H), 1.88 - 1.77 (m, 1H). LCMS (ESI) [M-tBu+H]$^+$= 317.28

Example 34

Synthesis of Compound C10

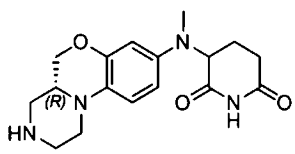

3-(((R)-1,2,3,4,4a,5-Hexahydrobenzo[b]pyrazino[1,2-d][1,4]oxazine-8-yl)(methyl)amino)piperidine-2,6-dione

Synthetic Scheme

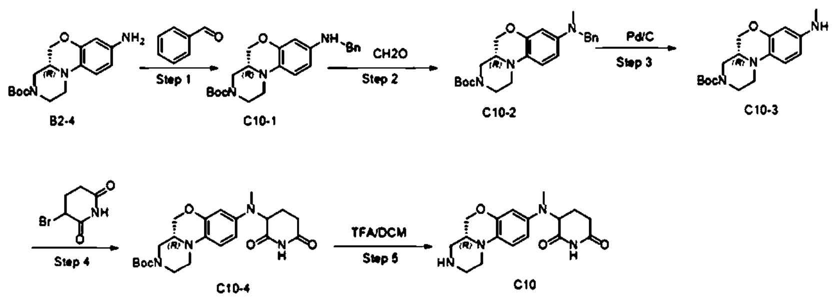


Step 1:

(R)-8-(Benzylamino)-1,2,4a,5-tetrahydrobenzo[b]pyrazino[1,2-d][1,4]oxazine-3(4H)-carboxylic acid tert-butyl ester (Compound C10-1)

The compound B2-4 (500 mg, 1.64 mmol, 1.0 eq) was dissolved in methanol (12 mL). The mixture was titrated with acetic acid to pH=5. Benzaldehyde (173.76 mg, 1.64 mmol, 1.0 eq) and sodium cyanoborohydride (294.97 mg, 4.91 mmol, 3.0 eq) were added at 0°C. The reaction mixture was warmed to 25°C and reacted for 1 hour at this temperature. The reaction mixture was concentrated under reduced pressure. The crude product obtained was purified by column chromatography to obtain a yellow oily compound C10-1 (572 mg, 88%).

$^1$H NMR (400 MHz, DMSO-$d_6$) δ 7.35 - 7.26 (m, 4H), 7.20 (t, J = 6.8 Hz, 1H), 6.63 (d, J = 8.8 Hz, 1H), 6.10 (dd, J = 8.7, 2.4 Hz, 1H), 5.99 (d, J = 2.4 Hz, 1H), 5.76 (t, J = 6.0 Hz, 1H), 4.22 - 4.10 (m, 3H), 3.99 - 3.73 (m, 3H), 3.57 (d, J = 11.6 Hz, 1H), 2.71 (t, J = 9.7 Hz, 1H), 2.32 (td, J = 11.7, 2.9 Hz, 1H), 1.40 (s, 9H).
LCMS (ESI): [M+H]$^+$ = 396.49

Step 2:

(R)-8-(Benzyl(methyl)amino)-1,2,4a,5-tetrahydrobenzo[b]pyrazino[1,2-d][1,4]oxazine-3(4H)-carboxylic acid tert-butyl ester (Compound C10-2)

The compound C10-1 (572 mg, 1.45 mmol, 1.0 eq) was dissolved in methanol (14 mL). The mixture was titrated with acetic acid to pH=5. At 0°C, the aqueous formaldehyde solution (37%) (469.46 mg, 5.79 mmol, 4.0 eq) and sodium cyanoborohydride (363.54 mg, 5.79 mmol, 4.0 eq) were added. The reaction mixture was heated to 25°C and reacted at this temperature for 1 h. The resulting reaction mixture was concentrated under reduced pressure. The crude product obtained was purified by column chromatography to obtain a yellow oily compound C10-2 (358 mg, 60%).

$^1$H NMR (600 MHz, DMSO-$d_6$) δ 7.30 - 7.27(m, 2H), 7.20 (dd, J = 17.2, 7.5 Hz, 3H), 6.72 (d, J = 8.8 Hz, 1H), 6.24 (d, J = 7.5 Hz, 1H), 6.13 (s, 1H), 4.40 (s, 2H), 4.22 (d, J = 9.7 Hz, 1H), 4.05 - 3.78 (m, 3H), 3.62 (d, J = 11.0 Hz, 1H), 2.86 (s, 3H), 2.75 (s, 1H), 2.37 (t, J = 10.6 Hz, 1H), 1.41 (s, 9H).
LCMS (ESI): [M+H]$^+$ = 410.40

Step 3:

(R)-8-(Methylamino)-1,2,4a,5-tetrahydrobenzo[b]pyrazino[1,2-d][1,4]oxazine-3(4H)-carboxylic acid tert-butyl ester (Compound C10-3)

The compound C10-2 (358 mg, 0.874 mmol, 1.0 eq) was dissolved in ethanol (10 mL). Palladium carbon (282 mg) was added to the system. After three exchanges of hydrogen gas, the mixture was stirred at 25 °C for 16 hours. After reaction completion, the mixture was filtered, and the filtrate was concentrated under reduced pressure. The crude product was purified by column chromatography to obtain a yellow oily compound C10-3 (262 mg, 94%).
LCMS (ESI): [M+H]$^+$ = 320.38

Step 4:

(4aR)-8-((2,6-Dioxopiperidin-3-yl)(methyl)amino)-1,2,4a,5-tetrahydrobenzo[b]pyrazino[1,2-d][1,4]oxazine-3(4H)-carboxylic acid tert-butyl ester (Compound C10-4)

The compound C10-3 (262 mg, 0.82 mmol, 1.0 eq) was dissolved in N,N-dimethylformamide (7 mL). 3-Bromopiperidine-2,6-dione (315 mg, 1.64 mmol, 2.0 eq) and sodium bicarbonate (137.8 mg, 1.64 mmol, 2.0 eq) to the system. The reaction mixture was heated to 80°C and reacted at this temperature for 16 hours. After reaction completion, the mixture was cooled to room temperature, quenched with water, and the aqueous phase extracted three times with ethyl acetate. The combined organic phase was washed four times with saturated sodium chloride solution. The organic phase was concentrated under reduced pressure. The crude product was purified by column chromatography to obtain a yellow-green oily compound C10-4 (289 mg, 82%).

$^1$H NMR (600 MHz, DMSO-$d_6$) δ 10.73 (s, 1H), 6.74 (d, J = 8.9 Hz, 1H), 6.32 (dd, J = 8.9, 2.7 Hz, 1H), 6.26 (t, J = 2.2 Hz, 1H), 4.69 (dd, J = 12.6, 4.9 Hz, 1H), 4.25 (dd, J = 10.7, 2.5 Hz, 1H), 4.05 - 3.82 (m, 3H), 3.66 (d, J = 11.5 Hz, 1H), 2.96 (s, 1H), 2.85 - 2.73 (m, 2H), 2.63 (s, 3H), 2.62 - 2.50 (m, 2H), 2.38 (td, J = 11.9, 3.2 Hz, 1H), 2.22 (qd, J = 13.0, 4.2 Hz, 1H), 1.86 - 1.78 (m, 1H), 1.41 (s, 9H).
LCMS (ESI): [M+H]$^+$ = 430.39

Step 5:

3-(((R)-1,2,3,4,4a,5-Hexahydrobenzo[b]pyrazino[1,2-d][1,4]oxazine-8-yl)(methyl)amino)piperidine-2,6-dione (Compound C10)

The compound C10-4 (289 mg, 0.67 mmol) was dissolved in dichloromethane:trifluoroacetic acid = 5:1 (12 mL) and reacted at room temperature for 1 hour. The reaction mixture was concentrated, and the crude product was purified by

reverse-phase column chromatography to obtain a pale pink solid compound C10 (139.0 mg, 63%).

$^1H$ NMR (600 MHz, DMSO-$d_6$) δ 10.75 (s, 1H), 8.92 - 8.79 (m, 2H), 6.78 (d, J = 8.7 Hz, 1H), 6.36 (dt, $J$ = 8.9, 3.3 Hz, 1H), 6.29 (dd, J = 5.9, 2.8 Hz, 1H), 4.71 (dd, J = 12.7, 4.9 Hz, 1H), 4.25 (dd, J = 10.8, 2.4 Hz, 1H), 3.92 (dd, $J$ = 10.8, 8.4 Hz, 1H), 3.87 (d, $J$ = 13.0 Hz, 1H), 3.40 (d, J = 12.4 Hz, 1H), 3.33 (d, J = 12.1 Hz, 1H), 3.20 - 3.14 (m, 1H), 3.07 (dd, $J$ = 23.3, 12.3 Hz, 1H), 2.85 - 2.69 (m, 3H), 2.64 (s, 3H), 2.56-2.52 (m, 1H), 2.24 (qd, $J$ = 12.9, 4.1 Hz, 1H). 1.83 - 1.81 (m, 1H).
LCMS (ESI): $[M+H]^+$ = 331.39

Example 35

Synthesis of Compound C11

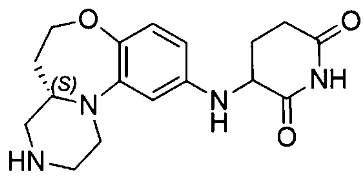

3-(((S)-2,3,4,4a,5,6-Hexahydro-1H-benzo[b]pyrazine[1,2-d][1,4]oxacyclopropane-10-yl)amino)piperidine-2,6-dione

Synthetic scheme

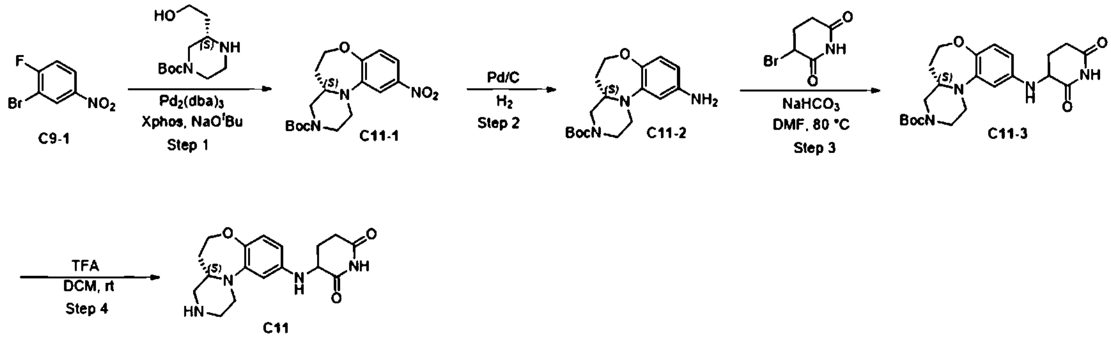


The synthetic method for compound C11 is the same as that for compound C9.

$^1H$ NMR (600 MHz, DMSO-$d_6$) δ 10.79 (d, J = 2.3 Hz, 1H), 8.83 (d, J = 45.1 Hz, 2H), 6.59 (d, J = 8.5 Hz, 1H), 6.34 (d, J = 1.9 Hz, 1H), 6.23 (dd, J = 8.5, 2.2 Hz, 1H), 4.22 (dd, J = 11.2, 4.7 Hz, 1H), 4.18 - 4.13 (m, 1H), 3.99 - 3.93 (m, 1H), 3.35 - 3.27 (m, 3H), 3.24 - 3.15 (m, 2H), 3.15 - 3.07 (m, 1H), 3.05 - 2.95 (m, 1H), 2.71 (dddd, J = 17.0, 11.7, 5.1, 1.2 Hz, 1H), 2.58 (dt, J = 17.5, 4.2 Hz, 1H), 2.08 (d, J = 6.4 Hz, 1H), 2.01 - 1.93 (m, 1H), 1.88 -1.79 (m, 2H).
LC-MS(ESI): $[M+H]^+$ = 331.39

Example 36

Synthesis of Compound C12

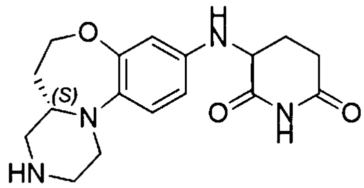

3-((S)-2,3,4,4a,5,6-Hexahydro-1H-benzo[b]pyrazino[1,2-d][1,4]oxazepin-9-yl)amino)piperidine-2,6-dione

Synthetic Scheme

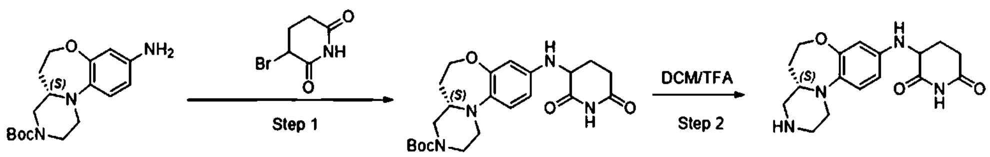


B11-4 C12-1 C12

The Synthetic method of compound C12 is the same as that of Compound C9.

$^1$H NMR (600 MHz, DMSO-$d_6$) δ 10.77 (d, J = 4.2 Hz, 1H), 8.76 (d, J = 41.0 Hz, 2H), 6.74 (d, J = 8.6 Hz, 1H), 6.29 (d, J = 8.6 Hz, 1H), 6.19 (d, J = 2.5 Hz, 1H), 4.44 (t, J = 10.1 Hz, 1H), 4.21 (dd, J = 11.3, 4.5 Hz, 1H), 4.04 - 3.97 (m, 1H), 3.29 (d, J = 10.4 Hz, 1H), 3.19 - 2.95 (m, 6H), 2.77 - 2.68 (m, 1H), 2.62 - 2.53 (m, 1H), 2.07 - 1.99 (m, 2H), 1.87 - 1.80 (m, 2H). LCMS (ESI): $[M+H]^+$ = 331.39

Example 37

Synthesis of Compound C13

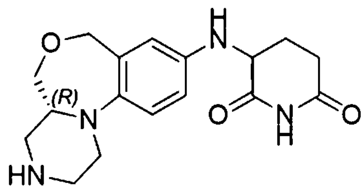

3-((R)-1,2,3,4,4a,5-Hexahydro-7H-benzo[e]pyrazino[2,1-c][1,4]oxazepin-9-yl)amino)piperidine-2,6-dione

Synthetic Scheme

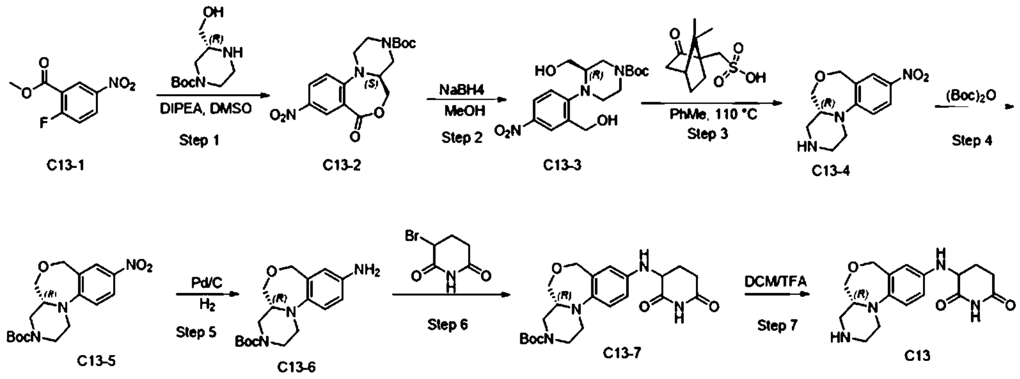


C13-1 C13-2 C13-3 C13-4 C13-5 C13-6 C13-7 C13

Step 1:

(S)-9-Nitro-7-oxo-1,2,4a,5-tetrahydro-7H-benzo[e]pyrazino[2,1-c][1,4]oxazepine-3(4H)-carboxylic acid tert-butyl ester (Compound C13-2)

The compound C13-1 (5.0 g, 25.11 mmol, 1.0 eq) was dissolved in dimethyl sulfoxide (80 mL). (R)-3-(Hydroxymethyl)piperazine-1-carboxylic acid (8.2 g, 37.66 mmol, 1.5 eq) and N,N-diisopropylethylamine (16.2 mg, 125.54 mmol, 5.0 eq) were added to the system. The reaction mixture was heated to 80°C and reacted at this temperature for 16 hours. Upon completion, the system was cooled to room temperature, quenched with water, and the aqueous phase was extracted three times with ethyl acetate. The combined organic phases were washed four times with saturated sodium chloride solution. The organic phase was concentrated under reduced pressure. The crude product obtained was purified by normal-phase column chromatography to obtain a yellow-green oily compound C13-2 (9.48 g, >99%).

$^1$H NMR (600 MHz, $CDCl_3$) δ 8.56 (d, *J* = 1.4 Hz, 1H), 8.32 (dd, *J* = 9.0, 1.4 Hz, 1H), 6.97 (d, *J* = 9.0 Hz, 1H), 4.50 (d, *J* = 13.7 Hz, 1H), 4.27 (d, *J* = 13.7 Hz, 1H), 4.14 (m, 2H), 3.41 (s, 1H), 3.28 (d, *J* = 10.5 Hz, 1H), 3.19 (dd, *J* = 11.7, 9.4 Hz, 2H), 3.02 (s, 1H), 1.49 (s, 9H).
LCMS (ESI): $[M-tBu+H]^+$ = 264.27

Step 2 to Step 5:

(R)-9-Amino-1,2,4a,5-tetrahydro-7H-benzo[e]pyrazino[2,1-c][1,4]oxepin-3(4H)-carboxylic acid tert-butyl ester

Refer to Step 3 to Step 6 of Compound B10 for preparation.
LCMS (ESI): $[M-tBu+H]^+$ = 320.38

Step 6 to Step 7:

3-((R)-1,2,3,4,4a,5-Hexahydro-7H-benzo[e]pyrazino[2,1-c][1,4]oxazepin-9-yl)amino)piperidine-2,6-dione (Compound C13)

Refer to Step 3 to Step 4 of Compound C9 for preparation.

$^1$H NMR (600 MHz, DMSO-$d_6$) δ 10.79 (d, J = 6.5 Hz, 1H), 8.82 (d, J = 40.7 Hz, 2H), 6.78 (d, J = 8.5 Hz, 1H), 6.69 - 6.60 (m, 2H), 4.89 (d, J = 11.2 Hz, 1H), 4.42 (dd, J = 11.3, 4.7 Hz, 1H), 4.25 (dd, J = 11.3, 4.8 Hz, 1H), 3.60 - 3.55 (m, 2H), 3.34 (d, J = 11.8 Hz, 1H), 3.30 - 3.25 (m, 2H), 3.35 - 3.10 (m, 3H), 2.83 (d, J = 10.3 Hz, 1H), 2.73 - 2.70 (m, 1H), 2.62 - 2.55 (m, 1H), 2.13 - 2.05 (m, 1H), 1.89 - 1.82 (m, 1H).
LCMS (ESI): $[M+H]^+$ = 331.30

Example 38

Synthesis of Compound C14

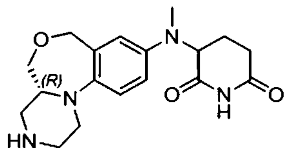

3-((R)-1,2,3,4,4a,5-Hexahydro-7H-benzo[e]pyrazino[2,1-c][1,4]oxazepin-9-yl)(methyl)amino)piperidine-2,6-dione

Synthetic Scheme

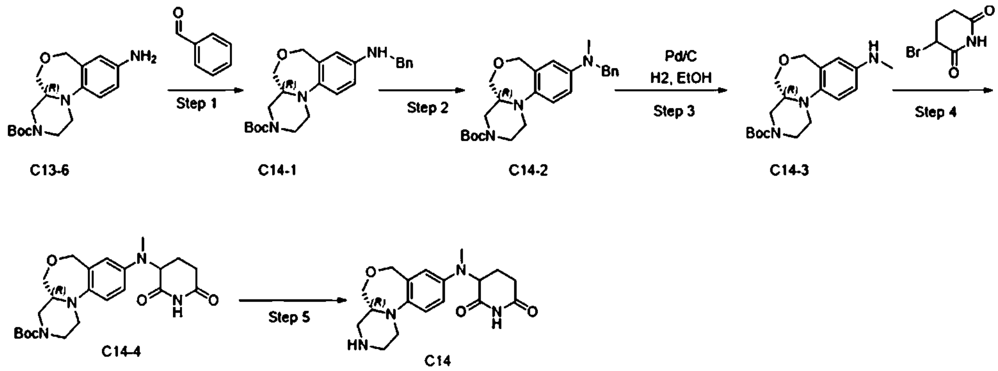


C13-6 C14-1 C14-2 C14-3 C14-4 C14

The synthetic method of Compound C9 is the same as that of Compound C10

$^1$H NMR (600 MHz, DMSO-$d_6$) δ 10.79 (s, 1H), 8.83 (d, J = 38.8 Hz, 2H), 6.85 (d, J = 8.4 Hz, 1H), 6.78 (d, J = 10.5 Hz, 2H), 4.91 (d, J = 11.3 Hz, 1H), 4.82 (dd, J = 12.6, 4.8 Hz, 1H), 4.50 (dd, J = 11.3, 3.0 Hz, 1H), 3.61 - 3.58 (m, 1H), 3.51 (d, J = 12.9 Hz, 1H), 3.32 - 3.26 (m, 3H), 3.23 - 3.10 (m, 3H), 2.90 - 2.77 (m, 2H), 2.70 (s, 3H), 2.55 (d, J = 16.6 Hz, 1H), 2.32 - 2.26 (m, 1H), 1.89 - 1.82 (m, 1H).
LCMS (ESI): $[M+H]^+$ = 345.39

Example 39

Synthesis of Compound C15

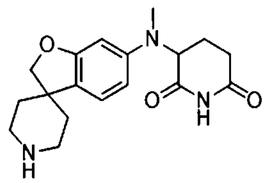

3-(Methyl(2H-spiro[benzofuran-3,4'-piperidine]-6-yl)amino)piperidine-2,6-dione

Synthetic Scheme

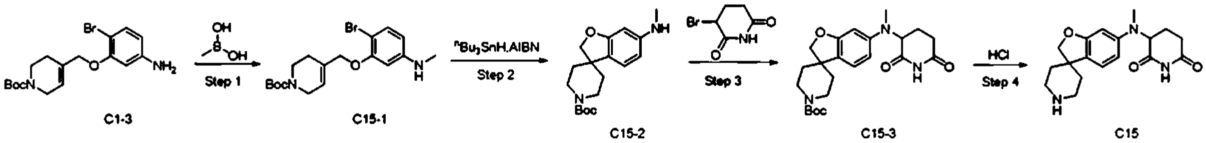


C1-3 C15-1 C15-2 C15-3 C15

Step 1:

4-((2-Bromo-5-(methylamino)phenoxy)methyl)-3,6-dihydropyridine-1(2H)-carboxylic acid tert-butyl ester (Compound C15-1)

The compound C1-3 (4.0 g, 10.4 mmol, 1.0 eq) was dissolved in 1,4-dioxane (40 mL). Copper acetate (2.8 g, 15.7 mmol, 1.5 eq) and pyridine (1.2 g, 15.7 mmol, 1.5 eq) were added, and the mixture was stirred at room temperature for 15 min. Methyl boronic acid (937 mg, 15.7 mmol, 1.5 eq) was added, and the mixture was allowed to react at 65°C for 12 hours. After cooling the reaction mixture to room temperature, methyl boronic acid (937 mg, 15.7 mmol, 1.5 eq) was added and the mixture reacted at 65°C for 12 hours. After cooling the reaction mixture to room temperature, the crude product was concentrated under reduced pressure and purified by column chromatography to obtain a yellow oily compound C15-1 (1.6 g, 38.6%).

$^1$H NMR (600 MHz, DMSO-$d_6$) δ 7.17 (d, *J* = 8.6 Hz, 1H), 6.25 (d, *J* = 2.5 Hz, 1H), 6.08 (dd, *J* = 8.6, 2.5 Hz, 1H), 5.86 - 5.82 (m, 2H), 4.46 (s, 2H), 3.90 - 3.83 (m, 2H), 3.48 - 3.42 (m, 2H), 2.65 (d, *J* = 5.0 Hz, 3H), 2.17 - 2.11 (m, 2H), 1.41 (s, 9H).

LCMS (ESI): $[M+H]^+$ = 399.25.

Step 2:

6-(Methylamino)-2H-spiro[benzofuran-3,4'-piperidine]-1'-carboxylic acid tert-butyl ester (Compound C15-2)

The compound C15-1 (1.0 g, 2.5 mmol, 1.0 eq) was dissolved in toluene (10 mL). Azobis(isobutyronitrile) (867 mg, 5.0 mmol, 2.0 eq) and tributylstannyl hydride (2.2 g, 7.6 mmol, 3.0 eq) were added at room temperature, and the mixture was allowed to react at 110°C for 15 hours. The reaction mixture was concentrated, and the crude product was purified by column chromatography to obtain a white oily compound C15-2 (500 mg, 62.4%).

$^1$H NMR (600 MHz, DMSO-$d_6$) δ 6.89 (d, *J* = 8.1 Hz, 1H), 6.03 (dd, *J* = 8.1, 2.0 Hz, 1H), 5.95 (d, *J* = 1.9 Hz, 1H), 5.54 (q, *J* = 5.1 Hz, 1H), 4.31 (s, 2H), 3.90 - 3.81 (m, 2H), 2.99 - 2.80 (m, 2H), 2.61 (d, *J* = 5.0 Hz, 3H), 1.69 - 1.51 (m, 6H), 1.42 (s, 9H).

LCMS (ESI) $[M+H]^+$= 319.35.

Step 3:

6-((2,6-Dioxopiperidin-3-yl)(methyl)amino)-2H-spiro[benzofuran-3,4'-piperidine]-1'-carboxylic acid tert-butyl ester (Compound C15-3)

The compound C15-2 (300 mg, 0.94 mmol, 1.0 eq) was dissolved in N, N,N-dimethylformamide (5 mL), and 3-bromopiperidine-2,6-dione (271 mg, 1.4 mmol, 1.5 eq) and sodium bicarbonate (158 mg, 1.9 mmol, 2.0 eq) were added. The mixture was allowed to react at 80°C for 12 hours. After cooling the reaction mixture to room temperature, water was added. The mixture was extracted with ethyl acetate, washed with saturated saline solution, and the combined organic phase was dried over anhydrous sodium sulfate. The crude product obtained from the concentrated organic phase was purified by column chromatography to obtain a light green solid compound C15-3 (150 mg, 37.1%).

LCMS (ESI) $[M+H]^+$= 440.49.

Step 6:

3-(Methyl(2H-spiro[benzofuran-3,4'-piperidine]-6-yl)amino)piperidine-2,6-dione (Compound C15)

The compound C15-3 (100 mg, 0.23 mmol, 1.0 eq) was dissolved in dichloromethane (1 mL), and a solution of hydrochloric acid in 1,4-dioxane (1 mL) was added. The mixture was reacted at room temperature for 2 hours. The reaction mixture was concentrated, and the crude product was purified by reverse-phase column chromatography to obtain a white solid compound C15 (50 mg, 65.2%).

$^1$H NMR (600 MHz, DMSO-$d_6$) δ 10.81 (s, 1H), 6.92 (d, *J* = 8.2 Hz, 1H), 6.36 - 6.30 (m, 2H), 4.83 (dd, *J* = 12.7, 5.0 Hz, 1H), 4.41 (s, 2H), 3.33 - 3.27 (m, 2H), 3.04 - 2.94 (m, 2H), 2.84 (ddd, *J* = 17.2, 13.7, 5.5 Hz, 1H), 2.69 (s, 3H), 2.57 - 2.51 (m, 1H), 2.29 (qd, *J* = 12.9, 4.4 Hz, 1H), 1.93 (td, *J* = 13.7, 13.3, 4.2 Hz, 2H), 1.88 - 1.81 (m, 1H), 1.81 - 1.73 (m, 2H).

LCMS (ESI) $[M+H]^+$= 330.35.

Example 40

Synthesis of Compound C16

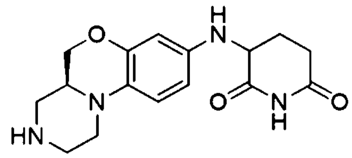

3-(((S)-1,2,3,4,4a,5-hexahydrobenzo[b]pyrazine[1,2-d][1,4]oxazine-8-yl)amino)piperidine-2,6-dione

Synthetic Scheme

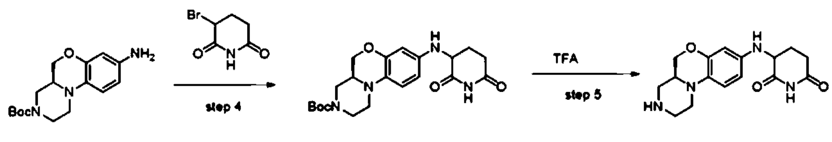


B1-4 C16-1 C16

Step 1:

(4aS)-8-((2,6-dioxopiperidin-3-yl)amino)-1,2,4a,5-tetrahydrobenzo[b]pyrazine[1,2-d][1,4]oxazine-3(4H)-carboxylic acid tert-butyl ester (Compound C16-1)

B1-4 (0.24 g, 0.78 mmol, 1 eq) was dissolved in anhydrous N,N-dimethylformamide (10 mL). 3-bromopiperidine-2,6-dione (0.23 g, 1.18 mmol, 1.5 eq) and sodium bicarbonate (0.13 g, 1.57 mmol, 2 eq) were added in the system, and reacted the mixture at 80°C for 12 h. The reaction mixture was filtered, and the resulting solution was purified by reverse-phase column chromatography to obtain a black oily compound C16-1 (0.29 g, 88.6%).

LCMS (ESI) $[M+H]^+$= 417.39.

Step 2:

3-(((S)-1,2,3,4,4a,5-hexahydrobenzo[b]pyrazine[1,2-d][1,4]oxazine-8-yl)amino)piperidine-2,6-dione (Compound C16)

The compound C16-1 (0.29 g, 0.69 mmol, 1.0 eq) was dissolved in dichloromethane (2 mL), and trifluoroacetic acid (2 mL) was added in the mixture. The mixture reacted at room temperature for 1 h. The reaction mixture was concentrated under reduced pressure and purified by preparative HPLC to obtain a white solid compound C16 (0.23 g, 76.8%).

$^1$H NMR (600 MHz, DMSO-d6) δ 10.77 (s, 1H), 8.96-8.89 (m, 1H), 8.89-8.82 (m, 1H), 6.72 (d, J = 8.8 Hz, 1H), 6.28-6.24 (m, 1H), 6.21-6.18 (m, 1H), 4.23 (dd, J = 10.9, 2.6 Hz, 1H), 4.18 (dd, J = 11.3, 4.8 Hz, 1H), 3.90 (dd, J = 10.8, 8.3 Hz, 1H), 3.84 (d, J = 13.1 Hz, 1H), 3.40 (d, J = 12.6 Hz, 1H), 3.33 (d, J = 12.1 Hz, 1H), 3.19-3.13 (m, 1H), 3.12-3.03 (m, 1H), 2.81 - 2.68 (m, 3H), 2.57 (dt, J = 17.6, 4.4 Hz, 1H), 2.10-2.04 (m, 1H), 1.83 (qd, J = 12.0, 4.6 Hz, 1H)..
LCMS (ESI) $[M+H]^+$= 317.38

Example 41

Synthesis of Compound D1

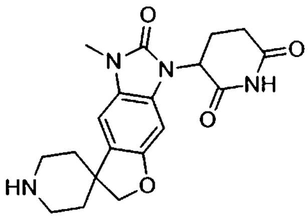

3-(1-Methyl-2-oxo-1,2-dihydro-3H,6H-spiro[benzofuran[5,6-d]imidazole-7,4'-piperidine]-3-yl)piperidine-2,6-dione

Synthetic Scheme

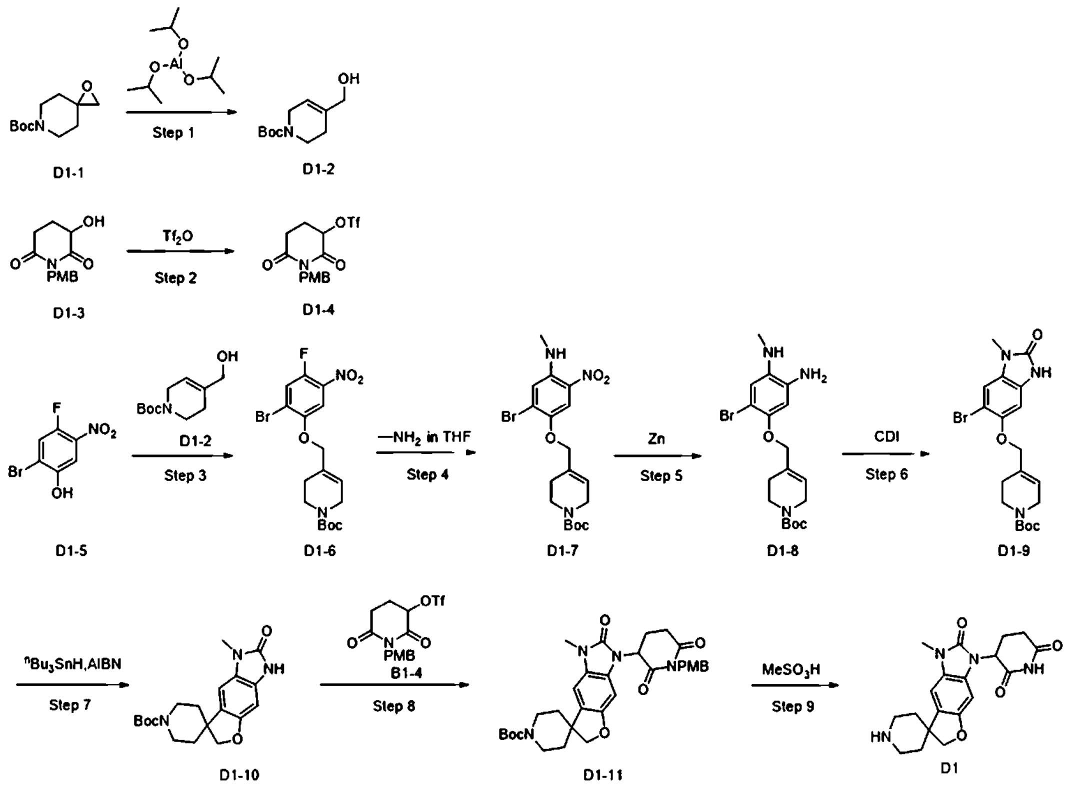


Step 1: 4-(Hydroxymethyl)-3,6-dihydropyridine-1(2H)-carboxylic acid tert-butyl ester (Compound D1-2)

Compound D1-1 (10.0 g, 46.9 mmol) was dissolved in toluene (100 mL). Aluminum isopropoxide (12.5 g, 61.0 mmol) was added, and reacted at 120°C for 24 hours. The reaction mixture was cooled to room temperature, and dilute hydrochloric acid (1.0 mol/L, 200 mL) was added. The mixture was extracted three times with ethyl acetate, and the organic layer was collected. The crude product obtained from the concentrated organic phase was purified by column chromatography to obtain a colorless oily compound D1-2 (4.7 g, 47%).

$^1$H NMR (600 MHz, $CDCl_3$) δ 5.67 (s, 1H), 4.07 (s, 2H), 3.98 - 3.92 (m, 2H), 3.54 (dd, $J$ = 8.2, 4.0 Hz, 2H), 2.15 (d, $J$ = 6.8 Hz, 2H), 1.49 (s, 9H).
LC-MS(ESI): $[M-tBu+H]^+$ = 158.20

Step 2:

1-(4-Methoxybenzyl)-2,6-dioxopiperidin-3-yl trifluoromethanesulfonate (Compound D1-4)

The compound D1-3 (1.0 g, 4.0 mmol) was dissolved in ultradry dichloromethane (10 mL) and the reaction was

cooled to -10°C under nitrogen. At -10°C,pyridine (634 mg, 8.0 mmol) and trifluoromethanesulfonyl trifluoromethanesulfonate (1.7 g, 6.0 mmol) were added to the reaction mixture and reacted at this temperature for 2 hours. The reaction mixture was concentrated, and the crude product was purified by column chromatography to obtain a pale yellow solid compound D1-4 (1.2 g, 78%).

LC-MS(ESI): $[M+H]^+$= 382.20.

Step 3:

4-((2-Bromo-4-fluoro-5-nitrophenoxy)methyl)-3,6-dihydropyridine-1(2H)-carboxylic acid tert-butyl ester (Compound D1-6)

Compound D1-5 (500 mg, 2.8 mmol), Compound D1-2 (587 mg, 2.8 mmol), and triphenylphosphine (1.1 g, 4.2 mmol) were dissolved in tetrahydrofuran (5 mL). The mixture was stirred at 0°C under nitrogen for 15 min. Diethyl azodicarboxylate (738 mg, 4.2 mmol) was slowly added dropwise to the reaction system. and allowed to react at room temperature for 15 hours. The reaction mixture was concentrated, and the crude product was purified by column chromatography to obtain a white solid compound D1-6 (645 mg, 71%).

$^1H$ NMR (600 MHz, $CDCl_3$) δ 7.60 - 7.57 (m, 2H), 5.95 - 5.87 (m, 1H), 4.57 (s, 2H), 4.02 - 3.98 (m, 2H), 3.62 - 3.57 (m, 2H), 2.28 - 2.23 (m, 2H), 1.50 (s, 9H).

LC-MS(ESI): $[M-Boc+H]^+$ = 331.15.

Step 4:

4-((2-Bromo-4-(methylamino)-5-nitrophenoxy)methyl)-3,6-dihydropyridine-1(2H)-carboxylic acid tert-butyl ester (Compound D1-7)

The compound D1-6 (500 mg, 21.6 mmol) was dissolved in tetrahydrofuran (5 mL), methylamine in tetrahydrofuran solution (2 M, 5 mL)was added , and the mixture was allowed to reacted at 60°C for 15 hours. The reaction mixture was cooled to room temperature, saturated sodium bicarbonate aqueous solution was added, and extracted with ethyl acetate. The mixture was combined with organic layers, and were dried over anhydrous sodium sulfate. The crude product obtained from the concentrated organic phase was purified by column chromatography to obtain an orange-red oily compound D1-7 (450 mg, 88%).

$^1H$ NMR (600 MHz, DMSO-$d_6$) δ 8.10 - 8.05 (m, 1H), 7.66 (s, 1H), 7.30 (s, 1H), 5.89 - 5.83 (m, 1H), 4.53 (s, 2H), 3.89 - 3.83 (m, 2H), 3.48 - 3.40 (m, 2H), 2.97 - 2.93 (m, 3H), 2.17 - 2.12 (m, 2H), 1.41 (s, 9H).

LC-MS(ESI): $[M-Boc+H]^+$ = 341.99.

Step 5:

4-((5-Amino-2-bromo-4-(methylamino)phenoxy)methyl)-3,6-dihydropyridine-1(2H)-carboxylic acid tert-butyl ester (Compound D1-8)

The compound D1-7 (400 mg, 0.9 mmol) was dissolved in methanol:ammonium chloride solution = 2:1 (6 mL), and zinc powder (237 mg, 3.6 mmol) was added. The reaction mixture was stirred at room temperature for 12 hours. Water was added to the reaction mixture. The organic layer was extracted three times with ethyl acetate, combined, dried over anhydrous sodium sulfate, and concentrated to obtain a crude compound D1-8 (400 mg).

LC-MS(ESI): $[M-Boc+H]^+$ = 312.18.

Step 6:

4-(((6-bromo-1-methyl-2-oxo-2,3-dihydro-1H-benzo[d]imidazol-5-yl)oxy)methyl)-3,6-dihydropyridine-1(2H)-carboxylic acid tert-butyl ester (Compound D1-9)

The compound D1-8 (400 mg, 1.0 mmol) was dissolved in acetonitrile (5 mL), and carbonyl diimidazole (315 mg, 2.0 mmol) was added. The mixture was allowed to reacted under nitrogen at 85°C for 2 hours. The reaction mixture was concentrated, and the crude product was purified by column chromatography to obtain a white solid compound D1-9 (300 mg, 71%).

$^1H$ NMR (600 MHz, DMSO-$d_6$) δ 10.93 (s, 1H), 7.34 (s, 1H), 6.78 (s, 1H), 5.87 - 5.79 (m, 1H), 4.50 (s, 2H), 3.89 - 3.83 (m, 2H), 3.48 - 3.42 (m, 2H), 3.24 (s, 3H), 2.18 - 2.13 (m, 2H), 1.41 (s, 9H).

LC-MS(ESI): $[M-Boc+H]^+$= 338.19.

Step 7:

1-Methyl-2-oxo-2,3-dihydro-1H,6H-spiro[benzofuran[5,6-d]imidazole-7,4'-piperidine]-1'-carboxylic acid tert-butyl ester (Compound D1-10)

The compound D1-9 (200 mg, 0.5 mmol) was dissolved in toluene (2 mL). Azobis(isobutyronitrile) (150 mg, 0.9 mmol) and tributylstannyl hydride (398 mg, 1.4 mmol) were added at room temperature. The mixture was reacted at 110°C for 15 hours. The reaction mixture was concentrated, and the crude product was purified by column chromatography to obtain a white solid compound D1-10 (100 mg, 61%).

$^1$H NMR (600 MHz, DMSO-$d_6$) δ 10.64 (s, 1H), 7.03 (s, 1H), 6.43 (s, 1H), 4.38 (s, 2H), 3.99 - 3.89 (m, 2H), 3.23 (s, 3H), 2.09 - 2.07 (m, 2H), 1.80 - 1.74 (m, 2H), 1.64 - 1.58 (m, 2H), 1.43 (s, 9H).

LC-MS(ESI): $[M+H]^+$= 360.39.

Step 8:

3-(1-(4-Methoxybenzyl)-2,6-dioxopiperidin-3-yl)-1-methyl-2-oxo-2,3-dihydro-1H, 6H-spiro[benzofuran[5,6-d]imidazole-7,4'-piperidine]-1'-carboxylic acid tert-butyl ester (Compound D1-11)

The compound D1-10 (100 mg, 0.3 mmol) and potassium tert-butoxide (38 mg, 0.3 mmol) were dissolved in ultradry tetrahydrofuran (1 mL). The mixture was stirred at 0°C under nitrogen for 2 hours, followed by addition of a solution of compound D1-4 in ultradry tetrahydrofuran (1 mL). The reaction mixture was allowed to react at room temperature for 2 hours. The reaction mixture was concentrated, and the crude product was purified by reverse-phase column chromatography to obtain a yellow solid compound D1-11 (90 mg, 55%).

$^1$H NMR (600 MHz, DMSO-$d_6$) δ 7.22 - 7.19 (m, 2H), 7.16 (s, 1H), 6.87 - 6.83 (m, 2H), 6.60 (s, 1H), 5.45 (dd, *J* = 13.0, 5.4 Hz, 1H), 4.83 (d, *J* = 14.4 Hz, 1H), 4.75 (d, *J* = 14.4 Hz, 1H), 4.43 - 4.37 (m, 2H), 4.00 - 3.89 (m, 2H), 3.72 (s, 3H), 3.30 (s, 3H), 3.07 - 2.99 (m, 1H), 2.98 - 2.83 (m, 2H), 2.83 - 2.76 (m, 2H), 2.76 - 2.69 (m, 1H), 1.83 - 1.74 (m, 2H), 1.67 - 1.60 (m, 2H), 1.43 (s, 9H).
LC-MS(ESI): $[M-Boc+H]^+$= 491.19

Step 9:

3-(1-Methyl-2-oxo-1,2-dihydro-3H,6H-spiro[benzofuran[5,6-d]imidazo[7,4']-piperidin-3-yl)piperidine-2,6-dione (Compound D1)

The compound D1-11 (50 mg, 0.08 mmol) was dissolved in toluene (0.4 mL). Methylsulfonic acid (0.2 mL) was added at room temperature, and the mixture was reacted at 120°C for 2 hours under nitrogen protection. The reaction mixture was concentrated, and the crude product was purified by high-pressure preparative liquid chromatography phase to obtain a white solid compound D1 (15 mg, 48%).

$^1$H NMR (600 MHz, DMSO-$d_6$) δ 11.07 (s, 1H), 8.69 - 8.63 (m, 1H), 6.96 (s, 1H), 6.73 (s, 1H), 5.31 (dd, *J* = 13.0, 5.5 Hz, 1H), 4.49 - 4.43 (m, 2H), 3.39 - 3.31 (m, 5H), 3.06 - 2.98 (m, 2H), 2.87 (ddd, *J* = 16.8, 13.6, 5.3 Hz, 1H), 2.72 (qd, *J* = 13.0, 4.4 Hz, 1H), 2.64 - 2.57 (m, 1H), 2.09 - 2.01 (m, 2H), 2.00 - 1.94 (m, 1H), 1.87 - 1.81 (m, 2H).
LC-MS(ESI): $[M+H]^+$= 371.29

Example 42:

Synthesis of Compound D2

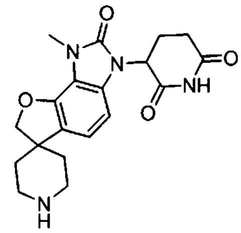

3-(1-Methyl-2-oxo-1,2-dihydro-3H,7H-spiro[benzofuro[6,7-d]imidazole-6,4'-piperidine]-3-yl)piperidine-2,6-dione

Synthetic Scheme

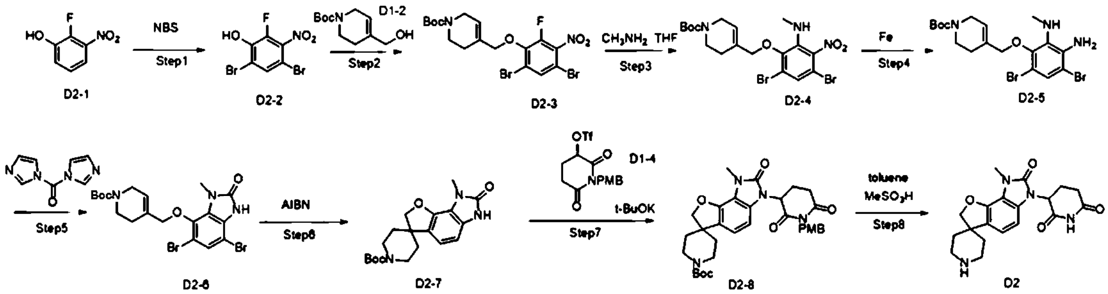


Step 1: 4,6-dibromo-2-fluoro-3-nitrophenol (Compound D2-2)

The compound D2-1 (2.5 g, 15.9 mmol) was dissolved in methanol (25 mL). N-bromobutanedicarboximide (6.2 g, 35.0 mmol) and trifluoroacetic acid (471 mg, 4.1 mmol) were added to the reaction mixture. The reaction mixture was heated to 25°C and reacted at this temperature for 3 hours. The resulting reaction mixture was concentrated under reduced pressure. The crude product obtained was purified by column chromatography to afford the yellow oily compound D2-2 (3.9 g, 77.8%).

$^1$H NMR (600 MHz, DMSO-$d_6$) δ 11.98 (s, 1H), 8.04 - 7.91 (m, 1H).
LC-MS(ESI): [M-H]$^-$ =313.98

Step 2:

4-((4,6-dibromo-2-fluoro-3-nitrophenoxy)methyl)-3,6-dihydropyridine-1(2H)-carboxylic acid tert-butyl ester (Compound D2-3)

The compound D2-2 (3.9 g, 12.4 mmol), compound D1-2 (3.4 g, 16.1 mmol), and triphenylphosphine (6.5 g, 24.8 mmol) were dissolved in tetrahydrofuran (40 mL). The mixture was cooled to 0°C under nitrogen protection, and diethyl azodicarboxylate (4.3 g, 24.8 mmol) was added. After completion of the addition, the mixture was recovered to room temperature and reacted for 10 hours. The reaction mixture was concentrated under reduced pressure. The crude product obtained was purified by column chromatography to obtain a yellow oily compound D2-3 (4.4 g, 69.6%).

$^1$H NMR (600 MHz, DMSO-$d_6$) δ 8.19 (d, $J$ = 1.8 Hz, 1H), 5.86 (s, 1H), 4.64 (s, 2H), 3.85 (s, 2H), 3.44 (t, $J$ = 5.7 Hz, 2H), 2.22 (dt, $J$ = 7.0, 3.7 Hz, 2H), 1.41 (s, 9H).
LC-MS(ESI): [M-Boc+H]$^+$ = 411.19

Step 3:

4-((4,6-dibromo-2-methylamino-3-nitrophenoxy)methyl)-3,6-dihydropyridine-1(2H)-carboxylic acid tert-butyl ester (Compound D2-4)

The compound D2-3 (4.4 g, 8.6 mmol) was dissolved in tetrahydrofuran (88 mL). A solution of methylamine in tetrahydrofuran (44 mL) was added. The reaction mixture was heated to 65°C and reacted at this temperature for 10 hours.

The reaction mixture was concentrated under reduced pressure. The crude product was purified by column chromatography to obtain a yellow solid compound D2-4 (4 g, 88.9%).

$^1H$ NMR (600 MHz, DMSO-$d_6$) δ 7.28 (s, 1H), 6.06 (q, *J* = 5.4 Hz, 1H), 5.85 (s, 1H), 4.31 (s, 2H), 3.87 (s, 2H), 3.46 (d, *J* = 5.9 Hz, 2H), 2.69 (d, *J* = 5.3 Hz, 3H), 2.25 (q, *J* = 4.5 Hz, 2H), 1.42 (s, 9H).

LC-MS(ESI): $[M\text{-}Boc+H]^+$ = 422.19.

Step 4:

4-((3-amino-4,6-dibromo-2-(methylamino)phenoxy)methyl)-3,6-dihydropyridine-1(2H)-carboxylic acid tert-butyl ester (Compound D2-5)

The compound D2-4 (4 g, 7.7 mmol), iron powder (2.1 g, 38.4 mmol), and ammonium chloride (2.1 g, 38.4 mmol) were dissolved in ethanol (40 mL) and water (80 mL). The reaction mixture was reacted at room temperature for 2 hours. The reaction mixture was filtered, and the filtrate was concentrated under reduced pressure to obtain a crude yellow oily compound D2-5 (3.6 g, 95.7%).
LC-MS(ESI): $[M\text{-}tBu+H]^+$= 436.19

Step 5:

4-((5,7-dibromo-3-methyl-2-oxo-2,3-dihydro-1H-benzimidazol-4-yl)oxy)methyl)-3,6-dihydropyridine-1(2H)-carboxylic acid tert-butyl ester (Compound D2-6)

The compound D2-5 (3.6 g, 7.3 mmol) was dissolved in acetonitrile (40 mL) with N,N'-carbonyldiimidazole (2.4 g, 14.7 mmol) in acetonitrile (40 mL) and reacted at 85°C under nitrogen for 12 hours. The reaction mixture was cooled to room temperature and concentrated under reduced pressure. The crude product was purified by column chromatography to obtain a yellow solid compound D2-6 (1.4 g, 37.5%).

$^1H$ NMR (600 MHz, DMSO-$d_6$) δ 11.54 (s, 1H), 7.43 (s, 1H), 5.91 (s, 1H), 4.40 (s, 2H), 3.89 (s, 2H), 3.48 (s, 2H), 3.44 (s, 3H), 2.26 (s, 2H), 1.42 (s, 9H).

LC-MS(ESI): $[M\text{-}Boc+H]^+$= 418.29.

Step 6:

1-methyl-2-oxo-2,3-dihydro-1H,7H-spiro[benzofuran[6,7-d]imidazole-6,4'-piperidine]-1'-carboxylic acid tert-butyl ester (Compound D2-7)

The compound D2-6 (1.2 g, 2.4 mmol), tri-n-butylstannyl hydride (2.1 g, 7.1 mmol), and azobisisobutyronitrile (775 mg, 4.7 mmol) were dissolved in toluene (20 mL). The reaction mixture was heated to 110°C and reacted at this temperature for 12 hours. After the reaction system was cooled to room temperature, the crude product obtained after concentrating the reaction mixture under reduced pressure was purified by column chromatography to obtain a white solid compound D2-7 (425 mg, 50.1%).

$^1H$ NMR (600 MHz, DMSO-$d_6$) δ 10.76 (s, 1H), 6.82 (d, *J* = 7.8 Hz, 1H), 6.51 (d, *J* = 7.8 Hz, 1H), 4.54 - 4.53 (m, 2H), 4.08 - 3.79 (m, 2H), 3.37 (s, 3H), 2.88 (brs, 2H), 1.73 - 1.62 (m, 4H), 1.42 (s, 9H).
LC-MS(ESI): $[M+H]^+$= 360.39

Step 7:

3-(1-(4-methoxybenzyl)-2,6-dioxopiperidin-3-yl)-1-methyl-2-oxo-2, 3-dihydro-1H,7H-spiro[benzofuran[6,7-d]imidazole-6,4'-piperidine]-1'-carboxylic acid tert-butyl ester (Compound D2-8)

The compound D2-7 (420 mg, 1.2 mmol) was dissolved in tetrahydrofuran (5 mL), and potassium tert-butoxide (158 mg, 1.4 mmol) was added to the system. Under nitrogen atmosphere, the mixture was cooled to 0°C and maintained at this temperature for 2 hours. 3d (535 mg, 1.4 mmol) dissolved in tetrahydrofuran (5 mL) was added dropwise to the mixture at 0°C. The reaction was recovered to proceed at room temperature for 5 hours. The saturated ammonium chloride aqueous solution (10 mL) was added to the reaction mixture,extracted three times with ethyl acetate, combined organic layers, washed with saturated saline solution, and dried over anhydrous sodium sulfate. The crude product obtained from the concentrated organic phase was purified by column chromatography to give a white solid compound D2-8 (400 mg, 57.9%).

$^1$H NMR (600 MHz, DMSO-$d_6$) δ 7.29 - 7.13 (m, 2H), 6.86 - 6.81 (m, 3H), 6.63 - 6.46 (m, 1H), 5.47 (dd, *J* = 13.3, 5.4 Hz, 1H), 4.89 - 4.72 (m, 2H), 4.56 - 4.53 (m, 2H), 3.92 (brs, 2H), 3.73 (s, 3H), 3.44 (s, 3H), 3.08 - 3.02 (m, 1H), 2.88 (brs, 2H), 2.11 - 1.85 (m, 2H), 1.73 - 1.65 (m, 5H), 1.42 (d, *J* = 2.6 Hz, 9H).
LC-MS(ESI): [M-Boc+H]$^+$= 491.49

Step 8:

3-(1-methyl-2-oxo-1,2-dihydro-3H,7H-spiro)benzofuran[6,7-d]imidazole-6,4'-piperidine]-3-yl)piperidine-2,6-dione (Compound D2)

The compound D2-8 (380 mg, 1.2 mmol) was dissolved in toluene (10 mL), and methylsulfonic acid (2.5 mL) was added to the system. Under nitrogen atmosphere, the mixture was heated to 120°C and reacted for 2 hours. The reaction mixture was cooled to room temperature and concentrated under reduced pressure. The crude product was purified by reverse-phase column chromatography and lyophilized to obtain a white solid compound D2 (25 mg, 10.5%).

$^1$H NMR (600 MHz, DMSO-$d_6$) δ 11.09 (s, 1H), 6.83 (d, *J* = 8.0 Hz, 1H), 6.67 (d, *J* = 8.0 Hz, 1H), 5.33 (dd, *J* = 12.7, 5.5 Hz, 1H), 4.60 (s, 2H), 3.19 (dd, *J* = 12.7, 5.5 Hz, 2H), 2.93 - 2.84 (m, 4H), 2.72 - 2.57 (m, 2H), 2.04 - 1.89 (m, 4H), 1.83 - 1.73 (m, 2H)
LC-MS(ESI): [M+H]$^+$= 371.39

Example 43

Synthesis of Compound D3

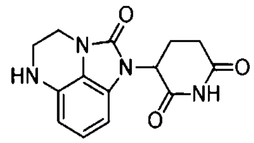

3-(2-oxo-5,6-dihydro-4H-imidazo[1,5,4-de]quinoxalin-1(2H)-yl)piperidine-2,6-dione

Synthetic Scheme

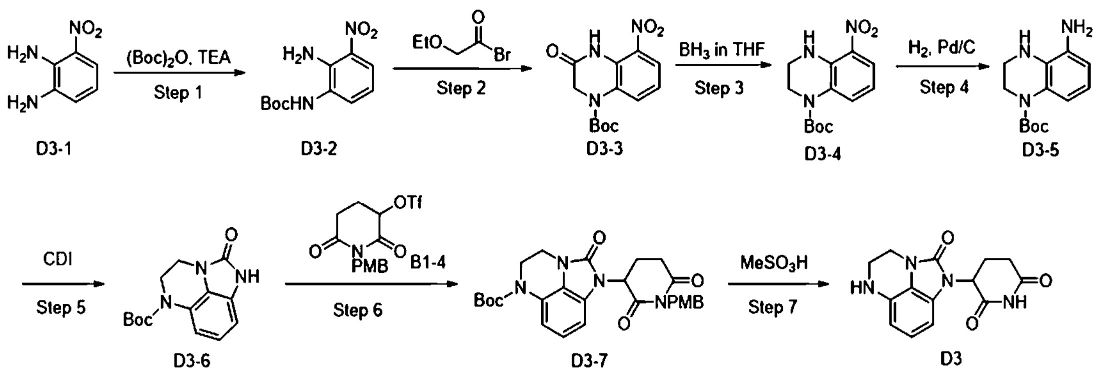


Step 1: (2-amino-3-nitrophenyl)carbamic acid tert-butyl ester (Compound D3-2)

The compound D3-1 (5.0 g, 32.7 mmol) was dissolved in tetrahydrofuran (50 mL). Under stirring at room temperature, triethylamine (3.3 g, 32.7 mmol) and di-tert-butyl dicarbonate (8.6 g, 39.2 mmol) were added. Under nitrogen protection,the mixture was reacted at 65°C for 18 hours. The reaction mixture was cooled to room temperature, and di-tert-butyl dicarbonate (2.9 g, 13.1 mmol) was added under strirring. The reaction mixture was reacted under nitrogen protection at 65°C for 18 hours. It was concentrated, and the crude product was purified by column chromatography to obtain an

orange solid compound D3-2 (5.0 g, 60%).

$^1H$ NMR (600 MHz, $CDCl_3$) δ 8.07 (d, *J* = 8.6 Hz, 1H), 7.50 (d, *J* = 7.6 Hz, 1H), 6.73 (t, *J* = 8.1 Hz, 1H), 6.27 (s, 2H), 6.01 (s, 1H), 1.54 (s, 9H).

LC-MS(ESI): $[M+H]^+$ = 254.25.

Step 2: 5-nitro-3-oxo-3,4-dihydroquinoxaline-1(2H)-carboxylic acid tert-butyl ester (Compound D3-3)

The sodium hydride (1.66 g, 41.5 mmol) was added to ultradry tetrahydrofuran (20 mL). The mixture was cooled to -60°C under nitrogen and maintained at this temperature. A solution of compound D3-2 (5.0 g, 19.7 mmol) dissolved in anhydrous tetrahydrofuran was added. After being stirred at -60°C for 30 minutes, ethyl bromoacetate (3.6 g, 21.7 mmol) was slowly added dropwise. The reaction mixture was allowed to react at room temperature for 16 hours. The reaction mixture was quenched with water, extracted three times with ethyl acetate, and the organic phase was collected. The crude product was concentrated and purified by column chromatography to obtain a yellow solid compound D3-3 (5.4 g, 93%).

$^1H$ NMR (600 MHz, $CDCl_3$) δ 10.23 (s, 1H), 8.10 (dd, *J* = 8.5, 1.3 Hz, 1H), 7.98 (d, *J* = 7.7 Hz, 1H), 7.19 (td, *J* = 8.3, 1.1 Hz, 1H), 4.46 (d, *J* = 1.1 Hz, 2H), 1.56 (s, 9H).

LC-MS(ESI): $[M-tBu+H]^+$ = 238.16.

Step 3: 5-nitro-3,4-dihydroquinoxaline-1(2H)-carboxylic acid tert-butyl ester (Compound D3-4)

The compound D3-3 (5.0 g, 17.1 mmol) was dissolved in tetrahydrofuran (50 mL), and borane-tetrahydrofuran complex (1 M, 50 mL) was added. The mixture was reacted at room temperature for 1 hour. The reaction was quenched with methanol, and the crude product was purified by column chromatography to obtain a yellow solid compound D3-4 (3.6 g, 76%).

$^1H$ NMR (600 MHz, $CDCl_3$) δ 8.36 (s, 1H), 8.00 - 7.95 (m, 1H), 7.65 (s, 1H), 6.68 - 6.58 (m, 1H), 3.82 (t, *J* = 5.2 Hz, 2H), 3.68 - 3.59 (m, 2H), 1.54 (s, 9H).

LC-MS(ESI): $[M-tBu+H]^-$= 224.20.

Step 4: 5-amino-3,4-dihydroquinoxaline-1(2H)-carboxylic acid tert-butyl ester (Compound D3-5)

The compound D3-4 (3.0 g, 10.7 mmol) was dissolved in methanol: dichloromethane = 3:1 (30 mL), and palladium carbon (600 mg) was added. The mixture was reacted at room temperature under hydrogen atmosphere for 15 hours. The reaction mixture was filtered through diatomaceous earth, and the filtrate was concentrated to obtain a crude white solid compound D3-5 (2.0 g).

LC-MS(ESI): $[M-Boc+H]^+$ = 150.12.

Step 5: 2-oxo-1,2,4,5-tetrahydro-6-imidazo[1,5,4-de]quinoxaline-6-carboxylic acid tert-butyl ester (Compound D3-6)

The compound D3-5 (2.0 g, 8.0 mmol) was dissolved in acetonitrile (20 mL), and carbonyl diimidazole (2.6 g, 16.0 mmol) was added. It was reacted under nitrogen at 85°C for 2 hours. The reaction mixture was concentrated, andthe crude product was purified by column chromatography to obtain a yellow solid compound D3-6 (1.6 g, 72%).

$^1H$ NMR (600 MHz, DMSO-$d_6$) δ 10.77 (s, 1H), 7.28 (s, 1H), 6.90 (t, *J* = 8.1 Hz, 1H), 6.70 (d, *J* = 7.7 Hz, 1H), 3.92 (t, *J* = 5.1 Hz, 2H), 3.82 (dd, *J* = 5.9, 4.3 Hz, 2H), 1.52 (s, 9H).

LC-MS(ESI): $[M+H]^+$= 276.30.

Step 6:

1-(4-methoxybenzyl)-3-(6-(4-methylbenzyl)-2-oxo-5,6-dihydro-4H-imidazo[1,5,4-de]quinoxalin-1(2H)-yl)piperidine-2,6-dione (Compound D3-7)

The compound D3-6 (1 g, 3.6 mmol) and potassium tert-butylate (489 mg, 4.4 mmol) were dissolved in anhydrous tetrahydrofuran (10 mL). The mixture was stirred at 0°C under nitrogen protection for 2 hours. A solution of compound D1-4 (1.7 g, 4.4 mmol) in anhydrous tetrahydrofuran (5 mL) was then added. The reaction mixture was allowed to react at room temperature for 2 hours. The reaction mixture was concentrated, and the crude product was purified by reverse-phase column chromatography to obtain a yellow solid compound D3-7 (750 mg, 41%).

$^1H$ NMR (600 MHz, DMSO-$d_6$) δ 7.34 (s, 1H), 7.23 - 7.20 (m, 2H), 6.95 - 6.90 (m, 1H), 6.89 - 6.84 (m, 2H), 6.75 - 6.70 (m, 1H), 5.50 (dd, *J* = 13.2, 5.4 Hz, 1H), 4.86 - 4.75 (m, 2H), 4.00 - 3.94 (m, 2H), 3.92 - 3.87 (m, 2H), 3.73 (s, 3H), 3.08 (ddd, *J* = 17.1, 13.8, 5.4 Hz, 1H), 2.86 - 2.81 (m, 1H), 2.72 (qd, *J* = 13.2, 4.4 Hz, 1H), 2.12 - 2.05 (m, 1H), 1.52 (s, 9H).

LC-MS(ESI): $[M+H]^+$= 507.35.

Step 7:

3-(2-oxo-5,6-dihydro-4H-imidazo[1,5,4-de]quinoxalin-1(2H)-yl)piperidine-2,6-dione (Compound D3)

The compound D3-7 (500 mg, 1.0 mmol) was dissolved in toluene (2 mL). Methyl sulfonic acid (1 mL) was added at room temperature. It was reacted under nitrogen at 120°C for 2 hours. The reaction mixturewas concentrated, and the crude product was purified by preparative HPLC to obtain a white solid compound D3 (100 mg, 35%).

$^1$H NMR (600 MHz, DMSO-$d_6$) δ 11.06 (s, 1H), 6.74 (t, *J* = 7.9 Hz, 1H), 6.40 (d, *J* = 7.9 Hz, 1H), 6.33 (d, *J* = 7.9 Hz, 1H), 6.02 - 6.00 (m, 1H), 5.29 - 5.24 (m, 1H), 3.81 (dd, *J* = 6.0, 4.1 Hz, 2H), 3.40 - 3.35 (m, 2H), 2.95 - 2.87 (m, 1H), 2.70 - 2.57 (m, 2H), 2.04 - 1.98 (m, 1H).
LC-MS(ESI): $[M+H]^+$= 287.25

Example 44

Synthesis of Compound D4

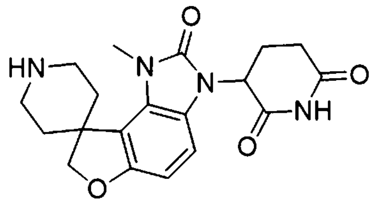

3-(1-Methyl-2-oxo-1,2-dihydro-3H,7H-spiro[benzofuran[4,5-d]imidazole-8,4'-piperidine]-3-yl)piperidine-2,6-dione

Synthetic Scheme

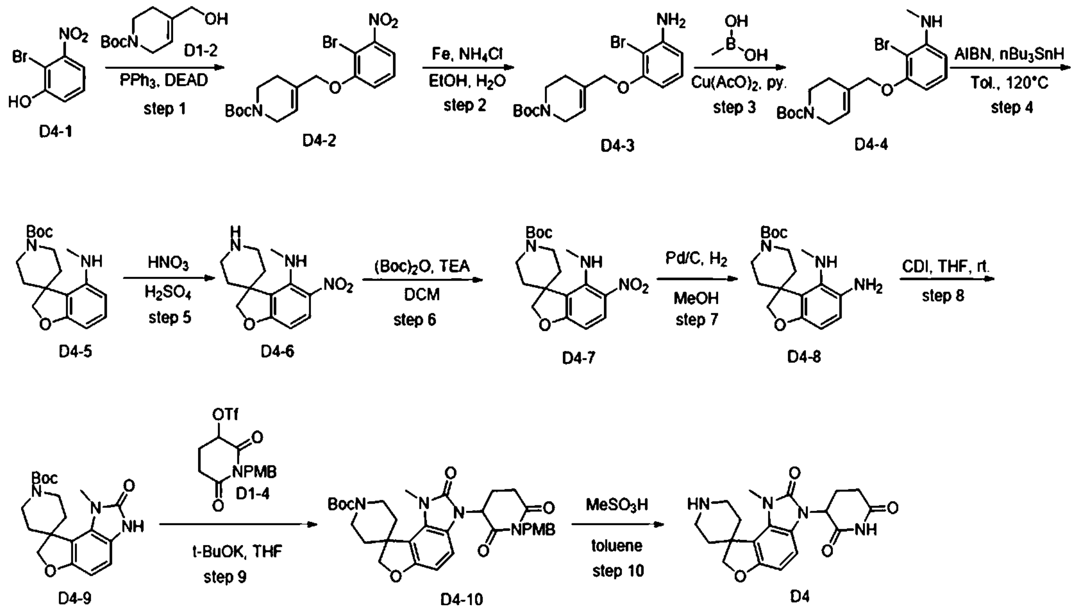


Step 1:

4-((2-bromo-3-nitrophenoxy)methyl)-3,6-dihydropyridine-1(2H)-carboxylic acid tert-butyl ester (Compound D4-2)

The compound D4-1 (10.0 g, 45.8 mmol) and D1-2 (14.7 g, 68.8 mmol) were dissolved in tetrahydrofuran (100 mL). Triphenylphosphine (18 g, 68.8 mmol) was added with stirring at room temperature, followed by diethyl azodicarboxylate (12 g, 68.8 mmol) was added dropwise under an ice bath. The reaction was allowed to proceed at 23°C under nitrogen protection for 18 hours. The reaction mixture was concentrated, and the crude product was purified by column chromatography to afford the yellow solid compound D4-2 (7.4 g, 39%).
$^1$H NMR (600 MHz, DMSO-$d_6$) δ 7.60 - 7.50 (m, 2H), 7.42 (dd, *J* = 8.3, 1.5 Hz, 1H), 5.88 (s, 1H), 4.67 (d, *J* = 2.1 Hz, 2H), 3.87 (s, 2H), 3.45 (t, *J* = 5.8 Hz, 2H), 2.15 (q, *J* = 5.2 Hz, 2H), 1.41 (s, 9H).
LC-MS(ESI): $[M-tBu+H]^+$ = 357.15.

Step 2:

4-((3-amino-2-bromophenoxy)methyl)-3,6-dihydropyridine-1(2H)-carboxylic acid tert-butyl ester (Compound D4-3)

The compound D4-2 (7.0 g, 16.94 mmol) was dissolved in ethanol (60 mL) and water (10 mL).Iron powder (4.7 g, 84.7 mmol) and ammonium chloride (4.5 g, 84.7 mmol) were added. The mixture was heated to 60°C under nitrogen for 1 hour. The reaction mixture was filtered. The crude product was concentrated and purified by column chromatography to obtain a yellow solid compound D4-3 (5.0 g, 77%).
$^1$H NMR (400 MHz, DMSO-$d_6$) δ 6.96 (t, *J* = 8.1 Hz, 1H), 6.41 (dd, *J* = 8.1, 1.3 Hz, 1H), 6.26 (dd, *J* = 8.2, 1.3 Hz, 1H), 5.29 (s, 2H), 4.45 *(d, J*= 2.1 Hz, 2H), 3.86 (s, 2H), 3.45 (q, *J* = 7.1, 5.8 Hz, 3H), 2.12 (d, *J* = 6.9 Hz, 2H), 1.41 (s, 9H).
LC-MS(ESI): $[M-tBu+H]^+$ =327.15.

Step 3:

4-((2-bromo-3-(methylamino)phenoxy)methyl)-3,6-dihydropyridine-1(2H)-carboxylic acid tert-butyl ester (Compound D4-4)

The compound D4-3 (5.0 g, 13.1 mmol) was dissolved in 1,4-dioxane (50 mL). Pyridine (1.6 g, 19.6 mmol) and copper acetate (3.6 g, 19.6 mmol) were added. It wasreacted at room temperature for 15 min, methylboronic acid (1.6 g, 26.1 mmol) was added to the system and heated to 60°C for 16 hours. The reaction mixture was concentrated, and the crude product was purified by column chromatography to obtain a white solid compound D4-4 (4 g, 77%).
$^1$H NMR (400 MHz, $CDCl_3$) δ 7.15 (t, *J* = 8.2 Hz, 1H), 6.32 (ddd, *J* = 8.2, 7.0, 1.3 Hz, 2H), 5.85 (s, 1H), 4.49 (d, *J* = 1.6 Hz, 3H), 3.97 (q, *J* = 2.5 Hz, 2H), 3.58 (t, *J* = 5.7 Hz, 2H), 2.92 (s, 3H), 2.26 (td, *J* = 5.9, 2.7 Hz, 2H), 1.49 (s, 9H).
LC-MS(ESI): $[M+H]^+$ = 397.25.

Step 4: 4-(Methylamino)-2H-spiro[benzofuran-3,4'-piperidine]-1'-carboxylic acid tert-butyl ester (Compound D4-5)

The compound D4-4 (4.0 g, 10.1 mmol) was dissolved in toluene (40 mL), and azobis(isobutyronitrile) (3.3 g, 20.1 mmol) and tributylstannyl hydride (8.8 g, 30.2 mmol) were added. The mixture was reacted at 110°C for 15 hours. The reaction mixture was concentrated, and the crude product was purified by column chromatography to obtain a white solid compound D4-5 (3 g, 93%).
$^1$H NMR (600 MHz, $CDCl_3$) δ 7.08 (t, *J* = 8.0 Hz, 1H), 6.29 (d, *J* = 7.9 Hz, 1H), 6.24 (d, *J* = 8.1 Hz, 1H), 4.42 (brs, 2H), 4.20 (s, 2H), 2.88 (s, 3H), 2.77 (brs, 2H), 2.20 (td, *J* = 13.5, 4.9 Hz, 2H), 1.77 - 1.70 (m, 2H), 1.52 (s, 9H).
LC-MS(ESI): $[M+H]^+$= 319.35.

Step 5: N-Methyl-5-nitro-2H-spiro[benzofuran-3,4'-piperidine]-4-amine (Compound D4-6)

The compound D4-5 (3.0 g, 9.4 mmol) was dissolved in concentrated sulfuric acid (30 mL). It wascooled to -30°C, and 68% nitric acid (873 mg, 9.4 mmol) was added,reacted at this temperature for 30 min. The reaction mixture was poured into an ice-water bath, the pH was adjusted to 8 with sodium hydroxide, extracted with ethyl acetate, and concentrated to obtain a crude compound D4-6 (3 g) for direct use in the next step.
LC-MS(ESI): $[M+H]^+$ = 264.25.

Step 6:

4-(Methylamino)-5-nitro-2H-spiro[benzofuran-3,4'-piperidine]-1'-carboxylic acid tert-butyl ester (Compound D4-7)

The compound D4-6 (3 g, 11.4 mmol) and triethylamine (3.5 g, 34.2 mmol) were dissolved in dichloromethane (30 mL). Dibutyldicarbonate (3.7 g, 17.1 mmol) was added at 0°C. The reaction was allowed to proceed at room temperature for 16 hours. The reaction mixture was concentrated, and the crude product was purified by reverse-phase column chromatography to obtain a yellow solid compound D4-7 (1.7 g, 41%).

$^1$H NMR (600 MHz, $CDCl_3$) δ 7.95 (d, *J* = 9.0 Hz, 1H), 6.41 (d, *J* = 9.0 Hz, 1H), 5.51 (s, 1H), 4.52 (s, 2H), 4.22 (s, 2H), 2.89 (s, 3H), 2.78 (s, 2H), 2.37 (td, *J* = 13.4, 5.0 Hz, 2H), 1.74 (d, *J* = *13.7* Hz, 2H), 1.51 (s, 9H).

LC-MS(ESI): $[M+H]^+$= 364.35.

Step 7:

5-Amino-4-(methylamino)-2H-spiro[benzofuran-3,4'-piperidine]-1'-carboxylic acid tert-butyl ester (Compound D4-8)

The compound D4-7 (1.7 g, 4.7 mmol) was dissolved in methanol (17 mL), and palladium carbon (170 mg) was added. The reaction mixture was stirred at room temperature under a hydrogen atmosphere for 16 hours. The reaction mixture was filtered, and the crude product was purified by column chromatography to obtain a yellow solid compound D4-8 (1.3 g, 83%).

$^1$H NMR (400 MHz, DMSO-$d_6$) δ 6.44 (d, *J* = 8.2 Hz, 1H), 6.23 (d, *J* = 8.2 Hz, 1H), 4.27 (s, 2H), 4.22 (s, 2H), 3.94 (d, *J* = 13.4 Hz, 2H), 3.37 (s, 1H), 2.78 (s, 2H), 2.60 - 2.52 (m, 3H), 2.16 (td, *J*= 13.1, 4.8 Hz, 2H), 1.60 - 1.52 (m, 2H), 1.43 (s, 9H).

LC-MS(ESI): $[M+H]^+$= 334.35.

Step 8:

1-Methyl-2-oxo-2,3-dihydro-1H,7H-spiro[benzofuran[4,5-d]imidazole-8,4'-piperidine]-1'-carboxylic acid tert-butyl ester (Compound D4-9)

The compound D4-8 (1.2 g, 3.6 mmol) was dissolved in tetrahydrofuran (20 mL). Carbonyl diimidazole (1.2 g, 7.2 mmol) was added over an ice bath. The mixture was reacted under nitrogen protection at 0°C for 2 hours. The reaction mixture was concentrated, and the crude product was purified by column chromatography to obtain a white solid compound D4-9 (300 mg, 71%).

$^1$H NMR (400 MHz, DMSO-$d_6$) δ 10.77 (s, 1H), 6.75 (d, *J* = 8.2 Hz, 1H), 6.46 (d, *J* = 8.2 Hz, 1H), 4.45 (s, 2H), 3.97 (d, *J* = 13.4 Hz, 2H), 3.44 (s, 3H), 2.86 (s, 2H), 2.13 (td, *J* = 13.3, 4.9 Hz, 2H), 1.77 (d, *J* = 13.5 Hz, 2H), 1.43 (s, 9H).

LC-MS(ESI): $[M-tBu+H]^+$= 304.25.

Step 9:

3-(1-(4-methoxybenzyl)-2,6-dioxopiperidin-3-yl)-1-methyl-2-oxo-2,3-dihydro-1H, 7H-spiro[benzofuran[4,5-d]imidazo [8,4']-piperidine]-1'-carboxylic acid tert-butyl ester (Compound D4-10)

The compound D4-9 (300 mg, 0.8 mmol) and potassium tert-butoxide (141 mg, 1.3 mmol) were dissolved in ultrapure tetrahydrofuran (3 mL) and stirred at 0°C under nitrogen for 2 hours. A solution of compound D1-4 (477 mg, 1.3 mmol) dissolved in ultradry tetrahydrofuran (1 mL). The reaction mixture was allowed to react at room temperature for 16 hours. The reaction mixture was concentrated, and the crude product was purified by column chromatography to obtain a yellow solid compound D4-10 (400 mg, 81%).

LC-MS(ESI): $[M-tBu+H]^+$ = 535.40.

Step 10:

3-(1-Methyl-2-oxo-1,2-dihydro-3H,7H-spiro[benzofuran[4,5-d]imidazo[8,4']pyrimidin]-3-yl)piperidine-2,6-dione (Compound D4)

The compound D4-10 (200 mg, 0.3 mmol) was dissolved in toluene (2 mL). Methylsulfonic acid (1 mL) was added at room temperature, and the mixture was reacted at 120°C under nitrogen for 2 hours. The reaction mixture was concentrated, and the crude product was purified by preparative HPLC to obtain a white solid compound D4 (13 mg, 8%).

$^1$H NMR (400 MHz, DMSO-$d_6$) δ 11.09 (s, 1H), 8.64 (brs, 1H), 6.96 (d, *J* = 8.4 Hz, 1H), 6.56 (d, *J* = 8.4 Hz, 1H), 5.35 (dd,

*J* = 12.7, 5.4 Hz, 1H), 4.52 (s, 2H), 3.60 (s, 3H), 3.39 - 3.35 (m, 2H), 3.15 - 2.80 (m, 4H), 2.75 - 2.58 (m, 2H), 2.40 (tt, *J* = 13.7, 3.8 Hz, 2H), 2.01 - 1.95 (m, 2H).
LC-MS(ESI): $[M+H]^+$= 371.30

Example 45

Synthesis of Compound D5

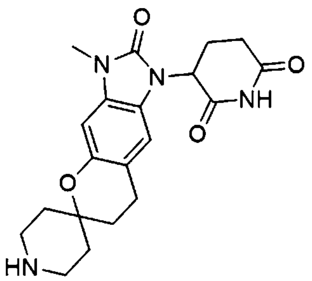

3-(3-Methyl-2-oxo-2,3,7,8-tetrahydro-1H-spiro[chromene[6,7-d]imidazo[6,4']-pyrimidin-1-yl)pyrimidine-2,6-dione

Synthetic Scheme

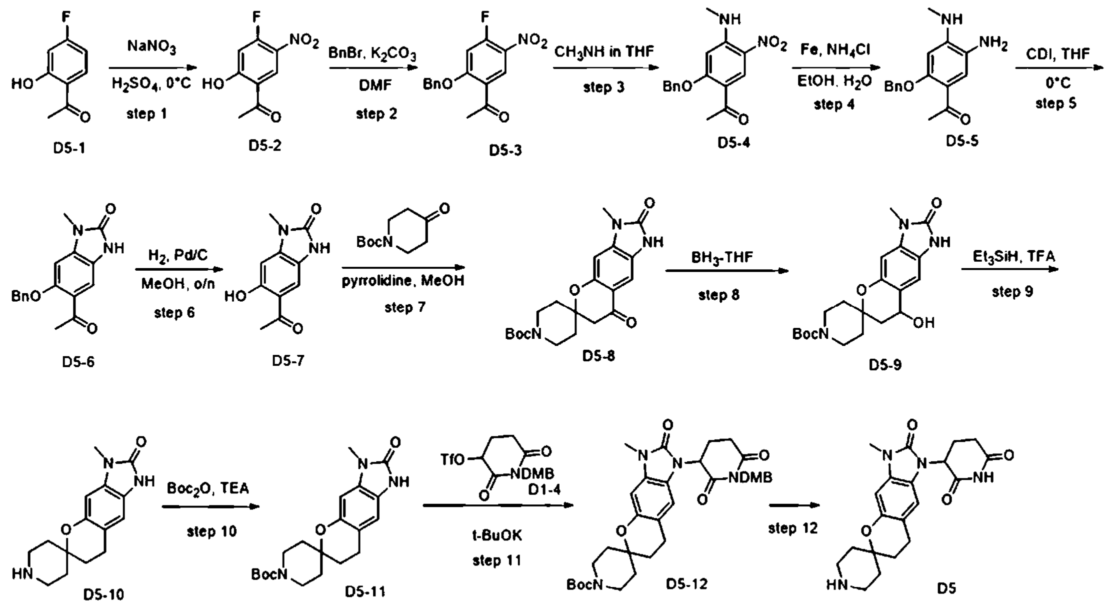


Step 1: 1-(4-Fluoro-2-hydroxy-5-nitrophenyl)ethan-1-one (Compound D5-2)

The compound D5-1 (20.0 g, 129.7 mmol) was dissolved in sulfuric acid (200 mL). Sodium nitrate (11 g, 129.7 mmol) was added at -10°C, and the mixture reacted at 0°C for 2 hours. The reaction mixture was poured into an ice-water bath, extracted with ethyl acetate, and the crude product was purified by column chromatography to obtain a yellow solid compound D5-2 (11 g, 43%) .

$^1$H NMR (600 MHz, DMSO-$d_6$) δ 12.77 (s, 1H), 8.54 - 8.54 (m, 1H), 7.08 - 7.05 (m, 1H), 2.66 (s, 3H).

LC-MS(ESI): $[M-H]^-$ = 197.95.

Step 2: 1-(2-(Benzyloxy)-4-fluoro-5-nitrophenyl)ethan-1-one (Compound D5-3)

The compound D5-2 (11.0 g, 55.2 mmol) was dissolved in N,N-dimethylformamide (110 mL). Benzyl bromide (11.3 g, 66.3 mmol) and potassium carbonate (15.3 g, 110.5 mmol) were added over an ice bath. The mixture was reacted at 25°C for 16 hours. The reaction mixture was filtered. Concentrated crude product was purified by column chromatography to obtain a yellow solid compound D5-3 (12.0 g, 75%).

$^1$H NMR (600 MHz, DMSO-$d_6$) δ 8.41 (d, *J* = 9.0 Hz, 1H), 7.59 (d, *J* = 13.6 Hz, 1H), 7.56 - 7.53 (m, 2H), 7.47 - 7.43 (m, 2H), 7.42 - 7.38 (m, 1H), 5.40 (s, 2H), 2.52 (s, 3H).

Step 3:

1-(2-(Benzyloxy)-4-(methylamino)-5-nitrophenyl)ethan-1-one (Compound D5-4)

The compound D5-3 (12.0 g, 41.5 mmol) was dissolved in a methanesulfonic acid tetrahydrofuran solution (60 mL) and heated to 60°C for 16 hours. The mixture was cooled to room temperature, filtered, and the filtrate cake was washed with methanol to obtain a yellow solid compound D5-4 (10 g, 80%).

$^1$H NMR (600 MHz, $CDCl_3$) δ 8.83 (s, 1H), 8.42 (s, 1H), 7.52 - 7.39 (m, 5H), 6.19 (s, 1H), 5.27 (s, 2H), 3.04 (d, *J* = 5.1 Hz, 3H), 2.55 (s, 3H).

LC-MS(ESI): $[M+H]^+$ = 301.30.

Step 4: 1-(5-Amino-2-(benzyloxy)-4-(methylamino)phenyl)ethan-1-one (Compound D5-5)

The compound D5-4 (10.0 g, 33.3 mmol) was dissolved in ethanol (100 mL) and water (20 mL). Iron powder (9.3 g, 166.5 mmol) and ammonium chloride (8.9 g, 166.5 mmol) were added. Under nitrogen atmosphere, the mixture was heated to 60°C and reacted for 1 hour. The reaction mixture was filtered. The crude product was concentrated and purified by column chromatography to obtain a yellow solid compound D5-5 (6.0 g, 67%).

LC-MS(ESI): $[M+H]^+$ = 271.25.

Step 5:

5-Acetyl-6-(benzyloxy)-1-methyl-1,3-dihydro-2H-benzo[d]imidazol-2-one (Compound D5-6)

The compound D5-5 (6.0 g, 22.2 mmol) was dissolved in tetrahydrofuran (100 mL). Carbonylimidazole (5.4 g, 33.3 mmol) was added over an ice bath. The mixture was reacted under nitrogen protection at 0°C for 2 hours. The mixture was filtered, and the cake was washed with tetrahydrofuran to obtain a yellow solid compound D5-6 (6 g, 91%).

$^1$H NMR (600 MHz, DMSO-$d_6$) δ 10.85 (s, 1H), 7.57 - 7.53 (m, 2H), 7.43 (t, *J* = 7.6 Hz, 2H), 7.37 (d, *J* = 7.4 Hz, 1H), 7.27 (s, 1H), 7.12 (s, 1H), 5.26 (s, 2H), 3.32 (s, 3H), 2.47 (s, 3H).

LC-MS(ESI): $[M+H]^+$= 297.25.

Step 6:5-Acetyl-6-hydroxy-1-methyl-1,3-dihydro-2H-benzo[d]imidazol-2-one (Compounds D5-7)

The compound D5-6 (6.0 g, 20.3 mmol) was dissolved in methanol (100 mL), and palladium carbon (600 mg) was added. The reaction mixture was stirred at room temperature under a hydrogen atmosphere for 16 hours. The reaction mixture was filtered to obtain a yellow solid compound D5-7 (3 g, 72%).

$^1$H NMR (600 MHz, DMSO-$d_6$) δ 12.71 (s, 1H), 10.91 (s, 1H), 7.34 (s, 1H), 6.69 (s, 1H), 3.26 (s, 3H), 2.61 (s, 3H).

LC-MS(ESI): $[M+H]^+$= 207.25.

Step 7:

3-Methyl-2,8-dioxo-2,3,7,8-tetrahydro-1H-spiro[chrom[6,7-d]imidazole-6,4'-piperidine]-1'-carboxylic acid tert-butyl ester (Compound D5-8)

The compound D5-7 (1.0 g, 4.9 mmol) was dissolved in methanol (10 mL). N-tert-Butoxycarbonyl-4-piperidone (966 mg, 4.9 mmol) and tetrahydropyrole (345 mg, 4.9 mmol) were added. The reaction mixture was stirred at 70°C under a hydrogen atmosphere for 16 hours. The reaction mixture was filtered to obtain a yellow solid compound D5-8 (1.3 g, 69%).

$^1$H NMR (600 MHz, DMSO-$d_6$) δ 10.91 (s, 1H), 7.20 (s, 1H), 6.83 (s, 1H), 3.72 (d, *J* = 12.5 Hz, 2H), 3.27 (s, 3H), 3.15 (brs, 2H), 2.75 (s, 2H), 1.89 (d, *J*= 13.6 Hz, 2H), 1.65 - 1.56 (m, 2H), 1.40 (s, 9H).

LC-MS(ESI): $[M-H]^-$= 386.15.

Step 8:

8-Hydroxy-3-methyl-2-oxo-2,3,7,8-tetrahydro-1H-pyro[chromene[6,7-d]imidazo[6,4']-pyrimidine]-1'-carboxylic acid tert-butyl ester (Compound D5-9)

The compound D5-8 (1.0 g, 2.6 mmol) was dissolved in tetrahydrofuran (10 mL). A solution of borane in tetrahydrofuran (7.7 mL, 7.8 mmol) was added over an ice bath. The mixture was reacted at room temperature under nitrogen for 2 hours. The reaction mixture was concentrated to afford the crude white solid compound D5-9 (500 mg).
LC-MS(ESI): $[M+H]^+$= 390.35.

Step 9:

3-Methyl-1,3,7,8-tetrahydro-2H-spiro[chrom[6,7-d]imidazole-6,4'-piperidine]-2-one (Compound D5-10)

The compound D5-9 (500 mg, 1.3 mmol) was dissolved in trifluoroacetic acid (3 mL). Triethylsilane (746 mg, 6.4 mmol) was added over an ice bath. The mixture was reacted at 50°C under nitrogen for 2 hours. The reaction mixture was concentrated to obtain a crude yellow oily product D5-10 (350 mg).
LC-MS(ESI): $[M+H]^+$ = 274.25.

Step 10:

3-Methyl-2-oxo-2,3,7,8-tetrahydro-1H-spiro[chrom[6,7-d]imidazole-6,4'-piperidine]-1'-carboxylic acid tert-butyl ester (Compound D5-11)

The compound D5-10 (350 mg, 1.3 mmol) was dissolved in dichloromethane (5 mL). Triethylamine was added over an ice bath tand the pH was adjusted to 7, followed by di-tert-butyl dicarbonate (420 mg, 1.9 mmol). The mixture was reacted at room temperature under nitrogen for 16 hours. The reaction mixture was concentrated, the crude product obtained was purified by column chromatography to obtain a white solid compound D5-11 (410 mg, 86.4%).
$^1$H NMR (600 MHz, DMSO-$d_6$) δ 10.49 (s, 1H), 6.65 (s, 1H), 6.55 (s, 1H), 3.69 (d, *J*= 13.1 Hz, 2H), 3.19 - 3.07 (m, 5H), 2.72 (t, *J* = 6.8 Hz, 2H), 1.75 (t, *J* = 6.8 Hz, 2H), 1.70 - 1.64 (m, 2H), 1.52 - 1.49 (m, 2H), 1.40 (s, 9H).
LC-MS(ESI): $[M+H]^+$= 374.35.

Step 11:

1-(1-(2,4-Dimethoxybenzyl)-2, 6-dioxopiperidin-3-yl)-3-methyl-2-oxo-2,3,7,8-tetrahydro-1H-pyran[chromen[6,7-d]imidazo[6,4']-piperidin]-1'-carboxylic acid tert-butyl ester (Compound D5-12)

The compound D5-11 (600 mg, 1.6 mmol) and potassium tert-butoxide (216 mg, 1.9 mmol) were dissolved in ultradry tetrahydrofuran (6 mL). Under nitrogen protection, the mixture was stirred at 0°C for 2 hours. Then, a solution of compound D1-4 (793 mg, 1.9 mmol) dissolved in anhydrous tetrahydrofuran (1 mL). The reaction mixture was allowed to react at room temperature for 16 hours. The reaction mixture was concentrated, and the crude product was purified by column chromatography to obtain a yellow solid compound D5-12 (600 mg, 59%).
$^1$H NMR (600 MHz, DMSO-$d_6$) δ 6.94 (d, *J* = 8.4 Hz, 1H), 6.77 - 6.70 (m, 1H), 6.67 (s, 1H), 6.55 (d, *J* = 2.4 Hz, 1H), 6.44 (dd, *J* = 8.4, 2.4 Hz, 1H), 5.50 (dd, *J* = 13.0, 5.4 Hz, 1H), 4.82 - 4.67 (m, 2H), 3.79 (s, 3H), 3.74 (s, 5H), 3.26 (s, 6H), 2.87 - 2.60 (m, 4H), 2.10 - 2.01 (m, 1H), 1.77 (t, *J* = 6.8 Hz, 2H), 1.71 - 1.63 (m, 2H), 1.55 - 1.47 (m, 2H), 1.41 (s, 9H).
LC-MS(ESI): $[M+H]^+$= 635.50.

Step 12:

3-(3-Methyl-2-oxo-2,3,7,8-tetrahydro-1H-spiro[chromeno[6,7-d]imidazo[6,4']-piperidin-1-yl)piperidine-2,6-dione (Compound D5)

The compound D5-12 (600 mg, 1.0 mmol) was dissolved in trifluoroacetic acid (5 mL), trifluoromethanesulfonic acid (1 mL) was added at room temperature, and reacted under nitrogen at 70°C for 2 hours. The reaction system was concentrated, and the crude product was purified by high-pressure preparative liquid chromatography to obtain a white solid compound D5 (345 mg, 69%).

$^1$H NMR (400 MHz, DMSO-$d_6$) δ 11.06 (s, 1H), 8.67 (s, 1H), 8.43 (s, 1H), 6.87 (s, 1H), 6.71 (s, 1H), 5.30 (dd, *J* = 12.9,

5.4 Hz, 1H), 3.28 (s, 3H), 3.25 - 3.05 (m, 4H), 2.95 - 2.86 (m, 1H), 2.81 - 2.56 (m, 4H), 1.99 - 1.70 (m, 6H). LC-MS(ESI): $[M+H]^+$= 385.25

Example 46

Synthesis of Compound D6

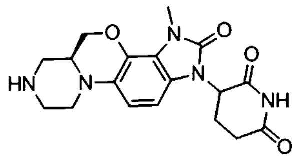

Synthetic scheme

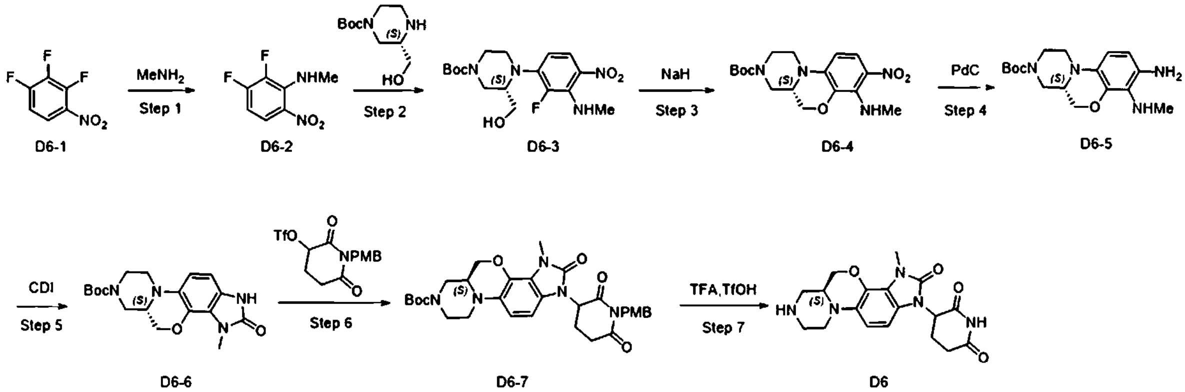


Step 1: 2,3-Difluoro-N-methyl-6-nitroaniline (Compound D6-1)

Under a nitrogen atmosphere, methylamine (192.93 mg, 6.21 mmol) was added to a solution of 1,2,3-trifluoro-4-nitrobenzene (1 g, 5.65 mmol) in tetrahydrofuran (10 mL) and reacted overnight at 60°C. The solvent was rotary evaporated off, and the product was purified by column chromatography (PE:EA = 0-30%) to afford the yellow solid compound D6-1 (728.60 mg, 68.58%).

$^1$H NMR (600 MHz, DMSO-$d_6$) δ 8.06 - 7.88 (m, 2H), 6.75 (dt, $J$ = 16.7, 8.4 Hz, 1H), 3.17 (dd, $J$ = 8.1, 5.4 Hz, 3H).

LC-MS(ESI): $[M+H]^+$ = 189.24.

Step 2:

(S)-4-(2-Fluoro-3-(methylamino)-4-nitrophenyl)-3-(hydroxymethyl)piperazine-1-carboxylic acid tert-butyl ester (Compound D6-2)

Under air atmosphere, (S)-3- (hydroxymethyl)piperazine-1-carboxylic acid tert-butyl ester (500 mg, 2.31 mmol) was added in dimethyl sulfoxide (10 mL), 2,3-difluoro-N-methyl-6-nitroaniline (478.42 mg, 2.54 mmol) was added to a solution of (S)-3-(hydroxymethyl)piperazine-1-carboxylic acid tert-butyl ester (500 mg, 2.31 mmol) in dimethyl sulfoxide (10 mL) and reacted at 120°C overnight. After the reaction was completed, the reaction mixture was extracted with EA. The combined organic layers were washed with saturated saline solution, dried over anhydrous sodium sulfate, and the solvent was rotary evaporated. The mixture was purified by column chromatography (PE:EA = 0-10%) to obtain a yellow solid compound D6-2 (568.1 mg, 63.93%).

$^1$H NMR (600 MHz, DMSO-$d_6$) δ 7.89 (d, $J$ = 3.3 Hz, 1H), 7.79 (d, $J$ = 9.5 Hz, 1H), 6.49 - 6.41 (m, 1H), 4.76 (t, J = 5.1 Hz, 1H), 3.97 (d, $J$ = 13.1 Hz, 1H), 3.81 (br, 1H), 3.81 (br, 1H), 3.53 - 3.41 (m, 2H), 3.27 (d, $J$ = 5.9 Hz, 2H), 3.22 - 2.90 (m, 5H), 1.42 (s, 9H).

LC-MS(ESI): $[M+H]^+$ = 329.26.

Step 3:

(S)-7-(Methylamino)-8-nitro-1,2,4a,5-tetrahydrobenzo[b]pyrazino[1,2-d][1,4]oxazine-3(4H)-carboxylic acid tert-butyl ester (Compound D6-3)

Under a nitrogen atmosphere at 0°C, sodium hydride (39.01 mg, 1.63 mmol, 60% wt) was slowly added in portions to a solution of compound D6-2 (568.1 mg, 1.48 mmol) in N,N-dimethylformamide (5 mL). The mixture was then transferred to 110°C and reacted for 2 hours. The reaction was quenched by slowly adding water. The reaction mixture was extracted with ethyl acetate (EA), washed with saturated saline solution, and the combined organic layers were dried over anhydrous sodium sulfate. The solvent was removed by rotary evaporation, and the product was purified by column chromatography (PE:EA=0-30%) to obtain a yellow oily liquid compound D6-3 (396.5 mg, 73.63% yield).

$^1$H NMR (400 MHz, DMSO-$d_6$) δ 7.74 - 7.56 (m, 2H), 6.46 (d, *J* = 9.9 Hz, 1H), 4.34 (dd, *J* = 11.1, 3.4 Hz, 1H), 4.06 - 3.79 (m, 4H), 3.44 - 3.35 (m, 1H), 3.04 (d, *J* = 5.3 Hz, 3H), 2.92 (d, *J* = 8.3 Hz, 2H), 2.80 - 2.55 (, 1H), 1.42 (s, 9H).

LC-MS(ESI): $[M+H]^+$ = 365.49.

Step 4:

(S)-8-Amino-7-(methylamino)-1,2,4a,5-tetrahydrobenzo[b]pyrazino[1,2-d][1,4]oxazine-3(4H)-carboxylic acid tert-butyl ester (Compound D6-4)

Under hydrogen atmosphere, palladium on carbon (79.3 mg, 0.67 mmol) was added to a solution of compound D6-3 (396.5 mg, 1.09 mmol) in tetrahydrofuran (7 mL) and reacted overnight at room temperature. After reaction completion, the solution was filtered through diatomaceous earth, and the solvent was rotary evaporated to dryness, obtaining a brown solid compound D6-4 (291.6 mg, 80.14%).

$^1$H NMR (400 MHz, DMSO-$d_6$) δ 6.31 (d, *J* = 8.8 Hz, 1H), 6.16 (d, *J* = 8.6 Hz, 1H), 4.30 (dd, *J* = 10.6, 2.5 Hz, 1H), 4.15 (br, 2H), 3.98 - 3.78 (m, 3H), 3.71 - 3.43 (m, 2H), 3.34 (s, 1H), 2.93 (br, 1H), 2.80 - 2.70 (m, 1H), 2.55 (s, 3H), 2.36 (td, *J* = 11.9, 3.2 Hz, 1H), 1.42 (s, 9H).

LC-MS(ESI): $[M+H]^+$ = 335.49.

Step 5: (S)-3-Methyl-2-oxo-2,3,5a,6,8,9-hexahydro-1H-imidazo[4',5':5,6] benzo[1,2-b]pyrazino[1,2-d][1,4]oxazine-7(5H)-carboxylic acid tert-butyl ester (compound D6-5)

Under a nitrogen atmosphere, carbonyl diimidazole (212.08 mg, 1.31 mmol) was added to the acetonitrile (6 mL) containing compound D6-4 (291.6 mg, 0.87 mmol) and reacted at 80°C for 1h. After reaction completion, the solvent was removed by rotary evaporation and purified by column chromatography (PE:EA=0-50%) to obtain a yellow solid compound D6-5 (257.8 mg, 82.03%).

$^1$H NMR (400 MHz, DMSO-$d_6$) δ 10.53 (s, 1H), 6.56 (d, *J* = 8.7 Hz, 1H), 6.45 (d, *J* = 8.5 Hz, 1H), 4.39 (dd, *J* = 10.7, 2.6 Hz, 1H), 4.04 - 3.87 (m, 3H), 3.70 (d, *J* = 12.0 Hz, 1H), 3.39 (s, 3H), 3.03 - 2.86 (m, 2H), 2.62 (br, 1H), 2.48 (d, *J* = 3.3 Hz, 1H), 1.42 (s, 9H).

LC-MS(ESI): $[M+H]^+$ = 360.39.

Step 6:

(5aS)-1-(1-(3,4-dimethylbenzyl)-2,6-dioxopiperidin-3-yl)-3-methyl-2-oxo-2,3,5a,6,8,9-hexahydro-1H-imidazo[4',5':5,6] benzo[1,2-b]pyrazino[1,2-d][1,4]oxazine-7(5H)-carboxylic acid tert-butyl ester (Compound D6-6)

Under a nitrogen atmosphere at 0°C, a solution of potassium tert-butylate in tetrahydrofuran (0.2 mL, 0.49 mmol) was added to a solution of compound D6-5 in tetrahydrofuran (4 mL). After reacting for 1h, 1-(3,4-dimethylbenzyl)-2,6-dioxopiperidin-3-yl trifluoromethanesulfonate (189.46 mg, 0.49 mmol) was added and reacted at room temperature for 0.5 h. Solvent was rotary evaporated, and purified by column chromatography (PE:EA=0-50%) to obtain a purple solid compound D6-6 (38.8 mg, 15.81%).

$^1$H NMR (400 MHz, DMSO-$d_6$) δ 7.19 (d, *J* = 8.6 Hz, 2H), 6.85 (d, *J* = 8.6 Hz, 2H), 6.59 (d, *J* = 9.3 Hz, 1H), 6.49 (d, *J* = 8.4 Hz, 1H), 5.41 (dd, *J* = 12.8, 5.0 Hz, 1H), 4.77 (dd, *J* = 32.4, 14.4 Hz, 2H), 4.42 (d, *J* = 8.5 Hz, 1H), 4.10 - 3.85 (m, 3H), 3.70 - 3.65 (m, 4H), 3.46 (s, 3H), 2.99 (d, *J* = 31.9 Hz, 3H), 2.79 (d, *J* = 17.7 Hz, 1H), 2.74 - 2.60 (m, 2H), 2.05 - 1.93 (m, 2H), 1.42 (s, 9H).

LC-MS(ESI): $[M+H-tBu]^+$ = 536.48.

Step 7:

3-((R)-3-Methyl-2-oxo-2,3,5,5a,6,7,8,9-octahydro-1H-imidazo[4',5':5,6] benzo[1,2-b]pyrazino[1,2-d][1,4]oxazine-1-yl) piperidine-2,6-dione (Compound D6)

Under a nitrogen atmosphere, the compound D6-6 (38.8 mg, 0.065 mmol) was dissolved in a mixed solution of trifluoroacetic acid/trifluoromethanesulfonic acid (5/1, 6 mL) and reacted for two hours in an oil bath at 70°C. The solvent was rotary evaporated, and the solution was purified by preparative HPLC. After freeze-drying, yellow solid compound D6 (7.79 mg, 32.29%) was obtained.

$^1$H NMR (400 MHz, DMSO-$d_6$) δ 11.08 (s, 1H), 6.70 - 6.60 (m, 2H), 5.27 (dd, *J* = 12.7, 5.0 Hz, 1H), 4.40 (d, *J* = 9.1 Hz, 1H), 4.12 - 3.92 (m, 2H), 3.47 (s, 3H), 3.43 - 3.30 (m, 4H), 3.08 (d, *J* = 10.3 Hz, 1H), 2.94 - 2.79 (m, 3H), 2.74 - 2.56 (m, 2H), 2.02 - 1.89 (m, 1H).

LC-MS(ESI): $[M+H]^+$ = 372.39.

Example 47

Synthesis of Compound D7

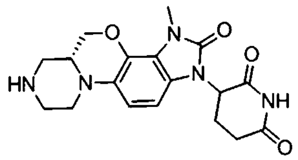

Synthetic scheme

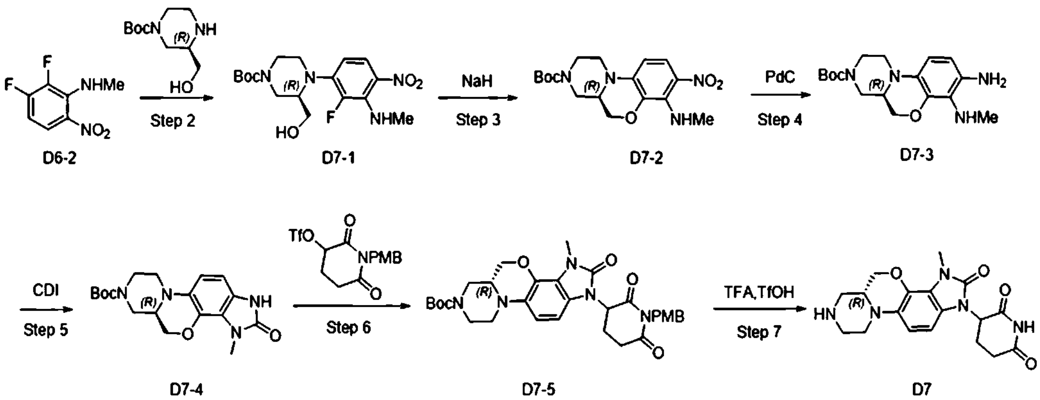


The synthesis method of compound D7 is the same as that of compound D6.

$^1$H NMR (600 MHz, DMSO-$d_6$) δ 11.09 (s, 1H), 6.66 (dd, *J* = 22.5, 8.7 Hz, 2H), 5.33 - 5.22 (m, 1H), 4.40 (d, *J* = 9.5 Hz, 1H), 4.06 (dd, J = 10.4, 8.1 Hz, 1H), 3.97 (d, *J* = 13.0 Hz, 1H), 3.47 (s, 3H), 3.09 (d, *J* = 10.2 Hz, 1H), 2.91 - 2.80 (m, 2H), 2.71 - 2.57 (m, 2H), 2.01 - 1.93 (m, 1H).

LCMS (ESI): $[M+H]^+$= 372.39.

Example 48

Synthesis of Compound D8

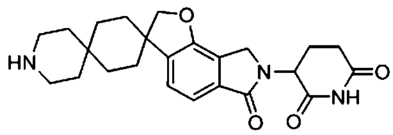

3-(6-Oxo-6,8-dihydro-2H,7H-dipyrrolo[2,3-e]isoindole-3,1'-cyclohexane-4'-,4'-piperidyl]-7-yl)piperidine-2,6-dione

Synthetic Scheme

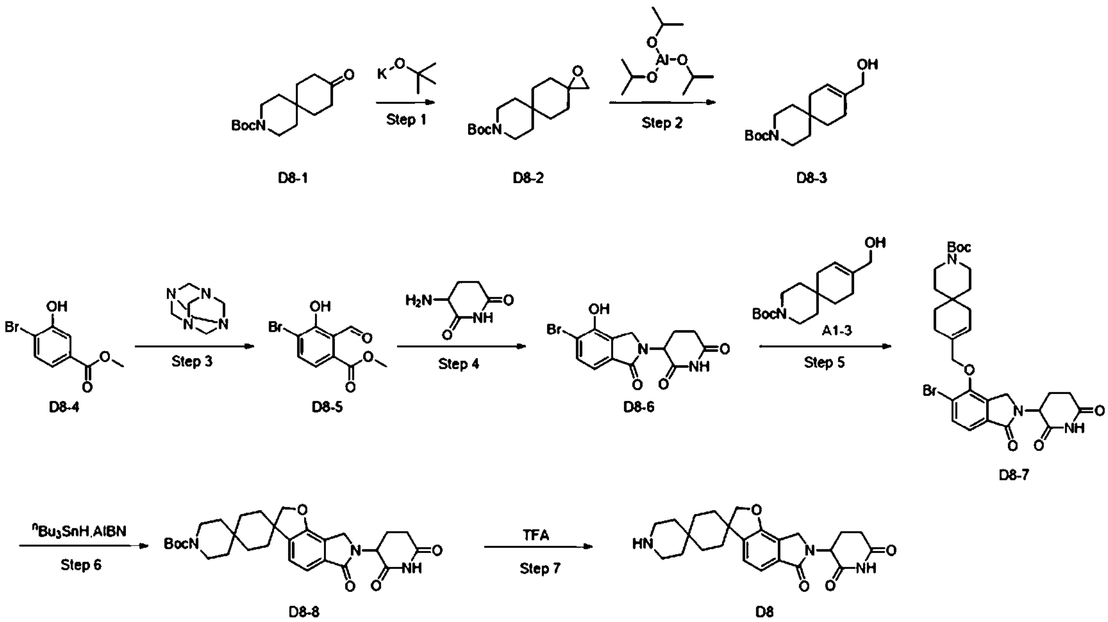


Step 1: 1-Oxa-9-azabiphosphino[2.2.56.23]tridecane-9-carboxylic acid tert-butyl ester (Compound D8-2)

Trimethyl sulfonyl iodide (6.2 g, 28.1 mmol) and potassium tert-butoxide (2.7 g, 24.3 mmol) were dissolved in dimethyl sulfoxide (20 mL),stirred at room temperature under nitrogen for 1.5 hours. The reaction mixture was cooled to 0°C. Ethylene glycol dimethyl ether (10 mL) was added to the reaction system. Compound 1a (5.0 g, 18.7 mmol) dissolved in dimethyl sulfoxide:ethylene glycol dimethyl ether = 3:1 (8 mL) was then added to the reaction system and reacted at this temperature for 1 hour. The reaction mixture was quenched with water at low temperature, extracted three times with ethyl acetate, and the organic phase was collected. The crude product was concentrated and purified by column chromatography to obtain a colorless oily compound D8-2 (3.5 g, 67%).

$^1$H NMR (600 MHz, DMSO-$d_6$) δ 3.33 - 3.28 (m, 4H), 2.54 (s, 2H), 1.60 - 1.54 (m, 2H), 1.53 - 1.44 (m, 4H), 1.40 - 1.34 (m, 15H).

LC-MS(ESI): [M-Boc+H]$^+$ = 182.34.

Step 2: 9-(Hydroxymethyl)-3-azaspiro[5.5]undec-8-ene-3-carboxylic acid tert-butyl ester (Compound D8-3)

The compound D8-2 (2.0 g, 7.1 mmol) was dissolved in toluene (20 mL), and aluminum isopropoxide (2.2 g, 10.7 mmol) was added. The mixture was reacted at 110°C for 24 hours. The reaction mixture was cooled to room temperature, dilute hydrochloric acid (1.0 mol/L, 200 mL) was added, extracted three times with ethyl acetate, and the organic layer was collected. The crude product obtained from the concentrated organic phase was purified by column chromatography to obtain a colorless oily compound D8-3 (1.0 g, 50%).

$^1$H NMR (600 MHz, DMSO-$d_6$) δ 5.48 (s, 1H), 4.59 (t, $J$ = 5.6 Hz, 1H), 3.77 (d, $J$ = 5.0 Hz, 2H), 3.40 - 3.35 (m, 2H), 3.28 - 3.21 (m, 2H), 1.94 - 1.85 (m, 4H), 1.45 (t, $J$ = 6.0 Hz, 2H), 1.39 (s, 9H), 1.30 - 1.26 (m, 4H).

LC-MS(ESI): [M-tBu+H]$^+$ = 226.30.

Step 3: Methyl 4-bromo-2-formyl-3-hydroxybenzoate (Compound D8-5)

The compound D8-4 (5.0 g, 21.6 mmol) was dissolved in trifluoroacetic acid (25 mL), and urotropine (4.55 g, 32.5 mmol) was added. The mixture was reacted at 80°C for 15 hours. After it was cooled to room temperature, water was added to the reaction mixture, which was then extracted with ethyl acetate. Washed with sodium hydroxide solution (5 mol/L), combined organic layers, and drried over anhydrous sodium sulfate. The crude product obtained from the concentrated organic phase was purified by column chromatography to obtain a white solid compound D8-5 (2.0 g, 36%).

$^1$H NMR (600 MHz, $CDCl_3$) δ 12.94 (s, 1H), 10.68 (s, 1H), 7.86 (d, *J* = 8.2 Hz, 1H), 7.43 (d, *J* = 8.2 Hz, 1H), 3.99 (s, 3H).

LC-MS(ESI): $[M+H]^-$ = 259.17.

Step 4: 3-(5-Bromo-4-hydroxy-1-oxo-isoindol-2-yl)piperidine-2,6-dione (Compound D8-6)

The compound D8-5 (2.0 g, 7.7 mmol) was dissolved in tetrahydrofuran (20 mL). 3-Aminopiperidine-2,6-dione hydrochloride (1.9 g, 11.6 mmol) and N,N-diisopropylethylamine (1 mL) were added. After the reaction mixture was stirred at room temperature for 1 hour, sodium triacetoxyborohydride (3.3 g, 15.4 mmol) and acetic acid (1 mL) were added. The reaction mixture was heated at 50°C for 1.5 hours. The reaction mixture was concentrated, and the crude product was purified by column chromatography to obtain a light blue solid compound D8-6 (1.5 g, 57%).

$^1$H NMR (600 MHz, DMSO-$d_6$) δ 11.02 (s, 1H), 10.49 (s, 1H), 7.66 (d, *J* = 7.9 Hz, 1H), 7.15 (d, *J* = 7.9 Hz, 1H), 5.11 (dd, *J* = 13.3, 5.0 Hz, 1H), 4.41 (d, *J* = 17.4 Hz, 1H), 4.29 (d, *J* = 17.4 Hz, 1H), 2.95 - 2.88 (m, 1H), 2.64 - 2.58 (m, 1H), 2.36 (qd, *J* = 13.2, 4.3 Hz, 1H), 2.07 - 2.00 (m, 1H).

LC-MS(ESI): $[M+H]^+$= 341.09.

Step 5:

9-(((5-bromo-2-(2,6-dioxopiperidin-3-yl)-1-oxo-isoindolin-4-yl)oxy)methyl)-3-azaspiro[5.5]undec-8-ene-3-carboxylic acid tert-butyl ester (Compound D8-7)

The compound D8-6 (500 mg, 1.5 mmol), compound D8-3 (498 mg, 1.8 mmol), and triphenylphosphine (1.2 g, 4.4 mmol) were dissolved in tetrahydrofuran (30 mL) and stirred at 0°C under nitrogen for 15 min. Diethyl azodicarboxylate (514 mg, 3.0 mmol) was slowly added dropwise to the reaction mixture, which was then allowed to react at room temperature for 15 h. The reaction mixture was concentrated, and the crude product was purified by column chromatography to obtain a white solid compound D8-7 (500 mg, 56%).

$^1$H NMR (600 MHz, DMSO-$d_6$) δ 11.03 (s, 1H), 7.77 (d, *J* = 7.9 Hz, 1H), 7.37 (d, *J* = 8.0 Hz, 1H), 5.13 (dd, *J* = 13.3, 5.2 Hz, 1H), 4.67 (d, *J* = 17.3 Hz, 1H), 4.61 - 4.55 (m, 2H), 4.51 (d, *J= 17.3* Hz, 1H), 3.41 - 3.35 (m, 2H), 3.30 - 3.21 (m, 2H), 2.97 - 2.88 (m, 1H), 2.64 - 2.58 (m, 1H), 2.48 - 2.40 (m, 1H), 2.21 - 2.14 (m, 2H), 2.04 - 1.98 (m, 1H), 1.96 - 1.91 (m, 2H), 1.55 - 1.49 (m, 2H), 1.39 (s, 9H), 1.32 - 1.27 (m, 4H).

LC-MS(ESI): $[M-Boc+H]^+$= 504.29.

Step 6:

7-(2,6-Dioxo-3-hydroxyflavone)-6-oxo-7, 8-dihydro-2H,6H-dipyrrolo[furan[2,3-e]isoindole-3,1'-cyclohexane-4'-,4'-piperidine]-1"-carboxylic acid tert-butyl ester (Compound D8-8)

The compound D8-7 (200 mg, 0.33 mmol) was dissolved in toluene (2 mL). Azobis(isobutyronitrile) (109 mg, 0.66 mmol) and tributylstannyl hydride (290 mg, 1.0 mmol) were added at room temperature. The mixture was reacted at 110°C for 15 hours. The reaction mixture was concentrated, and the crude product was purified by column chromatography to obtain a white solid compound D8-8 (100 mg, 58%).

$^1$H NMR (600 MHz, DMSO-$d_6$) δ 10.97 (s, 1H), 7.46 (d, *J* = 7.5 Hz, 1H), 7.27 (d, *J* = 7.5 Hz, 1H), 5.09 (dd, *J* = 13.3, 5.1 Hz, 1H), 4.51 (q, *J* = 9.0 Hz, 2H), 4.38 (d, *J* = 17.0 Hz, 1H), 4.21 (d, *J* = 16.9 Hz, 1H), 3.11 - 3.03 (m, 4H), 2.96 - 2.86 (m, 1H), 2.64 - 2.56 (m, 1H), 2.47 - 2.38 (m, 1H), 2.02 - 1.94 (m, 1H), 1.90 - 1.81 (m, 2H), 1.72 - 1.65 (m, 2H), 1.64 - 1.50 (m, 4H), 1.40 (s, 9H), 1.31 - 1.19 (m, 4H).

LC-MS(ESI): $[M+H]^+$= 524.39.

Step 7:

3-(6-oxo-6,8-dihydro-2H,7H-dipyrrolo[2,3-e]isoxazol-3,1'-cyclohexane-4'-,4'-piperidyl]-7-yl)piperidine-2,6-dione (Compound D8)

The compound D8-8 (50 mg, 0.1 mmol) was dissolved in dichloromethane: trifluoroacetic acid = 10:1 (1 mL) and reacted at room temperature for 2 hours. The reaction mixture was concentrated, and the crude product was purified by reverse-phase column chromatography to obtain a white solid compound D8 (20 mg, 50%).

$^1$H NMR (600 MHz, DMSO-$d_6$) δ 10.98 (s, 1H), 7.46 (d, *J* = 7.6 Hz, 1H), 7.29 (d, *J* = 7.6 Hz, 1H), 5.09 (dd, *J* = 13.3, 5.0 Hz, 1H), 4.52 (q, *J* = *9.0* Hz, 2H), 4.38 (d, *J* = 17.0 Hz, 1H), 4.21 (d, *J* = 17.0 Hz, 1H), 3.11 - 3.03 (m, 4H), 2.95 - 2.88 (m, 1H), 2.63 - 2.57 (m, 1H), 2.46 - 2.38 (m, 1H), 2.00 - 1.95 (m, 1H), 1.88 - 1.80 (m, 4H), 1.75 - 1.67 (m, 2H), 1.60 - 1.49 (m, 4H), 1.32 - 1.25 (m, 2H).
LC-MS(ESI): $[M+H]^+$= 424.30

Example 49

Synthesis of Compound D9

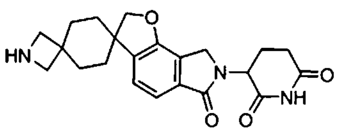

3-(6''-oxo-6''-,8''-dihydro-2''-H,7''-H-dipyrazolo[azetidin-3,1'-cyclohexane-4'-, 3"-furan[2,3-e]isoindole]-7"-yl)piperidine-2,6-dione

Synthetic scheme

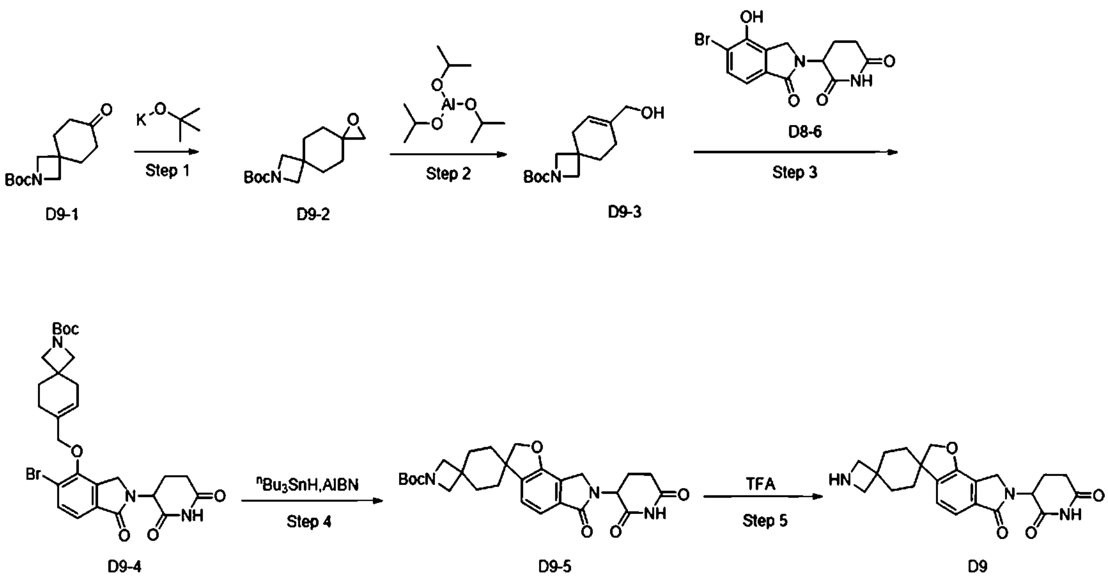


Step 1: 1-Oxa-8-azabipyridine[2.2.36.23]undecane-8-carboxylic acid tert-butyl ester (Compound D9-2)

Trimethyl sulfonyl iodide (6.9 g, 31.3 mmol) and potassium tert-butylate (3.1 g, 27.2 mmol) were dissolved in dimethyl sulfoxide (20 mL), stirred at room temperature under nitrogen protection for 1.5 hours. The reaction mixture was cooled to 0°C. Ethylene glycol dimethyl ether (10 mL) was added to the reaction system, followed by compound D9-1 (5.0 g, 20.9 mmol) dissolved in dimethyl sulfoxide: ethylene glycol dimethyl ether=3:1 (8 mL). The mixture was reacted at this temperature for 1 hour. The reaction mixture was quenched with water at low temperature, extracted three times with ethyl

acetate, and collected the organic layer. The crude product was concentrated and purified by column chromatography to afford the white solid compound D9-2 (3.1 g, 58.0%).

$^1$H NMR (600 MHz, DMSO-$d_6$) δ 3.62 - 3.49 (m, 4H), 2.56 (s, 2H), 1.80 - 1.69 (m, 4H), 1.59 - 1.51 (m, 2H), 1.41 - 1.34 (m, 11H).

LC-MS(ESI): [M-tBu+H]$^+$ = 198.30.

Step 2: 7-(Hydroxymethyl)-2-azaspiro[3.5]non-6-ene-2-carboxylic acid tert-butyl ester (Compound D9-3)

The compound D9-2 (3.0 g, 11.8 mmol) was dissolved in toluene (30 mL), and aluminum isopropoxide (3.6 g, 17.7 mmol) was added. The mixture was reacted at 110°C for 24 hours. The reaction mixture was cooled to room temperature, dilute hydrochloric acid (1.0 mol/L, 200 mL) was added, extracted three times with ethyl acetate, and collected the organic phase. The crude product obtained from the concentrated organic phase was purified by column chromatography to obtain a colorless oily compound D9-3 (1.2 g, 40%).

$^1$H NMR (600 MHz, DMSO-$d_6$) δ 5.50 - 5.47 (m, 1H), 4.64 (t, *J* = 5.6 Hz, 1H), 3.78 - 3.74 (m, 2H), 3.61 - 3.41 (m, 4H), 2.18 (dt, *J* = 4.0, 2.0 Hz, 2H), 2.01 - 1.97 (m, 2H), 1.71 (t, *J* = 6.4 Hz, 2H), 1.37 (s, 9H).

LC-MS(ESI): [M-tBu+H]$^+$ = 198.29.

Step 3:

7-(((5-bromo-2-(2,6-dioxopiperidin-3-yl)-1-oxo-4-isoxindolinyl)oxy)methyl)-2-azaspiro[3.5]non-6-ene-2-carboxylic acid tert-butyl ester (Compound D9-4)

The compound D8-6 (500 mg, 1.5 mmol), compound D9-3 (448 mg, 1.8 mmol), and triphenylphosphine (773 mg, 3.0 mmol) were dissolved in tetrahydrofuran (30 mL). The mixture was stirred at 0°C under nitrogen for 15 minutes. Diethyl azodicarboxylate (514 mg, 3.0 mmol) was slowly added dropwise to the reaction mixture. The reaction mixture was reacted at room temperature for 15 hours. The concentrated reaction mixture was purified by column chromatography to obtain a white solid compound D9-4 (500 mg, 59%).

$^1$H NMR (600 MHz, DMSO-$d_6$) δ 11.02 (s, 1H), 7.77 (d, *J* = 7.9 Hz, 1H), 7.37 (d, *J* = 7.9 Hz, 1H), 5.12 (dd, *J* = 13.4, 5.2 Hz, 1H), 4.69 - 4.47 (m, 5H), 3.62 - 3.55 (m, 2H), 3.53 - 3.44 (m, 2H), 2.97 - 2.89 (m, 1H), 2.64 - 2.59 (m, 2H), 2.48 - 2.42 (m, 1H), 2.03 - 1.97 (m, 2H), 1.80 - 1.75 (m, 2H), 1.49 - 1.44 (m, 1H), 1.38 (s, 9H), 1.33 - 1.29 (m, 1H).

LC-MS(ESI): [M-Boc+H]$^+$ = 476.19.

Step 4:

7"-(2,6-Dioxo-3-hydroxyflavone)-6"-oxo-7"-, 8"-dihydro-2"-H,6"- -H-diisobutyronitrile-3,1'-cyclohexane-4'-,3"-furan[2,3-e]isoindole]-1-carboxylic acid tert-butyl ester (Compound D9-5)

The compound D9-4 (200 mg, 0.35 mmol) was dissolved in toluene (2 mL). Azobis(isobutyronitrile) (114 mg, 0.66 mmol) and tributylstannyl hydride (304 mg, 1.0 mmol) were added at room temperature. The mixture was reacted at 110°C for 15 hours. The reaction mixture was concentrated, and the crude product was purified by column chromatography to obtain a white solid compound D9-5 (90 mg, 52%).

$^1$H NMR (600 MHz, $CDCl_3$) δ 7.48 (d, *J* = 7.6 Hz, 1H), 7.22 (d, *J* = 7.7 Hz, 1H), 5.23 (dd, *J*= 13.4, 5.0 Hz, 1H), 4.51 - 4.46 (m, 2H), 4.46 - 4.42 (m, 1H), 4.33 - 4.29 (m, 1H), 3.80 - 3.76 (m, 2H), 3.66 - 3.62 (m, 2H), 2.97 - 2.91 (m, 1H), 2.89 - 2.82 (m, 1H), 2.42 - 2.33 (m, 1H), 2.26 - 2.20 (m, 1H), 2.04 - 1.99 (m, 2H), 1.80 - 1.69 (m, 4H), 1.60 - 1.56 (m, 2H), 1.48 (s, 9H).

LC-MS(ESI): [M+H]$^+$= 496.39.

Step 5:

3-(6"-oxo-6"-,8" -dihydro-2"-H,7"-H-dipyrazolo[azetidin-3,1'-cyclohexane-4'-,3"-furan[2,3-e]isoindole]-7"-yl)piperidine-2,6-dione (Compound D9)

The compound D9-5 (50 mg, 0.1 mmol) was dissolved in dichloromethane: trifluoroacetic acid=10:1 (1 mL) and reacted at room temperature for 2 hours. The reaction mixture was concentrated, and the crude product was purified by reverse-phase column chromatography to obtain a white solid compound D9 (30 mg, 75%).

$^1$H NMR (600 MHz, DMSO-$d_6$) δ 10.98 (s, 1H), 8.72 (brs, 1H), 7.40 (d, *J* = 7.6 Hz, 1H), 7.29 (d, *J* = 7.6 Hz, 1H), 5.09 (dd, *J* = 13.4, 5.1 Hz, 1H), 4.49 *(q, J* = 9.1 Hz, 2H), 4.40 - 4.35 (m, 1H), 4.24 - 4.19 (m, 1H), 3.85 (t, *J* = 6.3 Hz, 2H), 3.66 (t, *J* = 6.2 Hz, 2H), 2.95 - 2.87 (m, 1H), 2.63 - 2.57 (m, 1H), 2.47 - 2.38 (m, 1H), 2.04 - 1.95 (m, 3H), 1.76 - 1.68 (m, 2H), 1.68 -

1.54 (m, 4H).
LC-MS(ESI): $[M+H]^+$= 396.39

Based on the aforementioned cereblon protein ligands, the following categories of PROTAC compounds targeting different proteins were further selectively synthesized. These PROTAC compounds were used to selectively verify that some of the cereblon protein ligands provided in the invention, as well as the PROTAC compounds generated using these ligands, exhibit favorable activity against different targets.

The first category comprises PROTAC compounds targeting the ER, synthesized according to the general formulas ER-T-1 to ER-T-9 below. The CRBN ligands used herein are the cereblon protein ligands provided in the claims of the invention. n1, n2, n3, and n4 are each independently selected from integers such as 1, 2, 3, 4, 5, etc., and are described in this manner for convenience of explanation, and are not related to the values of n1, n2, n3, and n4 in the claims.

General formula ER-T-1 for PROTAC synthesis

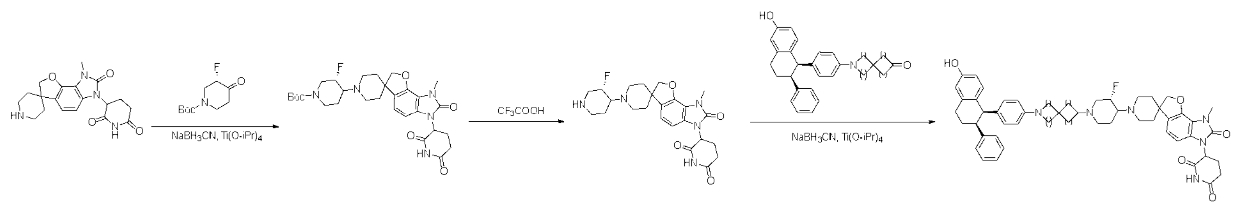


General formula ER-T-2 for PROTAC synthesis

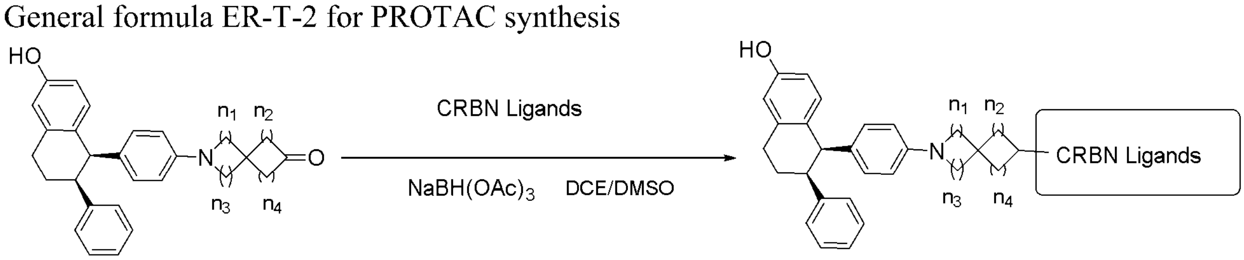


General formula ER-T-3 for PROTAC synthesis

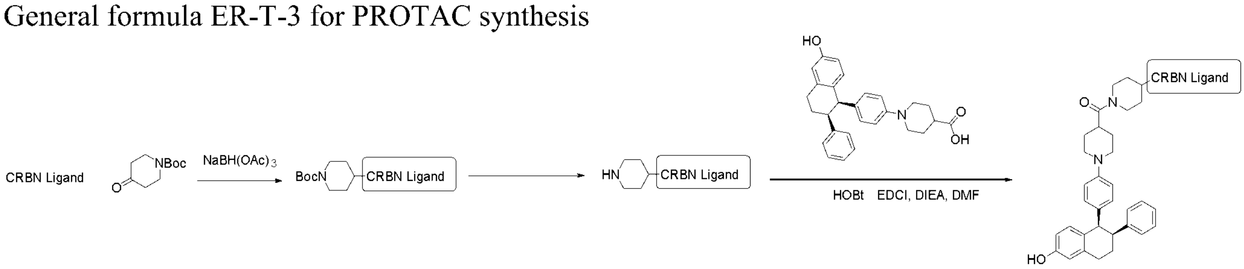


General formula ER-T-4 for PROTAC synthesis

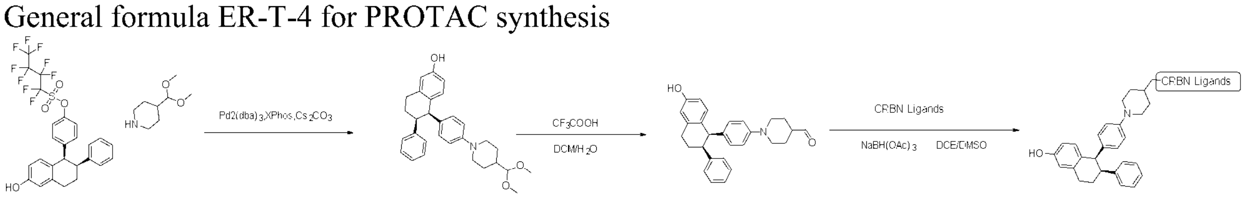

## General formula ER-T-5 for PROTAC synthesis

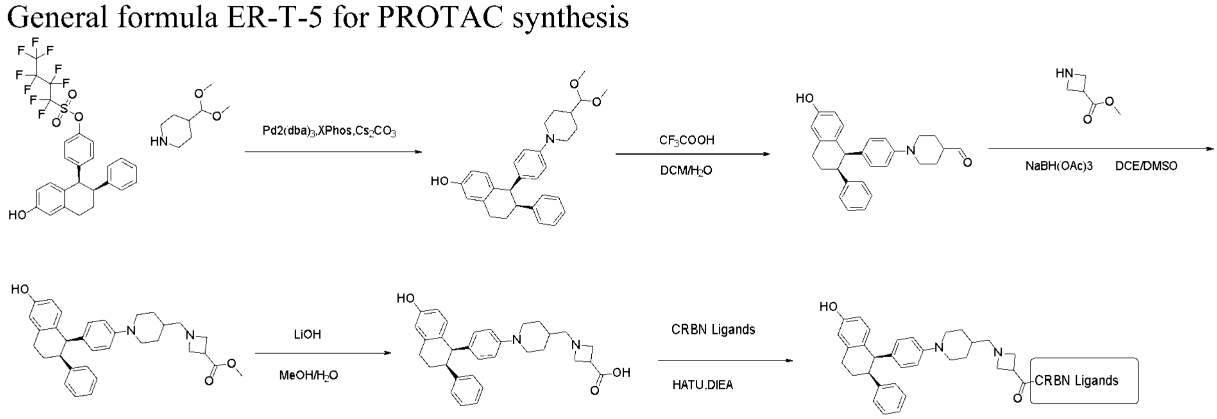


## General formula ER-T-6 for PROTAC synthesis

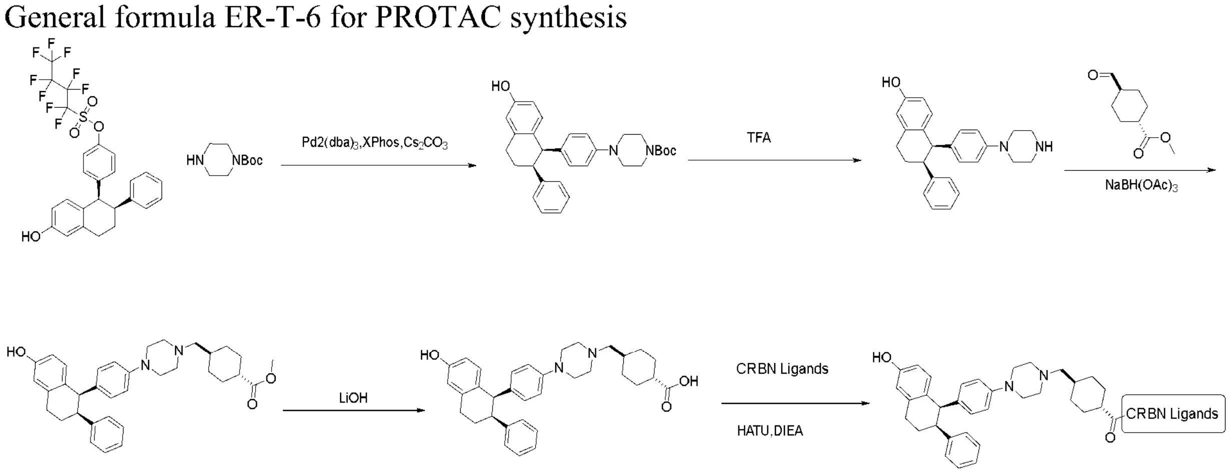


## General formula ER-T-7 for PROTAC synthesis

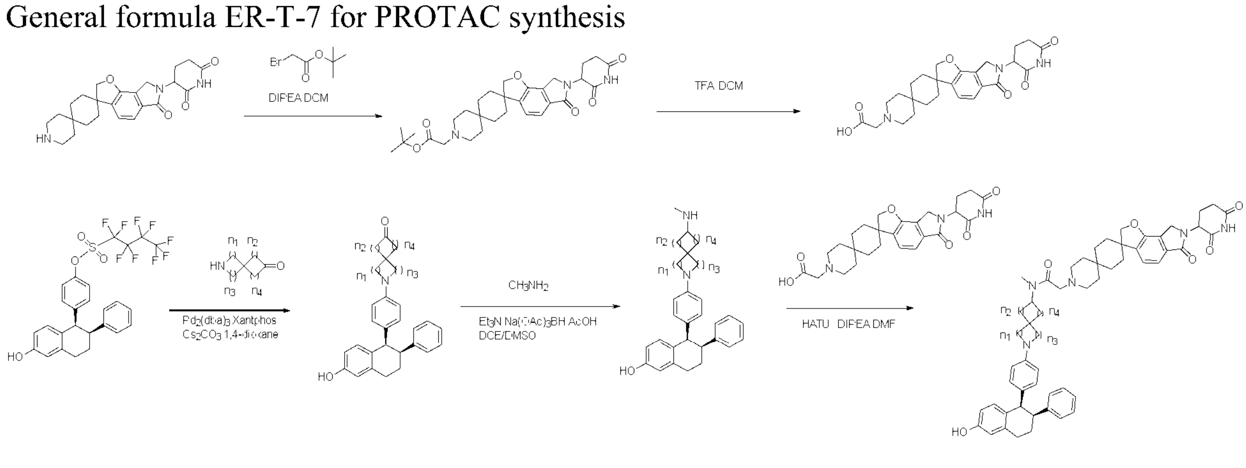


## General formula ER-T-8 for PROTAC synthesis

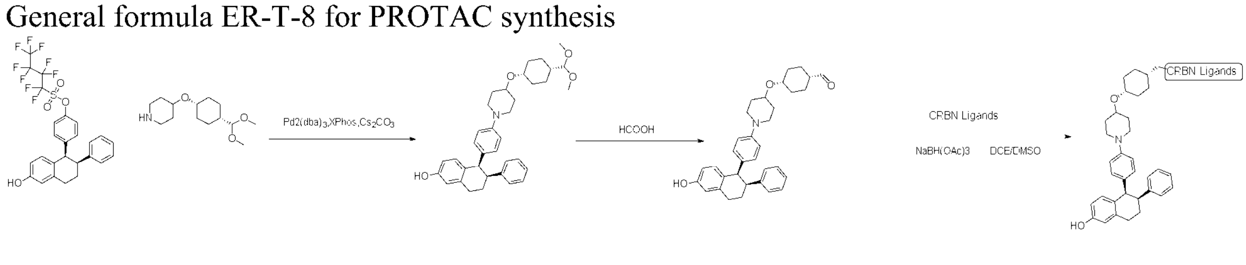

General formula ER-T-9 for PROTAC synthesis

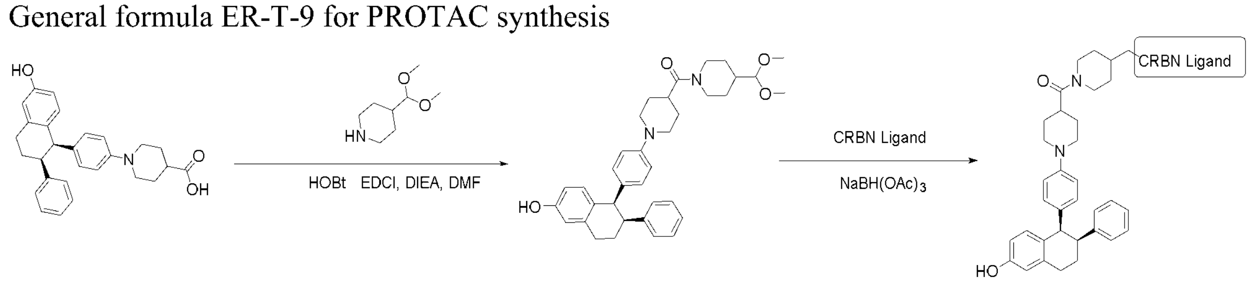


| Example | Comp ound | Structure and Name | $^1$H-NMR | LC-MS | Synthesis Method |
|---|---|---|---|---|---|
| 50 | ER-P-1 | 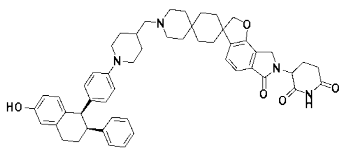 3-(1"-((1-(4-((1R,2S)-6-hydroxy-2-phenyl-1,2,3,4-tetrahydronaphthalen-1-yl)phenyl)piperidin-4-yl)methyl)-6-oxo-6,8-dihydro-2H,7H-dipyrazole[furo[2,3-e] isoindole-3,1'-cyclohexane-4',4'-piperidin]-7-yl)piperidine-2,6-dione | $^1$H NMR (600 MHz, DMSO-$d_6$) $\delta$ 10.98 (s, 1H), 9.12 (s, 1H), 7.47 (d, J = 7.6 Hz, 1H), 7.29 (d, J = 7.6 Hz, 1H), 7.14 (m, 3H), 6.84 (d, J = 7.2 Hz, 2H), 6.64 (d, J = 8.3 Hz, 1H), 6.60 (m, 3H), 6.49 (dd, J = 8.3, 2.3 Hz, 1H), 6.24 (d, J = 8.1 Hz, 2H), 5.09 (dd, J = 13.1, 2.9 Hz, 1H), 4.56 - 4.48 (m, 2H), 4.37 (d, J = 17.4 Hz, 1H), 4.21 (dd, J = 17.1, 3.3 Hz, 1H), 4.15 (d, J = 4.6 Hz, 1H), 3.55 (m, 2H), 3.10 - 2.86 (m, 9H), 2.60 (m, 3H), 2.45 - 2.38 (m, 2H), 2.34 (d, J = 13.9 Hz, 2H), 2.14 - 2.05 (m, 3H), 2.03 - 1.96 (m, 2H), 1.89 - 1.63 (m, 8H), 1.61 - 1.50 (m, 4H), 1.43 - 1.34 (m, 2H). | 819.66 | General formula ER-T-4 for PROTAC synthesis |

(continued)

| Example | Comp ound | Structure and Name | $^1$H-NMR | LC-MS | Synthesis Method |
|---|---|---|---|---|---|
| 51 | ER-P-2 | 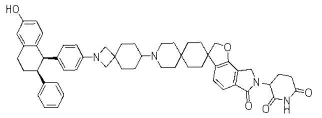 3-(1''-(2-(4-(((1R,2S)-6-hydroxy-2-phenyl-1,2,3,4-tetrahydronaphthalen-1-yl)phenyl)-2-azaspiro[3.5]nonan-7-yl)-6-oxo-6,8-dihydro-2H,7H-dipyrazole[furo[2,3-e]isoindole-3,1'-cyclohexane-4',4'-piperidin]-7-yl)piperidine-2,6-dione | $^1$H NMR (600 MHz, DMSO-$d_6$) $\delta$ 10.97 (s, 1H), 9.10 (s, 1H), 7.46 (d, J = 7.2 Hz, 1H), 7.28 (d, J = 7.5 Hz, 1H), 7.13 (m, 3H), 6.83 (d, J = 7.4 Hz, 2H), 6.63 (d, J = 8.3 Hz, 1H), 6.60 (s, 1H), 6.48 (dd, J = 8.3, 1.9 Hz, 1H), 6.17 (d, J = 8.2 Hz, 2H), 6.03 (d, J = 8.2 Hz, 2H), 5.08 (dd, J = 13.2, 5.0 Hz, 1H), 4.51 (m, 2H), 4.37 (d, J = 17.0 Hz, 1H), 4.21 (d, J = 17.0 Hz, 1H), 4.11 (d, J = 4.7 Hz, 1H), 3.46 - 3.38 (m, 2H), 3.28 (d, J = 14.8 Hz, 2H), 3.02 - 2.86 (m, 4H), 2.60 (m, 1H), 2.46 - 2.38 (m, 2H), 2.10 (m, 2H), 2.03 - 1.77 (m, 8H), 1.70 (m, 2H), 1.60 - 1.43 (m, 8H), 1.27 - 1.23 (m, 3H). | 845.56 | General formula ER-T-2 for PROTAC synthesis |
| 52 | ER-P-3 | 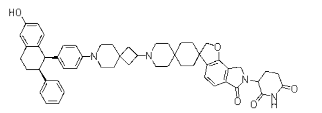 3-(1''-(7-(4-(((1R,2S)-6-hydroxy-2-phenyl-1,2,3,4-tetrahydronaphthalen-1-yl)phenyl)-7-azaspiro[3.5]nonan-2-yl)-6-oxo-6,8-dihydro-2H,7H-dihydro[furo[2,3-e]isoindole-3,1'-cyclohexane-4',4'-piperidin]-7-yl)piperidine-2,6-dione | $^1$H NMR (600 MHz, DMSO-$d_6$) $\delta$ 10.97 (s, 1H), 9.12 (s, 1H), 7.47 (d, J = 7.6 Hz, 1H), 7.29 (d, J = 7.6 Hz, 1H), 7.13 (m, 3H), 6.84 (d, J = 7.1 Hz, 2H), 6.64 (d, J = 8.3 Hz, 1H), 6.61 (s, 3H), 6.49 (dd, J = 8.3, 2.1 Hz, 1H), 6.24 (d, J = 7.5 Hz, 2H), 5.08 (dd, J = 13.1 Hz, 1H), 4.52 (m, 2H), 4.37 (d, J = 16.8 Hz, 1H), 4.21 (dd, J = 17.1, 3.1 Hz, 1H), 4.15 (d, J = 4.4 Hz, 1H), 3.76 (dd, J = 16.4, 8.3 Hz, 1H), 3.35 - 3.27(m, 2H),3.23 (s, 3H), 3.03 - 2.84 (m, 9H), 2.60 (d, J = 18.2 Hz, 1H), 2.44 - 2.37 (m, 2H), 2.17 (d, J = 8.3 Hz, 2H), 2.06 - 1.91 (m, 5H), 1.89 - 1.78 (m, 3H), 1.71 (s, 1H), 1.59 (d, J = 12.3 Hz, 7H), 1.45 - 1.35 (m, 2H). | 845.56 | General formula ER-T-2 for PROTAC synthesis |

(continued)

| Example | Comp ound | Structure and Name | $^1$H-NMR | LC-MS | Synthesis Method |
|---|---|---|---|---|---|
| 53 | ER-P-4 | 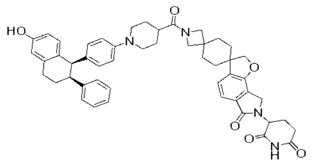 3-(1-(1-(4-(((1R,2S)-6-hydroxy-2-phenyl-1,2,3,4-tetrahydro-naphthalen-1-yl)phenyl)piperi-dine-4-carbonyl)-6'-oxo-6',8'-dihydro-2'H,7'H-diazetidine-3,1'-cyclohexane-4',3"-furo[2,3-e]isoindol]-7'-yl)piperidine-2,6-dione | $^1$H NMR (600 MHz, DMSO-$d_6$) δ 10.98 (s, 1H), 9.13 (s, 1H), 7.41 (d, J = 7.6 Hz, 1H), 7.37 (d, J = 7.5 Hz, 1H), 7.28 (dd, J = 7.6, 2.5 Hz, 1H), 7.14 (m, 3H), 6.84 (dd, J = 6.9, 3.3 Hz, 2H), 6.66 (d, J = 8.2 Hz, 1H), 6.61 (s, 2H), 6.49 (d, J = 8.2 Hz, 1H), 6.25 (s, 2H), 5.09 (d, J = 13.2 Hz, 1H), 4.52 (m, 2H), 4.38 (d, J = 17.1 Hz, 1H), 4.21 (d, J = 17.0 Hz, 1H), 4.16 (s, 1H), 4.02 (s, 1H), 3.83 (s, 1H), 3.68 (s, 1H), 3.56 (m, 2H), 3.51 (s, 1H), 3.03 - 2.87 (m, 5H), 2.63 - 2.57 (m, 2H), 2.44 - 2.38 (m, 2H), 2.09 (d, J = 19.0 Hz, 2H), 2.03 - 1.95 (m, 2H), 1.87 (s, 2H), 1.74 - 1.58 (m, 9H). | 805.4 6 | General formula ER-T-3 for PROTAC synthesis |
| 54 | ER-P-5 | 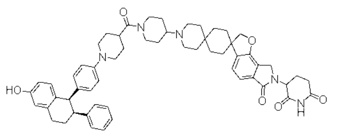 3-(1"-(1-(1-(4-(((1R,2S)-6-hydroxy-2-phenyl-1,2,3,4-tetrahydro-naphthalen-1-yl)phenyl)piperi-dine-4-carbonyl)piperidin-4-yl)-6-oxo-6,8-dihydro-2H,7H-dihydro [furo[2,3-e]isoindole-3,1'-cyclohexane-4',4'-piperidin]-7-yl)piper-idine-2,6-dione | $^1$H NMR (600 MHz, DMSO-$d_6$) δ 10.98 (s, 1H), 9.00 (s, 1H), 7.48 (d, J = 7.6 Hz, 1H), 7.29 (d, J = 7.6 Hz, 1H), 7.21 (s, 1H), 7.13 (m, 3H), 6.84 (d, J = 7.2 Hz, 2H), 6.65 (d, J = 8.4 Hz, 1H), 6.61 (s, 2H), 6.49 (dd, J = 8.2, 2.3 Hz, 1H), 6.25 (s, 2H), 5.09 (d, J = 14.5 Hz, 1H), 4.60 - 4.51 (m, 3H), 4.38 (d, J = 17.3 Hz, 1H), 4.21 (dd, J =17.0, 4.6 Hz, 1H), 4.18 - 4.10 (m, 2H), 3.55 (s, 2H), 3.13 - 2.86 (m, 8H), 2.77 (s, 1H), 2.65 - 2.58 (m, 1H), 2.45 - 2.41( m, 2H), 2.15 - 1.96 (m, 6H), 1.92 - 1.75 (m, 4H), 1.74 - 1.54 (m, 8H), 1.50 - 1.40 (m,4H), 1.40 - 1.30 ( m ,2 H). | 916.75 | General formula ER-T-3 for PROTAC synthesis |

(continued)

| Example | Comp ound | Structure and Name | $^1$H-NMR | LC-MS | Synthesis Method |
|---|---|---|---|---|---|
| 55 | ER-P-6 | 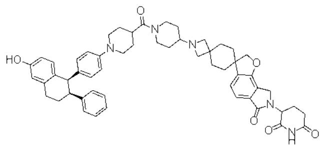 3-(1-(1-(1-(4-(((1R,2S)-6-hydroxy-2-phenyl-1,2,3,4-tetrahydronaphthalen-1-yl)phenyl)piperidine-4-carbonyl)-6'-oxo-6',8'-dihydro-2'H,7'H-diisoazetidine-3,1'-cyclohexane-4',3"-furo [2,3-e]isoindol]-7'-yl)piperidine-2,6-dione | $^1$H NMR (600 MHz, DMSO-$d_6$) δ 10.98 (s, 1H), 9.76 (s, 1H), 7.39 (d, J = 7.6 Hz, 1H), 7.30 (d, J = 7.6 Hz, 1H), 7.18 - 7.09 (m, 3H), 6.84 (d, J = 7.1 Hz, 2H), 6.69 - 6.57 (m, 4H), 6.49 (dd, J = 8.2, 2.2 Hz, 1H), 6.26 (s, 2H), 5.09 (dd, J = 13.3, 5.0 Hz, 1H), 4.54 - 4.47 (m, 3H), 4.37 (d, J = 17.0 Hz, 1H), 4.21 (d, J = 17.1 Hz, 1H), 4.18- 4.01 (m, 5H), 3.95 (s, 1H), 3.81 (s, 1H), 3.55 (m, 2H), 3.05 - 2.87 (m, 6H), 2.80 (s, 2H), 2.64 - 2.56 (m, 2H), 2.44 - 2.38 (m, 2H), 2.12 - 1.91 (m, 8H), 1.82 (d, J = 12.9 Hz, 2H), 1.74 - 1.66 (m, 8H). | 888.76 | General formula ER-T-3 for PROTAC synthesis |
| 56 | ER-P-7 | 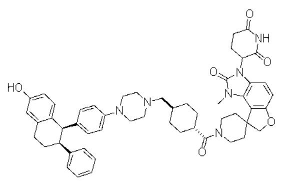 3-(1'-((1R,4r)-4-((4-(4-(((1R,2-S)-6-hydroxy-2-phenyl-1,2,3,4-tetrahydronaphthalen-1-yl)phenyl)piperazin-1-yl)methyl)cyclohexane-1-carbonyl)-1-methyl-2-oxo-1,2-dihydro-3H,7H-spiro [benzofuro[4,5]imid azole-8,4'-piperidin]-3-yl)piperidine-2,6-dione | $^1$H NMR (600 MHz, DMSO-$d_6$) δ 11.09 (s, 1H), 9.15 (s, 1H), 7.18 - 7.11 (m, 3H), 6.93 (d, J = 9.6 Hz, 1H), 6.87 - 6.83 (m, 2H), 6.66 - 6.60 (m, 4H), 6.55 (d, J = 8.4 Hz, 1H), 6.49 (dd, J = 8.3, 2.6 Hz, 1H), 6.27 (d, J = 8.5 Hz, 2H), 5.33 (m, 1H), 4.57 - 4.46 (m, 4H), 4.18 (d, J = 5.0 Hz, 1H), 4.02 (d, J = 13.3 Hz, 1H), 3.64 (t, J = 10.6 Hz, 2H), 3.55 (s, 2H), 3.47 (s, 2H), 3.17 (s, 2H), 3.07 (d, J = 11.1 Hz, 2H), 3.02 - 2.84 (m, 7H), 2.70 - 2.58 (m, 4H), 2.12 (m, 1H), 2.02 - 1.94 (m, 2H), 1.92 - 1.77 (m, 5H), 1.72 (s, 3H), 1.52 - 1.41(m, 2H), 1.13 - 1.03(m, 2H). | 877.60 | General formula ER-T-6 for PROTAC synthesis |

(continued)

| Example | Comp ound | Structure and Name | $^1$H-NMR | LC-MS | Synthesis Method |
|---|---|---|---|---|---|
| 57 | ER-P-8 | 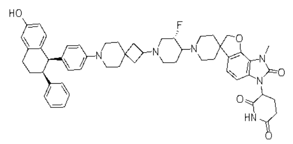 3-(1'-((3S)-3-fluoro-1-(7-(4-(((1R,2S)-6-hydroxy-2-phenyl-1,2,3,4-tetrahydronaphthalen-1-yl)phenyl)-7-azaspiro[3.5]nonan-2-yl)piperidin-4-yl)-1-methyl-2-oxo-1,2-dihydro-3H,7H-spiro[benzofuro[6,7-d]imidazole-6,4'-piperidin]-3-yl)piperidine-2,6-dione | $^1$H NMR (400 MHz, DMSO-d6) δ 11.11 (s, 1H), 9.15 (s, 1H), 7.14 (m, 3H), 6.87 - 6.77 (m, 3H), 6.70 (d, J = 7.9 Hz, 1H), 6.66 - 6.57 (m, 4H), 6.48 (dd, J = 8.2, 2.5 Hz, 1H), 6.24 (d, J = 8.2 Hz, 2H), 5.33 (dd, J = 12.5, 5.5 Hz, 1H), 4.63 (s, 2H), 4.15 (d, J = 4.9 Hz, 1H), 3.75 (s, 3H), 3.45 (s, 6H), 3.06 - 2.87 (m, 9H), 2.72 - 2.58 (m, 2H), 2.23 - 1.90 (m, 12H), 1.76 - 1.60 (m, 5H). | 863.65 | General formula ER-T-1 for PROTAC synthesis |
| 58 | ER-P-9 | 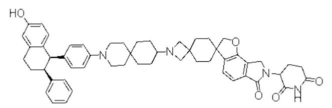 3-(1-(3-(4-(((1R,2S)-6-hydroxy-2-phenyl-1,2,3,4-tetrahydronaphthalen-1-yl)phenyl)-3-azaspiro[5.5]undecan-9-yl)-6'-oxo-6',8'-dihydro-2'H,7'H-dipyrazole[azetidine-3,1'-cyclohexane-4',3''-furo[2,3-e]isoindol]-7'-yl)piperidine-2,6-dione | $^1$H NMR (600 MHz, DMSO-d6) δ 10.97 (s, 1H), 10.03 (s, 1H), 7.39 (d, J = 7.6 Hz, 1H), 7.30 (d, J = 7.6 Hz, 1H), 7.17 - 7.11 (m, J = 13.9, 3H), 6.87 - 6.70 (m, 4H), 6.66 - 6.61 (m, 2H), 6.49 (dd, J = 8.4, 2.5 Hz, 1H), 6.31 (s, 2H), 5.09 (dd, J = 13.3, 5.1 Hz, 1H), 4.53 - 4.47 (m, 2H), 4.37 (dd, J = 17.0, 2.3 Hz, 1H), 4.22 (dd, J = 17.3, 3.8 Hz, 2H), 4.10 - 4.08 (m, 1H), 3.99 - 3.96 (m, 1H), 3.90 - 3.87 (m, 1H), 3.80 - 3.78 (m, 1H), 3.33 (dd, J = 13.9, 5.2 Hz, 1H), 3.20 - 3.05 (m, 4H), 3.01 - 2.87 (m, 3H), 2.65 - 2.56 (m, 1H), 2.45 - 2.39 (m, 1H), 2.11 - 2.07 (m, 2H), 2.01 - 1.89 (m, 2H), 1.89 - 1.54 (m, 13H), 1.48 (s, 2H), 1.37 - 1.25 (m, 2H), 1.13 - 1.08 (m, 2H). | 845.4 6 | General formula ER-T-2 for PROTAC synthesis |

(continued)

| Example | Comp ound | Structure and Name | $^1$H-NMR | LC-MS | Synthesis Method |
|---|---|---|---|---|---|
| 59 | ER-P-10 | 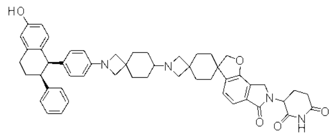 3-(1-(2-(4-((1R,2S)-6-hydroxy-2-phenyl-1,2,3,4-tetrahydronaphthalen-1-yl)phenyl)-2-azaspiro[3.5]nonan-7-yl)-6'-oxo-6',8'-dihydro-2'H,7'H-dipyrazole[azetidine-3,1'-cyclohexane-4',3"-furo [2,3-e]isoindol]-7'-yl)piperidine-2,6-dione | $^1$H NMR (600 MHz, DMSO-d6) δ 10.97 (s, 1H), 9.85 (s, 1H), 7.39 (d, J = 7.6 Hz, 1H), 7.30 (d, J = 7.5 Hz, 1H), 7.16 - 7.10 (m, 3H), 6.84 (d, J = 7.3 Hz, 2H), 6.68 - 6.57 (m, 2H), 6.48 (dd, J = 8.3, 2.5 Hz, 1H), 6.18 (d, J = 8.1 Hz, 2H), 6.05 (d, J = 8.0 Hz, 2H), 5.09 (dd, J = 13.2, 5.1 Hz, 1H), 4.52 - 4.47 (m, 2H), 4.37 (dd, J = 17.3, 1.9 Hz, 1H), 4.22 (dd, J = 17.2, 2.9 Hz, 2H), 4.12 (d, J = 5.0 Hz, 1H), 4.07 (d, J = 7.6 Hz, 1H), 4.02 - 3.94 (m, 1H), 3.91 - 3.88 (m, 1H), 2.79 - 2.76 (m, 1H), 3.45 - 3.35 (m, 4H), 3.30 - 3.26 (m, 1H), 3.20 - 3.17 (m, 1H), 3.00 - 2.87 (m, 3H), 2.64 - 2.57 (m, 1H), 2.46 - 2.39 (m, 1H), 2.11 - 2.06 (m, 2H), 2.00 - 1.96 (m, 4H), 1.88 - 1.86 (m, 2H), 1.73 - 1.58 (m, 6H), 1.48 - 1.43 (m, 2H), 1.22 - 1.12(m, 2H). | 817.4 3 | General formula ER-T-2 for PROTAC synthesis |
| 60 | ER-P-11 | 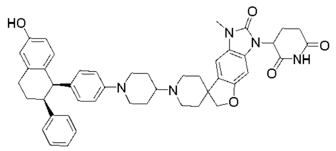 3-(1'-(1-(4-((1R,2S)-6-hydroxy-2-phenyl-1,2,3,4-tetrahydronaphthalen-1-yl)phenyl)piperidin-4-yl)-1-methyl-2-oxo-1,2-dihydro-3H,6H-spiro[benzofuro [5,6-d]imidazole-7,4'-piperidin]-3-yl)piperidine-2,6-dione | $^1$H NMR (600 MHz, DMSO-d6) δ 11.08 (s, 1H), 9.40 (s, 1H), 7.18 - 7.12 (m, 3H), 6.88 - 6.82 (m, 3H), 6.74 (d, J = 3.9 Hz, 1H), 6.67 - 6.59 (m, 4H), 6.49 (dd, J = 8.3, 2.6 Hz, 1H), 6.25 (d, J = 8.3 Hz, 2H), 5.32 (dd, J = 12.9, 5.4 Hz, 1H), 4.50 - 4.46 (m, 2H), 4.16 (d, J = 5.0 Hz, 1H), 3.76 - 3.70 (m, 2H), 3.55 (d, J = 11.9 Hz, 2H), 3.39 - 3.28 (m, 5H), 3.11 - 3.05 (m, 2H), 3.02 - 2.82 (m, 3H), 2.76 - 2.67 (m, 1H), 2.65 - 2.56 (m, 3H), 2.23 - 2.14 (m, 2H), 2.14 - 2.06 (m, 3H), 1.99 - 1.92 (m, 3H), 1.74 - 1.67 (m, 3H). | 752.3 7 | General formula ER-T-2 for PROTAC synthesis |

(continued)

| Example | Comp ound | Structure and Name | $^1$H-NMR | LC-MS | Synthesis Method |
|---|---|---|---|---|---|
| 61 | ER-P-12 | 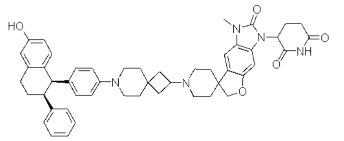 3-(1'-(7-(4-((1R,2S)-6-hydroxy-2-phenyl-1,2,3,4-tetrahydronaphthalen-1-yl)phenyl)-7-azaspiro[3.5]nonan-2-yl)-1-methyl-2-oxo-1,2-dihydro-3H,6H-spiro [benzofuro[5,6-d]imidazole-7,4'-piperidin]-3-yl)piperidine-2,6-dione | $^1$H NMR (600 MHz, DMSO-d6) δ 11.08 (s, 1H), 9.74 (s, 1H), 7.21 - 7.08 (m, 3H), 6.88 - 6.81 (m, 3H), 6.75 - 6.70 (m, 3H), 6.66 - 6.60 (m, 2H), 6.49 (dd, J = 8.3, 2.5 Hz, 1H), 6.28 (d, J = 8.1 Hz, 2H), 5.32 (dd, J = 13.3, 5.4 Hz, 1H), 4.46 (s, 2H), 4.18 (d, J = 5.0 Hz, 1H), 3.41 (d, J = 12.0 Hz, 2H), 3.32 (s, 4H), 3.09 (s, 2H), 3.05 - 2.96 (m, 3H), 2.91 - 2.84 (m, 3H), 2.74 - 2.71 (m, 1H), 2.66 - 2.57 (m, 1H), 2.24 - 2.20 (m, 2H), 2.18 - 2.06 (m, 3H), 2.05 - 2.00 (m, 2H), 2.00 - 1.90 (m, 3H), 1.74 - 1.67 (m, 6H). | 792.4 0 | General formula ER-T-2 for PROTAC synthesis |
| 62 | ER-P-13 | 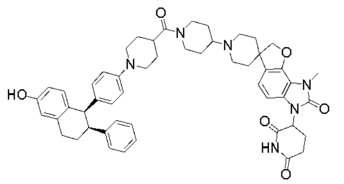 3-(1'-(1-(1-(4-((1R,2S)-6-hydroxy-2-phenyl-1,2,3,4-tetrahydronaphthalen-1-yl)phenyl)piperidine-4-carbonyl)piperidin-4-yl)-1-methyl-2-oxo-1,2-dihydro-3H,7H-spiro[benzofuro[6,7-d]imidazole-6,4'-piperidin]-3-yl)piperidine-2,6-dione | $^1$H NMR (600 MHz, DMSO-d6) δ 11.08 (s, 1H), 8.25 (s, 1H), 7.18 - 7.10 (m, 3H), 6.88 (d, J = 8.0 Hz, 1H), 6.87 - 6.81 (m, 2H), 6.65 (d, J = 8.4 Hz, 1H), 6.64 - 6.59 (m, 2H), 6.54 (d, J = 8.5 Hz, 2H), 6.49 (dd, J = 8.3, 2.6 Hz, 1H), 6.21 (d, J = 8.5 Hz, 2H), 5.31 (dd, J = 12.9, 5.5 Hz, 1H), 4.49 (s, 2H), 4.42 (d, J = 12.6 Hz, 1H), 4.13 (d, J = 5.0 Hz, 1H), 4.01 (d, J = 13.1 Hz, 1H), 3.61 - 3.50 (m, 3H), 3.44 (s, 3H), 3.04 - 2.80 (m, 7H), 2.73 - 2.66 (m, 2H), 2.64 - 2.56 (m, 3H), 2.27 - 2.17 (m, 2H), 2.14 - 2.07 (m, 1H), 2.02 - 1.96 (m, 1H), 1.86 - 1.54 (m, 11H). | 863.4 4 | General formula ER-T-3 for PROTAC synthesis |

(continued)

| Example | Comp ound | Structure and Name | $^1$H-NMR | LC-MS | Synthesis Method |
|---|---|---|---|---|---|
| 63 | ER-P-14 | 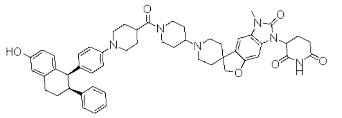 3-(1'-(1-(1-(4-((1R,2S)-6-hydroxy-2-phenyl-1,2,3,4-tetrahydronaphthalen-1-yl)phenyl)piperidine-4-carbonyl)piperidin-4-yl)-1-methyl-2-oxo-1,2-dihydro-3H,6H-spiro[benzofuro[5,6-d]imidazole-7,4'-piperidin]-3-yl)piperidine-2,6-dione | $^1$H NMR (600 MHz, DMSO-d6) δ 11.07 (s, 1H), 9.12 (s, 1H), 7.18 - 7.09 (m, 3H), 6.84 (d, J = 6.5 Hz, 3H), 6.74 (s, 1H), 6.65 (d, J = 8.3 Hz, 1H), 6.61 (d, J = 2.5 Hz, 1H), 6.55 (d, J = 8.2 Hz, 2H), 6.49 (dd, J = 8.3, 2.6 Hz, 1H), 6.22 (d, J = 8.3 Hz, 2H), 5.32 (dd, J = 13.0, 5.5 Hz, 1H), 4.57 (d, J = 12.6 Hz, 1H), 4.48 (s, 2H), 4.22 - 4.11 (m, 2H), 3.58 - 3.52 (m, 5H), 3.37 (s, 1H), 3.32 (s, 3H), 3.28 (dd, J = 4.9, 2.3 Hz, 1H), 3.07 (d, J = 12.3 Hz, 3H), 3.02 - 2.82 (m, 3H), 2.77 - 2.68 (m, 2H), 2.67 - 2.53 (m, 4H), 2.31 - 2.04 (m, 5H), 2.02 - 1.83 (m, 3H), 1.73 - 1.70 (m, 1H), 1.64-1.61 (m, 5H). | 863.4 4 | General formula ER-T-3 for PROTAC synthesis |
| 64 | ER-P-15 | 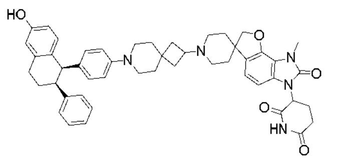 3-(1'-(7-(4-((1R,2S)-6-hydroxy-2-phenyl-1,2,3,4-tetrahydronaphthalen-1-yl)phenyl)-7-azaspiro[3.5]nonan-2-yl)-1-methyl-2-oxo-1,2-dihydro-3H,7H-spiro [benzofuro[6,7-d]imidazole-6,4'-piperidin]-3-yl)piperidine-2,6-dione | $^1$H NMR (600 MHz, DMSO-d6) δ 11.11 (s, 1H), 9.26 (s, 1H), 7.18 - 7.09 (m, 3H), 6.87 - 6.81 (m, 2H), 6.77 (d, J = 8.0 Hz, 1H), 6.69 (d, J = 8.0 Hz, 1H), 6.65 - 6.58 (m, 2H), 6.48 (dd, J = 8.3, 2.6 Hz, 1H), 6.19 (d, J = 8.3 Hz, 2H), 6.05 (d, J = 8.4 Hz, 2H), 5.34 (dd, J = 13.0, 5.3 Hz, 1H), 4.63 (s, 2H), 4.12 (d, J = 5.0 Hz, 1H), 3.53 - 3.46 (s, 6H), 3.37 (s, 2H), 3.31 - 3.22 (m, 2H), 3.08 (q, J = 12.0 Hz, 2H), 3.01 - 2.86 (m,2H), 2.73 - 2.59(m, 2 H), 2.17 - 2.08 (m, 3H), 2.04 - 1.91 (m, 7H), 1.71 - 1.70 (m, 1H), 1.56 - 1.48 (m, 4H). | 792.4 0 | General formula ER-T-2 for PROTAC synthesis |

(continued)

| Example | Comp ound | Structure and Name | $^1$H-NMR | LC-MS | Synthesis Method |
|---|---|---|---|---|---|
| 65 | ER-P-16 | 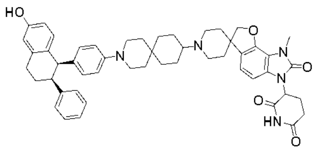 3-(1'-(3-(4-((1R,2S)-6-hydroxy-2-phenyl-1,2,3,4-tetrahydronaphthalen-1-yl)phenyl)-3-azaspiro[5.5]undecan-9-yl)-1-methyl-2-oxo-1,2-dihydro-3H,7H-spiro[benzofuro[6,7-d]imidazole-6,4'-piperidin]-3-yl)piperidine-2,6-dione | $^1$H NMR (600 MHz, DMSO-d6) δ 11.10 (s, 1H), 9.14 (s, 1H), 7.19 - 7.09 (m, 3H), 6.91 - 6.80 (m, 2H), 6.77 (d, J = 8.1 Hz, 1H), 6.75 - 6.53 (m, 4H), 6.49 (dd, J = 8.3, 2.6 Hz, 1H), 6.29 (s, 3H), 5.34 (dd, J = 13.2, 5.2 Hz, 1H), 4.63 (s, 2H), 4.19 (s, 1H), 3.54 - 3.52 (m, 2H) , 3.45 (s, 3H), 3.38 - 3.31 (m, 2H), 3.20 - 3.17 (m, 2H), 3.13 - 3.02 (m, 4H), 3.02 - 2.85 (m, 4H), 2.74 - 2.58 (m, 2H), 2.26 - 2.03 (m, 3H), 2.00 - 1.89 (m, 6H), 1.76 - 1.72 (m, 1H), 1.66 - 1.60 (m, 4H), 1.45 - 1.40 (m, 2H), 1.35 - 1.06 (m, 2H). | 820.4 4 | General formula ER-T-2 for PROTAC synthesis |
| 66 | ER-P-17 | 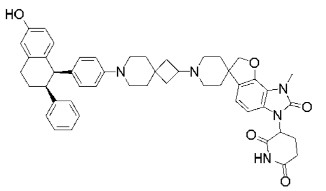 3-(1'-(7-(4-((1R,2S)-6-hydroxy-2-phenyl-1,2,3,4-tetrahydronaphthalen-1-yl)phenyl)-7-azaspiro[3.5]nonan-2-yl)-1-methyl-2-oxo-1,2-dihydro-3H,7H-spiro [benzofuro[6,7-d]imidazole-6,4'-piperidin]-3-yl)piperidine-2,6-dione | $^1$H NMR (600 MHz, DMSO-d6) δ 11.11 (s, 1H), 9.69 (s, 1H), 7.17 - 7.11 (m, 3H), 6.88 - 6.81 (m, 2H), 6.77 (d, J = 8.1 Hz, 1H), 6.70 (d, J = 7.8 Hz, 2H), 6.67 - 6.57 (m, 3H), 6.49 (dd, J = 8.3, 2.5 Hz, 1H), 6.27 (d, J = 8.0 Hz, 2H), 5.34 (dd, J = 13.0, 5.5 Hz, 1H), 4.62 (s, 2H), 4.17 (d, J = 5.0 Hz, 1H), 3.49 - 3.39 (m, 5H), 3.35 - 3.27 (m, 2H), 3.17 - 3.15 (m, 1H), 3.07 (s, 2H), 3.02 - 2.96 (m, 2H), 2.96 - 2.85 (m, 4H), 2.75 - 2.57 (m, 2H), 2.21 - 2.19 (m, 2H), 2.14 - 1.92 (m, 8H), 1.75 - 1.66 (m, 5H). | 792.4 0 | General formula ER-T-2 for PROTAC synthesis |

(continued)

| Example | Comp ound | Structure and Name | $^1$H-NMR | LC-MS | Synthesis Method |
|---|---|---|---|---|---|
| 67 | ER-P-18 | 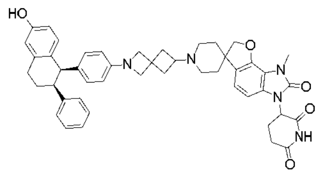 3-(1'-(2-(4-((1R,2S)-6-hydroxy-2-phenyl-1,2,3,4-tetrahydronaphthalen-1-yl)phenyl)-2-azaspiro[3.3]heptan-6-yl)-1-methyl-2-oxo-1,2-dihydro-3H,7H-spiro [benzofuro[6,7-d]imidazole-6,4'-piperidin]-3-yl)piperidine-2,6-dione | $^1$H NMR (600 MHz, DMSO-d6) δ 11.11 (s, 1H), 9.13 (s, 1H), 7.20 - 7.09 (m, 3H), 6.87 - 6.81 (m, 2H), 6.77 (d, J = 8.0 Hz, 1H), 6.69 (d, J = 8.0 Hz, 1H), 6.65 - 6.59 (m, 2H), 6.48 (dd, J = 8.3, 2.6 Hz, 1H), 6.18 (d, J = 8.3 Hz, 2H), 6.06 (d, J = 8.3 Hz, 2H), 5.34 (dd, J = 12.8, 5.5 Hz, 1H), 4.61 (s, 2H), 4.12 (d, J = 4.9 Hz, 1H), 3.78 - 3.71 (m, 2H), 3.67 - 3.60 (m, 3H), 3.45 (s, 3H), 3.41 - 3.39 (m, 2H), 3.31 - 3.25 (m, 1H), 2.99 - 2.87 (m, 5H), 2.74 - 2.58 (m, 2H), 2.46 - 2.35 (m, 3H), 2.16 - 1.87 (m, 7H), 1.71 - 1.69 (m, 1H). | 864.3 7 | General formula ER-T-2 for PROTAC synthesis |
| 68 | ER-P-19 | 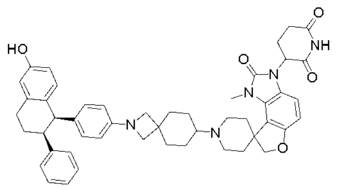 3-(1'-(2-(4-((1R,2S)-6-hydroxy-2-phenyl-1,2,3,4-tetrahydronaphthalen-1-yl)phenyl)-2-azaspiro[3.5]nonan-7-yl)-1-methyl-2-oxo-1,2-dihydro-3H,7H-spiro [benzofuro[4,5-d]imidazole-8,4'-piperidin]-3-yl)piperidine-2,6-dione | $^1$H NMR (600 MHz, DMSO-d6) δ 11.10 (s, 1H), 9.11 (s, 1H), 7.18 - 7.09 (m, 3H), 6.96 (d, J = 8.5 Hz, 1H), 6.84 (d, J = 7.4 Hz, 2H), 6.66 - 6.53 (m, 3H), 6.48 (dd, J = 8.3, 2.6 Hz, 1H), 6.19 (d, J = 8.2 Hz, 2H), 6.05 (d, J = 8.1 Hz, 2H), 5.36 (dd, J = 13.5, 5.0 Hz, 1H), 4.55 - 4.43 (m, 2H), 4.12 (d, J = 5.0 Hz, 1H), 3.60 (s, 3H), 3.38 (s, 2H), 3.32 - 3.18 (m, 4H), 3.12 - 3.10 (m, 2H), 3.03 - 2.82 (m, 2H), 2.08 - 2.02 (m, 8H), 1.72 - 1.69 (m, 1H), 1.56 - 1.49 (m, 5H). | 792.4 0 | General formula ER-T-2 for PROTAC synthesis |

(continued)

| Example | Comp ound | Structure and Name | $^1$H-NMR | LC-MS | Synthesis Method |
|---|---|---|---|---|---|
| 69 | ER-P-20 | 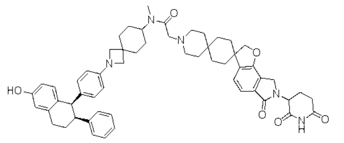 2-(7-(2,6-dioxohesperidin-3-yl)-6-oxo-7,8-dihydro-2H,6H-dipyrazole[furo[2,3-e]isoindole-3,1'-cyclohexane-4',4'-piperidin]-1"-yl)-N-(2-(4-((1R,2S)-6-hydroxy-2-phenyl-1,2,3,4-tetrahydronaphthalen-1-yl)phenyl)-2-azaspiro[3.5]nonan-7-yl)-N-methylacetamide | $^1$H NMR (400 MHz, DMSO-d6) δ 10.99 (s, 1H), 9.30 (s, 1H), 7.46 (d, J = 7.5 Hz, 1H), 7.29 (d, J = 7.5 Hz, 1H), 7.17 - 7.09 (m, 3H), 6.83 (d, J = 7.2 Hz, 2H), 6.67 - 6.57 (m, 2H), 6.48 (dd, J = 8.3, 2.5 Hz, 1H), 6.18 (d, J = 8.1 Hz, 2H), 6.03 (d, J = 8.2 Hz, 2H), 5.14 - 5.04 (m, 1H), 4.59 - 4.45 (m, 2H), 4.41 - 4.29 (m, 2H), 4.23 - 4.18 (m, 3H), 4.11 (d, J = 4.9 Hz, 1H), 3.39 - 3.23 (m, 8H), 3.17 - 3.10 (m, 2H), 2.98 - 2.86 (m, 3H), 2.77 (s, 3H), 2.69 - 2.56 (m, 1H), 2.34 - 2.32 (m, 1H), 2.12 - 2.08 (m, 1H), 1.97- 1.95 (m, 4H), 1.73 - 1.69 (m, 2H), 1.59 - 1.55 (m, 9H), 1.47 - 1.34 (m, 4H). | 916.4 9 | General formula ER-T-7 for PROTAC synthesis |
| 70 | ER-P-21 | 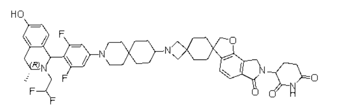 3-(1-(3-(4-((3R)-2-(2,2-difluoroethyl)-6-hydroxy-3-methyl-1,2,3,4-tetrahydroisoquinolin-1-yl)-3,5-difluorophenyl)-3-azaspiro[5.5]undecan-9-yl)-6"-oxo-6",8"-dihydro-2"H,7"H-dispiro[azetidine-3,1'-cyclohexane-4',3"-furo[2,3-e]isoindol]-7"-yl)piperidine-2,6-dione | $^1$H NMR (600 MHz, DMSO-d6) δ 10.98 (s, 1H), 9.69 (s, 1H), 9.16 (s, 1H), 7.39 (d, J = 7.5 Hz, 1H), 7.30 (d, J = 7.6 Hz, 1H), 6.55 - 6.50 (m, 3H), 6.49 (d, J = 2.4 Hz, 1H), 6.48 - 6.45 (m, 1H), 5.73 (t, J = 56.3 Hz, 1H), 5.09 (dd, J = 13.3, 5.1 Hz, 1H), 4.97 (s, 1H), 4.55 - 4.45 (m, 2H), 4.38 (d, J = 17.1 Hz, 1H), 4.22 (d, J = 17.0 Hz, 1H), 4.09 (s, 1H), 3.99 (s, 1H), 3.90 (dd, J = 10.5, 6.7 Hz, 1H), 3.79 (s, 1H), 3.21 - 3.15 (m, 4H), 3.05 - 2.98 (m, 2H), 2.95 - 2.87 (m, 2H), 2.64 - 2.57 (m, 2H), 2.48 (s, 1H), 2.45 - 2.38 (m, 2H), 2.07 (d, J = 13.0 Hz, 1H), 2.02 - 1.93 (m, 2H), 1.81 (d, J = 29.6 Hz, 4H), 1.74 - 1.59 (m, 4H), 1.54 (d, J = 5.7 Hz, 2H), 1.41 (t, J = 5.6 Hz, 2H), 1.34 - 1.26 (m, 2H), 1.13 (d, J = 16.3 Hz, 2H), 0.98 (d, J = 6.5 Hz, 3H). | 884.7 6 | General formula ER-T-2 for PROTAC synthesis |

(continued)

| Example | Comp ound | Structure and Name | $^1$H-NMR | LC-MS | Synthesis Method |
|---|---|---|---|---|---|
| 71 | ER-P-22 | 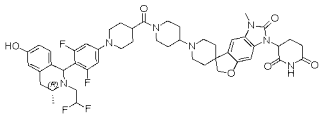 3-(1'-(1-(1-(4-((3R)-2-(2,2-difluoroethyl)-6-hydroxy-3-methyl-1,2,3,4-tetrahydroisoquinolin-1-yl)-3,5-difluorophenyl)piperidine-4-carbonyl)piperidin-4-yl)-1-methyl-2-oxo-1,2-dihydro-3H,6H-spiro[benzofuro[5,6-d]imidazole-7,4'-piperidin]-3-yl)piperidine-2,6-dione | $^1$H NMR (600 MHz, DMSO-d6) δ 11.07 (s, 1H), 9.39 (s, 1H), 9.16 (s, 1H), 6.83 (s, 1H), 6.74 (s, 1H), 6.56 - 6.50 (m, 3H), 6.48 (d, J = 2.4 Hz, 1H), 6.46 (d, J = 5.6 Hz, 1H), 5.73 (t, J = 56.2 Hz, 1H), 5.31 (dd, J = 13.4, 5.4 Hz, 1H), 4.97 (s, 1H), 4.56 (d, J = 12.8 Hz, 1H), 4.48 (s, 2H), 4.19 (d, J = 13.3 Hz, 1H), 3.77 (d, J = 11.9 Hz, 2H), 3.53 (d, J = 12.2 Hz, 3H), 3.32 (s, 3H), 3.08 (s, 3H), 2.99 (dd, J = 16.0, 4.8 Hz, 2H), 2.90 - 2.77 (m, 4H), 2.76 - 2.57 (m, 2H), 2.21 - 2.05 (m, 5H), 2.00 - 1.9o (m, 3H), 1.67 (d, J = 12.8 Hz, 2H), 1.65 - 1.55 (m, 4H), 0.97 (d, J = 6.5 Hz, 3H). | 902.55 | General formula ER-T-3 for PROTAC synthesis |
| 72 | ER-P-23 | 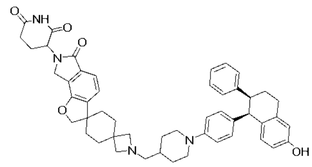 3-(1-((1-(4-((1R,2S)-6-hydroxy-2-phenyl-1,2,3,4-tetrahydronaphthalen-1-yl)phenyl)piperidin-4-yl)methyl)-6"-oxo-6",8"-dihydro-2"H,7"H-di(pyridyl)furan [azetidin e-3,1'-cyclohexane-4',3"-furo[2,3-e]isoindol]-7"-yl)piperidine-2,6-dione | $^1$H NMR (600 MHz, DMSO-d6) δ 10.98 (s, 1H), 9.16 (s, 1H), 7.40 (d, J = 7.6 Hz, 1H), 7.29 (d, J = 7.6 Hz, 1H), 7.15 (dt, J = 12.6, 6.8 Hz, 3H), 6.84 (d, J = 7.2 Hz, 2H), 6.70 (s, 2H), 6.67 - 6.60 (m, 2H), 6.49 (dd, J = 8.3, 2.5 Hz, 1H), 6.29 (d, J = 8.2 Hz, 2H), 5.08 (dd, J = 13.3, 5.1 Hz, 1H), 4.49 (qd, J = 9.1, 4.7 Hz, 2H), 4.37 (d, J = 17.1 Hz, 1H), 4.21 (ddd, J = 22.7, 8.7, 4.7 Hz, 3H), 3.95 - 3.91 (m, 2H), 3.82 - 3.80 (m, 1H), 3.55 (dd, J = 9.5, 4.2 Hz, 2H), 3.34 - 3.29 (m, 1H), 3.18 (h, J = 6.9 Hz, 2H), 3.03 - 2.87 (m, 3H), 2.75 - 2.55 (m, 3H), 2.48 - 2.37 (m, 1H), 2.16 - 2.04 (m, 2H), 2.04 - 1.94 (m, 2H), 1.81 - 1.57 (m, 10H), 1.37 - 1.23 (m, 2H). | 791.5 6 | General formula ER-T-4 for PROTAC synthesis |

(continued)

| Example | Comp ound | Structure and Name | $^1$H-NMR | LC-MS | Synthesis Method |
|---|---|---|---|---|---|
| 73 | ER-P-24 | 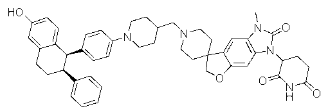 3-(1'-((1-(4-((1R,2S)-6-hydroxy-2-phenyl-1,2,3,4-tetrahydronaphthalen-1-yl)phenyl)piperidin-4-yl)methyl)-1-methyl-2-oxo-1,2-dihydro-3H,6H-spiro [benzofuro[5,6-d]imidazole-7,4'-piperidin]-3-yl)piperidine-2,6-dione | $^1$H NMR (600 MHz, DMSO-d6) δ 11.08 (s, 1H), 9.02 (s, 1H), 7.16 (dd, J = 7.9, 6.0 Hz, 2H), 7.14 - 7.10 (m, 1H), 6.87 - 6.80 (m, 3H), 6.74 (d, J = 2.9 Hz, 1H), 6.69 - 6.60 (m, 4H), 6.49 (dd, J = 8.3, 2.5 Hz, 1H), 6.27 (d, J = 8.1 Hz, 2H), 5.32 (dd, J = 13.0, 5.4 Hz, 1H), 4.47 (d, J = 2.0 Hz, 2H), 4.17 (d, J = 5.0 Hz, 1H), 3.36 (s, 1H), 3.33 (s, 5H), 3.10 - 2.82 (m, 8H), 2.78 - 2.56 (m, 5H), 2.23 (m, 2H), 2.10 (m, 2H), 1.97 (m, 2H), 1.89 (m, 2H), 1.81 (m, 2H), 1.72 (m, 1H), 1.32 (m, 1H). | 766.6 5 | General formula ER-T-4 for PROTAC synthesis |
| 74 | ER-P-25 | 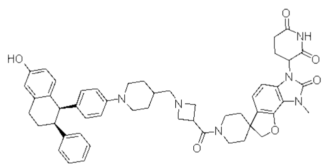 3-(1'-(1-((1-(4-((1R,2S)-6-hydroxy-2-phenyl-1,2,3,4-tetrahydronaphthalen-1-yl)phenyl)piperidin-4-yl)methyl)azetidine-3-carbonyl)-1-methyl-2-oxo-1,2-dihydro-3H,7H-spiro[benzofuro[6,7-d] imidazole-6,4'-piperidin]-3-yl)piperidine-2,6-dione | $^1$H NMR (600 MHz, DMSO-d6) δ 11.09 (s, 1H), 9.13 (s, 1H), 7.18 - 7.08 (m, 4H), 6.88 (dd, J = 8.0, 4.9 Hz, 1H), 6.86 - 6.82 (m, 2H), 6.66 - 6.60 (m, 4H), 6.49 (dd, J = 8.3, 2.5 Hz, 1H), 6.25 (d, J = 8.0 Hz, 2H), 5.35 - 5.30 (m, 1H), 4.62 (s, 2H), 4.43 - 4.36 (m, 2H), 4.34 (d, J = 12.4 Hz, 2H), 4.19 (dq, J = 28.1, 10.7, 9.2 Hz, 4H), 3.90 (t, J = 8.9 Hz, 2H), 3.34 - 3.27 (m, 2H), 3.18 (q, J = 8.9, 7.6 Hz, 2H), 3.11 (s, 2H), 3.01 - 2.81 (m, 6H), 2.70 - 2.59 (m, 3H), 2.09 (m, 2H), 1.98 (m, 1H), 1.81 (m, 1H), 1.72 (m, 5H), 1.27 (m, 3H). | 849.7 6 | General formula ER-T-5 for PROTAC synthesis |

(continued)

| Example | Comp ound | Structure and Name | $^1$H-NMR | LC-MS | Synthesis Method |
|---|---|---|---|---|---|
| 75 | ER-P-26 | 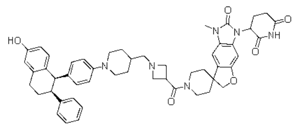 3-(1'-(1-((1-(1-(4-((1R,2S)-6-hydroxy-2-phenyl-1,2,3,4-tetrahydronaphthalen-1-yl)phenyl)piperidin-4-yl)methyl)azetidine-3-carbonyl)-1-methyl-2-oxo-1,2-dihydro-3H,6H-spiro[benzofuro[5,6-d]imidazole-7,4'-piperidin]-3-yl)piperidine-2,6-dione | $^1$H NMR (600 MHz, DMSO-d6) δ 11.07 (s, 1H), 9.13 (s, 1H), 7.18 - 7.10 (m, 3H), 7.06 (d, J = 12.7 Hz, 1H), 6.86 - 6.82 (m, 2H), 6.70 (d, J = 5.9 Hz, 1H), 6.66 - 6.59 (m, 4H), 6.49 (dd, J = 8.3, 2.6 Hz, 1H), 6.25 (d, J = 8.1 Hz, 2H), 5.30 (dt, J = 14.3, 4.0 Hz, 1H), 4.46 (s, 2H), 4.41 (d, J = 7.8 Hz, 1H), 4.39 - 4.31 (m, 2H), 4.19 (dt, J = 24.5, 5.4 Hz, 3H), 3.94 - 3.88 (m, 1H), 3.54 (d, J = 8.3 Hz, 4H), 3.29 (s, 3H), 3.19 (s, 1H), 3.12 (t, J = 7.7 Hz, 2H), 3.01 - 2.96 (m, 1H), 2.96 - 2.90 (m, 1H), 2.86 - 2.82 (m, 1H), 2.76 - 2.66 (m, 2H), 2.59 (m, 1H), 2.14 - 2.04 (m, 2H), 2.04 - 1.93 (m, 2H), 1.85 - 1.76 (m, 2H), 1.70 (m, 5H), 1.26 (m, 3H). | 849.6 6 | General formula ER-T-5 for PROTAC synthesis |
| 76 | ER-P-27 | 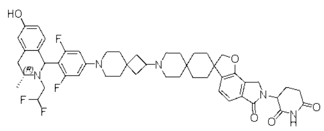 3-(1"-(7-(4-((3R)-2-(2,2-difluoroethyl)-6-hydroxy-3-methyl-1,2,3,4-tetrahydroisoquinolin-1-yl)-3,5-difluorophenyl)-7-azaspiro[3.5]nonan-2-yl)-6-oxo-6,8-dihydro-2H,7H-dispiro[furo[2,3-e]isoindole-3,1'-cyclohexane-4',4"-piperidin]-7-yl)piperidine-2,6-dione | $^1$H NMR (600 MHz, DMSO- *d*6)δ 10.98 (s, 1H), 9.16 (s, 1H), 7.48 (d, J = 7.6 Hz, 1H), 7.29 (d, J = 7.6 Hz, 1H), 6.58 - 6.50 (m, 3H), 6.49 (d, J = 2.3 Hz, 1H), 6.46 (dd, J = 8.4, 2.3 Hz, 1H), 5.74 (t, J = 56.4 Hz, 1H), 5.13 - 5.05 (m, 1H), 4.98 (s, 1H), 4.57 - 4.48 (m, 2H), 4.38 (d, J = 17.3 Hz, 1H), 4.21 (dd, J = 17.1, 2.4 Hz, 1H), 3.40-3.32(m, 1H), 3.29-3.18 (m, 4H), 3.17-3.10 (m, 2H), 3.05-2.94 (m, 2H), 2.94-2.82 (m, 3H), 2.69 - 2.55 (m, 2H), 2.48-2.37 (m, 3H), 2.22-2.14 (m, 2H), 2.08-1.93 (m, 5H), 1.93 - 1.78 (m, 2H), 1.66-1.53 (m, 8H), 1.48-1.34 (m, 4H), 0.98 (d, J = 6.4 Hz, 3H). | 884.5 6 | General formula ER-T-2 for PROTAC synthesis |

(continued)

| Example | Comp ound | Structure and Name | $^1$H-NMR | LC-MS | Synthesis Method |
|---|---|---|---|---|---|
| 77 | ER-P-28 | 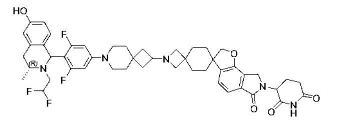 3-(1-(7-(4-((3R)-2-(2,2-difluoroethyl)-6-hydroxy-3-methyl-1,2,3,4-tetrahydroisoquinolin-1-yl)-3,5-difluorophenyl)-7-azaspiro[3.5]nonan-2-yl)-6"-oxo-6",8"-dihydro-2"H,7"H-dispiro[azetidine-3,1'-cyclohexane-4',3"-furo[2,3-e]isoindol]-7"-yl)piperidine-2,6-dione | $^1$H NMR (600 MHz, DMSO- *d*6)δ 10.98 (s, 1H), 9.16 (s, 1H), 7.39 (d, J = 7.6 Hz, 1H), 7.30 (d, J = 7.5 Hz, 1H), 6.55 (d, J = 13.1 Hz, 2H), 6.52 (d, J = 8.5 Hz, 1H), 6.49 (d, J = 2.3 Hz, 1H), 6.46 (dd, J = 8.4, 2.3 Hz, 1H), 5.74 (t, J = 56.3 Hz, 1H), 5.09 (dd, J = 13.3, 5.2 Hz, 1H), 4.97 (s, 1H), 4.54 - 4.44 (m, 2H), 4.38 (d, J = 17.1 Hz, 1H), 4.21 (dd, J = 17.2, 1.9 Hz, 1H), 4.16 - 4.02 (m, 2H), 3.88-3.78 (m, 2H), 3.70 (dd, J = 10.5, 6.2 Hz, 1H), 3.39 - 3.26 (m, 1H), 3.25 - 3.08 (m, 4H), 3.05-2.95 (m, 2H), 2.93 - 2.83 (m, 2H), 2.70 - 2.54 (m, 2H), 2.47-2.37 (m, 1H), 2.22 - 2.11 (m, 2H), 2.09 - 2.02 (m, 1H), 2.02 - 1.93 (m, 2H), 1.90 - 1.76 (m, 3H), 1.74 - 1.55 (m,9H), 0.98 (d, J = 6.4 Hz, 3H). | 856.6 6 | General formula ER-T-2 for PROTAC synthesis |
| 78 | ER-P-29 | 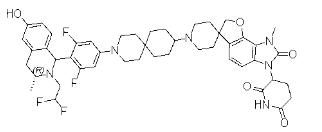 3-(1'-(3-(4-((3R)-2-(2,2-difluoroethyl)-6-hydroxy-3-methyl-1,2,3,4-tetrahydroisoquinolin-1-yl)-3,5-difluorophenyl)-3-azaspiro[5.5]undecan-9-yl)-1-methyl-2-oxo-1,2-dihydro-3H,7H-spiro[benzofuro[6,7-d]imidazole-6,4'-piperidin]-3-yl)piperidine-2,6-dione | $^1$H NMR (600 MHz, DMSO- *d*6)δ 11.12 (s, 1H), 9.20 (s, 1H), 6.78 (d, J = 8.0 Hz, 1H), 6.70 (d, J = 8.0 Hz, 1H), 6.56 - 6.50 (m, 3H), 6.49 (d, J = 2.4 Hz, 1H), 6.48 - 6.45 (m, 1H), 5.73 (t, J = 55.8 Hz, 1H),5.38 - 5.30 (m, 1H), 4.97 (s, 1H), 4.64 (s, 2H), 3.57-3.50 (m, 2H), 3.45 (s, 3H), 3.38 - 3.30 (m, 1H), 3.25 - 3.15 (m, 5H), 3.15 - 3.05 (m, 3H),3.04-2.98 (m, 2H), 2.71 - 2.57 (m, 2H), 2.15 (t, J = 14.1 Hz, 2H), 2.02-1.93 (m, 4H), 1.90 (d, J = 11.2 Hz, 2H), 1.85 (d, J = 12.9 Hz, 2H), 1.68-1.55 (m, 2H), 1.61-1.56 (m, 2H), 1.44-1.37 (m, 2H), 1.22-1.14 (m, 2H), 0.98 (d, J = 6.4 Hz, 3H). | 859.5 6 | General formula ER-T-2 for PROTAC synthesis |

(continued)

| Example | Comp ound | Structure and Name | $^1$H-NMR | LC-MS | Synthesis Method |
|---|---|---|---|---|---|
| 79 | ER-P-30 | 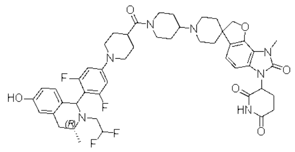 3-(1'-(1-(1-(4-((3R)-2-(2,2-difluoroethyl)-6-hydroxy-3-methyl-1,2,3,4-tetrahydroisoquinolin-1-yl)-3,5-difluorophenyl)piperidin e-4-carbonyl)piperidin-4-yl)-1-methyl-2-oxo-1,2-dihydro-3H,7H-spiro[benzofuro[6,7-d]imidazole-6,4'-piperidin]-3-yl)piperidine-2,6-dione | $^1$H NMR (600 MHz, DMSO- *d*6)δ 11.11 (s, 1H), 9.16 (s, 1H), 6.77 (d, J = 8.0 Hz, 1H), 6.70 (d, J = 8.0 Hz, 1H), 6.54 (d, J = 14.2 Hz, 3H), 6.49 (d, J = 2.4 Hz, 1H), 6.47 (dd, J = 8.0, 2.8 Hz, 1H), 5.74 (t, J = 54.7 Hz, 1H), 5.34 (dd, J = 12.9, 5.1 Hz, 1H), 4.98 (s, 1H), 4.64 (d, J = 6.3 Hz, 2H), 4.60 - 4.54 (m, 1H), 4.20 (d, J = 13.3 Hz, 1H), 3.82-3.74 (m, 2H), 3.57-3.47 (m, 3H), 3.46 (s, 3H), 3.42-3.33 (m, 1H), 3.15-3.05 (m, 3H), 3.05 - 2.96 (m, 2H), 2.94-2.86 (m, 2H), 2.85 - 2.78 (m, 2H), 2.72 - 2.59 (m, 3H), 2.58-2.53 (m,1H), 2.20-2.05 (m, 4H), 2.03 - 1.94 (m, 3H), 1.70-1.65 (m, 2H), 1.65-1.55 (m, 4H), 0.98 (d, J = 6.3 Hz, 3H). | 902.6 5 | General formula ER-T-3 for PROTAC synthesis |
| 80 | ER-P-31 | 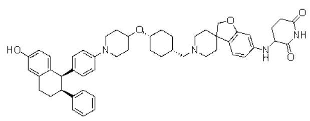 3-((1'-(((1S,4s)-4-((1-(4-((1R,2-S)-6-hydroxy-2-phenyl-1,2,3,4-tetrahydronaphthalen-1-yl)phenyl)piperidin-4-yl)oxy)cyclohexyl)methyl)-2H-spiro[benzofuran-3,4'-piperidin]-6-yl)amino)piperidine-2,6-dione | $^1$H NMR (600 MHz, DMSO-d6) δ 10.78 (s, 1H), 9.13 (s, 1H), 7.14 (m, 3H), 6.88 (d, J = 8.0 Hz, 1H), 6.84 (d, J = 7.3 Hz, 2H), 6.64 (d, J = 8.4 Hz, 1H), 6.60 (d, J = 2.5 Hz, 1H), 6.54 (d, J = 8.3 Hz, 2H), 6.48 (dd, J = 8.4, 2.6 Hz, 1H), 6.20 (d, J = 8.3 Hz, 2H), 6.17 (d, J = 8.0 Hz, 1H), 6.12 (s, 1H), 5.75 (d, J = 7.5 Hz, 1H), 4.25 (m, 2H), 4.13 (d, J = 5.1 Hz, 1H), 3.63 (t, J = 3.9 Hz, 1H), 3.28 (s, 2H), 3.02 - 2.88 (m, 3H), 2.74 (m, 5H), 2.60 - 2.54 (m, 1H), 2.16 - 2.04 (m, 4H), 1.91 (t, J = 11.7 Hz, 2H), 1.86 - 1.63 (m, 8H), 1.59 - 1.36 (m, 9H), 1.25 (m, 3H). | 809.66 | General formula ER-T-8 for PROTAC synthesis |

(continued)

| Example | Comp ound | Structure and Name | $^1$H-NMR | LC-MS | Synthesis Method |
|---|---|---|---|---|---|
| 81 | ER-P-32 | 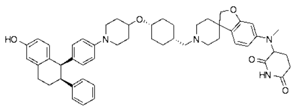 3-((1'-(((1S,4s)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-2H-spiro[benzofuran-3,4'-piperidin]-6-yl)(methyl) amino)piperid ine-2,6-dione | $^1$H NMR 1H NMR (600 MHz, DMSO-d6) δ 10.78 (s, 1H), 9.14 (s, 1H), 7.13 (m, 3H), 6.97 (d, J = 8.2 Hz, 1H), 6.84 (d, J = 7.3 Hz, 2H), 6.64 (d, J = 8.3 Hz, 1H), 6.60 (d, J = 2.6 Hz, 1H), 6.54 (d, J = 8.4 Hz, 2H), 6.48 (dd, J = 8.3, 2.5 Hz, 1H), 6.31 - 6.25 (m, 2H), 6.20 (d, J = 8.3 Hz, 2H), 4.81 (dd, J = 12.6, 5.0 Hz, 1H), 4.27 (s, 2H), 4.13 (d, J = 5.0 Hz, 1H), 3.63 (m, 1H), 3.27 (m, 3H), 3.01 - 2.80 (m, 5H), 2.78 - 2.70 (m, 4H), 2.68 (s, 3H), 2.54 (m, 1H), 2.27 (m, 2H), 2.10 (m, 3H), 1.91 (t, J = 11.8 Hz, 2H), 1.86 - 1.75 (m, 5H), 1.73 - 1.63 (m, 3H), 1.55 (d, J = 12.3 Hz, 3H), 1.44 (m, 6H). | 823.66 | General formula ER-T-8 for PROTAC synthesis |
| 82 | ER-P-33 | 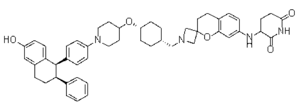 3-((1-(((1S,4s)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)spiro[azetidine-3,2'-chroman]-7'-yl)amino)piperidine-2,6-dione | $^1$H NMR1H NMR (600 MHz, DMSO-d6) δ 10.77 (s, 1H), 9.12 (s, 1H), 7.22 (s, 1H), 7.17 - 7.09 (m, 3H), 6.83 (d, J = 7.3 Hz, 2H), 6.73 (d, J = 8.3 Hz, 1H), 6.68 (s, 1H), 6.64 (d, J = 8.3 Hz, 1H), 6.60 (d, J = 2.5 Hz, 1H), 6.53 (d, J = 8.4 Hz, 2H), 6.48 (dd, J = 8.3, 2.5 Hz, 1H), 6.20 (t, J = 8.2 Hz, 2H), 6.09 (d, J = 2.3 Hz, 1H), 5.64 (d, J = 7.6 Hz, 1H), 5.33 (t, J = 5.0 Hz, 1H), 4.25 (m, 1H), 4.13 (d, J = 5.0 Hz, 1H), 3.60 (s, 1H), 3.30 - 3.28 (m, 4H), 3.01 - 2.90 (m, 4H), 2.78 - 2.69 (m, 3H), 2.63 - 2.55 (m, 3H), 2.39 (m, 1H), 2.31 (d, J = 6.6 Hz, 2H), 2.08 (m, 2H), 2.03 - 1.93 (m, 6H), 1.80 (m, 2H), 1.72 - 1.68 (m, 1H), 1.66 - 1.59 (m, 2H), 1.43 (m, 5H) | 795.96 | General formula ER-T-8 for PROTAC synthesis |

(continued)

| Example | Comp ound | Structure and Name | $^1$H-NMR | LC-MS | Synthesis Method |
|---|---|---|---|---|---|
| 83 | ER-P-34 | 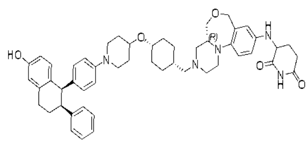 3-(((R)-3-(((1S,4S)-4-((1-(4-((1-R,2S)-6-hydroxy-2-phenyl-1,2,3,4-tetrahydronaphthalen-1-yl)phenyl)piperidin-4-yl)oxy)cyclohexyl)methy l)-1,2,3,4,4a,5-hexahydro-7H-benzo[e]pyrazino[2,1-c][1,4]oxazepin-9-yl)amino)piperidine-2,6-dione | $^1$H NMR (600 MHz, DMSO-d6) δ 10.78 (d, J = 5.9 Hz, 1H), 9.14 (s, 1H), 7.19 - 7.10 (m, 3H), 6.83 (d, J = 7.3 Hz, 2H), 6.76 (d, J = 8.6 Hz, 1H), 6.64 (d, J = 8.3 Hz, 1H), 6.59 (dd, J = 8.7, 2.6 Hz, 2H), 6.56 - 6.51 (m, 3H), 6.48 (dd, J = 8.2, 2.6 Hz, 1H), 6.20 (d, J = 8.4 Hz, 2H), 5.55 (t, J = 8.0 Hz, 1H), 4.71 (d, J = 11.8 Hz, 1H), 4.43 (dd, J = 11.9, 3.4 Hz, 1H), 4.24 (m, 1H), 4.13 (d, J = 5.0 Hz, 1H), 3.66 - 3.57 (m, 4H), 3.10 (m, 2H), 3.02 - 2.88 (m, 4H), 2.78 - 2.64 (m, 6H), 2.60 - 2.54 (m, 2H), 2.23 (m, 2H), 2.15 - 2.07 (m, 4H), 1.82 - 1.77 (m, 2H), 1.72 - 1.62 (m, 3H), 1.60 - 1.53 (m, 1H), 1.49 - 1.37 (m, 6H), 1.29 - 1.22 (m, 3H). | 824.66 | General formula ER-T-8 for PROTAC synthesis |
| 84 | ER-P-35 | 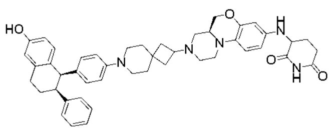 3-(((S)-3-(7-(4-(((1R,2S)-6-hydroxy-2-phenyl-1,2,3,4-tetrahydronaphthalen-1-yl)phenyl)-7-azaspiro[3.5]nonan-2-yl)-1,2,3,4,4a,5-hexahydrobenzo[b]pyrazi no[1,2-d][1,4]oxazin-8-yl)amino)piperidine-2,6-dione | $^1$H NMR (600 MHz, DMSO-$d_6$) δ 10.76 (s, 1H), 9.13 (s, 1H), 7.14 (dt, *J* = 12.6, 7.0 Hz, 3H), 6.84 (d, *J* = 7.3 Hz, 2H), 6.75 (d, *J* = 8.9 Hz, 1H), 6.71 - 6.59 (m, 4H), 6.49 (dd, *J* = 8.2, 2.5 Hz, 1H), 6.26 (d, *J* = 8.5 Hz, 3H), 6.20 (s, 1H), 4.26 (d, *J* = 10.7 Hz, 1H), 4.21 - 4.15 (m, 3H), 3.79 - 3.74 (m, 2H), 3.48 (d, *J* = 11.8 Hz, 3H), 3.33 - 3.28 (m, 1H), 3.26 - 3.18 (m, 1H), 3.09 - 2.85 (m, 8H), 2.84 - 2.68 (m, 2H), 2.68 - 2.55 (m, 2H), 2.19 (d, *J* = 10.1 Hz, 2H), 2.10 - 1.97 (m, 4H), 1.86 - 1.80 (m, 1H), 1.76 - 1.59 (m, 6H). | 738.3 9 | General formula ER-T-2 for PROTAC synthesis |

(continued)

| Example | Comp ound | Structure and Name | $^1$H-NMR | LC-MS | Synthesis Method |
|---|---|---|---|---|---|
| 85 | ER-P-36 | 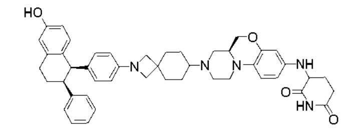 3-(((S)-3-(2-(4-((1R,2S)-6-hydroxy-2-phenyl-1,2,3,4-tetrahydronaphthalen-1-yl)phenyl)-2-azaspiro[3.5]nonan-7-yl)-1,2,3,4,4a,5-hexahydrobenzo[b]pyrazi no[1,2-d][1,4]oxazin-8-yl)amino)piperidine-2,6-dione | $^1$H NMR (600 MHz, DMSO-$d_6$) δ 10.76 (s, 1H), 9.75 (s, 1H), 7.14 (dt, *J* = 15.8, 7.2 Hz, 3H), 6.84 (d, *J* = 7.4 Hz, 2H), 6.75 (d, *J* = 8.8 Hz, 1H), 6.65 - 6.58 (m, 2H), 6.48 (dd, *J* = 8.2, 2.5 Hz, 1H), 6.26 (d, *J* = 8.7 Hz, 1H), 6.22 - 6.15 (m, 3H), 6.05 (d, *J*= 8.0 Hz, 2H), 4.24 (d, *J* = 10.6 Hz, 1H), 4.18 (dd, *J* = 11.2, 4.8 Hz, 1H), 4.12 (d, *J* = 5.0 Hz, 1H), 3.95 - 3.87 (m, 1H), 3.57 (t, *J* = 12.2 Hz, 4H), 3.48 - 3.40 (m, 3H), 3.37 (s, 2H), 3.31 - 3.18 (m, 4H),3.00 - 2.88 (m, 2H), 2.86 - 2.76 (m, 2H), 2.75 - 2.69 (m, 1H), 2.11 - 2.05 (m, 2H), 2.05 - 1.98 (m, 4H), 1.87 - 1.80 (m, 1H), 1.72 - 1.69 (m, 1H), 1.56 - 1.41 (m, 5H). | 738.3 9 | General formula ER-T-2 for PROTAC synthesis |
| 86 | ER-P-37 | 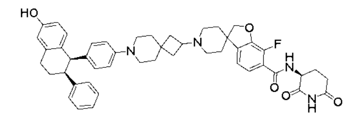 N-((S)-2,6-dioxopiperidin-3-yl)-7-fluoro-1'-(7-(4-((1R,2S)-6-hydroxy-2-phenyl-1,2,3,4-tetrahydronaphthalen-1-yl)phenyl)-7-azaspiro[3.5]nonan-2-yl)-2H-spiro [benzofuran-3,4'-piperidine]-6-carboxamide | $^1$H NMR (600 MHz, DMSO-$d_6$) δ 10.87 (s, 1H), 9.88 (s, 1H), 8.57 (dd, *J* = 8.3, 1.8 Hz, 1H), 7.21 - 7.09 (m, 4H), 7.05 (d, *J* = 7.8 Hz, 1H), 6.88 - 6.81 (m, 2H), 6.77 - 6.58 (m, 4H), 6.49 (dd, *J* = 8.3, 2.5 Hz, 1H), 6.34 - 6.21 (m, 2H), 4.84 - 4.65 (m, 1H), 4.67 (s, 2H), 4.18 (d, *J* = 5.0 Hz, 1H), 3.73 - 3.68 (m, 1H), 3.42 (d, *J* = 12.1 Hz, 2H), 3.35 - 3.27 (m, 2H), 3.08 (s, 2H), 3.01- 2.97 (m, 3H), 2.92 - 2.84 (m, 2H), 2.82 - 2.73 (m, 1H), 2.23 - 2.19 (m, 2H), 2.14 - 1.95 (m, 9H), 1.79 - 1.61 (m, 5H). | 783.3 8 | General formula ER-T-2 for PROTAC synthesis |

(continued)

| Example | Comp ound | Structure and Name | $^{1}$H-NMR | LC-MS | Synthesis Method |
|---|---|---|---|---|---|
| 87 | ER-P-38 | 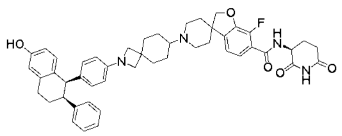 N-((S)-2,6-dioxopiperidin-3-yl)-7-fluoro-1'-(2-(4-((1R,2S)-6-hydroxy-2-phenyl-1,2,3,4-tetrahydronaphthalen-1-yl)phenyl)-2-azaspiro[3.5]nonan-7-yl)-2H-spiro [benzofuran-3,4'-piperidine]-6-carboxamide | $^{1}$H NMR (600 MHz, DMSO-$d_6$) δ 10.87 (s, 1H), 9.38 (s, 1H), 8.57 (dd, *J* = 8.2, 1.9 Hz, 1H), 7.23 - 7.08 (m, 4H), 7.03 (d, *J* = 7.8 Hz, 1H), 6.89 - 6.79 (m, 2H), 6.68 - 6.56 (m, 2H), 6.48 (dd, *J* = 8.3, 2.6 Hz, 1H), 6.19 (d, *J* = 8.2 Hz, 2H), 6.05 (d, *J* = 8.1 Hz, 2H), 4.84 - 4.65 (m, 1H), 4.69 (s, 2H), 4.12 (d, *J* = 5.0 Hz, 1H), 3.47 - 3.42 (m, 3H), 3.37 (s, 3H), 3.31 - 3.19 (m, 3H), 3.11 - 3.05 (m, 2H), 3.01 - 2.87 (m, 2H), 2.82 - 2.76 (m, 1H), 2.20 - 2.05 (m, 4H), 2.04 - 1.94 (m, 7H), 1.72 - 1.69 (m, 1H), 1.61 - 1.41 (m, 4H). | 783.3 8 | General formula ER-T-2 for PROTAC synthesis |
| 88 | ER-P-39 | 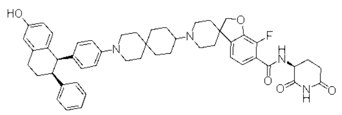 N-((S)-2,6-dioxopiperidin-3-yl)-7-fluoro-1'-(3-(4-((1R,2S)-6-hydroxy-2-phenyl-1,2,3,4-tetrahydronaphthalen-1-yl)phenyl)-3-azaspiro[5.5]undecan-9-yl)-2H-spiro [benzofuran-3,4'-piperidine]-6-carboxamide | $^{1}$H NMR (600 MHz, DMSO-$d_6$) δ 10.87 (s, 1H), 9.38 (s, 1H), 8.56 (dd, *J* = 8.4, 2.0 Hz, 1H), 7.20 (dd, J = 7.7, 5.6 Hz, 1H), 7.18 - 7.10 (m, 3H), 7.04 (d, *J* = 7.8 Hz, 1H), 6.84 (dd, *J = 6.8,* 1.8 Hz, 3H), 6.77 - 6.57 (m, 3H), 6.49 (dd, *J* = 8.3, 2.6 Hz, 1H), 6.30 (s, 2H), 4.84 - 4.65 m, 1H), 4.69 (s, 2H), 4.19 (s, 1H), 3.35 - 3.30 (m, 2H), 3.26 - 3.17 (m, 2H), 3.09 (q, *J* = 11.9, 11.5 Hz, 6H), 3.02 - 2.88 (m, 3H), 2.82 - 2.76 (m, 1H), 2.23 - 2.06 (m, 4H), 2.02 - 1.98 (m, 3H), 1.95 - 1.84 (m, 3H), 1.77 - 1.69 (m, 2H), 1.68 - 1.54 (m, 4H), 1.46 (s, 2H), 1.18 (s, 2H). | 811.4 2 | General formula ER-T-2 for PROTAC synthesis |

(continued)

| Example | Comp ound | Structure and Name | $^1$H-NMR | LC-MS | Synthesis Method |
|---|---|---|---|---|---|
| 89 | ER-P-40 | 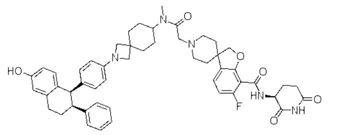 N-((S)-2,6-dioxopiperidin-3-yl)-6-fluoro-1'-(2-((2-(4-((1R,2S)-6-hydroxy-2-phenyl-1,2,3,4-tetrahydronaphthalen-1-yl)phenyl)-2-azaspiro[3.5]nonan-7-yl)(methyl)amino)-2-oxoethyl)-2H-spiro[benzofuran-3,4'-piperidine]-7-carboxamide | $^1$H NMR (600 MHz, DMSO-$d_6$) δ 10.87 (s, 1H), 9.60 (s, 1H), 8.69 (dd, *J* = 8.2, 2.2 Hz, 1H), 7.20 (dd, *J* = 8.3, 5.2 Hz, 1H), 7.14 (dt, *J* = 16.0, 7.1 Hz, 3H), 6.87 - 6.78 (m, 3H), 6.66 - 6.58 (m, 2H), 6.48 (dd, *J* = 8.3, 2.5 Hz, 1H), 6.18 (d, *J* = 8.0 Hz, 2H), 6.04 (dd, *J* = 8.5, 3.0 Hz, 2H), 4.84 - 4.65 (m, 1H), 4.61 (d, *J* = 4.2 Hz, 2H), 4.35 (d, *J* = 4.8 Hz, 1H), 4.28 - 4.17 (m, 2H), 4.12 (d, *J* = 4.9 Hz, 2H), 3.55 - 3.43 (m, 4H), 3.30 - 3.24 (m, 1H), 3.17 - 3.07 (m, 2H), 3.01 - 2.84 (m, 3H), 2.83 - 2.72 (m, 4H), 2.32 - 2.18 (m, 2H), 2.14 - 2.08 (m, 2H), 2.04 - 1.85 (m, 6H), 1.72 - 1.69 (m, 1H), 1.67 - 1.51 (m, 5H), 1.46 (s, 1H). | 854.4 2 | General formula ER-T-7 for PROTAC synthesis |
| 90 | ER-P-41 | 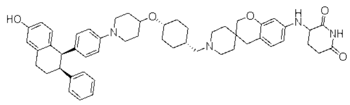 3-((1'-(1S,4s)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)spiro[chromane-3,4'-piperidin]-7-yl)amino)piperidine-2,6-dione | $^1$H NMR (400 MHz, DMSO-$d_6$) δ 10.79 (s, 1H), 9.16 (s, 1H), 7.14 (q, *J* = 7.8, 6.9 Hz, 3H), 6.88 - 6.80 (m, 2H), 6.75 (dd, *J* = 8.4, 4.0 Hz, 2H), 6.69 - 6.57 (m, 3H), 6.48 (dd, *J* = 8.3, 2.6 Hz, 1H), 6.34 - 6.21 (m, 3H), 6.10 (d, *J* = 2.2 Hz, 1H), 4.23 (dd, *J* = 11.4, 4.8 Hz, 1H), 4.17 (d, *J* = 4.8 Hz, 1H), 4.02 (s, 1H), 3.72 (s, 1H), 3.65 (s, 1H), 3.51 (s, 1H), 3.40 -3.27 (m, 4H), 3.16-2.81 (m, 8H), 2.74-2.65 (m, 2H), 2.59 (t, *J* = 4.4 Hz, 1H), 2.40 (s, 1H), 2.14-1.99 (m, 3H), 1.93-1.78 (m, 4H), 1.76-1.61 (m, 6H), 1.58-1.42 (m, 7H), 1.37-1.31 (m, 3H). | 823.4 7 | General formula ER-T-8 for PROTAC synthesis |

(continued)

| Example | Comp ound | Structure and Name | $^1$H-NMR | LC-MS | Synthesis Method |
|---|---|---|---|---|---|
| 91 | ER-P-42 | 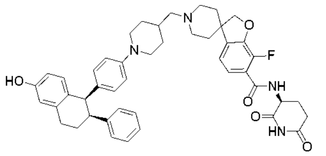 N-((S)-2,6-dioxopiperidin-3-yl)-7-fluoro-1'-((1-(4-((1R,2S)-6-hydroxy-2-phenyl-1,2,3,4-tetrahydronaphthalen-1-yl)phenyl)piperidin-4-yl)methyl)-2H-spiro[benzofuran-3,4'-piperidine]-6-carboxamide | $^1$H NMR (600 MHz, DMSO-$d_6$) δ 10.87 (s, 1H), 9.03 (s, 1H), 8.57 (dd, *J* = 8.3, 1.9 Hz, 1H), 7.22 - 7.11 (m, 5H), 7.03 (d, *J* = 7.8 Hz, 1H), 6.87 - 6.82 (m, 2H), 6.65 (s, 1H), 6.64 (s, 1H), 6.62 (d, *J* = 2.6 Hz, 1H), 6.49 (dd, *J* = 8.3, 2.6 Hz, 1H), 6.26 (d, *J* = 8.1 Hz, 2H), 4.84 - 4.65 (m, 2H), 4.68 (s, 2H), 4.16 (d, *J* = 5.0 Hz, 1H), 3.31 (dt, *J* = 13.2, 3.4 Hz, 2H), 3.05 (s, 3H), 3.02 - 2.90 (m, 3H), 2.79 (m, 2H), 2.57 - 2.54 (m, 1H), 2.20 - 2.14 (m, 2H), 2.13 - 2.04 (m, 3H), 2.02 - 1.92 (m, 5H), 1.80 (m, 2H), 1.72 (m, 1H), 1.31 (m, 2H), 1.24 (m, 1H). | 757.6 6 | General formula ER-T-4 for PROTAC synthesis |
| 92 | ER-P-43 | 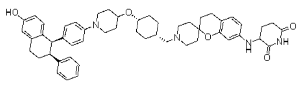 3-((1'-(((1S,4s)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)spiro[chromane-2,4'-piperidin]-7-yl)amino)piperidine-2,6-dione | $^1$H NMR (400 MHz, DMSO-$d_6$) δ 10.79 (d, *J* = 4.9 Hz, 1H), 9.15 (s, 1H), 8.83 (s, 1H), 7.14 (q, *J* = *7.7,* 6.8 Hz, 3H), 6.81 (dd, *J* = 13.4, 7.6 Hz, 3H), 6.74 - 6.57 (m, 4H), 6.48 (dd, *J* = 8.3, 2.6 Hz, 1H), 6.25 (qd, *J* = 6.3, 4.1, 3.7 Hz, 2H), 6.11 (dd, *J* = 12.4, 2.3 Hz, 1H), 4.25 - 4.12 (m, 2H), 3.66 (s, 2H), 3.32 - 3.29 (m,2H), 3.15 - 2.80 (m, 9H), 2.76 - 2.65 (m, 1H), 2.65- 2.55 (m, 2H), 2.15 - 2.05 (m, 1H), 1.96 - 1.79 (m, 8H), 1.76 - 1.66 (m, 4H), 1.60 - 1.27 (m, 6H). | 823.4 9 | General formula ER-T-8 for PROTAC synthesis |

(continued)

| Example | Comp ound | Structure and Name | $^1$H-NMR | LC-MS | Synthesis Method |
|---|---|---|---|---|---|
| 93 | ER-P-44 | 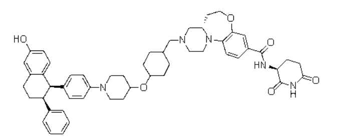 (S)-N-((S)-2,6-dioxopiperidin-3-yl)-3-((4-((1-(4-((1R,2S)-6-hydroxy-2-phenyl-1,2,3,4-tetrahydronaphthalen-1-yl)phenyl)piperidin-4-yl)oxy)cyclohexyl)methy l)-2,3,4,4a,5,6-hexahydro-1H-benzo[b]pyrazino[1,2-d][1,4]oxazepine-9-carboxamide | $^1$H NMR (600 MHz, DMSO-$d_6$) δ 10.86 (s, 1H), 9.25 (d, J= 131.7 Hz, 2H), 8.61 (d, *J* = 8.4 Hz, 1H), 7.55 (dd, *J* = 8.3, 1.8 Hz, 1H), 7.39 (d, *J* = 1.9 Hz, 1H), 7.12 (ddd, *J* = 28.6, 14.2, 7.6 Hz, 4H), 6.83 (d, *J* = 7.0 Hz, 2H), 6.72 - 6.53 (m, 3H), 6.48 (dd, *J* = 8.3, 2.2 Hz, 1H), 6.25 (s, 2H), 4.78 - 4.69 (m, 1H), 4.42 (t, *J* = 8.5 Hz, 1H), 4.20 - 4.09 (m, 2H), 3.49 (d, *J* = 11.4 Hz, 2H), 3.44 (s, 2H), 3.35 (d, *J* = 10.7 Hz, 3H), 3.30 (d, *J = 13.2* Hz, 1H), 3.13 (d, *J* = 10.9 Hz, 2H), 3.08 - 2.88 (m, 6H), 2.85 - 2.74 (m, 2H), 2.54 (d, *J* = 2.8 Hz, 1H), 2.14 - 2.03 (m, 3H), 1.94 (s, 3H), 1.84 (s, 4H), 1.74 (dd, *J* = 35.6, 7.9 Hz, 3H), 1.49 (s, 2H), 1.31 - 1.09 (m, 3H), 1.04 (d, *J* = 10.3 Hz, 2H). | 852.6 6 | General formula ER-T-8 for PROTAC synthesis |
| 94 | ER-P-45 | 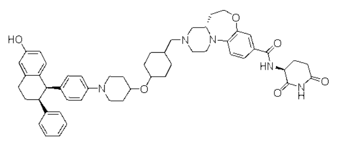 (S)-N-((S)-2,6-dioxopiperidin-3-yl)-3-((4-((1-(4-((1R,2S)-6-hydroxy-2-phenyl-1,2,3,4-tetrahydronaphthalen-1-yl)phenyl)piperidin-4-yl)oxy)cyclohexyl)methy l)-2,3,4,4a,5,6-hexahydro-1H-benzo[b]pyrazino[1,2-d][1,4]oxazepine-9-carboxamide | $^1$H NMR (600 MHz, DMSO-$d_6$) δ 10.85 (s, 1H), 9.33 (s, 1H), 9.14 (s, 1H), 8.61 (d, *J* = 8.3 Hz, 1H), 7.55 (d, *J* = 8.2 Hz, 1H), 7.39 (s, 1H), 7.12 (ddd, *J* = 28.5, 14.1, 7.6 Hz, 4H), 6.83 (d, *J* = 6.9 Hz, 2H), 6.64 (d, *J* = 8.3 Hz, 2H), 6.61 (d, *J* = 1.6 Hz, 1H), 6.48 (dd, *J* = 8.2, 2.2 Hz, 1H), 6.25 (s, 2H), 4.77 - 4.70 (m, 1H), 4.42 (t, *J* = 8.4 Hz, 1H), 4.19 - 4.09 (m, 2H), 3.67 (s, 2H), 3.45 - 3.27 (m, 9H), 3.13 (d, *J* = 11.3 Hz, 2H), 3.03 (d, *J* = 10.8 Hz, 3H), 2.98 - 2.88 (m, 2H), 2.87 - 2.72 (m, 2H), 2.10 (dd, *J* = 12.2, 3.7 Hz, 3H), 1.97 - 1.90 (m, 2H), 1.84 (s, 3H), 1.72 (s, 3H), 1.62-1.40 (m, 6H), 1.40-1.30 (m, 2H). | 852.6 6 | General formula ER-T-8 for PROTAC synthesis |

(continued)

| Example | Comp ound | Structure and Name | $^1$H-NMR | LC-MS | Synthesis Method |
|---|---|---|---|---|---|
| 95 | ER-P-46 | 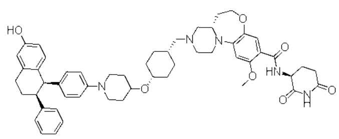 (S)-N-((S)-2,6-dioxopiperidin-3-yl)-3-(((1S,4R)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-10-methoxy-2,3,4,4a,5,6-hexahydro-1H-benzo [b]pyrazino[1,2-d][1,4]oxazepine-9-carboxamide | $^1$H NMR (600 MHz, DMSO-$d_6$) δ 10.90 (s, 1H), 9.33 (s, 1H), 9.14 (s, 1H), 8.52 (d, *J* = 7.1 Hz, 1H), 7.39 (s, 1H), 7.13 (dq, *J* = 14.0, 7.0 Hz, 3H), 6.83 (d, *J* = 7.2 Hz, 2H), 6.69 (s, 1H), 6.67 - 6.53 (m, 3H), 6.48 (dd, *J* = 8.2, 2.1 Hz, 1H), 6.25 (s, 2H), 4.74 - 4.66 (m, 1H), 4.24 - 4.13 (m, 2H), 4.06 - 4.04 (m, 1H), 3.90 (s, 3H), 3.88 (s, 1H), 3.71 (s, 1H), 3.67 (s, 2H), 3.52 (s, 2H), 3.44 (s, 1H), 3.39 - 3.27 (m, 5H), 3.19 - 3.10 (m, 2H), 3.05 - 2.89 (m, 4H), 2.85 - 2.71 (m, 2H), 2.13 - 1.98 (m, 4H), 1.89 - 1.84 (m, 4H), 1.72 (s, 3H), 1.56 - 1.44 (m, 6H), 1.40 - 1.36 (m, 2H). | 882.7 6 | General formula ER-T-8 for PROTAC synthesis |
| 96 | ER-P-47 | 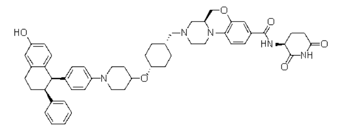 (S)-N-((S)-2,6-dioxopiperidin-3-yl)-3-(((1S,4R)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-1,2,3,4,4a,5-hexahydrobenzo[b]pyrazi no[1,2-d][1,4]oxazine-8-carboxamide | $^1$H NMR (600 MHz, DMSO-$d_6$) δ 10.85 (s, 1H), 9.49 (s, 1H), 9.14 (s, 1H), 8.52 (d, J = 8.4 Hz, 1H), 7.48 - 7.41 (m, 1H), 7.32 (d, J = 1.5 Hz, 1H), 7.17 - 7.01 (m, 4H), 6.83 (d, J = 7.1 Hz, 2H), 6.62 (dd, J = 17.7, 5.1 Hz, 3H), 6.48 (dd, J = 8.2, 2.3 Hz, 1H), 6.24 (s, 2H), 4.75 - 4.68 (m, 1H), 4.33 (d, J = 8.8 Hz, 1H), 4.17 (d, J = 14.7 Hz, 2H), 4.02 - 3.97 (m, 1H), 3.66 (s, 3H), 3.50 - 3.49 (m, 1H), 3.37 - 3.28 (m, 4H), 3.17 - 2.72 (m, 11H), 2.14 - 2.04 (m, 2H), 1.93 - 1.92 (m, 4H), 1.72 (s, 3H), 1.54 -1.44 (m, 6H), 1.35 - 1.33 (m, 2H). | 838.6 6 | General formula ER-T-8 for PROTAC synthesis |

(continued)

| Example | Comp ound | Structure and Name | $^1$H-NMR | LC-MS | Synthesis Method |
|---|---|---|---|---|---|
| 97 | ER-P-48 | 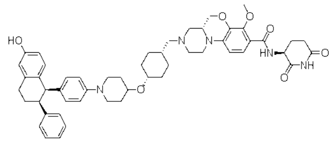 (R)-N-((S)-2,6-dioxopiperidin-3-yl)-3-(((1S,4S)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-7-methoxy-1,2,3,4,4a,5-hexahydrobenzo[b]pyrazi no[1,2-d][1,4]oxazine-8-carboxamide | $^1$H NMR (600 MHz, DMSO-$d_6$) δ 10.91 (s, 1H), 9.56 (s, 1H), 9.13 (s, 1H), 8.53 (d, *J* = 6.0 Hz, 1H), 7.38 (d, *J* = 8.9 Hz, 1H), 7.16 - 7.10 (m, 3H), 6.85 (dd, *J* = 24.4, 8.1 Hz, 3H), 6.62 (dd, *J* = 18.6, 5.2 Hz, 3H), 6.48 (dd, *J* = 8.3, 2.4 Hz, 1H), 6.24 (s, 2H), 4.78 - 4.72 (m, 1H), 4.41 - 4.37 (m, 1H), 4.14 (s, 2H), 4.14 - 4.04 (m, 1H), 3.82 (s, 3H), 3.66 (s, 2H), 3.34 (d, *J* = 10.8 Hz, 3H), 3.30 (d, *J* = 13.9 Hz, 2H), 3.21 - 3.14 (m, 2H), 3.09 (s, 1H), 3.02 - 3.00 (m, 2H), 2.98 - 2.91 (m, 2H), 2.87 - 2.74 (m, 4H), 2.12 - 2.00 (m, 4H), 1.82 (s, 3H), 1.71 (s, 3H), 1.56 - 1.43 (m, 6H), 1.38 - 1.30 (m, 2H). | 868.6 6 | General formula ER-T-8 for PROTAC synthesis |
| 98 | ER-P-49 | 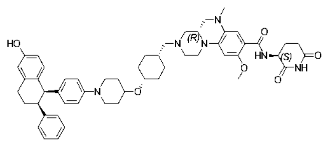 (R)-N-((S)-2,6-dioxopiperidin-3-yl)-3-(((1S,4S)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-9-methoxy-6-methyl-2,3,4,4a,5,6-hexahydro-1H-pyrazino[1,2-a]quinoxaline-8-carboxamide | $^1$H NMR (600 MHz, DMSO-$d_6$) δ 10.88 (s, 1H), 9.43 (s, 1H), 9.14 (s, 1H), 8.51 (d, *J* = 6.9 Hz, 1H), 7.17 - 7.10 (m, 4H), 6.83 (d, *J* = 7.1 Hz, 2H), 6.64 - 6.56 (m, 4H), 6.48 (dd, *J* = 8.3, 2.4 Hz, 1H), 6.24 (s, 2H), 4.70 - 4.65 (m, 1H), 4.27 (d, *J = 13.3* Hz, 1H), 4.15 (s, 1H), 3.87 (s, 3H), 3.67 (s, 2H), 3.36 (s, 2H), 3.30 (d, *J* = 13.0 Hz, 2H), 3.25 - 3.17 (m, 3H), 3.09 (s, 1H), 3.05 - 2.99 (m, 3H), 2.96 (dd, *J* = 11.5, 6.6 Hz, 2H), 2.94 - 2.90 (m, 1H), 2.87 - 2.80 (m, 2H), 2.76 (s, 3H), 2.15 - 2.03 (m, 4H), 1.93 - 1.83 (m, 5H), 1.71 (s, 4H), 1.56 (d, *J* = 10.3 Hz, 1H), 1.50 - 1.43 (m, 4H), 1.38 - 1.32 (m, 2H). | 881.7 6 | General formula ER-T-8 for PROTAC synthesis |

(continued)

| Example | Comp ound | Structure and Name | $^1$H-NMR | LC-MS | Synthesis Method |
|---|---|---|---|---|---|
| 99 | ER-P-50 | 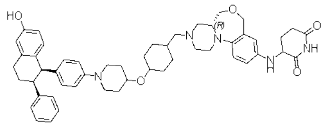 3-(((R)-3-((4-((1-(4-(4-((1R,2-S)-6-hydroxy-2-phenyl-1,2,3,4-tetrahydronaphthalen-1-yl)phenyl)piperidin-4-yl)oxy)cyclohexyl) methy l)-1,2,3,4,4a,5-hexahydro-7H-benzo[e]pyrazino[2,1-c] [1,4]oxazepin-9-yl)amino)piperi-dine-2,6-dione | $^1$H NMR (600 MHz, DMSO-$d_6$) δ 10.79 (d, *J* = 7.1 Hz, 1H), 9.28 (s, 1H), 9.14 (s, 1H), 7.13 (dq, *J* = 14.1, 6.9 Hz, 3H), 6.83 (d, *J* = 7.1 Hz, 2H), 6.77 (d, *J* = 8.6 Hz, 1H), 6.70 - 6.57 (m, 6H), 6.48 (dd, *J* = 8.2, 2.3 Hz, 1H), 6.24 (s, 2H), 4.93 (d, *J* = 11.0 Hz, 1H), 4.42 (dd, *J* = 11.1, 4.7 Hz, 1H), 4.27 - 4.25 (m, 1H), 4.15 (s, 1H), 3.44 (s, 2H), 3.37 - 3.33 (m, 3H), 3.30 - 3.29 (m, 2H), 3.21 - 3.08 (m, 4H), 3.05 (s, 2H), 3.01 - 2.84 (m, 4H), 2.78 - 2.68 (m, 2H), 2.62 - 2.55 (m, 1H), 2.12 - 2.05 (m, 2H), 1.93 (s, 2H), 1.90 - 1.75 (m, 7H), 1.71 - 1.70 (m, 1H), 1.47 (s, 2H), 1.23 - 1.15 (m, 3H), 1.03 - 1.01 (m, 2H). | 824.6 6 | General formula ER-T-8 for PROTAC synthesis |
| 100 | ER-P-51 | 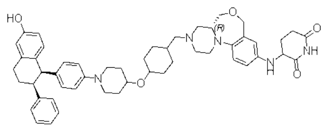 3-(((R)-3-((4-((1-(4-(4-((1R,2-S)-6-hydroxy-2-phenyl-1,2,3,4-tetrahydronaphthalen-1-yl)phenyl)piperidin-4-yl)oxy)cyclohexyl) methy l)-1,2,3,4,4a,5-hexahydro-7H-benzo[e]pyrazino[2,1-c] [1,4]oxazepin-9-yl)amino)piperi-dine-2,6-dione | $^1$H NMR (600 MHz, DMSO-$d_6$) δ 10.79 (d, *J* = 7.1 Hz, 1H), 9.27 (s, 1H), 9.14 (s, 1H), 7.13 (dq, *J* = 14.1, 6.9 Hz, 3H), 6.83 (d, *J* = 7.1 Hz, 2H), 6.77 (d, *J* = 8.6 Hz, 1H), 6.68 - 6.60 (m, 6H), 6.48 (dd, *J* = 8.3, 2.3 Hz, 1H), 6.25 (s, 2H), 4.93 (d, *J* = 10.9 Hz, 1H), 4.42 (dd, *J* = 11.1, 4.8 Hz, 1H), 4.29 - 4.23 (m, 1H), 4.16 (s, 1H), 3.66 (s, 1H), 3.43 (s, 2H), 3.36 (s, 3H), 3.30 (d, *J* = 9.6 Hz, 4H), 3.17 (dd, *J* = 21.2, 11.6 Hz, 2H), 3.13 - 3.05 (m, 3H), 3.01 - 2.82 (m, 5H), 2.77 - 2.69 (m, 1H), 2.61 - 2.57 (m, 1H), 2.13 - 2.05 (m, 2H), 1.92 - 1.80 (m, 4H), 1.71 (s, 3H), 1.55 - 1.46 (m, 6H), 1.37 - 1.33 (m, 2H). | 824.6 6 | General formula ER-T-8 for PROTAC synthesis |

(continued)

| Example | Comp ound | Structure and Name | $^1$H-NMR | LC-MS | Synthesis Method |
|---|---|---|---|---|---|
| 101 | ER-P-52 | 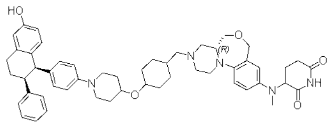 3-(((R)-3-((4-((1-(4-((1R,2S)-6-hydroxy-2-phenyl-1,2,3,4-tetrahydronaphthalen-1-yl)phenyl)piperidin-4-yl)oxy)cyclohexyl) methy l)-1,2,3,4,4a,5-hexahydro-7H-benzo[e]pyrazino[2,1-c][1,4]oxazepin-9-yl)(methyl)amino)piperid ine-2,6-dione | $^1$H NMR (600 MHz, DMSO-$d_6$) 10.79 (s, 1H), 9.25 (s, 1H), 9.14 (s, 1H), 7.16 - 7.11 (m, 3H), 6.87 - 6.76 (m, 5H), 6.71 - 6.55 (m, 4H), 6.48 (d, *J* = 8.3 Hz, 1H), 6.26 (s, 2H), 4.94 (d, *J* = 11.1 Hz, 1H), 4.82 (dd, *J* = 12.6, 4.8 Hz, 1H), 4.49 (d, *J* = 11.2 Hz, 1H), 4.16 (s, 1H), 3.45 (s, 2H), 3.36 (s, 3H), 3.33 - 3.29 (m, 4H), 3.18 - 3.16 (m, 3H), 3.06 (s, 2H), 2.97 - 2.89 (m, 4H), 2.82 - 2.81 (m, 2H), 2.71 (d, *J* = 2.2 Hz, 3H), 2.59 - 2.52 (m, 1H), 2.31 - 2.29 (m, 1H), 2.10 - 2.07 (m, 1H), 1.94 (s, 2H), 1.90 - 1.74 (m, 6H), 1.71 - 1.70 (m, 1H), 1.48 (s, 2H), 1.30 - 1.12 (m, 3H), 1.07 - 0.97 (m, 2H). | 838.7 6 | General formula ER-T-8 for PROTAC synthesis |
| 102 | ER-P-53 | 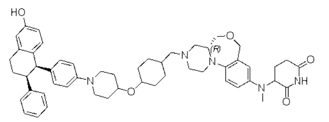 3-(((R)-3-((4-((1-(4-((1R,2S)-6-hydroxy-2-phenyl-1,2,3,4-tetrahydronaphthalen-1-yl)phenyl)piperidin-4-yl)oxy)cyclohexyl) methy l)-1,2,3,4,4a,5-hexahydro-7H-benzo[e]pyrazino[2,1-c][1,4]oxazepin-9-yl)(methyl)amino)piperid ine-2,6-dione | $^1$H NMR (600 MHz, DMSO-$d_6$) 10.79 (s, 1H), 9.24 (s, 1H), 9.14 (s, 1H), 7.16 - 7.10 (m, 3H), 6.86 - 6.77 (m, 5H), 6.62 (dd, *J* = 17.7, 5.2 Hz, 4H), 6.48 (dd, *J* = 8.2, 2.4 Hz, 1H), 6.26 (s, 2H), 4.94 (d, *J* = 11.1 Hz, 1H), 4.82 (dd, *J* = 12.7, 4.9 Hz, 1H), 4.49 (dd, *J* = 11.1, 2.6 Hz, 1H), 4.16 (s, 1H), 3.67 (s, 1H), 3.58 (s, 2H), 3.36 (s, 3H), 3.32 (d, *J* = 7.0 Hz, 3H), 3.30 (s, 1H), 3.21 - 3.05 (m, 5H), 3.01 - 2.87 (m, 4H), 2.85 - 2.79 (m, 2H), 2.71 (d, *J* = 2.4 Hz, 3H), 2.58 - 2.51 (m, 1H), 2.33 - 2.26 (m, 1H), 2.12 -2.05 (m, 1H), 1.92 - 1.80 (m, 4H), 1.71 (s, 3H), 1.53 - 1.46 (m, 6H), 1.37 - 1.33 (m, 2H). | 838.6 6 | General formula ER-T-8 for PROTAC synthesis |

(continued)

| Example | Comp ound | Structure and Name | $^1$H-NMR | LC-MS | Synthesis Method |
|---|---|---|---|---|---|
| 103 | ER-P-54 | 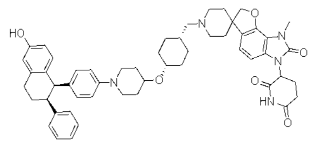 3-(1'-(((1S,4S)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-1-methyl-2-oxo-1,2-dihydro-3H,7H-spiro[benzofuro[6,7-d] imidazole-6,4'-piperidin]-3-yl)piperidine-2,6-dione | $^1$H NMR (600 MHz, DMSO-$d_6$) δ 11.11 (s, 1H), 9.14 (s, 1H), 8.84 (s, 1H), 7.13 (dq, $J$ = 14.1, 7.0 Hz, 3H), 6.83 (d, $J$ = 7.2 Hz, 2H), 6.75 (d, $J$ = 8.0 Hz, 1H), 6.68 (d, $J$ = 8.0 Hz, 1H), 6.65 - 6.57 (m, 4H), 6.48 (dd, $J$ = 8.2, 2.1 Hz, 1H), 6.24 (s, 2H), 5.34 (dd, $J$ = 12.5, 5.1 Hz, 1H), 4.61 (s, 2H), 4.15 (s, 1H), 3.66 (s, 2H), 3.44 (s, 3H), 3.35 (s, 4H), 3.30 (d, $J$ = 10.0 Hz, 1H), 3.08 - 2.98 (m, 4H), 2.98 - 2.85 (m, 4H), 2.81 (s, 1H), 2.70 - 2.58 (m, 2H), 2.22 - 2.03 (m, 3H), 2.02 - 1.94 (m, 1H), 1.92 - 1.79 (m, 5H), 1.71 (s, 3H), 1.48 (dd, $J$ = 33.7, 12.5 Hz, 6H), 1.34 (d, $J$ = 11.4 Hz, 2H). | 864.6 6 | General formula ER-T-8 for PROTAC synthesis |
| 104 | ER-P-55 | 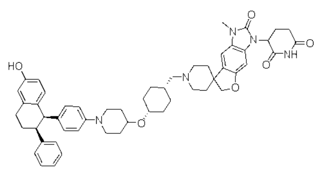 3-(1'-(((1S,4S)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-1-methyl-2-oxo-1,2-dihydro-3H,6H-spiro[benzofuro[5,6-d] imidazole-7,4'-piperidin]-3-yl)piperidine-2,6-dione | $^1$H NMR (600 MHz, DMSO-$d_6$) δ 11.08 (s, 1H), 9.14 (s, 1H), 8.85 (s, 1H), 7.16 - 7.10 (m, 3H), 6.88 - 6.78 (m, 3H), 6.74 (s, 1H), 6.68 - 6.56 (m, 3H), 6.49 - 6.47 (m, 1H), 6.25 (s, 2H), 5.33 - 5.30 (m, 1H), 4.48 - 4.43 (m, 2H), 4.16 (s, 1H), 3.67 (s, 2H), 3.53 (s, 2H), 3.35 (s, 3H), 3.32 (s, 3H), 3.07 - 2.77 (m, 9H), 2.77 - 2.68 (m, 1H), 2.60 (d, $J$ = 14.4 Hz, 1H), 2.27 - 2.04 (m, 3H), 1.99 - 1.92 (m, 1H), 1.87 (d, $J$ = 13.3 Hz, 5H), 1.75 - 1.71 (m, 3H), 1.61 - 1.43 (m, 6H), 1..40 - 1.34 (m, 2H). | 864.6 6 | General formula ER-T-8 for PROTAC synthesis |

(continued)

| Example | Comp ound | Structure and Name | $^1$H-NMR | LC-MS | Synthesis Method |
|---|---|---|---|---|---|
| 105 | ER-P-56 | 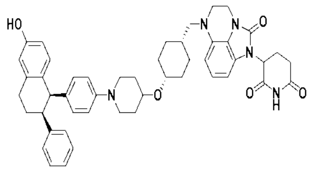 3-(6-(((1S,4s)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-2-oxo-5,6-dihydro-4H-imidazo[1,5,4-de]quinoxalin-1(2H)-yl)piperidine-2,6-dione | $^1$H NMR (600 MHz, DMSO-$d_6$) δ 11.07 (s, 1H), 9.15 (s, 1H), 7.13 (dq, *J* = 14.1, 7.0 Hz, 4H), 6.86 - 6.77 (m, 4H), 6.67 - 6.59 (m, 3H), 6.51 - 6.47 (m, 1H), 6.42 (d, *J* = 7.9 Hz, 1H), 6.37 (d, *J* = 8.2 Hz, 1H), 6.32 (s, 1H), 5.26 (dd, *J* = 12.9, 5.3 Hz, 1H), 4.20 (s, 1H), 3.86 - 3.84 (m, 2H), 3.65 (s, 1H), 3.39 (s, 2H), 3.32 (d, *J* = 11.3 Hz, 2H), 3.09 (d, *J* = 7.0 Hz, 3H), 3.03 - 2.86 (m, 5H), 2.70 - 2.57 (m, 3H), 2.09 - 2.06 (m, 1H), 2.02 - 1.97 (m, 1H), 1.90 (s, 2H), 1.73 (d, *J* = 9.0 Hz, 4H), 1.57 (s, 1H), 1.49 (d, *J* = 8.8 Hz, 2H), 1.43 - 1.31 (m, 5H). | 780.6 6 | General formula ER-T-8 for PROTAC synthesis |
| 106 | ER-P-57 | 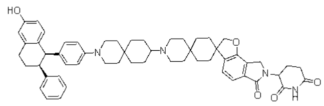 3-(3-(4-((1R,2S)-6-hydroxy-2-phenyl-1,2,3,4-tetrahydronaphthalen-1-yl)phenyl)-3-azaspiro[5.5]undecan-9-yl)-6-oxo-6,8-dihydro-2H,7H-dipyridyl [2,3-e] isoindole-3,1-cyclohexane-4,4-piperidin]-7-yl)piperidine-2,6-dione | $^1$H NMR (600 MHz, DMSO-$d_6$) δ 10.98 (s, 1H), 9.08 (s, 1H), 7.48 (d, J = 7.6 Hz, 1H), 7.29 (d, J = 7.6 Hz, 1H), 7.18 - 7.11 (m, 3H), 6.82 (t, J = 11.7 Hz, 4H), 6.63 (dd, J = 11.5, 5.1 Hz, 2H), 6.49 (dd, J = 8.3, 2.2 Hz, 1H), 6.32 (d, J = 7.2 Hz, 2H), 5.12 - 5.03 (m, 1H), 4.57 - 4.47 (m, 2H), 4.42 - 4.33 (m, 1H), 4.22 (dd, J = 17.1, 5.1 Hz, 2H), 3.17 - 3.07 (m, 8H), 3.02 - 2.84 (m, 4H), 2.60 (d, J = 16.9 Hz, 1H), 2.46 - 2.36 (m, 2H), 2.11 - 2.01 (m, 2H), 1.99 - 1.94 (m, 1H), 1.93 - 1.79 (m, 5H), 1.75 - 1.72 (m, 2H), 1.69 - 1.55 (m, 8H), 1.51 - 1.37 (m, 5H), 1.27 - 1.10 (m, 4H). | 873.7 0 | General formula ER-T-2 for PROTAC synthesis |

(continued)

| Example | Comp ound | Structure and Name | $^1$H-NMR | LC-MS | Synthesis Method |
|---|---|---|---|---|---|
| 107 | ER-P-58 | 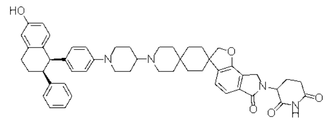 3-(1-(1-(4-((1R,2S)-6-hydroxy-2-phenyl-1,2,3,4-tetrahydro-naphthalen-1-yl)phenyl)piperi-din-4-yl)-6-oxo-6,8-dihydro-2H,7H-dipyridole-3,1'-cyclohexane-4,4-piperidin]-7-yl)piperi-dine-2,6-dione | $^1$H NMR (600 MHz, DMSO-$d_6$) δ 10.98 (s, 1H), 9.15 (s, 1H), 7.48 (d, *J* = 7.6 Hz, 1H), 7.29 (d, J = 7.5 Hz, 1H), 7.15 (dt, J = 13.7, 6.9 Hz, 3H), 6.84 (d, J = 7.2 Hz, 2H), 6.62 (dd, J = 20.1, 9.2 Hz, 4H), 6.49 (dd, J = 8.2, 1.9 Hz, 1H), 6.24 (d, J = 8.4 Hz, 2H), 5.09 (dd, J = 9.5, 3.7 Hz, 1H), 4.58 - 4.46 (m, 2H), 4.38 (d, J = 17.1 Hz, 1H), 4.22 (dd, J = 17.0, 4.4 Hz, 1H), 4.16 (d, J = 4.7 Hz, 1H), 3.44 - 3.24 (m, 5H), 3.06 - 3.00 (m, 2H), 3.01 - 2.85 (m, 3H), 2.61 - 2.55 (m, 3H), 2.43 - 2.39 (m, 2H), 2.11 - 2.04 (m, 4H), 2.01 - 1.95 (m, 1H), 1.89 - 1.78 (m, 2H), 1.76 - 1.63 (m, 4H), 1.63 - 1.52 (m, 4H), 1.47 - 1.40 (m, 2H), 1.36 - 1.34 (m, 1H), 1.26 - 1.14 (m, 1H). | 805.6 5 | General formula ER-T-2 for PROTAC synthesis |
| 108 | ER-P-59 | 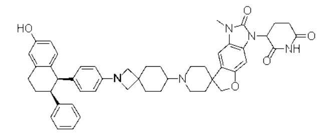 3-(1R,2S)-6-hydroxy-2-phenyl-1,2,3,4-tetrahydronaphthalen-1-yl)phenyl)-2-azaspiro[3.5] nonan-7-yl)-1-methyl-2-oxo-1,2-dihydro-3H,6H-spiro[benzofuro [5,6-d]imidazole-7,4'-piperidin]-3-yl)piperidine-2,6-dione | $^1$H NMR (600 MHz, DMSO-$d_6$) δ 11.08 (s, 1H), 9.36 (s, 1H), 7.13 (dt, J = 22.8, 7.2 Hz, 3H), 6.84 (d, J = 8.4 Hz, 3H), 6.74 (s, 1H), 6.66 - 6.59 (m, 2H), 6.48 (d, J = 8.4 Hz, 1H), 6.19 (d, J = 8.1 Hz, 2H), 6.05 (d, J = 8.1 Hz, 2H), 5.32 (dd, J = 12.7, 5.1 Hz, 1H), 4.52 - 4.43 (m, 2H), 4.12 (d, J = 4.5 Hz, 1H), 3.55 - 3.40 (m, 5H), 3.37 (d, J = 6.0 Hz, 2H), 3.34 - 3.20 (m, 5H), 3.10 - 3.05 (m, 2H), 2.99 - 2.80 (m, 3H), 2.76 - 2.67 (m, 1H), 2.62 - 2.60 (m, 1H), 2.21 - 2.19 (m, 2H), 2.13 - 2.07 (m, 1H), 2.05 - 1.96 (m, 4H), 1.93 - 1.90 (m, 2H), 1.72 - 1.70 (m, 1H), 1.58 - 1.42 (m, 4H). | 792.6 0 | General formula ER-T-2 for PROTAC synthesis |

(continued)

| Example | Comp ound | Structure and Name | $^1$H-NMR | LC-MS | Synthesis Method |
|---|---|---|---|---|---|
| 109 | ER-P-60 | 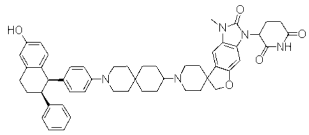 3-(3-(4-((1R,2S)-6-hydroxy-2-phenyl-1,2,3,4-tetrahydronaphthalen-1-yl)phenyl)-3-azaspiro[5.5]undecan-9-yl)-1-methyl-2-oxo-1,2-dihydro-3H,6H-spiro[benzofuro[5,6-d]imidazole-7,4'-piperidin]-3-yl)piperidine-2,6-dione | $^1$H NMR (600 MHz, DMSO-$d_6$) δ 11.08 (s, 1H), 9.51 (s, 1H), 7.14 (dq, J = 14.0, 6.8 Hz, 3H), 6.95 - 6.68 (m, 6H), 6.67 - 6.59 (m, 2H), 6.50 (dd, J = 8.2, 1.8 Hz, 1H), 6.33 (d, J = 6.5 Hz, 2H), 5.32 (dd, J = 12.8, 5.2 Hz, 1H), 4.56 - 4.44 (m, 2H), 4.22 - 4.18 (m, 1H), 3.58 - 3.53 (m, 5H), 3.20 (d, J = 12.7 Hz, 1H), 3.13 - 3.05 (dd, J = 29.1, 17.1 Hz, 6H), 3.00 - 2.82 (m, 3H), 2.72 - 2.68 (m, 1H), 2.62 - 2.60 (m, 1H), 2.22 - 2.20 (m, 2H), 2.14 - 2.01 (m, 2H), 2.00 - 1.78 (m, 7H), 1.75 - 1.56 (m, 5H), 1.50 - 1.48 (m, 2H), 1.26 - 1.17 (m, 2H). | 820.6 5 | General formula ER-T-2 for PROTAC synthesis |
| 110 | ER-P-61 | 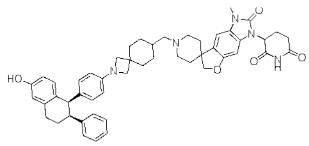 3-(2-(4-(1R,2S)-6-hydroxy-2-phenyl-1,2,3,4-tetrahydronaphthalen-1-yl)phenyl)-2-azaspiro[3.5]nonan-7-yl)methyl)-1-methyl-2-oxo-1,2-dihydro-3H,6H-spiro[benzofuro[5,6-d]imidazole-7,4'-piperidin]-3-yl)piperidine-2,6-dione | $^1$H NMR (600 MHz, DMSO-$d_6$) δ 11.07 (s, 1H), 9.11 (s, 1H), 7.18 - 7.11 (m, 3H), 6.83 (d, J = 7.8 Hz, 3H), 6.74 (s, 1H), 6.65 - 6.59 (m, 2H), 6.48 (dd, J = 8.3, 2.1 Hz, 1H), 6.19 (d, J = 8.3 Hz, 2H), 6.06 (t, J = 8.6 Hz, 2H), 5.32 (dd, J = 12.7, 5.0 Hz, 1H), 4.52 - 4.42 (m, 2H), 4.12 (d, J = 4.7 Hz, 1H), 3.54 (d, J = 11.1 Hz, 3H), 3.43 (t, J = 8.4 Hz, 2H), 3.37 (t, J = 6.7 Hz, 3H), 3.25 (dd, J = 25.9, 12.9 Hz, 2H), 3.16 - 3.02 (m, 2H), 3.00 - 2.81 (m, 5H), 2.75 - 2.68 (m, 1H), 2.61 (d, J = 16.3 Hz, 1H), 2.30 - 2.05 (m, 3H), 2.01 - 1.93 (m, 1H), 1.89 - 1.87 (m, 4H), 1.71 (d, J = 10.9 Hz, 3H), 1.49 (t, J = 11.4 Hz, 2H), 1.24 - 1.18 (m, 1H), 1.12 - 0.98 (m, 2H). | 806.6 0 | General formula ER-T-4 for PROTAC synthesis |

(continued)

| Example | Comp ound | Structure and Name | $^1$H-NMR | LC-MS | Synthesis Method |
|---|---|---|---|---|---|
| 111 | ER-P-62 | 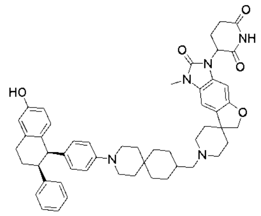 3-(3-(4-((1R,2S)-6-hydroxy-2-phenyl-1,2,3,4-tetrahydronaphthalen-1-yl)phenyl)-3-azaspiro[5.5]undecan-9-yl)methyl)-1-methyl-2-oxo-1,2-dihydro-3H,6H-spiro[benzofuro[5,6-d]imidazole-7,4'-piperidin]-3-yl)piperidine-2,6-dione | $^1$H NMR (600 MHz, DMSO-$d_6$) $\delta$ 11.07 (s, 1H), 9.35 (s, 1H), 7.19 - 7.12 (m, 3H), 7.03 (s, 2H), 6.84 (d, J = 5.2 Hz, 3H), 6.74 (s, 1H), 6.65 (d, J = 8.6 Hz, 2H), 6.50 (dd, J = 8.2, 2.1 Hz, 1H), 6.41 (d, J = 7.5 Hz, 2H), 5.32 (dd, J = 12.8, 5.1 Hz, 1H), 4.52 - 4.43 (m, 2H), 4.26 (d, J = 4.5 Hz, 1H), 3.54 (d, J = 11.2 Hz, 3H), 3.42 - 3.33 (m, 3H), 3.23 (s, 4H), 3.11 - 2.98 (m, 5H), 2.97 - 2.90 (m, 1H), 2.89 - 2.81 (m, 1H), 2.75 - 2.68 (m, 1H), 2.61 (d, J = 16.2 Hz, 1H), 2.28 - 2.26 (m, 2H), 2.12 - 2.04 (ddd, J = 25.1, 12.8, 6.2 Hz, 1H), 2.01 - 1.93 (m, 1H), 1.89 (t, J = 14.7 Hz, 2H), 1.83 - 1.65 (m, 6H), 1.63 (s, 2H), 1.56 (s, 2H), 1.19 (d, J = 6.8 Hz, 4H). | 834.60 | General formula ER-T-4 for PROTAC synthesis |
| 112 | ER-P-63 | 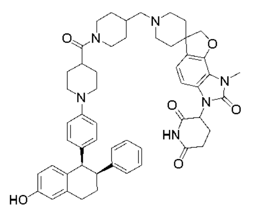 3-(1'-((1-(1-(4-((1R,2S)-6-hydroxy-2-phenyl-1,2,3,4-tetrahydronaphthalen-1-yl)phenyl)piperidine-4-carbonyl)piperidin-4-yl) methyl)-1-methyl-2-oxo-1,2-dihydro-3H,7H-spiro[benzofuro[6,7-d] imidazole-6,4'-piperidin]-3-yl)piperidine-2,6-dione | $^1$H NMR (600 MHz, DMSO-$d_6$) $\delta$ 11.11 (s, 1H), 7.18 - 7.11 (m, 3H), 6.83 (d, J = 7.1 Hz, 2H), 6.78 (d, J = 7.9 Hz, 1H), 6.68 (dd, J = 32.5, 8.0 Hz, 4H), 6.61 (s, 1H), 6.49 (d, J = 6.6 Hz, 1H), 6.27 (d, J = 7.0 Hz, 2H), 5.33 (d, J = 7.1 Hz, 1H), 4.63 (s, 2H), 4.45 - 4.36 (m, 1H), 4.17 (d, J = 3.5 Hz, 1H), 3.04 (d, J = 19.4 Hz, 6H), 2.98 (s, 1H), 2.91 (d, J = 16.3 Hz, 3H), 2.75 (d, J = 24.0 Hz, 3H), 2.71 - 2.65 (m, 1H), 2.64 - 2.56 (m, 2H), 2.28 (s, 2H), 2.10 (dd, J = 12.9, 6.0 Hz, 3H), 1.98 (d, J = 4.8 Hz, 2H), 1.91 - 1.78 (m, 4H), 1.66 (s, 5H), 1.58 (s, 3H), 1.41 (s, 2H), 1.14 (d, J = 10.2 Hz, 1H), 1.04 (d, J = 8.7 Hz, 1H). | 877.60 | General formula ER-T-9 for PROTAC synthesis |

(continued)

| Example | Comp ound | Structure and Name | $^1$H-NMR | LC-MS | Synthesis Method |
|---|---|---|---|---|---|
| 113 | ER-P-64 | 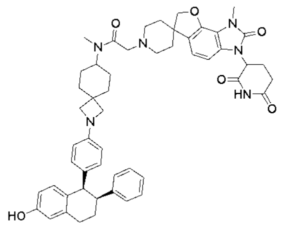 2-(3-(2,6-dioxopiperidin-3-yl)-1-methyl-2-oxo-2,3-dihydro-1H,7H-spiro[benzofuro[6,7-d]imidazole-6,4'-piperidin]-1'-yl)-N-(2-(4-((1R,2S)-6-hydroxy-2-phenyl-1,2,3,4-tetrahydronaphthalen-1-yl)phenyl)-2-azaspiro[3.5]nonan-7-yl)-N-methylacetamide | $^1$H NMR (600 MHz, DMSO-$d_6$) δ 11.12 (s, 1H), 7.18 - 7.11 (m, 3H), 6.82 (dd, J = 21.2, 7.6 Hz, 3H), 6.70 (d, J = 7.0 Hz, 1H), 6.65 - 6.58 (m, 2H), 6.48 (d, J = 8.1 Hz, 1H), 6.18 (d, J = 7.6 Hz, 2H), 6.07 - 5.97 (m, 2H), 5.44 - 5.22 (m, 1H), 4.62 (d, J = 3.7 Hz, 2H), 4.37 (s, 1H), 4.24 (d, J = 33.9 Hz, 2H), 4.12 (d, J = 4.2 Hz, 1H), 3.36 (d, J = 15.1 Hz, 5H), 3.27 (d, J = 10.5 Hz, 3H), 3.22 - 3.06 (m, 3H), 3.02 - 2.84 (m, 4H), 2.72 - 2.58 (m, 2H), 2.26 (s, 2H), 2.19 - 2.03 (m, 2H), 2.01 - 1.85 (m, 5H), 1.74 - 1.42 (m, 8H), 1.31 - 1.19 (m, 1H). | 863.76 | General formula ER-T-7 for PROTAC synthesis |
| 114 | ER-P-65 | 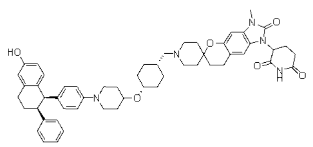 3-(1'-(((1S,4S)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-3-methyl-2-oxo-2,3,7,8-tetrahydro-1H-spiro[benzopyrano [6,7-d]imidazole-6,4'-piperidin]-1-yl)piperidine-2,6-dione | $^1$H NMR (400 MHz, DMSO-$d_6$) δ 11.07 (s, 1H), 9.14 (s, 1H), 7.19 - 7.09 (m, 3H), 6.85 (dd, J = 17.4, 7.0 Hz, 3H), 6.77 - 6.56 (m, 5H), 6.48 (dd, J = 8.3, 2.3 Hz, 1H), 6.27 (d, J = 6.4 Hz, 2H), 5.30 (dd, J = 12.8, 5.2 Hz, 1H), 4.17 (d, J = 4.0 Hz, 1H), 3.57 - 3.52 (m, 2H), 3.43 - 3.27 (m, 6H), 3.27 (s, 3H), 3.13 (d, J = 9.8 Hz, 2H), 3.04 - 2.85 (m, 6H), 2.83 - 2.68 (m, 4H), 2.60 (d, J = 16.5 Hz, 1H), 2.01 - 1.89 (m, 7H), 1.86 - 1.67 (m, 8H), 1.49 (s, 2H), 1.33 - 1.09 (m, 4H). | 878.6 5 | General formula ER-T-8 for PROTAC synthesis |

(continued)

| Example | Comp ound | Structure and Name | $^1$H-NMR | LC-MS | Synthesis Method |
|---|---|---|---|---|---|
| 115 | ER-P-66 | 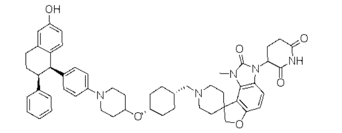 3-(1'-(((1S,4s)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-1-methyl-2-oxo-1,2-dihydro-3H,7H-spiro[benzofuro[4,5-d] imidazole-8,4'-piperidin]-3-yl)piperidine-2,6-dione | $^1$H NMR (400 MHz, DMSO-$d_6$) δ 11.11 (s, 1H), 9.33 (s, 1H), 7.18 - 7.11 (m, 3H), 6.96 (d, J = 8.4 Hz, 1H), 6.83 (d, J = 6.5 Hz, 2H), 6.75 - 6.59 (m, 4H), 6.57 (d, J = 8.4 Hz, 1H), 6.49 (dd, J = 8.3, 2.3 Hz, 1H), 6.28 (d, J = 7.6 Hz, 2H), 5.36 (dd, J = 12.4, 5.2 Hz, 1H), 4.55 - 4.45 (m, 2H), 4.17 (d, J = 4.4 Hz, 2H), 3.68 (s, 1H), 3.59 (s, 3H), 3.53 (d, J = 3.3 Hz, 3H), 3.43 - 3.24 (m, 4H), 3.04 (s, 4H), 2.99 - 2.80 (m, 5H), 2.73 - 2.59 (m, 2H), 2.11 - 1.95 (m, 4H), 1.91 - 1.86 (m, 3H), 1.72 (s, 3H), 1.53 - 1.44 (m, 6H), 1.40 - 1.34 (m, 2H). | 864.6 5 | General formula ER-T-8 for PROTAC synthesis |
| 116 | ER-P-67 | 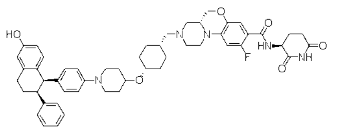 (R)-N-((S)-2,6-dioxopiperidin-3-yl)-9-fluoro-3-(((1S,4S)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-1,2,3,4,4a,5-hexahydrobenzo[b]pyrazi no[1,2-d][1,4]oxazine-8-carboxamide | $^1$H NMR (400 MHz, DMSO-$d_6$) δ 10.86 (s, 1H), 9.61 (s, 1H), 7.17 - 7.08 (m, 5H), 6.83 (d, J = 6.5 Hz, 2H), 6.62 (dd, J = 12.3, 5.3 Hz, 4H), 6.48 (dd, J = 8.3, 2.4 Hz, 1H), 6.24 (d, J = 7.5 Hz, 2H), 4.77 - 4.68 (m, 1H), 4.31 (d, J = 9.3 Hz, 1H), 4.15 (d, J = 3.9 Hz, 2H), 4.00 (dd, J = 11.2, 6.3 Hz, 1H), 3.35 (d, J = 12.4 Hz, 5H), 2.97 (d, J = 5.6 Hz, 7H), 2.90 - 2.69 (m, 5H), 2.10 (td, J = 12.6, 5.1 Hz, 2H), 2.01 - 1.80 (m, 5H), 1.71 (s, 3H), 1.48 - 1.42 (m, 7H), 1.35 (s, 2H). | 856.6 5 | General formula ER-T-8 for PROTAC synthesis |

(continued)

| Example | Comp ound | Structure and Name | $^{1}$H-NMR | LC-MS | Synthesis Method |
|---|---|---|---|---|---|
| 117 | ER-P-68 | 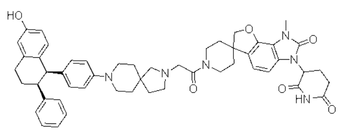 3-(1'-(2-(8-(4-((1R,2S)-6-hydroxy-2-phenyl-1,2,3,4-tetrahydronaphthalen-1-yl)phenyl)-2,8-diazaspiro[4.5]decan-2-yl)acetyl)-1-methyl-2-oxo-1,2-dihydro-3H,7H-spiro[benzofuro[6,7-d]imidazole-6,4'-piperidin]-3-yl)piperidine-2,6-dione | $^{1}$H NMR (600 MHz, DMSO-d6) δ 11.10 (s, 1H), 9.13 (s, 1H), 7.14 (ddd, *J* = 13.3, 7.7, 6.0 Hz, 3H), 6.84 (dd, *J* = 7.8, 5.2 Hz, 3H), 6.72 - 6.55 (m, 5H), 6.49 (dd, *J* = 8.3, 2.5 Hz, 1H), 6.25 (d, *J* = 8.2 Hz, 2H), 5.33 (dt, J = 10.7, 4.4 Hz, 1H), 4.68 - 4.60 (m, 2H), 4.53 - 4.37 (m, 3H), 4.34 (d, *J* = 12.4 Hz, 1H), 4.16 (d, *J* = 5.0 Hz, 1H), 3.45 (s, 5H), 3.34 - 3.26 (m, 2H), 3.21 (q, *J*= 12.6, 11.2 Hz, 2H), 3.04 (s, 2H), 2.96 (td, *J* = 17.8, 17.4, 8.9 Hz, 3H), 2.89 (dd, *J* = 13.5, 8.7 Hz, 2H), 2.72 - 2.59 (m, 2H), 2.08 (td, J = 12.7, 6.3 Hz, 1H), 2.04 - 1.95 (m, 3H), 1.87 (d, *J = 14.5* Hz, 2H), 1.73 (d, *J* = 24.9 Hz, 5H), 1.25 (d, *J* = 5.3 Hz, 3H). | 849.6 5 | General formula ER-T-7 for PROTAC synthesis |
| 118 | ER-P-69 | 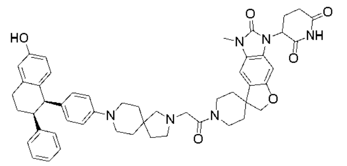 3-(1'-(2-(8-(4-((1R,2S)-6-hydroxy-2-phenyl-1,2,3,4-tetrahydronaphthalen-1-yl)phenyl)-2,8-diazaspiro[4.5]decan-2-yl)acetyl)-1-methyl-2-oxo-1,2-dihydro-3H,6H-spiro[benzofuro[5,6-d]imidazole-7,4'-piperidin]-3-yl)piperidine-2,6-dione | $^{1}$H NMR (600 MHz, DMSO-d6) δ 11.07 (s, 1H), 9.13 (s, 1H), 7.14 (dq, *J* = 14.1, 6.9 Hz, 3H), 7.03 (d, *J* = 1.5 Hz, 1H), 6.84 (dd, *J* = 6.7, 1.8 Hz, 2H), 6.72 (d, *J* = 4.4 Hz, 1H), 6.67 - 6.59 (m, 4H), 6.49 (dd, J = 8.3, 2.5 Hz, 1H), 6.25 (d, *J* = 8.3 Hz, 2H), 5.36 - 5.28 (m, 2H), 4.52 - 4.41 (m, 4H), 4.34 (d, *J* = 13.1 Hz, 1H), 4.16 (d, *J* = 4.9 Hz, 1H), 3.69 - 3.62 (m, 3H), 3.30 (d, *J* = 5.4 Hz, 3H), 3.21 (dd, *J* = 21.9, 9.5 Hz, 3H), 3.04 (s, 2H), 2.95 (d, *J* = 21.7 Hz, 3H), 2.90 - 2.84 (m, 2H), 2.72 (qd, J = 12.9, 4.4 Hz, 2H), 2.14 - 2.05 (m, 2H), 2.03 - 1.97 (m, 2H), 1.87 (dt, *J* = 13.5, 8.3 Hz, 2H), 1.77 - 1.69 (m, 5H), 1.24 (d, *J* = 4.6 Hz, 3H). | 849.6 0 | General formula ER-T-7 for PROTAC synthesis |

(continued)

| Example | Comp ound | Structure and Name | $^1$H-NMR | LC-MS | Synthesis Method |
|---|---|---|---|---|---|
| 119 | ER-P-70 | 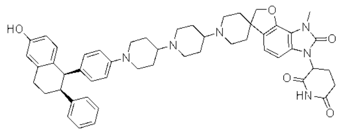 3-(2'-(1'-(4-((1R,2S)-6-hydroxy-2-phenyl-1,2,3,4-tetrahydronaphthalen-1-yl)phenyl)-[1,4'-bipiperidin]-4-yl)-1-methyl-2-oxo-1,2-dihydro-3H,7H-spiro [benzofuro[6,7-d]imidazole-6,4'-piperidin]-3-yl)piperidine-2,6-dione | $^1$H NMR (600 MHz, DMSO-$d_6$) δ 11.11 (s, 1H), 9.14 (s, 1H), 7.15 (dt, *J* = 13.2, 6.8 Hz, 3H), 6.85 (d, *J* = 7.3 Hz, 2H), 6.77 (d, *J* = 8.0 Hz, 1H), 6.70 (d, *J* = 8.1 Hz, 1H), 6.65 - 6.59 (m, 4H), 6.49 (dd, *J* = 8.2, 2.5 Hz, 1H), 6.24 (d, *J* = 8.4 Hz, 2H), 5.34 (dd, *J* = 13.1, 5.5 Hz, 1H), 4.65 (s, 2H), 4.16 (d, *J* = 5.0 Hz, 1H), 3.70 (dd, *J* = 25.3, 11.3 Hz, 5H), 3.46 - 3.43 (m, 3H) 3.31 *(d, J* = 15.3 Hz, 3H), 3.12 - 2.88 (m, 8H), 2.71 - 2.54 (m, 5H), 2.34 (d, *J* = 12.6 Hz, 1H), 2.17 (t, *J* = 12.9 Hz, 2H), 2.11 - 2.07 (m, 1H), 2.07 - 2.03 (m, 2H), 2.01 - 1.96 (m, 3H), 1.96 - 1.88 (m, 2H), 1.70 (ddd, *J* = 27.5, 12.2, 5.1 Hz, 3H). | 835.6 0 | General formula ER-T-1 for PROTAC synthesis |

(continued)

| Example | Comp ound | Structure and Name | $^1$H-NMR | LC-MS | Synthesis Method |
|---|---|---|---|---|---|
| 120 | ER-P-71 | 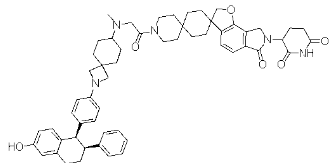 3-(1"-(N-(2-(4-((1R,2S)-6-hydroxy-2-phenyl-1,2,3,4-tetrahydronaphthalen-1-yl)phenyl)-2-azaspiro[3.5]nonan-7-yl)-N-methylglycyl)-6-oxo-6,8-dihydro-2H,7H-dipyridole[furo[2,3-e]isoindole-3,1'-cyclohexane-4',4"-piperidin]-7-yl) piperidine-2,6-dione | $^1$H NMR (600 MHz, DMSO-$d_6$) δ 11.00 (s, 1H), 9.15 - 9.12 (m, 1H), 7.48 (dd, *J* = 15.1, 7.6 Hz, 1H), 7.30 (t, *J* = 7.5 Hz, 1H), 7.19 - 7.09 (m, 3H), 6.83 (d, *J* = 7.4 Hz, 2H), 6.64 - 6.57 (m, 2H), 6.48 (dd, *J* = 8.3, 2.5 Hz, 1H), 6.18 (d, *J* = 8.0 Hz, 2H), 6.07 - 6.00 (m, 2H), 5.09 (dd, *J* = 13.3, 5.2 Hz, 1H), 4.53 (q, *J* = 9.0 Hz, 2H), 4.38 (dd, *J* = 17.1, 3.2 Hz, 1H), 4.31 (t, *J* = 18.8 Hz, 2H), 4.21 *(d, J* = 17.1 Hz, 1H), 4.13 (t, *J* = 11.6 Hz, 3H), 3.29 (s, 2H), 3.16 (s, 2H), 3.01 - 2.86 (m, 4H), 2.78 - 2.69 (m, 3H), 2.66 - 2.56 (m, 2H), 2.43 (d, *J* = 15.1 Hz, 2H), 2.10 (dd, *J* = 12.6, 6.0 Hz, 2H), 1.98 (d, *J* = 12.4 Hz, 3H), 1.90 (d, *J* = 30.9 Hz, 3H), 1.69 (d, *J* = 28.8 Hz, 4H), 1.58 (d, *J* = 10.8 Hz, 2H), 1.53 - 1.40 (m, 5H), 1.37 - 1.22 (m, 5H). | 916.7 0 | General formula ER-T-7 for PROTAC synthesis |

(continued)

| Example | Comp ound | Structure and Name | $^1$H-NMR | LC-MS | Synthesis Method |
|---|---|---|---|---|---|
| 121 | ER-P-72 | 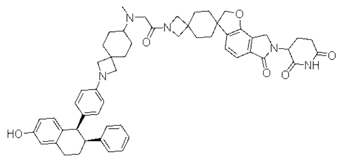 3-(1-(N-(2-(4-((1R,2S)-6-hydroxy-2-phenyl-1,2,3,4-tetrahydronaphthalen-1-yl)phenyl)-2-azaspiro[3.5]nonan-7-yl)-N-methylglycyl)-6"-oxo-6",8"-dihydro-2"H,7"H-dipyridole[azetidine-3,1'-cyclohexane-4',3"-furo[2,3-e]isoindol]-7"-yl)piperidine-2,6-dione | $^1$H NMR (600 MHz, DMSO-$d_6$) $\delta$ 10.99 (s, 1H), 9.12 (s, 1H), 7.39 (dd, $J$ = 20.0, 7.6 Hz, 2H), 7.30 (dd, $J$ = 7.6, 4.4 Hz, 1H), 7.14 (dt, $J$ = 16.4, 7.8 Hz, 3H), 6.83 (d, $J$ = 7.4 Hz, 1H), 6.64 - 6.59 (m, 2H), 6.48 (dd, $J$ = 8.2, 2.5 Hz, 1H), 6.21 - 6.16 (m, 2H), 6.03 (dd, $J$ = 8.5, 2.7 Hz, 2H), 5.11 - 5.08 (m, 1H), 4.53 (q, $J$ = 9.2 Hz, 2H), 4.38 (d, $J$ = 13.7 Hz, 1H), 4.22 (dd, $J$ = 17.1, 3.4 Hz, 2H), 4.15 - 4.06 (m, 3H), 4.04 - 4.01 (m, 1H), 3.88 - 3.82 (m, 3H), 3.66 (s, 1H), 2.99 - 2.88 (m, 4H), 2.74 (dd, $J$ = 10.2, 4.7 Hz, 2H), 2.61 (s, 1H), 2.44 - 2.41 (m, 1H), 2.10 (dd, $J$ = 12.0, 5.9 Hz, 2H), 1.99 (s, 4H), 1.91 (s, 3H), 1.67 (d, $J$ = 23.2 Hz, 5H), 1.49 (s, 3H), 1.27 - 1.21 (m, 3H). | 888.7 0 | General formula ER-T-7 for PROTAC synthesis |
| 122 | ER-P-73 | 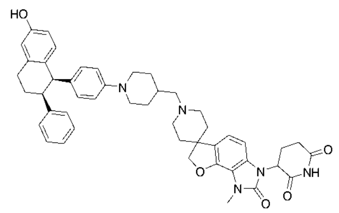 3-(1'-((1-(4-((1R,2S)-6-hydroxy-2-phenyl-1,2,3,4-tetrahydronaphthalen-1-yl)phenyl)piperidin-4-yl)methyl)-1-methyl-2-oxo-1,2-dihydro-3H,7H-spiro[benzofuro[6,7-d]imidazole-6,4'-piperidin]-3-yl)piperidine-2,6-dione | $^1$H NMR (600 MHz, DMSO-$d_6$) $\delta$ 11.10 (s, 1H), 7.14 (dt, $J$ = 13.2, 6.9 Hz, 3H), 6.83 (d, $J$ = 7.2 Hz, 2H), 6.76 (d, $J$ = 8.0 Hz, 1H), 6.73 - 6.55 (m, 5H), 6.48 (dd, $J$ = 8.3, 2.5 Hz, 1H), 6.26 (d, $J$ = 8.2 Hz, 2H), 5.33 (dd, $J$ = 12.8, 5.5 Hz, 1H), 4.62 (s, 2H), 4.16 (d, J = 5.1 Hz, 1H), 3.58 - 3.53 (m, 5H), 3.33 - 3.26 (m, 3H), 3.09 - 2.87 (m, 7H), 2.74 - 2.58 (m, 4H), 2.21 - 2.06 (m, 3H), 1.98 (dp, $J$ = 12.1, 7.2, 5.6 Hz, 2H), 1.90 (d, $J$ = 14.1 Hz, 2H), 1.81 (d, $J$ = 12.9 Hz, 2H), 1.71 (dd, $J$ = 12.4, 6.1 Hz, 1H), 1.38 - 1.25 (m, 2H). | 766.6 | General formula ER-T-8 for PROTAC synthesis |

(continued)

| Example | Comp ound | Structure and Name | $^1$H-NMR | LC-MS | Synthesis Method |
|---|---|---|---|---|---|
| 123 | ER-P-74 | 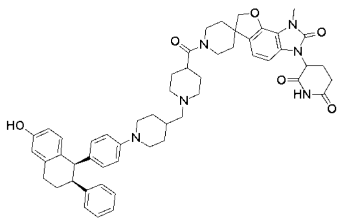 3-(1'-(1-(4-(((1R,2S)-6-hydroxy-2-phenyl-1,2,3,4-tetrahydronaphthalen-1-yl)phenyl)piperidin-4-yl)methyl)piperidine-4-carbonyl)-1-methyl-2-oxo-1,2-dihydro-3H,7H-spiro[benzofuro[6,7-d] imidazole-6,4'-piperidin]-3-yl)piperidine-2,6-dione | $^1$H NMR (600 MHz, DMSO-$d_6$) δ 11.08 (s, 1H), 9.11 (s, -2H), 7.14 (dt, *J* = 13.7, 6.7 Hz, 4H), 6.86 (dd, *J* = 33.6, 7.5 Hz, 4H), 6.65 - 6.55 (m, 7H), 6.48 (dd, *J* = 8.3, 2.6 Hz, 1H), 6.22 (d, *J* = 8.2 Hz, 3H), 5.32 (d, *J* = 12.9 Hz, 1H), 4.68 - 4.52 (m, 2H), 4.36 (d, *J* = 13.0 Hz, 1H), 4.14 (d, *J* = 5.0 Hz, 1H), 3.98 (d, *J* = 13.7 Hz, 1H), 3.54 (d, *J* = 11.4 Hz, 6H), 3.02 - 2.85 (m, 13H), 2.09 (dd, *J* = 12.6, 6.3 Hz, 2H), 1.97 (d, *J* = 11.9 Hz, 2H), 1.87 (s, 4H), 1.70 (s, 4H), 1.25 (d, *J* = 14.3 Hz, 3H). | 877.6 | General formula ER-T-5 for PROTAC synthesis |
| 124 | ER-P-75 | 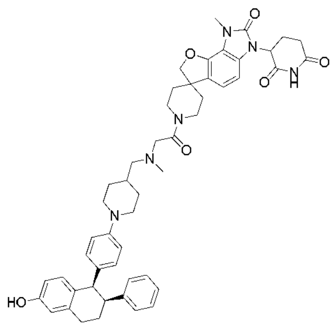 3-(1'-(N-((1-(4-((1R,2S)-6-hydroxy-2-phenyl-1,2,3,4-tetrahydronaphthalen-1-yl)phenyl)piperidin-4-yl)methyl)-N-methylglycyl)-1-methyl-2-oxo-1,2-dihydro-3H,7H-spiro[benzofuro[6,7-d] imidazole-6,4'-piperidin]-3-yl)piperidine-2,6-dione | $^1$H NMR (600 MHz, DMSO-$d_6$) δ 11.09 (s, 1H), 9.11 (s, 2H), 7.14 (dt, *J* = 13.9, 7.1 Hz, 3H), 6.87 - 6.81 (m, 3H), 6.70 - 6.52 (m, 5H), 6.48 (dd, *J* = 8.2, 2.5 Hz, 1H), 6.28 - 6.19 (m, 2H), 5.48 - 5.29 (m, 1H), 5.07 (d, *J* = *5.9* Hz, 1H), 4.69 - 4.58 (m, 2H), 4.34 (q, *J* = 21.1, 19.7 Hz, 3H), 4.15 (d, *J* = 5.0 Hz, 1H), 3.63 (d, *J* = 13.8 Hz, 2H), 3.32 - 3.27 (m, 2H), 3.19 (t, *J* = 12.6 Hz, 2H), 3.05 (dd, *J* = 19.6, 13.7 Hz, 2H), 2.97 (td, *J* = 16.2, 15.8, 6.1 Hz, 3H), 2.92 - 2.83 (m, 5H), 2.78 (d, *J* = 17.2 Hz, 1H), 2.71 - 2.64 (m, 1H), 2.14 - 1.82 (m, 6H), 1.73 (d, J = 24.2 Hz, 6H), 1.29 (s, 2H). | | General formula ER-T-7 for PROTAC synthesis |

(continued)

| Example | Comp ound | Structure and Name | $^1$H-NMR | LC-MS | Synthesis Method |
|---|---|---|---|---|---|
| 125 | ER-P-76 | 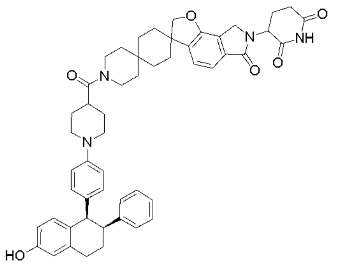 3-(1"-(1-(4-((1R,2S)-6-hydroxy-2-phenyl-1,2,3,4-tetrahydro-naphthalen-1-yl)phenyl)piperi-dine-4-carbonyl)-6-oxo-6,8-dihydro-2H,7H-dipyridole[furo[2,3-e] isoindole-3,1'-cyclohexane-4',4"-piperidin]-7-yl)piperidine-2,6-dione | $^1$H NMR (600 MHz, DMSO-$d_6$) δ 10.98 (s, 1H), 9.17 (s, 1H), 7.46 (dd, $J$ = 13.9, 7.6 Hz, 1H), 7.28 (t, $J$ = 6.9 Hz, 1H), 7.15 (dtt, $J$ = 13.1, 7.6, 3.8 Hz, 3H), 6.90 - 6.80 (m, 3H), 6.72 - 6.60 (m, 2H), 6.54 - 6.22 (m, 4H), 5.09 (dd, $J$ = 13.3, 5.1 Hz, 1H), 4.53 (t, $J$ = 7.6 Hz, 2H), 4.38 (dd, $J$ = 17.0, 2.7 Hz, 1H), 4.21 (d, $J$ = 17.0 Hz, 2H), 3.81 (s, 8H), 3.58 - 3.43 (m, 5H), 3.38 - 3.31 (m, 1H), 3.10 - 2.85 (m, 4H), 2.66 - 2.55 (m, 1H), 2.46 - 2.38 (m, 1H), 2.17 - 2.05 (m, 1H), 1.98 (ddd, $J$ = 10.8, 5.5, 3.3 Hz, 1H), 1.90 - 1.67 (m, 6H), 1.62 - 1.52 (m, 2H), 1.37 (s, 1H), 1.31 - 1.21 (m, 3H). | 833.6 0 | General formula ER-T-9 for PROTAC synthesis |
| 126 | ER-P-77 | 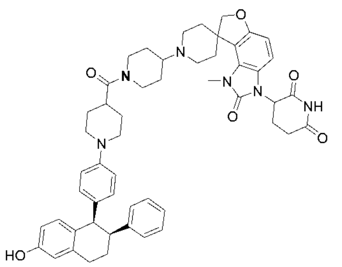 3-(1'-(1-(4-(((1R,2S)-6-hydroxy-2-phenyl-1,2,3,4-tetrahydro-naphthalen-1-yl)phenyl)piperi-dine-4-carbonyl)piperidin-4-yl)-1-methyl-2-oxo-1,2-dihydro-3H,7H-spiro[benzofuro[4,5-d]imidazole-8,4'-piperidin]-3-yl)piperi-dine-2,6-dione | $^1$H NMR (600 MHz, DMSO-$d_6$) δ 11.10 (s, 1H), 9.29 (s, 1H), 7.20 - 7.08 (m, 3H), 6.97 (d, $J$ = 8.5 Hz, 1H), 6.87 - 6.79 (m, 2H), 6.68 - 6.56 (m, 4H), 6.49 (dd, $J$ = 8.3, 2.5 Hz, 1H), 6.27 (s, 2H), 5.36 (dd, $J$ = 13.1, 5.2 Hz, 1H), 4.65 - 4.46 (m, 3H), 4.17 (s, 2H), 3.60 (s, 6H), 3.32 (d, $J$ = 13.4 Hz, 3H), 3.09 (d, $J$ = 21.1 Hz, 5H), 3.01 - 2.82 (m, 3H), 2.73 - 2.55 (m, 4H), 2.19 - 2.07 (m, 5H), 2.01 - 1.91 (m, 1H), 1.66 (d, $J$ = 44.4 Hz, 5H), 1.52 - 1.41 (m, 2H), 1.24 (s, 1H). | 863.6 5 | General formula ER-T-9 for PROTAC synthesis |

(continued)

| Example | Comp ound | Structure and Name | $^1$H-NMR | LC-MS | Synthesis Method |
|---|---|---|---|---|---|
| 127 | ER-P-78 | 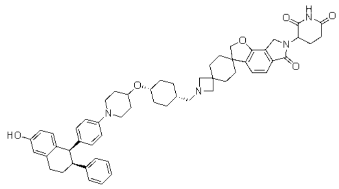 3-(1-(((1S,4S)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-6"-oxo-6'',8''-dihydro-2"H,7"H-dipyrine[azetidine-3,1'-cyclohexane-4',3"-furo [2,3-e]isoindol]-7"-yl)piperidine-2,6-dione | $^1$H NMR (600 MHz, DMSO-$d_6$) δ 11.00 (s, 1H), 9.16 (s, 1H), 7.41 (d, *J* = 7.6 Hz, 1H), 7.29 (*d, J* = 7.6 Hz, 1H), 7.14 (dt, *J* = 13.2, 6.8 Hz, 4H), 6.83 (d, *J* = 7.3 Hz, 2H), 6.68 - 6.61 (m, 3H), 6.49 (dd, *J* = 8.3, 2.6 Hz, 1H), 6.31 - 6.23 (m, 2H), 5.11 - 5.07 (m, 1H), 4.49 (t, *J* = 7.9 Hz, 2H), 4.37 (d, *J* = 17.1 Hz, 1H), 4.18 (d, *J* = 11.7 Hz, 3H), 3.31 (d, *J* = 10.6 Hz, 5H), 3.09 (q, *J* = 5.9 Hz, 2H), 3.00 - 2.88 (m, 4H), 2.61 - 2.57 (m, 1H), 2.43 (dt, *J* = 13.2, 6.7 Hz, 2H), 2.15 - 2.08 (m, 2H), 1.97 (dd, *J* = 25.3, 8.9 Hz, 4H), 1.86 (s, 2H), 1.80 - 1.67 (m, 5H), 1.61 (p, *J* = 10.4, 8.5 Hz, 5H), 1.51 (s, 3H), 1.13 (qd, *J* = 13.1, 11.4, 5.6 Hz, 3H), 1.01 (q, *J* = 11.7, 11.2 Hz, 3H). | 889.7 0 | General formula ER-T-8 for PROTAC synthesis |

(continued)

| Example | Comp ound | Structure and Name | $^1$H-NMR | LC-MS | Synthesis Method |
|---|---|---|---|---|---|
| 128 | ER-P-79 | 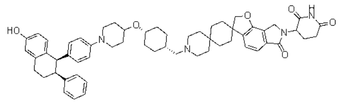 3-(1"-(((1S,4S)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-6-oxo-6,8-dihydro-2H,7H-dispiro[furo[2,3-e]isoindole-3,1'-cyclohexane-4',4"-piperidin]-7-yl)piperidine-2,6-dione | $^1$HNMR(600MHz, DMSO-$d_6$) δ 11.00 (s, 1H), 8.90 (s, 1H), 7.48 (d, *J* = 7.6 Hz, 1H), 7.29 (d, *J* = 7.6 Hz, 1H), 7.13 (dq, *J* = 14.0, 7.2 Hz, 3H), 6.83 (d, *J* = 7.2 Hz, 2H), 6.75 - 6.60 (m, 4H), 6.49 (dd, *J* = 8.3, 2.5 Hz, 1H), 6.27 (d, *J* = 8.1 Hz, 2H), 5.09 (dt, *J* = 13.4, 3.9 Hz, 1H), 4.58 - 4.45 (m, 2H), 4.37 (d, *J* = 17.0 Hz, 1H), 4.25 - 4.15 (m, 2H), 3.34 (dd, *J* = 24.5, 8.9 Hz, 6H), 3.06 - 2.81 (m, 10H), 2.64 - 2.56 (m, 1H), 2.42 (dd, *J*= 13.3, 4.4 Hz, 1H), 2.32 (d, *J* = 14.4 Hz, 1H), 2.08 (dq, *J* = 12.2, 6.3 Hz, 2H), 1.97 (dt, *J* = 10.8, 5.4 Hz, 1H), 1.84 (qd, *J* = 14.3, 11.6, 4.4 Hz, 5H), 1.69 (tt, J = 13.8, 10.0, 8.0 Hz, 4H), 1.50 (ddt, *J* = 49.3, 42.7, 14.8 Hz, 12H), 1.32 (q, *J* = 11.6, 10.9 Hz, 3H), 1.17 (dd, *J*= 9.1, 5.3 Hz, 1H). | 917.2 0 | General formula ER-T-8 for PROTAC synthesis |
| 129 | ER-P-80 | 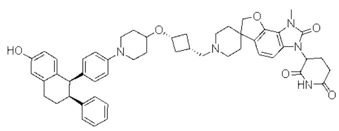 3-(1'-(((1S,3s)-3-((1-(4-((1R,2-S)-6-hydroxy-2-phenyl-1,2,3,4-tetrahydronaphthalen-1-yl)phenyl)piperidin-4-yl)oxy)cyclobutyl) methyl )-1-methyl-2-oxo-1,2-dihydro-3H,7H-spiro[benzofuro[6,7-d] imidazole-6,4'-piperidin]-3-yl)piperidine-2,6-dione | $^1$H NMR (400 MHz, DMSO-$d_6$) δ 11.12 (s, 1H), 9.25 (s, 1H), 7.14 (d, *J* = 7.3 Hz, 3H), 6.84 - 6.81 (m, 2H), 6.75 (d, *J* = 8.0 Hz, 1H), 6.70 - 6.60 (m, 5H), 6.50 - 6.47 (m, 1H), 6.26 (d, *J* = 8.1 Hz, 2H), 5.34 (d, *J* = 7.8 Hz, 1H), 4.61 (s, 2H), 3.74 - 3.66 (m, 14H), 3.24 (d, *J* = 6.0 Hz, 1H), 3.17 (*d, J* = 5.9 Hz, 1H), 3.07 - 2.89 (m, 6H), 2.64 (q, *J* = 5.0 Hz, 2H), 2.16 - 2.06 (m, 5H), 1.94 - 1.81 (m, 5H), 1.70 (t, *J* = 9.3 Hz, 2H), 1.51 (s, 2H). | 836.6 5 | General formula ER-T-8 for PROTAC synthesis |

(continued)

| Example | Comp ound | Structure and Name | $^1$H-NMR | LC-MS | Synthesis Method |
|---|---|---|---|---|---|
| 130 | ER-P-81 | 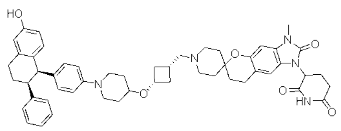 3-(1'-(((1S,3s)-3-((1-(4-((1R,2-S)-6-hydroxy-2-phenyl-1,2,3,4-tetrahydronaphthalen-1-yl)phenyl)piperidin-4-yl)oxy)cyclobutyl) methyl)-3-methyl-2-oxo-2,3,7,8-tetrahydro-1H-spiro[chromeno [6,7-d]imidazole-6,4'-piperidin]-1-yl)piperidine-2,6-dione | $^1$H NMR (400 MHz, DMSO-$d_6$) $\delta$ 10.99 (s, 1H), 9.15 (s, 1H), 7.41 (d, *J* = 7.6 Hz, 1H), 7.29 (*d, J* = 7.6 Hz, 1H), 7.14 (d, *J* = 7.3 Hz, 3H), 6.85 - 6.81 (m, 2H), 6.70 - 6.59 (m, 4H), 6.48 (dd, *J* = 8.3, 2.5 Hz, 1H), 6.25 (d, *J* = 8.1 Hz, 2H), 5.11 - 5.06 (m, 1H), 4.47 (d, *J* = 4.5 Hz, 2H), 4.35 (s, 1H), 4.24 - 4.14 (m, 3H), 4.10 (d, *J* = 9.5 Hz, 1H), 3.95 (t, *J* = 7.3 Hz, 2H), 3.66 (s, 3H), 3.37 (s, 4H), 2.95 (dd, *J* = 15.9, 9.3 Hz, 3H), 2.79 (s, 2H), 2.38 (dt, *J* = 13.0, 5.9 Hz, 3H), 2.08 (t, *J* = *9.7* Hz, 3H), 1.98 (q, *J* = 6.9 Hz, 3H), 1.84 (s, 2H), 1.74 - 1.59 (m, 7H), 1.49 (d, *J* = 10.0 Hz, 3H). | 850.6 5 | General formula ER-T-8 for PROTAC synthesis |
| 131 | ER-P-82 | 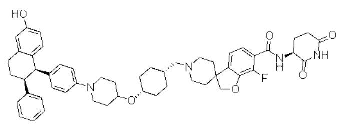 N-((S)-2,6-dioxopiperidin-3-yl)-7-fluoro-1'-(((1S,4R)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-2H-spiro[benzofuran-3,4'-piperidine]-6-carboxamide | $^1$H NMR (600 MHz, DMSO-$d_6$) $\delta$ 10.88 (s, 1H), 9.14 (s, 1H), 8.58 (d, *J* = 8.3 Hz, 1H), 7.23 - 7.10 (m, 3H), 7.02 (d, *J* = 7.7 Hz, 1H), 6.83 (d, *J* = 7.3 Hz, 1H), 6.73 - 6.41 (m, 5H), 6.24 (s, 4H), 4.74 (ddd, *J* = 13.0, 8.2, 5.4 Hz, 1H), 4.67 (s, 1H), 4.59 (s, 1H), 4.15 (s, 1H), 2.97 (d, *J* = 5.9 Hz, 5H), 2.78 (d, *J* = 12.5 Hz, 3H), 2.24 - 2.05 (m, 4H), 2.03 - 1.66 (m, 8H), 1.47 (s, 3H), 1.28 - 0.93 (m, 5H). | 855.6 0 | General formula ER-T-8 for PROTAC synthesis |

(continued)

| Example | Comp ound | Structure and Name | $^1$H-NMR | LC-MS | Synthesis Method |
|---|---|---|---|---|---|
| 132 | ER-P-83 | 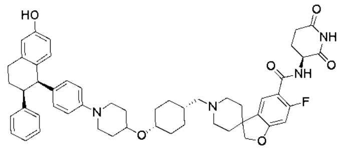 N-((S)-2,6-dioxopiperidin-3-yl)-6-fluoro-1'-(((1S,4R)-4-((1-(4-((1R,2S)-6-hydroxy-2-phenyl-1,2,3,4-tetrahydronaphthalen-1-yl)phenyl)piperidin-4-yl)oxy)cyclohexyl)methyl)-2H-spiro[benzofuran-3,4'-piperidine]-5-carboxamide | $^1$H NMR (600 MHz, DMSO-$d_6$) δ 10.88 (d, *J* = 8.1 Hz, 1H), 8.91 (s, 1H), 8.34 (dd, *J* = 8.2, 4.9 Hz, 1H), 7.48 (d, *J* = 7.3 Hz, 1H), 7.14 (dt, *J* = 13.7, 7.1 Hz, 3H), 6.89 (d, *J* = *11.5* Hz, 1H), 6.83 (d, *J* = 7.3 Hz, 2H), 6.73 - 6.58 (m, 4H), 6.48 (dd, *J* = 8.2, 2.5 Hz, 1H), 6.25 (d, *J* = 7.7 Hz, 2H), 4.77 (td, *J* = 7.7, 4.0 Hz, 1H), 4.63 (s, 2H), 3.54 (d, *J* = 12.7 Hz, 4H), 3.40 - 3.25 (m, 6H), 3.04 - 2.93 (m, 5H), 2.80 (td, *J* = 13.2, 12.5, 6.7 Hz, 2H), 2.21 (dq, *J* = 14.4, 4.4 Hz, 2H), 2.10 (ddd, *J* = 16.4, 8.4, 4.0 Hz, 2H), 2.03 - 1.92 (m, 5H), 1.80 (td, *J* = 34.6, 34.1, 16.7 Hz, 6H), 1.48 (s, 2H), 1.25 - 1.16 (m, 3H), 1.04 (t, *J* = 12.5 Hz, 2H). | 855.6 5 | General formula ER-T-8 for PROTAC synthesis |
| 133 | ER-P-84 | 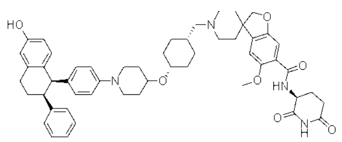 N-((S)-2,6-dioxopiperidin-3-yl)-1'-(((1S,4R)-4-((1-(4-((1R,2S)-6-hydroxy-2-phenyl-1,2,3,4-tetrahydronaphthalen-1-yl)phenyl)piperidin-4-yl)oxy)cyclohexyl)methyl)-5-methoxy-2H-spiro[benzofuran-3,4'-piperidine]-6-carboxamide | $^1$H NMR (600 MHz, DMSO-$d_6$) δ 10.91 (s, 1H), 9.07 (s, 1H), 8.67 (d, *J* = 7.3 Hz, 1H), 7.24 (s, 1H), 7.14 (ddd, *J* = 13.5, 7.7, 6.1 Hz, 3H), 6.87 (s, 1H), 6.85 - 6.82 (m, 2H), 6.67 - 6.60 (m, 3H), 6.49 (dd, *J* = 8.3, 2.6 Hz, 1H), 6.26 (s, 2H), 4.75 - 4.71 (m, 1H), 4.51 (s, 2H), 4.16 (d, *J* = 4.6 Hz, 1H), 3.90 (s, 3H), 3.54 (d, *J* = 12.3 Hz, 4H), 3.40 - 3.31 (m, 5H), 3.05 - 3.00 (m, 2H), 2.99 - 2.90 (m, 4H), 2.81 - 2.74 (m, 2H), 2.29 - 2.23 (m, 2H), 2.09 (td, *J* = 11.7, 10.4, 4.1 Hz, 3H), 1.96 - 1.85 (m, 5H), 1.81 - 1.69 (m, 4H), 1.49 (s, 2H), 1.28 - 1.16 (m, 4H), 1.05 (t, *J* = 12.8 Hz, 2H). | 867.3 6 | General formula ER-T-8 for PROTAC synthesis |

(continued)

| Example | Comp ound | Structure and Name | $^1$H-NMR | LC-MS | Synthesis Method |
|---|---|---|---|---|---|
| 134 | ER-P-85 | 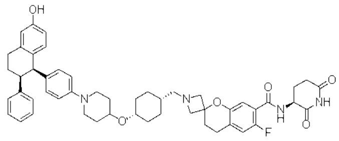 N-((S)-2,6-dioxopiperidin-3-yl)-6'-fluoro-1-(((1 S,4R)-4-((1-(4-((1R,2S)-6-hydroxy-2-phenyl-1,2,3,4-tetrahydronaphthalen-1-yl)phenyl)piperidin-4-yl)oxy)cyclohexyl)methy l)spiro[azetidine-3,2'-chromane]-7'-carboxamide | $^1$H NMR (600 MHz, DMSO-$d_6$) δ 10.89 (s, 1H), 9.15 (s, 1H), 8.47 (dt, *J* = 8.6, 4.4 Hz, 1H), 7.21 - 7.07 (m, 5H), 6.83 (d, *J* = 7.3 Hz, 2H), 6.76 - 6.57 (m, 4H), 6.48 (dd, *J* = 8.2, 2.6 Hz, 1H), 6.27 (s, 2H), 4.77 - 4.72 (m, 1H), 4.42 (dd, *J* = 11.8, 6.1 Hz, 1H), 4.29 (td, *J* = 16.0, 14.0, 6.4 Hz, 2H), 4.21 - 4.16 (m, 2H), 3.57 (s, 2H), 3.40 - 3.27 (m, 5H), 3.18 (d, *J* = 12.9 Hz, 1H), 3.12 (t, *J* = 6.5 Hz, 1H), 3.00 - 2.92 (m, 2H), 2.87 (t, *J* = 6.6 Hz, 1H), 2.79 (ddd, *J* = 23.2, 12.4, 5.9 Hz, 3H), 2.18 (dt, *J* = 14.7, 6.9 Hz, 2H), 2.10 (ddd, *J* = 17.9, 10.9, 5.2 Hz, 2H), 2.00 (dq, *J* = *10.3,* 4.6, 3.7 Hz, 1H), 1.95 - 1.90 (m, 2H), 1.85 (s, 2H), 1.73 (d, *J* = 13.0 Hz, 3H), 1.53 (d, *J* = 35.7 Hz, 3H), 1.11 (q, *J* = 12.1 Hz, 2H), 1.01 *(q, J* = 12.2 Hz, 2H). | 841.8 5 | General formula ER-T-8 for PROTAC synthesis |
| 135 | ER-P-86 | 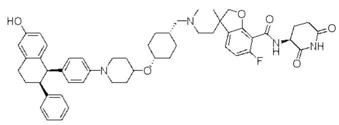 N-((S)-2,6-dioxopiperidin-3-yl)-6-fluoro-1'-((((1S,4R)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-2H-spiro[benzofuran-3,4'-piperidine]-7-carboxamide | $^1$H NMR (400 MHz, DMSO-$d_6$) δ 10.87 (s, 1H), 9.14 (s, 1H), 8.68 (d, *J* = 8.1 Hz, 1H), 7.14 (q, *J* = 8.0 Hz, 4H), 6.86 - 6.78 (m, 3H), 6.70 - 6.55 (m, 4H), 6.48 (dd, *J* = 8.2, 2.6 Hz, 1H), 6.23 (d, *J* = 8.0 Hz, 2H), 4.77 - 4.52 (m, 5H), 4.14 (d, *J* = 5.0 Hz, 1H), 3.08 - 2.62 (m, 13H), 2.11 (q, *J* = 13.2, 12.1 Hz, 4H), 2.00 (td, *J* = 10.0, 9.2, 4.0 Hz, 2H), 1.88 (dd, *J* = 22.3, 10.0 Hz, 4H), 1.72 (s, 2H), 1.58 - 1.21 (m, 10H). | 855.6 0 | General formula ER-T-8 for PROTAC synthesis |

(continued)

| Example | Comp ound | Structure and Name | $^1$H-NMR | LC-MS | Synthesis Method |
|---|---|---|---|---|---|
| 136 | ER-P-87 | 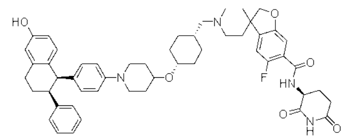 N-((S)-2,6-dioxopiperidin-3-yl)-5-fluoro-1'-(((1S,4R)-4-((1-(4-((1R,2S)-6-hydroxy-2-phenyl-1,2,3,4-tetrahydronaphthalen-1-yl)phenyl)piperidin-4-yl)oxy)cyclohexyl) methyl)-2H-spiro[benzofuran-3,4'-piperidine]-6-carboxamide | $^1$H NMR (400 MHz, DMSO-$d_6$) δ 10.89 (s, 1H), 9.15 (s, 1H), 8.52 (dd, *J* = 8.3, 3.1 Hz, 1H), 7.73 (d, *J* = 10.2 Hz, 1H), 7.14 (q, *J* = 8.0, 7.1 Hz, 2H), 7.08 - 6.96 (m, 2H), 6.87 - 6.79 (m, 2H), 6.72 - 6.54 (m, 3H), 6.48 (dd, *J* = 8.3, 2.5 Hz, 1H), 6.26 (s, 3H), 4.73 (ddd, *J* = 12.7, 8.2, 5.5 Hz, 1H), 4.51 (d, *J* = 33.9 Hz, 2H), 4.16 (d, *J* = 5.0 Hz, 1H), 3.10 - 2.61 (m, 9H), 2.24 - 1.64 (m, 13H), 1.48 (s, 3H), 1.31 - 0.80 (m, 6H). | 855.6 0 | General formula ER-T-8 for PROTAC synthesis |
| 137 | ER-P-88 | 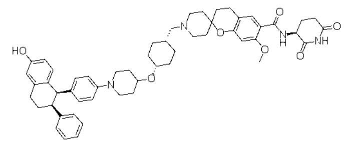 N-((S)-2,6-dioxopiperidin-3-yl)-1'-(((1S,4s)-4-((1-(4-((1R,2S)-6-hydroxy-2-phenyl-1,2,3,4-tetrahydronaphthalen-1-yl)phenyl)piperidin-4-yl)oxy)cyclohexyl) methyl)-7-methoxyspiro[pyran-2,4'-piperidine]-6-carboxamide | $^1$H NMR (600 MHz, DMSO-$d_6$) δ 10.90 (s, 1H), 9.14 (s, 1H), 8.48 (d, *J* = 7.1 Hz, 1H), 7.71 (d, *J* = 3.7 Hz, 1H), 7.13 (dq, *J* = 14.1, 7.1 Hz, 3H), 6.83 (d, *J* = 7.3 Hz, 2H), 6.76 - 6.53 (m, 5H), 6.48 (dd, *J* = 8.3, 2.5 Hz, 1H), 6.25 (s, 2H), 4.73 (dt, *J* = 11.0, 6.8 Hz, 1H), 4.16 (s, 2H), 3.88 (s, 3H), 3.15 (s, 3H), 3.07 - 2.87 (m, 5H), 2.83 - 2.64 (m, 5H), 2.16 - 1.64 (m, 16H), 1.47 (s, 4H), 1.30 - 1.13 (m, 4H), 1.02 (q, *J* = 12.9, 12.2 Hz, 3H). | 881.6 0 | General formula ER-T-8 for PROTAC synthesis |
| 138 | ER-P-89 | 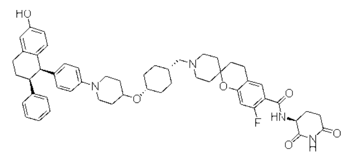 N-((S)-2,6-dioxopiperidin-3-yl)-7-fluoro-1'-(((1S,4s)-4-((1-(4-((1R,2S)-6-hydroxy-2-phenyl-1,2,3,4-tetrahydronaphthalen-1-yl)phenyl)piperidin-4-yl)oxy)cyclohexyl) methyl)spiro[chromane-2,4'-piperidine]-6-carboxamide | $^1$H NMR (600 MHz, DMSO-$d_6$) δ 10.87 (s, 1H), 9.15 (s, 1H), 8.27 (td, *J* = 8.0, 4.4 Hz, 1H), 7.55 (d, *J* = 8.3 Hz, 1H), 7.13 (dq, *J* = 14.1, 7.0 Hz, 3H), 6.87 - 6.72 (m, 3H), 6.70 - 6.55 (m, 3H), 6.48 (dd, *J* = 8.3, 2.5 Hz, 1H), 6.26 (s, 3H), 4.75 (ddd, *J* = 12.9, 8.1, 5.3 Hz, 2H), 4.16 (d, *J* = 4.5 Hz, 2H), 3.20 - 2.90 (m, 8H), 2.78 (dt, *J* = 17.3, 6.3 Hz, 4H), 2.54 (s, 1H), 2.16 - 1.68 (m, 14H), 1.49 (s, 2H), 1.33 - 0.94 (m, 6H). | 869.6 0 | General formula ER-T-8 for PROTAC synthesis |

(continued)

| Example | Comp ound | Structure and Name | $^1$H-NMR | LC-MS | Synthesis Method |
|---|---|---|---|---|---|
| 139 | ER-P-90 | 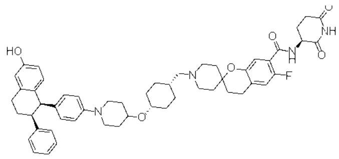 N-((S)-2,6-dioxopiperidin-3-yl)-6-fluoro-1'-((((1S,4R)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)spiro[chromane-2,4'-pi-peridine]-7-carboxamide | $^1$H NMR (600 MHz, DMSO-$d_6$) δ 10.88 (s, 1H), 8.92 (s, 1H), 8.43 (dd, *J* = 8.3, 4.2 Hz, 1H), 7.21 (d, *J* = 6.3 Hz, 1H), 7.17 - 7.11 (m, 4H), 6.85 - 6.82 (m, 2H), 6.73 - 6.59 (m, 4H), 6.49 (dd, *J* = 8.3, 2.6 Hz, 1H), 6.27 (s, 2H), 4.77 - 4.73 (m, 1H), 4.17 (s, 1H), 3.44 - 3.29 (m, 7H), 3.16 (dd, *J* = 14.8, 6.4 Hz, 2H), 3.06 (t, *J* = 6.2 Hz, 2H), 2.99 - 2.89 (m, 3H), 2.84 - 2.74 (m, 4H), 2.10 (td, *J* = 12.6, 5.7 Hz, 2H), 1.99 (dt, *J* = 12.7, 4.8 Hz, 2H), 1.93 - 1.79 (m, 9H), 1.74 - 1.69 (m, 3H), 1.54 - 1.49 (m, 3H), 1.44 (d, *J* = 13.4 Hz, 3H), 1.34 (t, *J* = 10.2 Hz, 2H). | 869.8 5 | General formula ER-T-8 for PROTAC synthesis |
| 140 | ER-P-91 | 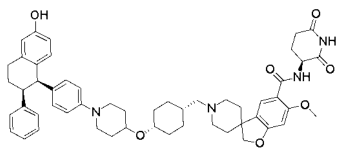 N-((S)-2,6-dioxopiperidin-3-yl)-1'-(((1S,4R)-4-((1-(4-((1R,2-S)-6-hydroxy-2-phenyl-1,2,3,4-tetrahydronaphthalen-1-yl)phenyl)piperidin-4-yl)oxy)cyclohexyl) methy l)-6-methoxy-2H-spiro [benzofuran-3,4'-piperidine]-5-carboxamide | $^1$H NMR (600 MHz, DMSO-$d_6$) δ 10.91 (s, 1H), 8.77 (s, 1H), 8.53 (d, *J* = 7.3 Hz, 1H), 7.74 (s, 1H), 7.17 - 7.10 (m, 4H), 6.84 (dd, *J* = 6.9, 1.9 Hz, 2H), 6.70 (s, 1H), 6.66 - 6.61 (m, 3H), 6.49 (dd, *J* = 8.3, 2.5 Hz, 1H), 6.28 (s, 2H), 4.58 (s, 2H), 4.18 (s, 1H), 3.69 - 3.66 (m, 2H), 3.54 (d, *J* = *12.6* Hz, 4H), 3.34 (d, *J* = 41.9 Hz, 6H), 3.01 (d, *J* = 6.9 Hz, 4H), 2.19 (*t, J* = 13.4 Hz, 3H), 2.13 - 2.05 (m, 4H), 1.88 (d, *J* = 13.7 Hz, 5H), 1.76 - 1.69 (m, 4H), 1.55 - 1.44 (m, 7H), 1.39 - 1.31 (m, 3H). | 867.7 0 | General formula ER-T-8 for PROTAC synthesis |

(continued)

| Example | Comp ound | Structure and Name | $^1$H-NMR | LC-MS | Synthesis Method |
|---|---|---|---|---|---|
| 141 | ER-P-92 | 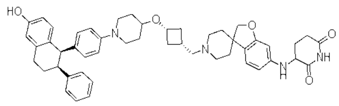 3-((1'-(((1S,3s)-3-((1-(4-((1R,2-S)-6-hydroxy-2-phenyl-1,2,3,4-tetrahydronaphthalen-1-yl)phenyl)piperidin-4-yl)oxy)cyclobutyl) methyl )-2H-spiro[benzofuran-3,4'-piperidin]-6-yl)amino)piperidine-2,6-dione | $^1$H NMR (400 MHz, DMSO-$d_6$) δ 10.79 (s, 1H), 9.18 (d, J = 48.9 Hz, 2H), 7.14 (q, J = 7.3, 6.7 Hz, 3H), 6.86 - 6.75 (m, 3H), 6.75 - 6.57 (m, 4H), 6.48 (dd, J = 8.3, 2.5 Hz, 1H), 6.32 - 6.19 (m, 3H), 6.16 (d, J = 2.1 Hz, 1H), 4.37 (s, 2H), 4.31 - 4.14 (m, 3H), 4.00 - 3.90 (m, 2H), 3.42 - 3.34 (m, 5H), 3.16 (t, J = 6.1 Hz, 2H), 2.96 (tt, J = 11.6, 7.2 Hz, 4H), 2.73 (ddd, J = 17.4, 12.1, 5.3 Hz, 2H), 2.48 - 2.42 (m, 2H), 2.05 (dtd, J = 41.6, 15.1, 14.0, 8.1 Hz, 6H), 1.83 (dt, J = 23.6, 9.3 Hz, 5H), 1.69 (dd, J = 15.3, 7.9 Hz, 3H), 1.62 - 1.39 (m, 3H). | 781.6 5 | General formula ER-T-8 for PROTAC synthesis |

The second category comprises PROTAC compounds targeting the AR (androgen receptor), synthesized according to the general formulas AR-T-1 to AR-T-4 below.

General formula AR-T-1 for PROTAC synthesis

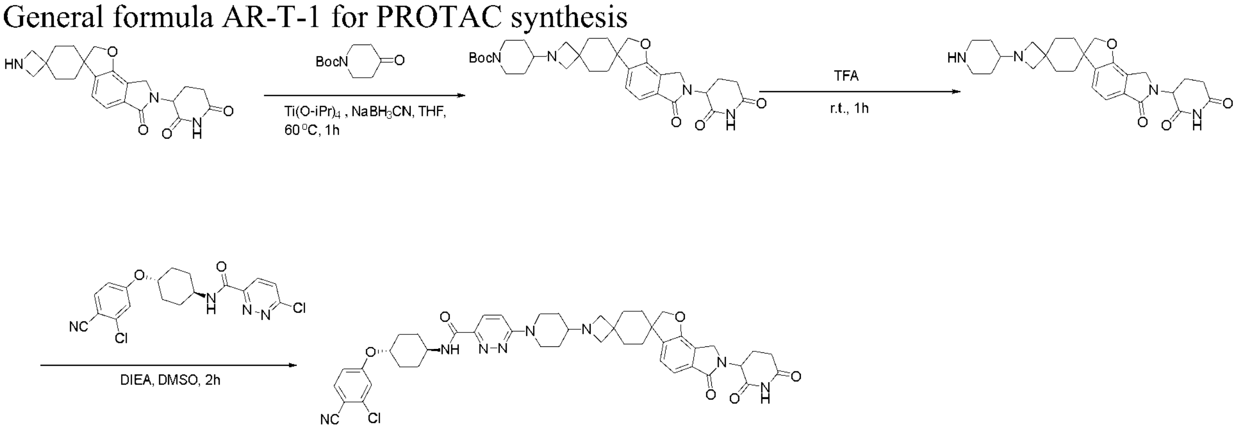


General formula AR-T-2 for PROTAC synthesis

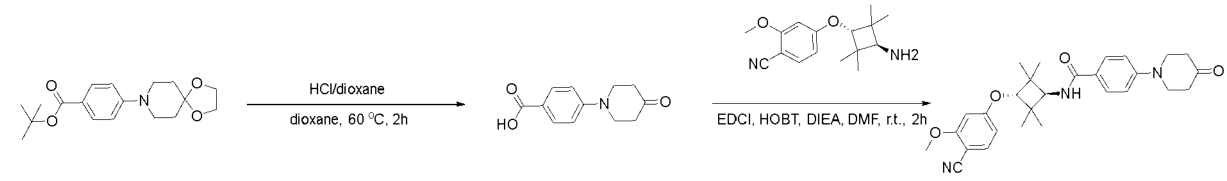


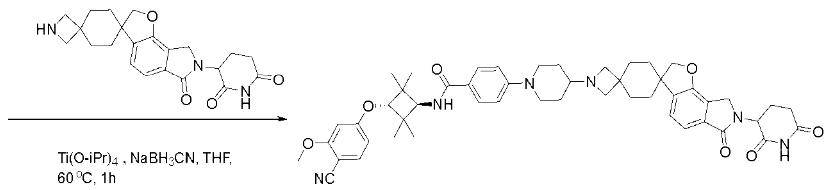


General formula AR-T-3 for PROTAC synthesis

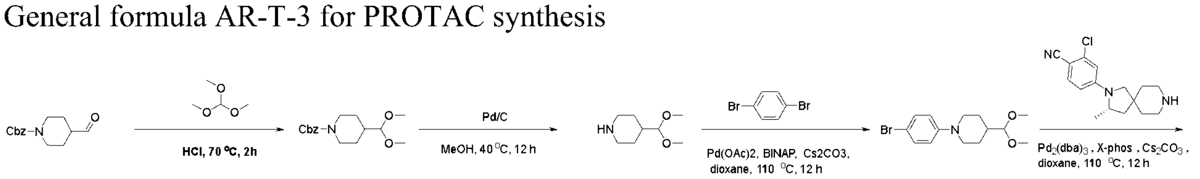


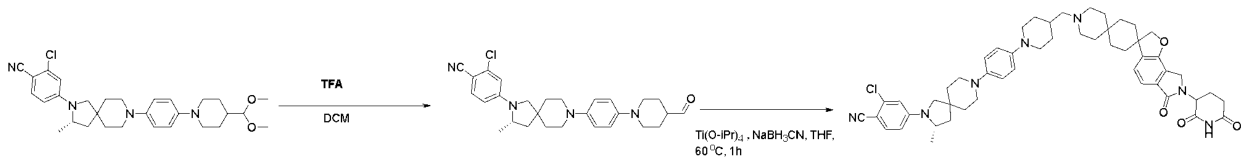


General formula AR-T-4 for PROTAC synthesis

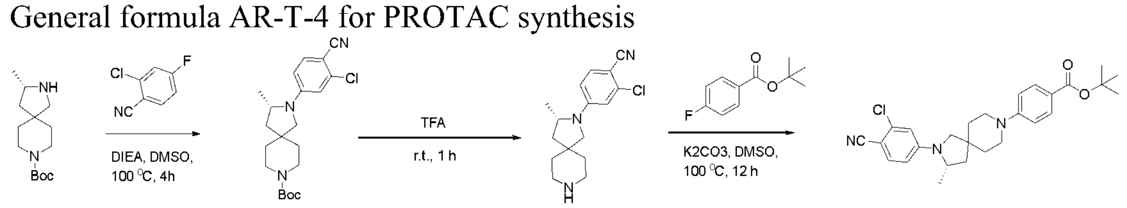


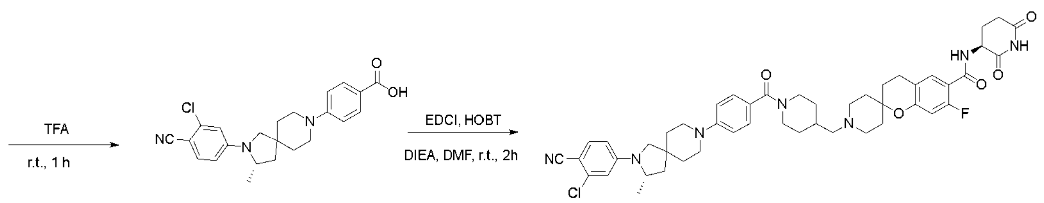

| Exam ple | Co mp oun d | Structure and Name | 1H-NMR | LC-MS | Synthesi s Method |
|---|---|---|---|---|---|
| 142 | A-P-1 | 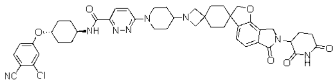 N-((1r,4r)-4-(3-chloro-4-cyanophenoxy)cyclohexy l)-6-(4-(7'-(2,6-dioxopiperidin-3-yl)-6'-oxo-7',8'-dihydro-2'-H,6'-H-dihydro[azetidine-3,1'-cyclohexane-4',3"-furo [2,3-e]isoindol]-1-yl)piperidin-1-yl) pyridazine-3-carboxamide | $^1$H NMR (600 MHz, DMSO-d6) δ 10.98 (s, 1H), 8.63 (d, *J* = 8.2 Hz, 1H), 7.88 (dd, *J* = 13.1, 9.1 Hz, 2H), 7.47 (d, *J* = 9.6 Hz, 1H), 7.39 (d, *J* = 2.6 Hz, 2H), 7.31 (d, *J* = 7.6 Hz, 1H), 7.14 (dd, *J* = 8.8, 2.4 Hz, 1H), 5.09 (dd, *J* = 13.3, 5.1 Hz, 1H), 4.63 (d, *J* = 13.2 Hz, 2H), 4.58 - 4.44 (m, 3H), 4.38 (d, *J* = 17.0 Hz, 1H), 4.22 (d, *J* = 17.0 Hz, 1H), 4.15 - 4.03 (m, 2H), 4.01 - 3.95 (m, 1H), 3.83 (s, 2H), 3.58 (d, *J*= 10.4 Hz, 1H), 3.02 (s, 2H), 2.91 (s, 1H), 2.60 (d, *J* = 13.9 Hz, 1H), 2.42 (dd, *J* = 13.2, 4.7 Hz, 2H), 2.16 - 2.08 (m, 2H), 2.06 (d, *J* = 11.7 Hz, 2H), 2.04 - 1.77 (m, 6H), 1.74 - 1.58 (m, 6H), 1.57 - 1.47 (m, 2H), 1.33 (m, 2H). | 833.5 6 | AR-T-1 |
| 143 | A-P-2 | 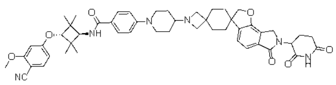 N-((1r,3r)-3-(4-cyano-3-methoxyphenoxy)-2,2,4,4-tetramethylcyclobutyl)-4-(4-(7'-(2,6-dioxopiperidin-3-yl)-6'-oxo-7',8'-dihydro-2'H,6'H-diisoazetidine-3,1'-cyclohexane-4',3"-furo[2,3-e]isoindol]-1-yl)piperidin-1-yl)benzamide | $^1$H NMR (600 MHz, DMSO-d6) δ 10.98 (s, 1H), 7.79 (d, *J* = 8.3 Hz, 2H), 7.66 (d, *J* = 8.5 Hz, 1H), 7.54 (t, *J* = 8.0 Hz, 1H), 7.40 (dd, *J*= 7.9, 3.4 Hz, 1H), 7.30 (dd, *J* = 7.6, 2.5 Hz, 1H), 7.04 (d, *J* = 8.6 Hz, 2H), 6.65 (d, *J* = 2.1 Hz, 1H), 6.55 (d, *J* = 8.7 Hz, 1H), 5.09 (dd, *J* = 13.3, 5.1 Hz, 1H), 4.38 (d, *J* = 16.9 Hz, 1H), 4.29 (s, 1H), 4.22 (d, *J* = 16.9 Hz, 1H), 4.13 (s, 2H), 4.08 - 4.01 (m, 4H), 3.99 - 3.93 (m, 2H), 3.92 (s, 2H), 3.84 (d, *J* = 8.7 Hz, 2H), 2.91 (m, 2H), 2.81 (d, *J* = 8.2 Hz, 2H), 2.59 (s, 2H), 2.43 (dd, *J* = 13.2, 4.7 Hz, 2H), 2.09 - 2.04 (m, 1H), 1.99 (m, 4H), 1.84 (t, *J* = 13.8 Hz, 1H), 1.66 (m, 5H), 1.19 (d, *J* = 44.8 Hz, 12H). | 855.6 6 | AR-T-2 |

(continued)

| Exam ple | Co mp oun d | Structure and Name | $^1$H-NMR | LC-MS | Synthesi s Method |
|---|---|---|---|---|---|
| 144 | A-P-3 | 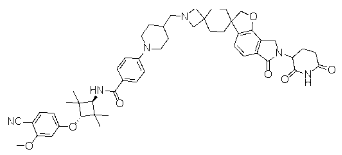 N-((1r,3r)-3-(4-cyano-3-methoxyphenoxy)-2,2,4,4-tetramethylcyclobutyl)-4-(4-((7,6-dioxopiperidin-3-yl)-6-oxo-7,8-dihydro-2,6H-dipyridyl[azetidine-3,1"-cyclohexane-4,3,3-e]isoindol]-1-yl)methyl) piperidin-1-yl)benzamide | $^1$H NMR (600 MHz, DMSO) δ 10.98 (s, 1H), 7.77 (d, *J* = 8.8 Hz, 2H), 7.66 (d, *J* = 8.6 Hz, 1H), 7.51 (d, *J* = 9.2 Hz, 1H), 7.41 (d, *J* = 7.6 Hz, 1H), 7.30 (d, *J* = 7.6 Hz, 1H), 6.99 (d, *J* = 8.9 Hz, 2H), 6.64 (d, *J* = 2.0 Hz, 1H), 6.54 (dd, *J* = 8.7, 2.1 Hz, 1H), 5.09 (dd, *J* = 13.2, 5.1 Hz, 1H), 4.54 - 4.46 (m, 2H), 4.38 (d, *J* = 17.2 Hz, 1H), 4.30 - 4.18 (m, 3H), 4.06 (d, *J* = 9.1 Hz, 1H), 3.99 - 3.80 (m, 8H), 3.19 (s, 3H), 2.95 - 2.87 (m, 2H), 2.79 (t, *J* = 11.6 Hz, 2H), 2.63 - 2.56 (m, 2H), 2.45 - 2.36 (m, 3H), 2.14 (d, *J*= 12.5 Hz, 2H), 2.04 - 1.95 (m, 3H), 1.87 - 1.57 (m, 10H), 1.34 - 1.28 (m, 2H), 1.15 (s, 4H). | 869.4 5 | AR-T-2 |
| 145 | A-P-4 | 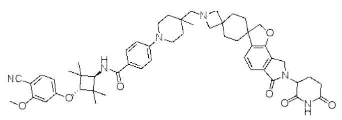 N-((1r,3r)-3-(4-cyano-3-methoxyphenoxy)-2,2,4,4-tetramethylcyclobutyl)-4-((7'-(2,6-dioxopiperidin-3-yl)-6'-oxo-7',8'-dihydro-2"H,6"H-dipyrrolyl[azetidine-3,1'-cyclohexane-4',3'-furo [2,3-e]isoindol]-1-yl)methyl)-4-methylpiperidin-1-yl)benzamide | $^1$H NMR (400 MHz, DMSO-$d_6$) δ 10.98 (s, 1H), 7.78 (d, *J* = 8.6 Hz, 2H), 7.65 (d, *J* = 8.6 Hz, 1H), 7.51 (d, *J* = 9.2 Hz, 1H), 7.42 (d, *J* = 7.6 Hz, 1H), 7.30 (d, *J* = 7.6 Hz, 1H), 7.03 - 6.94 (m, 2H), 6.64 (d, *J* = 2.2 Hz, 1H), 6.54 (dd, *J* = 8.7, 2.2 Hz, 1H), 5.09 (*dd, J* = 13.2, 5.1 Hz, 1H), 4.54 -4.44 (m, 2H), 4.38 (d,*J* = 17.1 Hz, 2H), 4.28 (s, 1H), 4.25 - 4.17 (m, 1H), 4.04 (p, *J* = 8.3 Hz, 4H), 3.90 (s, 3H), 3.59 - 3.45 (m, 3H), 3.37 - 3.23 (m, 2H), 3.14 (t, *J* = 11.1 Hz, 2H), 2.91 (ddd, *J* = 17.8, 13.8, 5.4 Hz, 1H), 2.65 - 2.56 (m, 1H), 2.41 (td, *J* = 13.1, 4.5 Hz, 1H), 2.23 (d, *J* = 13.2 Hz, 1H), 2.01 (ddd, *J* = 22.4, 11.1, 6.2 Hz, 2H), 1.78 (d, *J* = 16.0 Hz, 1H), 1.62 (d, *J* = 10.2 Hz, 6H), 1.48 (d, *J* = 12.8 Hz, 2H), 1.34 - 1.05 (m, 15H). | 883.8 6 | AR-T-2 |

(continued)

| Exam ple | Co mp oun d | Structure and Name | $^1$H-NMR | LC-MS | Synthesi s Method |
|---|---|---|---|---|---|
| 146 | A-P-5 | 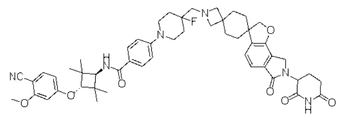 N-((1r,3r)-3-(4-cyano-3-methoxyphenoxy)-2,2,4,4-tetramethylcyclobutyl)-4-(((7'-(2,6-dioxopiperidin-3-yl)-6'-oxo-7',8'-dihydro-2'H,6'H-diisoazetidine-3,1'-cyclohexane-4',3"-furo[2,3-e]isoindol]-1-yl)methyl)-4-fluoropiperi-din-1-yl)benzamide | $^1$H NMR (600 MHz, DMSO-$d_6$) $\delta$ 10.98 (s, 1H), 7.79 (d, $J$ = 8.6 Hz, 2H), 7.65 (d, $J$ = 8.6 Hz, 1H), 7.54 (d, $J$ = 9.3 Hz, 1H), 7.43 (d, $J$ = 7.6 Hz, 1H), 7.30 (d, $J$ = 7.6 Hz, 1H), 7.05 (d, $J$ = 8.7 Hz, 2H), 6.64 (d, $J$ = 2.2 Hz, 1H), 6.55 (dd, $J$ = 8.7, 2.2 Hz, 1H), 5.08 (dd, $J$ = 13.3, 5.1 Hz, 1H), 4.49 (qd, $J$ = 9.1, 4.7 Hz, 2H), 4.36 (t, $J$ = 17.3 Hz, 2H), 4.28 (s, 1H), 4.21 (d, $J$ = 17.0 Hz, 1H), 4.05 (dd, $J$ = 15.8, 8.1 Hz, 3H), 3.91 (s, 3H), 3.79 (d, $J$ = 12.8 Hz, 2H), 3.71-3.61 (m, 2H), 3.10 (s, 2H), 2.90 (s, 1H), 2.64 - 2.56 (m, 1H), 2.43 (td, $J$ = 13.1, 4.6 Hz, 1H), 2.21 (d, $J$ = 13.2 Hz, 1H), 2.09 - 1.75 (m, 8H), 1.72 - 1.57 (m, 5H), 1.23 (s, 6H), 1.15 (s, 6H). | 887.0 7 | AR-T-2 |
| 147 | A-P-6 | 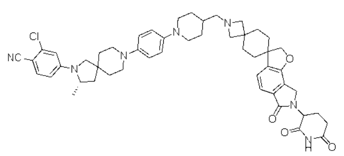 2-chloro-4-((3S)-8-(4-(4-((7'-(2,6-dioxopiperidin-3-yl)-6'-oxo-7',8'-dihydro-2'H,6'H-dipyrazole[azetidine-3,1'-cyclohexane-4',3'-furo [2,3-e]isoindol]-1-yl)methyl)piperidin-1-yl)phenyl)-3-methyl-2,8-diazaspiro[4.5]decan-2-yl)benzoni-trile | $^1$H NMR (400 MHz, DMSO-$d_6$) $\delta$ 10.98 (s, 1H), 7.60 (d, $J$ = 8.9 Hz, 1H), 7.38 (d, $J$ = 7.6 Hz, 1H), 7.28 (d, $J$ = 7.6 Hz, 1H), 6.84 (s, 4H), 6.80 (d, $J$ = 2.3 Hz, 1H), 6.66 (dd, $J$ = 9.0, 2.4 Hz, 1H), 5.09 (dd, $J$ = 13.3, 5.1 Hz, 1H), 4.54 - 4.47 (m, 2H), 4.38 (d, $J$ = 17.1 Hz, 1H), 4.21 (d, $J$ = 17.1 Hz, 1H), 4.03 (h, $J$ = 6.3 Hz, 1H), 3.49 (d, $J$ = 11.7 Hz, 4H), 3.08 (dp, $J$ = 11.0, 4.6 Hz, 3H), 2.94 (tdd, $J$ = 19.0, 17.2, 10.8, 4.3 Hz, 4H), 2.70 - 2.55 (m, 3H), 2.48 - 2.34 (m, 2H), 2.22 (dd, $J$ = 12.8, 7.7 Hz, 1H), 2.03 - 1.91 (m, 3H), 1.80 - 1.67 (m, 7H), 1.67 - 1.45 (m, 9H), 1.35 - 1.22 (m, 3H), 1.20 (d, $J$ = 6.1 Hz, 3H). | 856.6 6 | AR-T-2 |

(continued)

| Exam ple | Co mp oun d | Structure and Name | $^1$H-NMR | LC-MS | Synthesi s Method |
|---|---|---|---|---|---|
| 148 | A-P-7 | 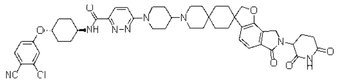 N-((1r,4r)-4-(3-chloro-4-cyanophenoxy)cyclohexy 1)-6-(4-(7-(2,6-dioxopiperidin-3-yl)-6-oxo-7,8-dihydro-2H,6H-dipyridyl[2,3-e]isoindole-3,1'-cyclohexane-4',4'''-piperidin]-1"-yl)pyridazine-3-carboxamide | $^1$H NMR (600 MHz, Methanol-$d_4$)) δ 7.17 (d, *J* = 9.6, 1.8 Hz, 1H), 6.90 (d, *J* = 9.3, 1.6 Hz, 1H), 6.61 (d, *J* = 9.1 , 1.3 Hz, 1H), 6.60 (d, J = 8.8, 0.9 Hz, 2H), 6.41 (d, *J* = 2.2 Hz, 1H), 6.25 (dd, *J* = 8.8, 2.1 Hz, 1H), 4.38 - 4.31 (m, 1H), 3.97 (d, *J* = 13.8 Hz, 2H), 3.77 (s, 2H), 3.72 (s, 1H), 3.67 - 3.56 (m, 2H), 3.20 (s, 1H), 2.80 - 2.77 (m, 1H), 2.75 - 2.63 (m, 2H), 2.42 - 2.38 (m, 2H), 2.34 - 2.26 (m, 2H), 2.11 - 2.07 (m, 1H), 2.02 - 1.95 (m, 1H), 1.78 - 1.67 (m, 2H), 1.51 - 1.48 (m, 2H), 1.42 (s, 2H), 1.36 (s, 2H), 1.31 (s, 2H), 1.22 - 1.19 (m, 1H), 1.17 - 1.07 (m, 2H), 1.00 - 0.98 (m, 2H), 0.96 (d, *J* = 8.1 Hz, 2H), 0.91 - 0.82 (m, 6H), 0.81 - 0.67 (m, 4H). | 861.5 6 | AR-T-1 |
| 149 | A-P-8 | 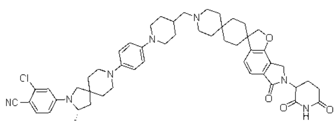 (S)-1'-((1-(4-(6-chloro-7-cyanospiro-spiro[tryptophan-3,4'-piperidin]-1'-yl) benzoyl) piperidin -4-yl) methyl)-N-(2,6-dioxopiperidin-3-yl)-7-fluoro-spiro[tryptophan-2,4'-pyridyl]-6- carboxamide | $^1$H NMR (400 MHz, DMSO-d6) δ 10.87 (s, 1H), 8.28 (dd, J = 8.1, 4.6 Hz, 1H), 7.66 - 7.38 (m, 3H), 7.27 (d, J = 8.5 Hz, 2H), 6.97 (d, J = 8.6 Hz, 2H), 6.75 (d, J = 12.0 Hz, 1H), 4.85 - 4.61 (m, 1H), 4.04 (s, 3H), 3.23 (s, 5H), 3.15 - 3.06 (m, 3H), 2.89 (s, 3H), 2.86 - 2.72 (m, 6H), 2.10 (dd, J = 12.8, 4.2 Hz, 2H), 2.04 - 1.91 (m, 5H), 1.90 - 1.73 (m, 4H), 1.64 - 1.40 (m, 5H), 1.29 - 1.13 (m, 3H). | 837.5 5 | AR-T-3 |
| 150 | A-P-9 | 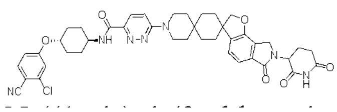 N-((1r,4r)-4-(3-chloro-4-cyanophenoxy)cyclohexy 1)-6-(7-(2,6-dioxopiperidin-3-yl)-6-oxo-7,8-dihydro-2H,6H-dipyridyl[2,3-e]isoindole-3,1'-cyclohexane-4',4'''-piperidin]-1"-yl)pyridazine-3-carboxamide | $^1$H NMR (600 MHz, Methanol-$d_4$) δ 11.79 (s, 1H), 9.41 (d, *J* = 8.1 Hz, 1H), 8.66 (t, *J* = 9.0 Hz, 2H), 8.31 *(d, J* = 7.5 Hz, 1H), 8.23 (d, *J* = 9.7 Hz, 1H), 8.20 (d, *J*= 2.4 Hz, 1H), 8.10 *(d, J* = 7.5 Hz, 1H), 7.95 (dd, *J* = 8.8, 2.4 Hz, 1H), 5.90 (m, 1H), 5.35 (q, *J* = 8.8 Hz, 4H), 5.19 (d, *J* = 16.9 Hz, 3H), 5.03 (d, *J* = 16.9 Hz, 1H), 4.56 - 4.12 (m, 4H), 3.72 - 3.61 (m, 1H), 3.44 - 3.38 (m, 1H), 3.24 - 3.16 (m, 1H), 2.96 - 2.89 (m, 2H), 2.79 - 2.77 (m, 1H), 2.75 - 2.68 (m, 4H), 2.63 - 2.54 (m, 4H). | 778.4 6 | AR-T-1 |

(continued)

| Exam ple | Co mp oun d | Structure and Name | $^1$H-NMR | LC-MS | Synthesi s Method |
|---|---|---|---|---|---|
| 151 | A-P-10 | 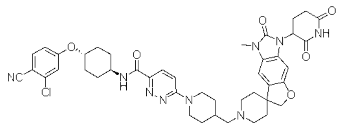 N-((1r,4r)-4-(3-chloro-4-cyanophenoxy)cyclohexy 1)-6-(4-(((3-(2,6-dioxopiperidin-3-yl)-1-methyl-2-oxo-2,3-dihydro-1H,6H-spiro[benzofuro[5,6-d]imidazole-7,4'-piperidin]-1'-yl)methyl)piperidin-1-yl) pyridazine-3-carboxamide | $^1$H NMR (600 MHz, DMSO) δ 11.08 (s, 1H), 8.59 (d, *J* = 8.2 Hz, 1H), 7.86 (t, *J* = 9.7 Hz, 2H), 7.40 (dd, *J*= 10.3, 6.0 Hz, 2H), 7.14 (dd, *J* = 8.8, 2.3 Hz, 1H), 6.84 (s, 1H), 6.75 (s, 1H), 5.32 (dd, *J* = 12.5, 5.1 Hz, 1H), 4.59 - 4.40 (m, 5H), 3.91 - 3.84 (m, 1H), 3.59 (d, *J* = 11.4 Hz, 2H), 3.33 (s, 3H), 3.12 - 3.03 (m, 5H), 2.93 - 2.84 (m, 1H), 2.77 - 2.68 (m, 1H), 2.62 (d, *J* = 16.0 Hz, 1H), 2.24 (s, 3H), 2.11 (d, *J* = 10.2 Hz, 2H), 1.93 (dt, *J* = 30.4, 10.7 Hz, 7H), 1.65 (dd, *J* = 23.9, 11.1 Hz, 2H), 1.57 - 1.48 (m, 2H), 1.31 - 1.21 (m, 3H). | 822.5 6 | AR-T-1 |
| 152 | A-P-11 | 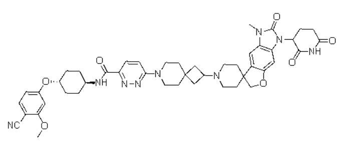 | $^1$H NMR (600 MHz, DMSO-$d_6$) δ 11.06 (s, 1H), 8.58 (d, *J* = 8.2 Hz, 1H), 7.81 (d, *J* = 9.5 Hz, 1H), 7.65 - 7.59 (m, 1H), 7.35 (d, *J* = 9.7 Hz, 1H), 7.14 (s, 1H), 6.76 - 6.68 (m, 2H), 6.66 (s, 1H), 5.29 (dd, *J* = 12.7, 5.4 Hz, 1H), 4.55 - 4.43 (m, 1H), 4.32 (d, *J* = 14.6 Hz, 2H), 3.94 - 3.80 (m, 4H), 3.68 (d, *J* = 26.4 Hz, 4H), 3.30 (s, 4H), 2.94 - 2.56 (m, 6H), 2.18 - 2.01 (m, 4H), 2.01 - 1.85 (m, 5H), 1.76 (t, *J* = 11.2 Hz, 2H), 1.71 - 1.39 (m, 12H). | 844.6 6 | AR-T-1 |
| 153 | A-P-12 | 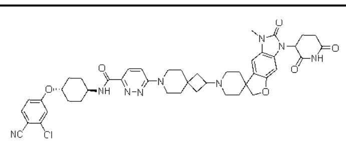 N-((1r,4r)-4-(3-chloro-4-cyanophenoxy)cyclohexy 1)-6-(2-(3-(2,6-dioxopiperidin-3-yl)-1-methyl-2-oxo-2,3-dihydro-1H,6H-spiro[benzofuro[5,6-d]imidazole-7,4'-piperidin]-1'-yl)-7-azaspiro[3.5]nonan-7-yl)pyridazine-3-carboxamide | $^1$H NMR (600 MHz, DMSO-$d_6$) δ 11.06 (s, 1H), 8.59 (d, *J* = 8.1 Hz, 1H), 7.86 (d, *J* =8.7 Hz, 1H), 7.81 (d, *J* = 9.4 Hz, 1H), 7.42 - 7.33 (m, 2H), 7.14 (d, *J* = 8.8 Hz, 1H), 7.09 (s, 1H), 6.69 (s, 1H), 5.31 (dd, J = 12.6, 4.9 Hz, 1H), 4.54 (t, *J* = 10.0 Hz, 1H), 4.38 (s, 2H), 3.86 (d, *J* = 8.1 Hz, 1H), 3.69 (d, *J* = 42.2 Hz, 4H), 3.32 (s, 4H), 3.13 - 2.78 (m, 4H), 2.76 - 2.54 (m, 3H), 2.11 (d, *J* = 10.1 Hz, 4H), 1.94 (dd, *J* = 40.3, 8.7 Hz, 6H), 1.85 - 1.69 (m, 3H), 1.69 - 1.61 (m, 4H), 1.61 - 1.45 (m, 4H). | 848.6 6 | AR-T-1 |

(continued)

| Exam ple | Co mp oun d | Structure and Name | $^1$H-NMR | LC-MS | Synthesi s Method |
|---|---|---|---|---|---|
| 154 | A-P-13 | 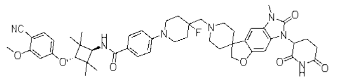 N-((1r,3r)-3-(4-cyano-3-methoxyphenoxy)-2,2,4,4-tetramethylcyclobutyl)-4-((3-(2,6-dioxopiperidin-3-yl)-1-methyl-2-oxo-2,3-dihydro-1H,6H-spiro[benzofuro[5,6-d] imidazole-7,4'-piperidin]-1'-yl) methyl)-4-fluoropiperidin-1-yl)benzamide | $^1$H NMR (400 MHz, DMSO-$d_6$) $\delta$ 10.98 (s, 1H), 7.78 (d, *J* = 8.6 Hz, 2H), 7.65 (d, *J* = 8.6 Hz, 1H), 7.51 (d, *J* = 9.2 Hz, 1H), 7.42 (d, *J* = 7.6 Hz, 1H), 7.30 (d, *J* = 7.6 Hz, 1H), 7.03 - 6.94 (m, 2H), 6.64 (d, *J* = 2.2 Hz, 1H), 6.54 (dd, *J* = 8.7, 2.2 Hz, 1H), 5.09 (dd, *J* = 13.2, 5.1 Hz, 1H), 4.54 - 4.44 (m, 2H), 4.38 (d, *J* = 17.1 Hz, 2H), 4.28 (s, 1H), 4.25 - 4.17 (m, 1H), 4.04 (p, *J* = 8.3 Hz, 4H), 3.90 (s, 3H), 3.59 - 3.45 (m, 3H), 3.37 - 3.23 (m, 2H), 3.14 (t, J= 11.1 Hz, 2H), 2.91 (ddd, *J* = 17.8, 13.8, 5.4 Hz, 1H), 2.65 - 2.56 (m, 1H), 2.41 (td, *J* = 13.1, 4.5 Hz, 1H), 2.23 (d, *J* = 13.2 Hz, 1H), 2.01 (ddd, *J* = 22.4, 11.1, 6.2 Hz, 2H), 1.78 (d, *J* = 16.0 Hz, 1H), 1.62 (d, *J* = 10.2 Hz, 6H), 1.48 (d, *J* = 12.8 Hz, 2H), 1.34 - 1.05 (m, 15H). | 883.8 6 | AR-T-2 |
| 155 | A-P-14 | 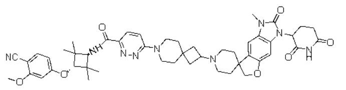 N-((1r,3r)-3-(4-cyano-3-methoxyphenoxy)-2,2,4,4-tetramethylcyclobutyl)-6-(2-(3-(2,6-dioxopiperidin-3-yl)-1-methyl-2-oxo-2,3-dihydro-1H,6H-spiro[benzofuro[5,6-d] imidazole-7,4'-piperidin]-1'-yl)-7-azaspiro[3.5]nonan-7-yl)pyridazine-3-carboxamide | $^1$H NMR (600 MHz, DMSO-$d_6$) $\delta$ 11.08 (s, 1H), 8.24 (d, *J* = 9.2 Hz, 1H), 7.86 (dd, *J* = 9.5, 3.8 Hz, 1H), 7.65 (d, *J* = 8.6 Hz, 1H), 7.43 (t, *J* = 9.4 Hz, 1H), 6.86 (d, *J* = 1.6 Hz, 1H), 6.75 (d, *J* = 2.6 Hz, 1H), 6.68 (d, *J* = 2.2 Hz, 1H), 6.58 (dd, *J* = 8.6, 2.2 Hz, 1H), 5.33 (dd, *J* = 12.8, 5.5 Hz, 1H), 4.48 (d, *J* = 2.0 Hz, 1H), 4.41 (d, *J*= 3.7 Hz, 1H), 4.01 (d, *J* = 9.1 Hz, 1H), 3.92 (s, 3H), 3.76 (s, 3H), 3.70 (t, *J* = 5.5 Hz, 3H), 3.45 (d, *J* = 11.9 Hz, 2H), 3.34 (s, 2H), 2.97 - 2.81 (*m, J* = 16.1, 8.3, 5.3 Hz, 3H), 2.78 - 2.67 (m, 1H), 2.66 - 2.57 (m, 1H), 2.37 - 2.24 (m, 2H), 2.09 (t, *J* = 6.4 Hz, 4H), 1.97 (d, *J* = 13.3 Hz, 3H), 1.81 (d, *J* = 38.6 Hz, 1H), 1.71 - 1.57 (m, *J* = 12.7, 5.7 Hz, 4H), 1.21 (d, *J*= 40.9 Hz, 12H). | 872.6 6 | AR-T-1 |

(continued)

| Exam ple | Co mp oun d | Structure and Name | $^1$H-NMR | LC-MS | Synthesi s Method |
|---|---|---|---|---|---|
| 156 | A-P-15 | 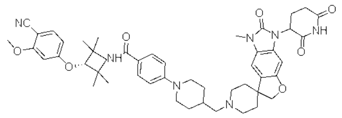 N-((1r,3r)-3-(4-cyano-3-methoxyphenoxy)-2,2,4,4-tetramethylcyclobutyl)-4-(((7'-(2,6-dioxopiperidin-3-yl)-6'-oxo-7',8'-dihydro-2'-H,6'-H-diisoazetidine-3,1'-cyclohexane-4',3"-furo[2,3-e]isoindol]-1-yl)methyl)-4-fluoropiperi-din-1-yl)benzamide | $^1$H NMR (600 MHz, DMSO-$d_6$) δ 11.08 (s, 1H), 7.78 (dd, *J* = 9.0, 2.4 Hz, 2H), 7.66 (d, *J* = 8.6 Hz, 1H), 7.52 (d, *J* = 9.2 Hz, 1H), 7.01 (dd, *J* = 9.0, 4.3 Hz, 2H), 6.84 (d, *J* = 3.2 Hz, 1H), 6.75 (d, *J* = 2.9 Hz, 1H), 6.65 (d, *J* = 2.2 Hz, 1H), 6.55 (dd, *J* = 8.6, 2.2 Hz, 1H), 5.33 (dd, *J* = 13.0, 5.4 Hz, 1H), 4.49 (d, *J* = 2.1 Hz, 2H), 4.28 (s, 1H), 4.06 (d, *J* = 9.1 Hz, 1H), 3.92 (s, 6H), 3.37 (s, 1H), 3.32 (d, *J* = 5.9 Hz, 3H), 3.14 - 3.02 (m, 4H), 2.93 - 2.80 (m, 3H), 2.73 (m, *J* = 12.9, 4.4 Hz, 1H), 2.62 (d, *J* = 17.2 Hz, 1H), 2.24 (d, *J* = 12.3 Hz, 2H), 2.09 (d, *J* = 11.7 Hz, 1H), 2.03 - 1.94 (m, 1H), 1.88 (dd, *J* = 35.8, 13.2 Hz, 4H), 1.33 (m, *J* = 12.5, 4.3 Hz, 2H), 1.19 (d, *J* = 46.5 Hz, 12H). | 844.7 6 | AR-T-2 |
| 157 | A-P-16 | 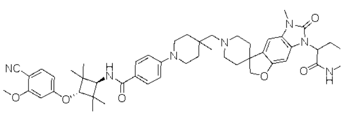 N-((1r,3r)-3-(4-cyano-3-methoxyphenoxy)-2,2,4,4-tetramethylcyclobutyl)-4-(4-(7,6-dioxopiperidin-3-yl)-6-oxo-7,8-dihydro-2,6-H-dipyridinyl[azetidine-3,1"-cyclohexane-4,3,3-e]isoindol]-1-yl) methyl) piperidin-1-yl)benzamide | $^1$H NMR (600 MHz, DMSO-d6) δ 11.07 (s, 1H), 7.78 (d, J = 8.5 Hz, 2H), 7.66 (d, J = 8.6 Hz, 1H), 7.51 (dt, J = 7.1, 3.4 Hz, 1H), 7.01 (d, J = 8.6 Hz, 2H), 6.82 (d, J = 6.7 Hz, 1H), 6.75 (d, J = 2.4 Hz, 1H), 6.65 (d, J = 2.2 Hz, 1H), 6.55 (dd, J = 8.6, 2.2 Hz, 1H), 5.32 (dd, J = 12.9, 5.4 Hz, 1H), 4.49 (d, J = 2.8 Hz, 2H), 4.29 (s, 1H), 4.06 (d, J = 9.2 Hz, 1H), 3.92 (s, 3H), 3.57 (s, 2H), 3.33 (s, 4H), 3.26 (q, J = 11.5 Hz, 2H), 3.18 - 3.12 (m, 3H), 2.88 (td, J = 17.3, 15.5, 5.4 Hz, 1H), 2.76 - 2.69 (m, 1H), 2.61 (d, J = 15.5 Hz, 1H), 2.41 (s, 2H), 2.27 (s, 1H), 2.06 - 1.80 (m, 4H), 1.71 (t, J = 11.0 Hz, 2H), 1.64 (s, 2H), 1.23 (s, 9H), 1.16 (s, 6H). | 858.7 6 | AR-T-2 |

(continued)

| Exam ple | Co mp oun d | Structure and Name | $^1$H-NMR | LC-MS | Synthesi s Method |
|---|---|---|---|---|---|
| 158 | A-P-17 | 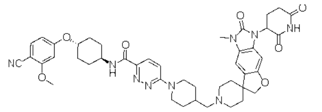 N-(1r,3r)-3-(4-cyano-3-methoxyphenoxy)2,2,4,4 -tetramethylcyclobutyl)-4-(4-((3-(2,6-dioxopiperidin-3-yl)-1-methyl-2-oxo-2,3-dihydro-1H,6H-spiro[benzofuro[5,6-d]imidazole-7,4'-piperidin]-1'-yl)methyl)piperidin-1-yl)benzamide | $^1$H NMR (600 MHz, DMSO) δ 11.08 (s, 1H), 8.59 (d, *J* = 8.2 Hz, 1H), 7.85 (d, *J* = 9.5 Hz, 1H), 7.62 (d, *J* = 9.1 Hz, 1H), 7.41 (d, *J* = 9.7 Hz, 1H), 6.84 (s, 1H), 6.77 - 6.69 (m, 3H), 5.32 (dd, *J* = 12.7, 5.2 Hz, 1H), 4.57 - 4.40 (m, 5H), 3.92 - 3.84 (m, 4H), 3.59 (d, *J* = 11.5 Hz, 2H), 3.33 (s, 3H), 3.13 - 3.03 (m, 5H), 2.93 - 2.84 (m, 1H), 2.77 - 2.69 (m, 1H), 2.62 (d, *J* = 16.4 Hz, 1H), 2.24 (s, 3H), 2.13 (d, *J* = 10.2 Hz, 2H), 1.93 (m, 7H), 1.65 (dd, *J*= 23.9, 10.8 Hz, 2H), 1.52 (q, *J* = 12.9 Hz, 2H), 1.32 - 1.21 (m, 3H). | 818.6 6 | AR-T-1 |
| 159 | A-P-18 | 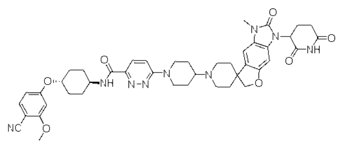 N-((1r,4r)-4-(4-cyano-3-methoxyphenoxy)cycloh exyl)-6-(4-(3-(2,6-dioxopyridyl-3-yl)-1-methyl-2-oxo-2,3-dihydro-1H,6H-spiro[benzofuro[5,6-d]imidazole-7,4'-piperidin]-1'-yl)piperidin-1-yl)pyridazine-3-carboxamide | $^1$H NMR (600 MHz, DMSO-$d_6$) δ 11.06 (s, 1H), 8.60 (d, *J* = 8.2 Hz, 1H), 7.83 (d, *J* = 9.5 Hz, 1H), 7.64 - 7.58 (m, 1H), 7.38 (d, *J* = 9.7 Hz, 1H), 7.10 (s, 1H), 6.76 - 6.69 (m, 2H), 6.66 (s, 1H), 5.29 (dd, *J* = 12.8, 5.3 Hz, 1H), 4.51 (dd, *J* = 21.0, 11.0 Hz, 3H), 4.35 (s, 2H), 3.94 - 3.78 (m, 4H), 3.30 (s, 4H), 3.09 - 2.79 (m, 5H), 2.79 - 2.54 (m, 3H), 2.31 (d, *J* = 19.0 Hz, 2H), 2.11 (d, *J* = 12.0 Hz, 2H), 2.00 - 1.83 (m, 6H), 1.73 - 1.58 (m, 4H), 1.58 - 1.41 (m, 4H). | 804.5 6 | AR-T-1 |

(continued)

| Exam ple | Co mp oun d | Structure and Name | $^1$H-NMR | LC-MS | Synthesi s Method |
|---|---|---|---|---|---|
| 160 | A-P-19 | 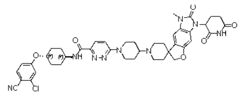 (S)-3-(1-(4-((1r,3r)-3-(4-cyano-3-methoxylphenoxy)-2,2,4,4-tetra-methylcyclobutyl) carbamoyl)phe-nyl)piperi din-4-yl)methyl)-N-((S) 2,6-dioxopiperidin-3-yl)-1,2,3,4a,5-hexahydrobenzopyrazine [1,2-d][1,4]oxazine-8-car-boxamide | $^1$H NMR (600 MHz, DMSO-$d_6$) δ 11.07 (s, 1H), 8.64 (d, $J$ = 8.2 Hz, 1H), 7.88 (dd, $J$ = 13.9, 9.1 Hz, 2H), 7.47 (d, $J$ = 9.6 Hz, 1H), 7.39 (s, 1H), 7.14 (d, $J$ = 8.8 Hz, 1H), 6.84 (s, 1H), 6.74 (s, 1H), 5.32 (dd, $J$ = 12.9, 4.9 Hz, 1H), 4.69 (d, $J$ = 12.2 Hz, 2H), 4.58 - 4.51 (m, 1H), 4.51 - 4.37 (m, 2H), 3.92 - 3.81 (m, 1H), 3.67 - 3.59 (m, 1H), 3.56 (d, $J$ = 10.8 Hz, 2H), 3.38 (s, 1H), 3.32 (s, 3H), 3.10 (dt, $J$ = 25.6, 13.9 Hz, 4H), 2.87 (t, $J$ = 12.6 Hz, 1H), 2.77 - 2.67 (m, 1H), 2.61 (d, $J$ = 15.0 Hz, 1H), 2.19 (d, $J$ = 10.1 Hz, 3H), 2.12 (d, $J$ = 10.6 Hz, 2H), 2.01 - 1.83 (m, 5H), 1.74 - 1.61 (m, 4H), 1.53 (q, $J$ = 22.9, 10.4 Hz, 2H). | 808.5 6 | AR-T-1 |
| 161 | A-P-20 | 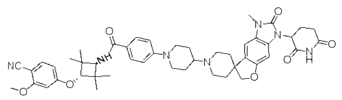 N-((1r,4r)-4-(4-cyano-3-methoxyphenoxy)cycloh exyl)-6-(4-(3-(2,6-dioxopiperidin-3-yl)-1-methyl-2-oxo-2,3-dihydro-1H,7H-spiro[benzofuro[6,7-d]imidazole-6,4'-piperidin]-1'-yl)methyl)piperidin-1-yl) pyridazine-3-carboxamide | $^1$H NMR (600 MHz, DMSO-$d_6$) δ 11.08 (s, 1H), 9.39 (d, $J$ = 30.1 Hz, 1H), 7.80 (dd, $J$ = 9.1, 4.3 Hz, 2H), 7.70 - 7.63 (m, 1H), 7.56 (dd, $J$ = 15.7, 9.0 Hz, 1H), 7.05 (d, $J$ = 8.7 Hz, 1H), 6.84 (s, 1H), 6.78 - 6.72 (m, 1H), 6.68 - 6.63 (m, 1H), 6.59 - 6.52 (m, 1H), 5.32 (dd, $J$ = 12.8, 5.5 Hz, 1H), 4.48 (d, $J$ = 20.0 Hz, 2H), 4.29 (s, 1H), 4.12 - 4.04 (m, 3H), 3.92 (s, 4H), 3.38 (s, 1H), 3.33 (d, $J$ = 5.4 Hz, 3H), 3.11 (m, $J$ = 11.9 Hz, 2H), 2.85 (m, $J$ = 13.1 Hz, 3H), 2.72 (m, $J$ = 12.8, 4.5 Hz, 1H), 2.61 (d, $J$ = 16.2 Hz, 1H), 2.30 - 2.09 (m, 4H), 2.01 - 1.91 (m, 3H), 1.85 (m, $J$ = 16.2 Hz, 1H), 1.80 - 1.66 (m, 2H), 1.20 (d, $J$ = 45.9 Hz, 12H). | 830.7 6 | AR-T-2 |

(continued)

| Exam ple | Co mp oun d | Structure and Name | $^1$H-NMR | LC-MS | Synthesi s Method |
|---|---|---|---|---|---|
| 162 | A-P-21 | 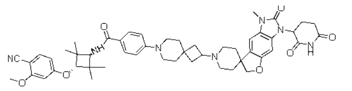 N-(1r,4r)-4-(3-chloro-4-cyanophenoxy)cyclohexy 1)-6-(4-(3-(2,6-dioxopiperidin-3-yl))-1-methyl-2-oxo-2,3-dihydro-1H,7H-spiro[benzofuro[6,7-d]imidazole-6,4'-piperidin]-1'-yl)piperidin-1-yl)pyridazine-3-carboxamide | $^1$H NMR (600 MHz, DMSO-$d_6$) δ 11.08 (s, 1H), 7.76 (d, *J* = 8.4 Hz, 2H), 7.66 (d, *J* = 8.6 Hz, 1H), 7.52 (d, *J* = 9.2 Hz, 1H), 6.99 (d, J = 8.4 Hz, 2H), 6.86 (s, 1H), 6.75 (s, 1H), 6.64 (d, *J* = 2.2 Hz, 1H), 6.54 (dd, *J* = 8.6, 2.2 Hz, 1H), 5.32 (dd, J = 13.0, 5.4 Hz, 1H), 4.48 (s, 1H), 4.28 (s, 1H), 4.06 (d, *J* = 9.1 Hz, 1H), 3.91 (s, 3H), 3.75 (m, *J* = 8.3 Hz, 1H), 3.33 (d, *J* = 19.5 Hz, 5H), 3.28 - 3.17 (m, 3H), 2.97 - 2.81 (m, 3H), 2.72 (m, *J* = 12.9, 4.4 Hz, 1H), 2.62 *(d, J= 17.2* Hz, 1H), 2.33 - 2.20 (m, 2H), 2.08 (m, *J*= 42.5, 11.5 Hz, 6H), 1.96 (d, *J* = 13.9 Hz, 3H), 1.87 - 1.74 (m, 1H), 1.66 (d, *J* = 9.6 Hz, 4H), 1.19 (d, *J* = 46.2 Hz, 12H). | 870.7 6 | AR-T-2 |
| 163 | A-P-22 | 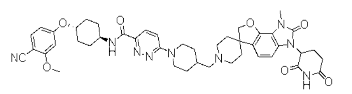 N-(1r,4r)-4-(3-chloro-4-cyanophenoxy)cyclohexy 1)-6-(4-(3-((2,6-dioxopiperidin-3-yl))-1-methyl-2-oxo-2,3-dihydro-1H,7H-spiro[benzofuro[6,7-d]imidazole-6,4'-piperidin]-1'-yl)methyl)piperidin-1-yl) pyridazine-3-carboxamide | $^1$H NMR (600 MHz, DMSO) δ 11.11 (s, 1H), 8.59 (d, *J* = 8.2 Hz, 1H), 7.85 (*d, J* = 9.6 Hz, 1H), 7.62 (d, *J* = 9.2 Hz, 1H), 7.41 (d, *J* = 9.7 Hz, 1H), 6.78 (d, *J* = 8.0 Hz, 1H), 6.74 - 6.69 (m, 3H), 5.34 (dd, *J* = 12.6, 5.3 Hz, 1H), 4.64 (s, 2H), 4.58 - 4.45 (m, 3H), 3.90 (s, 3H), 3.87 (dd, *J* = 7.8, 3.7 Hz, 1H), 3.46 (s, 3H), 3.45 (d, *J* = 5.2 Hz, 1H), 3.07 (dd, *J* = 14.9, 9.2 Hz, 6H), 2.90 (dd, *J* = 20.9, 9.5 Hz, 1H), 2.71 - 2.58 (m, 2H), 2.21 (s, 3H), 2.13 (d, *J* = 10.1 Hz, 2H), 2.03 - 1.96 (m, 1H), 1.91 (t, *J* = 21.9 Hz, 6H), 1.65 (dd, *J* = 23.9, 10.7 Hz, 2H), 1.52 (dd, *J* = 23.0, 9.9 Hz, 2H), 1.26 (dd, *J* = 18.7, 9.6 Hz, 3H). | 818.6 6 | AR-T-1 |

(continued)

| Exam ple | Co mp oun d | Structure and Name | $^1$H-NMR | LC-MS | Synthesi s Method |
|---|---|---|---|---|---|
| 164 | A-P-23 | 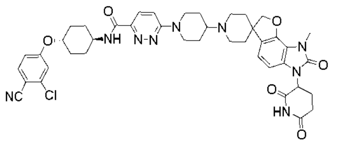 N-(1r,4r)-4-(3-chloro-4-cyanophenoxy)cyclohexy 1)-6-(4-(3-((2,6-dioxopiperidin-3-yl))-1-methyl-2-oxo-2,3-dihydro-1H,7H-spiro[benzofuro[6,7-d]imidazole-6,4'-piperidin]-1'-yl)piperidin-1-yl)pyrida-zine-3-carboxamide | $^1$H NMR (600 MHz, DMSO) δ 11.10 (s, 1H), 8.63 (d, *J* = 8.2 Hz, 1H), 7.88 (dd, *J* = 14.4, 9.1 Hz, 2H), 7.48 (t, *J* = 9.7 Hz, 1H), 7.39 (d, *J* = 2.4 Hz, 1H), 7.19 - 7.07 (m, 1H), 6.77 (d, *J* = 7.9 Hz, 1H), 6.70 (s, 1H), 5.34 (dd, *J* = 12.7, 5.2 Hz, 1H), 4.69 (d, *J* = 12.3 Hz, 2H), 4.64 (s, 2H), 4.58 - 4.51 (m, 2H), 3.91 - 3.83 (m, 4H), 3.17 - 3.02 (m, 4H), 2.90 (s, 1H), 2.70 - 2.59 (m, 2H), 2.17 (d, *J* = 16.4 Hz, 3H), 2.12 (d, *J* = 11.7 Hz, 3H), 1.98 (dd, *J* = 19.4, 8.4 Hz, 3H), 1.91 (d, *J* = 10.4 Hz, 2H), 1.67 (dt, *J* = 24.4, 8.2 Hz, 4H), 1.53 (dd, *J* = 23.0, 10.0 Hz, 2H), 1.24 (s, 2H). | 808.5 6 | AR-T-1 |
| 165 | A-P-24 | 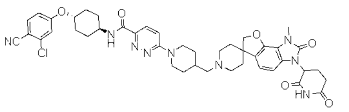 N-(1r,4r)-4-(3-chloro-4-cyanophenoxy)cyclohexy 1)-6-(4-(3-(2,6-dioxopiperidin-3-yl))-1-methyl-2-oxo-2,3-dihydro-1H,7H-spiro[benzofuro[6,7-d]imidazole-6,4'-piperidin]-1'-yl)methyl)piperidin-1-yl) pyridazine-3-carboxamide | $^1$H NMR (600 MHz, DMSO) δ 11.11 (s, 1H), 8.59 (d, *J* = 8.2 Hz, 1H), 7.85 (dd, *J* = 12.4, 9.2 Hz, 2H), 7.40 (t, *J* = 6.7 Hz, 2H), 7.14 (dd, *J* = 8.8, 2.4 Hz, 1H), 6.78 (d, *J* = 8.0 Hz, 1H), 6.71 (d, *J* = 7.7 Hz, 1H), 5.34 (dd, *J* = 12.4, 5.1 Hz, 1H), 4.64 (s, 1H), 4.57 - 4.48 (m, 3H), 3.59 (d, *J* = 11.3 Hz, 3H), 3.46 (s, 3H), 3.07 (dd, *J* = 15.1, 8.7 Hz, 5H), 2.90 (dd, *J* = 20.6, 9.6 Hz, 1H), 2.72 - 2.59 (m, 2H), 2.22 (s, 3H), 2.11 (d, *J* = 10.4 Hz, 2H), 2.04 - 1.96 (m, 2H), 1.90 (dd, *J* = 27.0, 13.7 Hz, 6H), 1.65 (dd, *J* = 24.0, 10.7 Hz, 2H), 1.52 (dd, *J* = 22.8, 10.0 Hz, 2H), 1.26 (dd, *J* = 20.7, 9.1 Hz, 3H). | 822.5 6 | AR-T-1 |

(continued)

| Exam ple | Co mp oun d | Structure and Name | $^1$H-NMR | LC-MS | Synthesi s Method |
|---|---|---|---|---|---|
| 166 | A-P-25 | 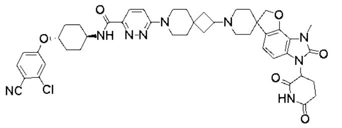 N-(1r,4r)-4-(3-chloro-4-cyanophenoxy)cyclohexyl)-6-(2-(2,6-dioxopiperidin-3-yl))-1-methyl-2-oxo-2,3-dihydro-1H,7H-spiro[benzofuro[6,7-d]imidazole-6,4'-piperidin]-1'-yl)-7-azaspiro[3.5]nonan-7-yl)pyridazine-3-carboxamide | $^1$H NMR (600 MHz, DMSO) δ 11.10 (s, 1H), 8.58 (d, *J* = 8.1 Hz, 1H), 7.86 (d, *J* = 8.8 Hz, 1H), 7.83 (d, *J* = 9.5 Hz, 1H), 7.41 - 7.36 (m, 2H), 7.13 (dd, *J* = 8.8, 2.4 Hz, 1H), 6.78 (d, *J* = 8.0 Hz, 1H), 6.70 (d, *J* = 7.8 Hz, 1H), 5.34 (dd, *J* = 12.4, 5.1 Hz, 1H), 4.63 (s, 2H), 4.58 - 4.50 (m, 2H), 3.44 (dd, *J* = 10.8, 7.4 Hz, 6H), 2.90 (dd, *J* = 21.7, 12.3 Hz, 3H), 2.70 - 2.59 (m, 2H), 2.27 (t, *J* = 9.4 Hz, 2H), 2.13 - 2.04 (m, 7H), 1.98 (d, *J* = 11.9 Hz, 3H), 1.90 (d, *J* = 10.7 Hz, 2H), 1.64 (d, *J* = 13.9 Hz, 7H), 1.52 (dd, *J* = 22.9, 10.0 Hz, 3H), 1.23 (s, 1H). | 848.6 6 | AR-T-1 |
| 167 | A-P-26 | 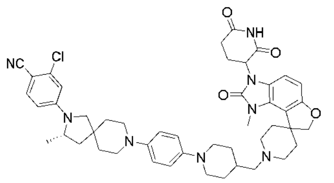 2-chloro-4-((3 S)-8-(4-(4-((3-((2,6-dioxopiperidin-3-yl))-1-methyl-2-oxo-2,3-dihydro-1H,7H-spiro[benzofuro[4,5-d]imidazole-8,4'-piperidin]-1'-yl)methyl)piperidin-1-yl) phenyl)-3-methyl-2,8-diazaspiro [4.5]decan-2-yl)benzonitrile | $^1$H NMR (400 MHz, DMSO-$d_6$) δ 11.10 (s, 1H), 7.63 (d, *J* = 8.8 Hz, 1H), 7.55 - 7.02 (m, 4H), 6.97 (d, *J* = 8.5 Hz, 1H), 6.82 (d, *J* = 2.3 Hz, 1H), 6.68 (dd, *J* = 9.0, 2.3 Hz, 1H), 6.58 (d, *J* = 8.4 Hz, 1H), 5.36 (dd, *J* = 12.8, 5.5 Hz, 1H), 4.54 (s, 2H), 4.07 (q, *J* = 6.6 Hz, 1H), 3.80 - 3.70 (m, 2H), 3.61 (s, 6H), 3.41 - 3.30 (m, 2H), 3.21 - 3.04 (m, 6H), 2.89 (ddd, *J* = 17.6, 13.4, 5.5 Hz, 2H), 2.70 (td, *J* = 13.0, 4.6 Hz, 1H), 2.66 - 2.60 (m, 1H), 2.59 - 2.52 (m, 2H), 2.18 - 2.05 (m, 3H), 2.05 - 1.77 (m, 6H), 1.76 - 1.55 (m, 3H), 1.42 (d, *J* = 31.6 Hz, 2H), 1.31 - 1.12 (m, 5H). | 831.6 6 | AR-T-3 |

(continued)

| Exam ple | Co mp oun d | Structure and Name | 1H-NMR | LC-MS | Synthesi s Method |
|---|---|---|---|---|---|
| 168 | A-P-27 | 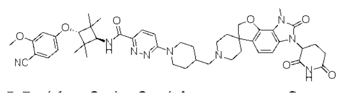<br>N-(1r,3r)-3-(4-cyano-3-methoxyphenoxy)-2,2,4,4-tetramethylcyclobutyl)-6-(4-(3-((2,6-dioxopiperidin-3-yl))-1-methyl-2-oxo-2,3-dihydro-1H,7H-spiro[benzofuro[6,7-d]imidazole-6,4'-piperidin]-1'-yl)methyl)piperidin-1-yl)pyridazine-3-carboxamide | 1H NMR (600 MHz, Methanol-d4) δ 7.93 (d, J = 9.6 Hz, 1H), 7.55 (d, J = 8.6 Hz, 1H), 7.35 (d, J = 9.7 Hz, 1H), 6.94 (d, J = 8.0 Hz, 1H), 6.68 (d, J = 8.1 Hz, 1H), 6.66 (d, J = 2.2 Hz, 1H), 6.59 (dd, J = 8.6, 2.2 Hz, 1H), 5.30 (dd, J = 12.6, 5.5 Hz, 1H), 4.63 - 4.51 (m, 4H), 4.33 (d, J = 1.0 Hz, 1H), 4.12 (s, 1H), 3.96 (s, 3H), 3.58 (s, 3H), 3.14 (t, 4H), 2.98 - 2.74 (m, 4H), 2.51 (s, 2H), 2.36 (s, 2H), 2.22 - 2.14 (m, 1H), 2.14 - 2.04 (m, 4H), 1.99 (d, J = 13.1 Hz, 2H), 1.84 (d, J = 13.4 Hz, 2H), 1.33 (s, 6H), 1.26 (s, 6H). | 846.7 6 | AR-T-1 |
| 169 | A-P-28 | 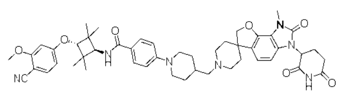<br>N-(1r,3r)-3-(4-cyano-3-methoxyphenoxy)-2,2,4,4-tetramethylcyclobutyl)-4-(4-(3-(2,6-dioxopiperidin-3-yl))-1-methyl-2-oxo-2,3-dihydro-1H,7H-spiro[benzofuro[6,7-d]imidazole-6,4'-piperidin]-1'-yl)methyl)piperidin-1-yl)benzamide | 1H NMR (600 MHz, Methanol-d4) δ 7.78 (d, J = 8.8 Hz, 2H), 7.54 (d, J = 8.6 Hz, 1H), 7.07 (d, J = 8.9 Hz, 2H), 6.93 (d, J = 8.0 Hz, 1H), 6.74 (d, J = 8.0 Hz, 1H), 6.65 (d, J = 2.2 Hz, 1H), 6.58 (dd, J = 8.6, 2.2 Hz, 1H), 5.32 (dd, J = 12.8, 5.6 Hz, 1H), 4.70 (s, 2H), 4.29 (s, 1H), 4.15 (s, 1H), 3.98 (d, J = 13.0 Hz, 2H), 3.95 (s, 3H), 3.73 (d, J = 12.8 Hz, 2H), 3.58 (s, 3H), 3.21 - 3.19 (m, 1H), 3.16 (d, J = 7.5 Hz, 2H), 2.99 - 2.93 (m, 2H), 2.93 - 2.89 (m, 1H), 2.85 - 2.76 (m, 2H), 2.29 (t, J = 14.4 Hz, 2H), 2.23 - 2.15 (m, 2H), 2.12 - 2.06 (m, 2H), 1.97 (d, J = 12.9 Hz, 2H), 1.56 - 1.47 (m, 2H), 1.38 - 1.34 (m, 1H), 1.31 (s, 6H), 1.26 (s, 6H). | 844.7 6 | AR-T-2 |

(continued)

| Exam ple | Co mp oun d | Structure and Name | $^1$H-NMR | LC-MS | Synthesi s Method |
|---|---|---|---|---|---|
| 170 | A-P-29 | 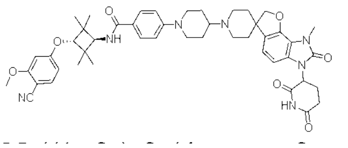 N-((1r,3r)-3-(4-cyano-3-methoxyphenoxy)-2,2,4,4-tetramethylcyclobutyl)-4-(4-(3-(2,6-dioxopiperidin-3-yl))-1-methyl-2-oxo-2,3-dihydro-1H,7H-spiro[benzofuro[6,7-d]imidazole-6,4'-piperidin]-1'-yl)pi-peridin-1-yl)benzamide | $^1$H NMR (600 MHz, DMSO-$d_6$) δ 10.85 (s, 1H), 8.26 (dd, J = 8.0, 4.6 Hz, 1H), 7.78 (d, J = 8.6 Hz, 2H), 7.65 (d, J = 8.6 Hz, 1H), 7.53 (dd, J = 20.9, 8.8 Hz, 2H), 7.01 (d, J = 9.0 Hz, 1H), 6.77 (dd, J = 12.1, 6.4 Hz, 1H), 6.64 (d, J = 2.3 Hz, 1H), 6.54 (dd, J = 8.7, 2.2 Hz, 1H), 4.74 (t, J = 6.3 Hz, 1H), 4.28 (s, 1H), 4.05 (d, J = 9.2 Hz, 2H), 3.91 (s, 3H), 3.65 (t, J = 6.5 Hz, 3H), 3.53 (d, J = 11.9 Hz, 2H), 3.46 (d, J = 6.7 Hz, 1H), 3.42 (d, J = 4.6 Hz, 1H), 3.32 (d, J = 11.9 Hz, 1H), 3.26 (s, 3H), 3.08 (t, J = 11.1 Hz, 2H), 2.84 - 2.73 (m, 3H), 2.14 - 2.03 (m, 3H), 2.01 - 1.91 (m, 6H), 1.84 (t, J = 6.7 Hz, 1H), 1.67 (s, 2H), 1.22 (s, 6H), 1.15 (s, 6H). | 879.7 6 | AR-T-2 |
| 171 | A-P-30 | 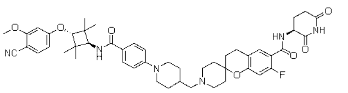 1'-((1-(4-((1r,3r)-3-(4-cyano-3-methoxyphenoxy)-2,2,4,4-tetra-methylcyclobutyl)ca rbamoyl)phe-nyl)piperidi n-4-yl) methyl)-N-((S)-2,6-dioxopiperidin-3-yl)-7-fluorospiro[2,4'-piperi-din]-6-carboxamide | $^1$H NMR (600 MHz, DMSO-$d_6$) δ 10.86 (s, 1H), 8.27 (td, *J* = 8.4, 4.5 Hz, 1H), 7.77 (d, *J* = 8.6 Hz, 2H), 7.66 (d, *J* = 8.6 Hz, 1H), 7.59 - 7.50 (m, 2H), 7.00 (d, *J* = 8.7 Hz, 2H), 6.77 (d, *J* = 11.9 Hz, 1H), 6.64 (d, *J* = 2.2 Hz, 1H), 6.54 (dd, *J* = 8.6, 2.2 Hz, 1H), 4.75 (ddd, *J* = 12.8, 8.0, 5.3 Hz, 1H), 4.28 (s, 1H), 4.06 (d, *J* = 9.2 Hz, 1H), 3.91 (s, 5H), 3.50 (d, *J* = 12.1 Hz, 3H), 3.25 - 3.07 (m, 4H), 2.88 - 2.72 (m, 5H), 2.54 (d, *J* = 3.9 Hz, 1H), 2.16 - 1.92 (m, 7H), 1.86 (dt, *J* = 15.5, 8.8 Hz, 3H), 1.36 - 1.27 (m, 2H), 1.19 (d, *J* = 46.2 Hz, 12H). | 849.6 6 | AR-T-2 |

(continued)

| Exam ple | Co mp oun d | Structure and Name | 1H-NMR | LC-MS | Synthesi s Method |
|---|---|---|---|---|---|
| 172 | A-P-31 | 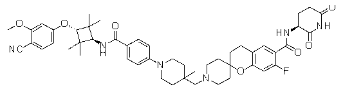 1'-((1-((4-((1r,3r)-3-(4-cyano-3-methoxyphenoxy)-2,2,4,4-tetra-methylcyclobutyl)carbamoyl)phe-nyl)-4-methylpiperidin-4-yl) methyl)-N-((S)-2,6-dioxopiperidin-3-yl)-7-fluorospiro[2,4'-piperi-din]-6-carboxamide | $^1$H NMR (600 MHz, DMSO-$d_6$) δ 10.86 (s, 1H), 8.28 (dd, *J* = 8.1, 4.3 Hz, 1H), 7.78 (d, *J* = 8.6 Hz, 2H), 7.65 (d, *J* = 8.6 Hz, 1H), 7.58 - 7.48 (m, 2H), 7.00 (d, *J* = 8.7 Hz, 2H), 6.78 (dd, *J*= 12.1, 8.6 Hz, 1H), 6.64 (d, *J*= 2.2 Hz, 1H), 6.54 (dd, *J*= 8.6, 2.2 Hz, 1H), 4.75 (ddd, *J* = 12.9, 8.0, 5.4 Hz, 1H), 4.28 (s, 1H), 4.06 (d, *J* = 9.2 Hz, 1H), 3.91 (s, 3H), 3.48 (d, *J* = 12.4 Hz, 3H), 3.34 (d, *J* = 12.0 Hz, 2H), 3.27 - 3.08 (m, 5H), 2.78 (dtd, *J* = 15.9, 8.2, 3.7 Hz, 3H), 2.54 (d, *J* = 3.9 Hz, 1H), 2.10 (ddd, *J* = 14.7, 9.9, 5.7 Hz, 3H), 2.05 - 1.89 (m, 4H), 1.85 (t, *J* = 6.8 Hz, 1H), 1.68 (q, *J* = 7.1, 4.2 Hz, 2H), 1.60 (d, *J* = 12.9 Hz, 2H), 1.27 - 1.11 (m, 15H). | 863.7 6 | AR-T-2 |
| 173 | A-P-32 | 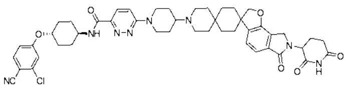 N-(1r,4r)-4-(3-chloro-4-cyanophenoxy)cyclohexy 1)-6-(4-(7-(2,6-dioxopiperidin-3-yl))-6-oxo-7,8-dihydro-2H,6H-dipyrido[2,3-e]isoindole-3,1'-cyclohexane-4',4'''-piperidin]-1''-yl)pyridazine-3-carboxa-mide | $^1$H NMR (600 MHz, DMSO-$d_6$) δ 10.86 (s, 1H), 8.38 (d, *J* = 2.8 Hz, 1H), 8.28 (dd, *J* = 8.2, 4.5 Hz, 1H), 8.10 (d, *J* = 9.1 Hz, 1H), 7.86 *(d, J* = 8.8 Hz, 1H), 7.65 (d, *J* = 8.6 Hz, 1H), 7.55 (d, *J* = 8.3 Hz, 1H), 7.46 (dd, *J* = 8.9, 2.8 Hz, 1H), 6.77 (t, *J* = 10.7 Hz, 1H), 6.67 (d, *J* = 2.2 Hz, 1H), 6.57 (dd, *J* = 8.7, 2.2 Hz, 1H), 4.75 (ddd, *J* = 13.0, 8.1, 5.5 Hz, 1H), 4.38 (s, 1H), 4.00 (d, *J* = 12.9 Hz, 2H), 3.95 (d, *J* = 9.0 Hz, 1H), 3.49 (s, 2H), 3.14 (d, *J* = 41.4 Hz, 5H), 2.91 (t, *J* = 12.3 Hz, 2H), 2.79 (dq, *J* = 15.4, 5.7, 5.3 Hz, 3H), 2.11 (qd, *J* = 12.3, 11.9, 4.5 Hz, 3H), 2.05 - 1.91 (m, 5H), 1.86 (q, *J* = 9.6, 8.4 Hz, 4H), 1.31 (q, *J* = 14.3, 13.8 Hz, 2H), 1.25 (d, *J* = 7.4 Hz, 2H), 1.21 (s, 6H), 1.15 (s, 6H). | 850.7 8 | AR-T-1 |

(continued)

| Exam ple | Co mp oun d | Structure and Name | ¹H-NMR | LC-MS | Synthesi s Method |
|---|---|---|---|---|---|
| 174 | A-P-33 | 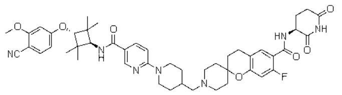 1'-((1-(5-(((1R,3R)-3-(4-cyano-3-methoxyphenoxy)-2,2,4,4-tetramethylcyclobutyl)ca rbamoyl)pyridin-2-yl)piperidin-4-yl) methyl)-N-((S)-2,6-dioxopiperidin-3-yl)-7-fluorospiro[chroman-2,4'-piperidin]-6-carboxamide | $^1$H NMR (600 MHz, DMSO-$d_6$) δ 10.86 (s, 1H), 8.62 (d, *J* = 2.2 Hz, 1H), 8.23 (q, *J* = 4.5 Hz, 1H), 7.93 (dd, *J* = 9.0, 2.5 Hz, 1H), 7.65 (d, *J* = 8.6 Hz, 1H), 7.60 (d, *J* = 9.3 Hz, 1H), 7.51 (d, *J* = 8.5 Hz, 1H), 6.85 (*d, J* = 9.1 Hz, 1H), 6.69 (*d, J* = 12.2 Hz, 1H), 6.64 (d, *J* = 2.2 Hz, 1H), 6.54 (dd, *J* = 8.6, 2.2 Hz, 1H), 4.74 (ddd, *J* = 12.8, 8.1, 5.4 Hz, 1H), 4.41 (d, *J* = 13.2 Hz, 2H), 4.26 (s, 1H), 4.05 (d, *J* = 9.2 Hz, 1H), 3.91 (s, 3H), 2.93 - 2.85 (m, 2H), 2.82 - 2.71 (m, 3H), 2.61 - 2.53 (m, 2H), 2.33 (t, *J* = 10.7 Hz, 2H), 2.19 (d, *J* = 7.1 Hz, 2H), 2.10 (qd, *J* = 12.9, 4.5 Hz, 1H), 2.00 (tdd, *J* = 8.5, 5.5, 2.9 Hz, 2H), 1.81 (p, *J* = 13.5, 12.3 Hz, 5H), 1.75 - 1.59 (m, 4H), 1.23 (s, 6H), 1.14 (s, 6H), 1.06 (qd, *J* = 12.7, 4.0 Hz, 2H). | 850.6 0 | AR-T-1 |
| 175 | A-P-34 | 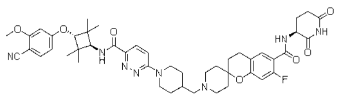 1'-((1-(6-(((1R,3R)-3-(4-cyano-3-methoxyphenoxy)-2,2,4,4-tetramethylcyclobutyl)ca rbamoyl)pyridazin-3-yl)piperidin-4-yl) methyl)-N-((S)-2,6-dioxopiperidin-3-yl)-7-fluorospiro[chroman-2,4'-piperidin]-6-carboxamide | $^1$H NMR (400 MHz, DMSO-$d_6$) δ 10.87 (s, 1H), 8.33 - 8.20 (m, 2H), 7.87 (d, *J* = 9.5 Hz, 1H), 7.65 (d, *J* = 8.6 Hz, 1H), 7.55 (dd, *J* = 8.6, 3.2 Hz, 1H), 7.43 (d, *J* = 9.7 Hz, 1H), 6.78 (dd, *J* = 12.2, 9.0 Hz, 1H), 6.67 (d, *J* = 2.2 Hz, 1H), 6.57 (dd, *J* = 8.6, 2.2 Hz, 1H), 4.75 (ddd, *J* = 12.8, 7.9, 5.5 Hz, 1H), 4.55 (d, *J* = 13.1 Hz, 2H), 4.41 (s, 1H), 4.01 (d, *J* = 9.2 Hz, 3H), 3.50 (d, *J* = 12.1 Hz, 3H), 3.28 - 3.01 (m, 7H), 2.88 - 2.72 (m, 3H), 2.58 - 2.52 (m, 1H), 2.24 (s, 1H), 2.11 (dd, *J* = 12.8, 4.3 Hz, 1H), 2.05 - 1.93 (m, 5H), 1.93 - 1.81 (m, 4H), 1.29 (d, *J* = 12.5 Hz, 1H), 1.24 (s, 6H), 1.17 (s, 6H). | 851.6 6 | AR-T-1 |

(continued)

| Exam ple | Co mp oun d | Structure and Name | 1H-NMR | LC-MS | Synthesi s Method |
|---|---|---|---|---|---|
| 176 | A-P-35 | 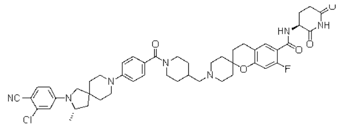 1'-((1-(4-((S)-2-(3-chloro-4-cyanophenyl)-3-methyl-2,8-diazaspiro [4.5]decan-8-yl)benzoyl)piperidin-4-yl)methyl)-N-((S)-2,6-dioxopiperidin-3-yl)-7-fluorospiro[chroman-2,4'-piperidin]-6-carboxa-mide | $^1$H NMR (400 MHz, DMSO) δ 10.86 (s, 1H), 8.28 (dd, *J* = 7.8, 4.6 Hz, 1H), 7.60 (d, *J* = 8.9 Hz, 1H), 7.55 (d, *J* = 8.4 Hz, 1H), 7.26 (d, *J* = 8.7 Hz, 2H), 6.97 (d, *J* = 8.7 Hz, 2H), 6.80 (d, *J* = 2.2 Hz, 1H), 6.78 - 6.72 (m, 1H), 6.66 (dd, *J* = 9.0, 2.2 Hz, 1H), 4.81 - 4.68 (m, 1H), 4.04 (dd, *J* = 13.1, 6.5 Hz, 2H), 3.51 - 3.42 (m, 3H), 3.34 (t, *J* = 9.5 Hz, 4H), 3.27 - 3.14 (m, 4H), 3.10 (s, 2H), 2.94 (s, 2H), 2.84 - 2.75 (m, 3H), 2.24 (dd, *J* = 12.7, 7.8 Hz, 1H), 2.18 - 2.07 (m, 2H), 1.99 (dd, J = 13.5, 7.9 Hz, 5H), 1.86 (t, *J* = 6.4 Hz, 2H), 1.82 - 1.68 (m, 4H), 1.59 (dd, *J* = 12.8, 6.5 Hz, 1H), 1.51 (s, 2H), 1.24 (s, 1H), 1.21 (d, *J* = 6.0 Hz, 3H), 1.18 (s, 2H). | 864.5 6 | AR-T-4 |
| 177 | A-P-36 | 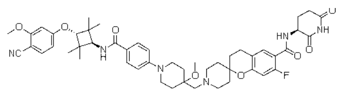 1'-((1-(4-(((1r,3r)-3-(4-cyano-3-methoxyphenoxy)-2,2,4,4-tetra-methylcyclobutyl)ca rbamoyl)phe-nyl)-4-methoxypiperidin-4-yl) methyl)-N-((S)-2,6-dioxopyrimidin-3-yl)-7-fluorospiro[chroman-2,4'-piperidin]-6-carboxa-mide | $^1$H NMR (600 MHz, DMSO-$d_6$) δ 10.85 (s, 1H), 8.26 (dd, J = 8.0, 4.6 Hz, 1H), 7.78 (d, J = 8.6 Hz, 2H), 7.65 (d, J = 8.6 Hz, 1H), 7.53 (dd, J = 20.9, 8.8 Hz, 2H), 7.01 (d, J = 9.0 Hz, 2H), 6.77 (dd, J = 12.1, 6.4 Hz, 1H), 6.64 (d, J = 2.3 Hz, 1H), 6.54 (dd, J = 8.7, 2.2 Hz, 1H), 4.74 (t, J = 6.3 Hz, 1H), 4.28 (s, 1H), 4.05 (d, J = 9.2 Hz, 1H), 3.91 (s, 3H), 3.65 (t, J = 6.5 Hz, 3H), 3.53 (d, J = 11.9 Hz, 2H), 3.46 (d, J = 6.7 Hz, 1H), 3.42 (d, J = 4.6 Hz, 1H), 3.32 (d, J = 11.9 Hz, 1H), 3.26 (s, 3H), 3.08 (t, J = 11.1 Hz, 2H), 2.84 - 2.73 (m, 3H), 2.14 - 2.03 (m, 3H), 2.01 - 1.91 (m, 6H), 1.84 (t, J = 6.7 Hz, 2H), 1.67 (s, 2H), 1.22 (s, 6H), 1.15 (s, 6H). | 879.7 6 | AR-T-1 |

(continued)

| Exam ple | Co mp oun d | Structure and Name | $^1$H-NMR | LC-MS | Synthesi s Method |
|---|---|---|---|---|---|
| 178 | A-P-37 | 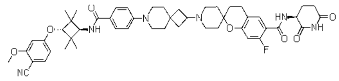 1'-(7-(4-(((1R,3R)-3-(4-cyano-3-methoxyphenoxy)-2,2,4,4-tetramethylcyclobutyl)ca rbamoyl)phenyl)-7-azaspiro[3.5]nonan-2-yl)-N-((S)-2,6-dioxopiperidin-3-yl)-7-fluorospiro[chroman-2,4'-piperidin]-6-carboxamide | 1H NMR (600 MHz, Methanol-d4) δ 7.76 (d, J = 8.6 Hz, 2H), 7.68 (d, J = 8.3 Hz, 1H), 7.54 (d, J = 8.6 Hz, 1H), 7.05 (d, J = 8.6 Hz, 2H), 6.78 (d, J = 12.4 Hz, 1H), 6.65 (d, J = 2.2 Hz, 1H), 6.58 (dd, J = 8.6, 2.2 Hz, 1H), 4.83 (dd, J = 12.7, 5.4 Hz, 1H), 4.28 (s, 1H), 4.15 (s, 1H), 3.95 (s, 3H), 3.89 - 3.80 (m, 1H), 3.47 (d, J = 12.3 Hz, 2H), 3.39 (t, J = 5.6 Hz, 2H), 3.31 (s, 2H), 3.23 - 3.14 (m, 2H), 2.89 (t, J = 6.8 Hz, 3H), 2.84 (ddd, J = 17.6, 13.6, 5.5 Hz, 1H), 2.76 - 2.69 (m, 1H), 2.43 (t, J = 10.0 Hz, 2H), 2.34 - 2.28 (m, 1H), 2.23 - 2.14 (m, 3H), 2.13 - 2.06 (m, 2H), 2.03 - 1.92 (m, 3H), 1.82 (dt, J = 29.9, 5.6 Hz, 4H), 1.30 (s, 6H), 1.25 (s, 6H). | 875.6 6 | AR-T-2 |
| 179 | A-P-38 | 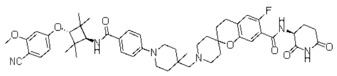 1-(1-(4-((1R,3R)-3-((4-cyano-3-methoxyphenoxy)-2,2,4,4-tetramethylcyclobutyl)ca rbamoyl)phenyl)-4-methylpiperidin-4-methyl)-N-((S)-2,6-dioxopiperidin-3-yl)-6-fluorospiro[chroman-2,4'-piperidin]-7-carboxamide | $^1$H NMR (600 MHz, DMSO-$d_6$) δ 10.87 (d, *J* = 4.0 Hz, 1H), 8.42 (td, *J* = 9.1, 8.2, 4.1 Hz, 1H), 7.77 (d, *J* = 8.6 Hz, 2H), 7.66 (d, *J* = 8.6 Hz, 1H), 7.51 (dd, *J*= 9.3, 2.9 Hz, 1H), 7.24 (d, *J*= 6.3 Hz, 1H), 7.13 *(d, J*= 10.9 Hz, 1H), 7.00 (d, *J* = 8.7 Hz, 2H), 6.64 (d, *J* = 2.2 Hz, 1H), 6.54 (dd, *J* = 8.7, 2.2 Hz, 1H), 4.75 (m, *J* = 12.9, 8.2, 5.1 Hz, 1H), 4.28 (s, 1H), 4.06 (d, *J* = 9.2 Hz, 2H), 3.91 (s, 3H), 3.62 - 3.51 (m, 3H), 3.45 (d, *J* = 11.5 Hz, 2H), 3.38 (q, *J* = 12.1 Hz, 2H), 3.23 (dd, *J* = 16.8, 3.9 Hz, 3H), 3.18 3.11 (m, 2H), 2.86 - 2.74 (m, 3H), 2.16 - 2.03 (m, 3H), 2.02 - 1.86 (m, 4H), 1.83 (t, *J* = 6.8 Hz, 2H), 1.68 (m, *J* = 11.3, 9.3, 4.3 Hz, 2H), 1.63 - 1.53 (m, 2H), 1.25 - 1.13 (m, 12H). | 863.6 6 | AR-T-2 |

(continued)

| Exam ple | Co mp oun d | Structure and Name | $^1$H-NMR | LC-MS | Synthesi s Method |
|---|---|---|---|---|---|
| 180 | A-P-39 | 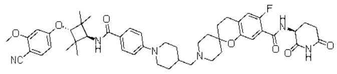 1-(1-(4-((1R,3R)-3-(4-cyano-3-methoxyphenoxy)-2,2,4,4-tetramethylcyclobutyl)ca rbamoyl)phenyl)piperidi n-4-yl) methyl)-N-((S)-2,6-dioxopiperidin-3-yl)-6-fluorospiro[chroman-2,4'-piperidin]-7-carboxamide | $^1$H NMR (600 MHz, DMSO-$d_6$) δ 10.87 (s, 1H), 8.43 (td, *J* = 8.7, 8.1, 3.9 Hz, 1H), 7.80 - 7.74 (m, 2H), 7.66 (d, *J* = 8.6 Hz, 1H), 7.52 (d, *J* = 9.2 Hz, 1H), 7.23 (d, *J* = 6.3 Hz, 1H), 7.13 (dd, *J* = 11.0, 4.0 Hz, 1H), 7.02 - 6.97 (m, 2H), 6.64 (d, *J*= 2.2 Hz, 1H), 6.54 (dd, *J* = 8.6, 2.2 Hz, 1H), 4.76 (m, *J* = 12.9, 8.0, 5.4 Hz, 1H), 4.28 (s, 1H), 4.06 (d, *J* = 9.2 Hz, 1H), 3.91 (s, 4H), 3.89 (d, *J* = 3.8 Hz, 1H), 3.47 (d, *J* = 11.6 Hz, 3H), 3.26 - 3.15 (m, 3H), 3.13 (t, *J* = 6.2 Hz, 2H), 2.88 - 2.73 (m, 5H), 2.16 - 2.05 (m, 2H), 2.03 - 1.98 (m, 1H), 1.93 (d, *J*= 10.8 Hz, 3H), 1.84 (q, *J* = 6.3, 5.8 Hz, 4H), 1.30 (m, *J* = 12.0, 3.6 Hz, 2H), 1.19 (d, *J* = 46.0 Hz, 12H). | 849.7 6 | AR-T-2 |
| 181 | A-P-40 | 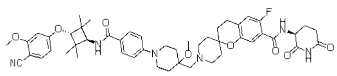 1'-(1-((4-((1r,3r)-3-(4-cyano-3-methoxyphenoxy)-2,2,4,4-tetramethylcyclobutyl)ca rbamoyl)phenyl)-4-methoxypiperidin-4-yl) methyl)-N-((S)-2,6-dioxopiperidin-3-yl)-6-fluorospiro[2,4'-piperidin]-7-carboxamide | $^1$H NMR (600 MHz, DMSO-$d_6$) δ 10.87 (s, 1H), 8.43 (dd, *J* = 8.1, 3.4 Hz, 1H), 7.75 (d, *J* = 8.8 Hz, 2H), 7.65 (d, *J*= 8.6 Hz, 1H), 7.51 *(d, J*= 9.2 Hz, 1H), 7.08 - 7.00 (m, 2H), 6.97 *(d, J*= 8.9 Hz, 2H), 6.64 (d, *J* = 2.1 Hz, 1H), 6.54 (dd, *J* = 8.6, 2.1 Hz, 1H), 4.77 - 4.71 (m, 1H), 4.27 (s, 1H), 4.05 (d, *J* = 9.2 Hz, 1H), 3.91 (s, 3H), 3.53 (d, *J* = 12.6 Hz, 2H), 3.16 (s, 3H), 3.05 (t, *J* = 10.7 Hz, 2H), 2.84 - 2.71 (m, 3H), 2.71 - 2.63 (m, 2H), 2.57 - 2.51 (m, 3H), 2.45 - 2.37 (m, 2H), 2.16 - 2.03 (m, 1H), 2.03 - 1.94 (m, 1H), 1.82 (d, *J* = 13.2 Hz, 2H), 1.76 (t, *J* = 6.7 Hz, 2H), 1.73 - 1.55 (m, 6H), 1.23 (s, 6H), 1.15 (s, 6H). | 879.7 6 | AR-T-2 |

(continued)

| Exam ple | Co mp oun d | Structure and Name | $^1$H-NMR | LC-MS | Synthesi s Method |
|---|---|---|---|---|---|
| 182 | A-P-41 | 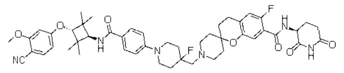 1'-(1-((4-((1r,3r)-3-(4-cyano-3-methoxyphenoxy)-2,2,4,4-tetramethylcyclobutyl)ca rbamoyl)phenyl)-4-fluoropiperidin-4-yl) methyl)-N-((S)-2,6-dioxopiperidin-3-yl)-6-fluorospiro[2,4'-piperidin]-7-carboxamide | $^1$H NMR (600 MHz, DMSO-$d_6$) δ 10.87 (s, 1H), 8.43 (dd, *J* = 8.0, 3.5 Hz, 1H), 7.77 (d, *J* = 7.3 Hz, 2H), 7.65 (d, *J*= 8.6 Hz, 1H), 7.52 (d, *J*= 9.2 Hz, 1H), 7.12 - 6.98 (m, 3H), 6.64 (d, *J*= 2.0 Hz, 1H), 6.54 (dd, *J* = 8.6, 2.1 Hz, 1H), 4.78 - 4.71 (m, 1H), 4.28 (s, 1H), 4.06 (d, *J* = 9.2 Hz, 1H), 3.91 (s, 3H), 3.79 (s, 1H), 3.66 (s, 2H), 3.48 (s, 1H), 3.12 (t, *J* = 11.4 Hz, 2H), 2.79 (ddd, *J*= 17.4, 14.7, 8.1 Hz, 3H), 2.66 (s, 1H), 2.61 (d, *J* = 10.9 Hz, 1H), 2.58 - 2.53 (m, 2H), 2.14 - 2.05 (m, 1H), 2.05 - 1.97 (m, 2H), 1.91 (s, 2H), 1.77 (d, *J*= *9.7* Hz, 4H), 1.66 (s, 3H), 1.31 - 1.18 (m, 8H), 1.15 (s, 6H). | 867.6 6 | AR-T-2 |
| 183 | AR -P-42 | 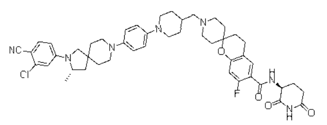 1'-((1-(4-((S)-2-(3-chloro-4-cyanophenyl)-3-methyl-2,8-diazaspiro [4.5]decan-8-yl)phenyl)piperidin-4-yl)methyl)-N-((S)-2,6-dioxopiperidin-3-yl)-7-fluorospiro[chroman-2,4'-piperidin]-6-carboxamide | $^1$H NMR (600 MHz, DMSO) δ 10.86 (s, 1H), 8.31 - 8.23 (m, 1H), 7.63 (d, *J*= 8.8 Hz, 1H), 7.56 (d, *J* = 8.4 Hz, 1H), 7.37 (s, 2H), 7.22 (s, 2H), 6.82 (s, 1H), 6.76 (d, *J* = 11.9 Hz, 1H), 6.69 (d, *J*= 7.7 Hz, 1H), 4.78 - 4.72 (m, 1H), 4.07 (dd, *J* = 12.8, 6.4 Hz, 3H), 3.74 (s, 3H), 3.45 (dd, *J* = 59.3, 10.6 Hz, 9H), 3.17 (d, *J*= 27.5 Hz, 5H), 2.81 (t, *J* = 6.4 Hz, 2H), 2.76 (d, *J* = 5.4 Hz, 1H), 2.37 - 2.22 (m, 1H), 2.17 - 2.06 (m, 2H), 1.98 (dd, *J* = 28.1, 17.0 Hz, 8H), 1.87 (t, *J*= 6.3 Hz, 2H), 1.69 - 1.61 (m, 2H), 1.44 (s, 2H), 1.22 (d, *J* = 6.0 Hz, 3H). | 836.4 6 | AR-T-3 |

(continued)

| Exam ple | Co mp oun d | Structure and Name | $^1$H-NMR | LC-MS | Synthesi s Method |
|---|---|---|---|---|---|
| 184 | AR -P-43 | 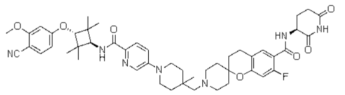 1'-((1-(6-(((1r,3r)-3-(4-cyano-3-methoxyphenoxy)-2,2,4,4-tetramethylcyclobutyl)ca rbamoyl)pyridin-3-yl)-4-methylpiperidin-4-yl) methyl)-N-((S)-2,6-dioxopiperidin-3-yl)-7-fluorospiro[chroman-2,4'-piperidin]-6-carboxamide | $^1$H NMR (600 MHz, DMSO-$d_6$) δ 10.85 (s, 1H), 8.40 - 8.27 (m, 2H), 8.11 (d, J = 9.1 Hz, 1H), 7.86 (d, J = 8.8 Hz, 1H), 7.63 (d, J = 8.6 Hz, 1H), 7.57 - 7.40 (m, 2H), 6.78 (t, J = 15.3 Hz, 1H), 6.71 - 6.50 (m, 2H), 3.96 - 3.89 (m, 7H), 3.20 (dd, J = 18.2, 8.2 Hz, 8H), 2.81 - 2.73 (m, 4H), 2.13 - 2.05 (m, 3H), 1.85 (d, J = 6.9 Hz, 2H), 1.62 (dd, J = 36.5, 11.7 Hz, 5H), 1.21 (d, J = 10.3 Hz, 5H), 1.19 (s, 6H), 1.13 (s, 6H), 1.05 (t, J = 7.0 Hz, 1H). | 864.6 6 | AR-T-2 |
| 185 | AR -P-44 | 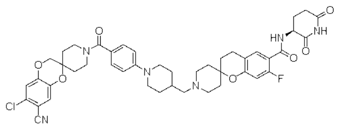 (S)-1'-((1-(4-(6-chloro-7-cyano-3H-spiro[benzo[b][1,4]dioxa ne-2,4'-piperidin]-1'-carbonyl)phenyl)piperidi n-4-yl)methyl)-N-(2,6-dioxopiperidin-3-yl)-7-fluorospiro [chroman-2,4'-piperidin]-6-carboxamide | $^1$H NMR (600 MHz, DMSO) δ 10.88 (s, 1H), 8.29 (dd, *J* = 7.9, 4.5 Hz, 1H), 7.59 (s, 1H), 7.55 (d, *J* = 8.4 Hz, 1H), 7.36 (s, 1H), 7.31 (d, *J* = 8.5 Hz, 2H), 6.98 (d, *J* = 8.7 Hz, 2H), 6.76 (d, *J*= 12.1 Hz, 1H), 4.77 - 4.71 (m, 1H), 4.20 (s, 2H), 3.85 (d, *J* = 12.5 Hz, 3H), 3.49 (d, *J*= 11.2 Hz, 3H), 3.32 (s, 2H), 3.24 - 3.14 (m, 3H), 3.11 (t, *J* = 5.5 Hz, 2H), 2.79 (t, *J* = 10.4 Hz, 5H), 2.15 - 2.05 (m, 2H), 2.03 - 1.97 (m, 2H), 1.94 (d, *J* = 23.9 Hz, 3H), 1.86 (dd, *J*= 18.5, 10.9 Hz, 4H), 1.73 (dd, *J*= 17.7, 7.2 Hz, 4H), 1.31 (dd, *J*= 21.6, 11.3 Hz, 2H). | 864.6 6 | AR-T-4 |

(continued)

| Exam ple | Co mp oun d | Structure and Name | $^1$H-NMR | LC-MS | Synthesi s Method |
|---|---|---|---|---|---|
| 186 | AR -P-45 | 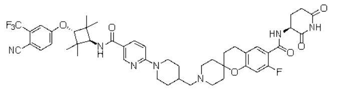 1'-((1-(5-(((1R,3R)-3-(4-cyano-3-(trifluoromethyl)phenoxy )-2,2,4,4-tetramethylcyclobutyl)ca rbamoyl)pyridin-2-yl)piperidin-4-yl)methyl)-N-((S)-2,6-dioxopiperidin-3-yl)-7-fluorospiro[chroman-2,4'-piperidin]-6-carboxamide | $^1$H NMR (400 MHz, DMSO-$d_6$) δ 10.87 (s, 1H), 8.63 (d, J = 2.4 Hz, 1H), 8.29 (dd, J = 8.0, 4.6 Hz, 1H), 8.11 (d, J = 8.7 Hz, 1H), 7.99 (dd, J = 9.0, 2.4 Hz, 1H), 7.66 (dd, J = 9.3, 2.7 Hz, 1H), 7.55 (d, J = 8.5 Hz, 1H), 7.41 (d, J = 2.5 Hz, 1H), 7.30 (dd, J = 8.7, 2.5 Hz, 1H), 6.93 (d, J = 9.1 Hz, 1H), 6.77 (dd, J = 12.2, 6.4 Hz, 1H), 4.75 (ddd, J = 12.7, 7.9, 5.4 Hz, 1H), 4.45 (d, J = 13.1 Hz, 2H), 4.39 (s, 1H), 4.08 (d, J = 9.2 Hz, 2H), 3.49 (s, 3H), 3.28 - 3.04 (m, 4H), 2.96 (t, J = 12.5 Hz, 2H), 2.86 - 2.70 (m, 3H), 2.26 - 2.14 (m, 1H), 2.14 - 2.03 (m, 2H), 2.03 - 1.92 (m, 4H), 1.85 (dt, J = 10.9, 5.2 Hz, 3H), 1.23 (s, 8H), 1.14 (s, 6H). | 888.6 6 | AR-T-2 |
| 187 | AR -P-46 | 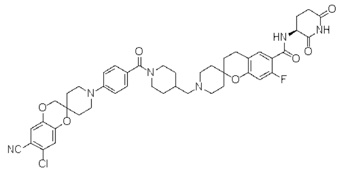 (S)-1'-((1-(4-(7-chloro-6-cyano-3H-spiro[benzo[b][1,4]dioxo -2,4'-piperidin]-1'-yl)benzoyl)piperidin-4-yl)methyl)-N-(2,6-dioxopiperidin-3-yl)-7-fluorospiro[chroman-2,4'-piperidin]-6-carboxamide | $^1$H NMR (400 MHz, DMSO-$d_6$) δ 10.88 (s, 1H), 8.29 (dt, *J*= 10.7, 5.4 Hz, 1H), 7.63 (s, 1H), 7.54 (dd, *J* = 8.5, 2.3 Hz, 1H), 7.34 (s, 1H), 7.28 (d, *J*= *8.6* Hz, 2H), 7.01 (d, *J*= 8.8 Hz, 2H), 6.77 (dd, *J*= 12.1, 7.9 Hz, 1H), 4.75 (ddd, *J* = 12.8, 8.0, 5.4 Hz, 1H), 4.18 (s, 2H), 3.62 (dt, J= 13.1, 4.8 Hz, 2H), 3.48 (d, *J* = 11.7 Hz, 2H), 3.26 - 3.20 (m, 2H), 3.17 (dd, *J* = 12.9, 6.4 Hz, 3H), 3.09 (d, *J*= *5.9* Hz, 2H), 2.94 (s, 2H), 2.78 (dt, *J*= 17.1, 6.6 Hz, 3H), 2.54 (d, *J* = 3.8 Hz, 1H), 2.21 - 2.03 (m, 3H), 1.97 (dq, *J* = 11.6, 5.7 Hz, 4H), 1.89 - 1.73 (m, 8H) | 888.6 6 | A-T-4 |

(continued)

| Exam ple | Co mp oun d | Structure and Name | $^1$H-NMR | LC-MS | Synthesi s Method |
|---|---|---|---|---|---|
| 188 | AR -P-47 | 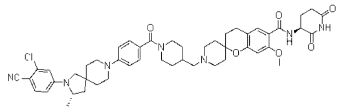 1'-(1-(4-(S)-2-(3-chloro-4-cyanophenyl)-3-methyl-2,8-diazaspiro [4.5]decan-8-yl)benzoyl)piperidin-4-yl)methyl)-N-((S)-2,6-dioxopiperidin-3-yl)-7-methoxyspiro [chroman-2,4'-piperidin]-6-carbox-amide | $^1$H NMR (400 MHz, DMSO-d6) δ 10.90 (s, 1H), 8.48 (d, J = 7.2 Hz, 1H), 7.71 (s, 1H), 7.61 (d, J = 8.8 Hz, 1H), 7.26 (d, J = 8.5 Hz, 2H), 6.97 (d, J = 8.5 Hz, 2H), 6.80 (d, J = 2.3 Hz, 1H), 6.66 (dd, J = 9.0, 2.3 Hz, 1 H), 6.59 (s, 1H), 4.74 (dd, J = 10.3, 7.3 Hz, 1H), 4.07 - 4.02 (m, 1H), 3.88 (s, 3H), 3.49 - 3.41 (m, 3H), 3.34 (t, J = 9.3 Hz, 3H), 3.28 - 3.03 (m, 7H), 3.00 - 2.85 (m, 2H), 2.81 - 2.67 (m, 3H), 2.24 (dd, J = 12.8, 7.7 Hz, 1H), 2.20 - 1.86 (m, 8H), 1.86 - 1.63 (m, 6H), 1.58 (dd, J = 12.8, 6.6 Hz, 1H), 1.50 (q, J = 5.4, 4.5 Hz, 2H), 1.39 (s, 1H), 1.27 - 1.14 (m, 5H). | 876.4 6 | A-T-4 |
| 189 | AR -P-48 | 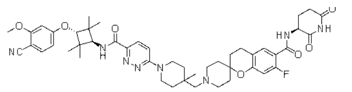 1'-((1-(6-(((1R,3R)-3-(4-cyano-3-methoxyphenoxy)-2,2,4,4-tetra-methylcyclobutyl)ca rbamoyl)pyri-dazin-3-yl)-4-methylpiperidin-4-yl) methyl)-N-((S)-2,6-dioxopiperidin-3-yl)-7-fluorospiro[chroman-2,4'-piperidin]-6-carboxa-amide | $^1$H NMR (600 MHz, DMSO-$d_6$) δ 10.86 (s, 1H), 8.32 - 8.20 (m, 2H), 7.87 (d, J = 9.5 Hz, 1H), 7.65 (d, J = 8.6 Hz, 1H), 7.55 (d, J = 8.3 Hz, 1H), 7.42 (d, J = 9.6 Hz, 1H), 6.77 (dd, J = 12.1, 7.4 Hz, 1H), 6.67 (d, J = 2.2 Hz, 1H), 6.57 (dd, J = 8.6, 2.2 Hz, 1H), 4.75 (ddd, J = 12.9, 7.9, 5.4 Hz, 1H), 4.41 (s, 1H), 4.21 - 4.11 (m, 2H), 4.01 (d, J = 9.2 Hz, 1H), 3.92 (s, 3H), 3.49 (td, J = 9.6, 4.6 Hz, 3H), 3.33 (q, J = 11.6 Hz, 2H), 3.24 (dd, J = 16.4, 3.8 Hz, 3H), 2.78 (ddq, J = 16.7, 8.8, 3.9, 3.3 Hz, 3H), 2.55 (s, 1H), 2.09 (dt, J = 16.5, 5.5 Hz, 3H), 2.03 - 1.96 (m, 2H), 1.93 (d, J = 13.9 Hz, 2H), 1.85 (t, J = 6.8 Hz, 1H), 1.61 (tt, J = 17.4, 11.6 Hz, 4H), 1.25 (d, J = 23.1 Hz, 9H), 1.17 (s, 6H). | 865.6 6 | A-T-1 |

(continued)

| Exam ple | Co mp oun d | Structure and Name | $^1$H-NMR | LC-MS | Synthesi s Method |
|---|---|---|---|---|---|
| 190 | AR -P-49 | 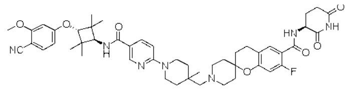 1'-((1-(5-(((1R,3R)-3-(4-cyano-3-methoxyphenoxy)-2,2,4,4-tetra-methylcyclobutyl)ca rbamoyl)pyri-din-2-yl)-4-methylpiperidin-4-yl) methyl)-N-((S)-2,6-dioxopiperidin-3-yl)-7-fluorospiro[chroman-2,4'-piperidin]-6-carboxa-mide | $^1$H NMR (400 MHz, DMSO-$d_6$) $\delta$ 10.86 (s, 1H), 8.64 (d, J = 2.4 Hz, 1H), 8.27 (dt, J = 8.5, 4.2 Hz, 1H), 8.00 (dd, J = 9.1, 2.5 Hz, 1H), 7.65 (t, J = 9.5 Hz, 2H), 7.55 (d, J = 8.4 Hz, 1H), 6.93 (d, J = 9.1 Hz, 1H), 6.77 (dd, J = 12.1, 5.8 Hz, 1H), 6.64 (d, J = 2.2 Hz, 1H), 6.54 (dd, J = 8.7, 2.2 Hz, 1H), 4.75 (ddd, J = 12.9, 8.0, 5.4 Hz, 1H), 4.27 (s, 1H), 4.07 (d, J = 9.2 Hz, 3H), 4.02 (s, 2H), 3.91 (s, 3H), 3.47 (t, J = 12.6 Hz, 2H), 3.42 - 3.28 (m, 3H), 3.22 (dd, J = 17.8, 3.9 Hz, 2H), 2.86 - 2.73 (m, 3H), 2.56 - 2.52 (m, 1H), 2.10 (dhept, J = 18.8, 4.5 Hz, 3H), 2.04 - 1.80 (m, 5H), 1.66 - 1.49 (m, 4H), 1.24 (d, J = 11.5 Hz, 8H), 1.15 (s, 6H). | 864.6 6 | A-T-2 |
| 191 | AR -P-50 | 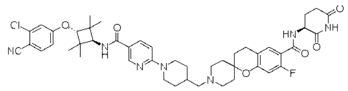 1'-((1-(5-(((1R,3R)-3-(3-chloro-4-cyanophenoxy)-2,2,4,4-tetra-methylcyclobutyl)ca rbamoyl)pyri-din-2-yl)piperidin-4-yl) methyl)-N-((S)-2,6-dioxopiperidin-3-yl)-7-fluorospiro[chroman-2,4'-piperidin]-6-carboxa-mide | $^1$H NMR (400 MHz, DMSO-$d_6$) $\delta$ 10.87 (s, 1H), 8.63 (d, J = 2.4 Hz, 1H), 8.28 (dt, J = 8.7, 4.3 Hz, 1H), 7.99 (dd, J = 9.0, 2.5 Hz, 1H), 7.91 (d, J = 8.7 Hz, 1H), 7.63 (d, J = 9.2 Hz, 1H), 7.56 (d, J = 8.4 Hz, 1H), 7.22 (d, J = 2.4 Hz, 1H), 7.01 (dd, J = 8.8, 2.4 Hz, 1H), 6.94 (d, J = 9.1 Hz, 1H), 6.77 (d, J = 12.1 Hz, 1H), 4.75 (ddd, J = 12.8, 8.0, 5.3 Hz, 1H), 4.45 (d, J = 13.1 Hz, 2H), 4.32 (s, 1H), 4.06 (d, J = 9.2 Hz, 1H), 3.49 (d, J = 12.1 Hz, 2H), 3.30 - 3.13 (m, 3H), 3.10 (t, J = 6.2 Hz, 2H), 3.02 - 2.92 (m, 2H), 2.85 - 2.75 (m, 3H), 2.19 (ddd, J = 11.1, 7.3, 3.9 Hz, 1H), 2.15 - 2.07 (m, 1H), 2.05 - 1.91 (m, 5H), 1.90 - 1.79 (m, 4H), 1.22 (m, 8H), 1.13 (s, 6H). | 854.7 6 | A-T-2 |

(continued)

| Exam ple | Co mp oun d | Structure and Name | $^1$H-NMR | LC-MS | Synthesi s Method |
|---|---|---|---|---|---|
| 192 | AR -P-51 | 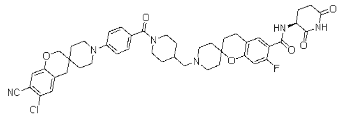 (S)-1'-((1-(4-(6-chloro-7-cyanospiro[tryptophan-3,4'-piperidin]-1'-yl)benzoyl)piperidin-4-yl) methyl)-N-(2,6-dioxopiperidin-3-yl)-7-fluorospiro[chroman-2,4'-pyridine]-6-carboxamide | $^1$H NMR (400 MHz, DMSO-d6) δ 10.87 (s, 1H), 8.28 (dd, J = 8.1, 4.6 Hz, 1H), 7.66 - 7.38 (m, 3H), 7.27 (d, J = 8.5 Hz, 2H), 6.97 (d, J = 8.6 Hz, 2H), 6.75 (d, J = 12.0 Hz, 1H), 4.85 - 4.61 (m, 1H), 4.04 (s, 3H), 3.23 (s, 5H), 3.15 - 3.06 (m, 3H), 2.89 (s, 3H), 2.86 - 2.72 (m, 6H), 2.10 (dd, J = 12.8, 4.2 Hz, 2H), 2.04 - 1.91 (m, 5H), 1.90 - 1.73 (m, 4H), 1.64 - 1.40 (m, 5H), 1.29 - 1.13 (m, 3H). | 837.5 5 | A-T-4 |
| 193 | AR -P-52 | 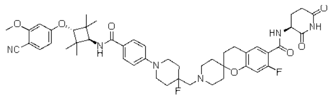 1'-((1-(4-(((1r,3r)-3-(4-cyano-3-methoxyphenoxy)-2,2,4,4-tetramethylcyclobutyl)ca rbamoyl)phenyl)-4-fluoropiperidin-4-yl) methyl)-N-((S)-2,6-dioxopiperidin-3-yl)-7-fluorospiro[chroman-2,4'-piperidin]-6-carboxamide | $^1$H NMR (600 MHz, DMSO-$d_6$) δ 10.86 (s, 1H), 8.27 (dd, *J* = 8.3, 4.5 Hz, 1H), 7.79 (d, *J*= 8.6 Hz, 2H), 7.66 (d, *J*= 8.6 Hz, 1H), 7.55 (dd, *J* = 8.9, 5.5 Hz, 2H), 7.05 (d, *J*= 8.8 Hz, 2H), 6.78 (d, *J*= 12.0 Hz, 1H), 6.64 (d, *J*= 2.2 Hz, 1H), 6.54 (dd, *J* = 8.6, 2.2 Hz, 1H), 4.75 (ddd, *J* = 12.9, 8.1, 5.4 Hz, 1H), 4.28 (s, 1H), 4.06 (d, *J* = 9.2 Hz, 1H), 3.91 (s, 3H), 3.80 (d, *J*= 12.9 Hz, 3H), 3.18 - 3.08 (m, 3H), 2.78 (dt, *J* = 18.5, 5.8 Hz, 4H), 2.55 (s, 1H), 2.15 - 1.80 (m, 15H), 1.23 (s, 6H), 1.15 (s, 6H). | 867.4 2 | A-T-2 |
| 194 | AR -P-53 | 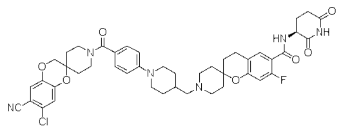 (S)-1'-((1-(4-(7-chloro-6-cyano-3H-spiro[benzo[b][1,4]dioxa ne-2,4'-piperidin]-1'-carbonyl)phenyl)piperidi n-4-yl)methyl)-N-(2,6-dioxopiperidin-3-yl)-7-fluorospiro [chroman-2,4'-piperidin]-6-carboxamide | $^1$H NMR (600 MHz, DMSO-$d_6$) δ 10.88 (s, 1H), 8.29 (dd, J = 8.1, 4.5 Hz, 1H), 7.63 (s, 1H), 7.55 (d, J = 8.4 Hz, 1H), 7.34 (s, 1H), 7.31 (d, J = 8.5 Hz, 2H), 6.98 (d, J = 8.6 Hz, 2H), 6.76 (d, J = 12.2 Hz, 1H), 4.75 (ddd, J = 12.9, 8.1, 5.3 Hz, 1H), 4.15 (s, 2H), 3.86 (dd, J = 13.3, 3.4 Hz, 3H), 3.49 (d, J = 12.4 Hz, 3H), 3.33 (s, 2H), 3.18 (td, J = 12.7, 5.5 Hz, 3H), 3.11 (t, J = 6.2 Hz, 2H), 2.79 (dt, J = 15.8, 9.1 Hz, 5H), 2.12 (td, J = 12.9, 4.5 Hz, 1H), 2.06 (dq, J = 10.9, 3.7, 3.2 Hz, 1H), 2.04 - 1.90 (m, 5H), 1.90 - 1.79 (m, 4H), 1.79 - 1.67 (m, 4H), 1.30 (qd, J = 12.5, 3.8 Hz, 2H). | 839.5 5 | A-T-4 |

(continued)

| Exam ple | Co mp oun d | Structure and Name | $^1$H-NMR | LC-MS | Synthesi s Method |
|---|---|---|---|---|---|
| 195 | AR -P-54 | 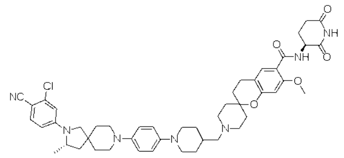 1'-(1-(4-(S)-2-(3-chloro-4-cyanophenyl)-3-methyl-2,8-diazaspiro [4.5]decan-8-yl)phenyl)piperidin-4-yl)methyl)-N-((S)-2,6-dioxopiperidin-3-yl)-7-methoxyspiro [chroman-2,4'-piperidin]-6-carbox-amide | $^1$H NMR (600 MHz, DMSO-d6) δ 10.90 (s, 1H), 9.23 (s, 1H), 8.49 (d, J = 7.2 Hz, 1H), 7.72 (d, J = 2.8 Hz, 1H), 7.63 (d, J = 8.8 Hz, 1H), 7.13 (dt, J = 66.6, 35.0 Hz, 3H), 6.82 (d, J = 2.3 Hz, 1H), 6.68 (dd, J = 8.9, 2.3 Hz, 1H), 6.59 (d, J = 16.9 Hz, 1H), 4.73 (dt, J = 11.2, 6.9 Hz, 1H), 4.06 (q, J = 6.6 Hz, 1H), 3.88 (d, J = 3.0 Hz, 3H), 3.50 (d, J = 11.6 Hz, 7H), 3.39 (s, 5H), 3.26 - 3.07 (m, 5H), 2.82 - 2.70 (m, 3H), 2.54 (s, 1H), 2.22 (s, 1H), 2.09 (tdd, J = 12.3, 8.1, 3.6 Hz, 3H), 2.04 - 1.87 (m, 6H), 1.85 (t, J = 6.8 Hz, 2H), 1.52 (d, J = 151.0 Hz, 6H), 1.22 (d, J = 6.0 Hz, 3H). | 848.5 6 | A-T-3 |
| 196 | AR -P-55 | 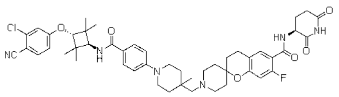 1'-(1-(4-(1R,3R)-3-(3-chloro-4-cyanophenoxy)-2,2,4,4-tetramethylcyclobutyl)ca rbamoyl)phenyl)-24-methylpiperidin-4-yl) methyl)-N-((S)-2,6-dioxopiperidin-3-yl)-7-fluorospiro[chroman-2,4'-piperidin]-6-carboxa-mide | $^1$H NMR (600 MHz, DMSO-d6)δ 10.87 (s, 1H), 7.92 (d, J = 8.7 Hz, 1H), 7.77 (d, J = 8.5 Hz, 2H), 7.54 (dd, J = 24.4, 8.9 Hz, 2H), 7.26 - 7.14 (m, 2H), 7.03 - 6.98 (m, 3H), 6.78 (d, J = 12.1 Hz, 1H), 4.79 - 4.70 (m, 1H), 4.33 (s, 1H), 4.06 (d, J = 9.1 Hz, 1H), 2.84 - 2.72 (m, 2H), 2.16 - 2.04 (m, 4H), 2.03 - 1.82 (m, 7H), 1.63 (dd, J = 47.7, 11.8 Hz, 5H), 1.46 (s, 2H), 1.24 (s, 5H), 1.22 (s, 6H), 1.20 (d, J = 11.6 Hz, 4H), 1.13 (s, 6H). | 867.3 6 | A-T-2 |
| 197 | AR -P-56 | 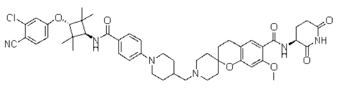 1'-(1-(4-(1R,3R)-3-(3-chloro-4-cyanophenoxy)-2,2,4,4-tetramethyl-cyclobutyl)ca rbamoyl)phenyl)pi-peridi n-4-yl)methyl)-N-((S)-2,6-dioxopiperidin-3-yl)-7-methoxyspiro [chroman-2,4'-piperidin]-6-carbox-amide | $^1$H NMR (400 MHz, DMSO) δ 10.90 (s, 1H), 8.48 (d, *J*= 7.2 Hz, 1H), 7.91 (d, *J*= 8.7 Hz, 1H), 7.76 (d, *J*= 8.7 Hz, 2H), 7.72 (s, 1H), 7.52 (d, *J*= 9.2 Hz, 1H), 7.21 (d, *J* = 2.2 Hz, 1H), 7.05 - 6.95 (m, 3H), 6.60 (s, 1H), 4.73 (dd, *J* = 17.4, 7.4 Hz, 1H), 4.33 (s, 1H), 4.06 (d, *J* = 9.1 Hz, 1H), 3.92 (s, 1H), 3.88 (s, 3H), 3.50 (d, 2H), 3.27 - 3.07 (m, 5H), 2.79 (dt, *J* = 15.2, 9.4 Hz, 5H), 2.53 (s, 1H), 2.08 (t, *J*= 11.3 Hz, 3H), 2.04 - 1.89 (m, 4H), 1.83 (d, *J* = 7.6 Hz, 3H), 1.35 - 1.25 (m, 3H), 1.22 (s, 6H), 1.13 (s, 6H). | 865.5 6 | A-T-2 |

(continued)

| Exam ple | Co mp oun d | Structure and Name | $^1$H-NMR | LC-MS | Synthesi s Method |
|---|---|---|---|---|---|
| 198 | AR -P-57 | 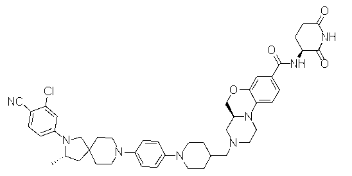 (S)-3-((1-(4-(((S)-2-(3-chloro-4-cyanophenyl)-3-methyl-2,8-diazaspiro[4.5]decan-8-yl)phenyl)piperidin-4-yl)methyl)-N-(((S)-2,6-dioxopiperidin-3-yl)-1,2,3,4,4a,5-hexahydrobenzo[b]pyrazi no[1,2-d][1,4] oxazine-8-carboxamide | $^1$H NMR (600 MHz, DMSO-d6) δ 10.86 (s, 1H), 9.89 (s, 1H), 8.54 (d, J = 8.3 Hz, 1H), 7.63 (d, J = 8.9 Hz, 1H), 7.51 - 7.29 (m, 3H), 7.27 - 6.99 (m, 3H), 6.87 - 6.61 (m, 2H), 4.73 (ddd, J = 13.0, 8.3, 5.3 Hz, 1H), 4.35 (dd, J = 11.4, 2.8 Hz, 1H), 4.21 (d, J = 13.2 Hz, 1H), 4.05 (dt, J = 11.4, 6.7 Hz, 2H), 3.75-3.60(m,4H),3.39 (s, 6H), 3.13 (s, 5H), 2.95 - 2.73 (m, 3H), 2.55 (d, J = 4.0 Hz, 3H), 2.11 (qd, J = 13.0, 4.5 Hz, 2H), 2.01 - 1.77 (m, 5H), 1.65 (s, 2H), 1.42 (d, J = 40.8 Hz, 2H), 1.22 (d, J = 6.0 Hz, 4H). | 817.5 6 | A-T-3 |
| 199 | AR -P-58 | 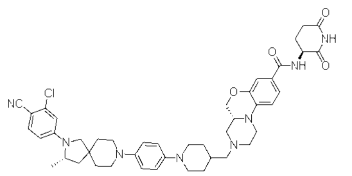 (R)-3-((1-(4-(((S)-2-(3-chloro-4-cyanophenyl)-3-methyl-2,8-diazaspiro[4.5]decan-8-yl)phenyl)piperidin-4-yl)methyl)-N-(((S)-2,6-dioxopiperidin-3-yl)-1,2,3,4,4a,5-hexahydrobenzo[b]pyrazi no[1,2-d][1,4] oxazine-8-carboxamide | $^1$H NMR (400 MHz, DMSO-d6) δ 10.85 (s, 1H), 8.54 (d, J = 8.4 Hz, 1H), 7.63 (d, J = 8.9 Hz, 1H), 7.46 (dd, J = 8.4, 2.0 Hz, 1H), 7.41 - 6.97 (m, 6H), 6.82 (d, J = 2.3 Hz, 1H), 6.68 (dd, J = 9.0, 2.3 Hz, 1H), 4.73 (ddd, J = 13.0, 8.2, 5.3 Hz, 1H), 4.36 (dd, J = 11.2, 2.6 Hz, 1H), 4.20 (d, J = 13.1 Hz, 1H), 4.05 (td, J = 11.4, 10.3, 6.7 Hz, 2H), 3.89-3.77(m,8H),3.09 (s, 6H), 2.86 - 2.60 (m, 3H), 2.29 (s, 1H), 2.11 (qd, J = 13.0, 4.8 Hz, 3H), 1.93 (ddt, J = 20.8, 16.0, 6.8 Hz, 5H), 1.76 - 1.58 (m, 3H), 1.42 (d, J = 13.2 Hz, 2H), 1.31 - 1.16 (m, 6H). | 817.5 6 | A-T-3 |

(continued)

| Exam ple | Co mp oun d | Structure and Name | ¹H-NMR | LC-MS | Synthesi s Method |
|---|---|---|---|---|---|
| 200 | AR -P-59 | 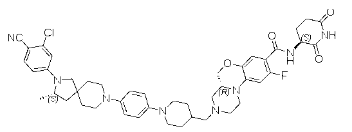 (R)-3-((1-(4-((S)-2-(3-chloro-4-cyanophenyl)-3-methyl-2,8-diazaspiro[4.5]decan-8-yl)phenyl)piperidin-4-yl)methyl)-N-((S)-2,6-dioxopiperidin-3-yl)-9-fluoro-1,2,3,4,4a,5-hexahydrobenzo[b] pyrazi no[1,2-d][1,4]oxazine-8-carboxamide | $^1$H NMR (600 MHz, Methanol-d4) δ 7.53 (d, J = 8.8 Hz, 1H), 7.47 - 7.43 (m, 2H), 7.31 (d, J = 7.2 Hz, 1H), 7.23 (d, J = 8.7 Hz, 2H), 6.89 (d, J = 13.6 Hz, 1H), 6.83 (d, J = 2.3 Hz, 1H), 6.70 (dd, J = 8.9, 2.3 Hz, 1H), 4.81 (dd, J = 12.8, 5.3 Hz, 1H), 4.37 (dd, J = 11.2, 2.7 Hz, 1H), 4.19 (d, J = 14.0 Hz, 1H), 4.15 - 4.05 (m, 2H), 3.86 (d, J = 12.6 Hz, 2H), 3.73 (d, J = 12.8 Hz, 3H), 3.65 - 3.52 (m, 5H), 3.48 (d, J = 10.5 Hz, 1H), 3.32 - 3.29 (m, 2H), 3.20 (s, 3H), 3.07 (s, 2H), 3.02 - 2.94 (m, 1H), 2.83 (ddd, J = 17.4, 13.6, 5.4 Hz, 1H), 2.72 (ddd, J = 17.6, 4.7, 2.5 Hz, 1H), 2.46 (s, 1H), 2.35 - 2.29 (m, 1H), 2.26 - 2.09 (m, 4H), 2.03 (t, J = 15.8 Hz, 2H), 1.87 (s, 2H), 1.77 (dd, J = 13.0, 6.9 Hz, 1H), 1.57 (q, J = 12.4 Hz, 2H), 1.33 (d, J = 6.1 Hz, 3H). | 823.6 6 | A-T-3 |
| 201 | AR -P-60 | 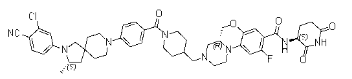 (R)-3-((1-(4-(S)-2-(3-chloro-4-cyanophenyl)-3-methyl-2,8-diazaspiro[4.5]decan-8-yl)benzoyl)piperidin-4-yl)methyl)-N-(S)-2,6-dioxopiperidin-3-yl)-9-fluoro-1,2,3,4,4a,5-hexahydrobenzo[b] pyrazi no[1,2-d][1,4]oxazine-8-carboxamide | $^1$H NMR (600 MHz, Methanol-d4) δ 7.51 (d, J = 8.8 Hz, 1H), 7.45 (d, J = 8.5 Hz, 2H), 7.31 (d, J = 7.2 Hz, 1H), 7.26 (d, J = 8.4 Hz, 2H), 6.89 (d, J = 13.6 Hz, 1H), 6.79 (d, J = 2.3 Hz, 1H), 6.68 (dd, J = 8.9, 2.4 Hz, 1H), 4.81 (dd, J = 12.7, 5.3 Hz, 1H), 4.61 (d, J = 46.0 Hz, 2H), 4.37 (dd, J = 11.2, 2.9 Hz, 1H), 4.20 (d, J = 13.5 Hz, 1H), 4.13 - 4.03 (m, 2H), 3.74 (d, J = 11.2 Hz, 2H), 3.66 (s, 1H), 3.56 - 3.47 (m, 3H), 3.46 - 3.38 (m, 3H), 3.31 - 3.25 (m, 2H), 3.24 - 3.16 (m, 3H), 3.02 (s, 2H), 2.83 (ddd, J = 17.5, 13.6, 5.4 Hz, 1H), 2.72 (ddd, J = 17.6, 4.7, 2.5 Hz, 1H), 2.40 (dd, J = 12.9, 7.6 Hz, 1H), 2.35 - 2.25 (m, 2H), 2.16 (qd, J = 13.0, 4.6 Hz, 1H), 2.02 - 1.91 (m, 3H), 1.90 - 1.79 (m, 2H), 1.78 - 1.67 (m, 3H), 1.36 (d, J = 11.1 Hz, 2H), 1.31 (d, J = 6.1 Hz, 3H). | 851.6 6 | A-T-4 |

(continued)

| Exam ple | Co mp oun d | Structure and Name | $^1$H-NMR | LC-MS | Synthesi s Method |
|---|---|---|---|---|---|
| 202 | AR -P-61 | 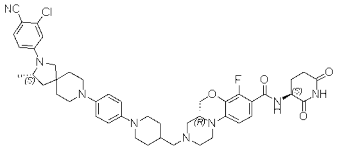 (R)-3-((1-(4-((S)-2-(3-chloro-4-cyanophenyl)-3-methyl-2,8-diazaspiro[4.5]decan-8-yl)phenyl)piperidin-4-yl)methyl)-N-((S)-2,6-dioxopiperidin-3-yl)-7-fluoro-1,2,3,4,4a,5-hexahydrobenzo[b]pyrazi no[1,2-d][1,4]oxazine-8-carboxamide | $^1$H NMR (400 MHz, DMSO-$d_6$) δ 10.88 (s, 1H), 8.23 (dd, *J* = 8.1, 4.3 Hz, 1H), 7.63 (d, *J*= 8.8 Hz, 1H), 7.24 (t, *J*= 8.0 Hz, 2H), 7.19 - 6.97 (m, 2H), 6.92 (d, *J*= 9.0 Hz, 1H), 6.81 (d, *J*= 2.2 Hz, 1H), 6.68 (d, *J* = 9.0 Hz, 1H), 4.72 (s, 1H), 4.42 (d, *J*= 10.9 Hz, 1H), 4.27 - 4.00 (m, 4H), 3.99 - 3.58 (m, 10H), 3.15 (d, *J*= 15.4 Hz, 4H), 2.92 (s, 1H), 2.82 - 2.73 (m, 2H), 2.53 (d, *J* = 6.8 Hz, 3H), 2.17 - 2.06 (m, 2H), 2.03 - 1.94 (m, 2H), 1.83 (d, *J*= 62.6 Hz, 3H), 1.63 (s, 2H), 1.36 (s, 2H), 1.21 (d, *J*= 6.0 Hz, 4H). | 823.4 1 | A-T-3 |
| 203 | AR -P-62 | 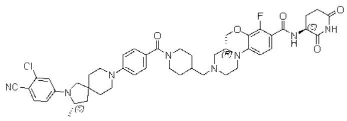 (R)-3-((1-(4-((S)-2-(3-chloro-4-cyanophenyl)-3-methyl-2,8-diazaspiro[4.5]decan-8-yl)benzoyl)piperidin-4-yl)methyl)-N-((S)-2,6-dioxopiperidin-3-yl)-7-fluoro-1,2,3,4,4a,5-hexahydrobenzo[b]pyrazi no[1,2-d][1,4]oxazine-8-carboxamide | $^1$H NMR (600 MHz, DMSO-$d_6$) δ 10.87 (s, 1H), 9.63 (s, 1H), 8.23 (dd, *J*= 8.2, 4.3 Hz, 1H), 7.61 (d, *J* = 8.9 Hz, 1H), 7.27 (d, *J* = 8.6 Hz, 2H), 6.95 (dd, *J*= 26.1, 8.8 Hz, 3H), 6.81 (d, *J* = 2.4 Hz, 1H), 6.67 (dd, *J* = 9.0, 2.4 Hz, 1H), 4.75 - 4.70 (m, 1H), 4.42 (dd, *J*= 11.2, 2.6 Hz, 1H), 4.20 (d, *J*= 13.4 Hz, 1H), 4.11 - 4.04 (m, 4H), 3.44 (d, *J* = 10.8 Hz, 2H), 3.40 - 3.31 (m, 4H), 3.27 - 3.03 (m, 7H), 2.92 (q, *J* = 11.1 Hz, 3H), 2.82 - 2.73 (m, 1H), 2.24 (dd, *J* = 12.8, 7.7 Hz, 1H), 2.12 (dt, *J* = 12.9, 7.4 Hz, 2H), 2.01 - 1.96 (m, 1H), 1.77 - 1.71 (m, 4H), 1.58 (dd, *J*= 12.8, 6.5 Hz, 1H), 1.50 (q, *J* = 6.1, 5.1 Hz, 2H), 1.21 (d, *J* = 6.0 Hz, 6H). | 851.6 0 | A-T-4 |

(continued)

| Exam ple | Co mp oun d | Structure and Name | 1H-NMR | LC-MS | Synthesi s Method |
|---|---|---|---|---|---|
| 204 | AR -P-63 | 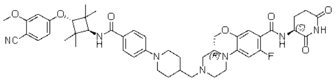 (R)-3-((1-(4-(((1r,3r)-3-((4-cyano-3-methoxyphenoxy)-2,2,4,4-tetramethylcyclobutyl)ca rbamoyl) phenyl)piperidin-4-yl) methyl)-N-((S)-2,6-dioxopiperidin-3-yl)-9-fluoro-1,2,3,4,4a,5-hexahydrobenzo[b]pyrazi no[1,2-d][1,4]oxazine-8-carboxamide | 1H NMR (400 MHz, $CDCl_3$-*d*) δ 7.93 (s, 1H), 7.72 (d, J = 8.8 Hz, 2H), 7.57 (d, J = 7.4 Hz, 1H), 7.50 - 7.39 (m, 2H), 6.99 (d, J = 8.7 Hz, 2H), 6.57 (d, J = 13.6 Hz, 1H), 6.49 (d, J = 2.2 Hz, 1H), 6.41 (dd, J = 8.6, 2.2 Hz, 1H), 6.18 (d, J = 8.2 Hz, 1H), 4.81 (dd, J = 12.5, 5.8 Hz, 1H), 4.32 (dd, J = 11.5, 2.7 Hz, 1H), 4.18 - 4.12 (m, 2H), 4.07 (s, 1H), 4.02 (d, J = 11.1 Hz, 1H), 3.94 (s, 4H), 3.86 (d, J = 12.8 Hz, 2H), 3.70 (t, J = 12.5 Hz, 3H), 3.05 - 2.97 (m, 2H), 2.93 (d, J = 12.9 Hz, 3H), 2.81 (t, J = 5.2 Hz, 1H), 2.78 - 2.69 (m, 2H), 1.57 (q, J = 11.1 Hz, 4H), 1.28 (s, 6H), 1.25 (s, 6H). | 836.7 0 | A-T-2 |
| 205 | AR -P-64 | 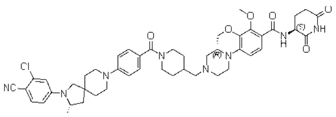 (R)-3-((1-(4-(S)-2-(3-chloro-4-cyanophenyl)-3-methyl-2,8-diazaspiro[4.5]decan-8-yl)benzoyl)piperidin-4-yl)methyl)-N-((S)-2,6-dioxopiperidin-3-yl)-7-methoxy-1,2,3,4,4a,5-hexahydrobenzo[b] pyrazi no[1,2-d][1,4]oxazine-8-carboxamide | 1H NMR (600 MHz, Methanol-d4) δ 7.56 (d, J = 8.8 Hz, 1H), 7.50 (d, J = 8.7 Hz, 1H), 7.36 (d, J = 8.5 Hz, 2H), 7.06 (d, J = 8.4 Hz, 2H), 6.89 (d, J = 8.9 Hz, 1H), 6.78 (d, J = 2.4 Hz, 1H), 6.67 (dd, J = 9.0, 2.3 Hz, 1H), 4.52 - 4.43 (m, 1H), 4.27 (d, J = 11.6 Hz, 1H), 4.20 - 4.12 (m, 1H), 4.08 (q, J = 6.7 Hz, 1H), 3.98 (s, 3H), 3.70 (d, J = 50.7 Hz, 3H), 3.24 - 3.19 (m, 6H),3.05 (d, J = 32.5 Hz, 2H), 2.85 (ddd, J = 18.4, 13.6, 5.6 Hz, 1H), 2.77 - 2.65 (m, 1H), 2.47 - 2.33 (m, 2H), 2.29 (s, 1H), 2.08 (qd, J = 13.1, 5.4 Hz, 1H), 1.87 (d, J = 19.6 Hz, 4H), 1.75 - 1.55 (m, 5H), 1.48 - 1.23 (m, 11H). | 863.7 6 | A-T-4 |

(continued)

| Exam ple | Co mp oun d | Structure and Name | $^1$H-NMR | LC-MS | Synthesi s Method |
| --- | --- | --- | --- | --- | --- |
| 206 | AR -P-65 | 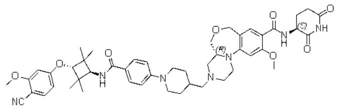 (R)-3-((1-((4-((1r,3r)-3-(4-cyano-3-methoxyphenoxy)-2,2,4,4-tetra-methylcyclobutyl)ca rbamoyl)phe-nyl)piperidi n-4-yl) methyl)-N-((S)-2,6-dioxopiperidin-3-yl)-10-methoxy-1,2,3,4,4a,5-hexahydro-7H-benzo[e]pyrazino [2,1-c][1,4]oxazepine-9-carboxa-mide | $^1$H NMR (600 MHz, DMSO-$d_6$) δ 10.91 (s, 1H), 8.51 (s, 1H), 7.86 - 7.68 (m, 3H), 7.66 (d, *J*= 8.6 Hz, 1H), 7.53 (d, *J*= 9.0 Hz, 1H), 6.99 (s, 2H), 6.71 - 6.59 (m, 2H), 6.54 (dd, *J*= 8.7, 2.1 Hz, 1H), 4.93 - 4.79 (m, 1H), 4.79 - 4.70 (m, 1H), 4.70 - 4.60 (m, 1H), 4.28 (s, 1H), 4.06 (d, *J* = 9.2 Hz, 1H), 4.01 - 3.94 (m, 3H), 3.91 (s, 5H), 3.80 - 3.68 (m, 2H), 3.68 - 3.56 (m, 2H), 3.56 - 3.41 (m, 1H), 3.31 - 3.12 (m, 4H), 2.90 - 2.68 (m, 4H), 2.58 - 2.52 (m, 1H), 2.24 (s, 1H), 2.19 - 2.03 (m, 3H), 2.03 - 1.73 (m, 3H), 1.38 - 1.27 (m, 1H), 1.19 *(d, J*= 47.4 Hz, 12H). | 862.6 6 | A-T-2 |
| 207 | AR -P-66 | 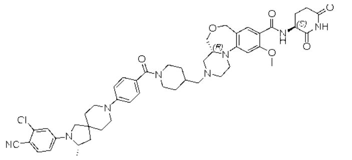 (R)-3-((1-(4-((S)-2-(3-chloro-4-cyanophenyl)-3-methyl-2,8-diazaspiro[4.5]decan-8-yl)benzoyl)piperidin-4-yl)methyl)-N-((S)-2,6-dioxo-piperidin-3-yl)-10-methox-y-1,2,3,4,4a,5-hexahydro-7H-benzo[e]pyrazino[2,1-c][1,4]oxypyri-dine-9-carboxamide | $^1$H NMR (600 MHz, DMSO-$d_6$) δ 10.90 (s, 1H), 8.50 (s, 1H), 7.83 - 7.65 (m, 1H), 7.60 (d, *J* = 8.8 Hz, 1H), 7.26 (d, *J* = 8.2 Hz, 2H), 6.96 (d, *J*= *8.6* Hz, 2H), 6.81 (d, *J*= 2.1 Hz, 1H), 6.71 - 6.56 (m, 2H), 4.79 - 4.55 (m, 3H), 4.08 - 4.00 (m, 1H), 3.96 (s, 3H), 3.73 (s, 2H), 3.63 (s, 2H), 3.44 (d, *J* = 10.8 Hz, 1H), 3.40 - 3.37 (m, 1H), 3.34 - 3.31 (m, 2H), 3.30 - 3.11 (m, 5H), 2.89 (s, 2H), 2.84 - 2.60 (m, 3H), 2.53 (d, *J*= *8.8* Hz, 1H), 2.33 - 2.15 (m, 3H), 2.15 - 2.04 (m, 2H), 2.03 - 1.67 (m, 5H), 1.58 (dd, *J*= 12.8, 6.5 Hz, 1H), 1.55 - 1.41 (m, 2H), 1.30 - 1.15 (m, 5H), 1.10 (s, 2H). | 877.5 6 | A-T-4 |

(continued)

| Exam ple | Co mp oun d | Structure and Name | $^1$H-NMR | LC-MS | Synthesi s Method |
|---|---|---|---|---|---|
| 208 | AR -P-67 | 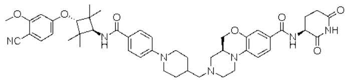 (S)-3-(1-(4-(1r,3r)-3-(4-cyano-3-methoxyphenoxy)-2,2,4,4-tetramethylcyclobutyl)ca rbamoyl)phenyl)piperidi n-4-yl) methyl)-N-((S)-2,6-dioxopiperidin-3-yl)-1,2,3,4,4a,5-hexahydrobenzo[b]pyrazi no[1,2-d][1,4]oxazine-8-carboxamide | $^1$H NMR (600 MHz, DMSO-$d_6$) δ 10.83 (s, 1H), 8.52 (dd, *J*= 8.1, 4.6 Hz, 1H), 7.77 (d, *J*= 8.6 Hz, 2H), 7.65 (d, *J*= 8.6 Hz, 1H), 7.52 (dd, *J* = 8.9, 4.4 Hz, 1H), 7.46 (dd, *J* = 8.6, 2.1 Hz, 1H), 7.33 (d, *J* = 2.1 Hz, 1H), 7.08 (d, *J* = 8.7 Hz, 1H), 7.00 (d, *J*= 8.5 Hz, 2H), 6.64 (d, *J* = 2.2 Hz, 1H), 6.54 (dd, *J* = 8.6, 2.2 Hz, 1H), 4.73 (ddd, *J*= 13.0, 8.3, 5.3 Hz, 1H), 4.36 (dd, *J*= 11.4, 2.8 Hz, 1H), 4.28 (s, 1H), 4.20 (d, J= 13.3 Hz, 1H), 4.05 (dd, *J*= 15.6, 9.7 Hz, 2H), 3.91 (s, 5H), 3.63 (s, 2H), 3.14 (d, *J* = 48.1 Hz, 4H), 2.94 - 2.72 (m, 4H), 2.55 (t, *J*= 3.9 Hz, 1H), 2.18 - 2.05 (m, 2H), 1.94 (ddd, *J*= 10.2, 5.5, 2.4 Hz, 1H), 1.85 (dd, *J*= 42.3, 12.7 Hz, 2H), 1.31 (qd, *J* = 12.9, 12.4, 4.0 Hz, 3H), 1.23 (s, 6H), 1.15 (s, 6H). | 818.6 6 | A-T-2 |
| 209 | AR -P-68 | 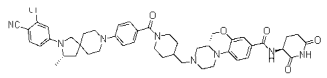 (R)-3-((1-(4-(((S)-2-(3-chloro-4-cyanophenyl)-3-methyl-2,8-diazaspiro[4.5]decan-8-yl)benzoyl)piperidin-4-yl)methyl)-N-(((S)-2,6-dioxopiperidin-3-yl)-1,2,3,4,4a,5-hexahydrobenzo[b]pyrazi no[1,2-d][1,4] oxazine-8-carboxamide | $^1$H NMR (400 MHz, DMSO-$d_6$)δ 10.84 (s, 1H), 8.52 (d, *J* = 8.3 Hz, 1H), 7.61 (d, *J*= 8.8 Hz, 1H), 7.45 (d, *J*= 8.4 Hz, 1H), 7.33 (d, *J* = 2.0 Hz, 1H), 7.26 (s, 2H), 7.08 (s, 1H), 6.97 (d, *J*= 8.5 Hz, 2H), 6.80 (d, *J* = 2.3 Hz, 1H), 6.67 (dd, *J* = 9.0, 2.4 Hz, 1H), 4.78 - 4.67 (m, 1H), 4.35 *(d, J= 11.0* Hz, 1H), 4.20 (d, *J* = 12.3 Hz, 1H), 4.04 (dt, *J*= 12.1, 6.7 Hz, 2H), 3.25 - 3.19 (m, 2H), 3.12 (s, 4H), 3.02 - 2.82 (m, 4H), 2.82 - 2.72 (m, 1H), 2.24 (dd, *J* = 12.8, 7.7 Hz, 2H), 2.13 (dd, *J*= 13.2, 8.6 Hz, 3H), 1.97 (dd, *J*= 27.5, 8.5 Hz, 3H), 1.75 (d, *J*= 6.8 Hz, 5H), 1.59 (dd, *J* = 12.9, 6.5 Hz, 1H), 1.50 (s, 2H), 1.28 - 1.18 (m, 9H). | 833.6 0 | A-T-4 |

(continued)

| Exam ple | Co mp oun d | Structure and Name | $^1$H-NMR | LC-MS | Synthesi s Method |
|---|---|---|---|---|---|
| 210 | AR -P-69 | 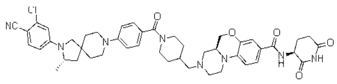 (S)-3-((1-(4-(((S)-2-(3-chloro-4-cyanophenyl)-3-methyl-2,8-diazaspiro[4.5]decan-8-yl)benzoyl)piperidin-4-yl)methyl)-N-(((S)-2,6-dioxopiperidin-3-yl)-1,2,3,4,4a,5-hexahydrobenzo[b]pyrazi no[1,2-d][1,4] oxazine-8-carboxamide | $^1$H NMR (400 MHz, DMSO-$d_6$) $\delta$ 10.84 (s, 1H), 8.51 (d, J = 8.4 Hz, 1H), 7.61 (d, J = 8.8 Hz, 1H), 7.45 (d, J = 8.4 Hz, 1H), 7.33 (d, J = 2.0 Hz, 1H), 7.27 (d, J = 8.5 Hz, 2H), 7.12 - 7.04 (m, 1H), 6.96 (d, J = 8.5 Hz, 2H), 6.80 (d, J = 2.3 Hz, 1H), 6.66 (dd, J = 8.9, 2.4 Hz, 1H), 4.80 - 4.68 (m, 1H), 4.35 (d, J = 11.1 Hz, 1H), 4.20 (d, J = 11.8 Hz, 1H), 4.05 (d, J = 7.0 Hz, 2H), 3.63 (d, J = 13.8 Hz, 2H), 3.12 (d, J = 11.4 Hz, 4H), 2.91 (s, 4H), 2.82 - 2.72 (m, 1H), 2.24 (dd, J = 12.7, 7.6 Hz, 2H), 2.19 - 2.06 (m, 3H), 2.05 - 1.88 (m, 3H), 1.75 (d, J = 7.4 Hz, 5H), 1.59 (dd, J = 12.9, 6.5 Hz, 1H), 1.50 (s, 2H), 1.26 - 1.19 (m, 9H). | 833.6 0 | A-T-4 |
| 211 | AR -P-70 | 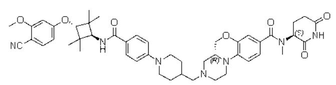 (R)-3-((1-(4-(1r,3r)-3-(4-cyano-3-methoxyphenoxy)-2,2,4,-tetramethylcyclobutyl)ca rbamoyl)phenyl) piperidi n-4-yl)methyl)-N-((S)-2,6-dioxopiperidin-3-yl)-N-methyl-1,2,3,4,4a,5-hexahydrobenzo[b] pyrazi no[1,2-d][1,4]oxazine-8-carboxamide | $^1$H NMR (600 MHz, DMSO-d6) $\delta$ 11.03 - 10.82 (m, 1H), 7.77 (d, J = 8.6 Hz, 2H), 7.66 (d, J = 8.6 Hz, 1H), 7.52 (t, J = 11.4 Hz, 1H), 7.04 (dd, J = 43.3, 20.5 Hz, 4H), 6.86 (t, J = 39.1 Hz, 1H), 6.64 (s, 1H), 6.54 (t, J = 8.0 Hz, 1H), 4.36 (d, J = 9.9 Hz, 2H), 4.28 (s, 1H), 4.15 (s, 2H), 4.06 (d, J = 9.2 Hz, 2H), 3.91 (s, 6H), 3.72 - 3.65 (m, 3H), 3.12 (s, 4H), 2.89 (s, 2H), 2.82 (t, J = 12.6 Hz, 3H), 2.77 (s, 1H), 2.43 - 2.29 (m, 1H), 2.09 (d, J = 6.6 Hz, 1H), 1.95 (dd, J = 17.7, 12.3 Hz, 1H), 1.88 (d, J = 12.0 Hz, 1H), 1.79 (t, J = 16.8 Hz, 1H), 1.36 - 1.27 (m, 2H), 1.23 (s, 6H), 1.15 (s, 6H). | 832.6 6 | A-T-2 |

(continued)

| Exam ple | Co mp oun d | Structure and Name | $^1$H-NMR | LC-MS | Synthesi s Method |
|---|---|---|---|---|---|
| 212 | AR -P-71 | 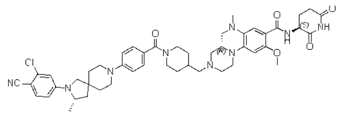 (R)-3-((1-(4-((S)-2-(3-chloro-4-cyanophenyl)-3-methyl-2,8-diazaspiro[4.5]decan-8-yl)benzoyl)piperidin-4-yl)methyl)-N-((S)-2,6-dioxopiperidin-3-yl)-9-methoxy-6-methyl-2,3,4,4a,5,6-hexahydro-1H-pyrazino[1,2-a]quinoxaline-8-carboxamide | $^1$H NMR (600 MHz, DMSO-$d_6$) δ 10.89 (s, 1H), 8.56 - 8.47 (m, 1H), 7.61 (d, J = 8.9 Hz, 1H), 7.27 (d, J = 8.5 Hz, 2H), 7.18 - 7.10 (m, 1H), 6.97 (d, J = 8.5 Hz, 2H), 6.81 (d, J = 1.7 Hz, 1H), 6.67 (d, J = 9.0 Hz, 1H), 6.56 (d, J = 24.2 Hz, 1H), 4.68 (dt, J = 11.9, 5.5 Hz, 1H), 4.30 (d, J = 13.1 Hz, 1H), 4.04 (dd, J = 13.1, 6.5 Hz, 2H), 3.87 (d, J = 10.0 Hz, 3H), 3.44 (d, J = 10.8 Hz, 2H), 3.35 (dd, J = 28.1, 13.6 Hz, 4H), 3.23 (dd, J = 27.1, 13.2 Hz, 4H), 3.09 (s, 4H), 3.01 - 2.83 (m, 4H), 2.81 - 2.70 (m, 4H), 2.52 (d, J = 7.0 Hz, 1H), 2.24 (dd, J = 12.7, 7.7 Hz, 1H), 2.19 - 2.10 (m, 2H), 2.06 (dt, J = 12.7, 7.8 Hz, 1H), 1.82 (s, 1H), 1.79 - 1.67 (m, 3H), 1.59 (dd, J = 12.8, 6.4 Hz, 1H), 1.55 - 1.45 (m, 2H), 1.30 - 1.12 (m, 6H). | 876.4 6 | A-T-4 |
| 213 | AR -P-72 | 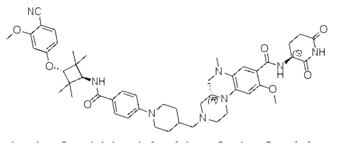 (R)-3-((1-(4-(1r,3r)-3-(4-cyano-3-methoxyphenoxy)-2,2,4,4-tetramethylcyclobutyl)ca rbamoyl)phenyl)piperidi n-4-yl) methyl)-N-((S)-2,6-dioxopiperidin-3-yl)-9-methoxy-6-methyl-2,3,4a,5,6-hexahydro-1H-pyrazino[1,2-a]quinoxaline-8-carboxamide | $^1$H NMR (600 MHz, DMSO-$d_6$) δ 10.90 (d, J = 11.4 Hz, 1H), 8.55 - 8.49 (m, 1H), 7.77 (d, J = 8.7 Hz, 2H), 7.65 (t, J = 10.5 Hz, 1H), 7.54 (d, J = 9.2 Hz, 1H), 7.14 (d, J = 13.8 Hz, 1H), 7.00 (d, J = 8.8 Hz, 2H), 6.64 (d, J = 1.8 Hz, 1H), 6.59 (s, 1H), 6.54 (dd, J = 8.7, 1.9 Hz, 1H), 4.69 (td, J = 12.2, 5.8 Hz, 1H), 4.30 (d, J = 18.3 Hz, 2H), 4.06 (d, J = 9.2 Hz, 2H), 3.91 (s, 5H), 3.88 (s, 2H), 3.32 (s, 1H), 3.24 (dd, J = 26.3, 11.7 Hz, 3H), 3.16 (d, J = 10.0 Hz, 1H), 3.11 (s, 3H), 3.04 - 2.96 (m, 1H), 2.94 - 2.86 (m, 1H), 2.82 (t, J = 13.3 Hz, 2H), 2.79 - 2.70 (m, 4H), 2.19 - 2.04 (m, 3H), 1.92 - 1.85 (m, 1H), 1.80 (d, J = 11.9 Hz, 1H), 1.36 - 1.25 (m, 3H), 1.23 (s, 6H), 1.15 (s, 6H). | 861.7 0 | A-T-2 |

(continued)

| Exam ple | Co mp oun d | Structure and Name | $^1$H-NMR | LC-MS | Synthesi s Method |
|---|---|---|---|---|---|
| 214 | AR -P-73 | 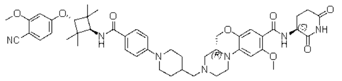 (R)-3-((1-(4-((((1r,3r)-3-((4-cyano-3-methoxyphenoxy)-2,2,4,4-tetramethylcyclobutyl)ca rbamoyl) phenyl)piperidi n-4-yl) methyl)-N-((S)-2,6-dioxopiperidin-3-yl)-9-methoxy-1,2,3,4,4a,5-hexahydrobenzo[b]pyrazi no[1,2-d][1,4]oxazine-8-carboxamide | $^1$H NMR (600 MHz, DMSO-$d_6$) δ 10.90 (s, 1H), 9.65 (s, 1H), 8.51 (d, *J*= 7.1 Hz, 1H), 7.77 (d, *J*= 8.6 Hz, 2H), 7.66 (d, *J*= 8.6 Hz, 1H), 7.53 (d, *J* = 9.3 Hz, 1H), 7.31 (s, 1H), 7.00 (d, *J* = 8.6 Hz, 2H), 6.72 (s, 1H), 6.65 (d, *J*= 2.2 Hz, 1H), 6.54 (dd, *J*= 8.7, 2.2 Hz, 1H), 4.70 (dt, *J* = 12.6, 6.4 Hz, 1H), 4.34 (s, 3H), 4.06 (d, *J* = 9.2 Hz, 1H), 3.98 (dd, *J* = 11.0, 6.5 Hz, 1H), 3.91 (d, *J*= 4.5 Hz, 8H), 3.70 (d, *J* = 11.5 Hz, 1H), 3.63 (s, 2H), 3.25 - 3.20 (m, 1H), 3.11 (s, 3H), 2.89 (d, *J*= 10.6 Hz, 1H), 2.82 (td, *J*= 13.7, 11.8, 4.2 Hz, 2H), 2.79 - 2.72 (m, 1H), 2.15 - 2.03 (m, 3H), 1.89 (d, *J* = 13.1 Hz, 1H), 1.80 (d, *J* = 12.8 Hz, 1H), 1.32 (t, *J*= 11.0 Hz, 2H), 1.23 (s, 6H), 1.15 (s, 6H). | 848.6 5 | A-T-2 |
| 215 | AR -P-74 | 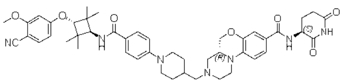 (R)-3-((1-(4-(((1R,3R)-3-(4-cyano-3-methoxyphenoxy)-2,2,4,4-tetramethylcyclobutyl)ca rbamoyl) phenyl)piperidi n-4-yl) methyl)-N-((S)-2,6-dioxopiperidin-3-yl)-1,2,3,4,4a,5-hexahydrobenzo[b]pyrazi no[1,2-d][1,4]oxazine-8-carboxamide | $^1$H NMR (400 MHz, DMSO-$d_6$) δ 10.85 (s, 1H), 8.53 (d, J = 8.4 Hz, 1H), 7.77 (d, J = 8.6 Hz, 2H), 7.66 (d, J = 8.6 Hz, 1H), 7.53 (d, J = 9.3 Hz, 1H), 7.46 (dd, J = 8.5, 2.1 Hz, 1H), 7.33 (d, J = 2.0 Hz, 1H), 7.08 (d, J = 8.8 Hz, 1H), 7.00 (d, J = 8.7 Hz, 2H), 6.64 (d, J = 2.2 Hz, 1H), 6.54 (dd, J = 8.7, 2.2 Hz, 1H), 4.73 (ddd, J = 12.9, 8.3, 5.4 Hz, 1H), 4.36 (dd, J = 11.2, 2.8 Hz, 1H), 4.28 (s, 1H), 4.20 (d, J = 12.8 Hz, 1H), 4.13 - 3.99 (m, 2H), 3.91 (s, 6H), 3.62 - 3.557 (m, 2H), 3.23 - 3.03 (m, 4H), 2.81 (tdd, J = 18.4, 11.7, 6.4 Hz, 4H), 2.21 - 2.03 (m, 2H), 1.99 - 1.74 (m, 4H), 1.23 (s, 8H), 1.15 (s, 6H). | 818.6 6 | A-T-2 |

(continued)

| Exam ple | Co mp oun d | Structure and Name | $^1$H-NMR | LC-MS | Synthesi s Method |
|---|---|---|---|---|---|
| 216 | AR -P-75 | 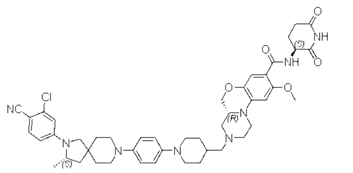 (R)-3-((1-(4-((S)-2-(3-chloro-4-cyanophenyl)-3-methyl-2,8-diazaspiro[4.5]decan-8-yl)phenyl)piperidin-4-yl)methyl)-N-(S)-2,6-dioxopiperidin-3-yl)-9-methoxy-1,2,3,4,4a,5-hexahydrobenzo[b] pyrazi no[1,2-d][1,4]oxazine-8-carboxamide | $^1$H NMR (600 MHz, DMSO-d6) δ 10.90 (s, 1H), 9.92 (s, 1H), 8.51 (d, J = 7.0 Hz, 1H), 7.63 (d, J = 8.8 Hz, 1H), 7.31 (s, 1H), 7.28 - 6.98 (m, 3H), 6.82 (d, J = 2.3 Hz, 1H), 6.76 - 6.62 (m, 2H), 4.70 (dt, J = 12.6, 6.4 Hz, 1H), 4.28 (dd, J = 11.2, 2.7 Hz, 2H), 4.02 (dt, J = 48.3, 7.7 Hz, 2H), 3.90 (s, 3H), 3.45 (s, 5H), 3.41 - 3.28 (m, 5H), 3.26 - 3.01 (m, 5H), 2.89 (s, 2H), 2.76 (ddd, J = 17.1, 13.3, 6.0 Hz, 2H), 2.53 (s, 1H), 2.23 (s, 1H), 2.15 - 2.00 (m, 3H), 1.90 (d, J = 44.7 Hz, 4H), 1.70 - 1.30 (m, 5H), 1.22 (d, J = 6.0 Hz, 3H). | 835.5 6 | A-T-3 |
| 217 | AR -P-76 | 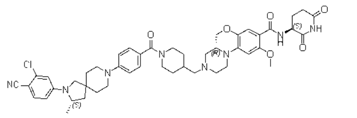 (R)-3-((1-(4-(S)-2-(3-chloro-4-cyanophenyl)-3-methyl-2,8-diazaspiro[4.5]decan-8-yl)benzoyl)piperidin-4-yl)methyl)-N-((S)-2,6-dioxopiperidin-3-yl)-9-methoxy-1,2,3,4,4a,5-hexahydrobenzo[b] pyrazi no[1,2-d][1,4]oxazine-8-carboxamide | $^1$H NMR (600 MHz, DMSO-d6) δ 10.90 (s, 1H), 8.51 (dd, J = 7.0, 5.0 Hz, 1H), 7.61 (d, J = 8.8 Hz, 1H), 7.30 (d, J = 1.2 Hz, 1H), 7.27 (d, J = 8.5 Hz, 2H), 6.97 (d, J = 8.5 Hz, 2H), 6.81 (d, J = 2.3 Hz, 1H), 6.72 (s, 1H), 6.67 (dd, J = 9.0, 2.4 Hz, 1H), 4.69 (dd, J = 12.2, 6.2 Hz, 1H), 4.35 - 4.25 (m, 2H), 4.04 (d, J = 6.8 Hz, 1H), 3.97 (s, 1H), 3.90 (s, 3H), 3.68 (d, J = 10.8 Hz, 2H), 3.62 (s, 2H), 3.44 (d, J = 10.8 Hz, 1H), 3.41 - 3.30 (m, 3H), 3.22 (m, 3H), 3.09 (s, 3H), 3.03 - 2.67 (m, 5H), 2.56 - 2.51 (m, 1H), 2.24 (dd, J = 12.8, 7.7 Hz, 1H), 2.19 - 2.04 (m, 3H), 1.82 (s, 1H), 1.73 (dtd, J = 21.5, 12.9, 11.2, 5.9 Hz, 3H), 1.58 (dd, J = 12.8, 6.5 Hz, 1H), 1.50 (h, J = 9.1 Hz, 2H), 1.21 (d, J = 6.1 Hz, 5H). | 863.6 6 | A-T-4 |

(continued)

| Exam ple | Co mp oun d | Structure and Name | $^1$H-NMR | LC-MS | Synthesi s Method |
|---|---|---|---|---|---|
| 218 | AR -P-77 | 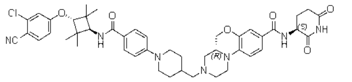 (R)-3-((1-(4-(1R,3R)-3-(3-chloro-4-cyanophenoxy)-2,2,4-tetramethylcyclobutyl)ca rbamoyl) phenyl)piperidi n-4-yl)) methyl)-N-((S)-2,6-dioxopiperidin-3-yl)-1,2,3,4a,5-hexahydrobenzo[b]pyrazi no[1,2-d][1,4]oxazine-8-carboxamide | $^1$H NMR (400 MHz, DMSO-$d_6$) δ 10.85 (s, 1H), 8.50 (dd, J = 24.8, 8.5 Hz, 1H), 7.91 (d, J = 8.7 Hz, 1H), 7.76 (d, J = 8.8 Hz, 2H), 7.52 (d, J = 9.2 Hz, 1H), 7.46 (dd, J = 8.5, 1.6 Hz, 1H), 7.33 (d, J = 1.7 Hz, 1H), 7.21 (d, J = 2.3 Hz, 1H), 7.08 (d, J = 8.8 Hz, 1H), 7.04 - 6.93 (m, 3H), 4.79 - 4.68 (m, 1H), 4.39 - 4.29 (m, 2H), 4.20 (d, J = 12.2 Hz, 1H), 4.09 - 3.98 (m, 2H), 3.91 (d, J = 9.9 Hz, 2H), 3.69 (d, J = 9.3 Hz, 1H), 3.22 - 3.01 (m, 4H), 2.93 - 2.72 (m, 4H), 2.59 - 2.53 (m, 1H), 2.17 - 2.02 (m, 2H), 2.01 - 1.73 (m, 4H), 1.39 - 1.17 (m, 9H), 1.13 (s, 6H). | 822.6 6 | A-T-2 |
| 219 | AR -P-78 | 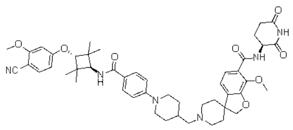 1'-((1-(4-(((1r,3r)-3-(4-cyano-3-methoxyphenoxy)-2,2,4,4-tetramethylcyclobutyl)ca rbamoyl)phenyl)piperidi n-4-yl) methyl)-N-((S)-2,6-dioxopiperidin-3-yl)-7-methoxy-2H-spiro[benzofuran-3,4'-piperidine]-6-carboxamide | $^1$H NMR (600 MHz, DMSO) δ 10.91 (s, 1H), 8.69 (d, J = 8.4 Hz, 1H), 7.77 (d, J = 8.8 Hz, 2H), 7.66 (d, J = 8.6 Hz, 1H), 7.54 (d, J = 9.3 Hz, 1H), 7.44 (s, 1H), 7.33 (s, 1H), 7.00 (d, J = 8.9 Hz, 2H), 6.64 (d, J = 2.0 Hz, 1H), 6.54 (dd, J = 8.6, 2.0 Hz, 1H), 4.82 - 4.77 (m, 1H), 4.62 (q, J = 9.5 Hz, 2H), 4.28 (s, 1H), 4.06 (d, J = 9.2 Hz, 1H), 3.91 (s, 3H), 3.85 (s, 3H), 3.60 (d, J = 11.6 Hz, 3H), 3.34 (d, J = 24.0 Hz, 1H), 3.07 (s, 4H), 2.89 - 2.78 (m, 3H), 2.59 - 2.53 (m, 1H), 2.23 (dd, J = 24.2, 13.2 Hz, 2H), 2.11 (td, J = 13.2, 4.7 Hz, 2H), 2.02 - 1.92 (m, 3H), 1.84 (d, J = 11.7 Hz, 2H), 1.32 (dd, J = 21.8, 10.7 Hz, 2H), 1.23 (s, 6H), 1.15 (s, 6H). | 847.5 6 | A-T-2 |

(continued)

| Exam ple | Co mp oun d | Structure and Name | 1H-NMR | LC-MS | Synthesi s Method |
|---|---|---|---|---|---|
| 220 | AR -P-79 | 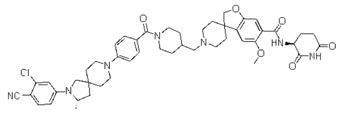 1'-((1-(4-(S)-2-(3-chloro-4-cyanophenyl)-3-methyl-2,8-diazaspiro [4.5]decan-8-yl)benzoyl)piperidin-4-yl)methyl)-N-((S)-2,6-dioxopiperidin-3-yl)-5-methoxy-2H-spiro[benzofuran-3,4'-piperidin]-6-carboxamide | 1H NMR (400 MHz, DMSO-d6) δ 10.91 (s, 1H), 8.67 (d, J = 7.2 Hz, 1H), 7.61 (d, J = 8.8 Hz, 1H), 7.30 - 7.21 (m, 3H), 6.97 (d, J = 8.4 Hz, 2H), 6.89 - 6.78 (m, 2H), 6.66 (dd, J = 8.9, 2.4 Hz, 1H), 4.80 - 4.65 (m, 1H), 4.49 (d, J = 19.6 Hz, 2H), 4.05 (p, J = 6.5 Hz, 2H), 3.90 (s, 3H), 3.41 - 3.16 (m, 8H), 3.15-2.82 (m, 6H),2.77 (t, J = 8.5 Hz, 1H), 2.24 (dd, J = 12.9, 8.3 Hz, 3H), 2.08 (td, J = 11.0, 10.4, 4.2 Hz, 3H), 1.93 (d, J = 13.6 Hz, 2H), 1.84 - 1.69 (m, 4H), 1.65 - 1.41 (m, 4H), 1.21 (q, J = 7.4 Hz, 6H). | 862.5 6 | A-T-4 |
| 221 | AR -P-80 | 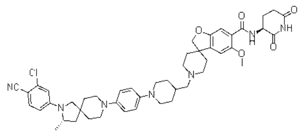 1'-((1-(4-((S)-2-(3-chloro-4-cyanophenyl)-3-methyl-2,8-diazaspiro [4.5]decan-8-yl)phenyl)piperidin-4-yl)methyl)-N-((S)-2,6-dioxopiperidin-3-yl)-5-methoxy-2H-spiro[benzofuran-3,4'-piperidin]-6-carboxamide | 1H NMR (600 MHz, DMSO-d6) δ 10.91 (s, 1H), 9.37 (s, 1H), 8.67 (d, J = 7.4 Hz, 1H), 7.63 (d, J = 8.8 Hz, 1H), 7.25 (s, 4H), 6.95 - 6.60 (m, 3H), 4.73 (dt, J = 10.9, 7.6 Hz, 1H), 4.53 (s, 2H), 4.13 - 3.86 (m, 5H), 3.38 (s, 8H), 3.08 (d, J = 15.4 Hz, 5H), 2.89 - 2.67 (m, 2H), 2.10 (ddt, J = 108.9, 101.2, 17.8 Hz, 13H), 1.71 - 1.17 (m, 9H). | 834.5 6 | A-T-3 |
| 222 | AR -P-81 | 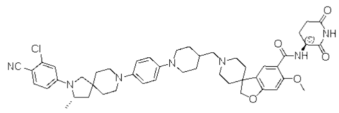 1'-((1-(4-((S)-2-(3-chloro-4-cyanophenyl)-3-methyl-2,8-diazaspiro [4.5]decan-8-yl)phenyl)piperidin-4-yl)methyl)-N-((S)-2,6-dioxopiperidin-3-yl)-6-methoxy-2H-spiro[benzofuran-3,4'-piperidin]-5-carboxamide | 1H NMR (600 MHz, Methanol-d4) δ 7.89 (s, 1H), 7.53 (d, J = 8.8 Hz, 1H), 7.45 (d, J = 8.5 Hz, 2H), 7.23 (d, J = 8.7 Hz, 2H), 6.83 (d, J = 2.4 Hz, 1H), 6.74 - 6.65 (m, 2H), 4.78 (dd, J = 12.8, 5.3 Hz, 1H), 4.66 (s, 2H), 4.12 (q, J = 6.7 Hz, 1H), 4.01 (s, 3H), 3.87 (d, J = 12.6 Hz, 2H), 3.73 (d, J = 12.6 Hz, 2H), 3.63 - 3.53 (m, 4H), 3.48 (d, J = 10.5 Hz, 1H), 3.21 - 3.13 (m, 4H), 3.10 - 3.01 (m, 2H), 2.89 - 2.79 (m, 1H), 2.77 - 2.68 (m, 1H), 2.46 (s, 1H), 2.43 - 2.36 (m, 2H), 2.29 (t, J = 14.2 Hz, 2H), 2.21 (s, 2H), 2.15 - 2.10 (m, 2H), 2.10 - 2.01 (m, 4H), 1.87 (s, 2H), 1.77 (dd, J = 13.0, 7.0 Hz, 1H), 1.58 (d, J = 12.8 Hz, 2H), 1.33 (d, J = 6.1 Hz, 3H). | 834.4 6 | A-T-3 |

(continued)

| Exam ple | Co mp oun d | Structure and Name | $^1$H-NMR | LC-MS | Synthesi s Method |
|---|---|---|---|---|---|
| 223 | AR -P-82 | 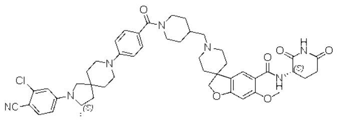 1'-((1-(4-((S)-2-(3-chloro-4-cyanophenyl)-3-methyl-2,8-diazaspiro [4.5]decan-8-yl)benzoyl)piperidin-4-yl)methyl)-N-((S)-2,6-dioxohesperidin-3-yl)-6-methoxy-2H-spiro[benzofuran-3,4'-piperidin]-5-carboxamide | 1H NMR (400 MHz, Methanol-d4) δ 7.88 (s, 1H), 7.50 (d, J = 8.8 Hz, 1H), 7.37 (d, J = 8.5 Hz, 2H), 7.08 (d, J = 8.6 Hz, 2H), 6.78 (d, J = 2.3 Hz, 1H), 6.70 (s, 1H), 6.69 - 6.64 (m, 1H), 4.82 - 4.74 (m, 1H), 4.65 (s, 1H), 4.57 (d, J = 6.1 Hz, 1H), 4.13 - 4.03 (m, 1H), 4.00 (s, 3H), 3.71 (d, J = 12.6 Hz, 2H), 3.51 - 3.36 (m, 4H), 3.20 - 3.14 (m, 2H), 3.14 - 3.07 (m, 2H), 2.94 - 2.79 (m, 3H), 2.77 - 2.65 (m, 2H), 2.50 - 2.34 (m, 3H), 2.25 (t, J = 13.8 Hz, 3H), 2.19 - 2.02 (m, 4H), 1.96 - 1.79 (m, 4H), 1.73 - 1.60 (m, 3H), 1.43 - 1.33 (m, 3H), 1.31 (d, J = 6.0 Hz, 3H). | 863.4 4 | A-T-4 |
| 224 | AR -P-83 | 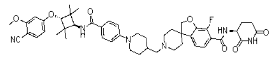 1-(1-(4-((1R,3R)-3-(4-cyano-3-methoxyphenoxy)-2,2,4,4-tetra-methylcyclobutyl)ca rbamoyl)phe-nyl)piperidi n-4-yl) methyl)-N-((S)-2,6-dioxopiperidin-3-yl)-7-fluoro-2H-spiro[benzofuran-3,4'-piperidin]-6-carboxamide | 1H NMR (600 MHz, DMSO-d6) δ 10.86 (s, 1H), 8.55 (d, J = 8.2 Hz, 1H), 7.76 (d, J = 8.4 Hz, 2H), 7.65 (d, J = 8.6 Hz, 1H), 7.51 (d, J = 9.2 Hz, 1H), 7.16 (s, 2H), 6.98 (d, J = 8.5 Hz, 2H), 6.64 (d, J = 2.2 Hz, 1H), 6.54 (dd, J = 8.7, 2.2 Hz, 1H), 4.74 (ddd, J = 13.1, 8.2, 5.5 Hz, 1H), 4.61 (s, 2H), 4.28 (s, 1H), 4.06 (d, J = 9.2 Hz, 1H), 3.91 (m, 6H), 2.78 (ddd, J = 18.1, 13.5, 6.0 Hz, 4H), 2.55 (d, J = 3.8 Hz, 2H), 2.13 - 2.03 (m, 2H), 2.00 (dt, J = 15.7, 6.6 Hz, 3H), 1.83 (d, J = 12.6 Hz, 3H), 1.24 (m, 12H), 1.15 (s, 6H). | 835.6 6 | A-T-2 |

(continued)

| Exam ple | Co mp oun d | Structure and Name | 1H-NMR | LC-MS | Synthesi s Method |
|---|---|---|---|---|---|
| 225 | AR -P-84 | 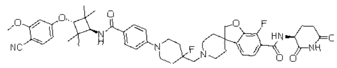 1-(1-(4-((1R,3R)-3-(4-cyano-3-methoxyphenoxy)-2,2,4,4-tetramethylcyclobutyl)ca rbamoyl)phenyl)-4-fluoropiperidin-4-yl) methyl)-N-((S)-2,6-dioxopiperidin-3-yl)-7-fluoro-2H-spiro[benzofuran-3,4'-piperidin]-6-carboxamide | 1H NMR (600 MHz, DMSO-d6) δ 10.86 (s, 1H), 8.57 (dd, J = 8.2, 1.8 Hz, 1H), 7.80 (d, J = 8.8 Hz, 2H), 7.65 (d, J = 8.6 Hz, 1H), 7.54 (d, J = 9.2 Hz, 1H), 7.32 - 7.16 (m, 1H), 7.05 (t, J = 9.4 Hz, *3H),* 6.64 (d, J = 2.2 Hz, 1H), 6.54 (dd, J = 8.7, 2.2 Hz, 1H), 4.74 (ddd, J = 12.9, 8.2, 5.4 Hz, 1H), 4.69 (s, 2H), 4.28 (s, 1H), 4.06 (d, J = 9.2 Hz, 1H), 3.91 (s, 3H), 3.64 - 3.50 (m, 4H), 3.23 (s, 2H), 3.19 - 3.08 (m, 3H), 2.78 (ddd, J = 17.2, 13.2, 5.6 Hz, 1H), 2.57 - 2.51 (m, 1H), 2.30 (d, J = 14.5 Hz, 2H), 2.12 - 1.79 (m, 9H), 1.23 (s, 6H), 1.15 (s, 6H). | 853.7 6 | A-T-2 |
| 226 | AR -P-85 | 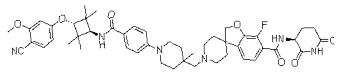 1'-((1-(4-(((1r,3r)-3-(4-cyano-3-methoxyphenoxy)-2,2,4,4-tetramethylcyclobutyl)ca rbamoyl)phenyl)-4-methylpiperidin-4-yl) methyl)-N-((S)-2,6-dioxopiperidin-3-yl)-7-fluoro-2H-spiro[benzofuran-3,4'-piperidin]-6-carboxamide | 1H NMR (600 MHz, DMSO-d6) δ 10.87 (s, 1H), 8.57 (dd, J = 8.2, 1.8 Hz, 1H), 7.78 (d, J = 8.6 Hz, 2H), 7.66 (d, J = 8.6 Hz, 1H), 7.52 (dd, J = 9.4, 4.6 Hz, 1H), 7.24 - 7.16 (m, 1H), 7.04 (d, J = 7.7 Hz, 1H), 7.01 (d, J = 8.7 Hz, 2H), 6.65 (d, J = 2.2 Hz, 1H), 6.54 (dd, J = 8.6, 2.2 Hz, 1H), 4.75 (ddd, J = 12.9, 8.2, 5.4 Hz, 1H), 4.69 (d, J = 6.5 Hz, 2H), 4.28 (s, 1H), 4.06 (d, J = 9.2 Hz, 1H), 3.91 (s, 2H), 3.59 (q, J = 12.5, 10.7 Hz, 4H), 3.38 - 3.11 (m, 7H), 2.79 (ddd, J = 17.1, 13.2, 5.6 Hz, 1H), 2.55 (d, J = 4.0 Hz, 1H), 2.39 - 2.25 (m, 2H), 2.08 (tt, J = 12.7, 6.4 Hz, 1H), 2.03 - 1.89 (m, 3H), 1.70 (td, J = 15.7, 12.1, 3.9 Hz, 2H), 1.62 (d, J = 13.7 Hz, 2H), 1.23 (s, 9H), 1.15 (s, 6H). | 849.6 6 | A-T-2 |

(continued)

| Exam ple | Co mp oun d | Structure and Name | 1H-NMR | LC-MS | Synthesi s Method |
|---|---|---|---|---|---|
| 227 | AR -P-86 | 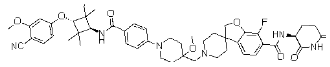 1'-((1-(4-(((1r,3r)-3-(4-cyano-3-methoxyphenoxy)-2,2,4,4-tetramethylcyclobutyl)ca rbamoyl)phenyl)-4-methoxypiperidin-4-yl) methyl)-N-((S)-2,6-dioxopiperidin-3-yl)-7-fluoro-2H-spiro[benzofuran-3,4'-piperidin]-6-carboxamide | 1H NMR (600 MHz, DMSO-d6) δ 10.87 (s, 1H), 8.90 (s, 1H), 8.60 - 8.54 (m, 1H), 7.79 (d, J = 8.6 Hz, 2H), 7.66 (d, J = 8.6 Hz, 1H), 7.52 (d, J = 9.3 Hz, 1H), 7.21 (td, J = 9.4, 8.6, 5.4 Hz, 1H), 7.04 - 7.01 (m, 2H), 6.64 (d, J = 2.1 Hz, 1H), 6.55 (dd, J = 8.6, 2.1 Hz, 1H), 4.75 (ddd, J = 12.9, 8.2, 5.4 Hz, 1H), 4.68 (d, J = 25.9 Hz, 2H), 4.28 (s, 1H), 4.06 (d, J = 9.2 Hz, 1H), 3.91 (s, 3H), 3.72 - 3.60 (m, 4H), 3.36 (s, 2H), 3.28 (d, J = 15.8 Hz, 5H), 3.08 (d, J = 11.7 Hz, 2H), 2.79 (ddd, J = 18.2, 13.2, 5.6 Hz, 1H), 2.55 (d, J = 4.0 Hz, 1H), 2.37 - 2.23 (m, 2H), 2.09 - 2.05 (m, 1H), 2.04 - 1.89 (m, 5H), 1.73 - 1.65 (m, 2H), 1.23 (s, 6H), 1.16 (s, 6H). | 865.7 6 | A-T-2 |
| 228 | AR -P-87 | 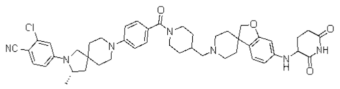 2-chloro-4-((3 S)-8-(4-(4-((6-((2,6-dioxopiperidin-3-yl)amino)-2H-spiro[benzofuran-3,4'-piperidin]-1'-yl)methyl)piperidin-1-carbonyl)phenyl)-3-methyl-2,8-diazaspiro[4.5]decan-2-yl)benzonitrile | 1H NMR (600 MHz, DMSO-d6) δ 10.78 (s, 1H), 9.01 (s, 1H), 7.59 (d, J = 8.9 Hz, 1H), 7.24 (d, J = 8.7 Hz, 2H), 6.95 (d, J = 8.4 Hz, 2H), 6.81 - 6.75 (m, 2H), 6.65 (dd, J = 8.9, 2.4 Hz, 1H), 6.22 (dd, J = 8.2, 2.0 Hz, 1H), 6.15 (d, J = 2.0 Hz, 1H), 4.38 (s, 2H), 4.26 (s, 2H), 4.03 (q, J = 6.6 Hz, 1H), 3.52 (s, 3H), 3.23 - 3.17 (m, 5H), 3.00 (q, J = 12.3, 9.1 Hz, 4H), 2.75 - 2.69 (m, 1H), 2.56 (dt, J = 17.9, 4.3 Hz, 1H), 2.23 (dd, J = 12.8, 7.7 Hz, 1H), 2.15 - 2.02 (m, 4H), 1.98 (d, J = 24.4 Hz, 1H), 1.84 (dd, J = 12.2, 4.5 Hz, 1H), 1.78 (t, J = 13.7 Hz, 3H), 1.75 - 1.67 (m, 3H), 1.57 (dd, J = 12.8, 6.5 Hz, 1H), 1.49 (q, J = 5.6 Hz, 3H), 1.23 (d, J = 6.1 Hz, 2H), 1.19 (d, J = 6.1 Hz, 4H). | 804.6 0 | A-T-4 |

(continued)

| Exam ple | Co mp oun d | Structure and Name | 1H-NMR | LC-MS | Synthesi s Method |
|---|---|---|---|---|---|
| 229 | AR -P-88 | 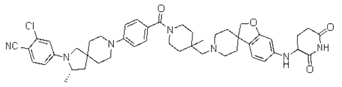 2-chloro-4-((3S)-8-(4-(4-((6-((2,6-dioxopiperidin-3-yl)amino)-2H-spiro[benzofuran-3,4'-piperidin]-1'-yl)methyl)-4-methylpiperidine-1-carbonyl)phenyl)-3-methyl-2,8-diazaspiro[4.5]decan-2-yl)benzonitrile | 1H NMR (600 MHz, Methanol-d4) δ 7.50 (d, J = 8.9 Hz, 1H), 7.45 - 7.37 (m, 2H), 7.14 (d, J = 8.7 Hz, 2H), 6.97 - 6.89 (m, 1H), 6.78 (d, J = 2.4 Hz, 1H), 6.67 (dd, J = 8.9, 2.4 Hz, 1H), 6.38 - 6.22 (m, 2H), 4.51 - 4.00 (m, 5H), 3.68 (d, J = 12.8 Hz, 2H), 3.54 - 3.36 (m, 8H), 3.32 - 3.16 (m, 4H), 2.88 - 2.66 (m, 2H), 2.46 - 2.15 (m, 4H), 1.92 (ddt, J = 19.4, 15.2, 6.5 Hz, 5H), 1.76 - 1.52 (m, 7H), 1.41 - 1.22 (m, 7H). | 818.6 6 | A-T-4 |
| 230 | AR -P-89 | 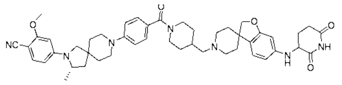 4-((3S)-8-(4-((6-((2,6-dioxopiperidin-3-yl)amino)-2H-spiro[benzofuran-3,4'-piperidin]-1'-yl)methyl) piperidin-1-carbonyl)phenyl)-3-methyl-2,8-diazaspiro[4.5]decan-2-yl)-2-methoxybenzonitrile | 1H NMR (400 MHz, DMSO-d6) δ 10.80 (s, 1H), 8.99 (s, 1H), 7.37 (d, J = 8.7 Hz, 1H), 7.26 (d, J = 8.3 Hz, 2H), 6.97 (d, J = 8.3 Hz, 2H), 6.79 (d, J = 8.0 Hz, 1H), 6.29 - 6.21 (m, 2H), 6.18 (dd, J = 9.2, 1.9 Hz, 2H), 4.39 (s, 2H), 4.28 (q, J = 6.2, 5.5 Hz, 2H), 4.04 (q, J = 6.7 Hz, 2H), 3.87 (s, 3H), 3.24 (t, J = 6.5 Hz, 4H), 3.02 (t, J = 10.5 Hz, 6H), 2.73 (ddd, J = 17.3, 11.7, 5.2 Hz, 3H), 2.25 (dd, J = 12.7, 7.7 Hz, 2H), 2.15 - 2.02 (m, 4H), 2.02 - 1.92 (m, 2H), 1.91 - 1.80 (m, 3H), 1.76 (t, J = 10.0 Hz, 5H), 1.58 (dd, J = 12.8, 6.6 Hz, 2H), 1.51 (d, J = 6.5 Hz, 2H), 1.23 (d, J = 6.1 Hz, 3H). | 800.6 6 | A-T-4 |

(continued)

| Exam ple | Co mp oun d | Structure and Name | 1H-NMR | LC-MS | Synthesi s Method |
|---|---|---|---|---|---|
| 231 | AR -P-90 | 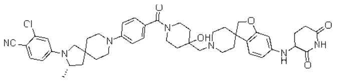 2-chloro-4-((3 S)-8-(4-(4-((6-((2,6-dioxopyrimidin-3-yl)amino)-2H-spiro[benzofuran-3,4'-piperidin]-1'-yl)methyl)-4-hydroxypiperidin-1-carbonyl)phenyl)-3-methyl-2,8-diazaspiro[4.5]decan-2-yl)benzonitrile | 1H NMR (600 MHz, Methanol-d4) δ 7.51 (dd, J = 8.8, 1.3 Hz, 1H), 7.41 7.36 (m, 2H), 7.15 - 7.06 (m, 2H), 6.98 - 6.90 (m, 1H), 6.78 (d, J = 2.3 Hz, 1H), 6.67 (dd, J = 8.9, 2.3 Hz, 1H), 6.34 (dd, J = 8.3, 2.0 Hz, 1H), 6.25 (d, J = 2.0 Hz, 1H), 4.47 (s, 1H), 4.39 (s, 1H), 4.29 (dd, J = 11.8, 4.9 Hz, 1H), 4.08 (h, J = 6.5 Hz, 1H), 3.77 (d, J = 13.0 Hz, 2H), 3.47 (d, J = 10.4 Hz, 3H), 3.45 - 3.42 (m, 2H), 3.39 (d, J = 10.7 Hz, 2H), 3.37 - 3.34 (m, 2H), 3.28 - 3.18 (m, 3H), 2.82 (ddd, J = 17.7, 12.6, 5.2 Hz, 1H), 2.76 - 2.69 (m, 1H), 2.38 (dd, J = 12.9, 7.6 Hz, 1H), 2.35 - 2.27 (m, 2H), 2.21 (t, J = 7.7 Hz, 1H), 2.03 (d, J = 33.1 Hz, 1H), 1.98 - 1.89 (m, 4H), 1.89 - 1.84 (m, 2H), 1.75 (s, 3H), 1.71 - 1.59 (m, 4H), 1.31 (dd, J = 6.0, 1.4 Hz, 3H). | 821.4 6 | A-T-4 |
| 232 | AR -P-91 | 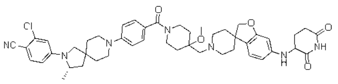 2-chloro-4-((3S)-8-(4-(4-((6-((2,6-dioxopiperidin-3-yl)amino)-2H-spiro[benzofuran-3,4'-piperidin]-1'-yl)methyl)-4-methoxypiperidin-1-carbonyl)phenyl)-3-methyl-2,8-diazaspiro[4.5]decan-2-yl)benzonitrile | 1H NMR (600 MHz, DMSO) δ 10.80 (s, 1H), 7.61 (d, J = 8.8 Hz, 1H), 7.28 (d, J = 8.5 Hz, 2H), 6.97 (d, J = 8.6 Hz, 2H), 6.79 (dd, J = 17.1, 5.1 Hz, 2H), 6.67 (dd, J = 8.9, 2.1 Hz, 1H), 6.20 (dd, J = 38.2, 4.7 Hz, 2H), 4.40 (s, 2H), 4.34 - 4.25 (m, 2H), 4.05 (dd, J = 13.2, 6.5 Hz, 2H), 3.56 (d, J = 10.1 Hz, 2H), 3.45 (d, J = 10.8 Hz, 1H), 3.40 - 3.12 (m, 14H), 2.78 - 2.69 (m, 1H), 2.61 - 2.54 (m, 1H), 2.28 - 2.17 (m, 3H), 2.11 - 2.03 (m, 1H), 1.95 - 1.81 (m, 3H), 1.74 (t, J = 15.4 Hz, 4H), 1.59 (dd, J = 12.7, 6.5 Hz, 3H), 1.51 (d, J = 10.9 Hz, 2H), 1.21 (d, J = 6.0 Hz, 3H). | 834.5 6 | A-T-4 |

(continued)

| Exam ple | Co mp oun d | Structure and Name | 1H-NMR | LC-MS | Synthesi s Method |
|---|---|---|---|---|---|
| 233 | AR -P-92 | 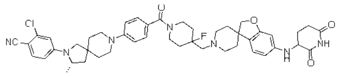 2-chloro-4-((3S)-8-(4-(4-((6-((2,6-dioxopiperidin-3-yl)amino)-2H-spiro[benzofuran-3,4'-piperidin]-1'-yl)methyl)-4-fluoropiperidin-1-carbonyl)phenyl)-3-methyl-2,8-diazaspiro[4.5]decan-2-yl)benzonitrile | 1H NMR (600 MHz, DMSO-d6) δ 10.80 (s, 1H), 7.61 (d, J = 8.8 Hz, 1H), 7.30 (d, J = 8.6 Hz, 2H), 6.98 (d, J = 8.6 Hz, 2H), 6.80 (dd, J = 10.4, 5.1 Hz, 2H), 6.67 (dd, J = 8.9, 2.2 Hz, 1H), 6.23 (d, J = 8.2 Hz, 1H), 6.17 (s, 1H), 4.40 (s, 2H), 4.34 - 4.24 (m, 2H), 4.05 (td, J = 12.6, 6.1 Hz, 3H), 3.53 (d, J = 15.6 Hz, 4H), 3.45 (d, J = 10.8 Hz, 1H), 3.42 - 3.29 (m, 4H), 3.29 - 3.12 (m, 5H), 2.79 - 2.68 (m, 1H), 2.59 (dd, J = 20.3, 16.3 Hz, 1H), 2.24 (dt, J = 16.0, 8.0 Hz, 1H), 2.19 (d, J = 12.2 Hz, 2H), 2.11 - 2.05 (m, 1H), 2.01 (s, 2H), 1.86 (dt, J = 17.7, 7.1 Hz, 2H), 1.80 (d, J = 13.7 Hz, 2H), 1.77 - 1.68 (m, 2H), 1.59 (dd, J = 12.8, 6.5 Hz, 1H), 1.55 - 1.45 (m, 2H), 1.30 - 1.10 (m, 4H). | 822.6 6 | A-T-4 |
| 234 | AR -P-93 | 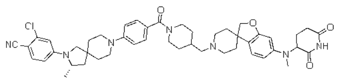 2-chloro-4-((3S)-8-(4-(4-((6-((2,6-dioxopyrimidin-3-yl)(methyl)amino)-2H-spiro[benzofuran-3,4'-piperidin]-1'-yl)methyl)piperidin-1-carbonyl)phenyl)-3-methyl-2,8-diazaspiro[4.5]decan-2-yl)benzonitrile | 1H NMR (600 MHz, DMSO-d6) δ 10.80 (s, 1H), 7.60 (d, J = 8.8 Hz, 1H), 7.26 (d, J = 8.3 Hz, 2H), 6.96 (d, J = 8.3 Hz, 2H), 6.87 (d, J = 8.3 Hz, 1H), 6.80 (s, 1H), 6.66 (d, J = 8.9 Hz, 1H), 6.35 (t, J = 7.1 Hz, 1H), 6.33 - 6.28 (m, 1H), 4.82 (dd, J = 12.4, 4.7 Hz, 1H), 4.42 (s, 2H), 4.10 - 4.01 (m, 2H), 3.53 (d, J = 11.4 Hz, 3H), 3.44 (d, J = 10.7 Hz, 1H), 3.40 - 3.29 (m, 4H), 3.27 - 3.16 (m, 3H), 2.99 (dd, J = 33.8, 22.3 Hz, 5H), 2.87 - 2.80 (m, 1H), 2.69 (d, *J* = 10.0 Hz, 3H), 2.54 (d, J = 13.1 Hz, 1H), 2.33 - 2.21 (m, 2H), 2.11 (dd, J = 25.4, 12.5 Hz, 3H), 1.90 - 1.70 (m, 6H), 1.58 (dd, J = 12.7, 6.5 Hz, 1H), 1.54 - 1.45 (m, 2H), 1.28 - 1.15 (m, 5H). | 818.6 6 | A-T-4 |

(continued)

| Exam ple | Co mp oun d | Structure and Name | $^1$H-NMR | LC-MS | Synthesi s Method |
|---|---|---|---|---|---|
| 235 | AR -P-94 | 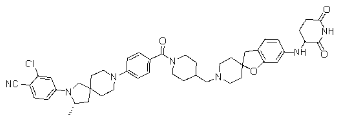 2-chloro-4-((3S)-8-(4-(4-((6-((2,6-dioxopiperidin-3-yl)amino)-3H-spiro[benzofuran-2,4'-piperidin]-1'-yl)methyl)piperidin-1-carbonyl)phenyl)-3-methyl-2,8-diazaspiro[4.5]decan-2-yl)benzonitrile | $^1$H NMR (600 MHz, DMSO-d6) δ 10.81 (d, J = 3.4 Hz, 1H), 7.61 (d, J = 8.9 Hz, 1H), 7.27 (d, J = 8.6 Hz, 2H), 6.98 (d, J = 8.6 Hz, 2H), 6.90 (d, J = 8.1 Hz, 1H), 6.81 (d, J = 2.2 Hz, 1H), 6.67 (dd, J = 9.0, 2.2 Hz, 1H), 6.20 (dd, J = 8.1, 1.9 Hz, 1H), 6.18 - 6.12 (m, 1H), 4.25 (dd, J = 11.3, 4.7 Hz, 3H), 4.05 (dt, J = 12.9, 6.2 Hz, 3H), 3.58 - 3.48 (m, 2H), 3.45 (d, J = 10.8 Hz, 1H), 3.42 - 3.30 (m, 3H), 3.22 (ddd, J = 20.9, 8.0, 4.4 Hz, 2H), 3.19 - 3.05 (m, 4H), 3.00 (s, 1H), 2.92 (s, 2H), 2.73 (ddd, J = 17.1, 12.0, 5.2 Hz, 1H), 2.63 - 2.54 (m, 1H), 2.25 (dd, J = 12.7, 7.7 Hz, 1H), 2.18 - 1.99 (m, 6H), 1.85 (ddd, J = 14.9, 11.8, 3.8 Hz, 1H), 1.81 - 1.67 (m, 4H), 1.59 (dd, J = 12.8, 6.5 Hz, 1H), 1.56 - 1.45 (m, 2H), 1.30 - 1.10 (m, 5H). | 804.4 6 | A-T-4 |
| 236 | AR -P-95 | 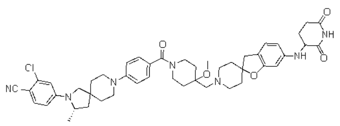 2-chloro-4-((3 S)-8-(4-(4-((6-((2,6-dioxopiperidin-3-yl)amino)-3H-spiro[benzofuran-2,4'-piperidin]-1'-yl)methyl)-4-methoxypiperidin-1-carbonyl)phenyl)-3-methyl-2,8-diazaspiro[4.5]decan-2-yl)benzonitrile | 1H NMR (600 MHz, DMSO-d6) δ 10.81 (d, J = 6.7 Hz, 1H), 7.61 (d, J = 8.8 Hz, 1H), 7.28 (d, J = 8.4 Hz, 2H), 6.97 (d, J = 8.5 Hz, 2H), 6.90 (dd, J = 7.9, 3.8 Hz, 1H), 6.81 (d, J = 1.6 Hz, 1H), 6.67 (dd, J = 8.9, 1.6 Hz, 1H), 6.20 (d, J = 8.1 Hz, 1H), 6.16 (d, J = 5.1 Hz, 1H), 4.30 - 4.23 (m, 1H), 4.09 - 3.99 (m, 2H), 3.55 (d, J = 11.4 Hz, 3H), 3.48 - 3.40 (m, 3H), 3.35 (dt, J = 22.3, 13.9 Hz, 5H), 3.27 - 3.11 (m, 7H), 2.99 (s, 1H), 2.90 (s, 2H), 2.75 (ddd, J = 16.9, 14.3, 5.7 Hz, 1H), 2.63 - 2.54 (m, 1H), 2.24 (dd, J = 12.7, 7.8 Hz, 1H), 2.19 - 2.04 (m, 4H), 2.01 (d, J = 13.8 Hz, 1H), 1.95 - 1.80 (m, 3H), 1.79 - 1.65 (m, 2H), 1.58 (dd, J = 12.7, 6.6 Hz, 3H), 1.50 (t, J = 12.6 Hz, 2H), 1.22 (t, J = 9.7 Hz, 3H). | 834.5 6 | A-T-4 |

(continued)

| Exam ple | Co mp oun d | Structure and Name | $^1$H-NMR | LC-MS | Synthesi s Method |
|---|---|---|---|---|---|
| 237 | AR -P-96 | 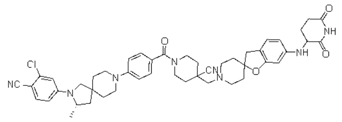 1-(4-(((S)-2-(3-chloro-4-cyanophenyl)-3-methyl-2,8-diazaspiro [4.5]decan-8-yl)benzoyl)-4-((6-((2,6-dioxopyrimidin-3-yl)amino)-3H-spiro[benzofuran-2,4'-piperidin]-1'-yl)methyl)pi-peridin-4-carbonitrile | $^1$H NMR (400 MHz, DMSO) δ 10.80 (s, 1H), 7.60 (d, J = 8.8 Hz, 1H), 7.30 (d, J = 8.6 Hz, 2H), 6.97 (d, J = 8.6 Hz, 2H), 6.84 (dd, J = 30.9, 4.7 Hz, 2H), 6.66 (dd, J = 8.9, 2.0 Hz, 1H), 6.15 (s, 2H), 4.25 (dd, J = 11.2, 4.7 Hz, 2H), 4.04 (dd, J = 13.0, 6.2 Hz, 2H), 3.39 - 3.29 (m, 3H), 3.27 - 2.82 (m, 9H), 2.79 - 2.54 (m, 5H), 2.35 - 1.95 (m, 7H), 1.91-1.44 (m, 10H), 1.20 (d, J = 6.0 Hz, 3H). | 829.6 6 | A-T-4 |
| 238 | AR -P-97 | 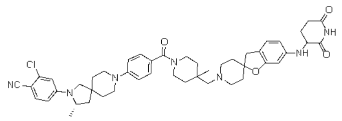 2-chloro-4-((3S)-8-(4-(4-((6-((2,6-dioxopyrimidin-3-yl)amino)-3H-spiro[benzofuran-2,4'-piperidin]-1'-yl)methyl)-4-methylpiperidine-1-carbonyl)phenyl)-3-methyl-2,8-diazaspiro[4.5]de-can-2-yl)benzonitrile | $^1$H NMR (600 MHz, DMSO) δ 10.81 (d, J = 6.7 Hz, 1H), 7.61 (d, J = 8.9 Hz, 1H), 7.28 (d, J = 8.7 Hz, 2H), 6.98 (d, J = 8.6 Hz, 2H), 6.90 (dd, J = 8.0, 5.0 Hz, 1H), 6.81 (d, J = 2.2 Hz, 1H), 6.66 (dd, J = 9.0, 2.3 Hz, 1H), 6.23 - 6.15 (m, 2H), 4.29 - 4.23 (m, 3H), 4.04 (dt, J = 19.6, 6.2 Hz, 3H), 3.60 - 3.41 (m, 4H), 3.41 - 3.14 (m, 11H), 2.93 (t, J = 25.5 Hz, 2H), 2.73 (ddd, J = 17.1, 12.0, 5.2 Hz, 1H), 2.62 - 2.51 (m, 2H), 2.24 (dd, J = 12.7, 7.7 Hz, 1H), 2.20 - 1.97 (m, 6H), 1.89 - 1.68 (m, 4H), 1.61 - 1.42 (m, 7H). | 814.8 6 | A-T-4 |

(continued)

| Exam ple | Co mp oun d | Structure and Name | $^1$H-NMR | LC-MS | Synthesi s Method |
|---|---|---|---|---|---|
| 239 | AR -P-98 | 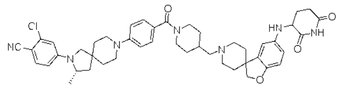 2-chloro-4-((3 S)-8-(4-(4-((5-((2,6-dioxopiperidin-3-yl)amino)-2H-spiro[benzofuran-3,4'-piperidin]-1'-yl)methyl)piperidin-1-carbonyl)phenyl)-3-methyl-2,8-diazaspiro[4.5]decan-2-yl)benzonitrile | 1H NMR (600 MHz, DMSO-d6) δ 10.83 (s, 1H), 8.99 (s, 1H), 7.60 (d, J = 8.8 Hz, 1H), 7.27 (d, J = 8.4 Hz, 2H), 6.98 (d, J = 8.3 Hz, 2H), 6.80 (d, J = 2.3 Hz, 1H), 6.66 (dd, J = 9.0, 2.4 Hz, 1H), 6.60 (d, J = 8.3 Hz, 1H), 6.53 (dd, J = 8.8, 2.5 Hz, 1H), 6.49 (d, J = 2.0 Hz, 1H), 4.37 (d, J = 4.0 Hz, 4H), 4.19 (dd, J = 11.2, 4.8 Hz, 3H), 4.04 (q, J = 6.6 Hz, 3H), 3.54 (d, J = 12.0 Hz, 2H), 3.44 (d, J = 10.8 Hz, 1H), 3.40 - 3.34 (m, 2H), 3.23 (dt, J = 18.9, 6.7 Hz, 2H), 3.03 (d, J = 6.2 Hz, 4H), 2.73 - 2.68 (m, 1H), 2.63 - 2.57 (m, 1H), 2.24 (dd, J = 12.8, 7.7 Hz, 1H), 2.19 - 2.04 (m, 4H), 1.92 - 1.81 (m, 3H), 1.81 - 1.68 (m, 4H), 1.58 (dd, J = 12.8, 6.5 Hz, 1H), 1.50 (q, J = 6.1, 5.4 Hz, 2H), 1.21 (d, J = 6.0 Hz, 4H). | 804.6 0 | A-T-4 |
| 240 | AR -P-99 | 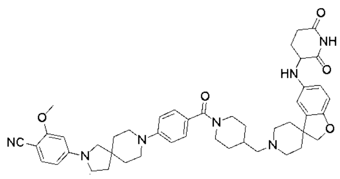 4-((3S)-8-(4-((5-((2,6-dioxopiperidin-3-yl)amino)-2H-spiro[benzofuran-3,4'-piperidin]-1'-yl)methyl) piperidin-1-carbonyl)phenyl)-3-methyl-2,8-diazaspiro[4.5]decan-2-yl)-2-methoxybenzonitrile | 1H NMR (600 MHz, DMSO-d6) δ 10.82 (s, 1H), 7.37 (d, J = 8.7 Hz, 1H), 7.27 (d, J = 8.6 Hz, 2H), 6.99 (t, J = 9.6 Hz, 2H), 6.60 (d, J = 8.5 Hz, 1H), 6.52 (dd, J = 8.6, 2.0 Hz, 1H), 6.49 (s, 1H), 6.27 (dd, J = 8.8, 1.8 Hz, 1H), 6.19 (d, J = 1.6 Hz, 1H), 4.41 - 4.36 (m, 3H), 4.18 (dd, J = 11.1, 4.7 Hz, 3H), 4.07 - 4.02 (m, 3H), 3.87 (s, 3H), 3.55 (d, J = 11.3 Hz, 2H), 3.43 (d, J = 10.6 Hz, 1H), 3.35 (dd, J = 23.8, 13.1 Hz, 3H), 3.29 - 3.20 (m, 2H), 3.04 (s, 4H), 2.71 (ddt, J = 19.6, 13.3, 6.6 Hz, 1H), 2.60 (dd, J = 13.2, 4.1 Hz, 1H), 2.25 (dd, J = 12.6, 7.7 Hz, 1H), 2.17 - 2.06 (m, 4H), 1.91 - 1.82 (m, 3H), 1.80 - 1.69 (m, 4H), 1.58 (dd, J = 12.8, 6.6 Hz, 1H), 1.55 - 1.47 (m, 2H), 1.26 - 1.17 (m, 5H). | 800.7 6 | A-T-4 |

(continued)

| Exam ple | Co mp oun d | Structure and Name | $^1$H-NMR | LC-MS | Synthesi s Method |
|---|---|---|---|---|---|
| 241 | AR -P-100 | 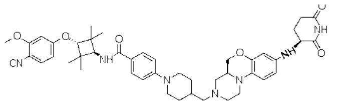 N-((1r,3r)-3-((4-cyano-3-methoxyphenoxy)-2,2,4,4-tetramethylcyclobutyl)-4-(4-(((S)-8-((((S)-2,6-dioxopiperidin-3-yl)amino)-1,2,4a,5-tetrahydrobenzo[b]pyrazino[1,2-d][1,4]oxazin-3(4H)-yl)methyl)piperidin-1-yl)benzamide | 1H NMR (600 MHz, DMSO) δ 10.77 (s, 1H), 9.64 (s, 1H), 7.77 (d, J = 8.6 Hz, 2H), 7.66 (d, J = 8.6 Hz, 1H), 7.52 (d, J = 9.2 Hz, 1H), 7.00 (d, J = 8.8 Hz, 2H), 6.76 (d, J = 8.9 Hz, 1H), 6.64 (d, J = 1.4 Hz, 1H), 6.54 (dd, J = 8.6, 1.6 Hz, 1H), 6.31 - 6.19 (m, 2H), 4.30 - 4.23 (m, 2H), 4.19 (dd, J = 11.1, 4.6 Hz, 1H), 4.06 (d, J = 9.2 Hz, 1H), 3.96 - 3.87 (m, 9H), 3.64 (s, 2H), 3.32 (d, J = 9.4 Hz, 1H), 3.11 (s, 3H), 2.85 (dt, J = 38.2, 12.4 Hz, 4H), 2.73 (ddd, J = 17.2, 11.8, 5.3 Hz, 1H), 2.63 - 2.54 (m, 1H), 2.13 - 2.04 (m, 2H), 1.91 - 1.77 (m, 3H), 1.31 (d, J = 12.9 Hz, 2H), 1.23 (s, 6H), 1.15 (s, 6H). | 790.6 0 | A-T-2 |
| 242 | AR -P-101 | 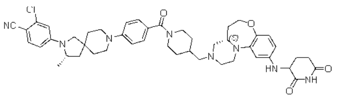 2-chloro-4-((3 S)-8-(4-(4aS)-10-((2,6-dioxopiperidin-3-yl)amino)-1,2,4,4a,5,6-hexahydrobenzo[b]pyrazi no[1,2-d][1,4]oxazepin-3-yl)methyl)piperidin-1-carbonyl)phenyl)-3-methyl-2,8-diazaspiro[4.5]de-can-2-yl)benzonitrile | $^1$H NMR (400 MHz, DMSO-$d_6$) δ 10.81 (d, *J*= 1.9 Hz, 1H), 9.46 (s, 1H), 7.61 (d, *J* = 8.8 Hz, 1H), 7.27 (d, *J*= 8.5 Hz, 2H), 6.97 (d, *J*= 8.5 Hz, 2H), 6.80 (d, *J* = 2.3 Hz, 1H), 6.66 (m, *J* = 8.9, 2.4 Hz, 1H), 6.61 (d, *J*= 8.5 Hz, 1H), 6.35 (t, *J* = 3.0 Hz, 1H), 6.25 (m, *J*= 9.1, 2.4 Hz, 1H), 4.26 - 4.16 (m, 3H), 4.04 (q, *J* = 6.5 Hz, 2H), 4.01 - 3.93 (m, 1H), 3.55 (m, *J* = 30.6, 11.3 Hz, 2H), 3.44 (d, *J*= 10.8 Hz, 1H), 3.40 - 3.29 (m, 6H), 3.23 (m, *J* = 14.4, 11.0, 5.4 Hz, 3H), 3.07 (d, *J*= 35.9 Hz, 4H), 2.91 (d, *J* = 15.3 Hz, 1H), 2.73 (m, *J*= 23.0, 12.6, 5.0 Hz, 1H), 2.64 - 2.54 (m, 1H), 2.24 (m, *J*= 12.8, 7.7 Hz, 1H), 2.19 - 1.96 (m, 3H), 1.90 - 1.66 (m, 6H), 1.58 (m , *J* = 12.8, 6.5 Hz, 1H), 1.51 (d, *J*= 7.5 Hz, 2H), 1.21 (d, *J*= 6.0 Hz, 5H). | 819.5 6 | A-T-4 |

(continued)

| Exam ple | Co mp oun d | Structure and Name | $^1$H-NMR | LC-MS | Synthesi s Method |
|---|---|---|---|---|---|
| 243 | AR -P-102 | 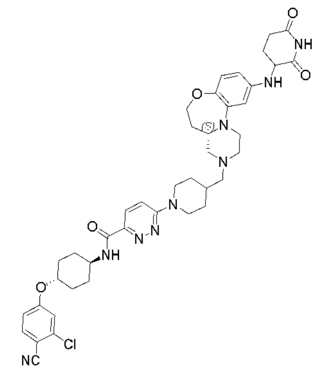 N-((1R,4R)-4-(3-chloro-4-cyanophenoxy)cyclohexy 1)-6-(4-(((4aS)-10-((2,6-dioxopiperidin-3-yl)amino)-1,2,4,4a,5,6-hexahydro-3H-benzo [b]pyrazino [1,2-d][1,4]oxirane-3-yl)methyl)piperidin-1-yl)pyridazin-3-carboxa-mide | $^1$H NMR (600 MHz, DMSO-$d_6$) $\delta$ 10.80 (d, *J*= 5.1 Hz, 1H), 8.61 (d, *J* = 8.2 Hz, 1H), 7.86 (m, *J* = 14.0, 9.1 Hz, 2H), 7.43 - 7.38 (m, 2H), 7.14 (m, J= 8.8, 2.5 Hz, 1H), 6.89 (d, *J* = 8.3 Hz, 1H), 6.39 - 6.30 (m, 2H), 4.83 (m, *J* = 12.6, 5.0 Hz, 1H), 4.53 (m, *J* = 14.0, 9.5, 4.9 Hz, 3H), 4.43 (s, 1H), 3.87 (m, *J*= 11.3, 3.4 Hz, 2H), 3.58 - 3.53 (m, 2H), 3.10 - 3.00 (m, 5H), 2.84 (m, *J* = 17.1, 13.5, 5.3 Hz, 1H), 2.71 *(d, J*= 3.7 Hz, 3H), 2.55 (d, *J*= 14.4 Hz, 1H), 2.29 (m, *J* = 12.9, 4.3 Hz, 1H), 2.18 - 2.08 (m, 4H), 1.94 - 1.79 (m, 7H), 1.69 - 1.61 (m, 2H), 1.53 (m, *J*= 11.7, 10.7, 3.5 Hz, 2H), 1.30 - 1.21 (m, 3H). | 783.6 6 | A-T-1 |
| | | | | | |
| 244 | A-P-103 | 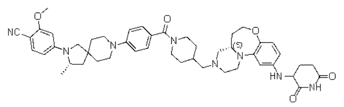 4-((3S)-8-(4-(4-((((4aS)-10-((2,6-dioxopiperidin-3-yl)amino)-1,2,4,4a,5,6-hexahydro-3H-benzo[b]pyrazino[1,2-d][1,4]oxazepin-3-yl)methyl)piperidin-1-carbonyl)phenyl)-3-methyl-2,8-diazaspiro[4.5]decan-2-yl)-2-methox-ybenzonitrile | $^1$H NMR (600 MHz, DMSO) $\delta$ 10.82 (d, *J*= 2.6 Hz, 1H), 9.54 (s, 1H), 7.37 (d, *J* = 8.7 Hz, 1H), 7.27 (d, *J* = 8.5 Hz, 2H), 6.98 (d, *J*= 8.6 Hz, 2H), 6.61 (d, *J*= 8.5 Hz, 1H), 6.36 (s, 1H), 6.26 (dd, *J*= 14.4, 5.4 Hz, 2H), 6.19 (s, 1H), 4.22 (dd, *J*= 13.9, 6.4 Hz, 3H), 4.04 (dd, *J* = 13.1, 6.5 Hz, 3H), 3.97 (dd, *J* = 6.1, 4.0 Hz, 2H), 3.87 (s, 3H), 3.59 (d, *J* = 9.2 Hz, 1H), 3.51 (d, *J*= 10.7 Hz, 1H), 3.44 (d, *J*= 10.6 Hz, 1H), 3.38 (d, *J*= 13.2 Hz, 3H), 3.33 (s, 2H), 3.29 - 3.23 (m, 2H), 3.13 (d, *J* = 19.7 Hz, 3H), 3.04 (d, *J*= 9.9 Hz, 1H), 2.93 (s, 1H), 2.75 - 2.69 (m, 1H), 2.59 (d, *J* = 17.9 Hz, 1H), 2.25 (dd, *J*= 12.7, 7.7 Hz, 1H), 2.15 (s, 1H), 2.12 - 2.07 (m, 1H), 2.04 - 1.99 (m, 1H), 1.88 - 1.80 (m, 2H), 1.79 - 1.69 (m, 4H), 1.58 (dd, *J* = 12.8, 6.6 Hz, 1H), 1.51 (dd, *J* = 13.3, 10.7 Hz, 2H), 1.23 (d, *J* = 6.0 Hz, 3H), 1.20 (d, *J*= 12.1 Hz, 2H). | 815.7 6 | A-T-4 |

(continued)

| Exam ple | Co mp oun d | Structure and Name | $^1$H-NMR | LC-MS | Synthesi s Method |
|---|---|---|---|---|---|
| 245 | A-P-104 | 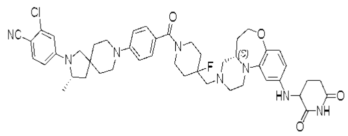 2-chloro-4-((3S)-8-(4-(4-((((4aS)-10-((2,6-dioxohesperidin-3-yl) amino)-1,2,4,4a,5,6-hexahydro-3H-benzo[b]pyrazino[1,2-d][1,4]oxirane-3-yl)methyl)-4-fluoropiperidin-1-carbonyl)phenyl)-3-methyl-2,8-diazaspiro[4.5]decan-2-yl)benzonitrile | $^1$H NMR (600 MHz, DMSO-$d_6$) δ 10.80 (d, J = 2.6 Hz, 1H), 9.93 (s, 1H), 7.61 (d, J = 8.8 Hz, 1H), 7.30 (d, J = 8.5 Hz, 2H), 6.99 (d, J = 8.4 Hz, 2H), 6.81 (d, J = 2.3 Hz, 1H), 6.67 (dd, J = 9.0, 2.3 Hz, 1H), 6.61 (d, J = 8.5 Hz, 1H), 6.37 - 6.32 (m, 1H), 6.25 (dt, J = 8.7, 2.1 Hz, 1H), 4.58 (q, J = 6.9 Hz, 1H), 4.25 - 4.14 (m, 3H), 4.05 (h, J = 6.3 Hz, 2H), 3.97 (s, 2H), 3.45 (d, J = 10.8 Hz, 6H), 3.41 - 3.30 (m, 5H), 3.29 - 3.07 (m, 6H), 2.72 (ddd, J = 17.4, 11.7, 5.4 Hz, 1H), 2.59 (dd, J = 18.1, 4.9 Hz, 1H), 2.25 (dd, J = 12.8, 7.7 Hz, 1H), 2.13 - 2.08 (m, 1H), 2.05 - 1.92 (m, 3H), 1.88 - 1.70 (m, 6H), 1.59 (dd, J = 12.8, 6.5 Hz, 1H), 1.51 (dd, J = 9.3, 5.8 Hz, 3H). | 837.6 6 | A-T-4 |
| 246 | A-P-105 | 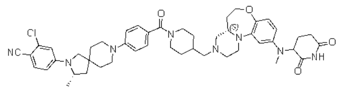 2-chloro-4-(3 S)-8-(4-(4-(4aS)-10-((2,6-dioxopiperidin-3-yl)(methyl)amino)-1,2,4,4a,5,6-hexahydro-1 3H-benzo[b]pyrazino[1,2-d][1,4]oxazepin-3-yl)methyl)piperidin-1-carbonyl)phenyl)-3-methyl-2,8-diazaspiro[4.5]decan-2-yl)benzonitrile | $^1$H NMR (600 MHz, DMSO-$d_6$) δ 10.80 (s, 1H), 7.61 (d, *J* = 8.8 Hz, 1H), 7.27 (d, *J* = 8.5 Hz, 2H), 6.98 (d, *J* = 8.5 Hz, 2H), 6.81 (d, *J* = 2.3 Hz, 1H), 6.70 - 6.65 (m, 2H), 6.40 - 6.34 (m, 2H), 4.78 (dd, *J*= 12.7, 5.0 Hz, 1H), 4.19 (dd, *J* = 18.4, 4.5 Hz, 2H), 4.02 (dq, *J*= 26.5, 6.1, 5.6 Hz, 3H), 3.58 - 3.49 (m, 3H), 3.45 (d, *J* = 10.7 Hz, 2H), 3.35 (dt, *J*= 24.5, 7.8 Hz, 4H), 3.28 - 3.17 (m, 3H), 3.17 - 3.08 (m, 2H), 3.02 (q, *J* = 10.9 Hz, 2H), 2.97 - 2.76 (m, 3H), 2.70 (d, *J* = 1.2 Hz, 2H), 2.59 - 2.53 (m, 1H), 2.27 (ddd, *J* = 25.6, 13.3, 8.6 Hz, 2H), 2.15 (s, 1H), 2.00 (t, *J*= 5.8 Hz, 1H), 1.87 - 1.69 (m, 5H), 1.59 (dd, *J* = 12.8, 6.5 Hz, 1H), 1.51 (q, *J* = 6.2, 5.4 Hz, 2H), 1.21 (d, *J* = 6.0 Hz, 5H). | 833.4 9 | A-T-4 |

(continued)

| Exam ple | Co mp oun d | Structure and Name | $^1$H-NMR | LC-MS | Synthesi s Method |
|---|---|---|---|---|---|
| 247 | A-P-106 | 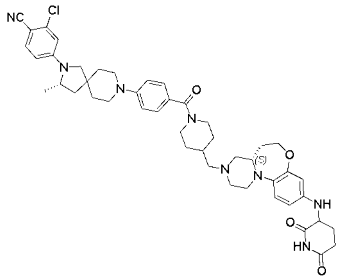 2-chloro-4-((3 S)-8-(4-((4aS)-9-((2,6-dioxopiperidin-3-yl)amino)-1,2,4,4a,5,6-hexahydro-3H-benzo[b]pyrazino[1,2-d][1,4]oxazepin-3-yl)methyl)piperidin-1-carbonyl)phenyl)-3-methyl-2,8-diazaspiro[4.5]decan-2-yl)benzonitrile | $^1$H NMR (600 MHz, DMSO-$d_6$) $\delta$ 10.77 (d, $J$ = 2.9 Hz, 1H), 7.60 (d, $J$ = 8.8 Hz, 1H), 7.24 (d, $J$ = 8.6 Hz, 2H), 6.95 (d, $J$= 8.7 Hz, 2H), 6.80 (d, $J$ = 2.1 Hz, 1H), 6.74 - 6.60 (m, 2H), 6.25 (d, $J$ = 8.5 Hz, 1H), 6.16 (s, 1H), 5.53 (s, 1H), 4.43 (s, 2H), 4.30 - 4.10 (m, 2H), 4.10 - 3.92 (m, 3H), 3.48 - 3.37 (m, 2H), 3.30 (s, 1H), 3.27 - 3.12 (m, 3H), 2.92 (s, 5H), 2.78 - 2.61 (m, 2H), 2.61 - 2.52 (m, 2H), 2.35 - 2.11 (m, 4H), 2.11 - 1.91 (m, 3H), 1.91 - 1.64 (m, 6H), 1.57 (dd, $J$ = 12.8, 6.5 Hz, 1H), 1.49 (s, 2H), 1.21 (d, $J$= 7.7 Hz, 3H), 1.08 (s, 2H). | 819.5 6 | A-T-4 |
| 248 | A-P-107 | 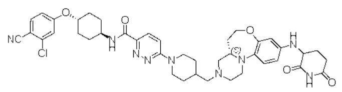 N-((1r,4r)-4-((3-chloro-4-cyanophenoxy)cyclohexy 1)-6-(4-(((4aS)-9-((2,6-dioxopiperidin-3-yl)amino)-1,2,4,4a,5,6-hexahydro-3H-benzo[b]pyrazino[1,2-d][1,4]oxirane-3-yl)methyl)piperidin-1-yl)pyridazin-3-carboxamide | $^1$H NMR (600 MHz, Methanol-d4) $\delta$ 7.96 (d, J = 9.6 Hz, 1H), 7.72 (d, J = 8.7 Hz, 1H), 7.40 (d, J = 9.7 Hz, 1H), 7.23 (d, J = 2.5 Hz, 1H), 7.07 (dd, J = 8.8, 2.4 Hz, 1H), 6.85 (d, J = 8.6 Hz, 1H), 6.50 - 6.42 (m, 1H), 6.37 (d, J = 2.7 Hz, 1H), 4.68 - 4.47 (m, 4H), 4.30 - 4.20 (m, 1H), 4.15 (s, 1H), 4.00 (s, 1H), 3.72 (d, J = 12.2 Hz, 1H), 3.55 (d, J = 11.9 Hz, 1H), 3.24 - 3.12 (m, 5H), 2.88 - 2.67 (m, 2H), 2.40 - 2.29 (m, 2H), 2.22 (d, J = 9.0 Hz, 3H), 2.12 (s, 2H), 2.03 - 1.90 (m, 3H), 1.81 (d, J = 16.4 Hz, 1H), 1.67 (t, $J$ = 9.5 Hz, 4H), 1.45 (dd, J = 13.1, 9.4 Hz, 2H), 1.42 - 1.28 (m, 4H). | 782.5 6 | A-T-1 |

(continued)

| Exam ple | Co mp oun d | Structure and Name | $^1$H-NMR | LC-MS | Synthesi s Method |
|---|---|---|---|---|---|
| 249 | A-P-108 | 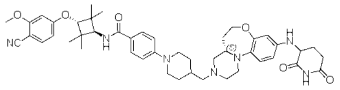 N-((1r,3r)-3-(4-cyano-3-methoxyphenoxy)-2,2,4,4-tetramethylcyclobutyl)-4-(4-((4aS)-9-((2,6-diox-opiperidin-3-yl)ami-no)-1,2,4,4a,5,6-hexahydro-3H-benzo[b]pyrazino[1,2-d][1,4]oxirane-3-yl)methyl)piperidin-1-yl) benzamide | $^1$H NMR (600 MHz, DMSO-$d_6$) δ 10.79 (d, *J* = 4.7 Hz, 1H), 9.38 (s, 1H), 7.77 (d, *J* = 8.6 Hz, 2H), 7.66 (d, *J* = 8.6 Hz, 1H), 7.53 (d, *J* = 9.3 Hz, 1H), 7.00 (d, *J*= 8.7 Hz, 2H), 6.75 (d, *J* = 8.6 Hz, 1H), 6.64 (d, *J* = 2.2 Hz, 1H), 6.54 (dd, *J*= 8.6, 2.2 Hz, 1H), 6.31 (dt, *J*= 8.5, 2.4 Hz, 1H), 6.22 (d, *J* = 2.6 Hz, 1H), 4.53 (t, *J* = 10.5 Hz, 1H), 4.28 (s, 1H), 4.23 (dd, *J* = 11.4, 4.8 Hz, 1H), 4.06 (d, *J* = 9.1 Hz, 2H), 3.91 (s, 5H), 3.59 (d, *J* = 11.4 Hz, 1H), 3.47 (d, *J*= 11.5 Hz, 1H), 3.33 - 3.25 (m, 1H), 3.13 (q, *J*= 12.9, 12.3 Hz, 4H), 3.04 (t, *J* = 11.4 Hz, 1H), 2.85 - 2.80 (m, 2H), 2.73 (td, *J* = 12.1, 6.0 Hz, 1H), 2.61 - 2.52 (m, 1H), 2.14 - 2.03 (m, 3H), 1.90 - 1.78 (m, 3H), 1.76 - 1.70 (m, 1H), 1.35 - 1.28 (m, 2H), 1.23 (s, 7H), 1.15 (s, 6H).. | 804.6 6 | A-T-2 |
| 250 | A-P-109 | 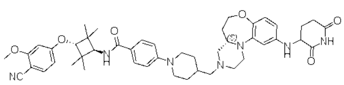 N-((1r,3r)-3-((4-cyano-3-methoxyphenoxy)-2,2,4,4-tetramethylcyclobutyl)-4-(4-(((4aS)-10-((2,6-di-oxopiperidin-3-yl)ami-no)-1,2,4,4a,5,6-hexahydro-3H-benzo[b]pyrazino[1,2-d][1,4]oxazepin-3-yl)methyl)piperidin-1-yl) benzamide | 1H NMR (400 MHz, Methanol-d4) δ 7.79 (d, J = 8.7 Hz, 2H), 7.55 (d, J = 8.6 Hz, 1H), 7.09 (d, J = 8.7 Hz, 2H), 6.82 (d, J = 8.5 Hz, 1H), 6.65 (d, J = 2.2 Hz, 1H), 6.61 - 6.52 (m, 3H), 4.35 (dd, J = 10.8, 5.0 Hz, 2H), 4.29 (s, 1H), 4.16 (s, 1H), 4.00 (d, 2H), 3.96 (s, 3H), 3.71 (d, J = 11.8 Hz, 2H), 3.63 (d, J = 7.0 Hz, 2H), 3.53 (d, J = 6.4 Hz, 1H), 3.51 - 3.45 (m, 2H), 3.22 (d, J = 7.0 Hz, 2H), 3.18 - 3.12 (m, 1H), 3.00 (t, J = 12.3 Hz, 3H), 2.79 - 2.73 (m, 2H), 2.27 (dd, J = 20.0, 7.5 Hz, 2H), 2.17 (d, J = 19.5 Hz, 1H), 2.03 - 1.87 (m, 4H), 1.52 (d, J = 12.0 Hz, 2H), 1.31 (s, 6H), 1.26 (s, 6H). | 804.5 6 | A-T-2 |

The third category comprises PROTAC compounds targeting the SMARCA2/4 proteins, synthesized according to the general formulas SM-T-1 to SM-T-6 below. The CRBN ligands used herein are the CRBN ligands provided in the claims of the invention.

General formula SM-T-1 for PROTAC synthesis

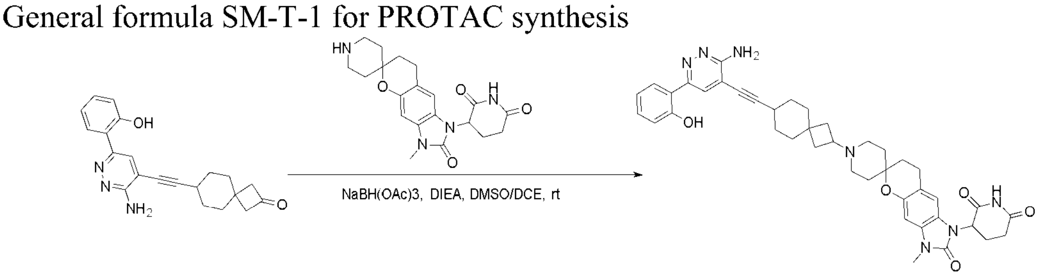


General formula SM-T-2 for PROTAC synthesis

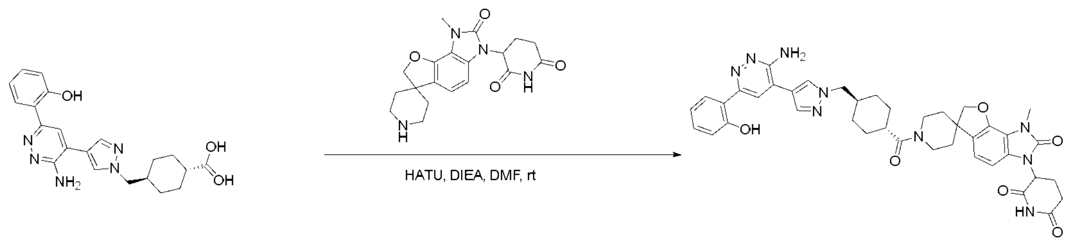


General formula SM-T-3 for PROTAC synthesis

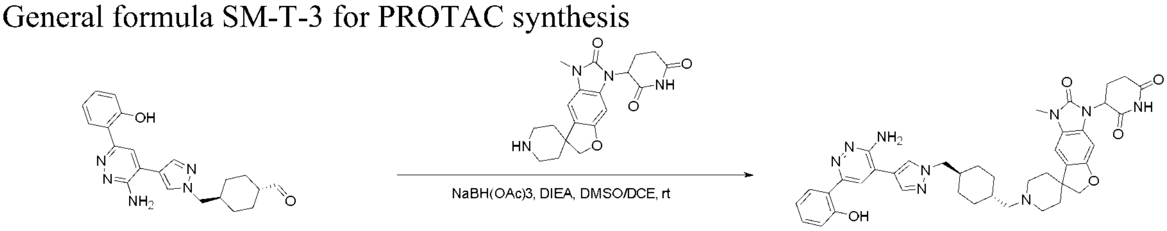


General formula SM-T-4 for PROTAC synthesis

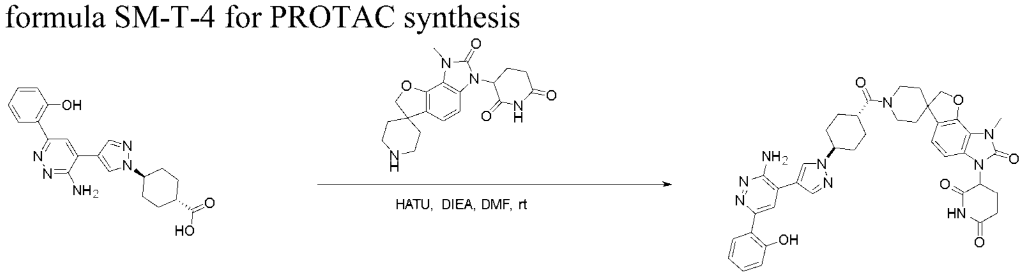


General formula SM-T-5 for PROTAC synthesis

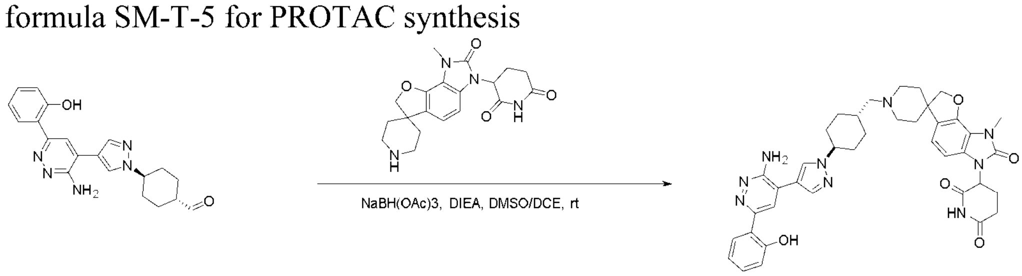

General formula SM-T-6 for PROTAC synthesis

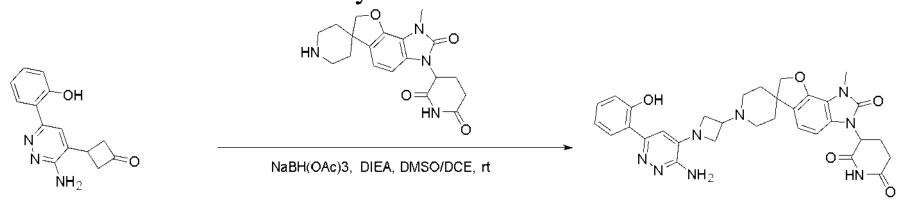


| Exa mpl e | Com poun d | Structure and Name | $^1$H-NMR | LC-MS | Synthesi s Method |
|---|---|---|---|---|---|
| 251 | SM-P-1 | 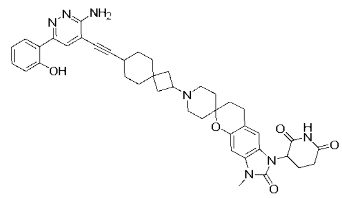 3-(1'-(7-((3-amino-6-(2-hydroxyphenyl)pyridazin -4-yl)ethynyl) spiro[3.5]nona n-2-yl)-3-methyl-2-oxo-2,3,7,8-tetrahydro-1H-spiro[chromeno[6,7-d] imidazole-6,4'-piperidin]-1-yl)pi-peridine-2,6-dione | $^1$H NMR (400 MHz, Methanol-$d_4$) δ 8.29 (s, 1H), 7.68 (dd, *J* = 8.1, 1.6 Hz, 1H), 7.38 (td, *J*= 7.7, 1.6 Hz, 1H), 7.03 - 6.98 (m, 2H), 6.86 (s, 1H), 6.75 (s, 1H), 5.29 (dd, *J* = 12.5, 5.4 Hz, 1H), 3.80 (d, *J* = 8.3 Hz, 1H), 3.43 (d, *J*= 12.0 Hz, 2H), 3.38 (s, 3H), 3.19 (t, *J*= 12.1 Hz, 2H), 2.96 - 2.88 (m, 3H), 2.86 - 2.77 (m, 3H), 2.43 (s, 1H), 2.30 (d, *J*= 7.9 Hz, 1H), 2.21 - 2.09 (m, 3H), 2.02 (dd, *J*= 11.8, 8.7 Hz, 3H), 1.93 (q, *J* = 10.3, 8.6 Hz, 6H), 1.79 (q, *J* = 13.8, 12.7 Hz, 2H), 1.73 - 1.65 (m, 1H), 1.60 (t, *J* = 11.4 Hz, 2H). | 716.5 7 | General formula SM-T-1 for PROTA C synthesis |
| 252 | SM-P-2 | 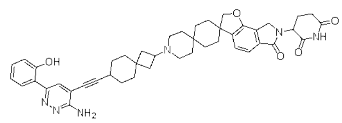 3-(1"-(7-(3-amino-6-(2-hydroxyphenyl)pyridazin -4-yl)ethynyl) spiro[3.5]nona n-2-yl)-6-oxo-6,8-dihydro-2h,7h-dihydro [furo[2,3-e]isoindole-3,1'-cyclohexane-4',4"-piperidin]-7-yl)piperi-dine-2,6-dione | $^1$H NMR (600 MHz, Methanol-$d_4$) δ 8.31 (d, *J*= 2.4 Hz, 1H), 7.68 (dt, *J*= 8.0, 1.4 Hz, 1H), 7.45 - 7.35 (m, 3H), 7.01 (m, 2H), 5.16 (dt, *J*= 13.4, 5.1 Hz, 1H), 4.62 - 4.54 (m, 2H), 4.50 - 4.37 (m, 2H), 3.72 (d, *J* = 8.4 Hz, 1H), 3.47 - 3.36 (m, 2H), 3.02 - 2.89 (m, 3H), 2.88 - 2.77 (m, 2H), 2.57 - 2.47 (m, 2H), 2.40 (d, *J* = 6.9 Hz, 1H), 2.28 (d, *J* = 9.9 Hz, 1H), 2.22 - 2.10 (m, 2H), 2.07 - 1.93 (m, 5H), 1.89 (m, *J* =, 2H), 1.83 - 1.77 (m, 1H), 1.75 - 1.66 (m, 5H), 1.56 (m, 5H), 1.39 - 1.27 (m, 2H). | 755.6 0 | General formula SM-T-1 for PROTA C synthesis |

(continued)

| Exa mpl e | Com poun d | Structure and Name | $^1$H-NMR | LC-MS | Synthesi s Method |
|---|---|---|---|---|---|
| 253 | SM-P-3 | 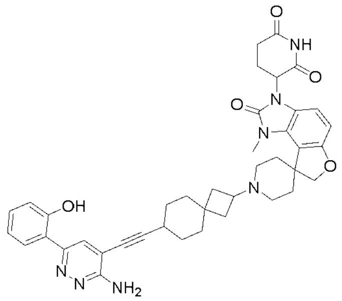 3-(1'-(7-((3-amino-6-(2-hydroxyphenyl)pyridazin -4-yl)ethyl)spiro [3.5]nonan-2-yl)-1-methyl-2-oxo-1,2-dihydro-3h,7-spiro[benzofuro[4,5-d]imidazole-8,4'-piperidin]-3-yl)piperidine-2,6-dione | $^1$H NMR (400 MHz, Metha-nol-$d_4$) δ 8.30 (s, 1H), 7.68 (dd, *J* = 8.1, 1.7 Hz, 1H), 7.38 (m, 1H), 7.04 - 6.97 (m, 3H), 6.64 (d, *J* = 8.5 Hz, 1H), 5.33 (dd, *J* = 12.3, 5.4 Hz, 1H), 4.57 (s, 2H), 3.79 - 3.71 (m, 1H), 3.70 (s, 3H), 3.63 (d, *J* = 12.6 Hz, 2H), 3.05 - 2.93 (m, 3H), 2.90 (d, *J* = 5.2 Hz, 1H), 2.86 - 2.81 (m, 2H), 2.78 (m, 1H), 2.64 (m, 2H), 2.46 - 2.38 (m, 1H), 2.35 - 2.26 (m, 1H), 2.23 *(d, J*= 14.3 Hz, 2H), 2.18 - 2.03 (m, 4H), 1.98 (d, *J* = 21.4 Hz, 2H), 1.90 (d, J = 13.9 Hz, 1H), 1.74 - 1.66 (m, 1H), 1.60 (t, *J* = 11.1 Hz, 2H) | 702.5 7 | General formula SM-T-1 for PROTA C synthesis |
| 254 | SM-P-4 | 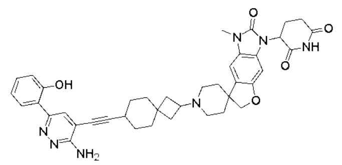 3-(1'-(7-((3-amino-6-(2-hydroxyphenyl)pyridazin -4-yl)ethynyl) spiro[3.5]nona n-2-yl)-1-methyl-2-oxo-1,2-dihydro-3H,6H-spiro[benzofuro[5,6-d]imidazole-7,4'-piperidin]-3-yl) piperidine-2,6-dione | $^1$H NMR (600 MHz, Metha-nol-$d_4$) δ 8.27 (s, 1H), 7.69 (dd, *J*= 8.1, 1.6 Hz, 1H), 7.37 (m, 2H), 7.00 (dd, *J* = 4.1, 3.0 Hz, 1H), 6.99 (s, 1H), 6.71 (s, 1H), 5.31 (dd, *J*= 12.6, 5.4 Hz, 1H), 4.53 (s, 2H), 3.72 (m, 1H), 3.63 - 3.55 (m, 2H), 3.44 (s, 3H), 2.98 - 2.89 (m, 3H), 2.87 - 2.77 (m, 3H), 2.44 (s, 1H), 2.31 (s, 1H), 2.23 (d, *J*= 14.4 Hz, 2H), 2.20 - 2.12 (m, 2H), 2.09 (d, *J*= 14.9 Hz, 2H), 2.03 (dd, *J*= 11.3, 8.2 Hz, 2H), 1.98 (s, 1H), 1.91 (d, *J* = 13.8 Hz, 1H), 1.84 *(d, J*= 11.7 Hz, 1H), 1.76 (d, *J* = 10.9 Hz, 1H), 1.69 (t, *J* = 10.9 Hz, 1H), 1.65 - 1.58 (m, 2H) | 702.4 7 | General formula SM-T-1 for PROTA C synthesis |

(continued)

| Exa mpl e | Com poun d | Structure and Name | $^1$H-NMR | LC-MS | Synthesi s Method |
|---|---|---|---|---|---|
| 255 | SM-P-5 | 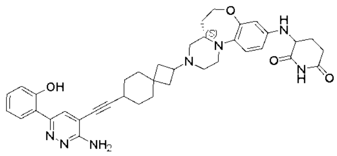 3-((S)-3-(7-(3-amino-6-(2-hydroxyphenyl)pyridazin -4-yl)ethyl) spiro[3.5]nonan-2-yl)-2,3,4,4,4,5,6-hexahydro-1h-benzo[b]pyrazino[1,2-d][1,4]oxazepin-9-yl)amino)piperidine-2,6-dione | $^1$H NMR (600 MHz, Methanol-$d_4$) δ 8.31 (t, *J* = 1.6 Hz, 1H), 7.70 - 7.67 (m, 1H), 7.39 (m, 1H), 7.01 (m, 2H), 6.83 (d, *J* = 8.6 Hz, 1H), 6.45 (dd, *J* = 8.6, 2.6 Hz, 1H), 6.35 (d, *J*= 2.6 Hz, 1H), 4.55 (t, *J* = 10.6 Hz, 1H), 4.24 (dd, *J*= 11.9, 4.9 Hz, 1H), 4.16 - 4.11 (m, 1H), 3.78 (m, 1H), 3.66 - 3.48 (m, 2H), 3.46 - 3.39 (m, 1H), 3.25 - 3.13 (m, 2H), 3.04 (t, *J* = 11.8 Hz, 1H), 2.96 (d, *J* = 11.7 Hz, 1H), 2.81 (m, 2H), 2.73 (m, 1H), 2.48 - 2.38 (m, 1H), 2.34 - 2.26 (m, 2H), 2.26 - 2.18 (m, 1H), 2.03 (m, 3H), 1.99 - 1.88 (m, 3H), 1.86 - 1.72 (m, 3H), 1.68 (d, *J* = 11.0 Hz, 1H), 1.60 (t, *J* = 11.8 Hz, 2H). | 662.5 7 | General formula SM-T-1 for PROTA C synthesis |
| 256 | SM-P-6 | 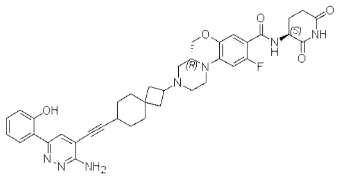 (R)-3-(7-((3-amino-6-(2-hydroxyphenyl)pyridazin -4-yl)ethynyl) spiro[3.5]nona n-2-yl)-n-((S)-2,6-dioxopiperidin-3-yl)-9-fluoro-1,2,3,4,4a,5-hexahydrobenzo [b]pyrazi no[1,2-d][1,4]oxazine-8-carboxamide | $^1$H NMR (600 MHz, Methanol-$d_4$) δ 8.28 (s, 1H), 7.69 (dd, *J* = 8.1, 1.7 Hz, 1H), 7.37 (m, 1H), 7.31 (d, *J* = 7.2 Hz, 1H), 7.02 - 6.98 (m, 2H), 6.89 (d, *J*= 13.6 Hz, 1H), 4.81 (m, 1H), 4.37 (dd, *J* = 11.3, 2.6 Hz, 1H), 4.23 (d, *J*= 14.2 Hz, 1H), 4.12 - 4.05 (m, 1H), 3.74 (s, 1H), 3.62 (s, 2H), 3.24 (d, *J* = 13.6 Hz, 1H), 3.02 (s, 1H), 2.87 - 2.78 (m, 3H), 2.72 (m, 1H), 2.43 (s, 1H), 2.31 (m, 2H), 2.22 - 2.12 (m, 2H), 2.08 - 2.00 (m, 3H), 1.96 (s, 1H), 1.89 (d, *J*= *13.8* Hz, 1H), 1.82 (d, *J* = 13.1 Hz, 1H), 1.71 (dd, *J* = 38.6, 10.8 Hz, 2H), 1.60 (m, 2H). | 694.5 7 | General formula SM-T-1 for PROTA C synthesis |

(continued)

| Exa mpl e | Com poun d | Structure and Name | $^1$H-NMR | LC-MS | Synthesi s Method |
|---|---|---|---|---|---|
| 257 | SM-P-7 | 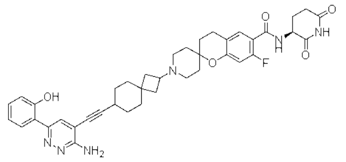 (S)-1'-(7-((3-amino-6-(2-hydroxyphenyl)pyridazin -4-yl)ethynyl) spiro[3.5]nona n-2-yl)-N-(2,6-dioxopyrimidin-3-yl)-7-fluorospiro [chromane-2,4'-piperidin]-6-car-boxamide | $^1$H NMR (400 MHz, Metha-nol-$d_4$) δ 8.29 (s, 1H), 7.68 (dd, *J* = 8.1, 1.8 Hz, 2H), 7.38 (m, 1H), 7.04 - 6.96 (m, 2H), 6.78 (d, *J* = 12.4 Hz, 1H), 4.83 (dd, *J*= 12.7, 5.4 Hz, 1H), 3.80 (d, *J*= 8.4 Hz, 1H), 3.46 (d, *J* = 12.3 Hz, 3H), 3.17 (t, *J* = 12.6 Hz, 2H), 2.94 - 2.87 (m, 2H), 2.87 - 2.77 (m, 2H), 2.72 (m, 1H), 2.42 (d, *J* = 11.3 Hz, 1H), 2.30 (m, 2H), 2.17 (m, 3H), 2.11 - 2.01 (m, 2H), 2.01 - 1.92 (m, 5H), 1.90 (d, *J* = 4.7 Hz, 1H), 1.78 (q, *J* = 12.1, 10.6 Hz, 2H), 1.74 - 1.65 (m, 1H), 1.60 (t, *J* = 11.5 Hz, 2H) | 707.5 7 | General formula SM-T-1 for PROTA C synthesis |
| 258 | SM-P-8 | 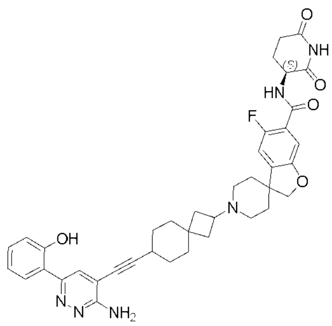 (S)-1'-(7-((3-amino-6-(2-hydroxyphenyl)pyridazin -4-yl)ethynyl) spiro[3.5]nona n-2-yl)-N-(2,6-dioxopyrimidin-3-yl)-5-fluoro-2H-spiro[benzofuran-3,4'-piperi-din]-6-carboxamide | $^1$H NMR (600 MHz, Metha-nol-$d_4$) δ 8.28 (s, 1H), 7.69 (dd, *J*= 8.1, 1.5 Hz, 1H), 7.40 - 7.35 (m, 1H), 7.20 (d, *J*= 5.4 Hz, 1H), 7.10 (d, *J* = 9.8 Hz, 1H), 7.00 (dt, *J*= 7.4, 3.2 Hz, 2H), 4.84 (dd, *J* = 12.7, 5.4 Hz, 1H), 4.60 (s, 2H), 3.70 (d, *J* = 8.4 Hz, 1H), 3.62 - 3.56 (m, 2H), 2.94 (t, *J* = 13.2 Hz, 2H), 2.85 (td, *J* = 13.2, 6.7 Hz, 2H), 2.73 (m, 1H), 2.42 (s, 1H), 2.34 - 2.26 (m, 2H), 2.25 - 2.15 (m, 3H), 2.12 (d, *J* = 14.2 Hz, 2H), 2.05 (m, 3H), 1.95 (s, 1H), 1.90 (d, *J* = 13.5 Hz, 1H), 1.83 (d, *J*= 11.6 Hz, 1H), 1.76 (d, *J* = 10.7 Hz, 1H), 1.69 (d, *J*= 10.9 Hz, 1H), 1.64 - 1.58 (m, 2H) | 693.5 7 | General formula SM-T-1 for PROTA C synthesis |

(continued)

| Exa mpl e | Com poun d | Structure and Name | $^1$H-NMR | LC-MS | Synthesi s Method |
|---|---|---|---|---|---|
| 259 | SM-P-9 | 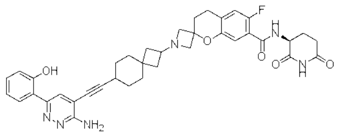 (S)-1-(7-((3-amino-6-(2-hydroxyphenyl)pyridazin -4-yl)ethynyl) spiro[3.5]nona n-2-yl)-N-(2,6-dioxopyrimidin-3-yl)-6'-fluorospiro [azetidine-3,2'-chromane]-7'-car-boxamide | $^1$H NMR (400 MHz, Methanol-$d_4$) δ 8.29 (s, 1H), 7.68 (dd, *J*= 8.1, 1.6 Hz, 1H), 7.38 (td, *J* = 7.6, 1.6 Hz, 2H), 7.07 (d, *J* = 11.0 Hz, 1H), 7.04 - 6.95 (m, 2H), 4.84 (dd, *J* = 12.5, 5.4 Hz, 1H), 4.43 (s, 1H), 4.27 (d, *J* = 30.9 Hz, 4H), 2.92 (d, *J* = 20.2 Hz, 2H), 2.83 (dt, *J*= 13.4, 5.1 Hz, 2H), 2.72 (m, 1H), 2.37 (s, 1H), 2.27 (dd, *J*= 15.4, 5.3 Hz, 4H), 2.19 (dd, *J*= 12.9, 4.8 Hz, 1H), 2.02 (d, *J*= 26.4 Hz, 1H), 1.90 (d, *J* = 12.8 Hz, 4H), 1.82 (d, *J* = 12.6 Hz, 1H), 1.76 - 1.70 (m, 1H), 1.66 (d, *J*= 10.4 Hz, 1H), 1.58 (dd, *J*= 16.4, 6.7 Hz, 2H) | 679.4 7 | General formula SM-T-1 for PROTA C synthesis |
| 260 | SM-P-10 | 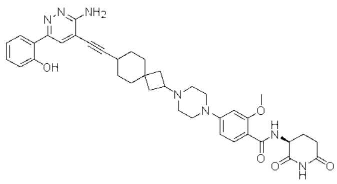 (S)-1'-(7-((3-amino-6-(2-hydroxyphenyl)pyridin-4-yl)ethyl)spiro [3.5]nonan-2-yl)-n--(2,6-dioxopiperidin-3-yl)-5-methoxy-2h-spiro [benzofuro-3,4'-piperidin]-6-car-boxamide | $^1$H NMR (600 MHz, Methanol-$d_4$) δ 8.28 (s, 1H), 7.69 (dd, *J*= 8.1, 1.6 Hz, 1H), 7.41 (s, 1H), 7.37 (td, *J* = 7.8, 7.3, 1.6 Hz, 1H), 7.03 - 6.98 (m, 3H), 4.79 (dd, *J*= 12.7, 5.2 Hz, 1H), 4.55 (s, 2H), 4.01 (s, 3H), 3.71 (p, *J* = 8.4 Hz, 1H), 3.59 (t, *J* = 7.3 Hz, 2H), 2.99 - 2.90 (m, 2H), 2.84 (ddd, *J* = 17.5, 13.6, 5.4 Hz, 2H), 2.73 (ddd, *J* = 17.7, 4.7, 2.4 Hz, 1H), 2.46 - 2.39 (m, 2H), 2.27 (ddd, *J*= 18.3, 12.3, 5.1 Hz, 3H), 2.12 - 2.01 (m, 6H), 1.96 (d, *J* = 11.7 Hz, 1H), 1.92 - 1.87 (m, 1H), 1.84 (d, *J*= 12.5 Hz, 1H), 1.76 (d, *J* = 11.0 Hz, 1H), 1.69 *(d, J*= 10.9 Hz, 1H), 1.64 - 1.57 (m, 2H). | 678.4 3 | General formula SM-T-1 for PROTA C synthesis |

(continued)

| Example | Compound | Structure and Name | $^1$H-NMR | LC-MS | Synthesis Method |
|---|---|---|---|---|---|
| 261 | SM-P-11 | 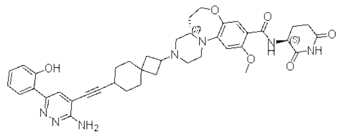 (S)-3-(7-((3-amino-6-(2-hydroxyphenyl)pyridin-4-yl)ethyl)spiro[3.5]nonan-2-yl)-n-((S)-2,6-dioxopiperidin-3-yl)-10-methoxy-2,3,4,4a,5,6-hexahydro-1h-benzo[b]pyrazino[1,2-d][1,4]oxazepine-9-carboxamide | $^1$H NMR (600 MHz, Methanol-$d_4$) $\delta$ 8.29 (d, $J$= 1.1 Hz, 1H), 7.69 (dd, $J$ = 8.1, 1.6 Hz, 1H), 7.57 (s, 1H), 7.38 (m, 1H), 7.03 - 6.98 (m, 2H), 6.74 (s, 1H), 4.77 (dd, $J$ = 12.8, 5.2 Hz, 1H), 4.27 (d, $J$= 6.5 Hz, 1H), 4.21 - 4.13 (m, 1H), 4.01 (s, 3H), 3.89 - 3.77 (m, 2H), 3.66 (s, 1H), 3.59 (s, 1H), 3.50 (q, $J$ = 12.9, 12.3 Hz, 2H), 3.10 (t, $J$ = 12.3 Hz, 1H), 2.97 (t, $J$ = 11.7 Hz, 1H), 2.84 (m, 2H), 2.72 (m, 1H), 2.42 (m, 2H), 2.31 (s, 1H), 2.19 - 2.12 (m, 1H), 2.11 - 2.01 (m, 4H), 1.96 (d, $J$ = 9.3 Hz, 2H), 1.91 (d, $J$= 13.5 Hz, 1H), 1.83 (d, $J$ = 12.7 Hz, 1H), 1.76 (d, $J$= 10.8 Hz, 1H), 1.69 (d, $J$= 11.0 Hz, 1H), 1.62 (m, 2H) | 720.5 7 | General formula SM-T-1 for PROTA C synthesis |
| 262 | SM-P-12 | 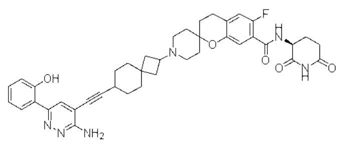 (S)-1'-(7-((3-amino-6-(2-hydroxyphenyl)pyridazin -4-yl)ethynyl) spiro[3.5]nona n-2-yl)-N-(2,6-dioxopyrimidin-3-yl)-6-fluorospiro [chromane-2,4'-piperidin]-7-carboxamide | $^1$H NMR (600 MHz, Methanol-$d_4$) $\delta$ 8.30 (s, 1H), 7.68 (dd, $J$ = 8.1, 1.7 Hz, 1H), 7.41 - 7.34 (m, 2H), 7.06 (d, $J$= 11.1 Hz, 1H), 7.03 - 6.98 (m, 2H), 4.84 (dd, $J$ = 12.6, 5.4 Hz, 1H), 3.81 (d, $J$ = 8.4 Hz, 1H), 3.43 (d, $J$ = 6.2 Hz, 2H), 3.18 (t, $J$ = 13.2 Hz, 2H), 2.93 (t, $J$= 6.9 Hz, 2H), 2.87 - 2.79 (m, 2H), 2.73 (ddd, $J$ = 17.6, 4.7, 2.6 Hz, 1H), 2.43 (s, 1H), 2.30 (ddq, $J$= 10.8, 5.4, 2.7 Hz, 2H), 2.23 - 2.11 (m, 3H), 2.08 - 2.00 (m, 2H), 2.00 - 1.93 (m, 4H), 1.93 - 1.84 (m, 3H), 1.81 (d, $J$ = 13.5 Hz, 1H), 1.75 (d, $J$ = 10.8 Hz, 1H), 1.68 (d, $J$= 10.8 Hz, 1H), 1.64 - 1.55 (m, 2H). | 707.4 7 | General formula SM-T-1 for PROTA C synthesis |

(continued)

| Example | Compound | Structure and Name | $^1$H-NMR | LC-MS | Synthesis Method |
|---|---|---|---|---|---|
| 263 | SM-P-13 | 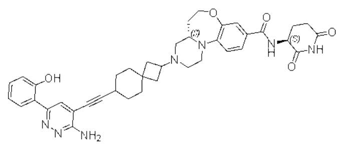 (S)-3-(7-((3-amino-6-(2-hydroxyphenyl)pyridazin -4-yl)ethynyl) spiro[3.5]nona n-2-yl)-N-((S)-2,6-dioxopyrimidin-3-yl)-2,3,4,4a,5,6-hexahydro-1H-benzo[b]pyrazino[1,2-d] [ 1,4] oxythiophene-9-carboxamide | $^1$H NMR (600 MHz, Methanol-$d_4$) δ 8.29 (d, *J* = 1.6 Hz, 1H), 7.68 (dd, *J*= 8.0, 1.6 Hz, 1H), 7.64 - 7.58 (m, 1H), 7.45 (d, *J* = 2.1 Hz, 1H), 7.41 - 7.36 (m, 1H), 7.11 *(d, J*= 8.4 Hz, 1H), 7.03 - 6.98 (m, 2H), 4.87 - 4.82 (m, 1H), 4.48 (t, *J* = 9.0 Hz, 1H), 4.25 - 4.20 (m, 1H), 3.85 - 3.76 (m, 1H), 3.66 (d, *J* = 13.6 Hz, 1H), 3.60 (s, 1H), 3.57 - 3.42 (m, 3H), 3.09 (t, *J*= 12.1 Hz, 1H), 3.00 (t, *J*= 11.6 Hz, 1H), 2.89 - 2.79 (m, 2H), 2.77 - 2.70 (m, 1H), 2.43 (d, *J*= 7.5 Hz, 1H), 2.31 (d, *J* = 6.1 Hz, 1H), 2.27 - 2.16 (m, 3H), 2.11 - 2.00 (m, 3H), 1.91 (d, *J*= 14.1 Hz, 3H), 1.83 (d, *J* = 12.2 Hz, 1H), 1.76 *(d, J*= 11.0 Hz, 1H), 1.69 (d, *J*= 11.0 Hz, 1H), 1.65 - 1.57 (m, 2H). | 690.5 7 | General formula SM-T-1 for PROTA C synthesis |
| 264 | SM-P-14 | 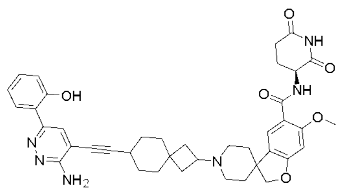 (S)-1'-(7-((3-amino-6-(2-hydroxyphenyl)pyridazin -4-yl)ethynyl) spiro[3.5]nona n-2-yl)-N-(2,6-dioxopiperidin-3-yl)-6-methoxy-2H-spiro[benzofuran-3,4'-piperidin]-5-carboxamide | $^1$H NMR (600 MHz, Methanol-$d_4$) δ 8.27 (d, *J*= 5.3 Hz, 1H), 7.87 (s, 1H), 7.69 (dd, *J*= 8.1, 1.6 Hz, 1H), 7.37 (ddd, *J*= *8.8,* 7.3, 1.6 Hz, 1H), 7.02 - 6.97 (m, 2H), 6.70 (s, 1H), 4.78 (dd, *J*= 12.7, 5.2 Hz, 1H), 4.63 (s, 2H), 4.01 (d, *J* = 3.2 Hz, 3H), 3.71 (d, *J* = 8.4 Hz, 1H), 3.62 - 3.56 (m, 2H), 2.98 - 2.91 (m, 2H), 2.88 - 2.81 (m, 2H), 2.73 (ddd, J = 17.6, 4.7, 2.4 Hz, 1H), 2.42 (ddd, *J*= 12.8, 6.4, 2.4 Hz, 2H), 2.31 (s, 1H), 2.15 (ddd, *J* = 17.7, 14.3, 8.0 Hz, 3H), 2.12 - 2.06 (m, 2H), 2.05 - 2.00 (m, 3H), 1.97 (d, *J*= 15.0 Hz, 1H), 1.90 (d, *J*= 13.7 Hz, 1H), 1.83 (d, *J* = 12.9 Hz, 1H), 1.76 (d, *J* = 10.9 Hz, 1H), 1.69 (d, *J* = 10.8 Hz, 1H), 1.65 - 1.59 (m, 2H) | 705.3 7 | General formula SM-T-1 for PROTA C synthesis |

(continued)

| Exa mpl e | Com poun d | Structure and Name | $^1$H-NMR | LC-MS | Synthesi s Method |
|---|---|---|---|---|---|
| 265 | SM-P-15 | 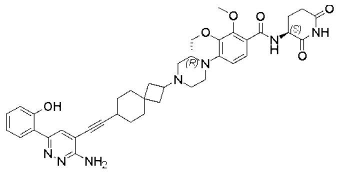 (R)-3-(7-((3-amino-6-(2-hydroxyphenyl)pyridazin -4-yl)ethyl)spiro [3.5]nonan-2-yl)-n-((S)-2,6-dioxopiperidin-3-yl)-7-methoxy-1,2,3,4,4,5-hexahydrobenzo [b]pyrazi no[1,2-d][1,4]oxazine-8-carboxamide | $^1$H NMR (600 MHz, Methanol-$d_4$) δ 8.27 (s, 1H), 7.69 (dd, *J* = 7.9, 1.5 Hz, 1H), 7.56 (d, *J* = 8.9 Hz, 1H), 7.37 (ddd, *J*= *8.8,* 7.3, 1.6 Hz, 1H), 7.03 - 6.97 (m, 2H), 6.89 (d, *J*= 9.0 Hz, 1H), 4.81 (dd, *J*= 12.8, 5.2 Hz, 1H), 4.48 (dd, *J*= 11.1, 2.6 Hz, 1H), 4.29 (d, *J* = 14.2 Hz, 1H), 4.16 (dd, *J*= 10.9, 6.4 Hz, 1H), 3.98 (s, 2H), 3.75 (s, 1H), 3.63 (s, 3H), 3.23 - 3.16 (m, 1H), 3.03 (d, *J* = 14.1 Hz, 1H), 2.84 (ddd, *J* = 13.8, 10.7, 7.0 Hz, 3H), 2.73 (ddd, *J* = 17.7, 4.6, 2.3 Hz, 1H), 2.43 (dtt, *J* = 7.4, 5.2, 2.4 Hz, 2H), 2.31 (s, 1H), 2.21 (t, *J*= 7.6 Hz, 1H), 2.12 - 2.00 (m, 4H), 1.96 (s, 1H), 1.89 (d, *J* = 13.2 Hz, 1H), 1.83 (d, *J* = 12.6 Hz, 1H), 1.75 (d, *J*= 10.6 Hz, 1H), 1.72 - 1.65 (m, 1H), 1.61 (dd, *J* = 13.7, 10.2 Hz, 2H). | 706.5 7 | General formula SM-T-1 for PROTA C synthesis |
| 266 | SM-P-16 | 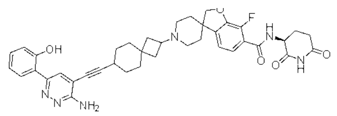 (S)-1'-(7-((3-amino-6-(2-hydroxyphenyl)pyridin-4-yl)ethyl)spiro [3.5]nonan-2-yl)-n-(2,6-dioxopiperidin-3-yl)-7-fluoro-2h-spiro [benzofuran-3,4'-piperidin]-6-carboxamide | $^1$H NMR (400 MHz, Methanol-$d_4$) δ 8.26 (s, 1H), 7.69 (dd, *J* = 8.1, 1.6 Hz, 1H), 7.40 - 7.32 (m, 2H), 7.10 (d, *J*= 7.9 Hz, 1H), 7.03 - 6.95 (m, 2H), 4.85 (dd, *J* = 12.6, 5.4 Hz, 1H), 4.71 (s, 2H), 3.71 (t, *J* = 8.2 Hz, 1H), 3.60 (d, *J*= 12.7 Hz, 2H), 2.95 (t, *J*= 12.5 Hz, 2H), 2.84 (ddq, *J*= 17.2, 9.5, 4.8, 4.0 Hz, 2H), 2.73 (ddd, *J* = 17.6, 4.8, 2.7 Hz, 1H), 2.43 (s, 1H), 2.29 (dtd, *J* = 10.9, 5.4, 2.6 Hz, 3H), 2.23 - 2.10 (m, 4H), 2.04 (q, *J* = 10.7 Hz, 3H), 1.94 *(d, J*= 14.6 Hz, 1H), 1.92 - 1.79 (m, 2H), 1.72 (dd, *J* = 27.5, 11.0 Hz, 2H), 1.65 - 1.57 (m, 2H). | 693.5 7 | General formula SM-T-1 for PROTA C synthesis |

(continued)

| Example | Compound | Structure and Name | $^{1}$H-NMR | LC-MS | Synthesis Method |
|---|---|---|---|---|---|
| 267 | SM-P-17 | 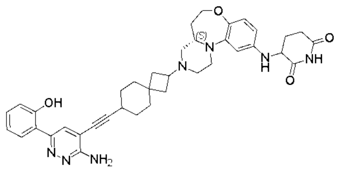 3-((S)-3-(7-((3-amino-6-(2-hydroxyphenyl)pyridazin -4-yl)ethynyl) spiro[3.5]nonan-2-yl)-2,3,4,4a,5,6-hexahydro-1H-benzo[b]pyrazino[1,2-d][1,4]oxaziridin-10-yl)amino)piperidine-2,6-dione | $^{1}$H NMR (600 MHz, Methanol-$d_4$) δ 8.32 (s, 1H), 7.71 - 7.65 (m, 1H), 7.39 (t, *J* = 7.9 Hz, 1H), 7.01 (t, *J*= 7.1 Hz, 2H), 6.75 (d, *J*= 8.5 Hz, 1H), 6.48 - 6.44 (m, 1H), 6.42 (dd, *J* = 8.5, 2.6 Hz, 1H), 4.26 (dt, *J* = 11.8, 6.6 Hz, 2H), 4.08 (dt, *J* = 11.2, 5.6 Hz, 1H), 3.78 (d, *J* = 8.6 Hz, 1H), 3.64 - 3.54 (m, 2H), 3.49 - 3.41 (m, 2H), 3.38 (d, *J* = 12.8 Hz, 1H), 3.06 (t, *J*= 12.1 Hz, 1H), 2.95 (t, *J*= 11.4 Hz, 1H), 2.81 (ddd, *J*= 17.6, 13.1, 6.3 Hz, 2H), 2.74 (dt, *J*= 17.8, 4.3 Hz, 1H), 2.43 (s, 1H), 2.35 - 2.25 (m, 2H), 2.12 (d, *J* = 17.4 Hz, 1H), 2.04 (dt, *J* = 19.3, 10.1 Hz, 3H), 1.96 (ddd, *J* = 12.4, 8.5, 3.5 Hz, 2H), 1.94 - 1.85 (m, 2H), 1.82 (d, *J* = 13.2 Hz, 1H), 1.76 (d, *J*= 10.9 Hz, 1H), 1.68 (d, *J*= 11.4 Hz, 1H), 1.61 (td, *J* = 11.9, 10.7, 3.3 Hz, 2H). | 662.5 7 | General formula SM-T-1 for PROTA C synthesis |
| 268 | SM-P-18 | 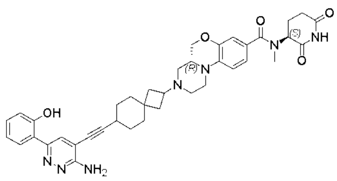 ((R)-3-(7-((3-amino-6-(2-hydroxyphenyl)pyridazin -4-yl)ethynyl) spiro[3.5]nonan-2-yl)-N-((S)-2,6-dioxopyrimidin-3-yl)-N-methyl-1,2,3,4,4a,5-hexahydrobenzo[b]pyrazi no[1,2-d][1,4]oxazine-8-carboxamide | 1H NMR (600 MHz, Methanol-$d_4$) δ 8.33 (s, 1H), 7.67 (dd, *J* = 8.2, 1.7 Hz, 1H), 7.40 (ddd, *J* = 8.6, 7.5, 1.7 Hz, 1H), 7.14 - 7.08 (m, 1H), 7.06 (d, *J*= *8.6* Hz, 1H), 7.03 - 6.97 (m, 3H), 4.75 (dd, *J*= 12.2, 5.0 Hz, 1H), 4.39 (d, *J* = 10.4 Hz, 1H), 4.25 (d, *J*= 14.2 Hz, 1H), 4.10 (t, *J*= 9.3 Hz, 1H), 3.74 (d, *J* = 10.0 Hz, 1H), 3.63 (s, 2H), 3.54 (s, 1H), 3.14 (t, *J* = 12.9 Hz, 1H), 3.04 (s, 3H), 2.94 (s, 1H), 2.86 - 2.73 (m, 3H), 2.71 - 2.58 (m, 1H), 2.56 - 2.47 (m, 1H), 2.43 (s, 1H), 2.30 (s, 1H), 2.20 - 2.13 (m, 1H), 2.11 - 1.99 (m, 3H), 1.96 (s, 1H), 1.88 (d, *J*= 13.4 Hz, 1H), 1.81 (s, 1H), 1.75 (d, *J*= 10.8 Hz, 1H), 1.68 (d, *J*= 10.7 Hz, 1H), 1.60 (d, *J* = 10.9 Hz, 2H) | | General formula SM-T-1 for PROTA C synthesis |

(continued)

| Example | Compound | Structure and Name | $^1$H-NMR | LC-MS | Synthesis Method |
|---|---|---|---|---|---|
| 269 | SM-P-19 | 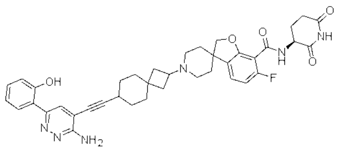 (S)-1'-(7-((3-amino-6-(2-hydroxyphenyl)pyridin-4-yl)ethyl)spiro [3.5]nonan-2-yl)-n-(2,6-dioxopiperidin-3-yl)-6-fluoro-2h-spiro [benzofuran-3,4'-piperidin]-7-carboxamide | $^1$H NMR (400 MHz, Methanol-$d_4$) δ 8.26 (s, 1H), 7.69 (dd, *J*= 8.1, 1.6 Hz, 1H), 7.36 (td, *J* = 7.7, 1.6 Hz, 1H), 7.28 (dd, *J* = 8.3, 5.1 Hz, 1H), 7.03 - 6.96 (m, 2H), 6.80 (dd, *J* = 10.5, 8.3 Hz, 1H), 4.85 (dd, *J* = 12.5, 5.2 Hz, 1H), 4.69 (s, 2H), 3.70 (p, *J*= 8.3 Hz, 1H), 3.59 (d, *J* = 12.7 Hz, 2H), 2.94 (t, *J* = 12.5 Hz, 2H), 2.84 (ddd, *J* = 18.2, 13.1, 5.4 Hz, 2H), 2.72 (ddd, *J* = 17.7, 4.8, 2.8 Hz, 1H), 2.43 (s, 1H), 2.32 (dtd, *J*= 13.2, 5.3, 2.9 Hz, 2H), 2.25 - 2.16 (m, 2H), 2.16 - 2.08 (m, 3H), 2.02 (t, *J*= 10.1 Hz, 3H), 1.97 - 1.86 (m, 2H), 1.86 - 1.73 (m, 2H), 1.68 (d, *J* = 11.7 Hz, 1H), 1.60 (dd, *J*= 13.4, 10.2 Hz, 2H). | 693.5 7 | General formula SM-T-1 for PROTA C synthesis |
| 270 | SM-P-20 | 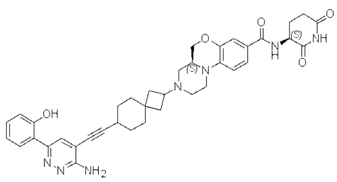 (S)-3-(7-((3-amino-6-(2-hydroxyphenyl)pyridazin -4-yl)ethynyl) spiro[3.5]nona n-2-yl)-N-((S)-2,6-dioxopyrimidin-3-yl)-1,2,3,4,4a,5-hexahydrobenzo [b]pyrazi no[1,2-d][1,4]oxazine-8-carboxamide | $^1$H NMR (400 MHz, Methanol-$d_4$) δ 8.28 (s, 1H), 7.68 (dd, *J*= 8.0, 1.6 Hz, 1H), 7.49 (dd, *J*= *8.6,* 2.1 Hz, 1H), 7.41 - 7.34 (m, 2H), 7.06 (d, *J* = 8.7 Hz, 1H), 7.04 - 6.96 (m, 2H), 4.87 - 4.80 (m, 1H), 4.39 (dd, *J* = 11.2, 2.6 Hz, 1H), 4.28 (d, *J* = 14.0 Hz, 1H), 4.10 (dd, *J*= 11.2, 6.8 Hz, 1H), 3.74 (d, *J* = 10.3 Hz, 1H), 3.62 (d, *J* = 13.4 Hz, 3H), 3.19 (d, *J* = 13.0 Hz, 1H), 3.04 (d, *J*= 12.5 Hz, 1H), 2.90 - 2.77 (m, 3H), 2.72 (dt, *J* = 17.5, 3.8 Hz, 1H), 2.43 (s, 1H), 2.30 (s, 1H), 2.19 (td, *J* = 12.1, 10.7, 4.5 Hz, 2H), 2.05 (q, *J* = 11.8, 10.9 Hz, 4H), 1.89 (d, *J* = 13.7 Hz, 1H), 1.85 - 1.78 (m, 1H), 1.71 (dd, *J*= 24.3, 11.4 Hz, 2H), 1.65 - 1.54 (m, 2H). | 676.5 7 | General formula SM-T-1 for PROTA C synthesis |

(continued)

| Example | Compound | Structure and Name | $^1$H-NMR | LC-MS | Synthesis Method |
|---|---|---|---|---|---|
| 271 | SM-P-21 | 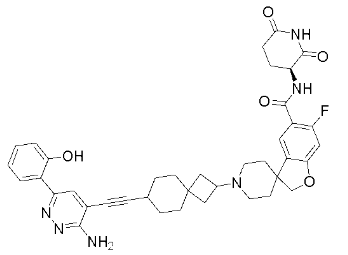 (S)-1'-(7-((3-amino-6-(2-hydroxyphenyl)pyridazin -4-yl)ethynyl) spiro[3.5]nona n-2-yl)-N-(2,6-dioxopyrimidin-3-yl)-6-fluoro-2H-spiro[benzofuran-3,4'-piperidin]-5-carboxamide | $^1$H NMR (600 MHz, Methanol-$d_4$) δ 8.29 (s, 1H), 7.72 - 7.66 (m, 2H), 7.38 (ddd, *J* = 8.7, 7.3, 1.6 Hz, 1H), 7.03 - 6.98 (m, 2H), 6.74 (d, *J*= 11.7 Hz, 1H), 4.83 (dd, *J* = 12.5, 5.4 Hz, 1H), 4.68 (s, 2H), 3.71 (p, *J* = 8.4 Hz, 1H), 3.64 - 3.56 (m, 2H), 2.95 (t, *J*= 13.1 Hz, 2H), 2.84 (ddd, *J*= 18.6, 13.4, 5.5 Hz, 2H), 2.73 (ddd, *J* = 17.6, 4.6, 2.5 Hz, 1H), 2.44 (s, 1H), 2.34 - 2.27 (m, 2H), 2.25 - 2.15 (m, 3H), 2.12 (d, *J* = 14.4 Hz, 2H), 2.08 - 2.00 (m, 3H), 1.97 (d, *J*= 17.5 Hz, 1H), 1.90 (d, *J* = 13.5 Hz, 1H), 1.82 (s, 1H), 1.76 (d, *J*= 10.8 Hz, 1H), 1.69 (d, *J*= 10.7 Hz, 1H), 1.62 (t, *J* = 10.0 Hz, 2H). | 693.5 7 | General formula SM-T-1 for PROTA C synthesis |
| 272 | SM-P-22 | 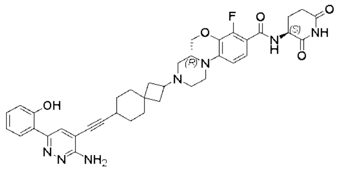 (R)-3-(7-((3-amino-6-(2-hydroxyphenyl)pyridazin -4-yl)ethynyl) spiro[3.5]nona n-2-yl)-N-((S)-2,6-dioxopyrimidin-3-yl)-7-fluoro-1,2,3,4,4a,5-hexahydrobenzo[b]pyrazi no[1,2-d][1,4] oxazine-8-carboxamide | $^1$H NMR (600 MHz, Methanol-$d_4$) δ 8.30 (s, 1H), 7.68 (dd, *J*= 8.1, 1.6 Hz, 1H), 7.39 (ddd, *J* = 13.5, 8.4, 6.8 Hz, 2H), 7.03 - 6.98 (m, 2H), 6.90 (d, *J*= 9.0 Hz, 1H), 4.82 (dd, *J* = 12.7, 5.3 Hz, 1H), 4.47 (dd, *J* = 11.2, 2.6 Hz, 1H), 4.28 (d, *J* = 14.2 Hz, 1H), 4.15 (dd, *J*= 11.3, 7.1 Hz, 1H), 3.75 (s, 1H), 3.63 (s, 3H), 3.22 *(t, J= 13.3* Hz, 1H), 3.03 (s, 1H), 2.88 - 2.79 (m, 3H), 2.72 (ddd, *J* = 17.6, 4.7, 2.5 Hz, 1H), 2.43 (s, 1H), 2.30 (dtd, *J* = 13.0, 5.3, 2.5 Hz, 2H), 2.18 (dd, *J* = 13.0, 4.6 Hz, 1H), 2.06 (dd, *J* = 10.8, 5.9 Hz, 3H), 1.96 (s, 1H), 1.89 (dd, *J* = 14.2, 4.2 Hz, 1H), 1.82 (d, *J* = 12.0 Hz, 1H), 1.70 (dt, *J* = 36.8, 10.9 Hz, 2H), 1.61 (ddd, *J* = 13.5, 10.4, 3.2 Hz, 2H). | 694.5 7 | General formula SM-T-1 for PROTA C synthesis |

(continued)

| Example | Compound | Structure and Name | $^1$H-NMR | LC-MS | Synthesis Method |
|---|---|---|---|---|---|
| 273 | SM-P-23 | 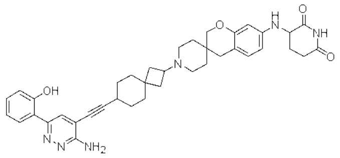 3-((1'-(7-((3-amino-6-(2-hydroxyphenyl)pyridazin -4-yl)ethynyl) spiro[3.5]nona n-2-yl)spiro[chromane-3,4'-piperidin]-7-yl)amino) piperidine-2,6-dione | $^1$H NMR (600 MHz, Methanol-$d_4$) δ 8.28 (s, 1H), 7.68 (dd, *J*= 8.1, 1.6 Hz, 1H), 7.40 - 7.36 (m, 1H), 7.03 - 6.98 (m, 2H), 6.86 (dd, *J* = 23.0, 8.3 Hz, 1H), 6.36 (dt, *J* = 8.2, 2.8 Hz, 1H), 6.22 (d, *J*= 2.2 Hz, 1H), 4.25 (dt, *J*= 11.9, 4.0 Hz, 1H), 4.08 (s, 1H), 3.82 (s, 1H), 3.75 (q, *J* = 8.4 Hz, 1H), 3.43 (s, 2H), 3.06 - 2.96 (m, 2H), 2.82 (dd, *J* = 13.6, 8.2 Hz, 2H), 2.79 (s, 1H), 2.76 - 2.70 (m, 1H), 2.54 (s, 1H), 2.41 (s, 1H), 2.35 - 2.26 (m, 2H), 2.02 (d, *J* = 20.0 Hz, 1H), 1.99 - 1.92 (m, 4H), 1.90 (dd, *J* = 12.4, 7.4 Hz, 1H), 1.82 (t, *J* = 17.7 Hz, 3H), 1.75 (d, *J* = 10.2 Hz, 1H), 1.73 - 1.65 (m, 3H), 1.59 (ddd, *J* = 13.9, 10.6, 3.3 Hz, 2H). | 661.6 7 | General formula SM-T-1 for PROTA C synthesis |
| 274 | SM-P-24 | 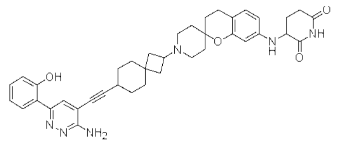 3-((1'-(7-((3-amino-6-(2-hydroxyphenyl)pyridazin -4-yl)ethynyl) spiro[3.5]nona n-2-yl)spiro[chromane-2,4'-piperidin]-7-yl)amino) piperidine-2,6-dione | $^1$H NMR (400 MHz, Methanol-$d_4$) δ 8.29 (s, 1H), 7.68 (dd, *J* = 8.1, 1.7 Hz, 1H), 7.38 (ddd, *J*= 8.3, 7.3, 1.7 Hz, 1H), 7.03 - 6.97 (m, 2H), 6.89 (d, *J*= 8.3 Hz, 1H), 6.37 (dd, *J* = 8.3, 2.4 Hz, 1H), 6.27 (d, *J* = 2.3 Hz, 1H), 4.23 (dd, *J* = 11.8, 4.9 Hz, 1H), 3.78 (p, *J* = 8.4 Hz, 1H), 3.42 (d, *J*= 12.3 Hz, 2H), 3.16 (t, *J* = 12.8 Hz, 2H), 2.89 - 2.79 (m, 2H), 2.78 - 2.69 (m, 3H), 2.41 (d, *J* = 11.6 Hz, 1H), 2.37 - 2.25 (m, 2H), 2.14 (d, *J*= 14.6 Hz, 2H), 1.98 (qd, *J*= 11.7, 11.1, 7.0 Hz, 5H), 1.88 (t, *J*= 6.8 Hz, 4H), 1.81 (d, *J*= 12.0 Hz, 2H), 1.76 - 1.65 (m, 2H), 1.60 (t, *J* = 11.3 Hz, 2H). | 661.4 7 | General formula SM-T-1 for PROTA C synthesis |

(continued)

| Example | Compound | Structure and Name | $^1$H-NMR | LC-MS | Synthesis Method |
|---|---|---|---|---|---|
| 275 | SM-P-25 | 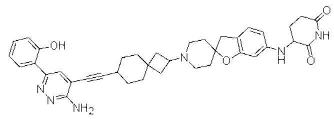 3-((1'-(7-((3-amino-6-(2-hydroxyphenyl)pyridazin -4-yl)ethynyl) spiro[3.5]nona n-2-yl)-3H-spiro [benzofuran-2,4'-piperidin]-6-yl) amino)piperidine-2,6-dione | $^1$H NMR (400 MHz, Methanol-$d_4$) δ 8.29 (s, 1H), 7.68 (dd, *J* = 8.1, 1.7 Hz, 1H), 7.38 (ddd, *J* = 8.5, 7.4, 1.7 Hz, 1H), 7.05 - 6.94 (m, 3H), 6.31 (dd, *J*= 8.1, 2.1 Hz, 1H), 6.24 (d, *J* = 2.0 Hz, 1H), 4.25 (dd, *J* = 11.8, 4.9 Hz, 1H), 3.77 (p, *J* = 8.3 Hz, 1H), 3.49 (d, *J* = 12.1 Hz, 2H), 3.19 (t, *J*= 12.5 Hz, 2H), 3.08 (s, 1H), 3.01 (s, 2H), 2.90 - 2.79 (m, 2H), 2.79 - 2.73 (m, 1H), 2.43 (s, 1H), 2.37 - 2.27 (m, 2H), 2.22 (d, *J* = 14.7 Hz, 2H), 2.07 - 1.95 (m, 5H), 1.94 - 1.87 (m, 2H), 1.78 (q, *J*= 11.8, 11.2 Hz, 2H), 1.68 (d, *J* = 11.3 Hz, 1H), 1.60 (t, *J*= 11.5 Hz, 2H). | 647.5 7 | General formula SM-T-1 for PROTA C synthesis |
| 276 | SM-P-26 | 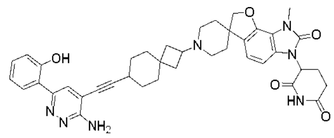 3-(1'-(7-((3-amino-6-(2-hydroxyphenyl)pyridazin -4-yl)ethynyl) spiro[3.5]nona n-2-yl)-1-methyl-2-oxo-1,2-dihydro-3H,7H-spiro[benzofuro[6,7-d]imidazole-6,4'-piperidin]-3-yl) piperidine-2,6-dione | $^1$H NMR (600 MHz, Methanol-$d_4$) δ 8.27 (s, 1H), 7.69 (dd, *J*= 8.1, 1.6 Hz, 1H), 7.37 (td, *J*= 7.9, 7.4, 1.6 Hz, 1H), 7.02 - 6.97 (m, 2H), 6.91 (d, *J*= 8.1 Hz, 1H), 6.74 (d, *J*= 8.1 Hz, 1H), 5.32 (dd, *J* = 12.8, 5.6 Hz, 1H), 4.68 (s, 2H), 3.71 (d, *J* = 8.4 Hz, 1H), 3.60 (s, 1H), 3.58 (s, 3H), 2.99 - 2.90 (m, 3H), 2.86 - 2.81 (m, 2H), 2.80 - 2.76 (m, 1H), 2.43 (s, 1H), 2.30 (s, 1H), 2.26 - 2.19 (m, 2H), 2.17 (dd, *J* = 5.9, 4.5 Hz, 2H), 2.09 (d, *J* = 14.6 Hz, 2H), 2.06 - 2.00 (m, 3H), 1.96 (s, 1H), 1.90 *(d, J*= 13.7 Hz, 1H), 1.83 (d, *J*= 12.4 Hz, 1H), 1.76 (d, *J*= 10.1 Hz, 1H), 1.69 (d, *J* = 11.0 Hz, 1H), 1.61 (td, *J* = 12.1, 10.8, 3.7 Hz, 2H). | 702.5 7 | General formula SM-T-1 for PROTA C synthesis |

(continued)

| Exa mpl e | Com poun d | Structure and Name | $^1$H-NMR | LC-MS | Synthesi s Method |
|---|---|---|---|---|---|
| 277 | SM-P-27 | 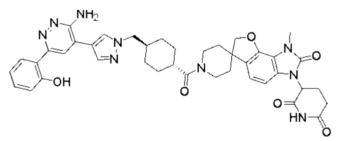 3-(1'-((1r,4r)-4-((4-(3-amino-6-(2-hydroxyphenyl)pyridazin -4-yl)-1H-pyrazol-1-yl)methyl)cyclohexane-1-carbonyl)-1-methyl-2-oxo-1,2-dihvdro-3H,7H-spiro [benzofuro[6,7-d]imidazole-6,4'-piperidin]-3-yl)piperidine-2,6-dione | $^1$H NMR (400 MHz, Methanol-$d_4$) δ 8.27 (d, $J$= 0.8 Hz, 1H), 8.13 (s, 1H), 8.08 (d, $J$ = 0.8 Hz, 1H), 7.88 (dd, $J$ = 8.2, 1.6 Hz, 1H), 7.29 (ddd, $J$= 8.8, 7.3, 1.6 Hz, 1H), 6.99 - 6.93 (m, 2H), 6.92 (d, $J$= 8.1 Hz, 1H), 6.66 (dd, $J$= 8.1, 2.7 Hz, 1H), 5.29 (d, $J$= 12.3 Hz, 1H), 4.64 (dd, $J$= 13.9, 4.8 Hz, 3H), 4.51 (d, $J$= 13.9 Hz, 1H), 4.14 (d, $J$= 7.1 Hz, 2H), 4.08 (d, $J$ = 14.2 Hz, 1H), 3.58 (s, 3H), 2.97 - 2.86 (m, 2H), 2.84 - 2.68 (m, 4H), 2.19 - 2.12 (m, 1H), 2.04 (d, $J$= 15.3 Hz, 1H), 1.88 (s, 3H), 1.80 (d, $J$= 15.5 Hz, 3H), 1.56 (dd, $J$ = 27.7, 13.6 Hz, 3H), 1.23 (d, $J$= 13.0 Hz, 3H). | 746.5 7 | General formula SM-T-2 for PROTA C synthesis |
| 278 | SM-P-28 | 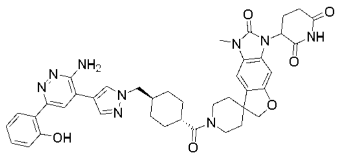 3-(1'-((1r,4r)-4-((4-(3-amino-6-(2-hydroxyphenyl)pyridazin -4-yl)-1H-pyrazol-1-yl)methyl)cyclohexane-1-carbonyl)-1-methyl-2-oxo-1,2-dihydro-3H,6H-spiro [benzofuro[5,6-d]imidazole-7,4'-piperidin]-3-yl)piperidine-2,6-dione | $^1$H NMR (400 MHz, Methanol-$d_4$) δ 8.31 - 8.26 (m, 1H), 8.13 (s, 1H), 8.08 (d, $J$ = 0.7 Hz, 1H), 7.88 (dd, $J$ = 8.2, 1.6 Hz, 1H), 7.29 (ddd, $J$ = 8.5, 7.3, 1.6 Hz, 1H), 7.05 (s, 1H), 7.00 - 6.93 (m, 2H), 6.64 (d, $J$ = 3.3 Hz, 1H), 5.32 - 5.22 (m, 1H), 4.52 (d, $J$ = 5.3 Hz, 3H), 4.14 (d, $J$ = 7.1 Hz, 2H), 4.09 (d, $J$ = 13.9 Hz, 1H), 3.40 (s, 3H), 3.31 - 3.23 (m, 1H), 2.99 - 2.84 (m, 2H), 2.83 - 2.62 (m, 4H), 2.15 (dt, $J$ = 8.6, 4.4 Hz, 1H), 2.06 - 1.98 (m, 1H), 1.89 (d, $J$= 16.9 Hz, 4H), 1.83 - 1.76 (m, 3H), 1.56 (dd, $J$= 29.0, 13.6 Hz, 2H), 1.29 - 1.17 (m, 2H). | 746.5 8 | General formula SM-T-2 for PROTA C synthesis |

(continued)

| Exa mpl e | Com poun d | Structure and Name | $^1$H-NMR | LC-MS | Synthesi s Method |
|---|---|---|---|---|---|
| 279 | SM-P-29 | 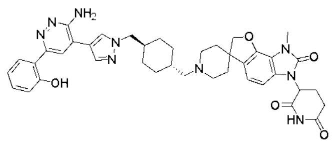 3-(1'-(((1r,4r)-4-((4-(3-amino-6-(2-hydroxyphenyl)pyridazin-4-yl)-1H-pyrazol-1-yl)methyl)cyclohexyl)me thyl)-1-methyl-2-oxo-1,2-dihydro-3H,7H-spiro [benzofuro[6,7-d]imidazole-6,4'-piperidin]-3-yl)piperidine-2,6-dione | $^1$H NMR (600 MHz, DMSO-$d_6$) δ 13.83 (s, 1H), 11.09 (s, 1H), 8.44 (s, 1H), 8.20 (d, *J* = 18.2 Hz, 2H), 8.02 (dd, *J* = 8.3, 1.5 Hz, 1H), 7.30 - 7.25 (m, 1H), 6.93 (dt, *J*= 7.4, 3.3 Hz, 2H), 6.85 (s, 1H), 6.65 (d, *J* = 8.0 Hz, 1H), 6.49 (s, 2H), 5.32 (dd, *J* = 12.8, 5.4 Hz, 1H), 4.53 (s, 2H), 4.04 (d, *J* = 7.0 Hz, 2H), 3.44 (s, 3H), 2.90 (td, *J* = 17.6, 15.4, 5.4 Hz, 3H), 2.74 - 2.56 (m, 3H), 2.06 - 1.91 (m, 3H), 1.84 (t, *J*= 15.7 Hz, 4H), 1.72 (s, 1H), 1.65 (d, *J* = 12.2 Hz, 2H), 1.45 (s, 2H), 1.13 - 0.98 (m, 2H), 0.91 (s, 2H). | 732.4 7 | General formula SM-T-3 for PROTA C synthesis |
| 280 | SM-P-30 | 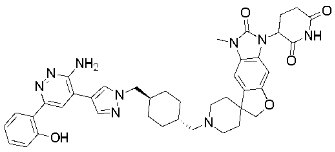 3-(1'-(((1r,4r)-4-((4-(3-amino-6-(2-hydroxyphenyl)pyridazin-4-yl)-1H-pyrazol-1-yl)methyl)cyclohexyl)me thyl)-1-methyl-2-oxo-1,2-dihydro-3H,6H-spiro [benzofuro[5,6-d]imidazole-7,4'-piperidin]-3-yl)piperidine-2,6-dione | $^1$H NMR (600 MHz, Methanol-$d_4$) δ 8.27 (s, 1H), 8.11 (d, *J* = 21.5 Hz, 2H), 7.88 (dd, *J* = 8.3, 1.5 Hz, 1H), 7.29 (td, *J* = 7.5, 1.5 Hz, 1H), 7.05 (s, 1H), 6.99 - 6.94 (m, 2H), 6.63 (s, 1H), 5.28 (dd, *J* = 12.8, 5.5 Hz, 1H), 4.44 (s, 2H), 4.13 (d, *J* = 7.1 Hz, 2H), 3.42 (s, 3H), 3.16 (s, 2H), 2.92 (ddd, *J* = 18.0, 14.2, 5.4 Hz, 1H), 2.84 - 2.74 (m, 2H), 2.51 (s, 2H), 2.20 - 2.09 (m, 3H), 1.95 (d, *J* = 13.0 Hz, 3H), 1.83 (d, *J* = 13.3 Hz, 2H), 1.76 (d, *J*= 12.4 Hz, 2H), 1.70 (s, 1H), 1.17 (dt, *J* = 16.0, 11.6 Hz, 2H), 1.07 (t, *J*= 12.4 Hz, 2H). | 732.4 7 | General formula SM-T-3 for PROTA C synthesis |

(continued)

| Exa mpl e | Com poun d | Structure and Name | $^1$H-NMR | LC-MS | Synthesi s Method |
|---|---|---|---|---|---|
| 281 | SM-P-31 | 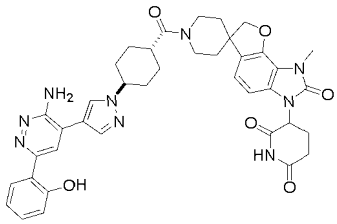 3-(1'-(1r,4r)-4-(4-(3-amino-6-(2-hydroxyphenyl)pyridin-4-yl)-1H-pyrazol-1-yl)cyclohexane-1-carbonyl)-1-methyl-2-oxo-1,2-dihydro-3H,7H-spiro[benzofuro[6,7-d]imidazole-6,4'-piperidin]-3-yl) piperidine-2,6-dione | $^1$H NMR (600 MHz, Methanol-$d_4$) δ 8.38 (s, 1H), 8.33 (s, 1H), 8.09 (s, 1H), 7.72 (dd, *J* = 7.8, 1.6 Hz, 1H), 7.42 (td, *J* = 7.8, 1.6 Hz, 1H), 7.04 (ddd, *J*= 9.2, 7.7, 1.7 Hz, 2H), 6.94 (d, *J* = 8.1 Hz, 1H), 6.70 - 6.64 (m, 1H), 5.30 (dt, *J* = 12.3, 6.3 Hz, 1H), 4.68 (q, *J* = 9.4 Hz, 2H), 4.55 (d, *J* = 13.9 Hz, 1H), 4.42 - 4.33 (m, 1H), 4.14 (d, *J* = 14.2 Hz, 1H), 3.59 (s, 3H), 3.41 - 3.34 (m, 1H), 2.98 - 2.86 (m, 3H), 2.84 - 2.75 (m, 2H), 2.28 (d, *J*= 9.9 Hz, 2H), 2.20 - 2.13 (m, 1H), 2.04 (d, *J*= 15.6 Hz, 4H), 1.97 - 1.87 (m, 3H), 1.85 - 1.72 (m, 3H) | | General formula SM-T-4 for PROTA C synthesis |
| 282 | SM-P-32 | 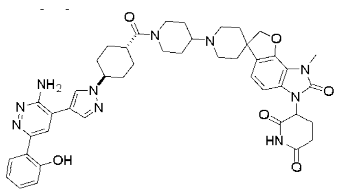 3-(1'-(1-(1r,4r)-4-(4-(3-amino-6-(2-hydroxyphenyl)pyridin-4-yl)-1h-pyrazol-1-yl)cyclohexane-1-carbonyl)piperidin-4-yl)-6-oxo-6,8-dihydro-2h,7h-spiro[furo [2,3-e]isoindol-3,4'-piperidin]-7-yl)piperidine-2,6-dione | $^1$H NMR (400 MHz, Methanol-$d_4$) δ 8.37 (s, 1H), 8.30 (s, 1H), 8.11 (s, 1H), 7.78 - 7.72 (m, 1H), 7.43 - 7.37 (m, 1H), 7.02 (q, *J* = 7.3 Hz, 2H), 6.70 (s, 1H), 5.30 (dd, *J* = 12.5, 5.5 Hz, 1H), 4.55 (d, *J* = 4.7 Hz, 2H), 4.35 (d, *J*= 12.2 Hz, 2H), 3.84 - 3.53 (m, 4H), 3.42 (d, *J* = 8.2 Hz, 2H), 3.21 (s, 2H), 2.98 - 2.87 (m, 2H), 2.87 - 2.67 (m, 4H), 2.28 (d, *J* = 12.2 Hz, 4H), 2.17 (ddd, *J*= 23.7, 16.8, 10.3 Hz, 4H), 2.08 - 1.94 (m, 4H), 1.92 - 1.70 (m, 4H), 1.65 (t, *J*= 13.1 Hz, 2H). | 815.6 6 | General formula SM-T-4 for PROTA C synthesis |

(continued)

| Example | Compound | Structure and Name | $^1$H-NMR | LC-MS | Synthesis Method |
|---|---|---|---|---|---|
| 283 | SM-P-33 | 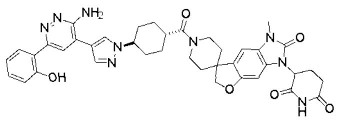 3-(1'-((1r,4r)-4-(4-(3-amino-6-(2-hydroxyphenyl)pyridin-4-yl)-1h-pyrazol-1-yl)cyclohexane-1-carbonyl)-1-methyl-2-oxo-1,2-dihydro-3h,6h-spiro[benzofuro[5,6-d]imidazole-7,4'-piperidin]-3-yl)piperidine-2,6-dione | $^1$H NMR (600 MHz, Methanol-$d_4$) δ 8.39 (s, 1H), 8.34 (d, *J* = 1.0 Hz, 1H), 8.09 (s, 1H), 7.72 (dd, *J* = 7.7, 1.6 Hz, 1H), 7.42 (ddd, *J*= *8.8,* 7.4, 1.6 Hz, 1H), 7.08 - 7.02 (m, 3H), 6.65 (d, *J* = 6.9 Hz, 1H), 5.29 (dt, *J* = 12.6, 6.7 Hz, 1H), 4.55 (qd, *J* = 12.2, 10.1, 6.5 Hz, 3H), 4.37 (td, *J* = 11.7, 5.8 Hz, 1H), 4.15 (d, *J* = 14.1 Hz, 1H), 3.41 (s, 3H), 3.36 (d, *J* = 11.0 Hz, 1H), 2.97 - 2.85 (m, 3H), 2.83 - 2.75 (m, 2H), 2.28 (d, *J* = 12.5 Hz, 2H), 2.19 - 2.12 (m, 1H), 2.10 - 1.97 (m, 5H), 1.91 (dd, *J*= 17.6, 13.8 Hz, 2H), 1.86 - 1.72 (m, 3H) | 732.4 7 | General formula SM-T-4 for PROTA C synthesis |
| 284 | SM-P-34 | 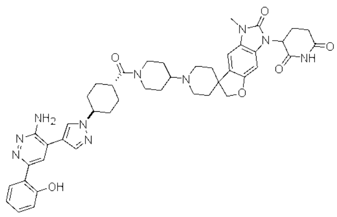 3-(1'-(1-(1r,4r)-4-(4-(3-amino-6-(2-hydroxyphenyl)pyridin-4-yl)-1h-pyrazol-1-yl)cyclohexane-1-carbonyl)piperidin-4-yl)-1-methyl-2-oxo-1,2-dihydro-3h,6h-spiro[benzofuro[5,6-d]imidazole-7,4'-piperidin]-3-yl)piperidine-2,6-dione | $^1$H NMR (400 MHz, Methanol-$d_4$) δ 8.37 (s, 1H), 8.30 (s, 1H), 8.11 (s, 1H), 7.78 - 7.72 (m, 1H), 7.43 - 7.37 (m, 1H), 7.02 (q, *J* = 7.3 Hz, 2H), 6.70 (s, 1H), 5.30 (dd, *J* = 12.5, 5.5 Hz, 1H), 4.55 (d, *J* = 4.7 Hz, 2H), 4.35 (d, *J*= 12.2 Hz, 2H), 3.84 - 3.53 (m, 4H), 3.42 (d, *J* = 8.2 Hz, 2H), 3.21 (s, 2H), 2.98 - 2.87 (m, 2H), 2.87 - 2.67 (m, 4H), 2.28 (d, *J*= 12.2 Hz, 4H), 2.17 (ddd, *J*= 23.7, 16.8, 10.3 Hz, 4H), 2.08 - 1.94 (m, 4H), 1.92 - 1.70 (m, 4H), 1.65 (t, *J*= 13.1 Hz, 2H) | 815.6 6 | General formula SM-T-4 for PROTA C synthesis |

(continued)

| Example | Compound | Structure and Name | $^1$H-NMR | LC-MS | Synthesis Method |
|---|---|---|---|---|---|
| 285 | SM-P-35 | 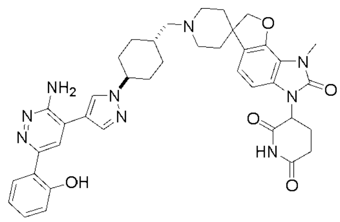 3-(1'-((1r,4r)-4-(4-(3-amino-6-(2-hydroxyphenyl)pyridazin -4-yl)-1H-pyrazol-1-yl)cyclohexyl) methyl)-1-methyl-2-oxo-1,2-dihydro-3h,7h-spiro[benzofuro[6,7-d] imidazole-6,4'-piperidin]-3-yl)piperidine-2,6-dione | $^1$H NMR (600 MHz, DMSO-$d_6$) δ 11.11 (s, 1H), 9.25 (s, 1H), 8.50 (d, *J* = 29.4 Hz, 1H), 8.25 (d, *J*= 7.2 Hz, 1H), 8.18 (d, *J*= 16.2 Hz, 1H), 7.83 (t, *J*= 7.6 Hz, 1H), 7.34 (t, *J* = 7.7 Hz, 1H), 7.24 (d, *J*= 30.0 Hz, 2H), 7.02 - 6.94 (m, 2H), 6.78 (d, *J* = 8.0 Hz, 1H), 6.71 (d, *J*= 7.7 Hz, 1H), 5.33 (td, *J*= 15.2, 14.1, 5.4 Hz, 1H), 4.67 - 4.60 (m, 2H), 4.26 (tt, *J* = 11.8, 3.9 Hz, 1H), 3.60 (d, *J* = 12.1 Hz, 2H), 3.45 (s, 1H), 3.20 - 3.15 (m, 1H), 3.13 - 3.02 (m, 3H), 2.90 (ddd, *J* = 16.2, 12.6, 7.4 Hz, 1H), 2.68 (td, *J*= 13.3, 7.4 Hz, 1H), 2.62 (dd, *J*= 17.7, 5.3 Hz, 1H), 2.27 - 2.14 (m, 4H), 2.00 (dd, J= 13.8, 7.7 Hz, 3H), 1.96 - 1.90 (m, 3H), 1.87 (td, *J* = 12.6, 3.4 Hz, 2H), 1.75 (qd, *J* = 10.4, 8.7, 4.2 Hz, 1H), 1.69 - 1.59 (m, 1H), 1.29 - 1.22 (m, 2H). | 718.5 7 | General formula SM-T-5 for PROTA C synthesis |
| 286 | SM-P-36 | 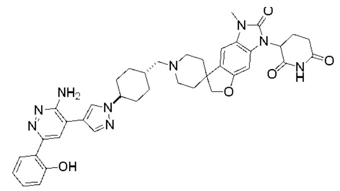 3-(1'-((1r,4r)-4-(4-(3-amino-6-(2-hydroxyphenyl)pyridazin -4-yl)-1h-pyrazol-1-yl)cyclohexyl) methyl)-1-methyl-2-oxo-1,2-dihydro-3h,6h-spiro[benzofuro[5,6-d] imidazole-7,4'-piperidin]-3-yl)piperidine-2,6-dione | $^1$H NMR (400 MHz, DMSO-$d_6$) δ 11.07 (d, *J* = 7.8 Hz, 1H), 9.09 (s, 1H), 8.50 (d, *J* = 20.5 Hz, 1H), 8.24 (d, *J*= 5.0 Hz, 1H), 8.19 (d, *J*= 11.0 Hz, 1H), 7.86 (d, *J* = 7.6 Hz, 1H), 7.36 - 7.27 (m, 1H), 7.11 (d, *J* = 36.6 Hz, 2H), 6.96 (dd, *J* = 11.8, 7.9 Hz, 2H), 6.83 (s, 1H), 6.75 (s, 1H), 5.32 (dd, *J* = 8.7, 4.7 Hz, 1H), 4.48 (d, *J* = 9.7 Hz, 2H), 4.31 - 4.22 (m, 1H), 3.33 (s, 3H), 3.07 (s, 3H), 2.91 - 2.81 (m, 2H), 2.73 (dd, *J*= 13.0, 4.8 Hz, 3H), 2.61 *(d, J*=16.5 Hz, 3H), 2.28 (s, 2H), 2.19 (d, *J* = 10.6 Hz, 2H), 2.04 - 1.95 (m, 4H), 1.90 (t, *J* = 11.3 Hz, 4H). | 718.5 7 | General formula SM-T-5 for PROTA C synthesis |

(continued)

| Example | Compound | Structure and Name | $^1$H-NMR | LC-MS | Synthesis Method |
|---|---|---|---|---|---|
| 287 | SM-P-37 | 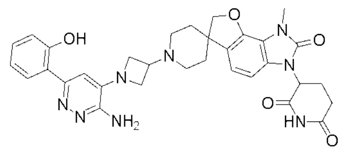 3-(1'-(1-(3-amino-6-(2-hydroxyphenyl)pyridazin -4-yl)azetidin-3-yl)-1-methyl-2-oxo-1,2-dihydro-3H,7H-spiro[benzofuro[6,7-d]imidazole-6,4'-piperidin]-3-yl) piperidine-2,6-dione | $^1$H NMR (600 MHz, Methanol-$d_4$) δ 7.55 (dd, *J*= 7.9, 1.6 Hz, 1H), 7.48 - 7.43 (m, 1H), 7.08 - 7.03 (m, 2H), 6.98 - 6.92 (m, 1H), 6.88 (s, 1H), 6.72 (d, *J* = 7.8 Hz, 1H), 5.31 (dd, *J*= 12.6, 5.3 Hz, 1H), 4.82 (s, 2H), 4.77 - 4.70 (m, 2H), 4.66 (s, 2H), 4.12 (s, 1H), 3.58 (s, 3H), 3.49 (s, 2H), 2.95 - 2.87 (m, 2H), 2.84 - 2.76 (m, 2H), 2.25 (s, 2H), 2.22 - 2.13 (m, 2H), 2.06 (d, *J* = 13.6 Hz, 2H) | 611.5 8 | General formula SM-T-6 for PROTA C synthesis |
| 288 | SM-P-38 | 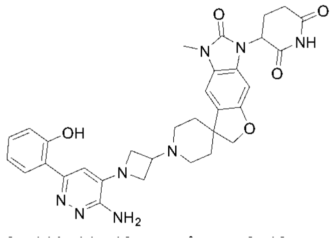 3-(1'-(1-(3-amino-6-(2-hydroxyphenyl)pyridazin -4-yl)azetidin-3-yl)-1-methyl-2-oxo-1,2-dihydro-3H,6H-spiro[benzofuro[5,6-d]imidazole-7,4'-piperidin]-3-yl) piperidine-2,6-dione | 1H NMR (600 MHz, Methanol-$d_4$) δ 7.56 (dd, *J* = 7.8, 1.6 Hz, 1H), 7.46 (ddd, *J* = 8.4, 7.4, 1.6 Hz, 1H), 7.06 (ddd, *J*= 12.2, 6.6, 1.5 Hz, 3H), 6.89 (s, 1H), 6.69 (s, 1H), 5.30 (dd, *J*= 12.7, 5.4 Hz, 1H), 4.82 (d, *J*= 8.7 Hz, 2H), 4.74 (s, 2H), 4.52 (s, 2H), 4.12 (s, 1H), 3.49 (d, *J*= 12.0 Hz, 2H), 3.43 (s, 3H), 2.98 - 2.84 (m, 3H), 2.84 - 2.77 (m, 2H), 2.29 (t, *J* = 13.6 Hz, 2H), 2.19 - 2.12 (m, 1H), 2.04 (d, J = 14.2 Hz, 2H). | 611.5 8 | General formula SM-T-6 for PROTA C synthesis |

Comparative Example 1

SMD-3040

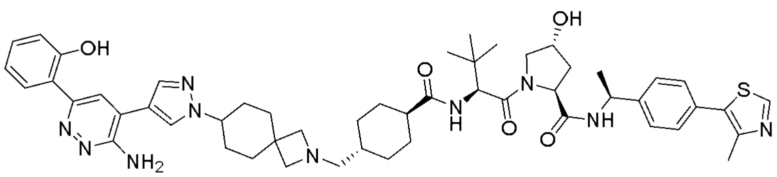

(2S,4R)-1-((S)-2-((1R,4S)-4-((7-(4-(3-amino-6-(2-hydroxyphenyl)pyridazin-4-yl)-1H-pyrazol-1-yl)-2-azaspiro[3.5]nonan-2-yl)methyl)cyclohexane-1-carboxamido)-3,3-dimethylbutanoyl)-4-hydroxy-N-((S)-1-(4-(4-methylthiazol-5-yl)phenyl)ethyl)pyrrolidine-2-carboxamide

The control compound was synthesized according to Reference (Yang L, Tu W, Huang L, Miao B, Kaneshige A, Jiang W, Leng L, Wang M, Wen B, Sun D, Wang S. Discovery of SMD-3040 as a Potent and Selective SMARCA2 PROTAC Degrader with Strong in vivo Antitumor Activity. J Med Chem. 2023 Aug 10;66(15):10761-10781. doi: 10.1021/acs.jmedchem.3c00953.).

$^1$H NMR (600 MHz, MeOD-$d_4$) δ 8.93 (d, *J* = 3.9 Hz, 1H), 8.35 (s, 1H), 8.32 (s, 1H), 8.05 (s, 1H), 7.70 - 7.67 (m, 1H), 7.47 - 7.34 (m, 5H), 7.04 - 6.98 (m, 2H), 5.03 - 4.97 (m, 1H), 4.64 - 4.52 (m, 2H), 4.46 - 4.41 (m, 1H), 4.34 - 4.23 (m, 2H), 4.12

- 4.04 (m, 1H), 4.00 - 3.93 (m, 1H), 3.92 - 3.82 (m, 2H), 3.76 (dd, *J*= 7.2, 4.0 Hz, 1H), 3.15 (d, *J*= 7.0 Hz, 2H), 2.41 - 2.26 (m, 2H), 2.24 - 2.08 (m, 4H), 2.04 - 1.73 (m, 10H), 1.73 - 1.44 (m, 8H), 1.20 - 1.06 (m, 2H), 1.04 (s, 9H).

LCMS (ESI) : $[M+H]^+$ = 943.75.

Test Example 1

Binding activity of the E3 ligase inhibitor disclosed in the present invention to E3 ubiquitin ligase CRBN

The binding activity of the E3 ligase inhibitor disclosed in the present invention and the positive control samples lenalidomide and pomalidomide with the E3 ubiquitin ligase CRBN is determined by the competitive binding of the E3 ligase inhibitor disclosed in the present invention, lenalidomide and pomalidomide with thalidomide labeled with XL665 to the CRBN protein, thereby preventing the occurrence of fluorescence resonance energy transfer (FRET), thereby determining the binding affinity of the compound to the CRBN protein.

CRBN binding activity test kit (CEREBLON BINDING KITS (PE, 64BD CRBN PEG).

The detection is based on homogeneous time-resolved fluorescence (HTRF) technology. When the donor and acceptor are close to each other, the donor can transfer energy to the acceptor, causing it to be excited and emit light at 665nm.

This kit uses a europium-labeled GST antibody (GST Eu Cryptate Antibody) as the donor and XL665-labeled thalidomide (Thalidomide-Red reagent) as the receptor. The donor binds to the GST-tagged CRBN protein. The E3 ligase inhibitors disclosed herein, as well as lenalidomide and pomalidomide, compete with thalidomide for binding to the CRBN protein. The binding activity of the compound is determined by the fluorescence emitted by the receptor at 665 nm. The stronger the binding affinity of the compounds, the weaker the signal.

The donor and receptor were each diluted 50-fold using the buffer provided in the kit (PROTAC binding buffer 1). The CRBN protein was diluted 45-fold. An 8mM solution of the compound disclosed herein was diluted 10-fold using the diluent provided in the kit (1X diluent), followed by 7 successive 5-fold serial dilutions of the resulting solution.

5 μl of compound, 5 μl of diluted CRBN protein, and 10 μl of equal volumes of donorreceptor mixture were sequentially added to the wells of a white 384-well plate (PE, Part number: 6008280) and incubated at room temperature for 3 hours.

The ratio of the acceptor and donor emission signals was calculated for each individual well.

$$\text{Ratio}=665\text{nm signal value}/620\text{nm signal value}*10^4$$

$$\text{Calculate the coefficient of variation (CV)} = \text{standard deviation (SD)} / \text{Ratio mean} * 100$$

$IC_{50}$ values was calculated based on compound concentration, Ratio, and coefficient of variation using GraphPad Prism.

Table 1 shows the $IC_{50}$ values of the E3 ligase inhibitors disclosed in the present invention and lenalidomide and pomalidomide.

| Compounds | IC50 (μM) | Compounds | IC50 (μM) |
|---|---|---|---|
| Lenalidomide | 3.1 | C2 | 90.47 |
| Pomalidomid | 1.3 | C3 | 5.53 |
| A1 | 15.47 | C4 | 10.07 |
| A2 | 11.89 | C5 | 5.45 |
| A3 | 52.80 | C6 | 3.38 |
| A4 | 23.48 | C7 | 10.24 |
| A5 | 20.76 | C8 | 16.43 |
| A6 | 35.27 | C9 | 12.77 |
| A7 | 27.35 | C10 | 2.06 |
| A8 | 24.55 | C11 | 21.06 |
| A9 | 29.10 | C12 | 5.46 |

(continued)

| Compounds | IC50 (μM) | Compounds | IC50 (μM) |
|---|---|---|---|
| A10 | 24.51 | C13 | 8.39 |
| A11 | 14.40 | C14 | 1.85 |
| A12 | 20.30 | C15 | 0.72 |
| B1 | 40.27 | C16 | 2.16 |
| B2 | 60.23 | D1 | 0.03 |
| B3 | 45.10 | D2 | 0.028 |
| B4 | 10.93 | D3 | 0.21 |
| B5 | 59.96 | D4 | 2.30 |
| B6 | 49.11 | D5 | 0.66 |
| B7 | 5.32 | D6 | 0.55 |
| B8 | 26.98 | D7 | 0.4 |
| B9 | 13.02 | D8 | 0.56 |
| B10 | 22.44 | D9 | 0.65 |
| B11 | 54.82 | | |
| B12 | 37.34 | | |
| C1 | 1.90 | | |
| Conclusion: The E3 ligase inhibitors disclosed in the present invention can bind well to the CRBN protein, and the binding ability of some E3 ligase inhibitors to CRBN is comparable to or even better than that of the positive control samples lenalidomide and pomalidomide. | | | |

Test Example 2 Inhibitory effect of PROTAC compounds provided by the present invention on cell proliferation in T-47D cells

The complete culture medium required for T-47D cells is 1640 (ATCC, Product number: A10491-01) basal culture medium containing 10% fetal bovine serum. T-47D cells were seeded in 96-well plates at a density of 3,000 cells/well, and different concentrations of PROTAC compounds 1-55 provided in the examples of the present invention and positive control sample ARV-471 (source: Inokai) were prepared to treat the cells and incubated at 37 °C, 5% $CO_2$ cell culture incubator for 6 days. According to the manufacturer's instructions, cell viability was detected using a CCK-8 kit (Beyotime, Product number: C0040). The $IC_{50}$ of each compound after treating for 6 days was calculated using GraphPadPrism software based on the inhibition rate. The results are shown in Table 2.

Table 2 Inhibition $IC_{50}$ value of T-47D cell proliferation by PROTAC compounds provided by the present invention and positive control sample

| Compounds | $IC_{50}$ (T-47D) [nM] | Compounds | $IC_{50}$ (T-47D) [nM] |
|---|---|---|---|
| ARV-471 | 0.0017 | ER-P-56 | 42.17 |
| ER-P-1 | 3.74 | ER-P-57 | 3.19 |
| ER-P-2 | 2.02 | ER-P-58 | 2.19 |
| ER-P-3 | 3.02 | ER-P-59 | 0.96 |
| ER-P-4 | 3.63 | ER-P-60 | 1.41 |
| ER-P-5 | 0.96 | ER-P-61 | 1.12 |
| ER-P-6 | 0.86 | ER-P-62 | 2.64 |
| ER-P-7 | 1.74 | ER-P-63 | 1.15 |
| ER-P-8 | - | ER-P-64 | 1.5 |

(continued)

| Compounds | $IC_{50}$ (T-47D) [nM] | Compounds | $IC_{50}$ (T-47D) [nM] |
|---|---|---|---|
| ER-P-9 | 1.96 | ER-P-65 | 7.34 |
| ER-P-10 | 1.63 | ER-P-66 | 13.98 |
| ER-P-11 | 20.83 | ER-P-67 | 92.51 |
| ER-P-12 | 2.4 | ER-P-68 | 1.18 |
| ER-P-13 | 0.47 | ER-P-69 | 0.28 |
| ER-P-14 | 0.81 | ER-P-70 | 1.71 |
| ER-P-15 | 0.65 | ER-P-71 | 8.54 |
| ER-P-16 | 0.62 | ER-P-72 | 1.34 |
| ER-P-17 | 20.89 | ER-P-73 | 49.43 |
| ER-P-18 | 13.73 | ER-P-74 | 1.22 |
| ER-P-19 | 10.32 | ER-P-75 | 1.16 |
| ER-P-20 | 5.02 | ER-P-76 | 5.75 |
| ER-P-21 | 1.30 | ER-P-77 | 1.87 |
| ER-P-22 | - | ER-P-78 | 5.8 |
| ER-P-23 | 1.61 | ER-P-79 | 8.46 |
| ER-P-24 | 19.16 | ER-P-80 | 0.9 |
| ER-P-25 | 0.84 | ER-P-81 | 2.57 |
| ER-P-26 | 0.51 | ER-P-82 | 28.08 |
| ER-P-27 | 1.34 | ER-P-83 | 138 |
| ER-P-28 | 1.56 | ER-P-84 | 17.74 |
| ER-P-29 | 0.92 | ER-P-85 | 70.87 |
| ER-P-30 | 2.42 | ER-P-86 | 68.84 |
| ER-P-31 | 2.71 | ER-P-87 | 52.97 |
| ER-P-32 | 19.46 | ER-P-88 | 9.59 |
| ER-P-33 | 15.48 | ER-P-89 | 104 |
| ER-P-34 | 26.93 | ER-P-90 | 171 |
| ER-P-35 | 22.71 | ER-P-91 | 150 |
| ER-P-36 | 25.07 | ER-P-92 | 4.56 |
| ER-P-37 | 18.89 | ER-P-47 | 1.47 |
| ER-P-38 | 18.57 | ER-P-48 | 115 |
| ER-P-39 | 14.24 | ER-P-49 | 1.17 |
| ER-P-40 | 28.40 | ER-P-50 | 45.57 |
| ER-P-41 | 37.27 | ER-P-51 | 74.28 |
| ER-P-42 | 10.44 | ER-P-52 | 248 |
| ER-P-43 | 95.09 | ER-P-53 | 252 |
| ER-P-44 | 1.26 | ER-P-54 | 0.15 |
| ER-P-45 | 1.48 | ER-P-55 | 0.18 |

(continued)

| Compounds | $IC_{50}$ (T-47D) [nM] | Compounds | $IC_{50}$ (T-47D) [nM] |
|---|---|---|---|
| ER-P-46 | 1.17 | | |
| Conclusion: The PROTAC compounds provided by the present invention can effectively inhibit the proliferation of T-47D cells, and the inhibitory effects of some compounds are comparable to or even better than those of the positive control. | | | |

**Test Example 3 Inhibitory effect of PROTAC compounds provided by the present invention on cell proliferation in MCF-7 cells**

The complete culture medium required for MCF-7 cells (source ATCC) is DMEM (gibco, Product number: 11995-065) basal culture medium containing 10% fetal bovine serum. MCF-7 cells were seeded in 96-well plates at a density of 800 cells/well, and different concentrations of PROTAC compounds 1-55 provided in the examples of the present invention and positive control sample ARV-471 (source: Inokai) were prepared to treat the cells and incubated at 37 °C, 5% $CO_2$ cell culture incubator for 6 days. According to the manufacturer's instructions, cell viability was detected using a CCK-8 kit (Beyotime, Product number: C0040). The $IC_{50}$ of each PROTAC compound after treating for 6 days was calculated using GraphPadPrism software based on the inhibition rate. The results are shown in Table 3.

Table 3 Inhibition $IC_{50}$ value of MCF-7 cell proliferation by PROTAC compounds of the present invention and positive control sample

| Compounds | IC50 (MCF-7) [nM] | Compounds | IC50 (MCF-7) [nM] |
|---|---|---|---|
| ARV-471 | 0.0017 | ER-P-56 | 2465 |
| ER-P-1 | <0.256 | ER-P-57 | 12.66 |
| ER-P-2 | 62.62 | ER-P-58 | 12.84 |
| ER-P-3 | <0.256 | ER-P-59 | 0.875 |
| ER-P-4 | 92010 | ER-P-60 | 0.362 |
| ER-P-5 | <0.256 | ER-P-61 | 28.83 |
| ER-P-6 | <0.256 | ER-P-62 | 183 |
| ER-P-7 | 603 | ER-P-63 | 3749 |
| ER-P-8 | - | ER-P-64 | 231 |
| ER-P-9 | 7.44 | ER-P-65 | 261 |
| ER-P-10 | 8.53 | ER-P-66 | 4099 |
| ER-P-11 | 3543 | ER-P-67 | 2986 |
| ER-P-12 | 25.21 | ER-P-68 | 8.27 |
| ER-P-13 | 18.89 | ER-P-69 | 1.43 |
| ER-P-14 | 34.56 | ER-P-70 | 11.69 |
| ER-P-15 | 2981 | ER-P-71 | 2015 |
| ER-P-16 | 885 | ER-P-72 | 14309 |
| ER-P-17 | 4560 | ER-P-73 | 3271 |
| ER-P-18 | 7239 | ER-P-74 | 2103 |
| ER-P-19 | 4456 | ER-P-75 | 3004 |
| ER-P-20 | 2231 | ER-P-76 | >100000 |
| ER-P-21 | 15.07 | ER-P-77 | 84.66 |
| ER-P-22 | - | ER-P-78 | 737 |
| ER-P-23 | <0.256 | ER-P-79 | 63.41 |

(continued)

| Compounds | IC50 (MCF-7) [nM] | Compounds | IC50 (MCF-7) [nM] |
|---|---|---|---|
| ER-P-24 | 1068 | ER-P-80 | - |
| ER-P-25 | 1.26 | ER-P-81 | 1.60 |
| ER-P-26 | 125 | ER-P-82 | 4391 |
| ER-P-27 | <0.256 | ER-P-83 | 4330 |
| ER-P-28 | <0.256 | ER-P-84 | 8330 |
| ER-P-29 | 140 | ER-P-85 | 956 |
| ER-P-30 | 4959 | ER-P-86 | 6665 |
| ER-P-31 | 54.79 | ER-P-87 | 4002 |
| ER-P-32 | 705 | ER-P-88 | 1026 |
| ER-P-33 | 2333 | ER-P-89 | 2740 |
| ER-P-34 | 2117 | ER-P-90 | 3648 |
| ER-P-35 | 876 | ER-P-91 | 7943 |
| ER-P-36 | 2278 | ER-P-92 | - |
| ER-P-37 | 1405 | ER-P-47 | 8426 |
| ER-P-38 | 505 | ER-P-48 | 7391 |
| ER-P-39 | 526 | ER-P-49 | 3727 |
| ER-P-40 | 6210 | ER-P-50 | 3271 |
| ER-P-41 | 377 | ER-P-51 | 1256 |
| ER-P-42 | 4502 | ER-P-52 | 854 |
| ER-P-43 | 1067 | ER-P-53 | 1313 |
| ER-P-44 | 805 | ER-P-54 | 1023 |
| ER-P-45 | 1630 | ER-P-55 | 113 |
| ER-P-46 | 731 | | |
| Conclusion: The PROTAC compounds of the present invention can effectively inhibit the proliferation of MCF-7 cells. The inhibitory effects of some PROTAC compounds on MCF-7 cells are comparable to or even better than those of the positive control. | | | |

**Test Example 4 Effect of PROTAC Compounds Disclosed in the Present Invention on ERα Degradation in MCF-7 Cells**

MCF-7 (ATCC) cells were cultured in DMEM medium (11995-065, Gibco) containing 10% FBS (10099141C, Gibco) at 37°C and 5% $CO_2$ until they entered the logarithmic growth phase. MCF-7 cells were plated at a density of $3\times10^5$ cells/well in a 6-well plate and incubated at 37°C, 5% $CO_2$ for 24 hours. After cell attachment, 1μL of compound in dimethyl sulfoxide (final concentration 0.15-1000nM) was added and incubated for another 4 hours at 37°C, 5% $CO_2$ incubator. After 4 hours, the culture medium was discarded, and each well was washed once with 1 mL of pre-chilled 1× DPBS. The cells were then lysed with 50 μL of RIPA lysis buffer (P0013B, Beyotime) and 1× protease inhibitor cocktail (P1005, Beyotime). After incubation on ice for 20 minutes, the cells were centrifuged at 14,000 g at 4°C for 30 minutes. The supernatant was carefully collected and analyzed for ERα protein levels by immunoblotting.

Western blotting: The total protein concentration in the collected cell lysate supernatant was determined using the BCA protein concentration assay kit (enhanced) (P0009, Beyotime). Based on the total protein concentration determined by BCA, the protein concentration was adjusted to 1 μg/μL using RIPA lysis buffer and 5× SDS-PAGE protein loading buffer (MB01015, GenScript). The protein was denatured in a 95°C water bath for 5 minutes. After denaturation, the condensed water on the tube wall was collected by centrifugation and used as the loading sample. 20μL of the loading sample (20 μg of total protein) was added to the sample well of a 12% precast gel (M00669, GenScript). Electrophoresis was performed at 100 V for 20 minutes, followed by switching to 150 V for 40 minutes. After electrophoresis, the proteins from the SDS-PAGE gel were transferred to a PVDF membrane at a constant voltage of 100 V for 60 minutes. After transfer, the PVDF membrane corresponding to the ERα protein and internal reference protein was cut and incubated in 1× QμickBlock blocking buffer (P0252, Beyotime) for 30 minutes at room temperature on a shaker. After blocking, the ERα primary antibody (1:1000, 8644S, CST) was added and incubated overnight at 4°C on a shaker. The PVDF membrane was washed three times with 1× TBST buffer for 5 minutes each. Rabbit IgG (H+L) secondary antibody (1:7500, SA5-35571, Invitrogen) was added and incubated for 1 hour at room temperature on a shaker. The membrane was then washed again three times with 1× TBST buffer for 5 minutes each. Finally, the membrane was imaged using a dual-color infrared laser imaging system (Odyssey DLx, LICOR). Protein maps were analyzed for grayscale value using Image Studio Lite software. The grayscale correction value for each sample was calculated using the formula: grayscale correction value = grayscale value of the target protein / grayscale value of the corresponding internal reference. Then the grayscale correction values of the compound group and the control group were compared to calculate the degradation rate of the compound.

Table 4 Degradation rate of ERα by PROTAC compounds of the present invention and positive control in MCF-7 cells (%)

| Degradation rate of ERα in MCF-7 cells (%) | | | | | |
|---|---|---|---|---|---|
| Compounds | 10nM | 1nM | Compounds | 10nM | 1nM |
| ARV-471 | 76 | 38 | ER-P-56 | 16 | -4 |
| ER-P-1 | 44 | 19 | ER-P-57 | 62 | 3 |
| ER-P-2 | 48 | 7 | ER-P-58 | 69 | 42 |
| ER-P-3 | 69 | 25 | ER-P-59 | 18 | 14 |
| ER-P-4 | 20 | 9 | ER-P-60 | 36 | 14 |
| ER-P-5 | 57 | 25 | ER-P-61 | 18 | 9 |
| ER-P-6 | 71 | 55 | ER-P-62 | 39 | 14 |
| ER-P-7 | 62 | 22 | ER-P-63 | 56 | 53 |
| ER-P-8 | | | ER-P-64 | 57 | 46 |
| ER-P-9 | 71 | 30 | ER-P-65 | 62 | 21 |
| ER-P-10 | 57 | 24 | ER-P-66 | 30 | 6 |
| ER-P-11 | -33 | -18 | ER-P-67 | 13 | 28 |
| ER-P-12 | -11 | -9 | ER-P-34 | 17 | 5 |
| ER-P-13 | 85 | 72 | ER-P-35 | -3 | 6 |
| ER-P-14 | 63 | 69 | ER-P-36 | -11 | 7 |
| ER-P-15 | 26 | 22 | ER-P-37 | -9 | -6 |
| ER-P-16 | 62 | 43 | ER-P-38 | -14 | 12 |
| ER-P-17 | 33 | 37 | ER-P-39 | -16 | -13 |
| ER-P-18 | 18 | 33 | ER-P-40 | -53 | -10 |
| ER-P-19 | 7 | -8 | ER-P-41 | 15 | 13 |
| ER-P-20 | 30 | 5 | ER-P-42 | -3 | -20 |
| ER-P-21 | 69 | 50 | ER-P-43 | -19 | -13 |
| ER-P-22 | - | | ER-P-44 | 39 | 10 |
| ER-P-23 | 48 | 26 | ER-P-45 | -24 | -15 |
| ER-P-24 | -39 | -68 | ER-P-46 | 36 | 6 |
| ER-P-25 | 53 | 49 | ER-P-47 | 26 | 21 |
| ER-P-26 | 40 | 43 | ER-P-48 | -1 | 13 |
| ER-P-27 | 62 | 26 | ER-P-49 | 23 | -12 |

(continued)

| Degradation rate of ERα in MCF-7 cells (%) | | | | | |
|---|---|---|---|---|---|
| Compounds | 10nM | 1nM | Compounds | 10nM | 1nM |
| ER-P-28 | 63 | 38 | ER-P-50 | 9 | 1 |
| ER-P-29 | 25 | 54 | ER-P-51 | 1 | 5 |
| ER-P-30 | 14 | 42 | ER-P-52 | 7 | -2 |
| ER-P-31 | 71 | 29 | ER-P-53 | 21 | 10 |
| ER-P-32 | 13 | 12 | ER-P-54 | 76 | 50 |
| ER-P-33 | -9 | -5 | ER-P-55 | 69 | 35 |
| Conclusion: The PROTAC compounds of the present invention can effectively inhibit the proliferation of MCF-7 cells. The inhibitory effects of some PROTAC compounds on MCF-7 cells are comparable to or even better than those of the positive control. | | | | | |

**Test Example 5 Degradation of ERα by the PROTAC compounds disclosed in the present invention in T-47D cells**

T-47D (ATCC) cells were cultured in PRMI-1640 medium (30-2001, ATCC) containing 10% FBS (10099141C, Gibco) at 37°C and 5% $CO_2$ until they entered the logarithmic growth phase. T-47D cells were seeded at a density of $3\times10^5$ cells/well in a 6-well plate and incubated at 37°C, 5% $CO_2$ for 24 hours. After cell attachment, 1 μL of compound dimethyl sulfoxide solution (final concentration 0.15-1000 nM) was added and incubated for another 4 hours at 37°C, 5% $CO_2$ incubator. After 4 hours, the culture medium was discarded, and each well was washed once with 1 mL of pre-chilled 1× DPBS. The cells were then lysed with 50 μL of RIPA lysis buffer (P0013B, Beyotime) and 1× protease inhibitor cocktail (P1005, Beyotime). After incubation on ice for 20 minutes, the cells were centrifuged at 14,000 g at 4°C for 30 minutes. The supernatant was carefully collected and analyzed for ERα protein levels by immunoblotting.

Table 5 Degradation rate of ERα by PROTAC compounds of the present invention and positive control sample in T-47D cells (%)

| Degradation rate of ERα in T-47D cells (%) | | | | | |
|---|---|---|---|---|---|
| Compounds | 10nM | 1nM | Compounds | 10nM | 1nM |
| ARV-471 | 85 | 61 | ER-P-56 | 24 | 22 |
| ER-P-1 | 81 | 52 | ER-P-57 | 68 | 12 |
| ER-P-2 | 54 | 26 | ER-P-58 | 71 | 35 |
| ER-P-3 | 73 | 28 | ER-P-59 | 63 | 40 |
| ER-P-4 | 54 | 21 | ER-P-60 | 51 | 21 |
| ER-P-5 | 79 | 39 | ER-P-61 | 43 | 22 |
| ER-P-6 | 83 | 61 | ER-P-62 | 52 | 13 |
| ER-P-7 | 77 | 44 | ER-P-63 | 65 | 56 |
| ER-P-8 | - | - | ER-P-64 | 80 | 56 |
| ER-P-9 | 83 | 46 | ER-P-65 | 77 | 20 |
| ER-P-10 | 77 | 51 | ER-P-66 | 49 | 18 |
| ER-P-11 | 12 | 12 | ER-P-67 | 20 | 6 |
| ER-P-12 | 38 | 34 | ER-P-34 | 24 | 15 |
| ER-P-13 | 74 | 79 | ER-P-35 | 21 | 21 |
| ER-P-14 | 61 | 64 | ER-P-36 | 1 | 0 |
| ER-P-15 | 31 | 44 | ER-P-37 | 4 | 6 |
| ER-P-16 | 73 | 69 | ER-P-38 | 3 | 1 |

(continued)

| Degradation rate of ERα in T-47D cells (%) | | | | | |
|---|---|---|---|---|---|
| Compounds | 10nM | 1nM | Compounds | 10nM | 1nM |
| ER-P-17 | 30 | 22 | ER-P-39 | -3 | -7 |
| ER-P-18 | 23 | 20 | ER-P-40 | 34 | 22 |
| ER-P-19 | 15 | 6 | ER-P-41 | 21 | 17 |
| ER-P-20 | 66 | 38 | ER-P-42 | -28 | -27 |
| ER-P-21 | 65 | 42 | ER-P-43 | -1 | 10 |
| ER-P-22 | | - | ER-P-44 | 30 | -29 |
| ER-P-23 | 67 | 40 | ER-P-45 | -3 | -5 |
| ER-P-24 | -49 | -16 | ER-P-46 | 55 | 35 |
| ER-P-25 | 71 | 77 | ER-P-47 | 25 | -14 |
| ER-P-26 | 70 | 58 | ER-P-48 | 0 | 6 |
| ER-P-27 | 53 | 20 | ER-P-49 | 30 | -11 |
| ER-P-28 | 59 | 41 | ER-P-50 | 18 | 4 |
| ER-P-29 | 41 | 53 | ER-P-51 | 12 | 14 |
| ER-P-30 | 17 | 47 | ER-P-52 | 6 | 6 |
| ER-P-31 | 79 | 26 | ER-P-53 | 33 | 18 |
| ER-P-32 | 15 | -2 | ER-P-54 | 81 | 67 |
| ER-P-33 | 32 | -16 | ER-P-55 | 87 | 62 |
| **Conclusion: The compounds disclosed in the present invention can effectively degrade ER protein, and the ability of most compounds to degrade ER protein is comparable to or even better than that of the positive control.** | | | | | |

**Test Example 6**

The degradation activity of the compounds disclosed in the present invention on AR protein in LNCaP cells (cell source: ATCC)

AR-positive human prostate cancer cells LNCaP FGC cultured in RPMI 1640 (ATCC catalog number 30-2001) containing 10% FBS (Hyclone catalog number SH30406.05) was inoculated into a six-well microplate (Thermo catalog number 140675) at a concentration of $1.5\times10^5$ cells/ well. After the cells were cultured overnight in a carbon dioxide incubator (ESCO), the prepared compound of the present invention and the positive control sample ARV-766 (source: Anaiji) solution of different concentrations were added at a volume of 1 μL/well to make the final concentrations of the compound and the positive control sample reach 100nM and 10nM, respectively. At the same time, the corresponding solvent control was set and cultured for 8 hours. After 8 hours of culture, the culture medium was removed, the cells were washed with PBS, and RIPA lysis buffer containing 1% Protease Inhibitor Cocktail was added. After lysis and centrifugation, the total protein extract was obtained, and the protein concentration in the extract was detected by BCA method. Protein electrophoresis was performed using SDS-PAGE, and then the protein was transferred to a PVDF (Millipore Sigma catalog number PSRP010R5) membrane at a constant voltage of 100V for 60 minutes. The PVDF membrane was placed in a 5% milk powder 1 ×TBST solution and blocked at room temperature for 1 hour. The primary antibody solution (Anti-Androgen receptor, Cell Signaling Technology catalog number: 5153S; 1:2000 dilution) was prepared and was incubated at 4°C overnight. The primary antibody solution was discarded and the PVDF membrane was washed with 1×TBST for 5 minutes each time, for three times. The secondary antibody solution (Thermo catalog number SA5-35571; 1:7500 dilution) was prepared and was incubated at room temperature for 1 hour. The secondary antibody solution was discarded and the PVDF membrane was washed with 1 ×TBST for 5 minutes each time, for three times. A dual-color infrared laser imaging system (Licor catalog number Odyssey CLX) was used for Western membrane imaging. The Image Studio software was used to analyze the grayscale of the bands. GAPDH protein bands were also detected for each sample as the reference. The AR protein degradation rate of the compound and the positive control sample was calculated based on the grayscale of the protein bands.

Table 6 Degradation rate of AR protein in LNCaP cells by the compounds disclosed in the present invention

| Compounds | 100 nM (%) | 10 nM (%) | Compounds | 100 nM (%) | 10 nM (%) |
|---|---|---|---|---|---|
| ARV-766 | 78% | 49% | A-P-37 | 63% | 10% |
| A-P-1 | 48% | 66% | A-P-38 | 0% | -2% |
| A-P-2 | 43% | -42% | A-P-39 | 39% | 22% |
| A-P-3 | 65% | 64% | A-P-40 | 46% | 26% |
| A-P-4 | 83% | 74% | A-P-41 | -31% | -37% |
| A-P-5 | 62% | 43% | A-P-42 | 77% | 44% |
| A-P-6 | 60% | 58% | A-P-43 | 35% | 8% |
| A-P-7 | 75% | 85% | A-P-44 | 9% | 20% |
| A-P-8 | 63% | -4% | A-P-45 | 85% | 52% |
| A-P-10 | 5% | 47% | A-P-46 | 15% | 4% |
| A-P-11 | 32% | 76% | A-P-48 | 63% | 45% |
| A-P-12 | 9% | 56% | A-P-49 | 62% | 48% |
| A-P-13 | 35% | 32% | A-P-50 | 91% | 73% |
| A-P-14 | 32% | 36% | A-P-51 | 12% | -23% |
| A-P-15 | 19% | 31% | A-P-52 | 23% | -56% |
| A-P-16 | 29% | 49% | A-P-53 | -3% | -11% |
| A-P-17 | -18% | 1% | A-P-54 | 74% | 40% |
| A-P-18 | 11% | 43% | A-P-55 | 15% | -15% |
| A-P-19 | 15% | 32% | A-P-57 | 76% | 39% |
| A-P-20 | 49% | 45% | A-P-58 | 87% | 63% |
| A-P-21 | 55% | 52% | A-P-61 | 79% | 24% |
| A-P-22 | 25% | 48% | A-P-62 | 72% | 47% |
| A-P-23 | 14% | 37% | A-P-63 | 68% | 27% |
| A-P-24 | 29% | 49% | A-P-67 | 85% | 55% |
| A-P-25 | 31% | 58% | A-P-68 | 84% | 51% |
| A-P-26 | 65% | 48% | A-P-69 | 79% | 52% |
| A-P-27 | 43% | 43% | A-P-77 | 62% | 20% |
| A-P-28 | 32% | 64% | A-P-78 | 14% | -2% |
| A-P-29 | -27% | 6% | A-P-80 | 73% | 22% |
| A-P-30 | 78% | 41% | A-P-81 | 30% | -15% |
| A-P-31 | 71% | 47% | A-P-82 | 83% | 55% |
| A-P-32 | 46% | 12% | A-P-83 | 65% | 41% |
| A-P-33 | 85% | 84% | A-P-84 | 33% | -6% |
| A-P-34 | 85% | 72% | A-P-85 | 40% | 7% |
| A-P-35 | 70% | 61% | A-P-86 | 39% | 37% |
| A-P-36 | 70% | 52% | A-P-100 | 48% | 1% |
| **Conclusion: The compounds disclosed in the present invention can effectively degrade AR protein, and the ability of most compounds to degrade AR protein is comparable to or even better than that of the positive control.** | | | | | |

**Test Example** 7

The degradation activity of the compounds disclosed in the present invention on AR protein in VCaP cells (cell source: ATCC)

The AR-positive human prostate cancer cells VCaP cultured in DMEM (GiBco catalog number 11995-065) containing 10% FBS (Hyclone catalog number SH30406.05) was inoculated in a six-well microplate (Thermo catalog number 140675) at a concentration of $3\times10^5$ cells/ well. After being cultured overnight in a carbon dioxide incubator (ESCO), the prepared compound of the present invention and the positive control sample ARV-766 (Annaiji) solution of different concentrations were added at a volume of 1 $\mu$L/well to make the final concentrations of the compound and the positive control sample reach 5nM and 0.5nM, respectively. At the same time, the corresponding solvent control was set and cultured for 8h. After 8h of culture, the culture medium was removed, and the cells were washed with PBS. RIPA lysis buffer containing 1% Protease Inhibitor Cocktail was added. After lysis and centrifugation, a total protein extract was obtained, and the protein concentration in the extract was detected by BCA method. Protein electrophoresis was performed using SDS-PAGE, and then the protein was transferred to a PVDF (Millipore Sigma catalog number PSRP010R5) membrane at a constant voltage of 100V for 60min. The PVDF membrane was placed in a 5% milk powder 1×TBST solution and blocked at room temperature for 1h. The primary antibody solution (Anti-Androgen receptor, Cell Signaling Technology catalog number: 5153S; 1:2000 dilution) was prepared and was incubated at 4°C overnight. The primary antibody solution was discarded and the PVDF membrane was washed with 1×TBST for 5 minutes each time, for three times. The secondary antibody solution (Thermo catalog number SA5-35571; 1:7500 dilution) was prepared and was incubated at room temperature for 1 hour. The secondary antibody solution was discarded and the PVDF membrane was washed with 1×TBST for 5 minutes each time, for three times. A dual-color infrared laser imaging system (Licor catalog number Odyssey CLX) was used for Western membrane imaging. Image Studio software was used to analyze the grayscale of the bands. GAPDH protein bands were also detected for each sample as the reference. The AR protein degradation rate of the compound and the positive control sample was calculated based on the grayscale of the protein bands.

The degradation rates of AR protein in VCaP cells by the compounds disclosed in the present invention are shown in Table 4 below.

Table 7 Degradation rate of AR protein in VCaP cells by the compounds disclosed in the present invention

| Compounds | 5 nM | 0.5 nM | Compounds | 5 nM | 0.5 nM |
|---|---|---|---|---|---|
| ARV-766 | 80% | 9% | A-P-32 | 59% | 14% |
| A-P-1 | 82% | 69% | A-P-33 | 84% | 56% |
| A-P-2 | 83% | 50% | A-P-34 | 47% | 0% |
| A-P-3 | 87% | 53% | A-P-35 | 18% | 9% |
| A-P-4 | 78% | 50% | A-P-36 | 45% | 9% |
| A-P-5 | 73% | 38% | A-P-37 | 49% | 25% |
| A-P-7 | 89% | 79% | A-P-38 | -5% | -21% |
| A-P-10 | 78% | 75% | A-P-39 | 9% | -6% |
| A-P-12 | 88% | 88% | A-P-40 | 10% | -8% |
| A-P-13 | 77% | 44% | A-P-41 | 18% | 13% |
| A-P-14 | 82% | 63% | A-P-42 | -4% | -40% |
| A-P-15 | 79% | 76% | A-P-43 | 32% | 33% |
| A-P-16 | 83% | 66% | A-P-45 | 77% | 44% |
| A-P-17 | 75% | 81% | A-P-48 | -1% | -23% |
| A-P-18 | 73% | 76% | A-P-49 | 45% | -15% |
| A-P-19 | 83% | 82% | A-P-50 | 87% | 84% |
| A-P-20 | 77% | 62% | A-P-52 | 14% | -1% |
| A-P-21 | 71% | 57% | A-P-57 | -21% | -31% |
| A-P-22 | 69% | 84% | A-P-58 | 37% | 12% |

(continued)

| Compounds | 5 nM | 0.5 nM | Compounds | 5 nM | 0.5 nM |
|---|---|---|---|---|---|
| A-P-23 | 74% | 85% | A-P-67 | 70% | 20% |
| A-P-24 | 85% | 85% | A-P-68 | 33% | 5% |
| A-P-25 | 90% | 91% | A-P-69 | 18% | -9% |
| A-P-26 | 18% | -43% | A-P-74 | 70% | 2% |
| A-P-27 | 80% | 78% | A-P-83 | 66% | 36% |
| A-P-28 | 80% | 80% | A-P-84 | -17% | -14% |
| A-P-29 | 68% | 70% | A-P-85 | 34% | 18% |
| A-P-30 | 71% | 36% | A-P-86 | 1% | -30% |
| A-P-31 | 41% | -13% | A-P-100 | 21% | -20% |
| **Conclusion: The compounds disclosed in the present invention can effectively degrade AR protein, and the ability of most compounds to degrade AR protein is comparable to or even better than that of the positive control.** | | | | | |

**Test Example 8**

The inhibitory activity of compounds disclosed in the present invention on LNCaP cell proliferation

LNCAP FGC cells (ATCC, CLR-1740) were cultured in RPMI 1640 (ATCC, 30-2001) complete medium containing 10% fetal bovine serum (Hyclone, SH30406.05). On the first day of the experiment, LNCaP FGC cells were seeded at a density of 5,000 cells/well in 96-well plates using RPMI 1640 medium supplemented with 10% fetal bovine serum, with 100 μL of cell suspension per well. The cells were cultured overnight in a 37°C, 5% $CO_2$ cell incubator. On the second day, 100 μL of different concentrations of the compound of the present invention and the positive control sample ARV-766 (source: Anaiji) prepared in culture medium were added to each well. The final concentration of the compound was 9 concentration points of 5-fold serial dilution starting from 10 μM. The wells to which 100 μL of RPMI 1640 culture medium containing 10% fetal bovine serum was added were set as controls. The cells were cultured in a cell culture incubator at 37°C and 5% $CO_2$ for 96 hours. On the sixth day, the 96-well cell culture plate was removed, and 1/10 volume of WST-8 (Beytime, C0040) reagent was added to each well. The absorbance at 450 nm was detected using a microplate reader (TECAN, F50). The 50% proliferation inhibition concentration ($IC_{50}$ value) was calculated by logistic regression based on the concentration of the compound and absorbance values using Graphpad Prism software.

The consistent $IC_{50}$ values of the compounds disclosed in the present invention on LNCaP cell proliferation are shown in Table 8 below.

Table 8 Consistent $IC_{50}$ values of compounds disclosed in the present invention on LNCaP cell proliferation

| Compounds | $IC_{50}$ (μM) | Compounds | $IC_{50}$ (μM) |
|---|---|---|---|
| ARV-766 | 1.074 | A-P-41 | 18.60 |
| A-P-1 | >10 | A-P-42 | 0.19 |
| A-P-2 | 2.89 | A-P-43 | 0.36 |
| A-P-3 | 3.43 | A-P-44 | 0.24 |
| A-P-4 | 3.52 | A-P-45 | 0.57 |
| A-P-5 | 1.00 | A-P-46 | 0.89 |
| A-P-6 | 1.02 | A-P-48 | 0.86 |
| A-P-7 | 1.88 | A-P-49 | 0.60 |
| A-P-8 | 0.20 | A-P-50 | 0.18 |
| A-P-10 | 136.56 | A-P-51 | 3.36 |
| A-P-12 | 8.11 | A-P-52 | 0.20 |
| A-P-13 | 60.98 | A-P-53 | 0.14 |

(continued)

| Compounds | $IC_{50}$ (μM) | Compounds | $IC_{50}$ (μM) |
|---|---|---|---|
| A-P-14 | 1.10 | A-P-54 | 0.91 |
| A-P-15 | 30.18 | A-P-55 | 0.95 |
| A-P-16 | >90 | A-P-57 | 0.67 |
| A-P-17 | 91.24 | A-P-58 | 0.51 |
| A-P-18 | 120.45 | A-P-59 | 0.15 |
| A-P-19 | 39.29 | A-P-60 | 0.69 |
| A-P-20 | 12.54 | A-P-61 | 0.21 |
| A-P-21 | 8.23 | A-P-62 | 9.54 |
| A-P-22 | 47.64 | A-P-63 | 0.24 |
| A-P-23 | 22.42 | A-P-67 | 7.22 |
| A-P-24 | 22.75 | A-P-68 | 0.97 |
| A-P-25 | 47.74 | A-P-69 | 0.28 |
| A-P-26 | 4.91 | A-P-74 | 0.50 |
| A-P-27 | 8.11 | A-P-75 | 0.35 |
| A-P-28 | 20.71 | A-P-76 | 2.84 |
| A-P-29 | 15.05 | A-P-77 | 0.44 |
| A-P-30 | 1.48 | A-P-78 | 50.70 |
| A-P-31 | 0.58 | A-P-80 | 1.94 |
| A-P-32 | 0.57 | A-P-81 | 14.47 |
| A-P-33 | 1.58 | A-P-82 | 15.71 |
| A-P-34 | 0.87 | A-P-83 | 0.71 |
| A-P-35 | 0.28 | A-P-84 | 0.11 |
| A-P-36 | 0.82 | A-P-85 | 0.29 |
| A-P-37 | 2.23 | A-P-86 | 0.39 |
| A-P-38 | 1.49 | A-P-87 | 0.14 |
| A-P-39 | 12.44 | A-P-94 | 0.39 |
| A-P-40 | 6.56 | A-P-98 | 0.43 |
| A-P-100 | 0.38 | | |
| Conclusion: The PROTAC compounds of the present invention can effectively inhibit the proliferation of LNCaP cells. The inhibitory effects of some PROTAC compounds on LNCaP cells are comparable to or even better than those of the positive control. | | | |

**Test Example 9**

The inhibitory activity of compounds disclosed in the present invention on VCaP cell proliferation

VCaP cells (ATCC, CRL-2876) were cultured in DMEM (Gibco, 11995-065) complete medium containing 10% fetal bovine serum (Hyclone, SH30406.05). On the first day of the experiment, VCaP cells were seeded at a density of 15,000 cells/well in a 96-well plate using DMEM medium containing 10% fetal bovine serum, with 100μL of cell suspension per well, and cultured overnight in a cell incubator at 37°C and 5% $CO_2$. On the second day, 100μL of the compound of the present invention and the positive control ARV-110 (source: Anaiji) prepared in culture medium at different concentrations were added to each well. The final concentration of the compound was 9 concentration points of 5-fold serial dilution starting from 10 μM. The wells to which 100 μL of DMEM medium containing 10% fetal bovine serum was added were set as blank controls and cultured in a cell incubator at 37°C and 5% $CO_2$ for 168 hours. On the ninth day, the 96-well cell

culture plate was removed and 1/10 volume of WST-8 (Beytime, C0040) reagent was added to each well. The absorbance at 450nm was measured using a microplate reader (TECAN, F50). The 50% proliferation inhibition concentration ($IC_{50}$ value) was calculated by logistic regression based on the concentration of the compound and absorbance values using Graphpad Prism software.

The inhibition $IC_{50}$ values of the compound disclosed in present invention on VCaP cell proliferation are shown in Table 9 below.

Table 9 Consistent $IC_{50}$ values of compounds disclosed in the present invention on VCaP cell proliferation

| Compounds | $IC_{50}$ (μM) | Compounds | $IC_{50}$ (μM) |
|---|---|---|---|
| ARV-766 | 0.779 | A-P-43 | 1.55 |
| A-P-1 | 0.000026 | A-P-44 | 2.89 |
| A-P-2 | 0.000060 | A-P-45 | 0.30 |
| A-P-3 | 0.000017 | A-P-46 | 3.75 |
| A-P-4 | 0.00019 | A-P-48 | 0.33 |
| A-P-5 | 0.0017 | A-P-49 | 0.041 |
| A-P-6 | 0.00016 | A-P-50 | 0.051 |
| A-P-7 | 0.0060 | A-P-51 | 8.44 |
| A-P-8 | 0.0050 | A-P-52 | 4.24 |
| A-P-10 | 0.0060 | A-P-53 | 1.32 |
| A-P-12 | 0.000023 | A-P-54 | 0.0070 |
| A-P-13 | 0.0043 | A-P-55 | 1.05 |
| A-P-14 | 0.0022 | A-P-57 | 3.02 |
| A-P-15 | 0.00048 | A-P-58 | 1.83 |
| A-P-16 | 0.0010 | A-P-59 | 0.16 |
| A-P-17 | 0.072 | A-P-60 | 0.94 |
| A-P-18 | <0.0000051 | A-P-61 | 0.19 |
| A-P-19 | <0.000026 | A-P-62 | 0.45 |
| A-P-20 | 0.050 | A-P-63 | 0.39 |
| A-P-21 | 0.0020 | A-P-67 | 0.17 |
| A-P-22 | 0.000026 | A-P-68 | 0.0045 |
| A-P-23 | 0.000046 | A-P-69 | 1.32 |
| A-P-24 | 0.0000081 | A-P-74 | 2.16 |
| A-P-25 | 0.00014 | A-P-75 | 0.042 |
| A-P-26 | 0.034 | A-P-76 | 0.078 |
| A-P-29 | 0.019 | A-P-77 | 0.0055 |
| A-P-30 | 1.00 | A-P-78 | >10 |
| A-P-31 | 3.17 | A-P-80 | 0.051 |
| A-P-32 | 1.23 | A-P-81 | 1.79 |
| A-P-33 | 0.45 | A-P-82 | 0.049 |
| A-P-34 | 0.00029 | A-P-83 | 2.58 |
| A-P-35 | 0.0040 | A-P-84 | 0.49 |
| A-P-36 | 1.66 | A-P-85 | 0.54 |
| A-P-37 | 2.89 | A-P-86 | 1.79 |

(continued)

| Compounds | $IC_{50}$ (μM) | Compounds | $IC_{50}$ (μM) |
|---|---|---|---|
| A-P-38 | >10 | A-P-87 | 0.0014 |
| A-P-39 | >10 | A-P-94 | 0.0015 |
| A-P-41 | >10 | A-P-98 | 0.0060 |
| A-P-40 | >10 | A-P-100 | 4.70 |
| A-P-42 | >10 | | |
| Conclusion: The PROTAC compounds of the present invention can effectively inhibit the proliferation of VCaP cells, among which some PROTAC compounds have an inhibitory effect on VCaP cells comparable to or even better than that of the positive control. | | | |

Test Example 10 Degradation rate (%) of SMARCA2/4 by the PROTAC compounds of the present invention in HeLa cells and SW1573 cells

SW1573 cells were seeded at $2x10^5$Cells/well in a 6-well plate. 2 mL of EMEM medium supplemented with 10% FBS was added to each well and was incubated overnight to allow cell attachment. The next day, 1 μL of 160 μM and 1 μL of 2 μM compound were added to the corresponding wells for treatment, resulting in final compound concentrations of 80 nM and 1 nM, respectively, and a final DMSO concentration of 0.05%. The cells were returned to the incubator and incubated for another 24 hours.

After 24 hours, cells were harvested, the medium was aspirated, and the cells were rinsed once with 1 mL/well PBS. The PBS was aspirated, and 40 μL/well of RIPA lysis buffer containing protease inhibitors was added. After lysis and centrifugation, total protein extracts were obtained, and protein concentration in the extracts was determined by BCA assay. Protein electrophoresis was performed by SDS-PAGE, followed by constant voltage electrophoresis at 100 V for 60 minutes and the protein was then transferred to a PVDF membrane. The PVDF membrane was blocked in 5% skim milk powder for 1 hour at room temperature. Primary antibodies (SMARCA2: CST#11966S, SMARCA4: CST#49360S, GAPDH: CST#5174S) were added and incubated overnight at 4°C. After washing the membrane, secondary antibodies were added and incubated at room temperature for 1 hour (protected from the light). After washing, the membrane was exposed using an Odyssey luminometer. Grayscale analysis of bands was performed using Image Studio software.

HeLa cells were seeded at 1.2 x $10^5$ cells/well in a 6-well plate. 2 mL of EMEM medium supplemented with 10% FBS was added to each well and the culture dish was incubated overnight to allow the cells to adhere. The next day, 1 μL of 160 μM and 1 μL of 2 μM compound were added to the corresponding wells, resulting in final compound concentrations of 80 nM and 1 nM, respectively, and a final DMSO concentration of 0.05%. The cells were returned to the incubator and incubated for another 4 hours.

After 4 hours, cells were harvested, the culture medium was aspirated, and the cells were rinsed once with 1 mL/well PBS. The PBS was aspirated, and 40 μL/well of RIPA lysis buffer containing protease inhibitors was added. After lysis and centrifugation, total protein extracts were obtained, and protein concentration in the extracts was determined by BCA assay. Protein electrophoresis was performed by SDS-PAGE, followed by constant voltage electrophoresis at 100 V for 60 minutes and the protein was then transferred to a PVDF membrane. The PVDF membrane was blocked in 5% skim milk powder for 1 hour at room temperature. Primary antibodies (SMARCA2: CST#11966S, SMARCA4: CST#49360S, GAPDH: CST#5174S) were added and incubated overnight at 4°C. After washing the membrane, secondary antibodies were added and incubated at room temperature for 1 hour (protected from the light). After washing, the membrane was exposed using an Odyssey luminometer. Grayscale analysis of bands was performed using Image Studio software.

The degradation rate (%) of SMARCA2/4 by the PROTAC compounds of the present invention in HeLa cells and SW1573 cells is shown in Table 10.

Table 10 Degradation rate of SMARCA2/4 by PROTAC compounds of the present invention in HeLa cells and SW1573 cells (%)

| Compounds | HeLa | | | | SW1573 | | | |
|---|---|---|---|---|---|---|---|---|
| | SMARCA2 | | SMARCA4 | | SMARCA2 | | SMARCA4 | |
| | 1 nM | 80 nM | 1 nM | 80 nM | 1 nM | 80 nM | 1 nM | 80 nM |
| SM-P-1 | 90 | 100 | 85 | 98 | 72 | 96 | 43 | 97 |
| SM-P-2 | 95 | 98 | 92 | 99 | 98 | 100 | 94 | 100 |

(continued)

| Compounds | HeLa | | | | SW1573 | | | |
|---|---|---|---|---|---|---|---|---|
| | SMARCA2 | | SMARCA4 | | SMARCA2 | | SMARCA4 | |
| | 1 nM | 80 nM | 1 nM | 80 nM | 1 nM | 80 nM | 1 nM | 80 nM |
| SM-P-3 | 84 | 99 | 53 | 93 | 70 | 94 | -19 | 88 |
| SM-P-4 | 100 | 99 | 99 | 98 | 100 | 100 | 99 | 100 |
| SM-P-5 | - | - | - | - | 16 | 36 | -9 | 6 |
| SM-P-6 | - | - | - | - | 20 | 34 | -30 | -41 |
| SM-P-7 | 7 | 68 | -52 | 28 | 1 | 66 | -29 | 39 |
| SM-P-8 | 28 | 64 | 30 | 74 | 9 | 61 | -27 | -11 |
| SM-P-9 | 11 | 15 | -7 | 21 | 3 | 7 | -8 | -5 |
| SM-P-10 | - | - | - | - | 23 | 79 | -44 | 46 |
| SM-P-11 | - | - | - | - | 36 | 89 | -16 | 73 |
| SM-P-12 | 6 | 14 | 17 | 24 | 32 | 32 | 28 | 5 |
| SM-P-13 | 38 | 96 | 19 | 65 | 32 | 88 | -11 | 32 |
| SM-P-14 | -7 | -3 | 4 | -21 | 22 | 14 | 6 | 7 |
| SM-P-15 | - | - | - | - | 20 | 23 | 22 | 3 |
| SM-P-16 | 0 | 31 | 15 | 40 | 2 | 35 | -53 | -47 |
| SM-P-17 | - | - | - | - | 12 | 72 | -18 | 12 |
| SM-P-18 | - | - | - | - | 16 | 25 | -32 | -34 |
| SM-P-19 | 14 | 59 | -11 | 24 | 11 | 31 | 1 | 0 |
| SM-P-20 | 23 | 86 | 32 | 65 | 12 | 76 | -56 | 22 |
| SM-P-21 | 15 | 4 | 17 | 18 | -12 | 14 | -7 | 16 |
| SM-P-22 | - | - | - | - | -9 | 28 | 14 | 4 |
| SM-P-23 | - | - | - | - | 38 | 95 | -14 | 93 |
| SM-P-24 | -10 | 29 | -34 | -1 | -29 | -75 | 12 | 11 |
| SM-P-25 | 10 | 53 | 6 | 42 | 11 | 50 | -21 | -10 |
| SM-P-26 | 100 | 99 | 97 | 100 | 100 | 99 | 98 | 97 |
| SM-P-27 | 51 | 90 | 22 | 79 | 59 | 88 | 40 | 82 |
| SM-P-28 | 17 | 98 | 38 | 99 | 14 | 77 | 11 | 95 |
| SM-P-29 | 100 | 90 | 100 | 92 | 100 | 99 | 100 | 97 |
| SM-P-30 | 99 | 90 | 100 | 94 | 99 | 97 | 100 | 98 |
| SM-P-31 | 61 | 98 | 79 | 99 | 56 | 72 | 94 | 99 |
| SM-P-32 | 74 | 98 | 87 | 98 | 83 | 82 | 98 | 98 |
| SM-P-33 | 45 | 98 | 49 | 99 | 57 | 89 | 84 | 95 |
| SM-P-34 | 97 | 99 | 100 | 100 | 93 | 87 | 100 | 100 |
| SM-P-35 | 100 | 99 | 100 | 100 | 100 | 100 | 100 | 100 |
| SM-P-36 | 99 | 97 | 100 | 97 | 100 | 98 | 99 | 95 |
| SM-P-37 | 24 | 33 | -12 | 15 | - | - | - | - |
| SM-P-38 | -6 | 10 | 7 | 26 | 24 | 29 | 17 | 6 |

(continued)

| Compounds | HeLa | | | | SW1573 | | | |
|---|---|---|---|---|---|---|---|---|
| | SMARCA2 | | SMARCA4 | | SMARCA2 | | SMARCA4 | |
| | 1 nM | 80 nM | 1 nM | 80 nM | 1 nM | 80 nM | 1 nM | 80 nM |
| SMD-3040 | - | - | - | - | 89 | 99 | 21 | 86 |
| **Conclusion: The compounds disclosed in the present invention can effectively degrade** SMARCA2/4 **protein, and the ability of most compounds to degrade** SMARCA2/4 **protein is comparable to or even better than that of the control samples.** | | | | | | | | |

The present invention has been described through the above-described embodiments. However, it should be understood that the above-described embodiments are for illustrative and illustrative purposes only and are not intended to limit the present invention to the described embodiments. Furthermore, it will be understood by those skilled in the art that the present invention is not limited to the above-described embodiments and that further variations and modifications may be made based on the teachings of the present invention, all of which fall within the scope of the present invention. The scope of protection of the present invention is defined by the appended claims and their equivalents.

## Claims

1. A compound represented by Formula I, or an isomer, isotopic derivative, polymorph, prodrug, or a pharmaceutically acceptable salt or a solvate thereof:

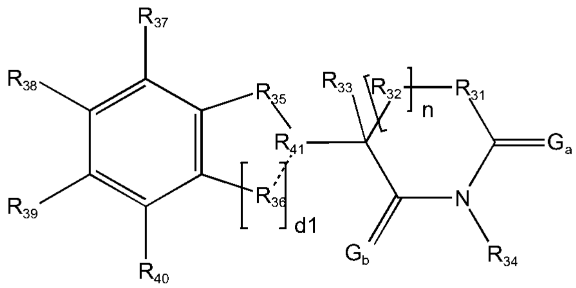

Formula I

wherein, $G_a$ and $G_b$ are independently selected from O, S, and Se; preferably, $G_a$ and $G_b$ are independently O;
each occurrence of $R_{31}$ and $R_{32}$ is independently $CR^aR^b$;
each occurrence of $R_{33}$, $R_{34}$, $R^a$, and $R^b$ is independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, each occurrence of $R_{33}$, $R_{34}$, $R^a$, and $R^b$ is independently selected from H, deuterium, F, Cl, Br, I, $C_1$-$C_6$ alkyl, hydroxyl, $C_1$-$C_6$ haloalkyl, $C_1$-$C_6$ hydroxyalkyl, $C_1$-$C_6$ alkoxy, $C_3$-$C_6$ cycloalkyl, and $C_3$-$C_6$ heterocyclyl containing 1, 2, 3, or 4 heteroatoms independently selected from N, O, P, and S; wherein the alkyl, haloalkyl, hydroxyalkyl, alkoxy, cycloalkyl, and heterocyclyl are each independently optionally substituted by one or more substituents selected from halogen, alkyl, heteroalkyl, alkoxy, haloalkyl, hydroxyl, hydroxyalkyl, cycloalkyl, and heterocyclyl; preferably, each occurrence of $R_{33}$, $R_{34}$, $R^a$, and $R^b$ is independently selected from H, deuterium, F, Cl, Br, I, $C_1$-$C_6$ alkyl, hydroxyl, $C_1$-$C_6$ haloalkyl, $C_1$-$C_6$ hydroxyalkyl, and $C_1$-$C_6$ alkoxy, wherein the alkyl, haloalkyl, hydroxyalkyl, and alkoxy are each independently optionally substituted by one or more substituents selected from halogen, alkyl, heteroalkyl, alkoxy, haloalkyl, and hydroxyl; preferably, each occurrence of $R_{33}$, $R_{34}$, $R^a$, and $R^b$ is independently selected from H, deuterium, F, Cl, Br, I, $C_1$-$C_3$ alkyl, hydroxyl, $C_1$-$C_3$ haloalkyl, $C_1$-$C_3$ hydroxyalkyl, and $C_1$-$C_3$ alkoxy;
n is 0, 1, 2, or 3;
when $R_{35}$ and $R_{36}$, together with the carbon atoms to which they are attached and $R_{41}$, form a ring, and d1 is 1: $R_{35}$

and $R_{36}$ are each independently selected from $CR^aR^b$, C(=S), C(=O), $NR^a$, or $SO_2$, and at least one of $R_{35}$ and $R_{36}$ is C(=O);
$R_{41}$ is N;
$R_{37}$ and $R_{38}$, together with the carbon atoms to which they are attached, form

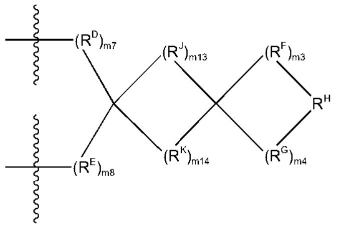
,
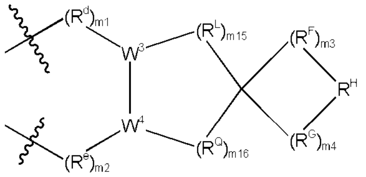
,
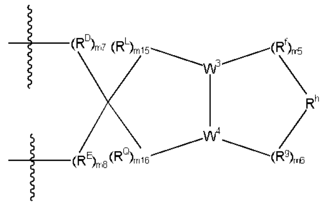
,

or

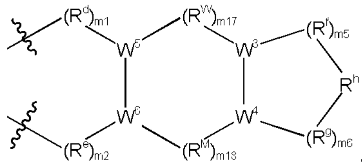
,

and $R_{39}$ and $R_{40}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R_{39}$ and $R_{40}$ are each independently selected from H, deuterium, halogen, alkyl, deuterated alkyl, haloalkyl, alkoxy, and hydroxyl;
or $R_{38}$ and $R_{39}$, together with the carbon atoms to which they are attached, form

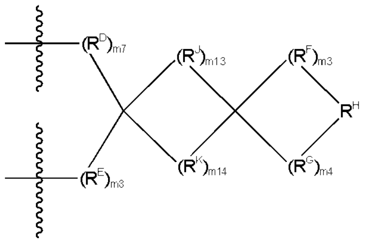
,
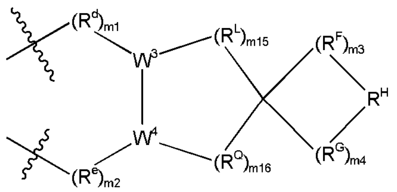
,
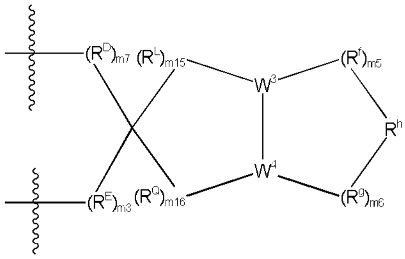
, or
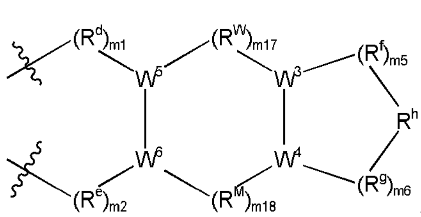
,

and $R_{37}$ and $R_{40}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl,

alkoxycarbonyl, alkylaminocarbonyl, aryl, and heteroaryl;

or $R_{39}$ and $R_{40}$, together with the carbon atoms to which they are attached, form

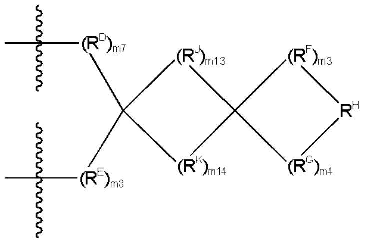

,

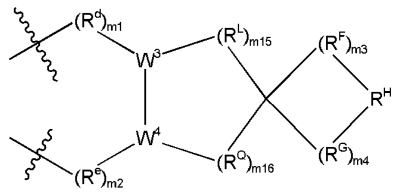

,

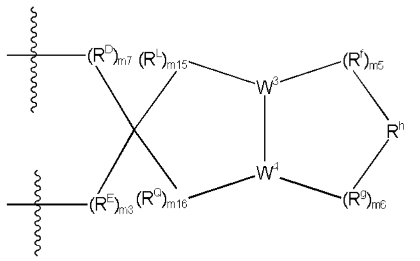

, or

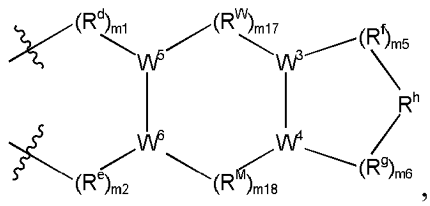

,

and $R_{37}$ and $R_{38}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl;

each occurrence of $R^d$, $R^e$, $R^f$, $R^g$, $R^D$, $R^E$, $R^F$, $R^J$, $R^K$, $R^L$, $R^Q$, $R^W$, $R^M$, and $R^G$ is independently $C(R^m)_2$, $NR^m$, C(=O), O, or S; preferably, for each occurrence of $R^d$ and $R^e$, at least one occurrence exists and at least one of them is O; and for each occurrence of $R^D$ and $R^E$, at least one occurrence exists and at least one of them is O; preferably, for each occurrence of $R^d$ and $R^e$, one occurrence exists and at least one of them is O, and for each occurrence of $R^D$ and $R^E$, one occurrence exists and at least one of them is O; preferably, for each occurrence of $R^d$ and $R^e$, at least one occurrence exists and one of them is O, and for each occurrence of $R^D$ and $R^E$, one occurrence exists and one of them is O;

each occurrence of $W^3$, $W^4$, $W^5$, and $W^6$ is independently $CR^m$ or N; preferably, each occurrence of $W^3$, $W^4$, $W^5$, and $W^6$ is independently CH or N; and

each occurrence of $R^h$ and $R^H$ is independently $NR^{1h}$, C(=O), $SO_2$, or $CR^{2h}R^{3h}$; preferably, each occurrence of $R^h$ and $R^H$ is independently NH, C(=O), C(Cl)H, or C(OH)H; preferably, each occurrence of $R^h$ and $R^H$ is independently NH;

when d1 is 0, $R_{35}$ and $R_{41}$ form a ring with the carbon atoms to which they are attached:

$R_{35}$ is $-N(R^a)-W^{11}-$, $W^{11}$ is selected from $CR^aR^b$, C(=O), C(=S), $NR^a$, and $SO_2$; preferably, $W^{11}$ is C(=O); preferably, $R_{35}$ is $-N(CH_3)-C(=O)-$;

$R_{41}$ is N;

$R_{37}$ and $R_{38}$, together with the carbon atoms to which they are attached, form

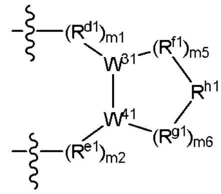

or

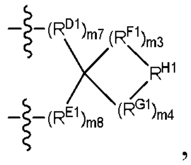

,

and $R_{39}$ and $R_{40}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R_{39}$ and $R_{40}$ are each independently selected from H, deuterium, halogen, alkyl, deuterated alkyl, haloalkyl, and hydroxyl;
or $R_{38}$ and $R_{39}$, together with the carbon atoms to which they are attached, form

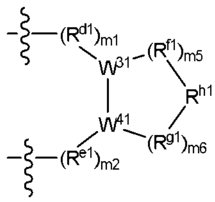 or 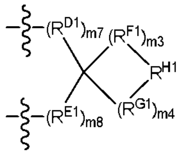 ,

and $R_{37}$ and $R_{40}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R_{37}$ and $R_{40}$ are each independently selected from H, deuterium, halogen, alkyl, deuterated alkyl, haloalkyl, and hydroxyl;
or $R_{39}$ and $R_{40}$, together with the carbon atoms to which they are attached, form

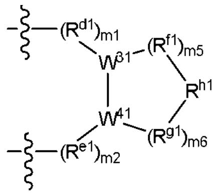 or 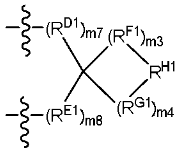 ,

and $R_{37}$ and $R_{38}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl;
each occurrence of $R^{d1}$, $R^{e1}$, $R^{f1}$, $R^{g1}$, $R^{D1}$, $R^{E1}$, $R^{F1}$, and $R^{G1}$ is independently $C(R^m)_2$, $NR^m$, C(=O), O , or S; preferably, for each occurrence of $R^{d1}$ and $R^{e1}$, at least one occurrence exists and at least one of them is O, and for each occurrence of $R^{D1}$ and $R^{E1}$, at least one occurrence exists and at least one of them is O;
each occurrence of $W^{31}$ and $W^{41}$ is independently $CR^m$ or N; preferably, each occurrence of $W^{31}$ and $W^{41}$ is independently CH or N; and
each occurrence of $R^{h1}$ and $R^{H1}$ is independently $NR^{1h}$, C(=O), $SO_2$, or $CR^{2h}R^{3h}$; preferably, each occurrence of $R^{h1}$ and $R^{H1}$ is independently NH, C=O, CHCl, or CHOH;
when no chemical bond exists between $R_{36}$ and $R_{41}$, $R_{35}$ and $R_{36}$ do not form a ring together with the carbon atoms to which they are attached and $R_{41}$, and d1 is 1:

$R_{35}$ is a single bond;

$R_{41}$ is selected from -NR$^a$-, -NR$^a$CO-, $C_1$-$C_6$ alkylene, and $C_1$-$C_6$ haloalkylene; preferably, $R_{41}$ is selected from -NH-CO-, -NH-, and -N($CH_3$)-;

$R_{37}$ and $R_{38}$, together with the carbon atoms to which they are attached, form

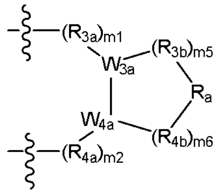

or

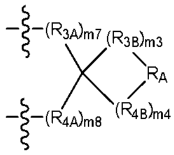

,

and $R_{39}$, $R_{40}$, and $R_{36}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R_{39}$, $R_{40}$, and $R_{36}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; preferably, $R_{39}$, $R_{40}$, and $R_{36}$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$haloalkoxy, and hydroxyl;

or $R_{38}$ and $R_{39}$, together with the carbon atoms to which they are attached, form

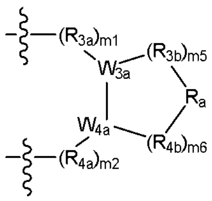

or

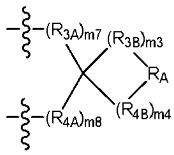

,

and $R_{37}$, $R_{40}$, and $R_{36}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R_{37}$, $R_{40}$, and $R_{36}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; preferably, $R_{37}$, $R_{40}$, and $R_{36}$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy, and hydroxyl;

or $R_{39}$ and $R_{40}$, together with the carbon atoms to which they are attached, form

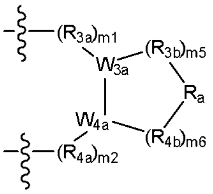 or 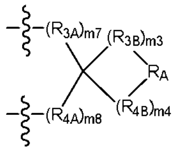,

and $R_{36}$, $R_{37}$, and $R_{38}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R_{36}$, $R_{37}$, and $R_{38}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; preferably, $R_{36}$, $R_{37}$, and $R_{38}$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy, and hydroxyl; or $R_{40}$ and $R_{36}$, together with the carbon atoms to which they are attached, form

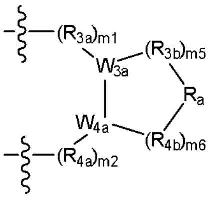 or 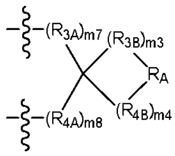,

and $R_{37}$, $R_{38}$, and $R_{39}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R_{37}$, $R_{38}$, and $R_{39}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; preferably, $R_{37}$, $R_{38}$, and $R_{39}$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy, and hydroxyl;
each occurrence of $R_{3a}$, $R_{3b}$, $R_{4a}$, $R_{4b}$, $R_{3A}$, $R_{3B}$, $R_{4A}$, and $R_{4B}$ is independently selected from $C(R^a)_2$, $NR^a$, C(O), O, and S, and, for each occurrence of $R_{3A}$ or $R_{4A}$, at least one occurrence exists and at least one of them is O, for each occurrence of $R_{3a}$ or $R_{4a}$, at least one occurrence exists and at least one of them is O or $N(CH_3)$; preferably, for each occurrence of $R_{3A}$ or $R_{4A}$, at least one occurrence exists and at least one of them is O, for each occurrence of $R_{3a}$ or $R_{4a}$, at least one occurrence exists and at least one of them is O;
each occurrence of $W_{3a}$ and $W_{4a}$ is independently $CR^a$ or N, and at least one of $W_{3a}$ and $W_{4a}$ is N;
each occurrence of $R_a$ and $R_A$ are independently $NR^{4h}$, C(=O), $SO_2$, or $CR^{2h}R^{3h}$; preferably, each occurrence of $R_a$ and $R_A$ are independently $NR^{4h}$, $SO_2$, or $CR^{2h}R^{3h}$; preferably, $R_A$ or $R_a$ is - NH or $-NC(O)CH_3$; and
$R^{4h}$ is selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkylaminocarbonyl, cycloalkyl, heterocyclyl, aryl, and heteroaryl; wherein the alkyl, heteroalkyl, alkoxy, cycloalkyl, heterocyclyl, aryl, and heteroaryl are each independently optionally substituted by one or more substituents selected from halogen, alkyl, heteroalkyl, alkenyl, alkynyl, alkoxy, haloalkyl, hydroxyl, hydroxyalkyl, cyano, amino, nitro, cycloalkyl, heterocyclyl, alkylamino, alkylacyl, alkylaminocarbonyl, aryl, and heteroaryl; preferably, each occurrence of $R^{4h}$ is independently selected from H, deuterium, F, Cl, Br, I, $C_1$-$C_3$ alkyl, $C_1$-$C_3$ deuterated alkyl, $C_1$-$C_3$ haloalkyl, hydroxyl, and $C_1$-$C_3$ alkoxy; and
when $R_{41}$ is selected from $-NR^aCO-$, and $R_{38}$ and $R_{39}$, together with the carbon atoms to which they are

attached, form

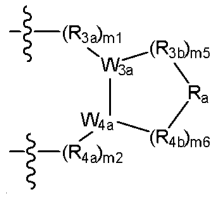

,

or $R_{39}$ and $R_{40}$, together with the carbon atoms to which they are attached, form

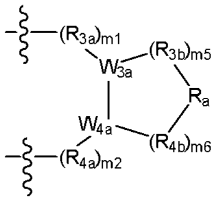

,

m1 is 2, the two occurrences of $R_{3a}$ are independently O and $CH_2$, and m2 is 0, at least one of $R_{37}$ and $R_{36}$ is not H, and $R_{36}$ is not F;

each occurrence of $R^m$, $R^{1h}$, $R^{2h}$, and $R^{3h}$ is selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkoxy, cycloalkyl, heterocyclyl, aryl, and heteroaryl are each independently optionally substituted by one or more substituents selected from halogen, alkyl, heteroalkyl, alkenyl, alkynyl, alkoxy, haloalkyl, hydroxyl, hydroxyalkyl, cyano, amino, nitro, cycloalkyl, heterocyclyl, alkylamino, alkylacyl, alkoxycarbonyl, alkylaminocarbonyl, aryl, and heteroaryl; preferably, each occurrence of $R^m$, $R^{1h}$, $R^{2h}$, and $R^{3h}$ is independently selected from H, deuterium, halogen, alkyl, deuterated alkyl, haloalkyl, alkoxy, hydroxyl, and alkylacyl; preferably, each occurrence of $R^m$, $R^{1h}$, $R^{2h}$, and $R^{3h}$ is independently selected from H, deuterium, F, Cl, Br, I, $C_1$-$C_3$ alkyl, $C_1$-$C_3$ deuterated alkyl, $C_1$-$C_3$ haloalkyl, hydroxyl, $C_1$-$C_3$ alkylacyl, and $C_1$-$C_3$ alkoxy;

each occurrence of m1 and m2 is independently an integer of 0, 1, 2, 3, 4, 5, or 6, and $m1 + m2 \leq 6$;

each occurrence of m3 is independently an integer of 0, 1, 2, 3, 4, 5, 6, or 7, m4 is an integer of 1, 2, 3, 4, 5, 6, 7, or 8, and $m3 + m4 \leq 8$;

each occurrence of m5 and m6 is independently an integer of 0, 1, 2, 3, 4, 5, 6, or 7, and $m5 + m6 \leq 7$;

each occurrence of m7 and m8 is independently an integer of 0, 1, 2, 3, 4, 5, 6, or 7, and $m7 + m8 \leq 7$;

each occurrence of m13 and m14 is independently an integer of 0, 1, 2, 3, 4, 5, 6, 7, or 8, and m13 and m14 are not 0 at the same time, $m13 + m14 \leq 8$;

each occurrence of m15 and m16 is independently an integer of 0, 1, 2, 3, 4, 5, 6, or 7, and $m15 + m16 \leq 7$;

each occurrence of m17 and m18 is independently an integer of 0, 1, 2, 3, 4, 5, or 6, and $m17 + m18 \leq 6$; and

the compound is not

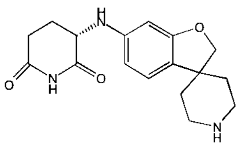

,

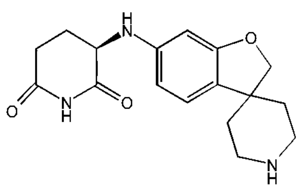

,

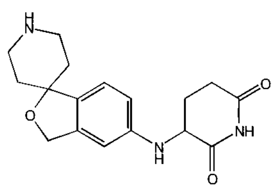

,

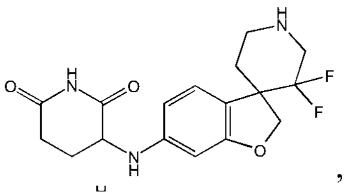, 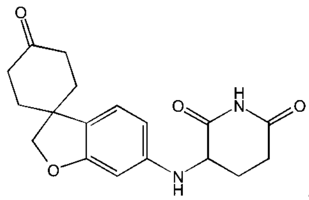, 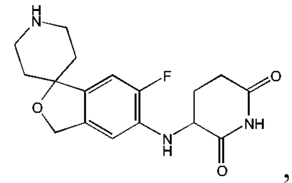,

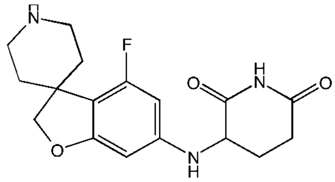, or 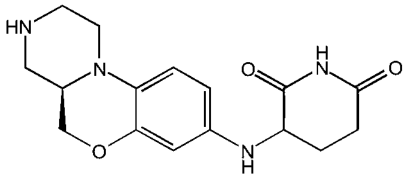.

**2.** The compound according to claim 1, wherein the compound is selected from:

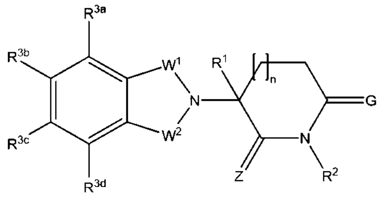

Formula IA,

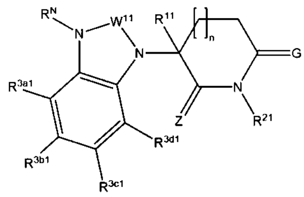

Formula 1B, and

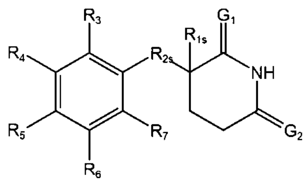

Formula 1C;

when the compound has the structure of Formula IA:

$W^1$ and $W^2$ are identical or different, and are independently $CR^aR^b$, C(=S), C(=O), $NR^a$, or $SO_2$, and at least one of $W^1$ and $W^2$ is C(=O);

G and Z are identical or different, independently selected from O, S, and Se; preferably, G and Z are independently O;

$R^{3b}$ and $R^{3c}$, together with the carbon atoms to which they are attached, form

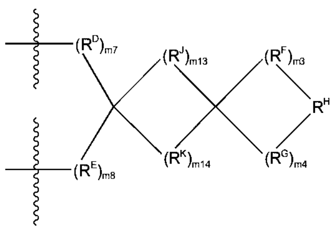, 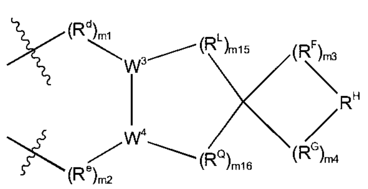, 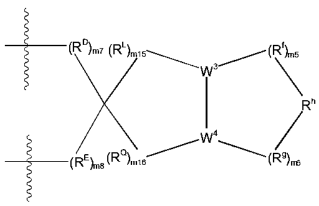,

or

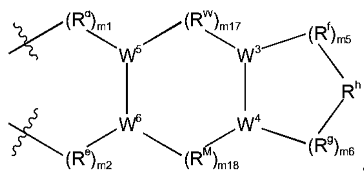,

and $R^{3a}$ and $R^{3d}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl,

alkoxycarbonyl, alkylaminocarbonyl, aryl, and heteroaryl;

or $R^{3c}$ and $R^{3d}$, together with the carbon atoms to which they are attached, form

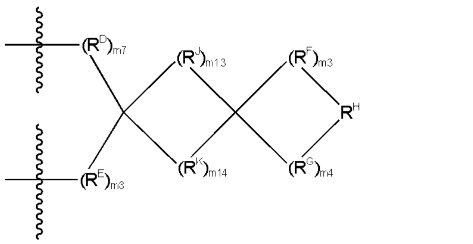,

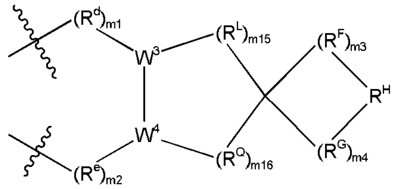,

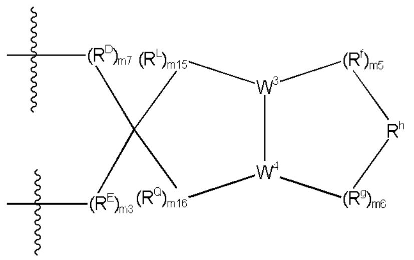, or

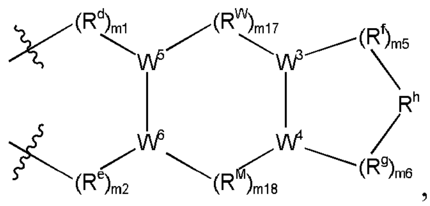,

and $R^{3a}$ and $R^{3b}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl;

or $R^{3a}$ and $R^{3b}$, together with the carbon atoms to which they are attached, form

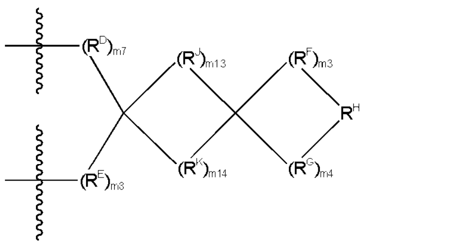,

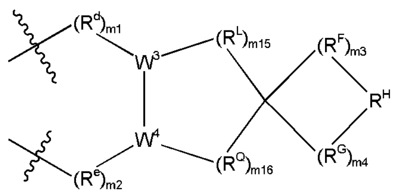,

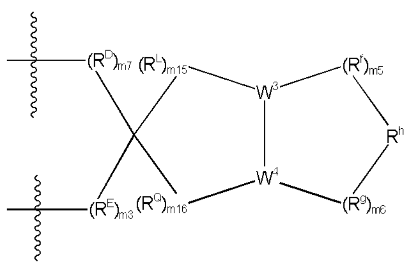, or

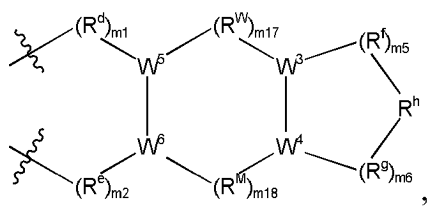,

and $R^{3c}$ and $R^{3d}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R^{3c}$ and $R^{3d}$ are each independently selected from H,

deuterium, halogen, alkyl, deuterated alkyl, haloalkyl, alkoxy, and hydroxyl;
each occurrence of $R^d$, $R^e$, $R^f$, $R^g$, $R^D$, $R^E$, $R^F$, $R^J$, $R^K$, $R^L$, $R^Q$, $R^W$, $R^M$, and $R^G$ is independently $C(R^m)_2$, $NR^m$, C(=O), O, or S; preferably, for each occurrence of $R^d$ and $R^e$, at least one occurrence exists and at least one of them is O, and for each occurrence of $R^D$ and $R^E$, at least one occurrence exists and at least one of them is O;
each occurrence of $W^3$, $W^4$, $W^5$, and $W^6$ is independently $CR^m$ or N; preferably, each occurrence of $W^3$, $W^4$, $W^5$, and $W^6$ is independently CH or N;
each occurrence of $R^h$ and $R^H$ is independently $NR^{1h}$, C(=O), $SO_2$, or $CR^{2h}R^{3h}$; preferably, each occurrence of $R^h$ and $R^H$ is independently NH, C(=O), C(Cl)H, or C(OH)H;
each occurrence of m1 and m2 is independently an integer of 0, 1, 2, 3, 4, 5, or 6, and $m1 + m2 \leq 6$;
each occurrence of m3 is independently an integer of 0, 1, 2, 3, 4, 5, 6, or 7, m4 is an integer of 1, 2, 3, 4, 5, 6, 7, or 8, and $m3 + m4 \leq 8$;
each occurrence of m5 and m6 is independently an integer of 0, 1, 2, 3, 4, 5, 6, or 7, and $m5 + m6 \leq 7$;
each occurrence of m7 and m8 is independently an integer of 0, 1, 2, 3, 4, 5, 6, or 7, and $m7 + m8 \leq 7$;
each occurrence of m13 and m14 is independently an integer of 0, 1, 2, 3, 4, 5, 6, 7, or 8, and m13 and m14 are not 0 at the same time, and $m13 + m14 \leq 8$;
each occurrence of m15 and m16 is independently an integer of 0, 1, 2, 3, 4, 5, 6, or 7, and $m15 + m16 \leq 7$;
each occurrence of m17 and m18 is independently an integer of 0, 1, 2, 3, 4, 5, or 6, and $m17 + m18 \leq 6$;
each occurrence of $R^m$ is selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, each occurrence of $R^m$ is independently selected from H, deuterium, halogen, alkyl, deuterated alkyl, haloalkyl, and hydroxyl; preferably, each occurrence of $R^m$ is independently selected from H, deuterium, F, Cl, Br, I, $C_1$-$C_3$ alkyl, $C_1$-$C_3$ deuterated alkyl, $C_1$-$C_3$ haloalkyl, and hydroxyl;
$R^{1h}$, $R^{2h}$, and $R^{3h}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkoxy, cycloalkyl, heterocyclyl, aryl, and heteroaryl are each independently optionally substituted by one or more substituents selected from halogen, alkyl, heteroalkyl, alkenyl, alkynyl, alkoxy, haloalkyl, hydroxyl, hydroxyalkyl, cyano, amino, nitro, cycloalkyl, heterocyclyl, alkylamino, alkylacyl, alkoxycarbonyl, alkylaminocarbonyl, aryl, and heteroaryl; preferably, $R^{1h}$, $R^{2h}$, and $R^{3h}$ are each independently selected from H, deuterium, halogen, alkyl, deuterated alkyl, haloalkyl, hydroxyl, and alkylacyl; preferably, each occurrence of $R^{1h}$, $R^{2h}$, and $R^{3h}$ is independently selected from H, deuterium, F, Cl, Br, I, $C_1$-$C_3$ alkyl, $C_1$-$C_3$ deuterated alkyl, $C_1$-$C_3$ haloalkyl, hydroxyl and $C_1$-$C_3$ alkoxy;
$R^1$ is selected from H, halogen, deuterium, $C_1$-$C_6$ alkyl, $C_1$-$C_6$ alkoxy, hydroxyl, cycloalkyl, $C_1$-$C_6$ haloalkyl, and hydroxyalkyl, and preferably, $R^1$ is selected from H, F, Cl, Br, I, $C_1$-$C_3$ alkyl, $C_1$-$C_3$ alkoxy, $C_3$-$C_6$ cycloalkyl, $C_1$-$C_3$ haloalkyl, hydroxyl, and $C_1$-$C_6$ hydroxyalkyl;
$R^2$, $R^a$, and $R^b$ are each independently selected from H, deuterium, F, Cl, Br, I, $C_1$-$C_6$ alkyl, hydroxyl, $C_1$-$C_6$ haloalkyl, $C_1$-$C_6$ hydroxyalkyl, and $C_1$-$C_6$ alkoxy; and
n is 0, 1, 2, or 3;
when the compound is selected from the structure of Formula 1B:

$W^{11}$ is selected from $CR^aR^b$, C(=O), C(=S), $NR^a$, or $SO_2$; preferably, $W^{11}$ is C(=O);
G and Z are identical or different, and are each independently selected from O, S, and Se;
$R^{3b1}$ and $R^{3c1}$, together with the carbon atoms to which they are attached, form

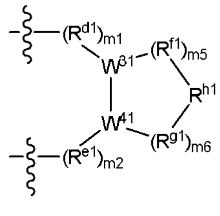
or
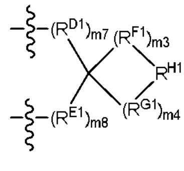
,

and $R^N$, $R^{3a1}$, and $R^{3d1}$ are each independently selected from H, deuterium, halogen, alkyl, deuterated alkyl,

heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R^N$, $R^{3a1}$, and $R^{3d1}$ are each independently selected from H, deuterium, halogen, alkyl, alkoxy, deuterated alkyl, haloalkyl, and hydroxyl;

or $R^{3c1}$ and $R^{3d1}$ together with the carbon atoms to which they are attached, form

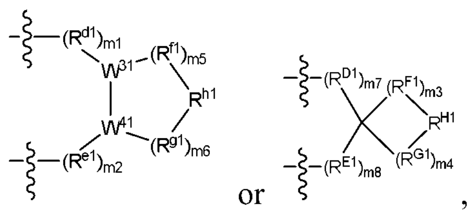

and $R^N$, $R^{3a1}$, and $R^{3b1}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R^N$, $R^{3a1}$, and $R^{3d1}$ are each independently selected from H, deuterium, halogen, alkyl, alkoxy, deuterated alkyl, haloalkyl, and hydroxyl;

or $R^{3a1}$ and $R^{3b1}$, together with the carbon atoms to which they are attached, form

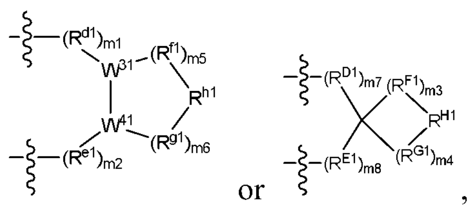

and $R^N$, $R^{3c1}$, and $R^{3d1}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R^N$, $R^{3c1}$, and $R^{3d1}$ are each independently selected from H, deuterium, halogen, alkyl, alkoxy, deuterated alkyl, haloalkyl, and hydroxyl;

each occurrence of $R^{d1}$, $R^{e1}$, $R^{f1}$, $R^{g1}$, $R^{D1}$, $R^{E1}$, $R^{F1}$, and $R^{G1}$ is independently $C(R^m)_2$, $NR^m$, C(=O), O, or S; preferably, for each occurrence of $R^{d1}$ and $R^{e1}$, at least one occurrence exists and at least one of them is O, and for each occurrence of $R^{D1}$ and $R^{E1}$, at least one occurrence exists and at least one of them is O;

each occurrence of $W^{31}$ and $W^{41}$ is independently $CR^m$ or N; preferably, each occurrence of $W^{31}$ and $W^{41}$ is independently CH or N;

each occurrence of $R^{h1}$ and $R^{H1}$ is independently $NR^{1h}$, C(=O), $SO_2$, or $CR^{2h}R^{3h}$; preferably, each occurrence of $R^{h1}$ and $R^{H1}$ is independently NH, C=O, CHCl, or CHOH;

each occurrence of m1 and m2 is independently an integer of 0, 1, 2, 3, 4, 5, or 6, and $m1 + m2 \leq 6$;

each occurrence of m3 is independently an integer of 0, 1, 2, 3, 4, 5, 6, or 7, m4 is an integer of 1, 2, 3, 4, 5, 6, 7, or 8, and $m3 + m4 \leq 8$;

each occurrence of m5 and m6 is independently an integer of 0, 1, 2, 3, 4, 5, 6, or 7, and $m5 + m6 \leq 7$;

each occurrence of m7 and m8 is independently an integer of 0, 1, 2, 3, 4, 5, 6, or 7, and $m7 + m8 \leq 7$;

each occurrence of $R^m$ is independently selected from H, deuterium, halogen, alkyl, deuterated alkyl,

heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, each occurrence of $R^m$ is independently selected from H, deuterium, halogen, alkyl, deuterated alkyl, haloalkyl, and hydroxyl; preferably, each occurrence of $R^m$ is independently selected from H, deuterium, F, Cl, Br, I, $C_1$-$C_3$ alkyl, hydroxyl, and $C_1$-$C_3$ alkoxy;

$R^{1h}$, $R^{2h}$, and $R^{3h}$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkoxy, cycloalkyl, heterocyclyl, aryl, and heteroaryl are each independently optionally substituted by one or more substituents selected from halogen, alkyl, heteroalkyl, alkenyl, alkynyl, alkoxy, haloalkyl, hydroxyl, hydroxyalkyl, cyano, amino, nitro, cycloalkyl, heterocyclyl, alkylamino, alkylacyl, alkoxycarbonyl, alkylaminocarbonyl, aryl, and heteroaryl; preferably, each occurrence of $R^{1h}$, $R^{2h}$, and $R^{3h}$ is independently selected from H, deuterium, halogen, alkyl, deuterated alkyl, haloalkyl, hydroxyl, and alkylacyl; preferably, each occurrence of $R^{1h}$, $R^{2h}$, and $R^{3h}$ is independently selected from H, deuterium, F, Cl, Br, I, $C_1$-$C_3$ alkyl, hydroxyl, and $C_1$-$C_3$ alkoxy;

$R^{11}$ is selected from H, halogen, deuterium, $C_1$-$C_6$ alkyl, $C_1$-$C_6$ alkoxy, hydroxyl, cycloalkyl, $C_1$-$C_6$ haloalkyl, and hydroxyalkyl, and preferably, $R^1$ is selected from H, F, Cl, Br, I, $C_1$-$C_3$ alkyl, $C_1$-$C_3$ alkoxy, $C_3$-$C_6$ cycloalkyl, $C_1$-$C_3$ haloalkyl, hydroxyl, and $C_1$-$C_6$ hydroxyalkyl;

$R^{21}$, $R^a$, and $R^b$ are each independently selected from H, deuterium, F, Cl, Br, I, $C_1$-$C_6$ alkyl, hydroxyl, $C_1$-$C_6$ haloalkyl, $C_1$-$C_6$ hydroxyalkyl, and $C_1$-$C_6$ alkoxy; and

n is 0, 1, 2, or 3; preferably, n is 0, 1, or 2;

when the compound is selected from the structure of Formula 1C:

$G_1$ and $G_2$ are each independently selected from O, S, and Se; preferably, $G_1$ and $G_2$ are each independently O;

$R_{2s}$ is selected from $-NR_{2A}$, $-NR_{2A}CO-$, $C_1$-$C_6$ alkylene, and $C_1$-$C_6$ haloalkylene; preferably, $R_{2s}$ is selected from -NH-CO-, -NH-, and $-N(CH_3)-$;

$R_3$ and $R_4$, together with the carbon atoms to which they are attached, form

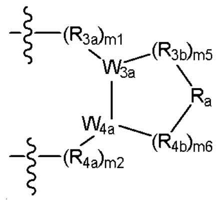

or

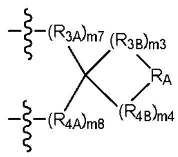

,

and $R_5$, $R_6$, and $R_7$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R_5$, $R_6$, and $R_7$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino;

preferably, $R_5$, $R_6$, and $R_7$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy and hydroxyl;
or $R_4$ and $R_5$, together with the carbon atoms to which they are attached, form

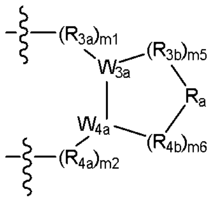 or 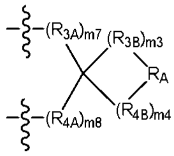 ,

and $R_3$, $R_6$, and $R_7$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R_3$, $R_6$, and $R_7$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; preferably, $R_3$, $R_6$, and $R_7$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy and hydroxyl;
or $R_5$ and $R_6$, together with the carbon atoms to which they are attached, form

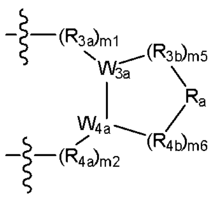 or 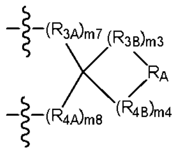 ,

and $R_3$, $R_4$, and $R_7$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R_3$, $R_4$, and $R_7$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; preferably, $R_3$, $R_4$, and $R_7$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy and hydroxyl;
or $R_6$ and $R_7$, together with the carbon atoms to which they are attached, form

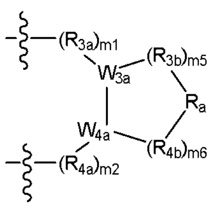 or 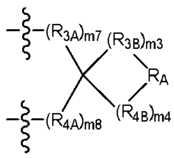 ,

and $R_3$, $R_4$, and $R_5$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkoxycarbonyl, alkylaminocarbonyl, cycloalkyl, heterocyclyl, aryl, and heteroaryl; wherein the alkyl, heteroalkyl, alkenyl, alkynyl, alkoxy, cycloalkyl, heterocyclyl, aryl, and heteroaryl are each independently optionally substituted by one or more substituents selected from halogen, alkyl,

heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkoxycarbonyl, alkylaminocarbonyl, aryl, and heteroaryl; preferably, $R_3$, $R_4$, and $R_5$ are each independently selected from H, deuterium, halogen, alkyl, deuterated alkyl, heteroalkyl, alkenyl, alkynyl, alkoxy, haloalkyl, haloalkoxy, hydroxyl, hydroxyalkyl, and amino; more preferably, $R_3$, $R_4$, and $R_5$ are each independently selected from H, deuterium, F, Cl, Br, I, $C_1$-$C_6$ alkyl, $C_1$-$C_6$ deuterated alkyl, $C_1$-$C_6$ alkoxy, $C_1$-$C_6$ haloalkyl, $C_1$-$C_6$ haloalkoxy and hydroxyl;

each occurrence of $R_{3a}$, $R_{3b}$, $R_{4a}$, $R_{4b}$, $R_{3A}$, $R_{3B}$, $R_{4A}$, and $R_{4B}$ is independently selected from $C(R_{2A})_2$, $NR_{2A}$, C(O), O, and S; preferably, for each occurrence of $R_{3A}$ or $R_{4A}$, at least one occurrence exists and at least one of them is O, and for each occurrence of $R_{3a}$ or $R_{4a}$, at least one occurrence exists and at least one of them is O or $N(CH_3)$; preferably, for each occurrence of $R_{3A}$ or $R_{4A}$, at least one occurrence exists and at least one of them is O, and for each occurrence of $R_{3a}$ or $R_{4a}$, at least one occurrence exists and at least one of them is O;

each occurrence of $W_{3a}$ and $W_{4a}$ is independently $CR_{2A}$ or N, and at least one of $W_{3a}$ and $W_{4a}$ is N, and

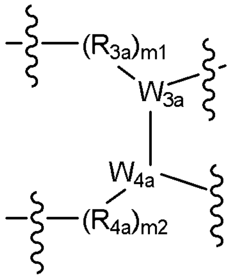

or

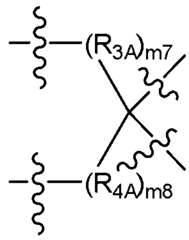

contains at least one of O or N;

each occurrence of $R_a$ and $R_A$ is independently $C(R_{2A})_2$, $NR_{2A}$, or C(O); preferably, each occurrence of $R_a$ and $R_A$ is independently -NH or $-NC(O)CH_3$;

each occurrence of m1 and m2 is independently an integer of 0, 1, 2, 3, 4, 5, or 6, and m1 + m2 $\leq$ 6;

each occurrence of m3 and m4 is independently an integer of 0, 1, 2, 3, 4, 5, 6, 7, or 8, and m3 + m4 $\leq$ 8, and m3 and m4 are not 0 at the same time;

each occurrence of m5 and m6 is independently an integer of 0, 1, 2, 3, 4, 5, 6, or 7, and m5 + m6 $\leq$ 7;

each occurrence of m7 and m8 is independently an integer of 0, 1, 2, 3, 4, 5, 6, or 7, and m7 + m8 $\leq$ 7;

each occurrence of $R_{2A}$ and $R_{1s}$ is independently selected from H, halogen, deuterium, $C_1$-$C_6$ alkyl, $C_1$-$C_6$ alkoxy, hydroxyl, cycloalkyl, $C_1$-$C_6$ haloalkyl, $-C(O)-CH_3$, and hydroxyalkyl; preferably, each occurrence of $R_{2A}$ and $R_{1s}$ is independently selected from H, F, Cl, Br, I, $C_1$-$C_3$ alkyl, $C_1$-$C_3$ alkoxy, $C_3$-$C_6$ cycloalkyl, $C_1$-$C_3$ haloalkyl, hydroxyl, $-C(O)-CH_3$, and $C_1$-$C_6$ hydroxyalkyl;

when $R_{2s}$ is selected from $-NR_{2A}CO-$, and $R_4$ and $R_5$ , together with the carbon atoms to which they are attached, form

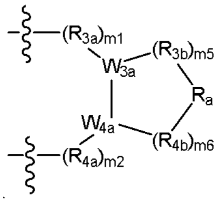

,

or $R_5$ and $R_6$, together with the carbon atoms to which they are attached, form

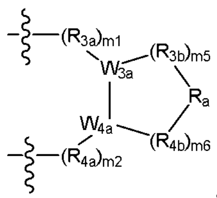

,

m1+m2=2, at least one of $R_3$ and $R_7$ is not H, and $R_7$ is not F; and

the compound is not

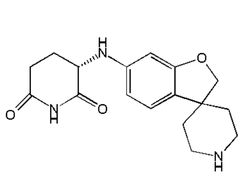, 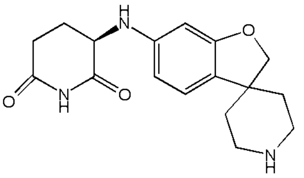, 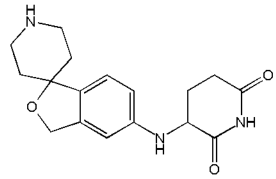,

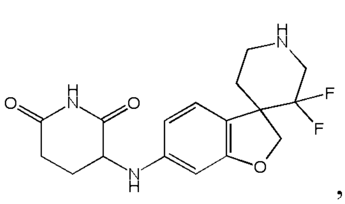, 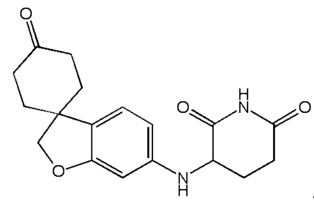, 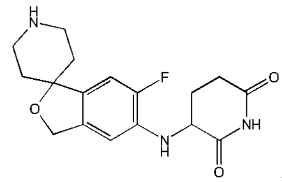,

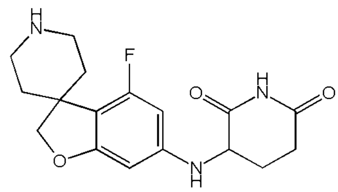, or 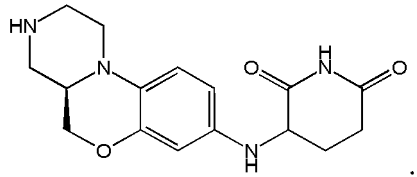.

**3.** The compound according to claim 2, wherein:

when the compound is of the structure of Formula IA, $W^1$ and $W^2$ are identical or different, and are each independently C(=S), $CH_2$, or C(=O), and at least one of $W^1$ and $W^2$ is C(=O); preferably, when $W^1$ is $CH_2$, and when $W^2$ is C(=O) or $W^1$ is C(=O), $W^2$ is $CH_2$ or C(=O); preferably, when $W^1$ is $CH_2$, and when $W^2$ is C(=O) or $W^1$ is C(=O), $W^2$ is $CH_2$; and/or
G and Z are O; and/or
$R^{3b}$ and $R^{3c}$, together with the carbon atoms to which they are attached, form

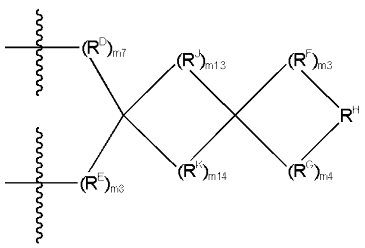, 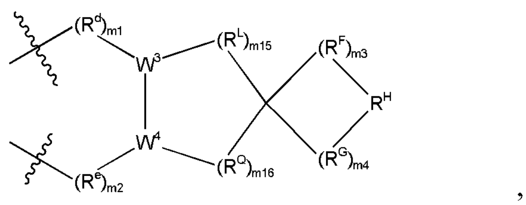,

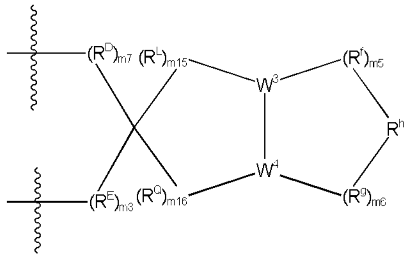, or 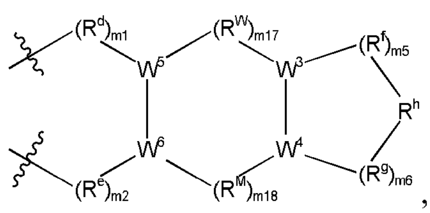,

and $R^{3a}$ and $R^{3d}$ are each independently selected from H, deuterium, halogen, $C_1$-$C_3$ alkyl, and $C_1$-$C_3$ alkoxy; preferably, $R^{3a}$ and $R^{3d}$ are each independently selected from H, deuterium, F, Cl, Br, I, $C_1$-$C_3$ alkyl, and $C_1$-$C_3$ alkoxy;
or $R^{3a}$ and $R^{3b}$, together with the carbon atoms to which they are attached, form

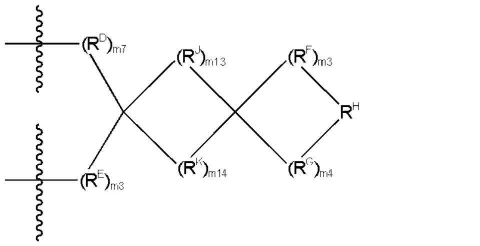,
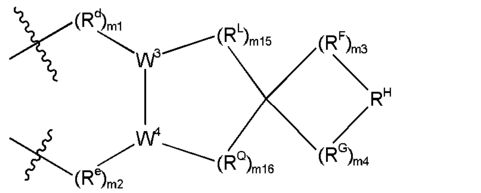,

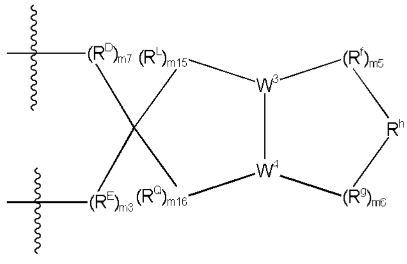, or 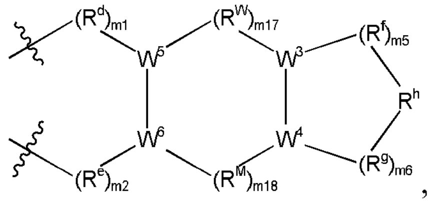,

and $R^{3c}$ and $R^{3d}$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{3c}$ and $R^{3d}$ are each independently selected from H, deuterium, F, Cl, Br, **I,** $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, **I,** $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{3c}$ and $R^{3d}$ are each independently selected from H, deuterium, halogen, $C_1$-$C_3$ alkyl, and $C_1$-$C_3$ alkoxy; preferably, $R^{3c}$ and $R^{3d}$ are each independently selected from H, deuterium, F, Cl, Br, **I,** $C_1$-$C_3$ alkyl, and $C_1$-$C_3$ alkoxy; and/or

each occurrence of $R^d$, $R^e$, $R^f$, $R^g$, $R^D$, $R^E$, $R^F$, $R^J$, $R^K$, $R^L$, $R^Q$, $R^W$, $R^M$, and $R^G$ is independently $C(R^m)_2$ or O; preferably, each occurrence of $R^d$, $R^e$, $R^f$, $R^g$, $R^D$, $R^E$, $R^F$, $R^J$, $R^K$, $R^L$, $R^Q$, $R^W$, $R^M$, and $R^G$ is independently $CF_2$, $CH_2$, or O; preferably, for each occurrence of $R^d$ and $R^e$, at least one occurrence exists and at least one of them is O, and for each occurrence of $R^D$ and $R^E$, at least one occurrence exists and at least one of them is O; preferably, for each occurrence of $R^d$, and $R^e$, one occurrence exists and at least one of them is O, and for each occurrence of $R^D$ and $R^E$, one occurrence exists and at least one of them is O; preferably, $R^d$ and $R^e$, one occurrence exists and one of them is O, and for each occurrence of $R^D$ and $R^E$, one occurrence exists and one of them is O; and/or $R^h$ and $R^H$ are $NR^{1h}$, C(=O), or $CR^{2h}R^{3h}$; $R^{1h}$, $R^{2h}$, and $R^{3h}$ are each independently selected from H, deuterium, halogen, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ **cycloalkyl,** $C_4$-$C_{10}$ heterocyclyl, $C_6$-$C_{10}$ aryl, and $C_5$-$C_{10}$ heteroaryl; preferably, $R^{1h}$, $R^{2h}$, and $R^{3h}$ are each independently selected from H, deuterium, F, Cl, Br, **I,** $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, **I,** $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{1h}$, $R^{2h}$, and $R^{3h}$ are each independently selected from H, deuterium,

halogen, $C_1$-$C_3$ alkyl, hydroxyl, and $C_1$-$C_3$ alkoxy; preferably, $R^{1h}$, $R^{2h}$, and $R^{3h}$ are each independently selected from H, deuterium, F, Cl, Br, **I,** $C_1$-$C_3$ alkyl, hydroxyl, and $C_1$-$C_3$ alkoxy; preferably, $R^h$, and $R^H$ are selected from NH, C(=O), C(Cl)H, and C(OH)H; and/or

each occurrence of m1 and m2 is independently an integer of 0, 1, 2, or 3, and $m1 + m2 \leq 3$; preferably, m1 + m2 = 1, m1 + m2 = 2, or m1 + m2 = 3; and/or

each occurrence of m3 is independently an integer of 0, 1, 2, 3, or 4, m4 is an integer of 1, 2, 3, 4, or 5, and $m3 + m4 \leq 5$; preferably, m3 + m4 = 1, m3 + m4 = 2, m3 + m4 = 3, or m3 + m4 = 4; and/or

each occurrence of m5 and m6 is independently an integer of 0, 1, 2, 3, or 4, and $m5 + m6 \leq 4$; preferably, m5 + m6 = 2 or m5 + m6 = 3; and/or

each occurrence of m7 and m8 is independently an integer of 0, 1, 2, 3, or 4, and $m7 + m8 \leq 4$; preferably, m7 + m8 = 2 or m7 + m8 = 3; and/or

each occurrence of m13 and m14 is independently an integer of 0, 1, 2, 3, or 4, m13 and m14 are not 0 at the same time, and $m13 + m14 \leq 5$; preferably, m13 + m14 = 3 or m13 + m14 = 4; preferably, both m13 and m14 are 2; and/or

each occurrence of m15 and m16 is independently an integer of 0, 1, 2, 3, or 4, and $m15 + m16 \leq 4$; preferably, m15 + m16 = 2 or m15 + m16 = 3; and/or

each occurrence of m17 and m18 is independently an integer of 0, 1, 2, 3, or 4, and $m15 + m16 \leq 3$; preferably, m15 + m16 = 1 or m15 + m16 = 2; and/or

each occurrence of $R^m$ is independently selected from H, deuterium, halogen, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, each occurrence of $R^m$ is independently selected from H, deuterium, F, Cl, Br, **I,** $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, **I,** $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, each occurrence of $R^m$ is independently selected from H, deuterium, halogen, $C_1$-$C_3$ alkyl, and $C_1$-$C_3$ alkoxy; preferably, each occurrence of $R^m$ is independently selected from H, deuterium, F, Cl, Br, **I,** $C_1$-$C_3$ alkyl, and $C_1$-$C_3$ alkoxy; and/or

$R^1$ is selected from H, halogen, $C_1$-$C_3$ alkyl, and hydroxyl; preferably, $R^1$ is selected from H, F, Cl, Br, **I,** $C_1$-$C_3$ alkyl, and hydroxyl; and/or

$R^2$ is selected from H and $C_1$-$C_3$ alkyl; and/or

n is 0 or 1; or

when the compound is of the structure of Formula 1B,

$W^{11}$ is selected from $CH_2$, C(=S), or C(=O); preferably, $W^{11}$ is C(=O), or C(=S); preferably, $W^{11}$ is C(=O); and/or

G and Z are O; and/or

$R^N$ is selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, aryl, and heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, aryl, and heteroaryl; preferably, $R^N$ is selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ haloalkyl, $C_1$-$C_6$ hydroxyalkyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^N$ is selected from H, deuterium, F, Cl, Br, I, $C_1$-$C_6$ alkyl, hydroxyl, $C_1$-$C_6$ haloalkyl, $C_1$-$C_6$ hydroxyalkyl, $C_1$-$C_6$ alkoxy, $C_3$-$C_8$ cycloalkyl, and $C_4$-$C_8$ heterocyclyl containing 1, 2, 3, or 4 heteroatoms independently selected from N, O, P, and S, wherein the $C_1$-$C_6$ alkyl, $C_1$-$C_6$ haloalkyl, $C_1$-$C_6$ hydroxyalkyl, $C_1$-$C_6$ alkoxy, $C_3$-$C_8$ cycloalkyl, and $C_4$-$C_8$ heterocyclyl containing 1, 2, 3, or 4 heteroatoms independently selected from N, O, P, and S, are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl,

hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl;

and/or

$R^{3b1}$ and $R^{3c1}$, together with the carbon atoms to which they are attached, form

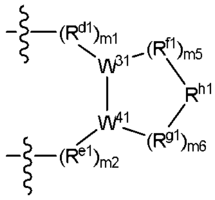
or
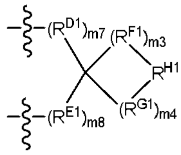
,

and $R^{3a1}$ and $R^{3a1}$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, aryl, and heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, aryl, and heteroaryl; preferably, $R^{3a1}$, and $R^{3d1}$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; more preferably, $R^{3a1}$ and $R^{3d1}$ are each independently selected from H, deuterium, halogen, $C_1$-$C_3$ alkyl, and $C_1$-$C_3$ alkoxy; or $R^{3c1}$ and $R^{3d1}$, together with the carbon atoms to which they are attached, form

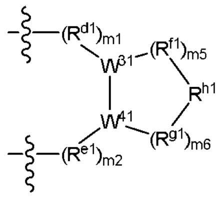
or

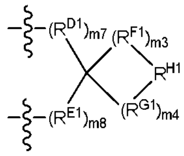
,

and $R^{3a1}$ and $R^{3b1}$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{3a1}$, and $R^{3b1}$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{3a1}$ and $R^{3b1}$ are each independently selected from H, deuterium, halogen, $C_1$-$C_3$ alkyl, and $C_1$-$C_3$ alkoxy; or $R^{3a1}$ and $R^{3b1}$, together with the carbon atoms to which they are

attached, form

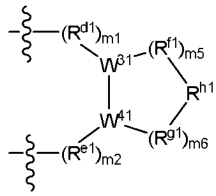

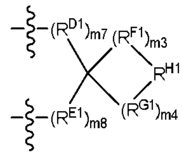

or ,

and $R^{3c1}$ and $R^{3d1}$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{3c1}$ and $R^{3d1}$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R^{3c1}$ and $R^{3d1}$ are each independently selected from H, deuterium, halogen, $C_1$-$C_3$ alkyl, and $C_1$-$C_3$ alkoxy; and/or

each occurrence of $R^{d1}$, $R^{e1}$, $R^{f1}$, $R^{g1}$, $R^{D1}$, $R^{E1}$, $R^{F1}$, and $R^{G1}$ is independently selected from $C(R^m)_2$ or O; each occurrence of $R^m$ is independently selected from H, deuterium, F, Cl, Br, I, $C_1$-$C_3$ alkyl, and $C_1$-$C_3$ alkoxy; and/or

$W^{31}$ and $W^{41}$ are CH or N; and/or

each occurrence of m1 and m2 is independently an integer of 0, 1, 2, or 3, and $m1 + m2 \leq 3$; preferably, m1 + m2 = 1, m1 + m2 = 2, or m1 + m2 = 3; and/or

each occurrence of m3 is independently an integer of 0, 1, 2, 3, or 4, m4 is an integer of 1, 2, 3, 4, or 5, and $m3 + m4 \leq 5$; preferably, m3 + m4 = 1, m3 + m4 = 2, m3 + m4 = 3, or m3 + m4 = 4; and/or

each occurrence of m5 and m6 is independently an integer of 0, 1, 2, 3, or 4, and $m5 + m6 \leq 4$; preferably, m5 + m6 = 2 or m5 + m6 = 3; and/or

each occurrence of m7 and m8 is independently an integer of 0, 1, 2, 3, or 4, and $m7 + m8 \leq 4$; preferably, m7 + m8 = 2 or m7 + m8 = 3; and/or

$R^{11}$ is selected from H, halogen, $C_1$-$C_3$ alkyl, and hydroxyl; $R^{11}$ is selected from H, F, Cl, Br, I, $C_1$-$C_3$ alkyl, and hydroxyl; and/or

$R^{21}$ is selected from H and $C_1$-$C_3$ alkyl; or

when the compound is of the structure of Formula IC,

$G_1$ and $G_2$ are each independently selected from O; and/or

$R_{2s}$ is selected from $-NR_{2A}-$, and $-NR_{2A}CO-$; each occurrence of $R_{2A}$ is each independently selected from H and $C_1$-$C_6$ alkyl; preferably, $R_{2s}$ is selected from -NH-CO-, -NH-, and $-N(CH_3)-$;

and/or

$R_3$ and $R_4$, together with the carbon atoms to which they are attached, form

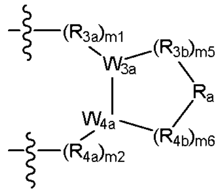

or

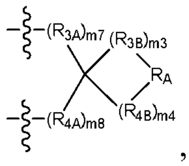

,

and $R_5$, $R_6$, and $R_7$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, aryl, and heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, aryl, and heteroaryl; preferably, $R_5$, $R_6$, and $R_7$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R_5$, $R_6$, and $R_7$ are each independently selected from H, deuterium, halogen, $C_1$-$C_3$ alkyl, and $C_1$-$C_3$ alkoxy; preferably, $R_5$, $R_6$, and $R_7$ are each independently selected from H, deuterium, F, Cl, Br, I, $C_1$-$C_3$ alkyl, and $C_1$-$C_3$ alkoxy; or $R_4$ and $R_5$, together with the carbon atoms to which they are attached, form

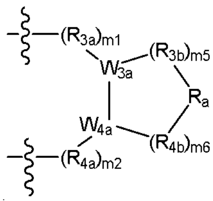

or

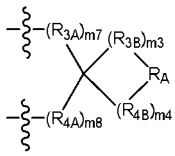

,

and $R_3$, $R_6$, and $R_7$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, aryl, and heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, aryl, and heteroaryl; preferably, $R_3$, $R_6$, and $R_7$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, **I**, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R_3$, $R_6$, and $R_7$ are each independently selected from H, deuterium, halogen, $C_1$-$C_3$ alkyl, and $C_1$-$C_3$ alkoxy; preferably, $R_3$, $R_6$, and $R_7$ are each independently selected from H, deuterium, F, Cl, Br, I, $C_1$-$C_3$ alkyl, and $C_1$-$C_3$ alkoxy; or $R_5$ and $R_6$, together with the carbon atoms to which they are attached, form

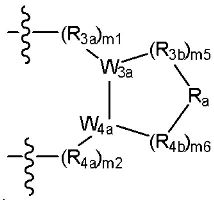 or 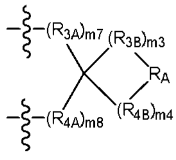 ,

and $R_3$, $R_4$, and $R_7$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, aryl, and heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, aryl, and heteroaryl; preferably, $R_3$, $R_4$, and $R_7$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R_3$, $R_4$, and $R_7$ are each independently selected from H, deuterium, F, Cl, Br, I, $C_1$-$C_3$ alkyl, and $C_1$-$C_3$ alkoxy; or $R_6$ and $R_7$, together with the carbon atoms to which they are attached, form

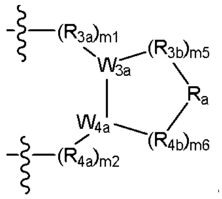

or

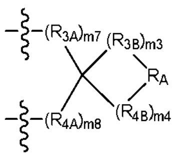
,

and $R_3$, $R_4$, and $R_5$ are each independently selected from H, deuterium, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl$_l$, $C_4$-$C_{10}$ heterocyclyl, aryl, and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, aryl, and heteroaryl are each independently optionally substituted by one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, aryl, and heteroaryl; preferably, $R_3$, $R_4$, and $R_5$ are each independently selected from H, deuterium, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted by one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl; preferably, $R_3$, $R_4$, and $R_5$ are each independently selected from H, deuterium, halogen, $C_1$-$C_3$ alkyl, and $C_1$-$C_3$ alkoxy; preferably, $R_3$, $R_4$, and $R_5$ are each independently selected from H, deuterium, F, Cl, Br, I, $C_1$-$C_3$ alkyl, and $C_1$-$C_3$ alkoxy; and/or

each occurrence of $R_{3a}$, $R_{3b}$, $R_{4a}$, $R_{4b}$, $R_{3A}$, $R_{3B}$, $R_{4A}$, and $R_{4B}$ is independently selected from $C(R_{2A})_2$, $NR_{2A}$, and O; and/or

each occurrence of $W_{3a}$ and $W_{4a}$ is independently $C(R_{2A})_2$ or N; and/or

each occurrence of $R_a$ and $R_A$ is independently $NR_{2A}$ or $CR^{2h}R^{3h}$; preferably, $R_A$ and $R_a$ are -NH or $-NC(O)CH_3$; and/or

each occurrence of $R_{2A}$ and $R_{1s}$ is independently selected from H, halogen, deuterium, $C_1$-$C_6$ alkyl, $C_1$-$C_6$ alkoxy, hydroxyl, cycloalkyl, $C_1$-$C_6$ haloalkyl, and hydroxyalkyl; preferably, each occurrence of $R_{2A}$ and $R_{1s}$ is independently selected from H, F, Cl, Br, I, $C_1$-$C_3$ alkyl, $C_1$-$C_3$ alkoxy, $C_3$-$C_6$ cycloalkyl, $C_1$-$C_3$ haloalkyl, hydroxyl, and $C_1$-$C_6$ hydroxyalkyl.

**4.** The compound according to any one of claims 1 to 3, wherein the compound is selected from the following structures:

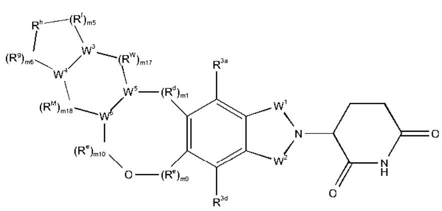,

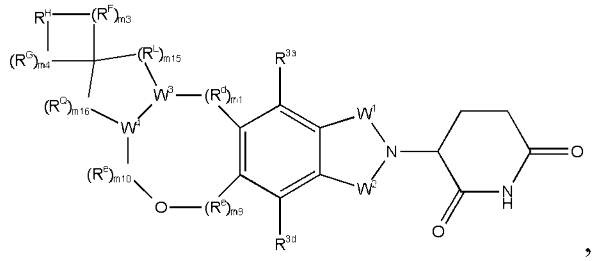,

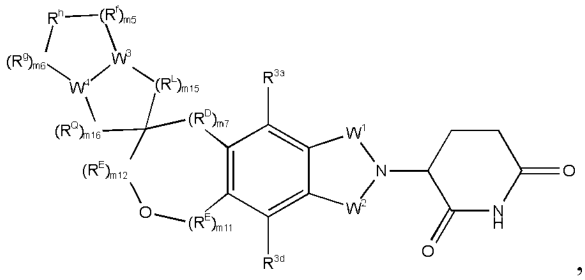,

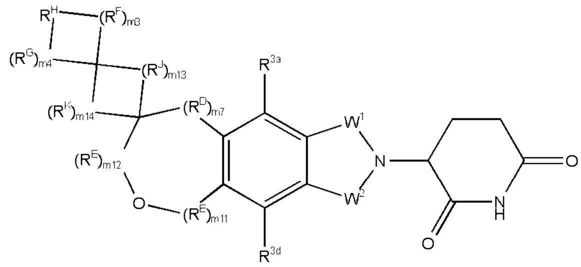

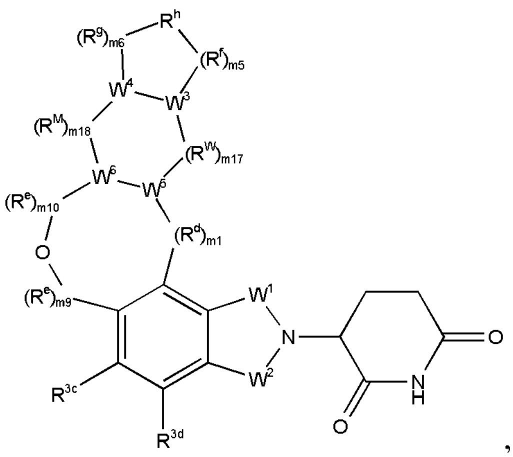,

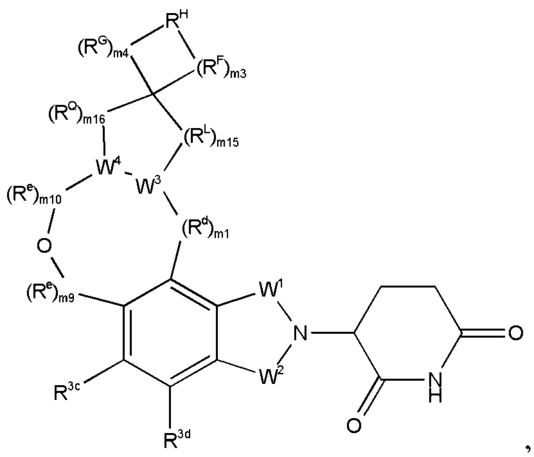,

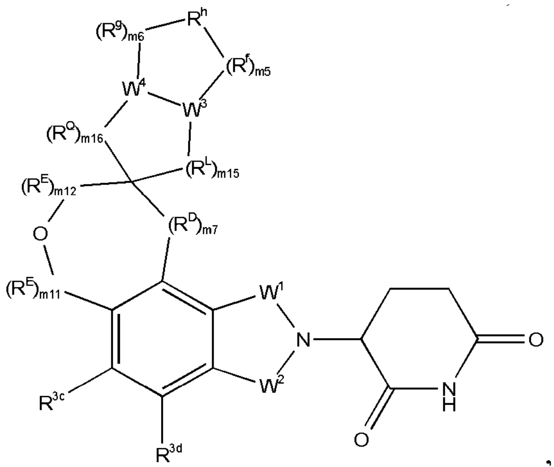

,

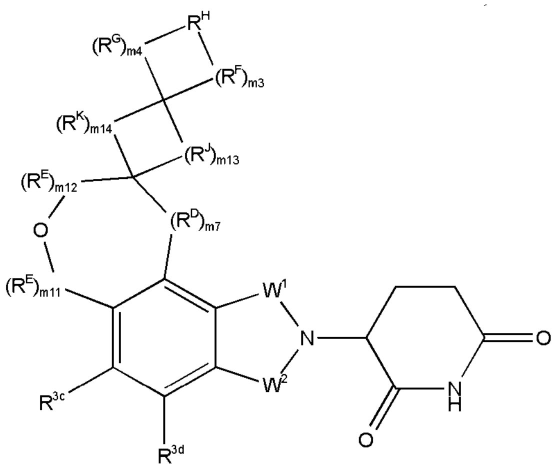

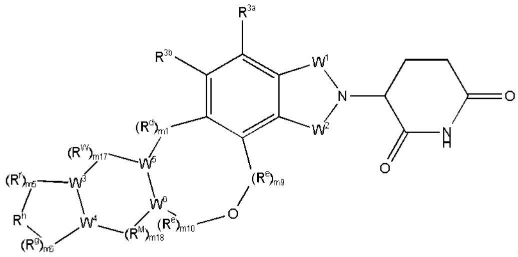

,

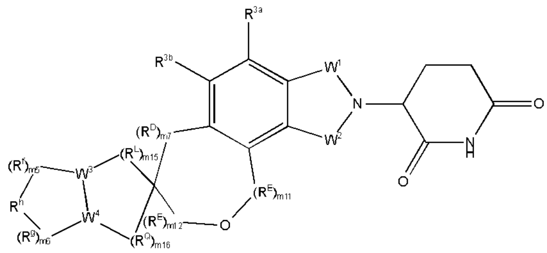

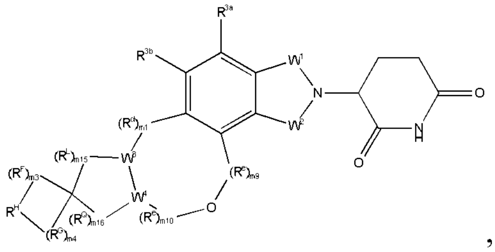

,

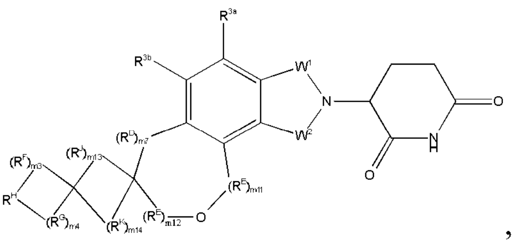

,

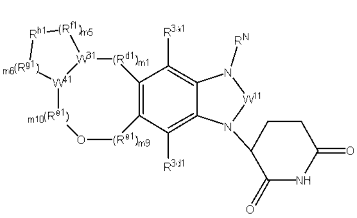

,

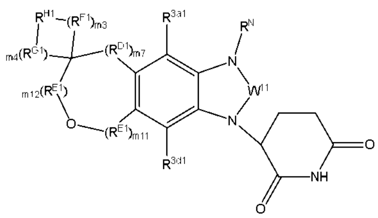

,

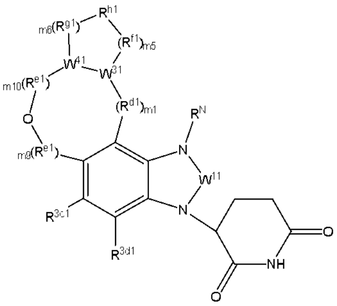

,

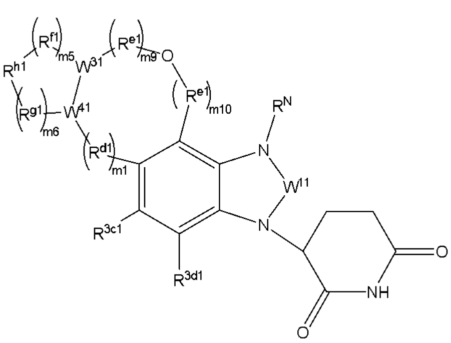

,

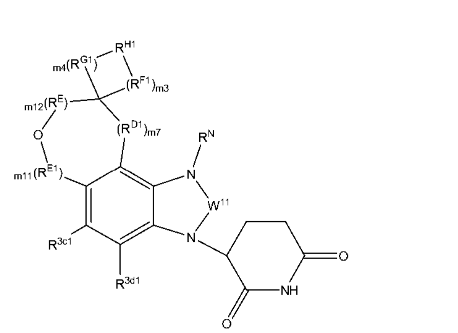

,

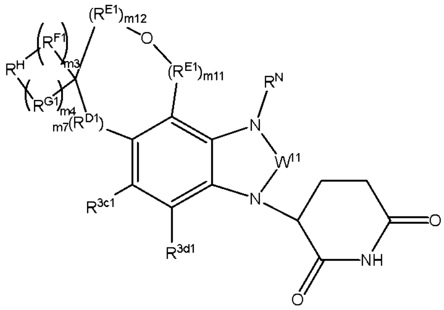,
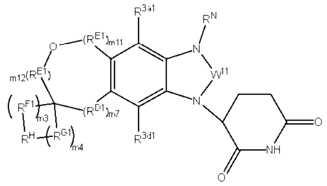,
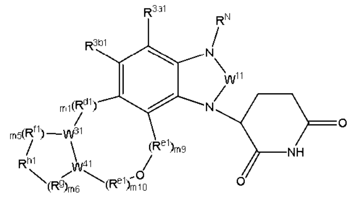,

and

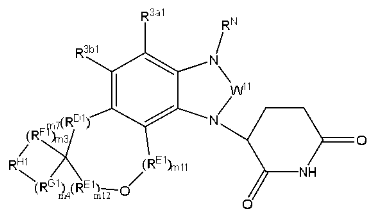;

wherein:

$R^{H1}$, $R^H$, $R^h$, $R^f$, $R^g$, $R^W$, $R^M$, $W^3$, $W^4$, $W^5$, $W^6$, $R^d$, $R^e$, $R^{3a}$, $R^{3b}$, $R^{3c}$, $R^{3d}$, $W^1$, $W^2$, $R^F$, $R^G$, $R^J$, $R^K$, $R^L$, $R^Q$, $R^{f1}$, $R^{g1}$, $W^{31}$, $W^{41}$, $R^{d1}$, $R^{e1}$, $R^{3a1}$, $R^{3b1}$, $R^{3c1}$, $R^{3d1}$, $W^{11}$, m3, m4, $R^{E1}$, $R^{D1}$, $R^{G1}$, $R^{F1}$, m2, m5, m6, m8, m13, m14, m15, m16, m17, m18, $W^{11}$, $W^{31}$, $W^{41}$, $R^N$, and $R^{h1}$ are respectively as defined in claim 1, 2, or 3;
each occurrence of m1, m9, and m10 is independently an integer of 0, 1, 2, 3, 4, or 5, and $m1 + m9 + m10 \leq 5$; preferably, each occurrence of m1, m9, and m10 is independently an integer of 0, 1, or 2, and $m1 + m9 + m10 \leq 2$; preferably, $m1 + m9 + m10 = 1$ or $m1 + m9 + m10 = 0$; and
each occurrence of m7, m11, and m12 is independently an integer of 0, 1, 2, 3, 4, 5, or 6, and $m7 + m11 + m12 \leq 6$; preferably, each occurrence of m7, m11, and m12 is independently an integer of 0, 1, 2, or 3, and $m7 + m11 + m12 \leq 3$; preferably, $m7 + m11 + m12 = 2$ or $m7 + m11 + m12 = 1$.

**5.** The compound according to any one of claims 1 to 3, wherein the compound is selected from the following structures:

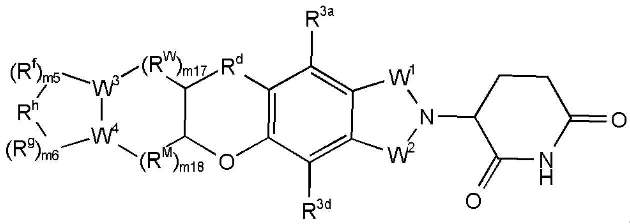,
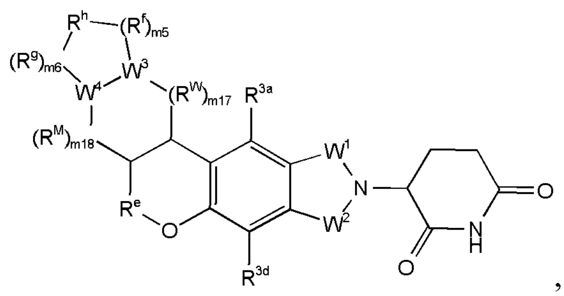,

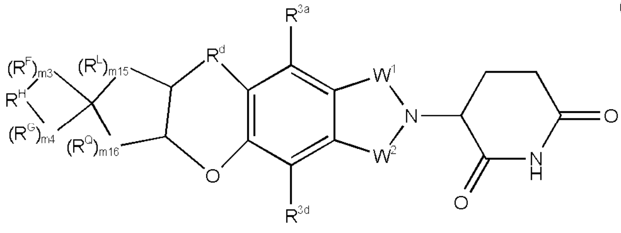
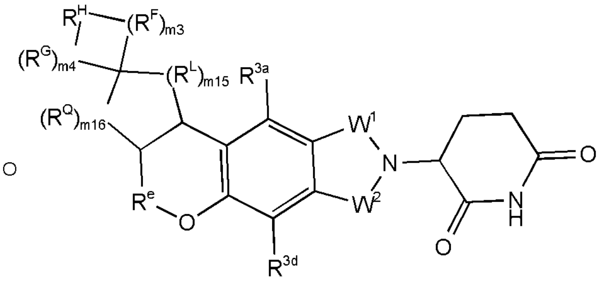

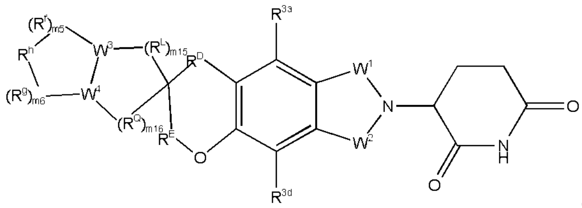
,
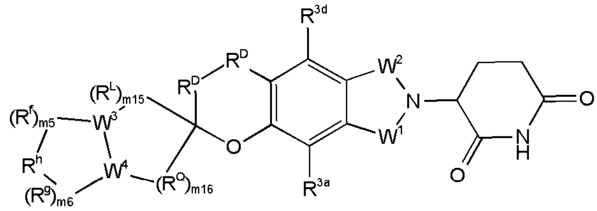
,

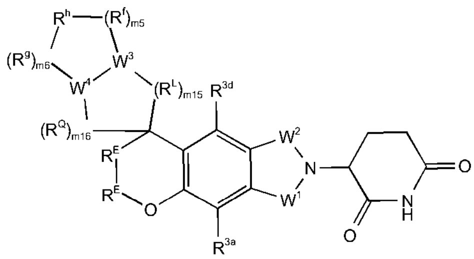
,
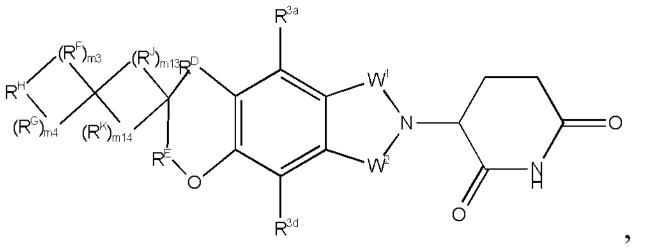
,

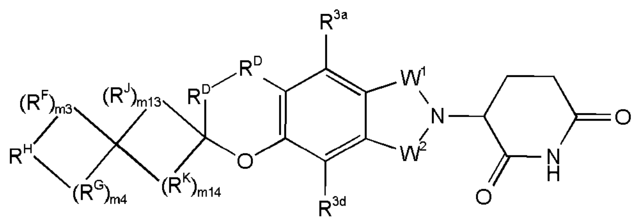
,
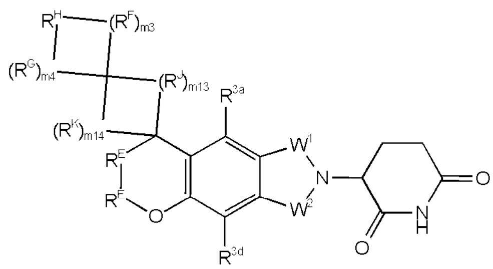

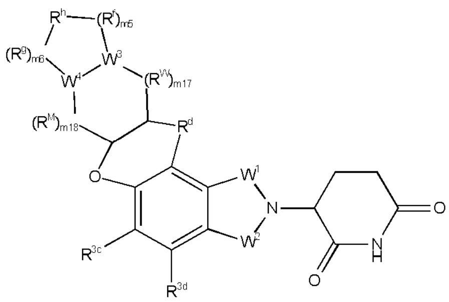
,
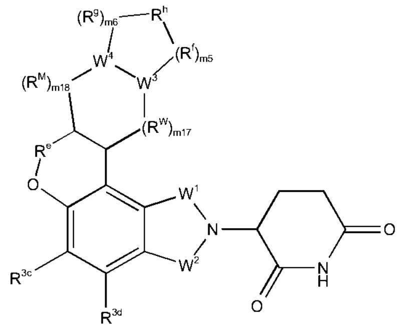
,

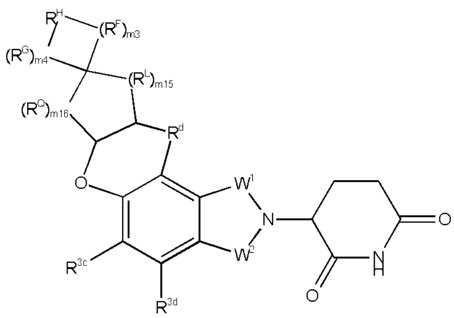
,
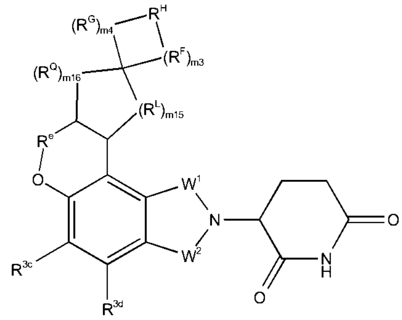
,

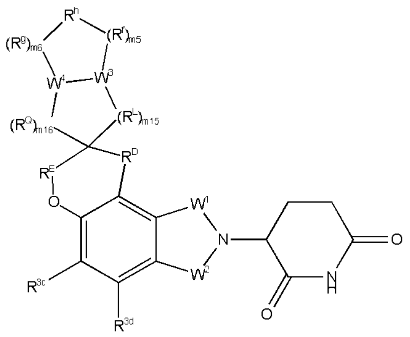

,

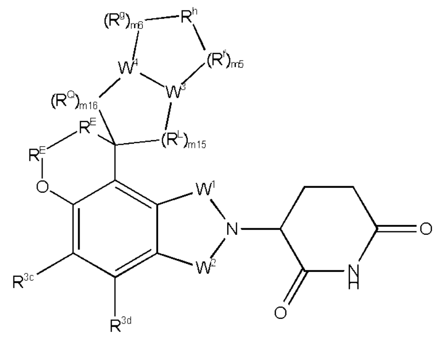

,

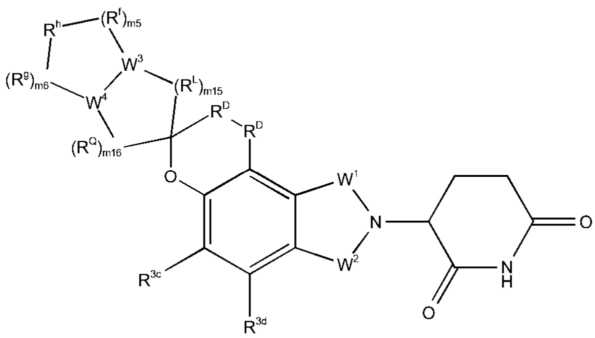

,

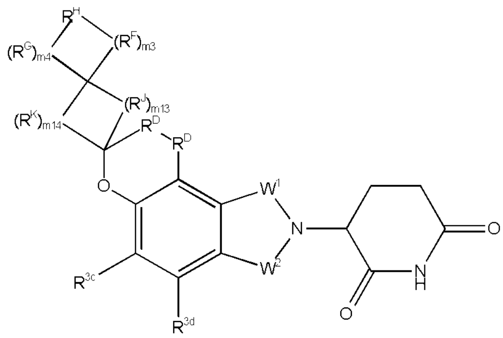

,

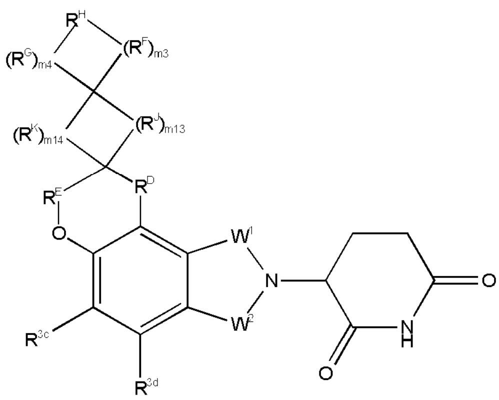

,

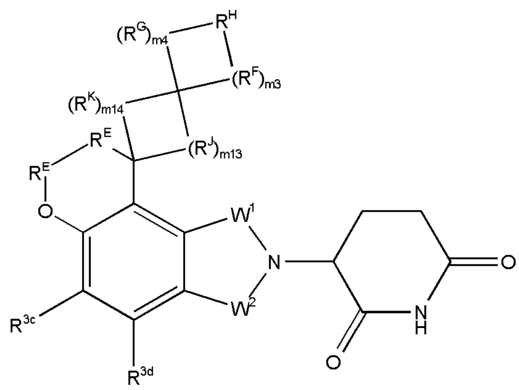

,

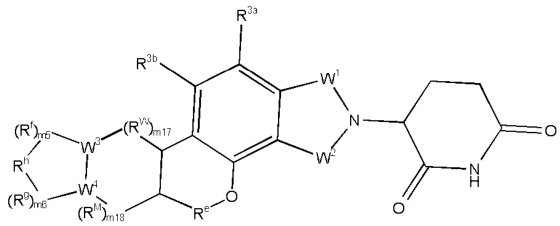 , 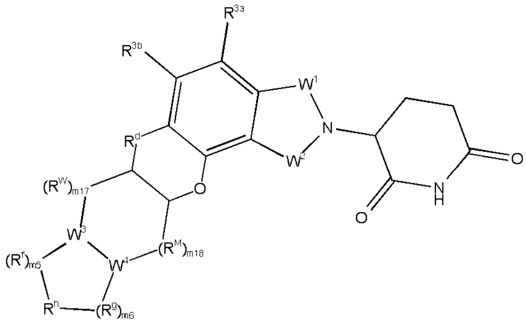 ,

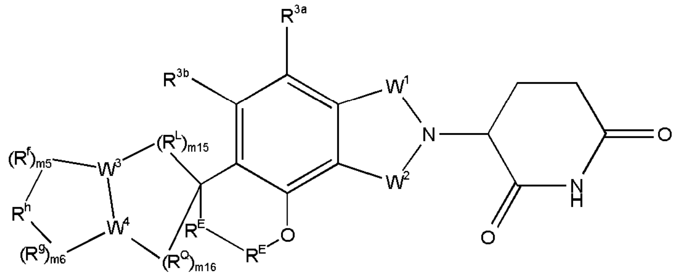 , 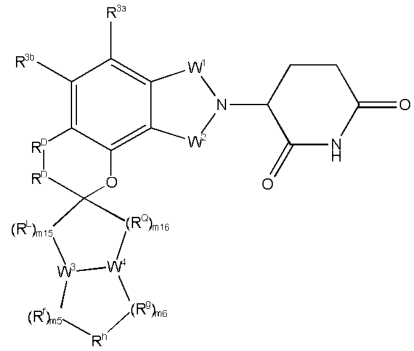 ,

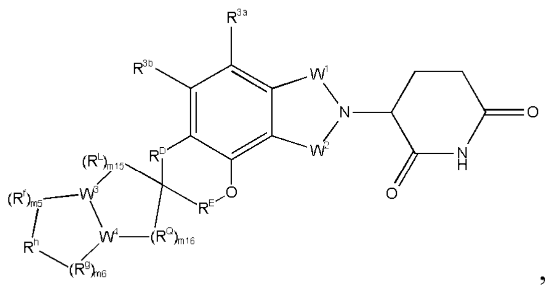 , 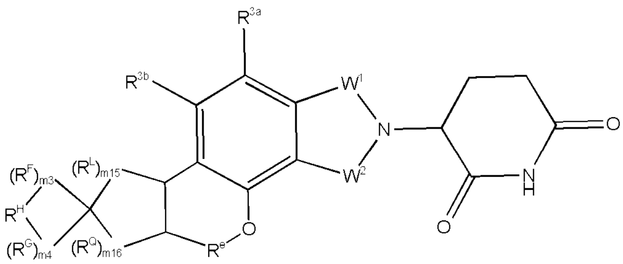 ,

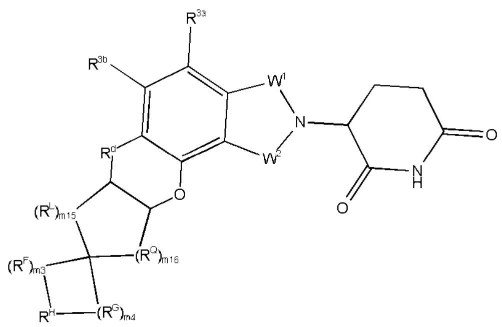 , 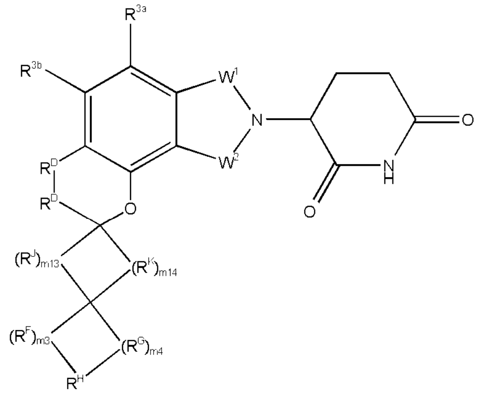 ,

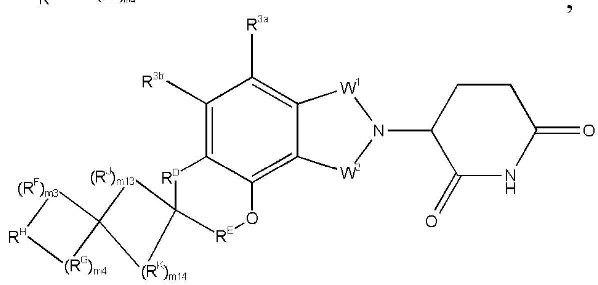 , 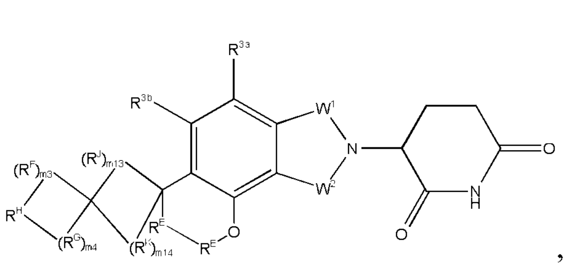 ,

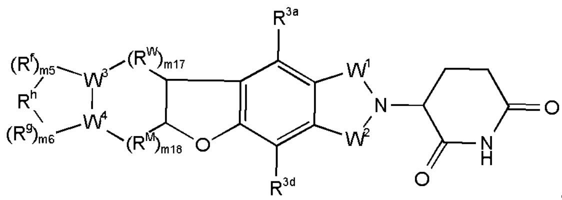
,
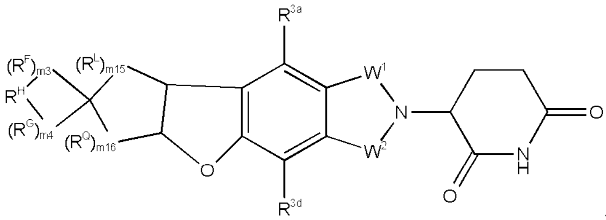
,
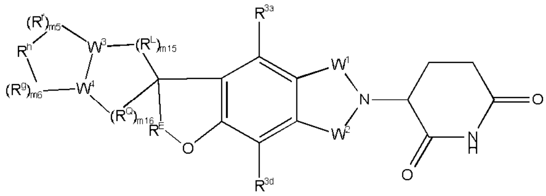
,
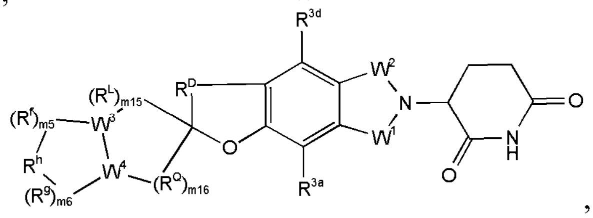
,
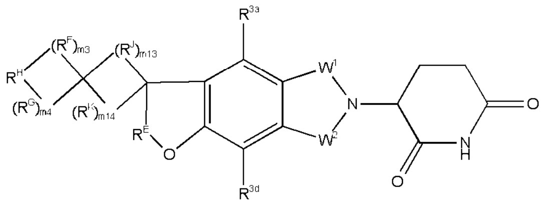
,
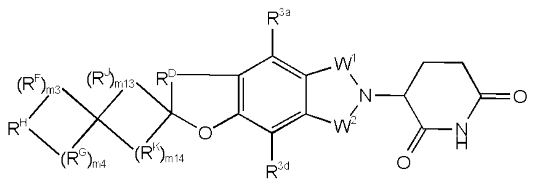
,
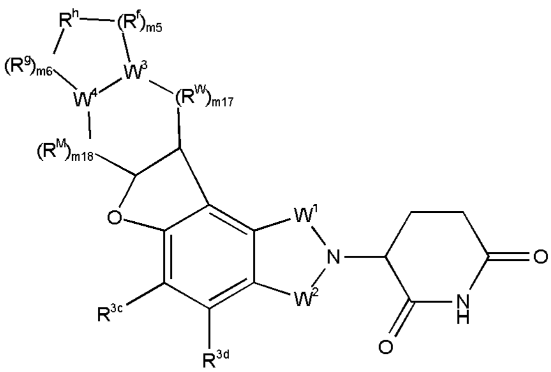
,
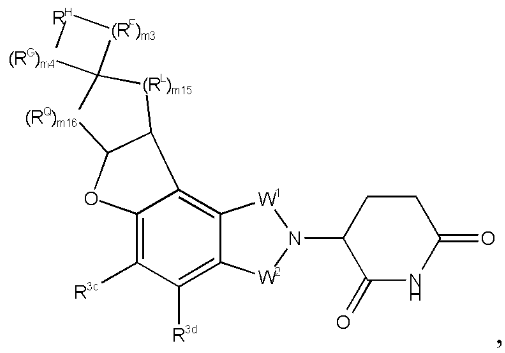
,
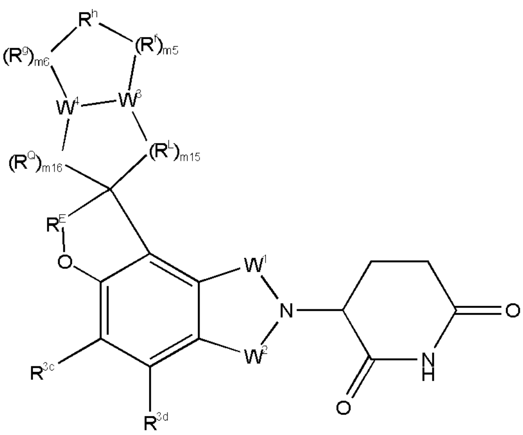
,
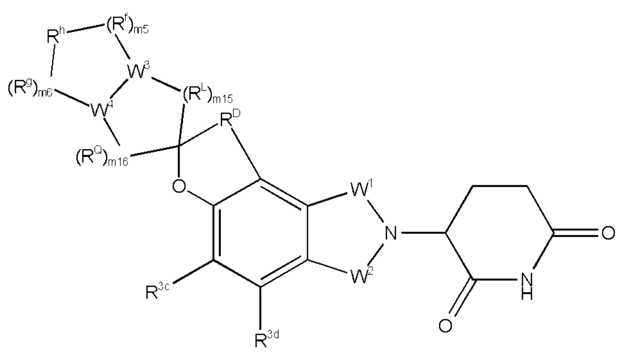
,

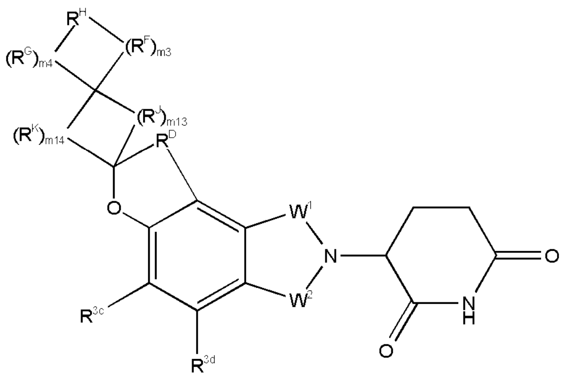,

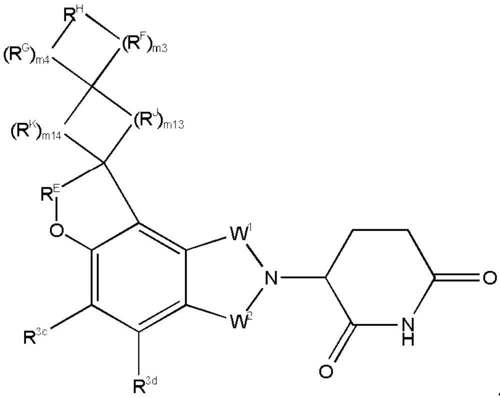,

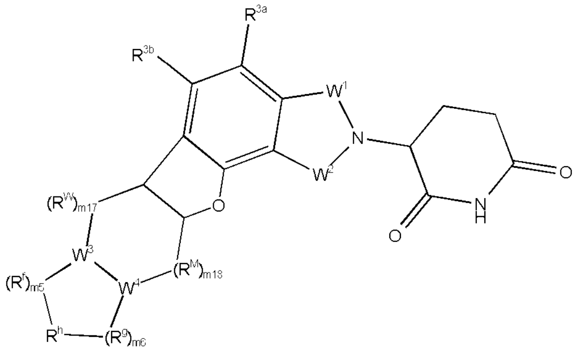,

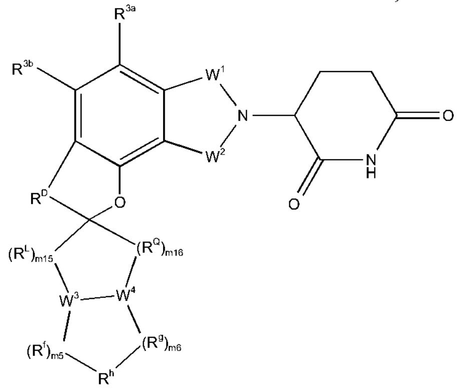,

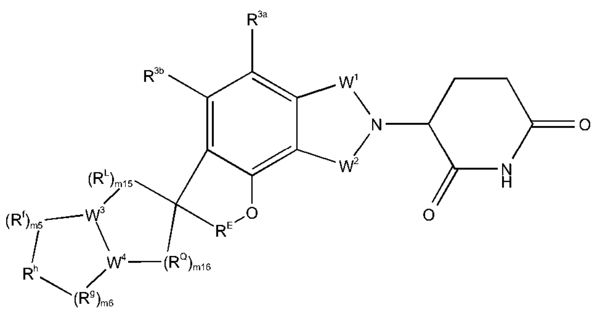,

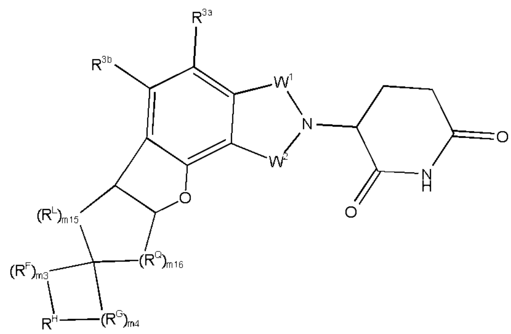,

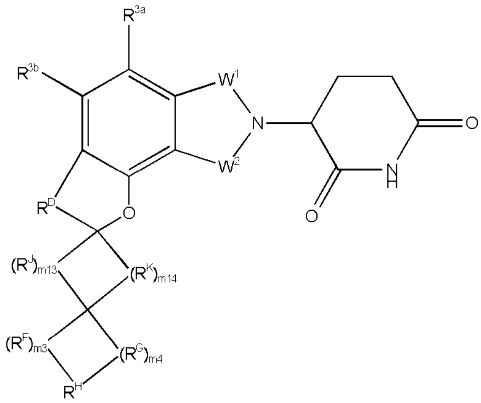,

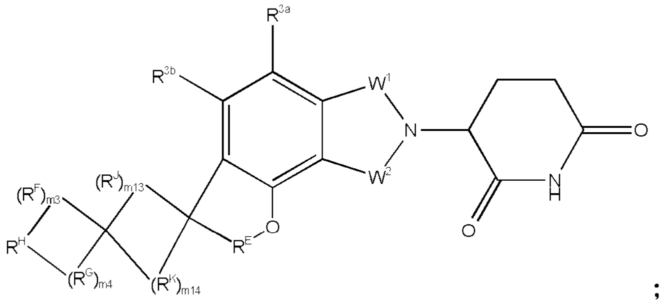;

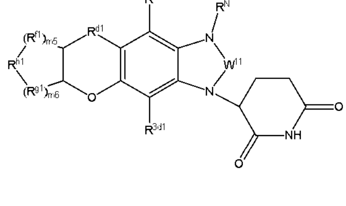,

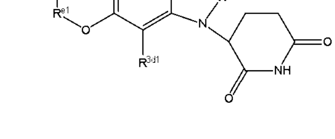,

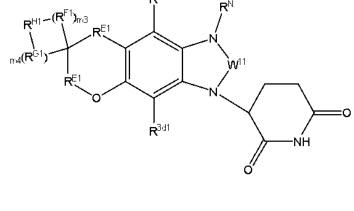,

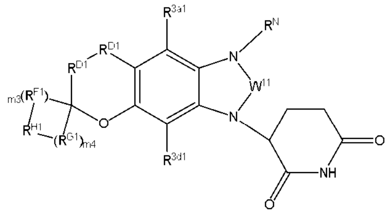 , 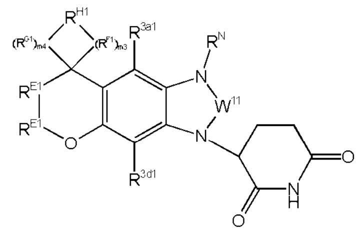 , 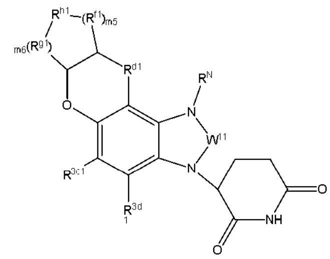 ,

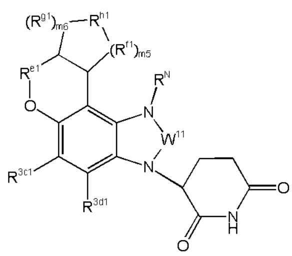 , 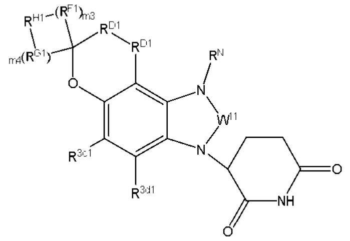 , 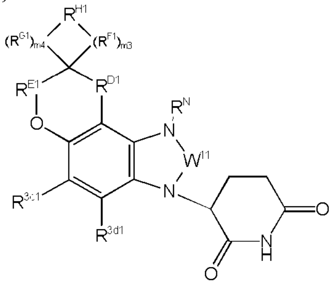 ,

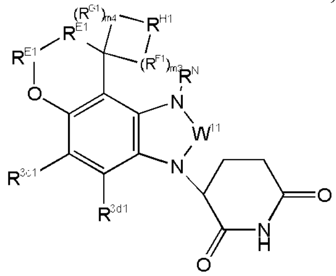 , 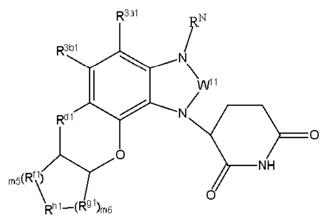 , 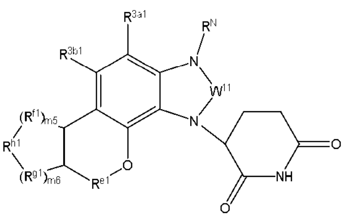 ,

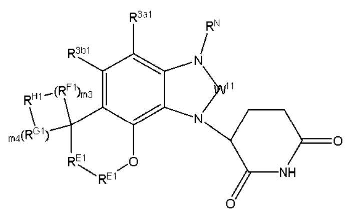 , 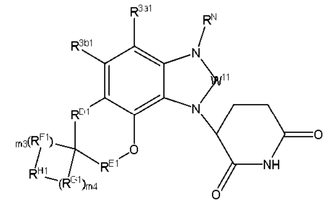 , 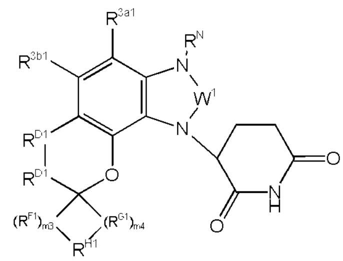 ,

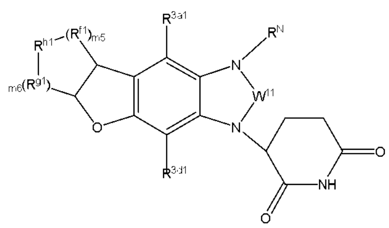 , 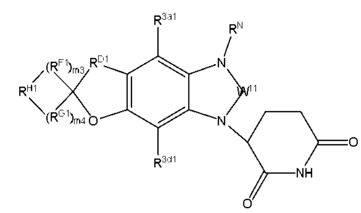 , 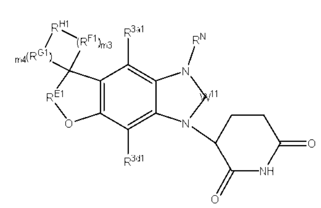 ,

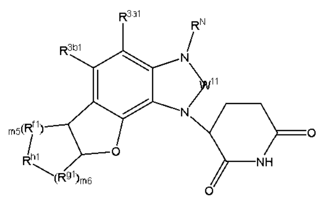 , 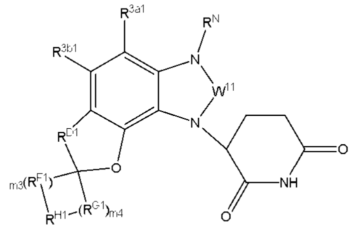 , 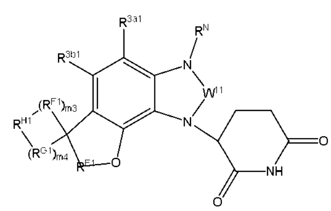 ,

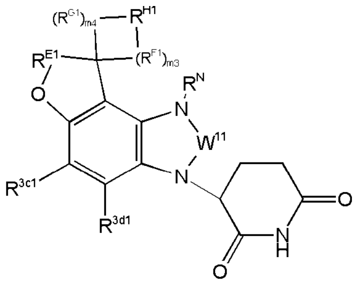
,
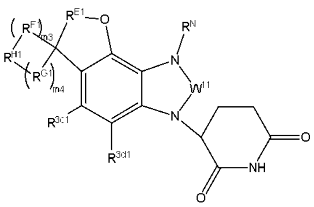
,
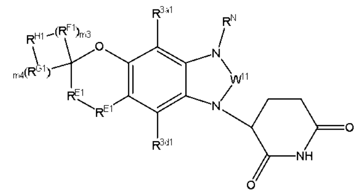
,
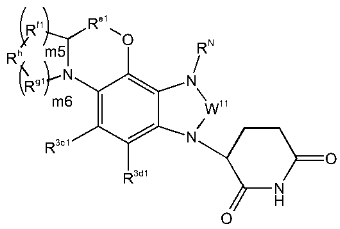
,
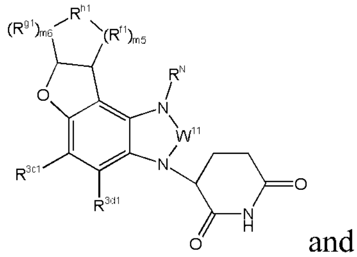
and
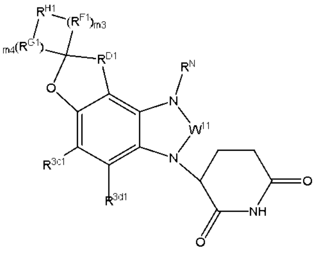
,
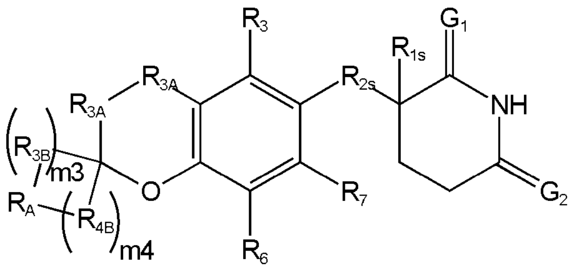
,
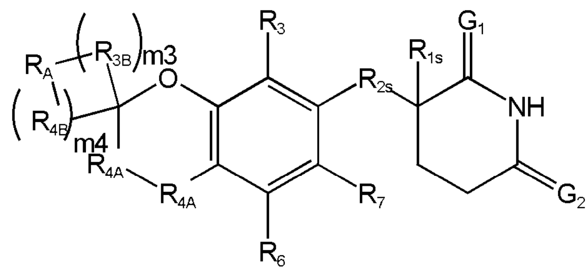
,
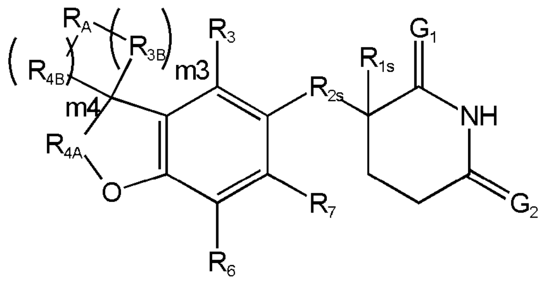
,
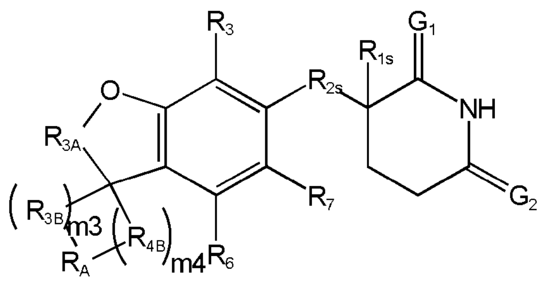
,
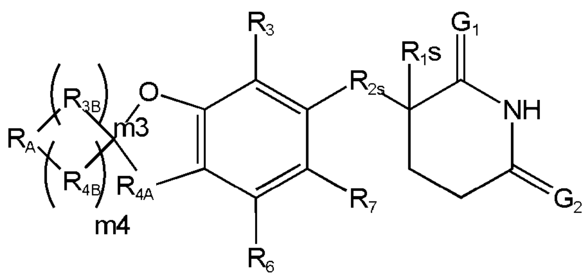
,
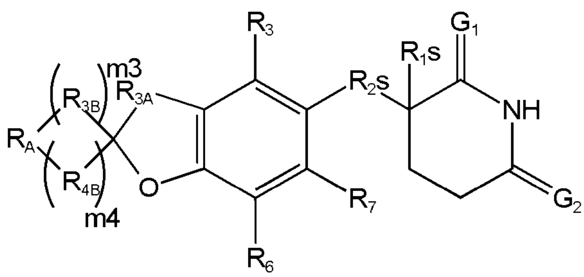
,

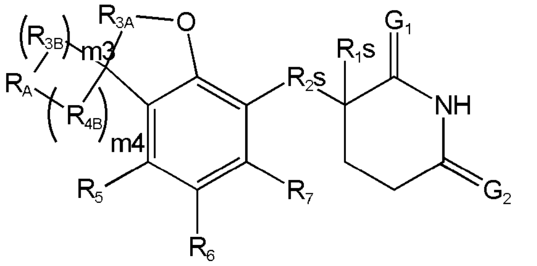
,
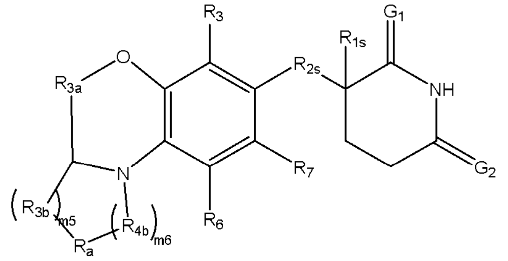
,
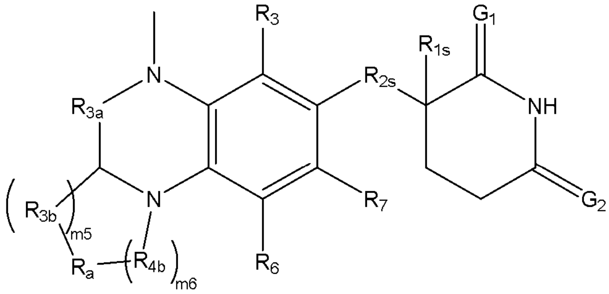
,
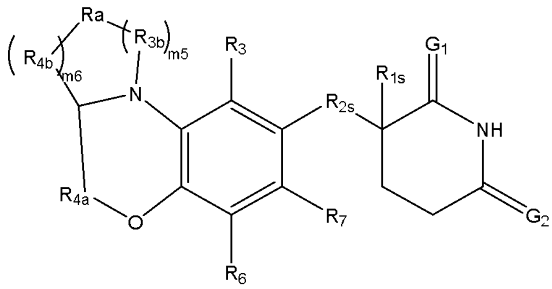
,
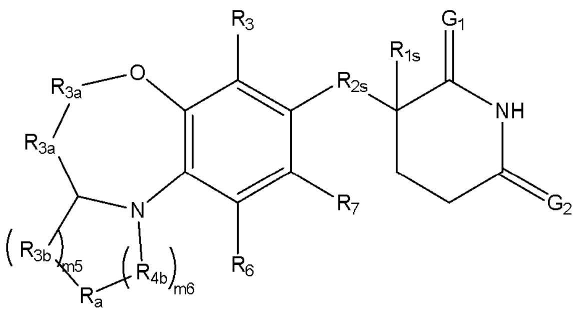
,
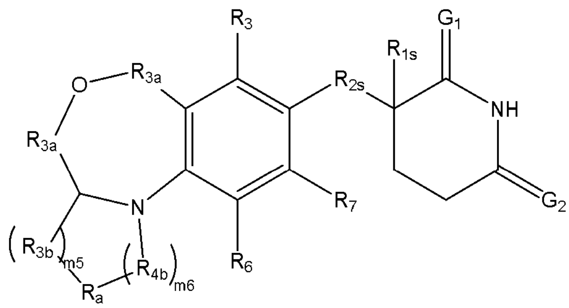
,
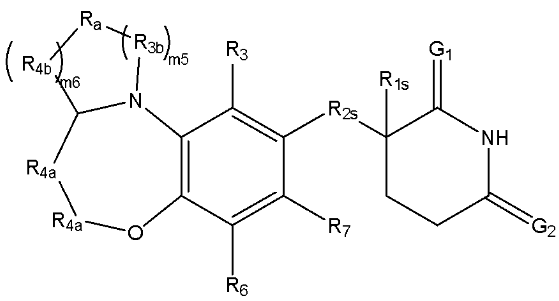
,
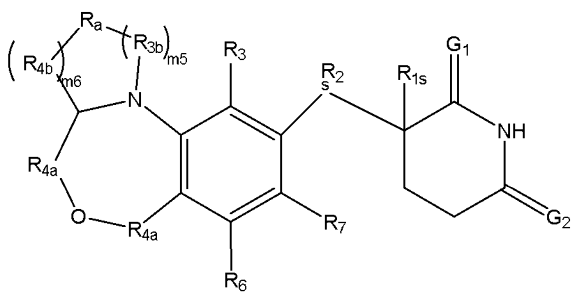
;

$R^{H1}$, $R^{H}$, $R^{h}$, $R^{f}$, $R^{g}$, $R^{W}$, $R^{M}$, $W^{3}$, $W^{4}$, $W^{5}$, $W^{6}$, $R^{d}$, $R^{e}$, $R^{3a}$, $R^{3b}$, $R^{3c}$, $R^{3d}$, $W^{1}$, $W^{2}$, $R^{F}$, $R^{G}$, $R^{J}$, $R^{K}$, $R^{L}$, $R^{Q}$, $R^{f1}$, $R^{g1}$, $W^{31}$, $W^{41}$, $R^{d1}$, $R^{e1}$, $R^{3a1}$, $R^{3b1}$, $R^{3c1}$, $R^{3d1}$, $W^{11}$, $R^{N}$, $G_1$, $G_2$, $R_{1s}$, $R_{2s}$, $R_3$, $R_4$, $R_5$, $R_6$, $R_{3A}$, $R_{4A}$, $R_{3B}$, $R_{4B}$, $R_{3a}$, $R_{4a}$, $R_{4b}$, $R_{4a}$, $R_a$, $R_b$, m3, m4, m5, m6, m13, m14, m15, m16, m17, m18, and $R^{h1}$ are respectively as defined in claim 1, 2, or 3.

**6.** The compound according to any one of claims 1 to 5, wherein the compound is selected from the following structures:

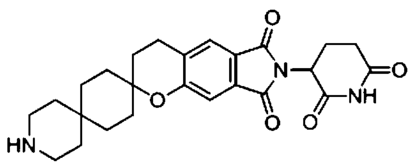
,
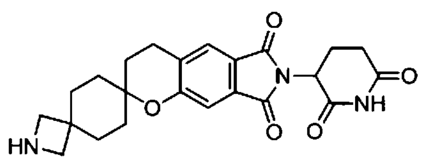
,

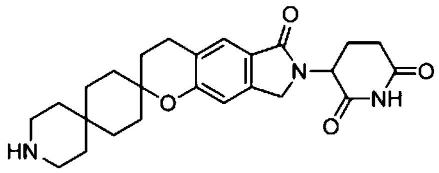,
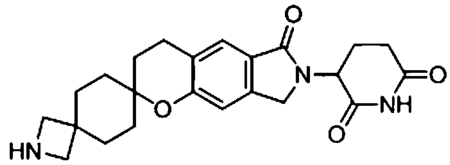,

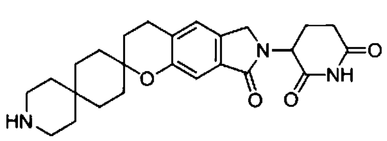,
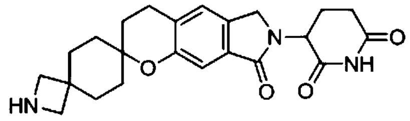,

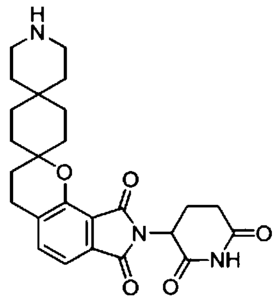,
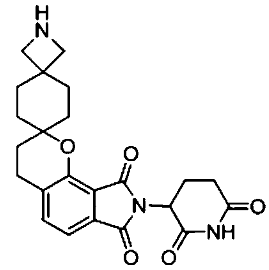,
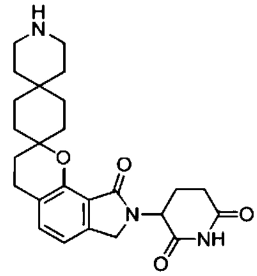,

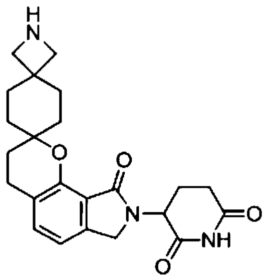,
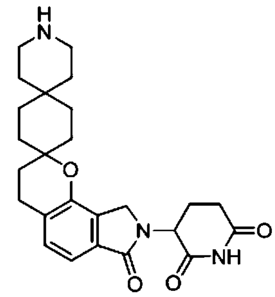,
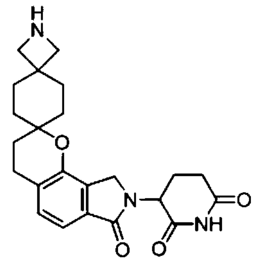,

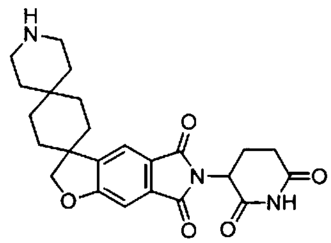,
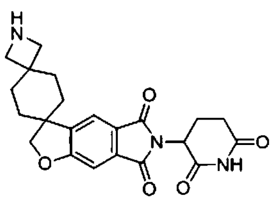,
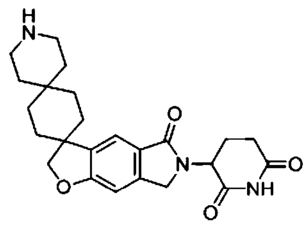,

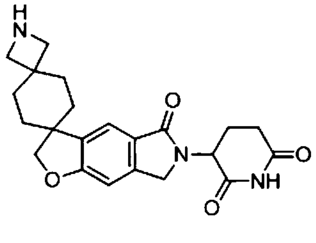,
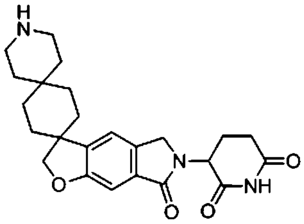,
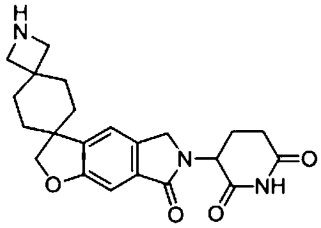,

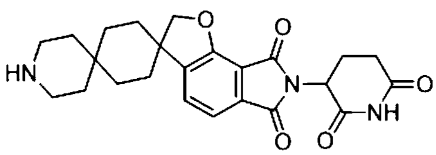 , 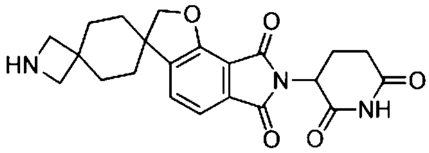 ,

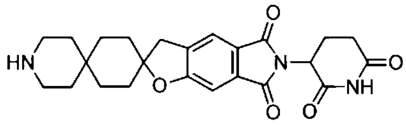 , 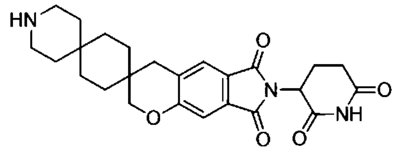 ,

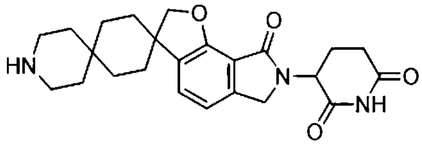 , 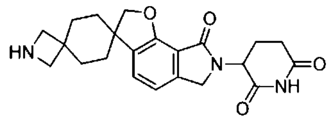 ,

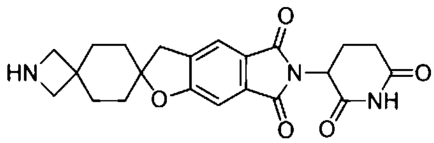 , 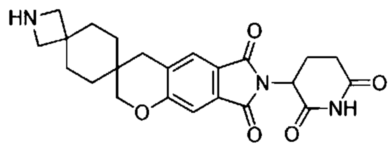 ,

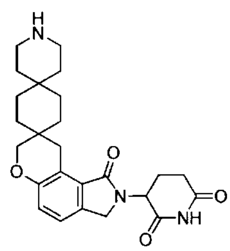 , 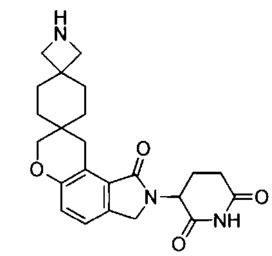 , 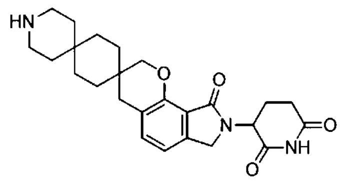 ,

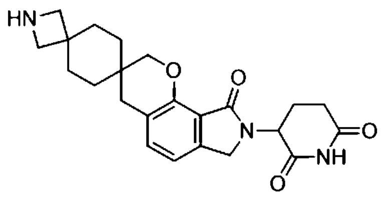,
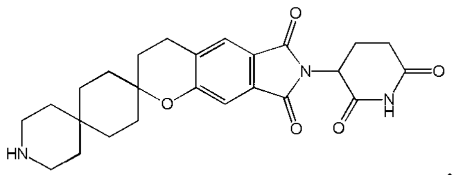,

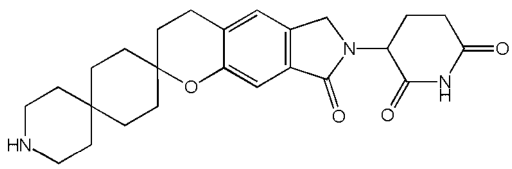,
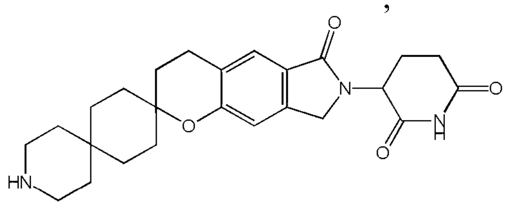,

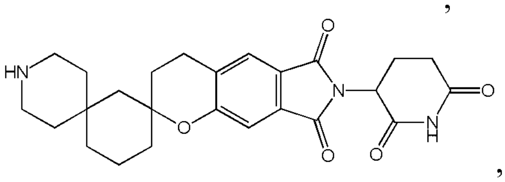,
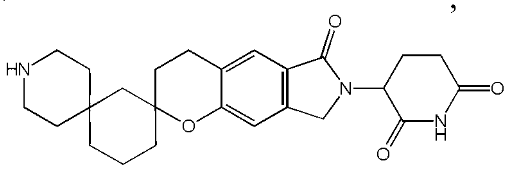,

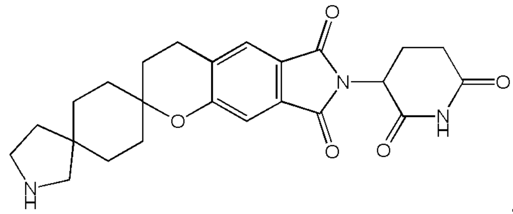,
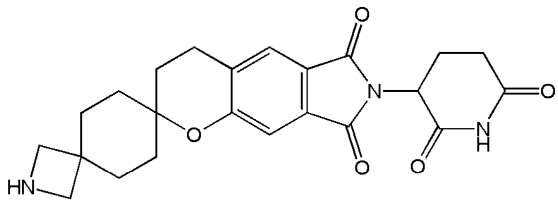,

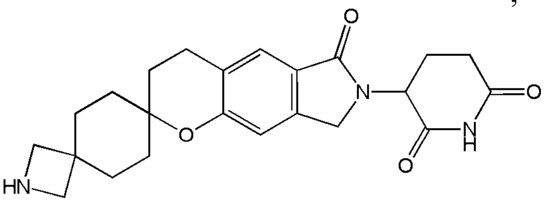,
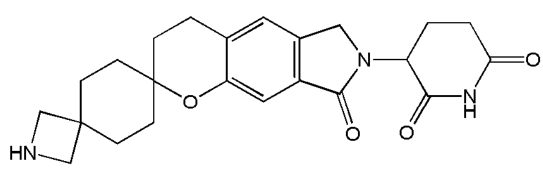,

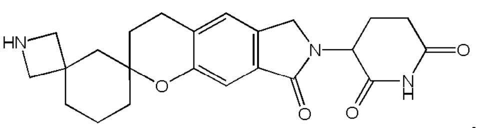,
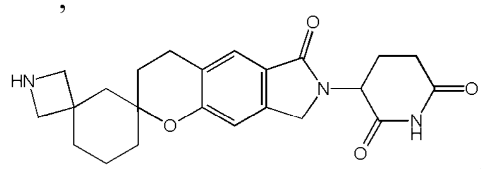,

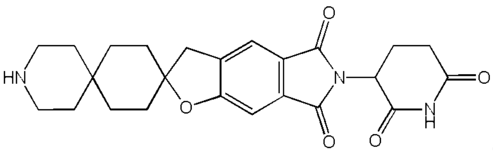,
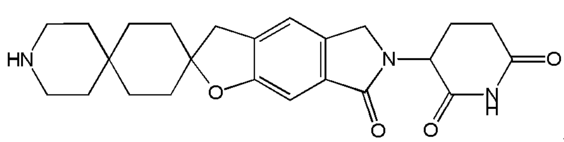,

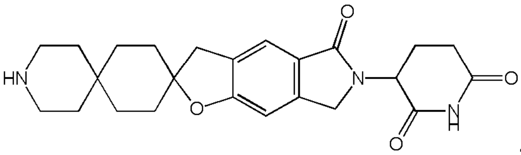,
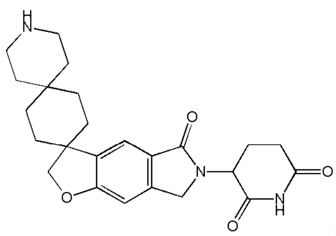,

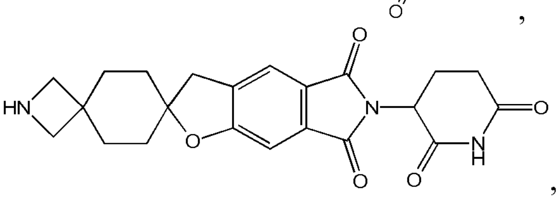,
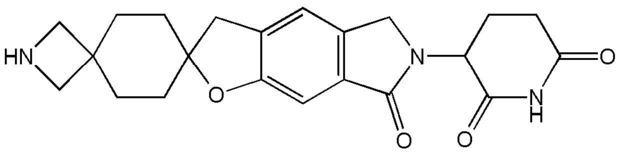,

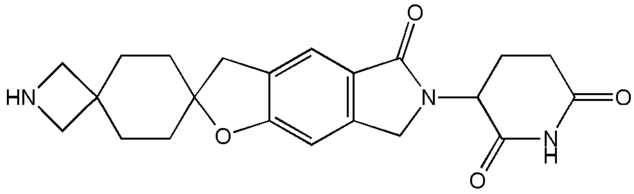,
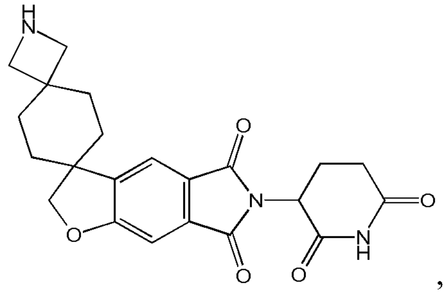,
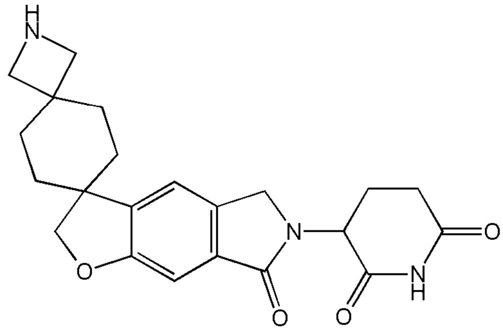,
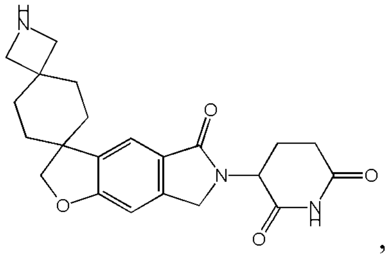,
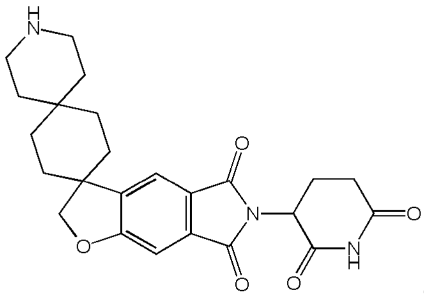,
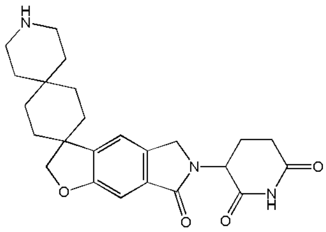,
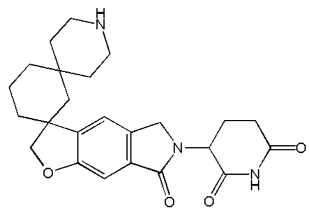,
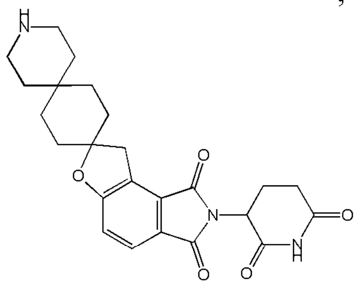,
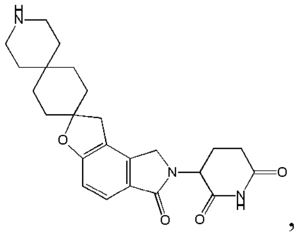,
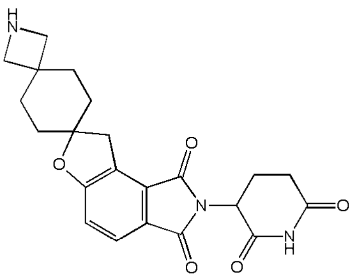,
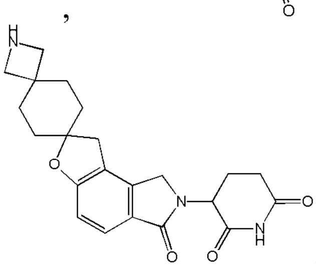,
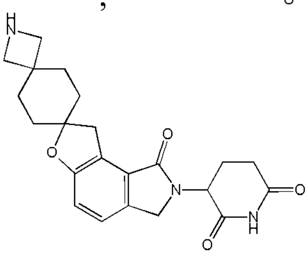,
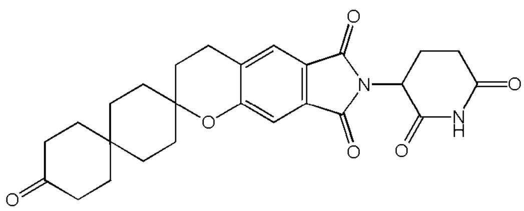,

,
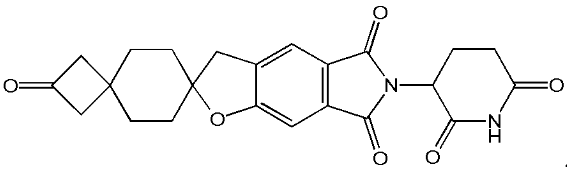
,
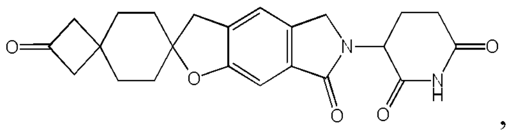
,

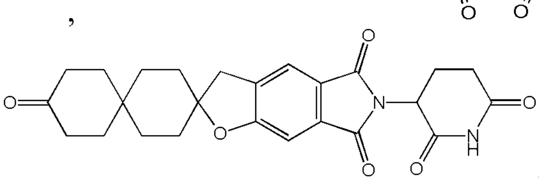
,
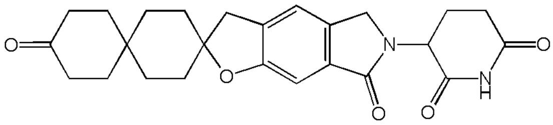
,

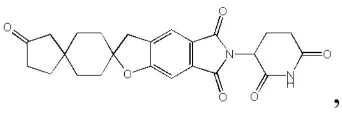
,
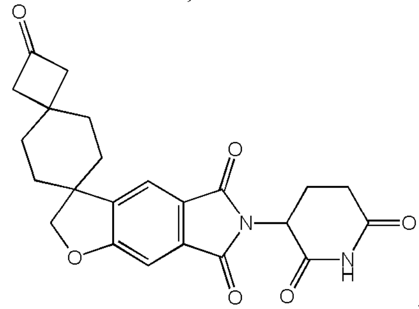
,
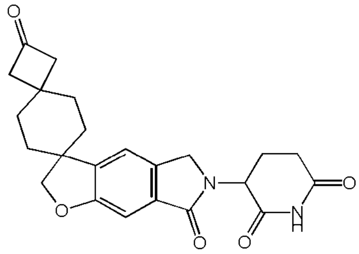
,

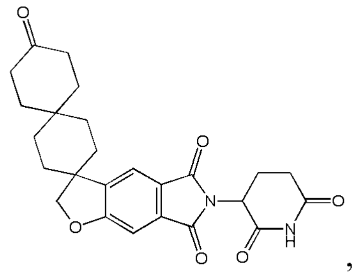
,
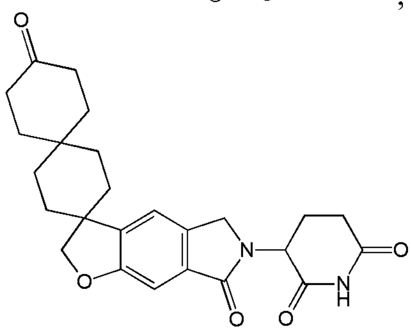
,
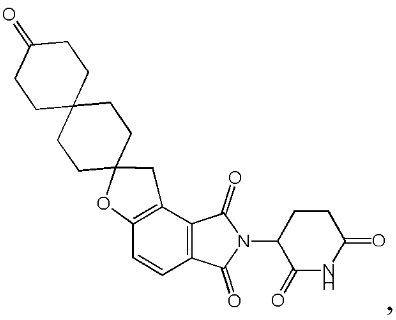
,

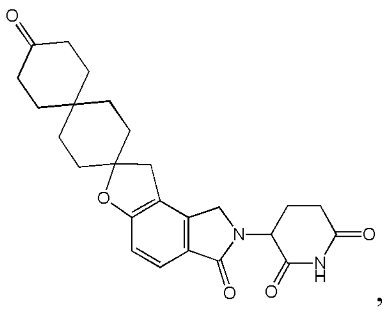
,
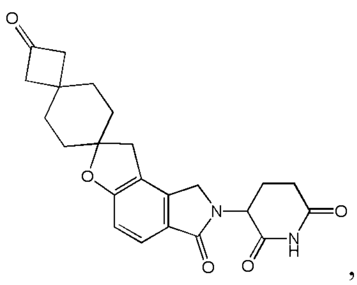
,
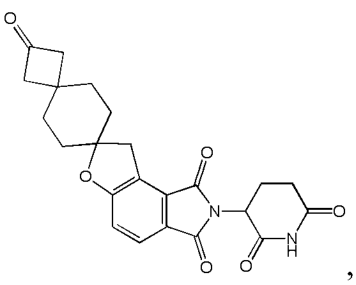
,

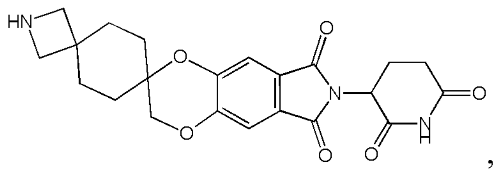
,
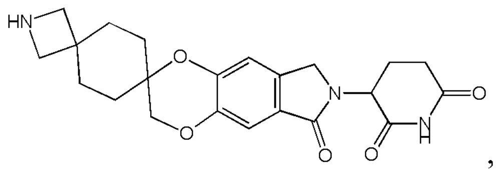
,

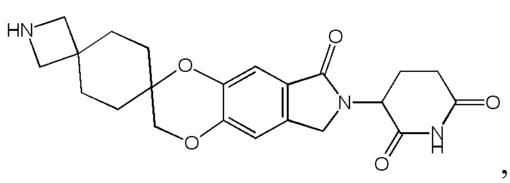
,
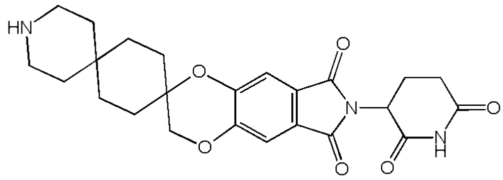
,

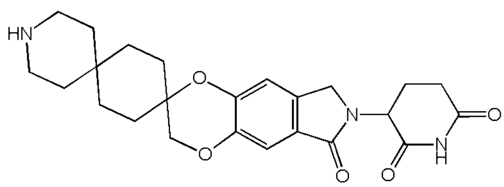 ,

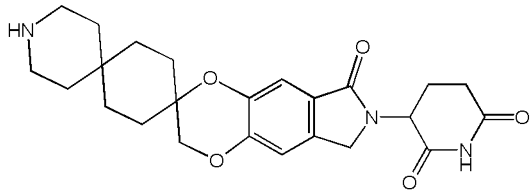 ,

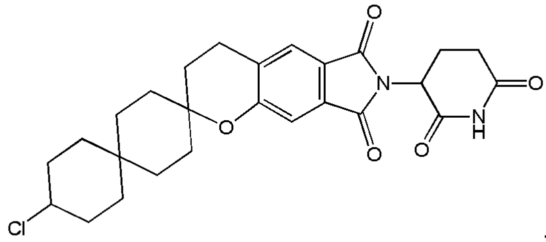 ,

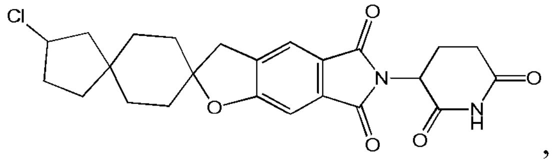 ,

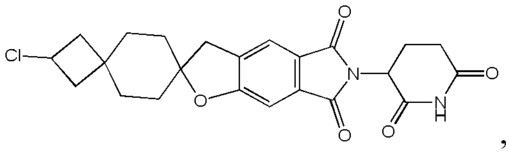 ,

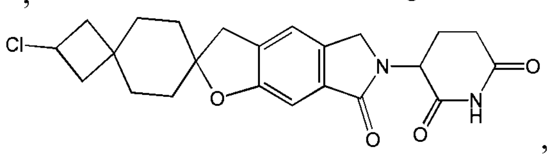 ,

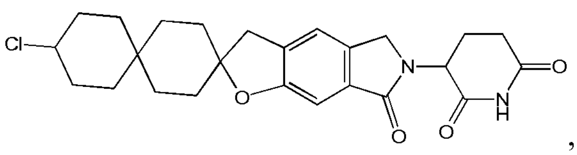 ,

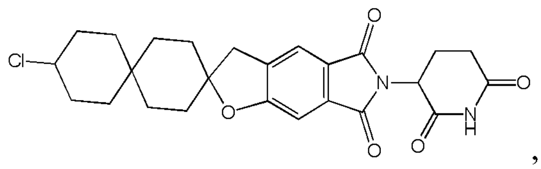 ,

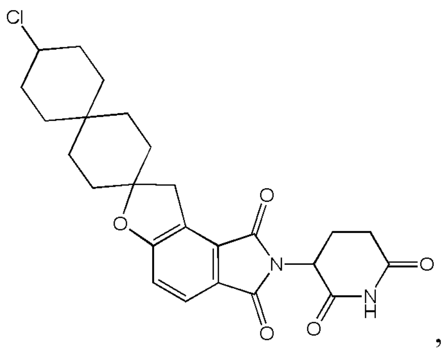 ,

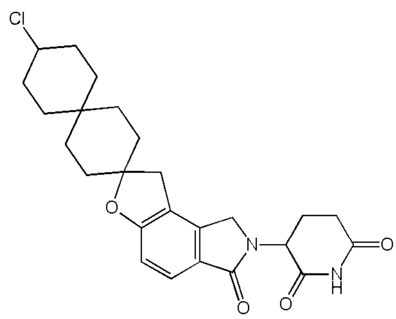 ,

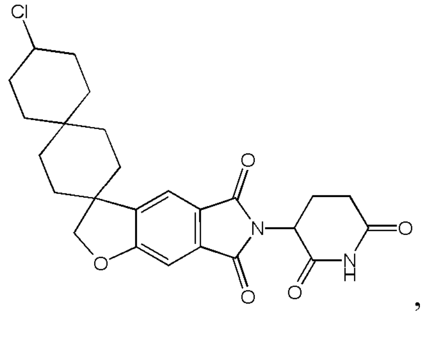 ,

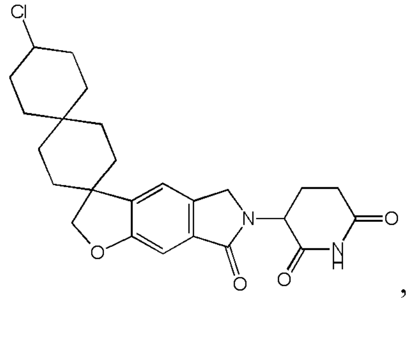 ,

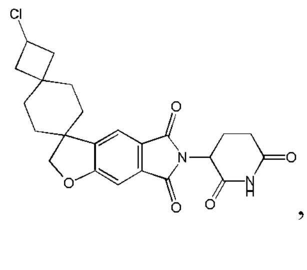 ,

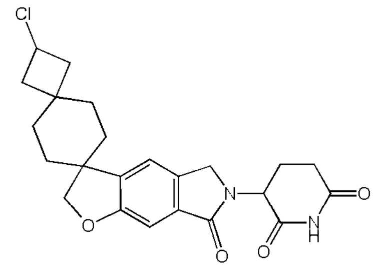 , 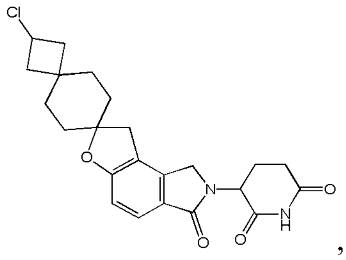 , 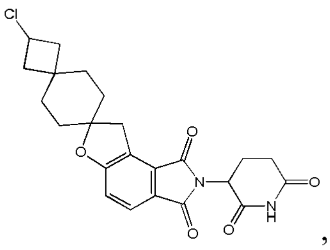 ,

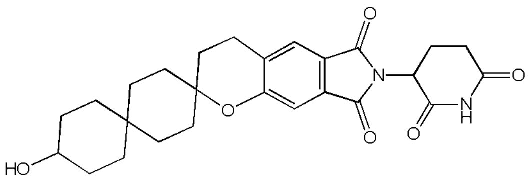 , 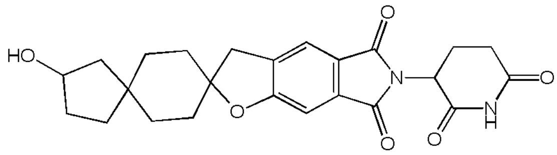 ,

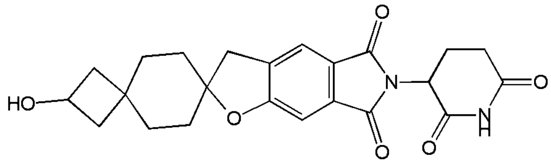 , 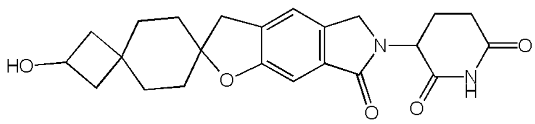 ,

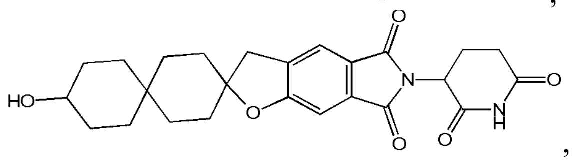 , 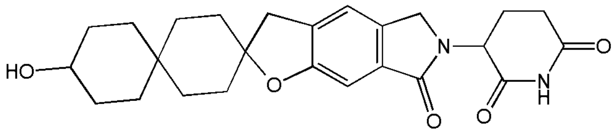 ,

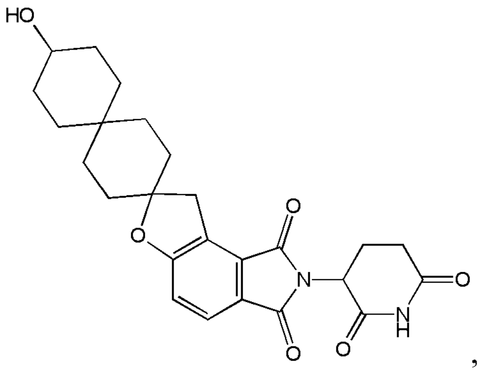 , 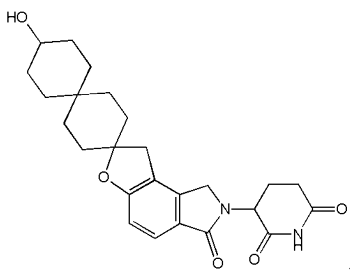 , 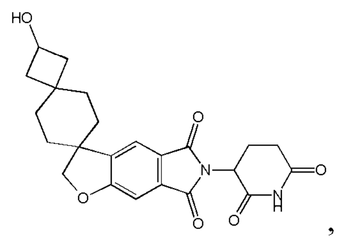 ,

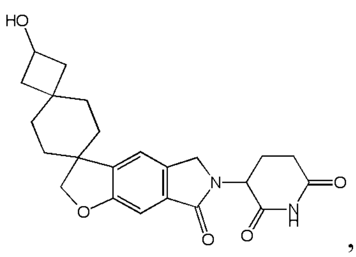 , 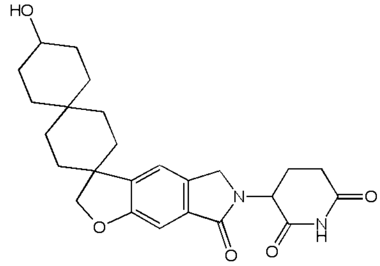 , 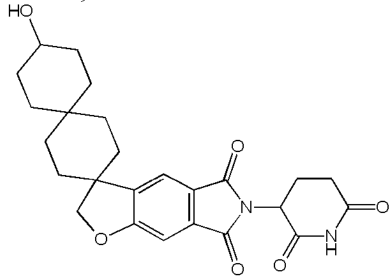 ,

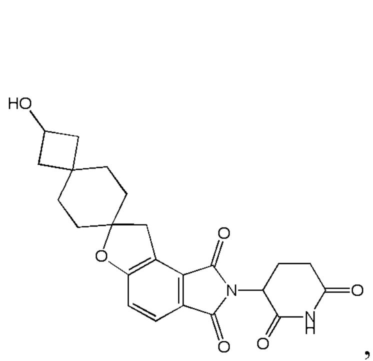,

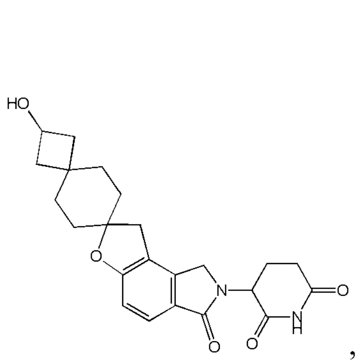,

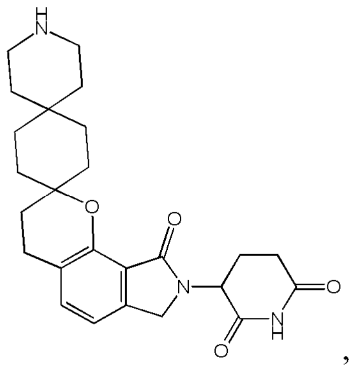,

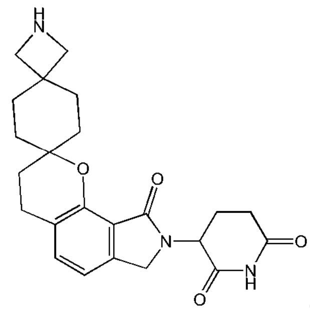,

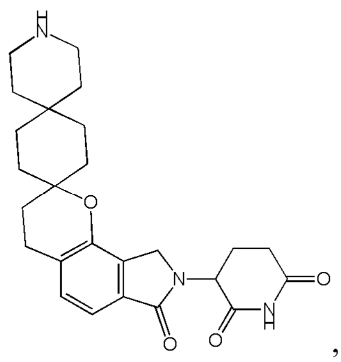,

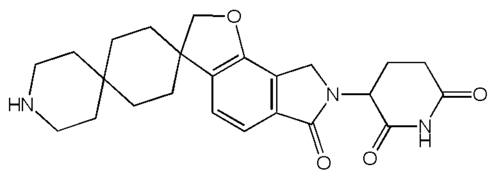,

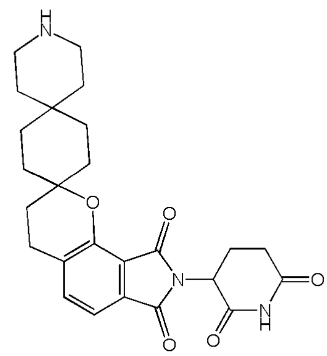,

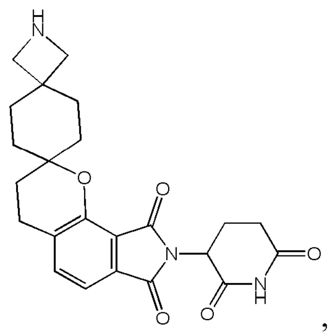,

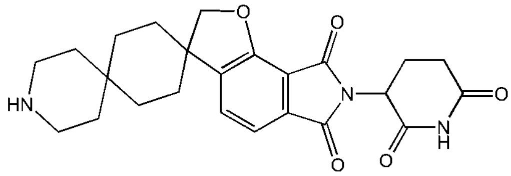,

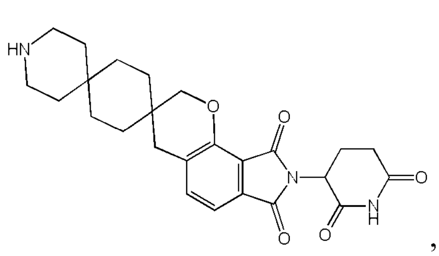,

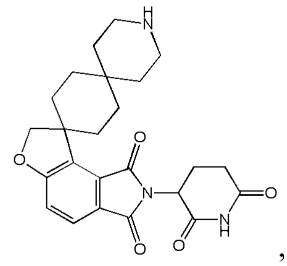,

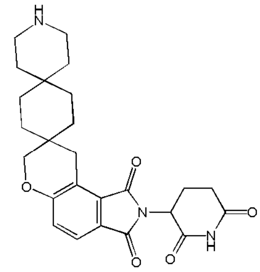,

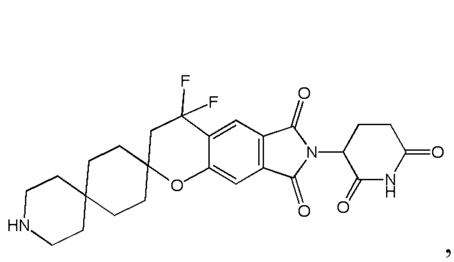,

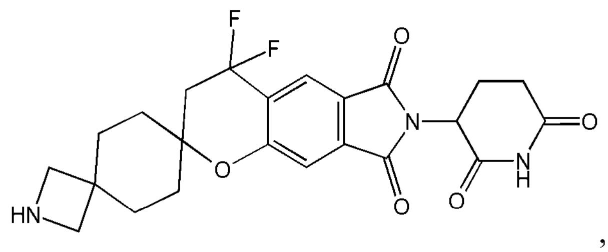,

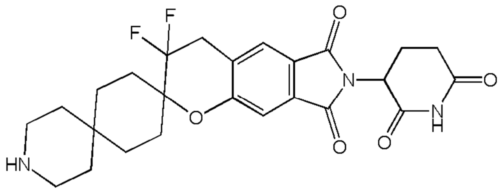,

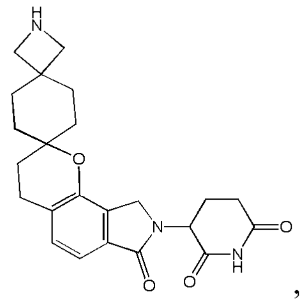,

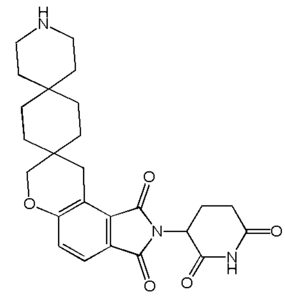,

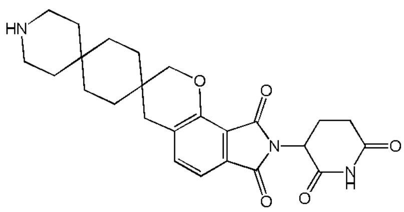,

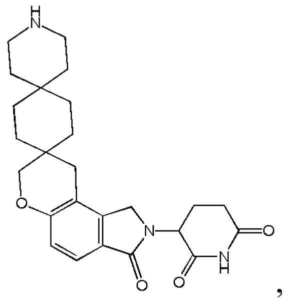,

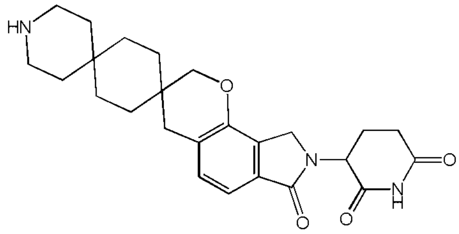,

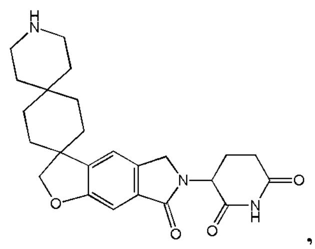,

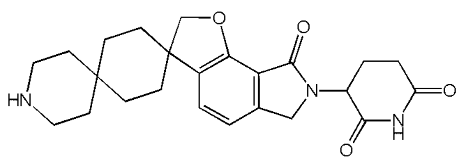,

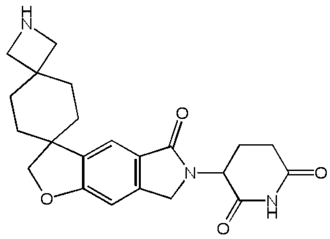,

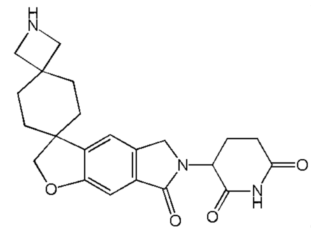,

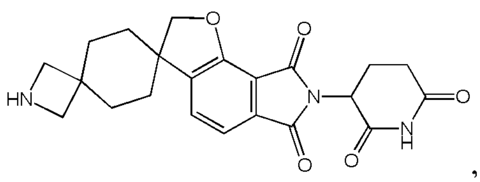,

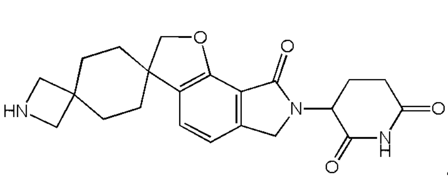,

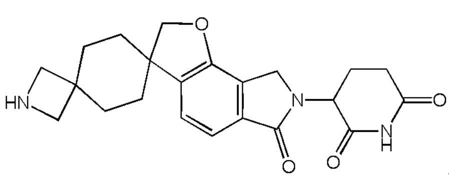,

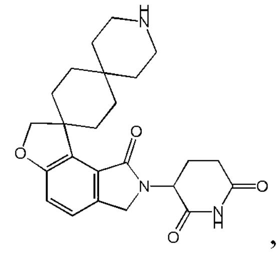,

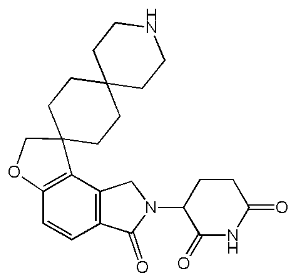,

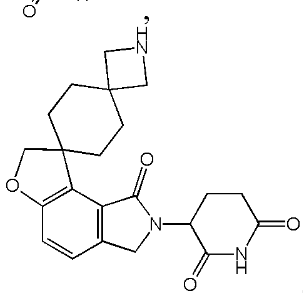,

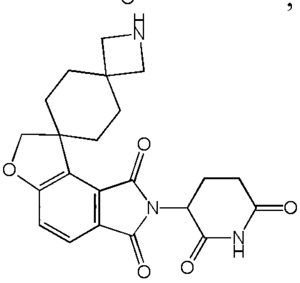

and

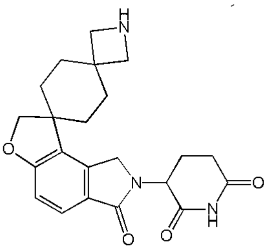,

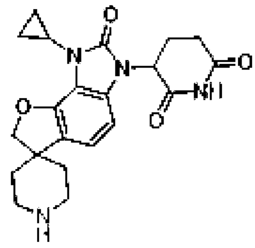

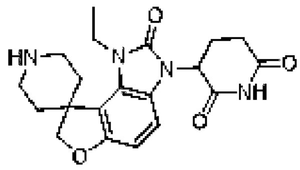

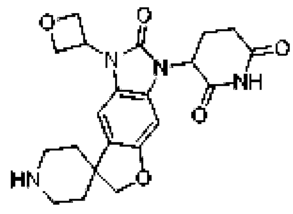

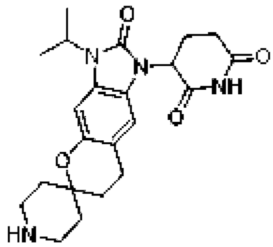

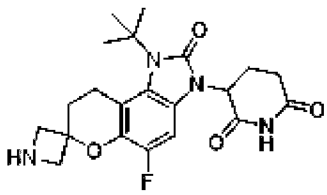

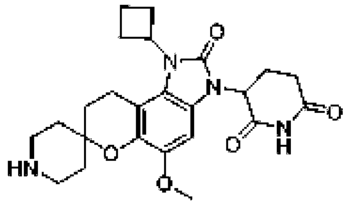

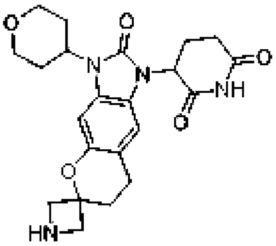

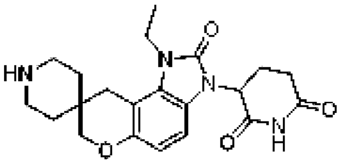

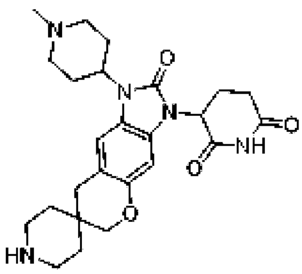

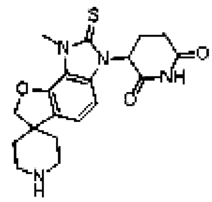

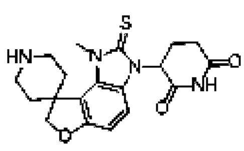

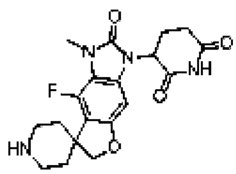

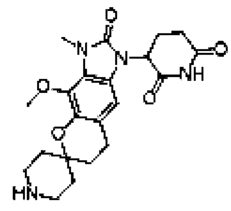

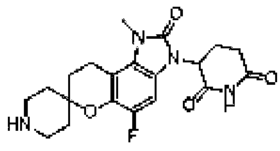

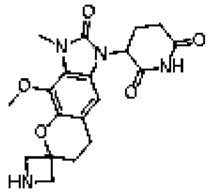

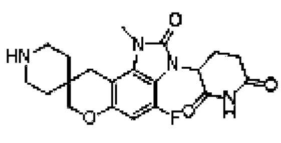

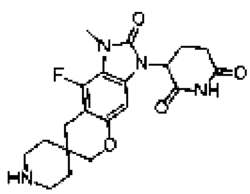

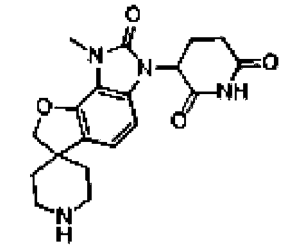,
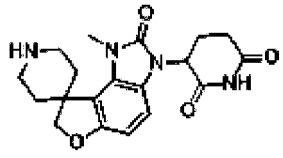,
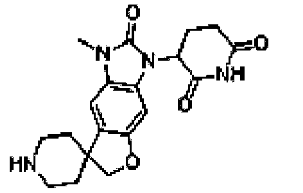,

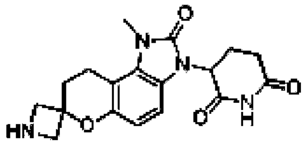,
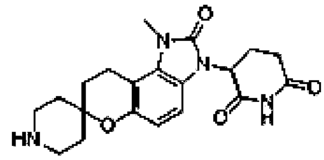,
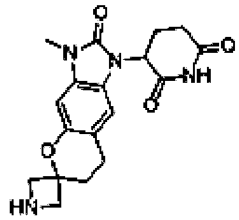,

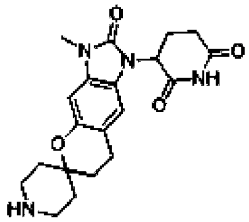,
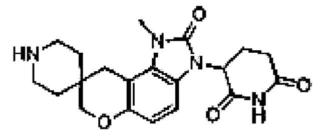,
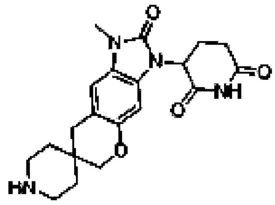,

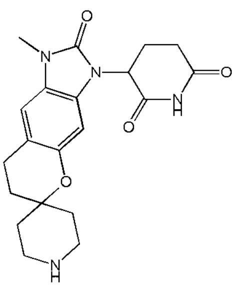,
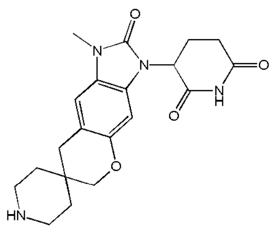,
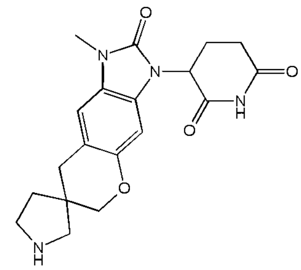,
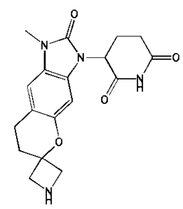,

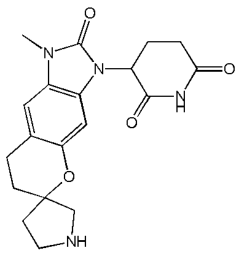,
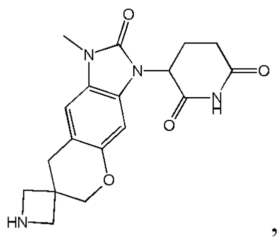,
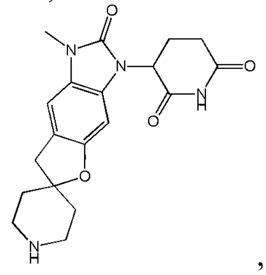,
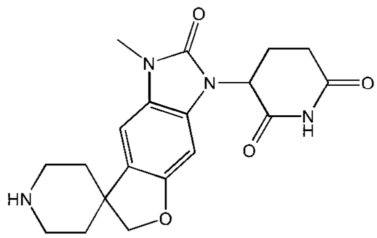,

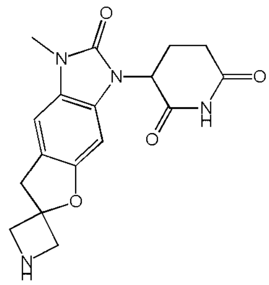,
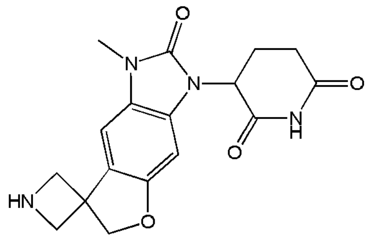,
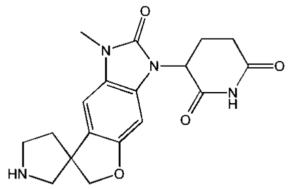,
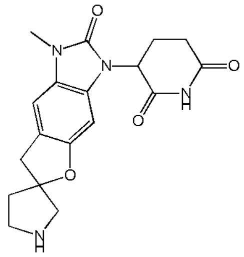,
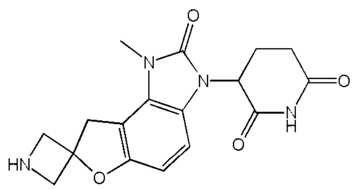,
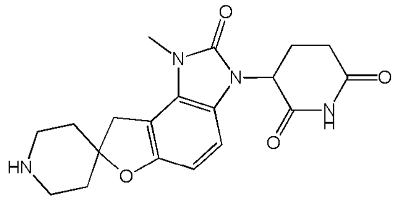,
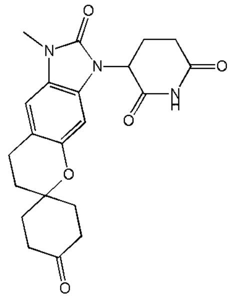,
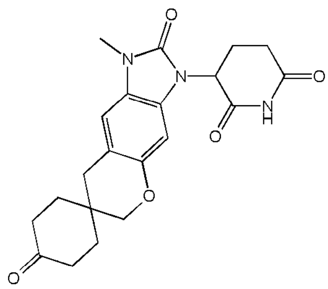,
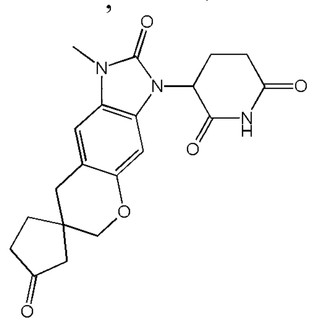,
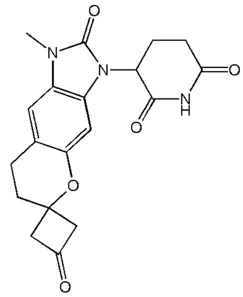,

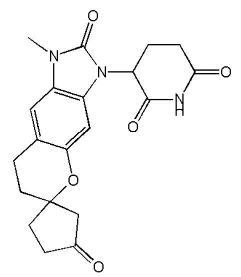 , 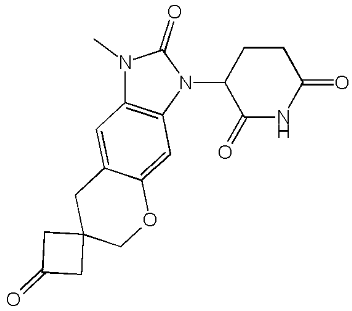 , 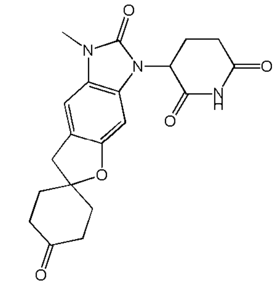 ,

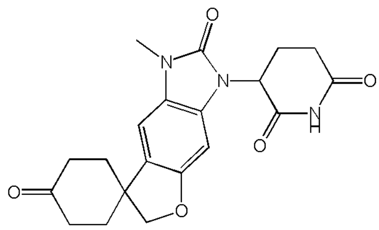 , 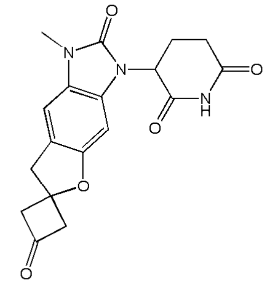 , 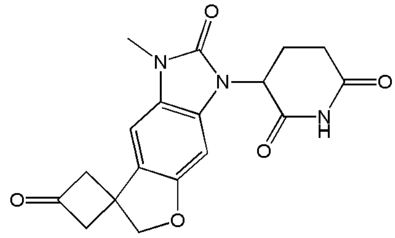 ,

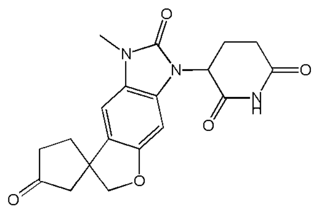 , 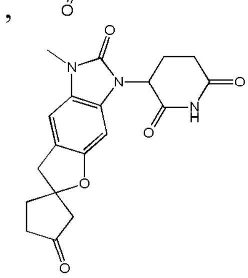 , 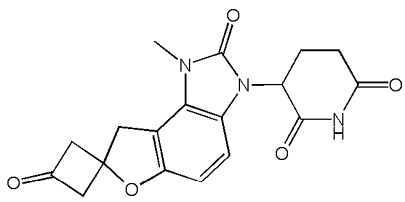 ,

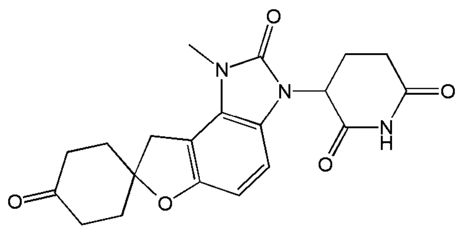 , 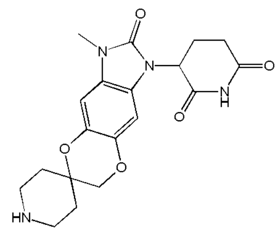 , 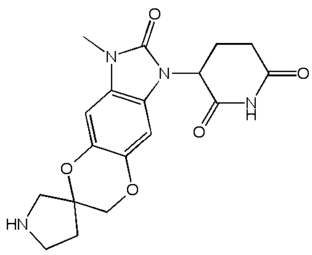 ,

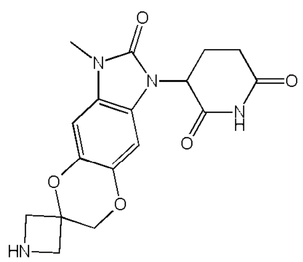 , 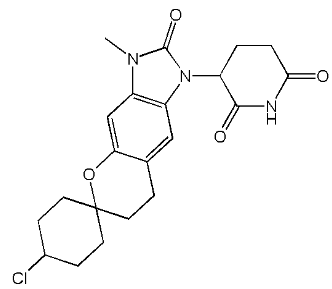 , 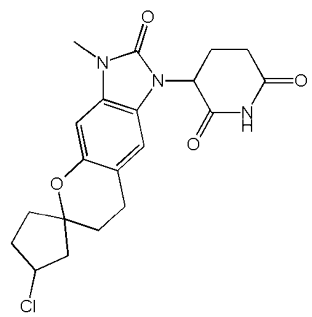 ,

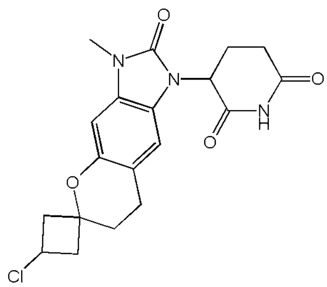 , 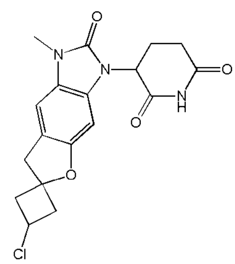 , 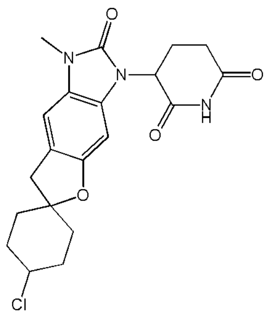 ,

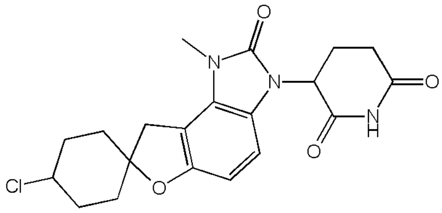,
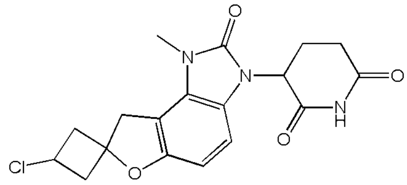,
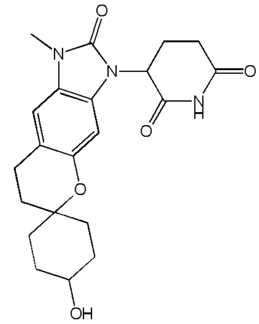,
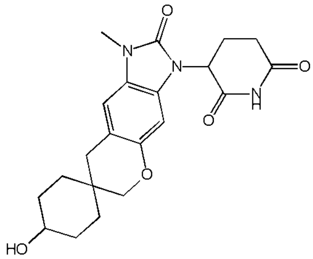,
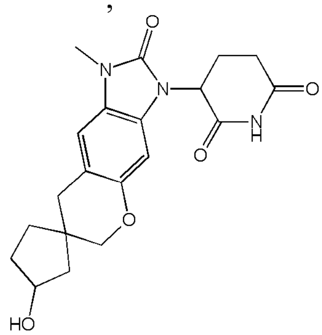,
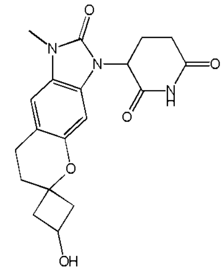,
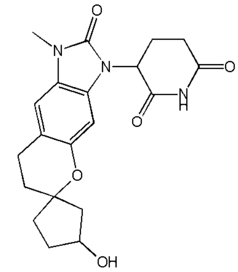,
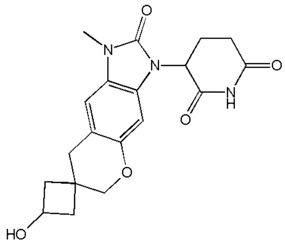,
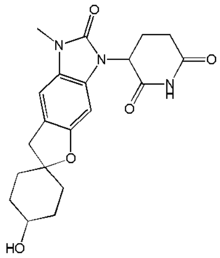,
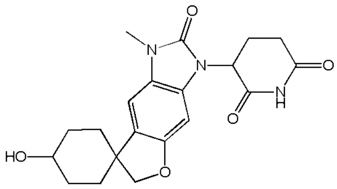,
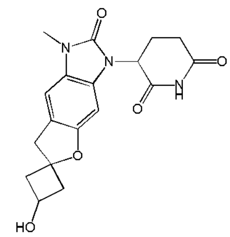,
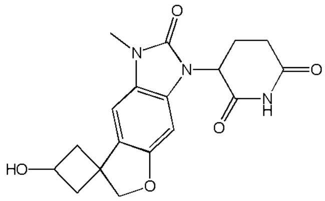,
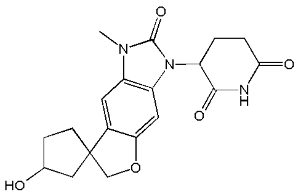,
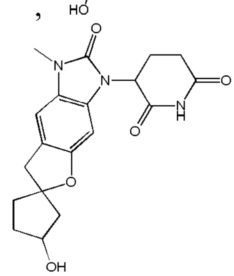,
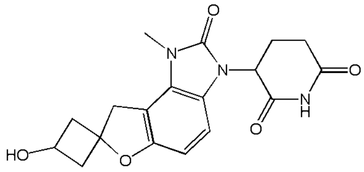,
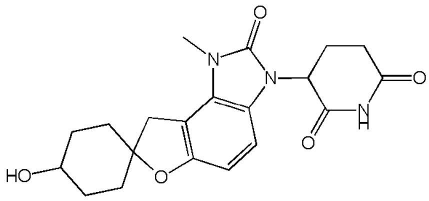,
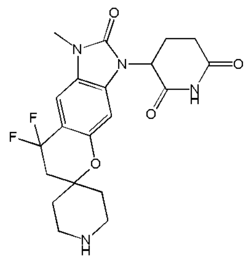,
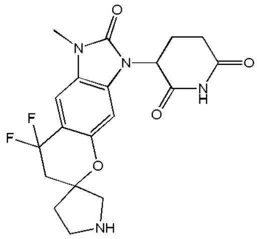,
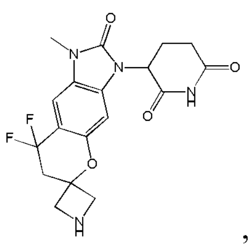,
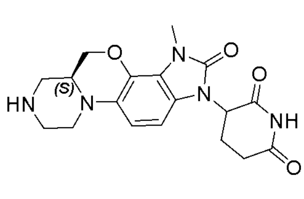,
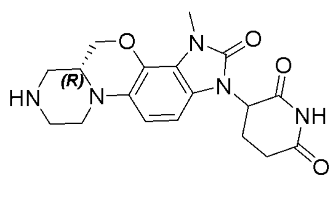

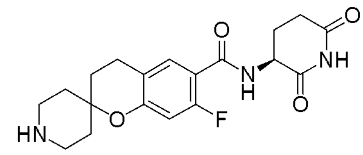
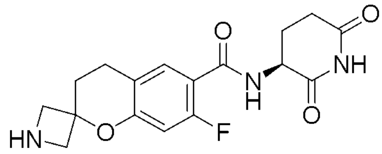
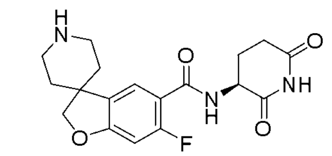
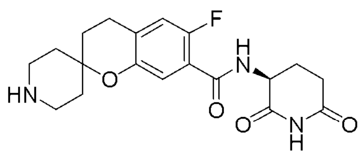
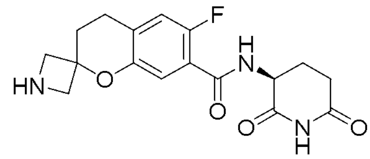
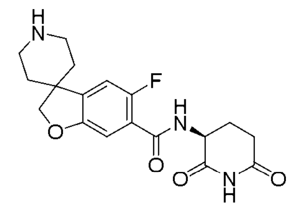
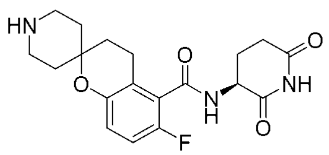
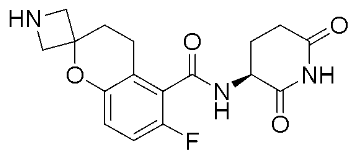
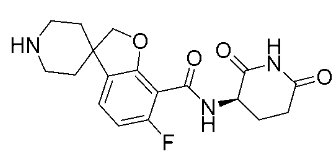
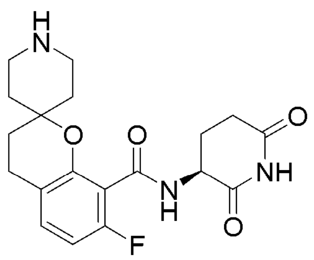
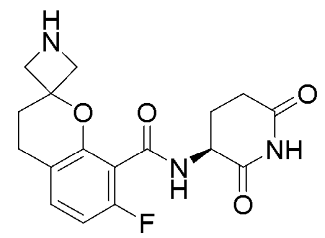
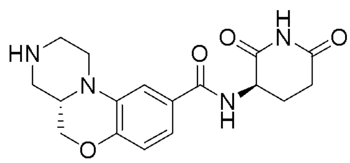
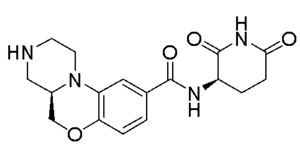
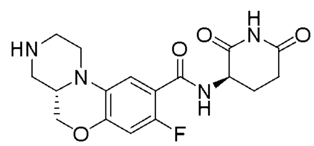
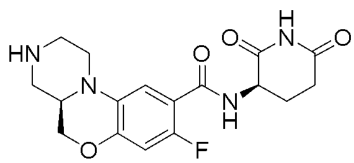
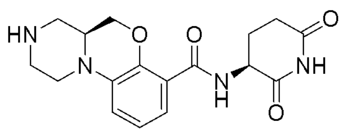
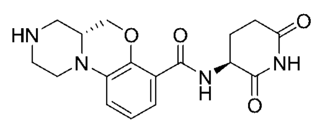
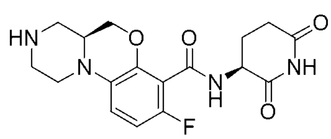
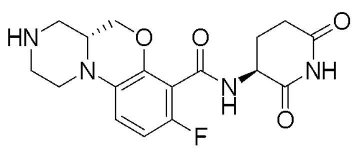
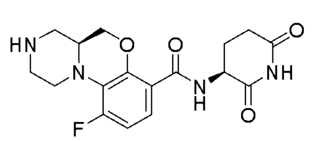
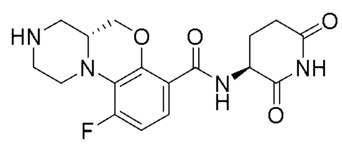
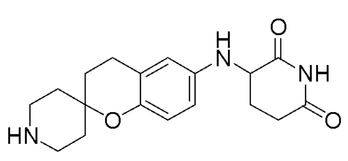
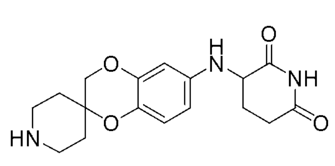
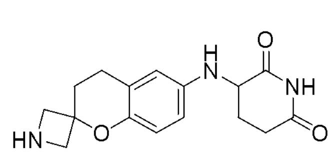

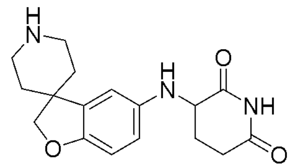
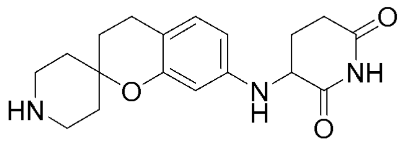
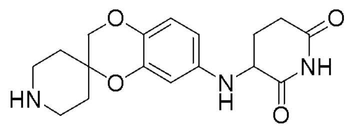

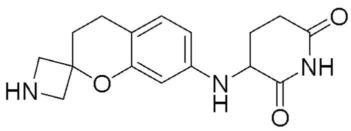
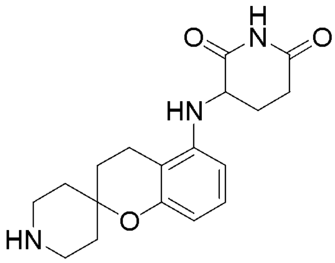
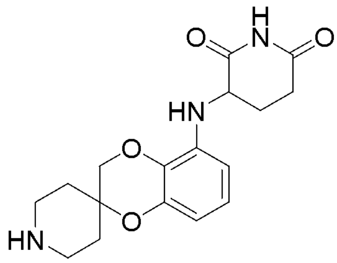

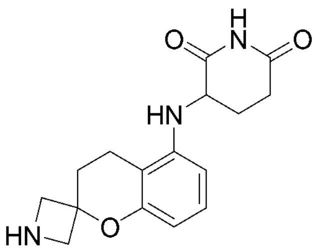
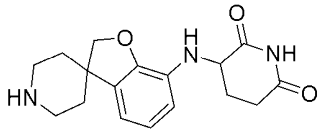
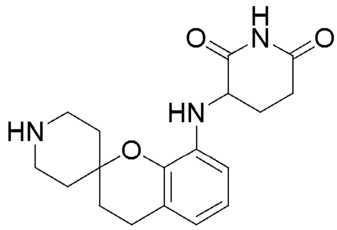

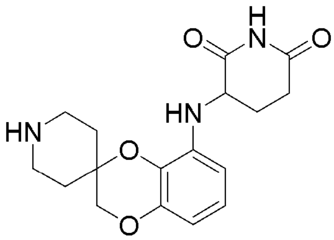
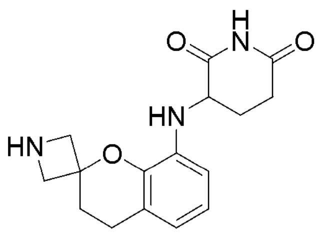
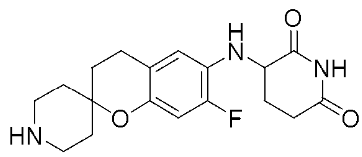

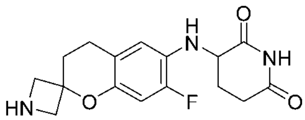
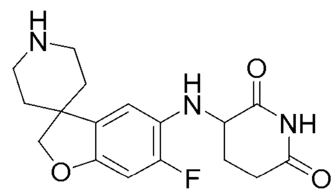
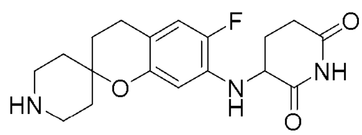

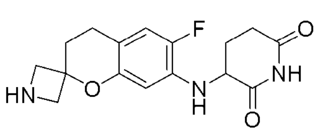
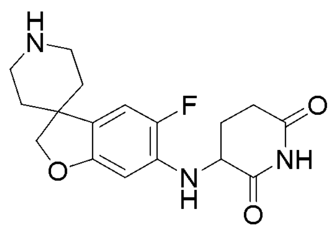
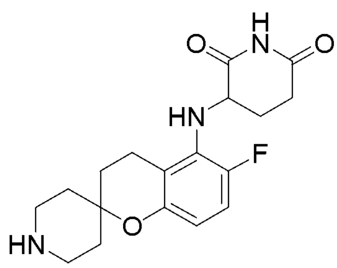

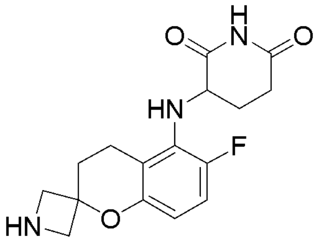
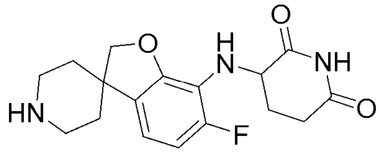
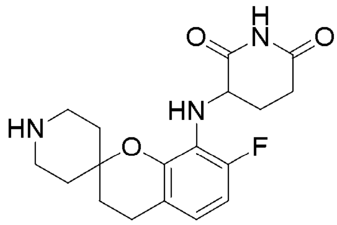

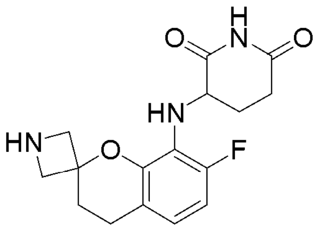
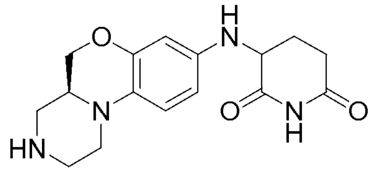
,
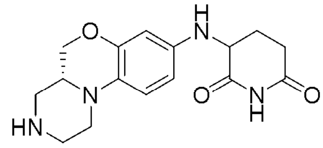

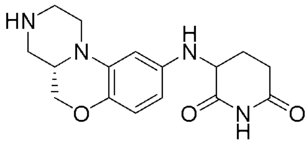
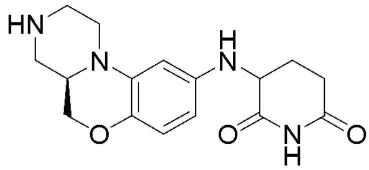
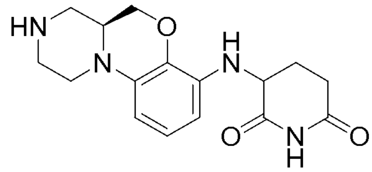

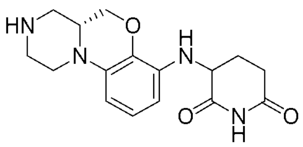
,
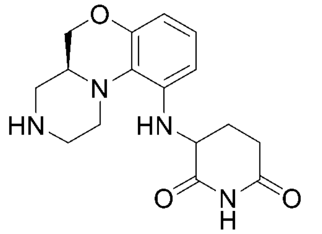
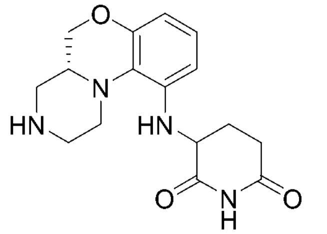
,

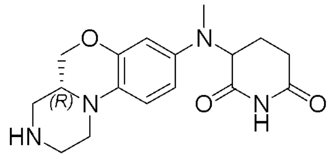
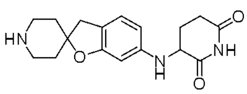
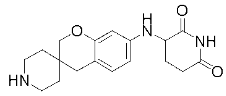
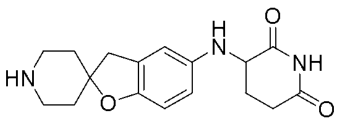
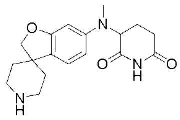

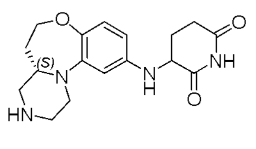
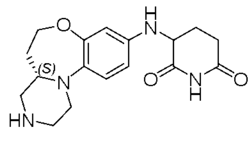
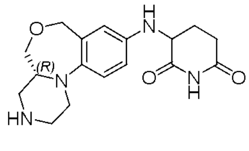
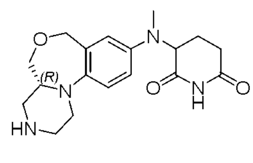

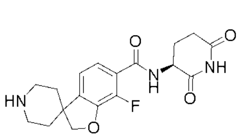
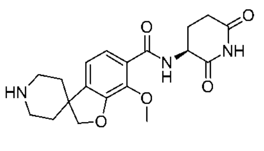
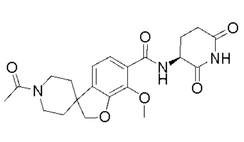
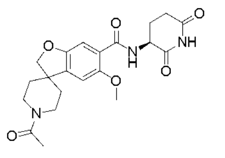

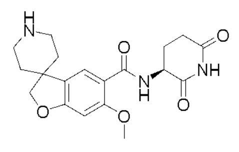 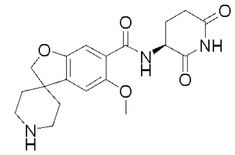 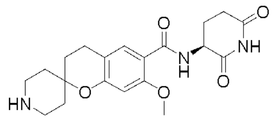 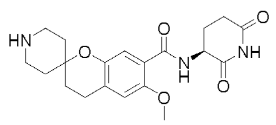

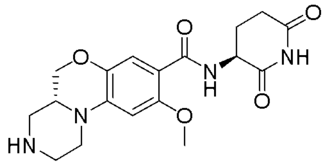 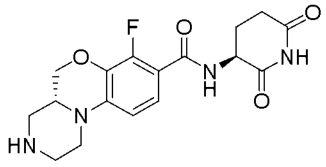 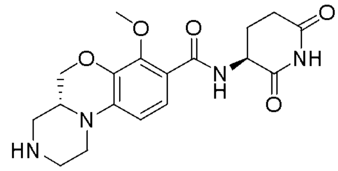

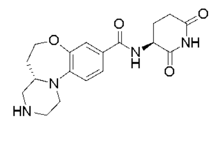 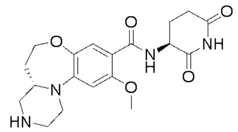 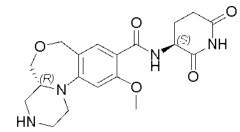 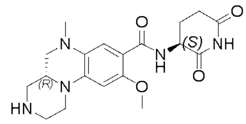

and

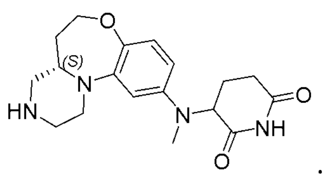

.

**7.** A compound represented by Formula II, or an isomer, isotopic derivative, polymorph, prodrug, or a pharmaceutically acceptable salt or a solvate thereof:

CLM-L-PTM (Formula II),

wherein:

the PTM is a moiety that binds to a target protein;
the L is a bond or a chemical linking moiety covalently connecting the CLM and the PTM; and
the CLM is a cereblon E3 ubiquitin ligase binding moiety, selected from the following structures:

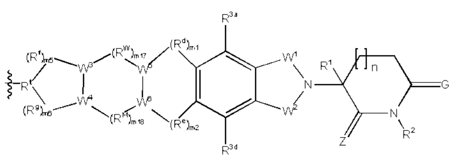 , 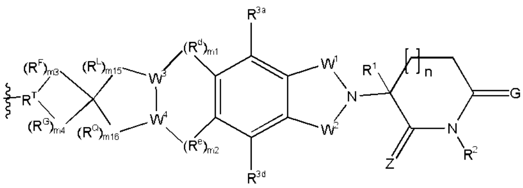 ,

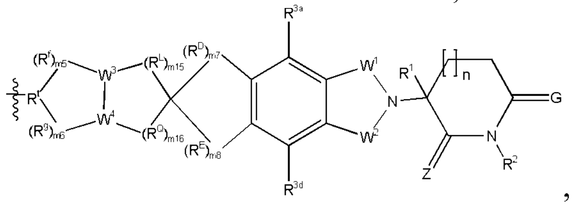 , 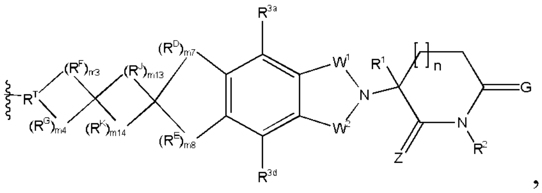 ,

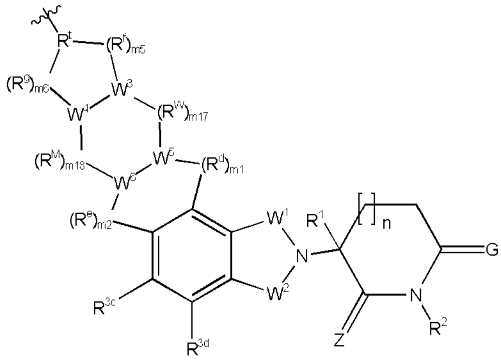

,

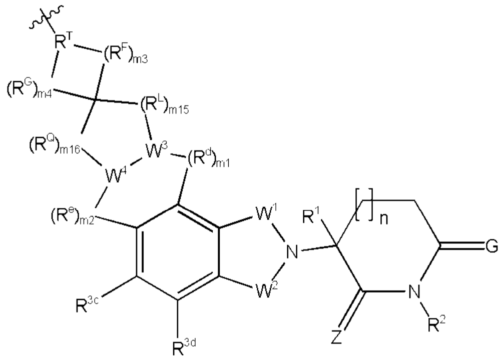

,

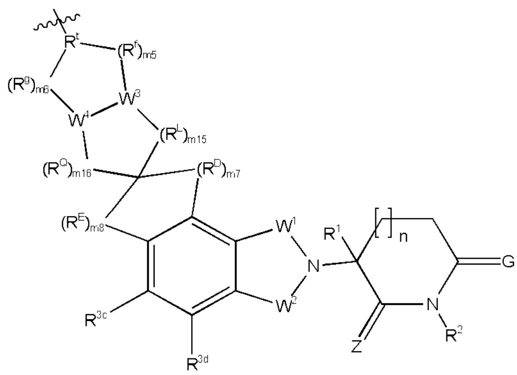

,

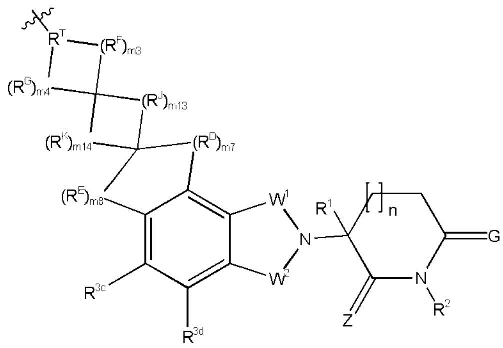

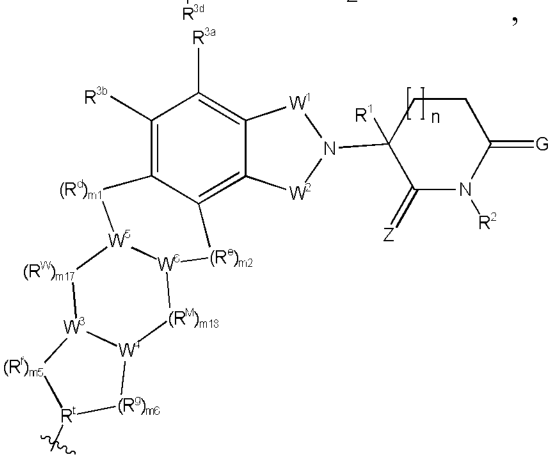

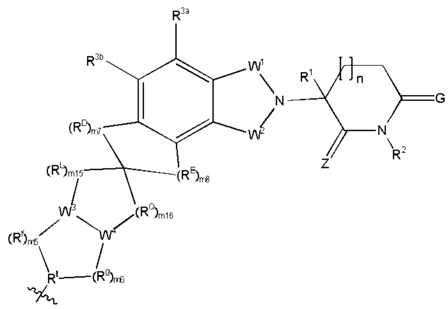

,

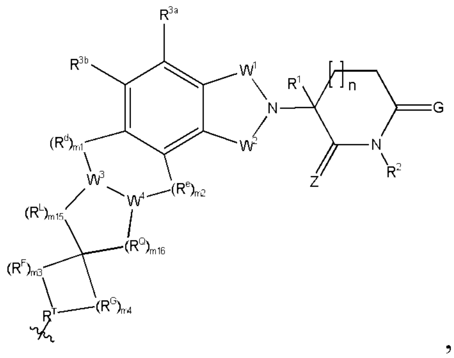

,

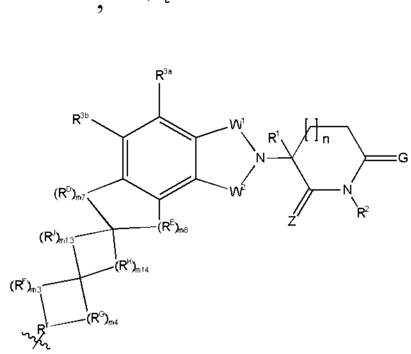

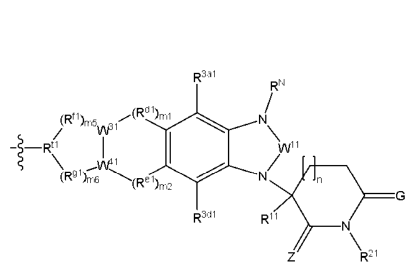,
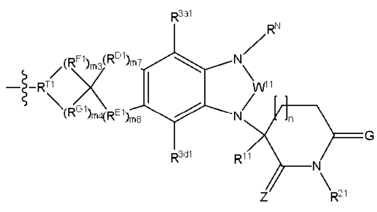,
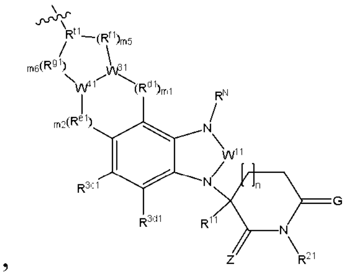,

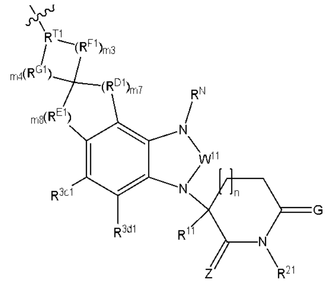,
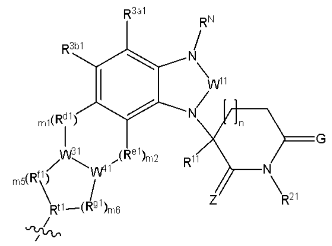,
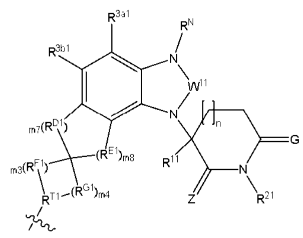,

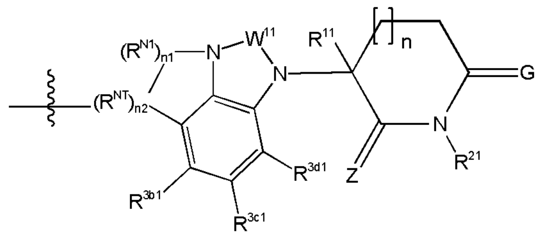,

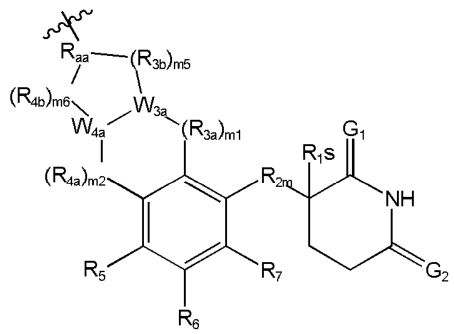,
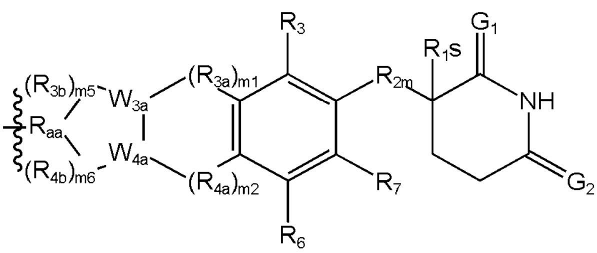,

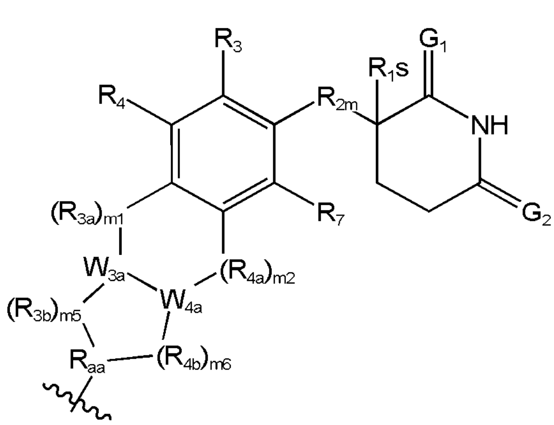,
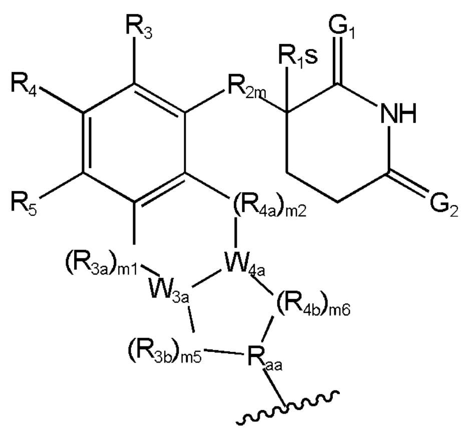,

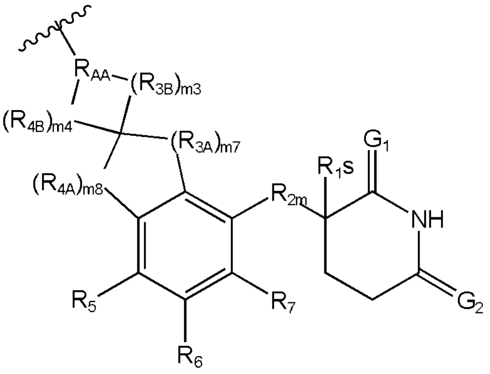

,

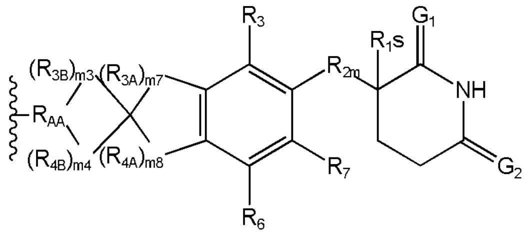

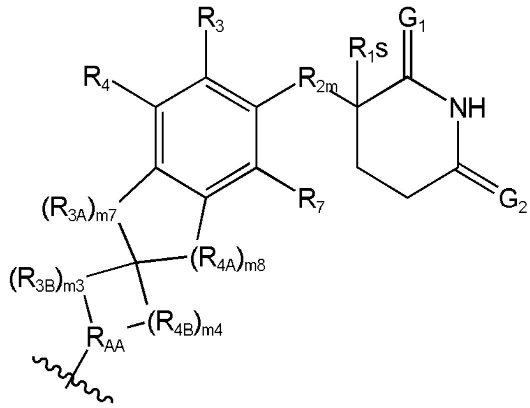

, and

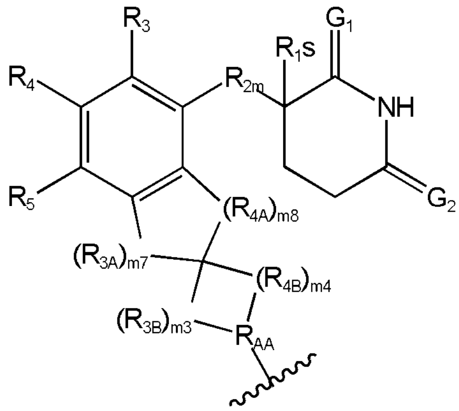

;

wherein:

$W^{11}$, $W^1$, $W^2$ , G, Z, $R^{3a}$, $R^{3b}$, $R^{3c}$, $R^{3d}$, $R^N$, $R^{3a1}$ , $R^{3bl}$, $R^{3c1}$, $R^{3d1}$ , $R^d$, $R^e$, $R^f$, $R^g$, $R^D$, $R^E$, $R^F$, $R^G$, $R^J$, $R^K$, $R^L$, $R^Q$, $R^W$, $R^M$, $R^{d1}$, $R^{e1}$, $R^{f1}$ , $R^{g1}$, $R^{D1}$, $R^{E1}$, $R^{F1}$, $R^{G1}$, $W^{31}$, $W^{41}$, $W^3$, $W^4$, $W^5$, $W^6$, m1, m2, m3, m4, m5, m6, m7, m8, m13, m14, m15, m16, m17, m18, $R^m$, $R^1$, $R^{11}$, $R^2$, $R^{21}$, n, $R^{2h}$, $G_1$, $G_2$, $R_{1s}$, $R_3$, $R_4$, $R_5$, $R_6$, $R_7$, $R_{3a}$, $R_{4a}$, $R_{3b}$, $R_{4b}$, $R_{3A}$, $R_{4A}$, $R_{3B}$, $R_{4B}$, $W_{3a}$, $W_{4a}$, and $R_{2A}$ are as defined in claim 1 or any one of claims 2-6;
each occurrence of $R^{N1}$ is independently $C(R^m)_2$, $NR^m$, C(=O), O, or S;
each occurrence of $R^{T1}$, $R^{t1}$, $R^{NT}$, $R^t$, and $R^T$ is independently N or $CR^{2h}$;
each occurrence of n1 and n2 is independently an integer of 0, 1, 2, 3, 4, or 5, and $n1 + n2 \leq 5$; preferably, n1 + n2 = 2, or n1 + n2 = 3; preferably, n1 is 1 or 2, and n2 is 1;
$R_{2m}$ is selected from $-NR_{2A}-$, $-NR_{2A}CO-$, $C_1$-$C_6$ alkylene, and $C_1$-$C_6$ haloalkylene;
each occurrence of $R_{aa}$, $R_{AA}$, $R_{BB}$, and $R_{bb}$ is independently selected from $CR^m$ or N;
when the PTM is

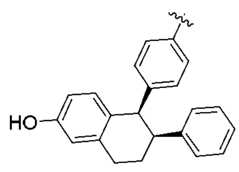

,

the CLM is

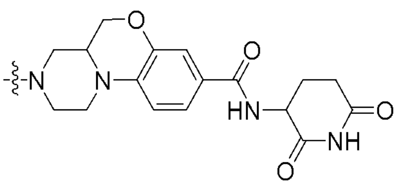

or

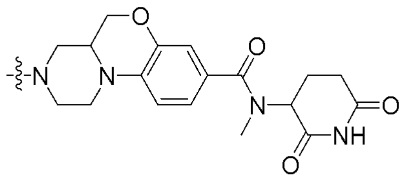

,

and the L is not

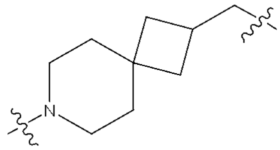

, 

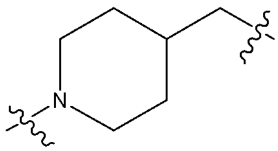

,

or

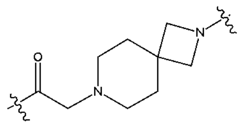

;

and
when the PTM is

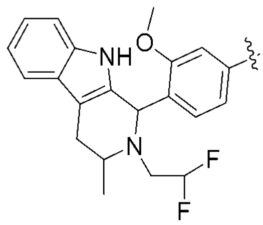

,

and the CLM is

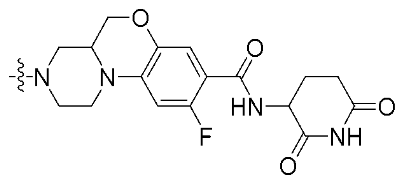

,

the L is not

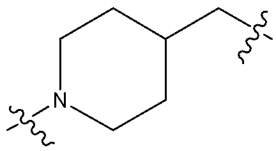

.

**8.** The compound according to claim 7, wherein the CLM is selected from the following structures:

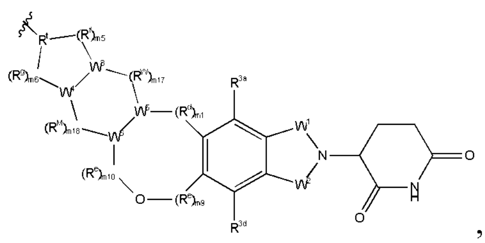
,
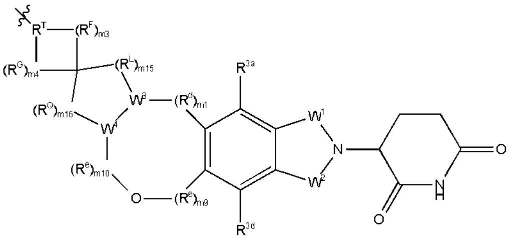
,

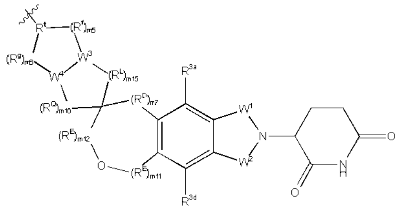
,

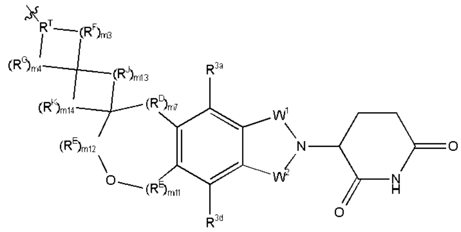

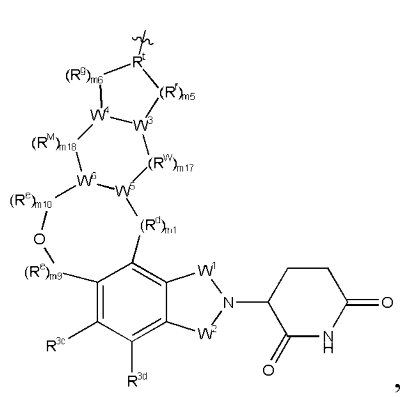
,
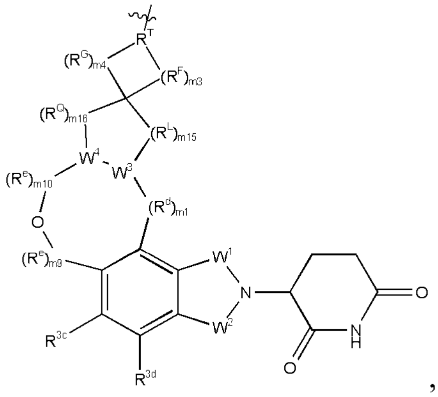
,

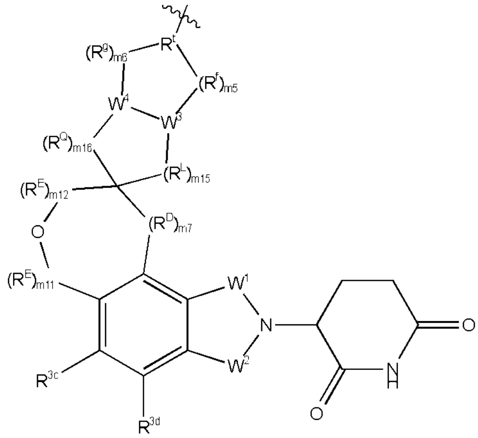
,
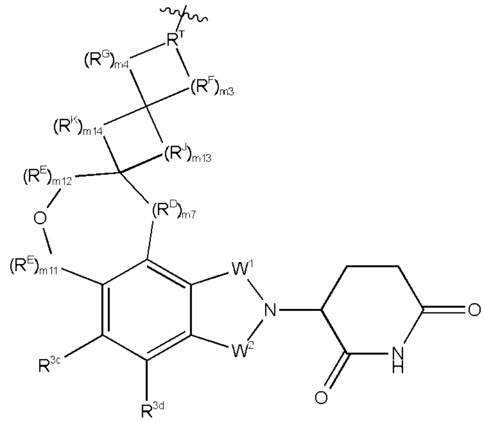

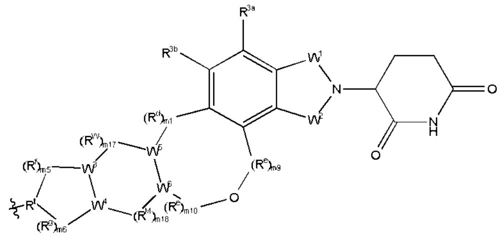

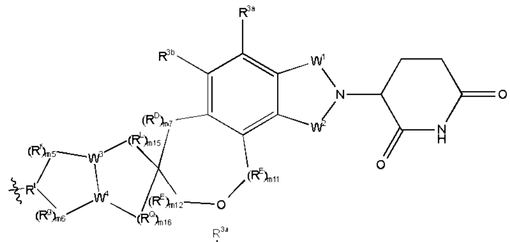

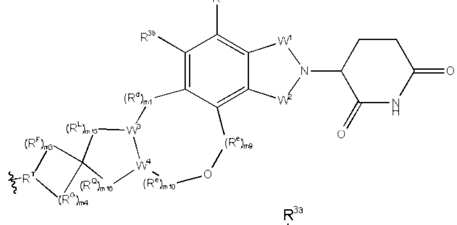

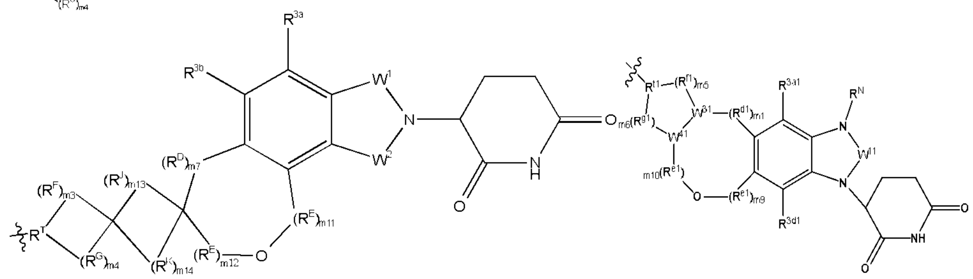

,

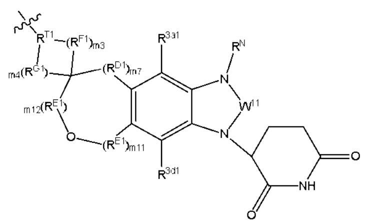

,

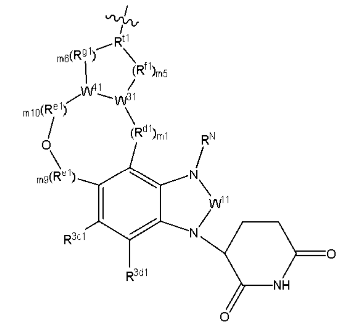

,

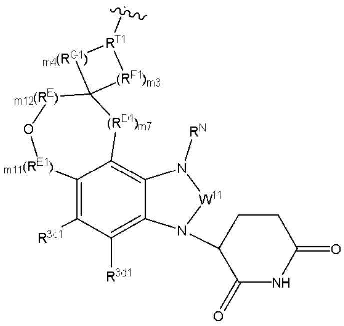

,

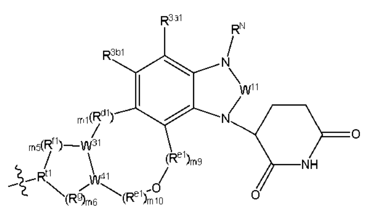

,

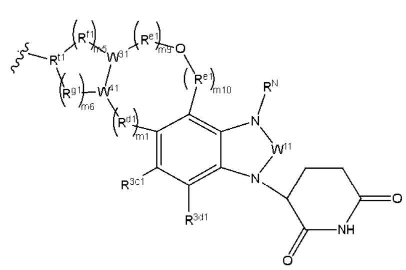

,

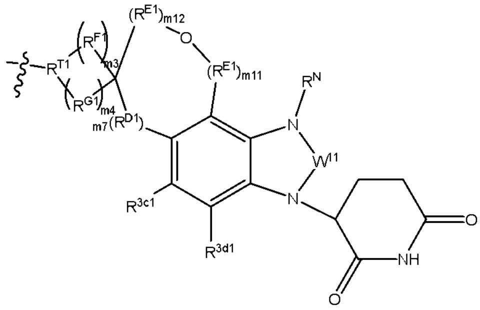

,

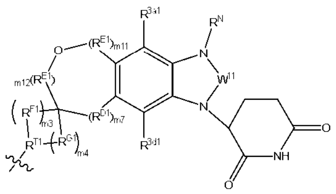

,

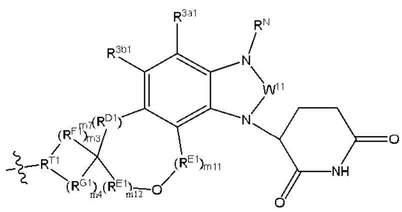

, and

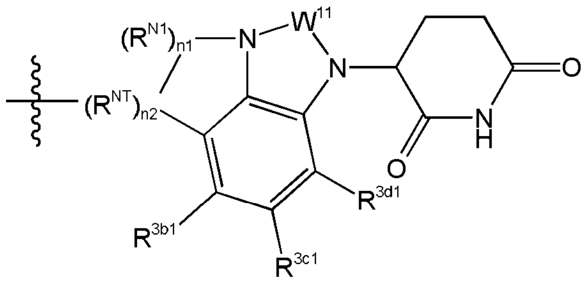

;

$W^{11}$, $W^1$, $W^2$ , $R^{3a}$, $R^{3b}$, $R^{3c}$, $R^{3d}$, $R^N$ $R^{3a1}$ , $R^{3b1}$, $R^{3c1}$, $R^{3d1}$, $R^d$, $R^e$, $R^f$, $R^g$, $R^D$, $R^E$, $R^F$, $R^G$ , $R^J$, $R^K$, $R^L$, $R^Q$, $R^W$, $R^M$, $R^{d1}$, $R^{e1}$, $R^{f1}$ , $R^{g1}$ , $R^{D1}$, $R^{E1}$, $R^{F1}$, $R^{G1}$, $W^{31}$, $W^{41}$, $W^3$, $W^4$, $W^5$, $W^6$, m1, m2, m3, m4, m5, m6, m7, m8, m13, m14, m15, m16, m17, m18, n1, n2, $R^{N1}$, $R^{NT}$, $R^t$, $R^T$, $R^{t1}$, and $R^{T1}$ are as defined in claim 1 or any one of claims 2-7.

**9.** The compound according to claim 7 or 8, wherein the CLM is selected from the following structures:

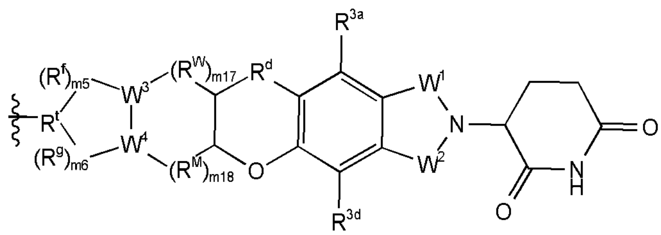

,

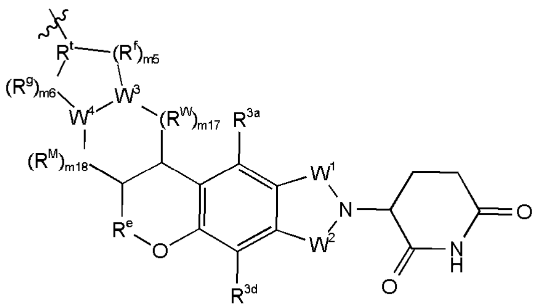

,

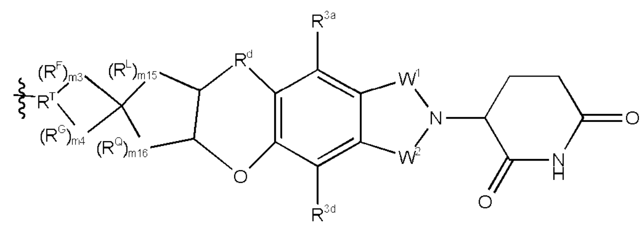

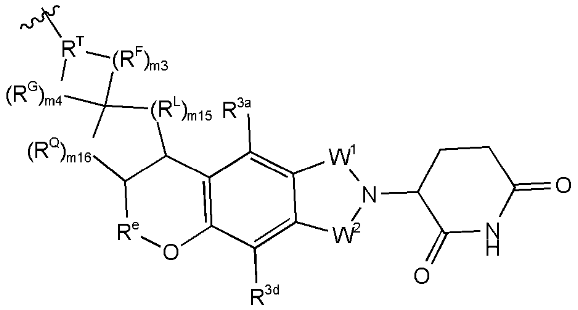

.

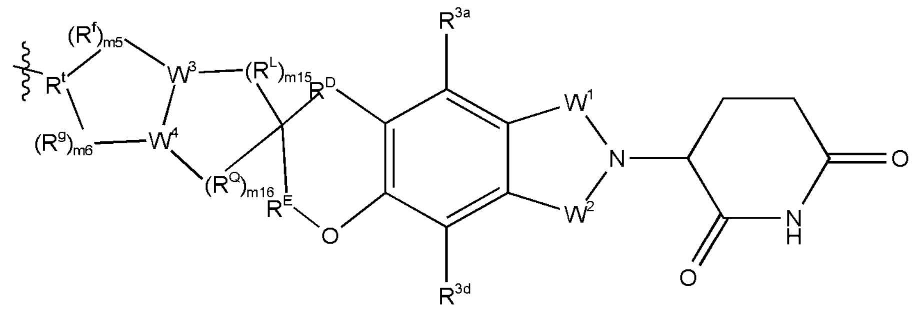

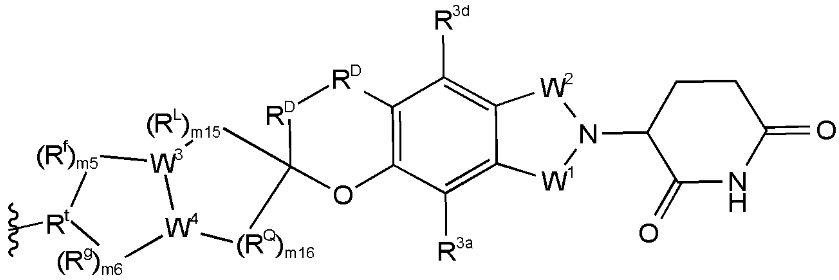

,

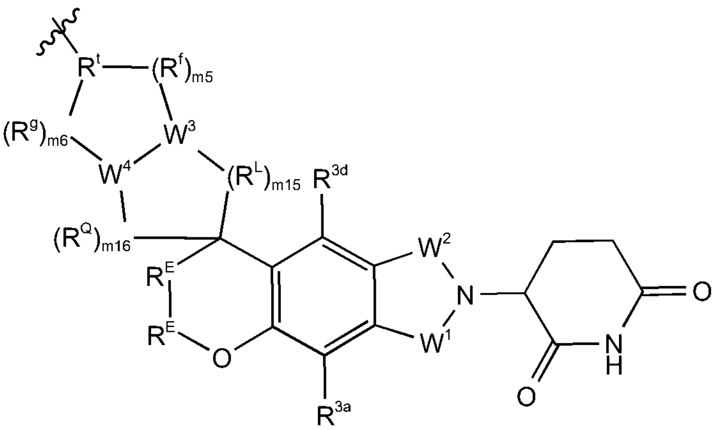

,

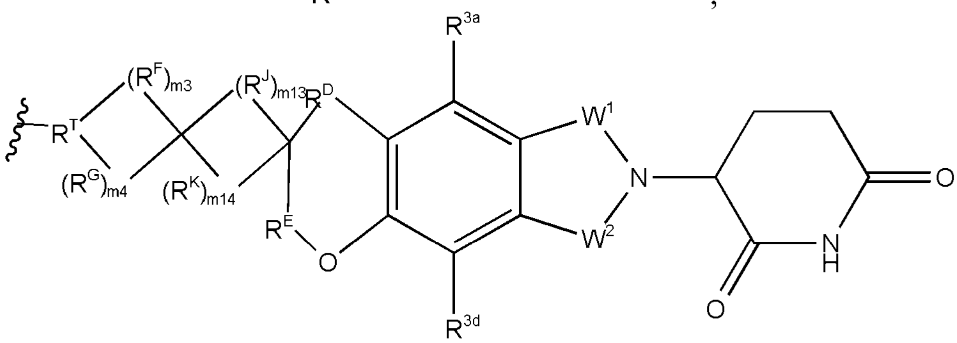

,

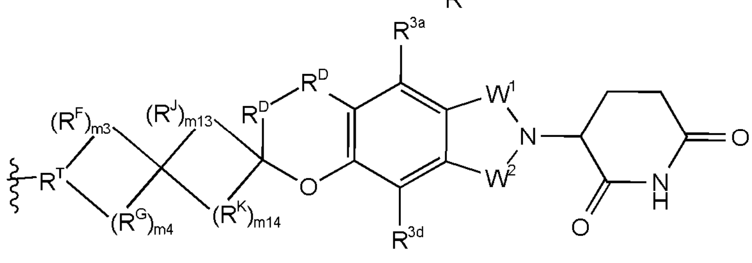

,

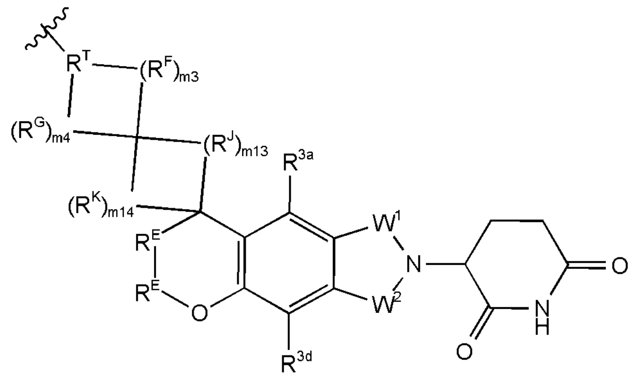

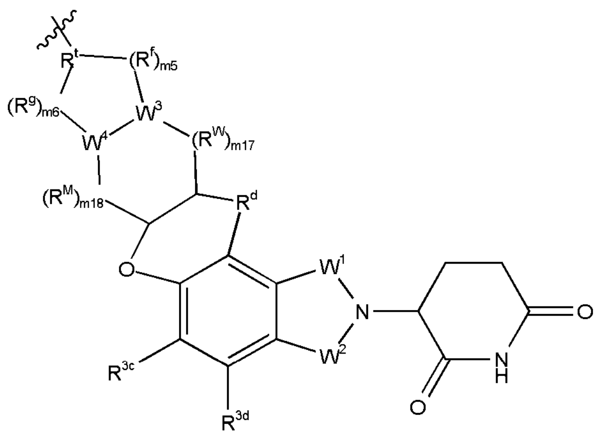

,

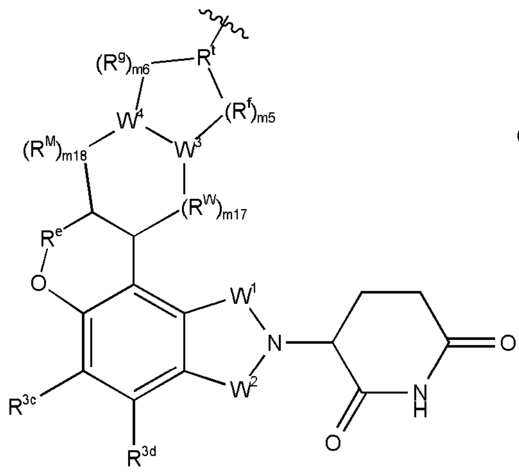

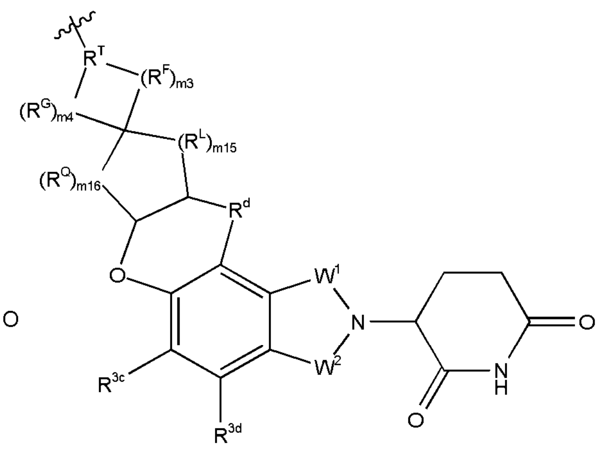

,

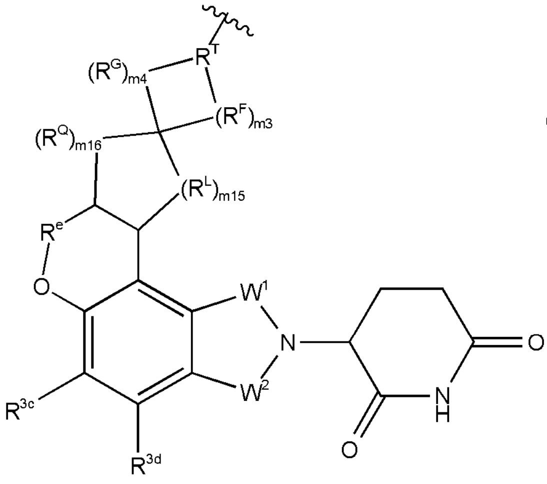
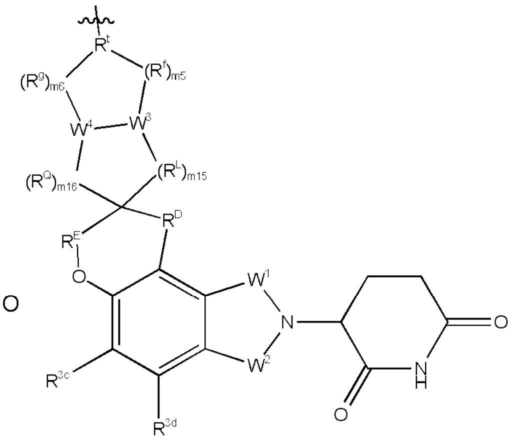
,

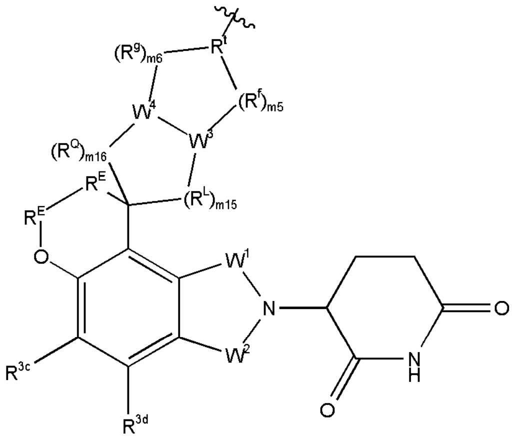
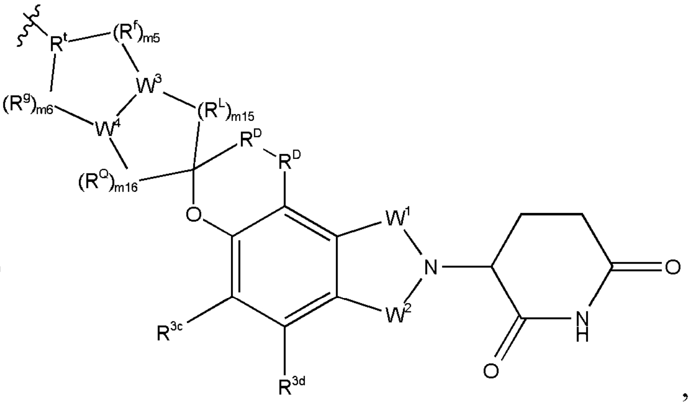
,

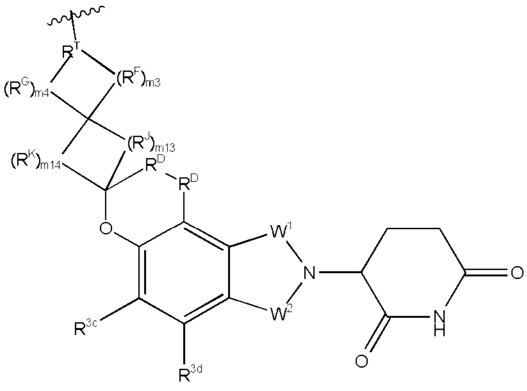
,
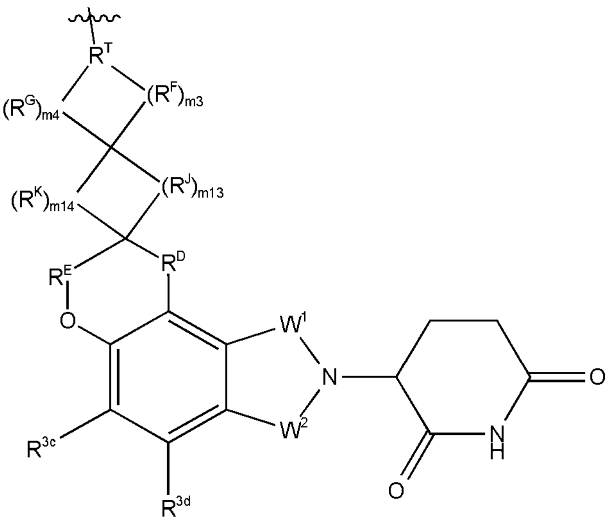
,

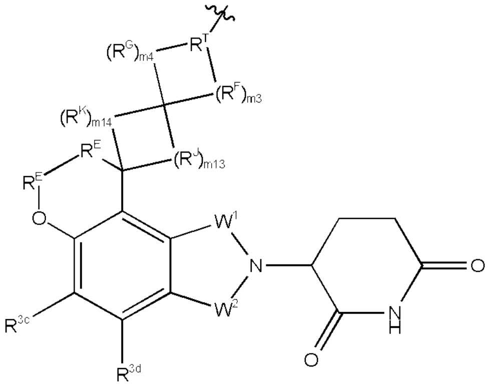

,

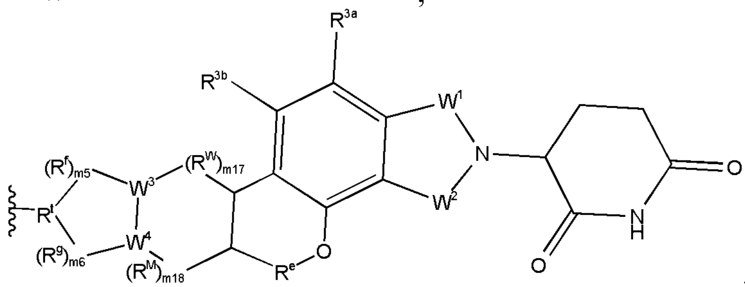

,

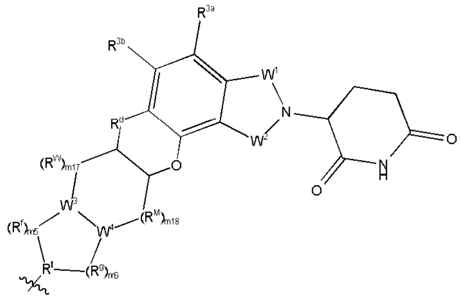

,

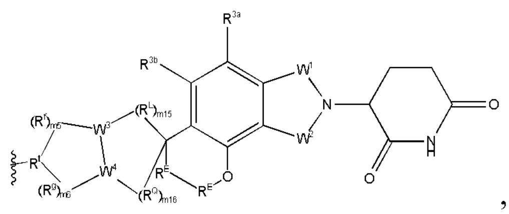

,

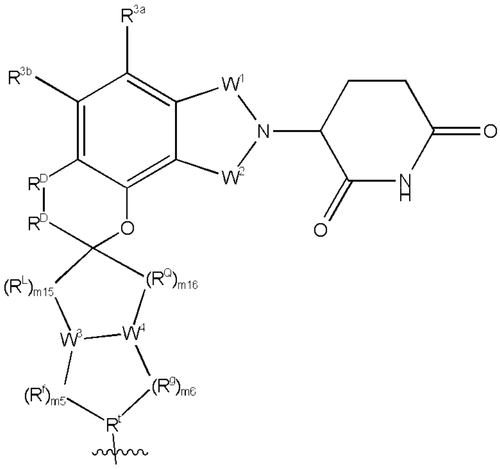

,

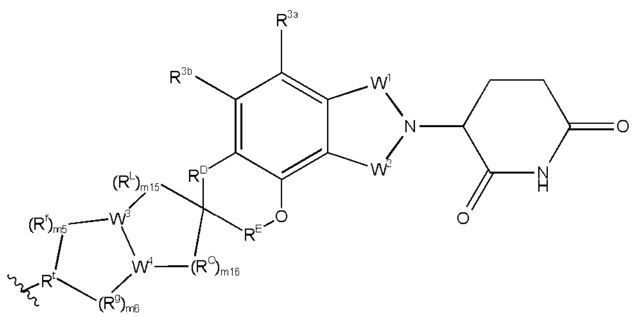

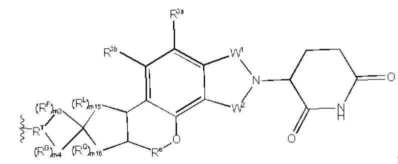

,

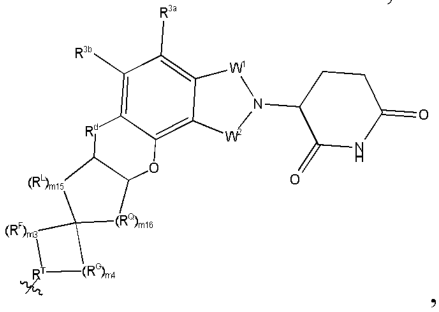

,

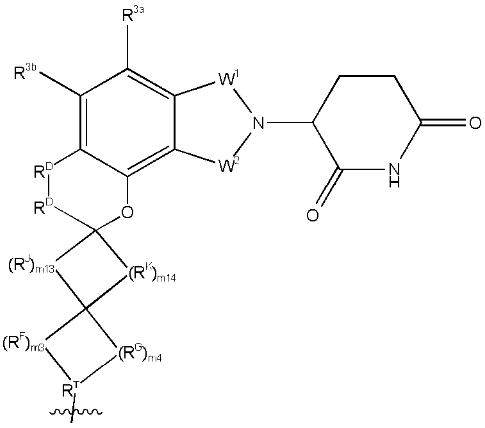

,

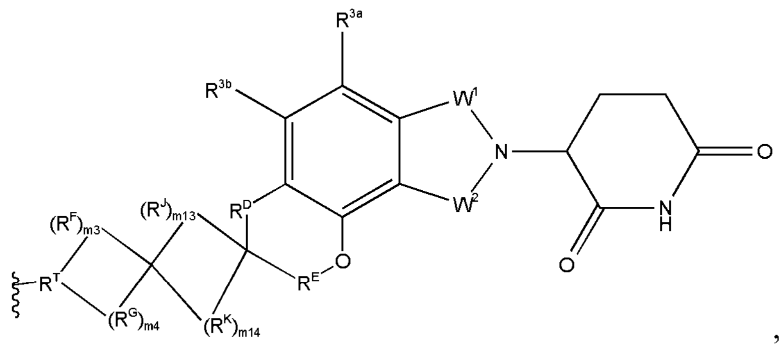

,

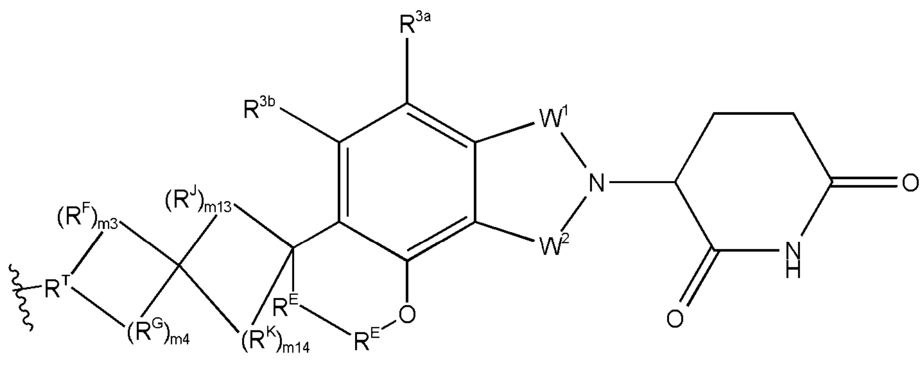

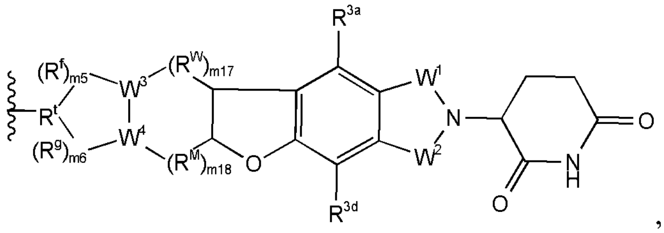

,

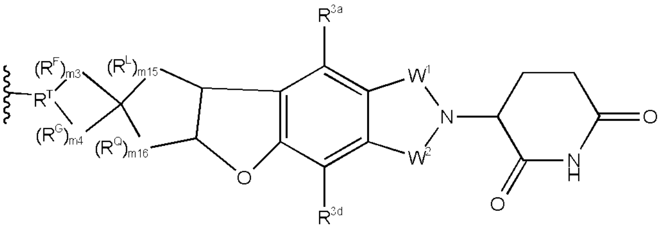

,

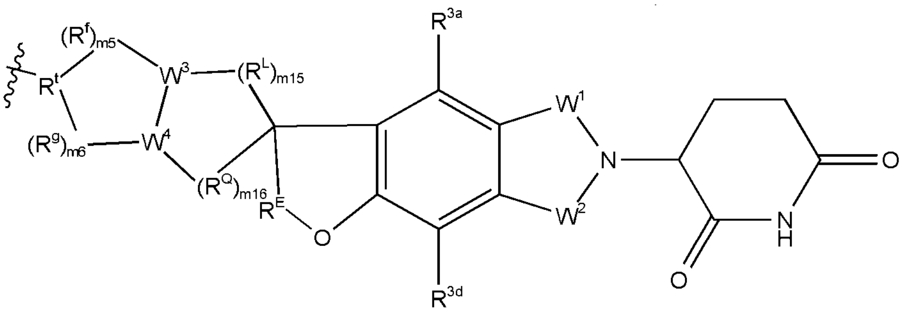

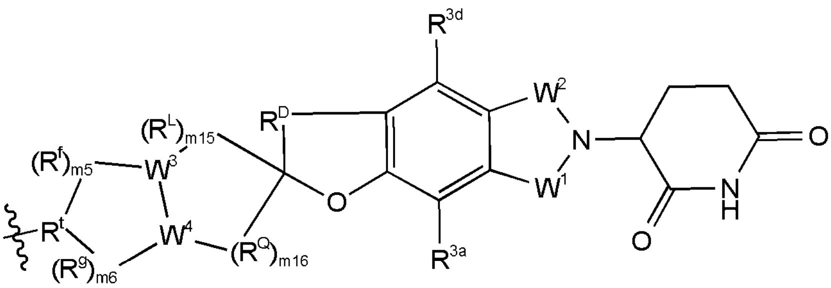

,

,

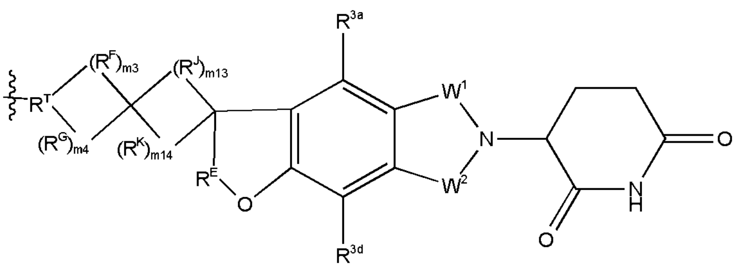

,

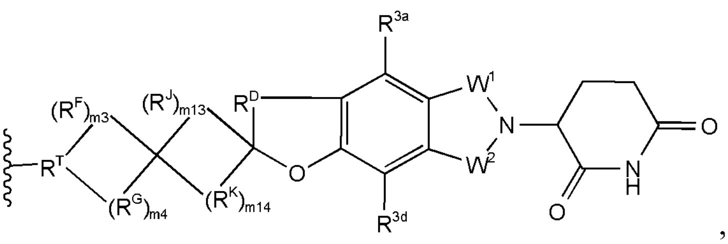

,

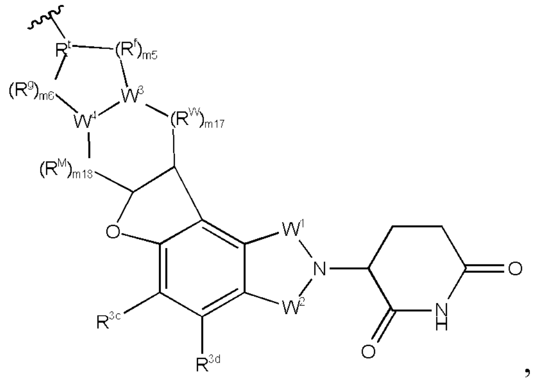,

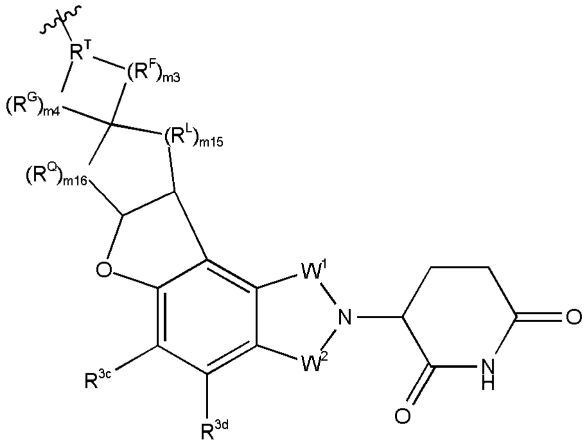,

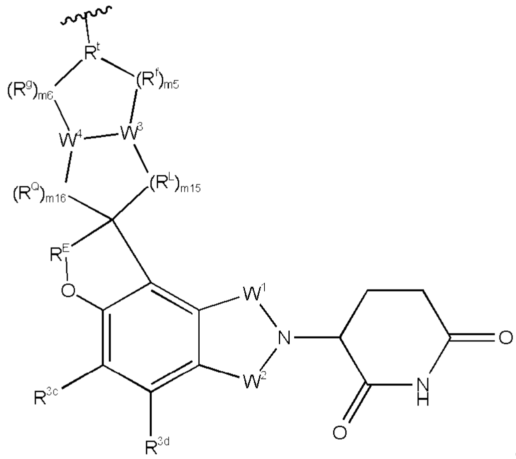,

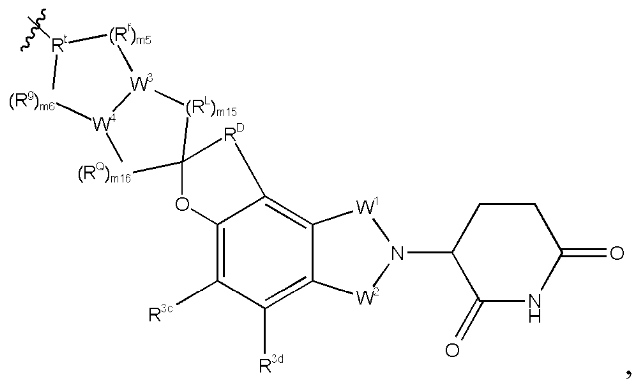,

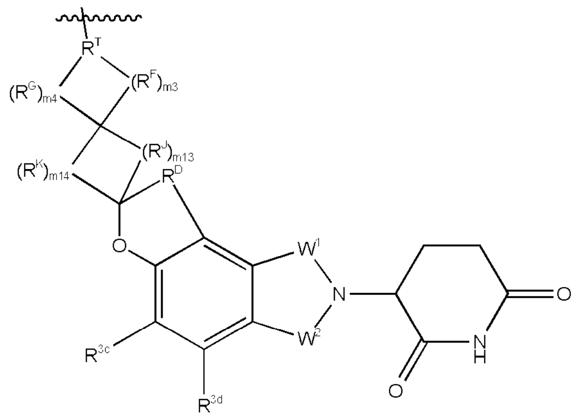,

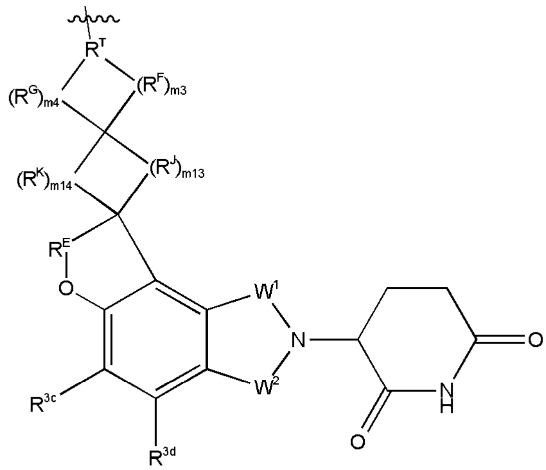,

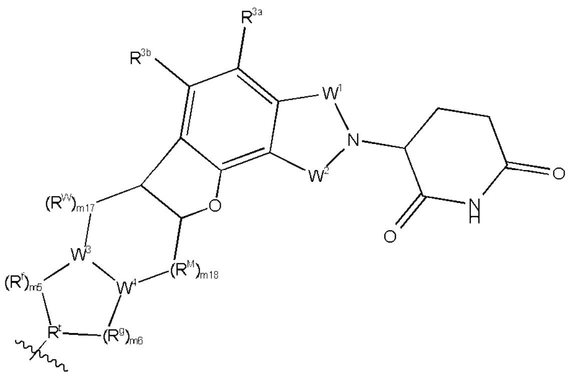

,

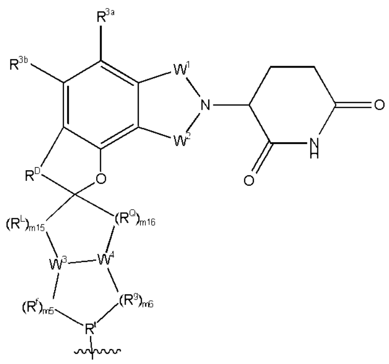

,

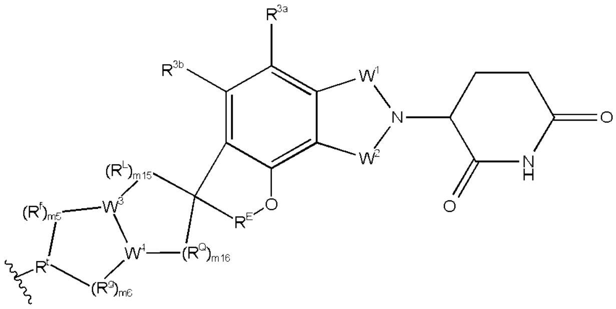

,

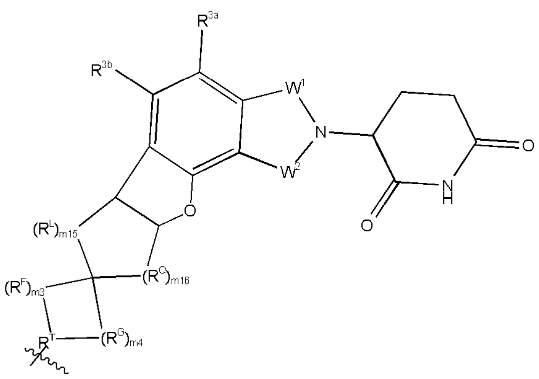

,

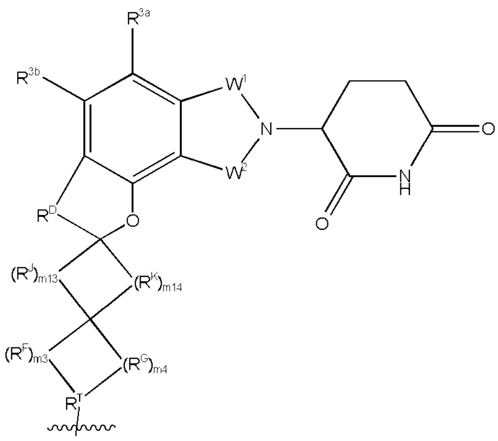

,

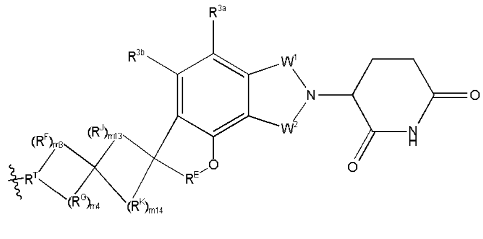

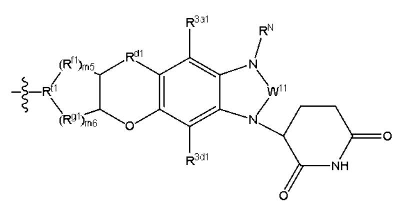

,

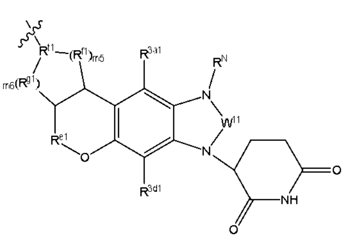

,

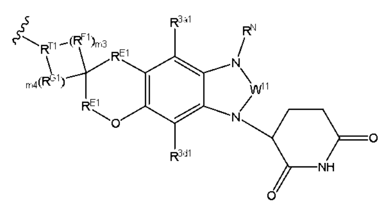

,

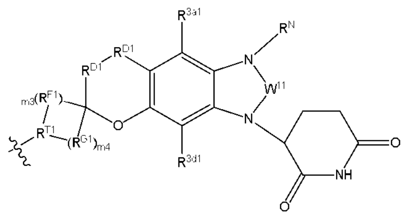 , 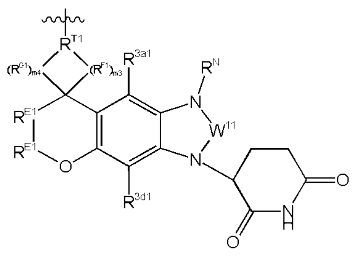 , 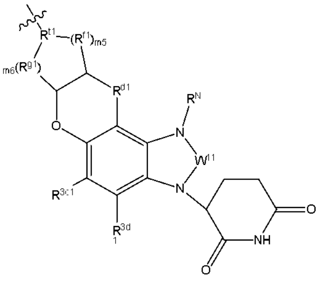 ,

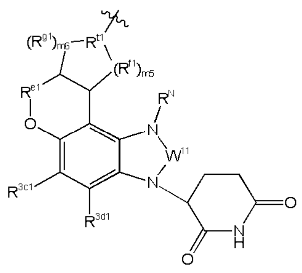 , 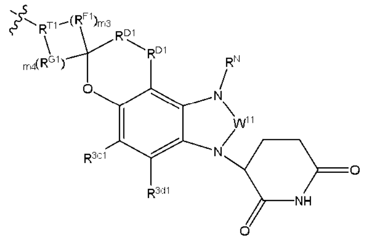 , 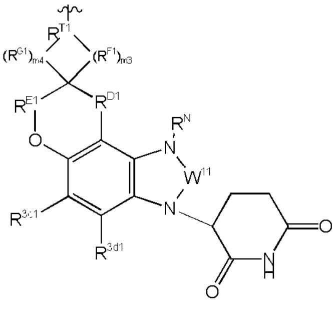 ,

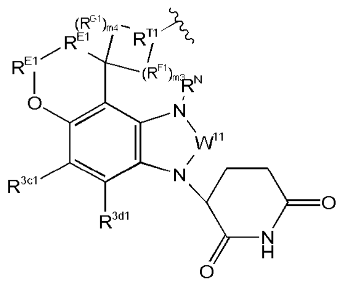 , 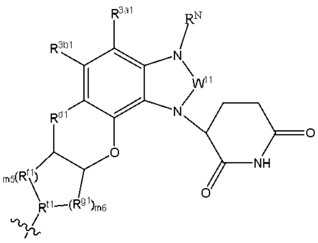 , 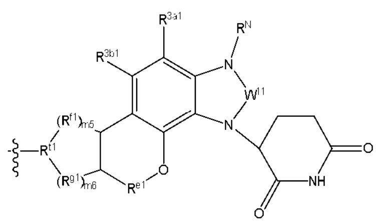 ,

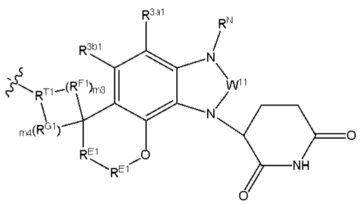 , 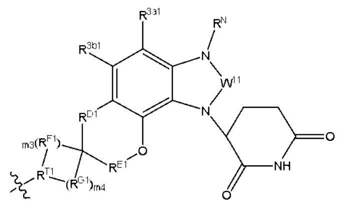 , 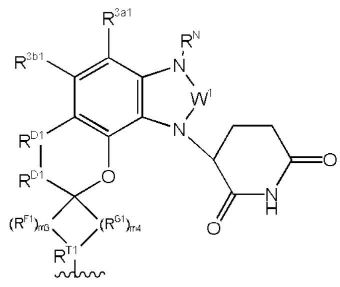 ,

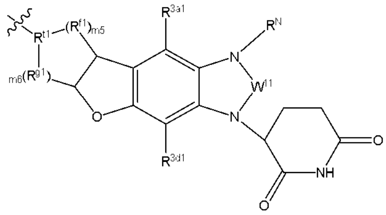 , 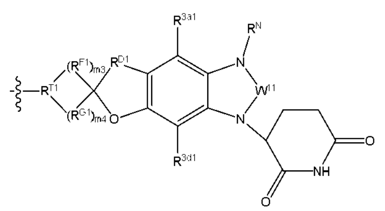 , 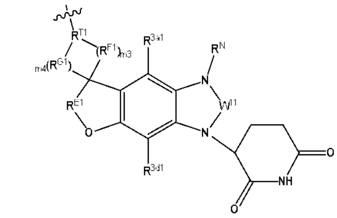 ,

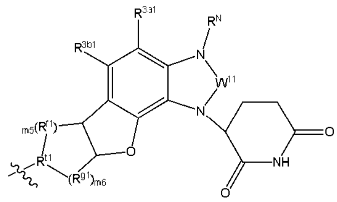 , 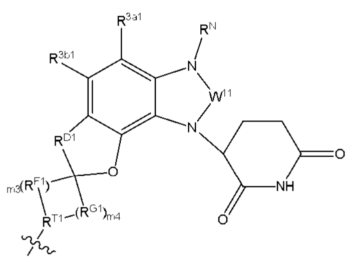 , 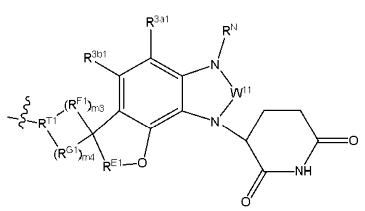 ,

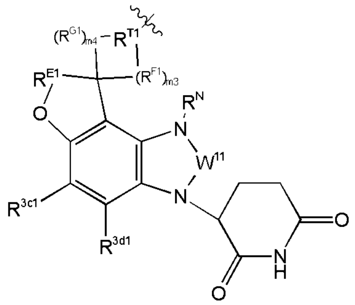
,
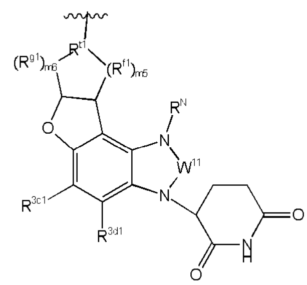
,
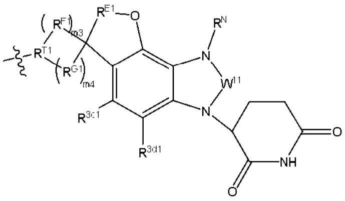
,

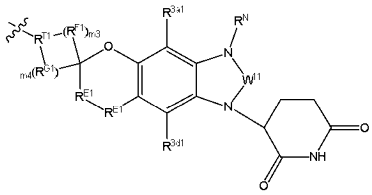
,
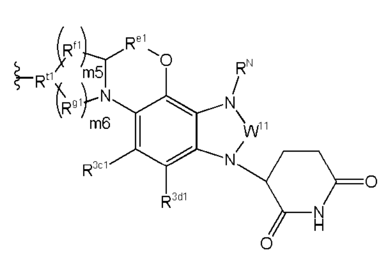
, 和
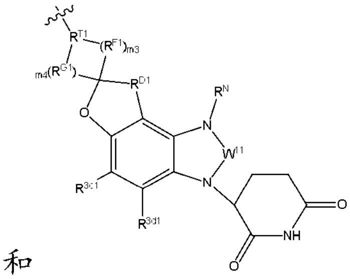
,

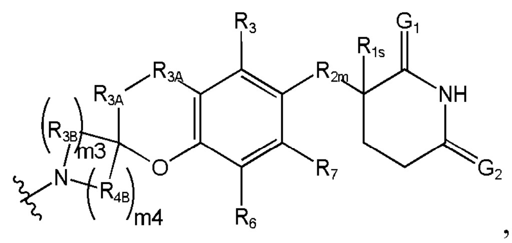
,
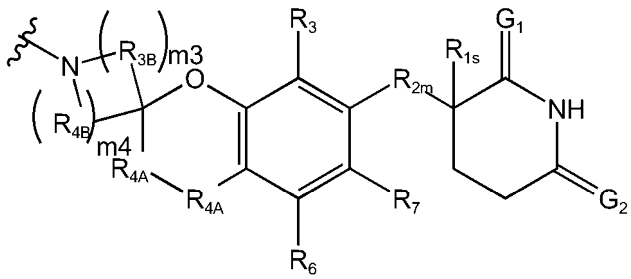
,

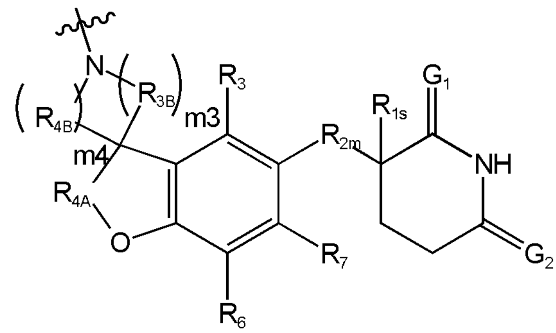
,
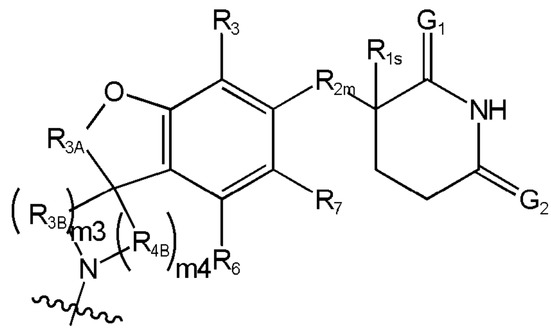
,

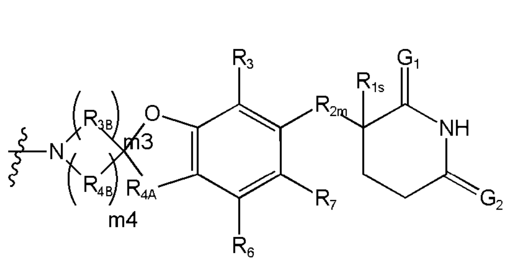
,
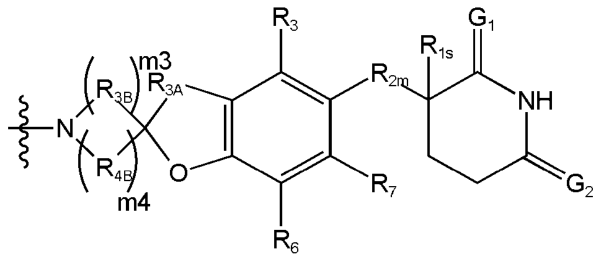
,

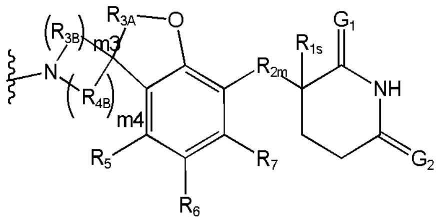,
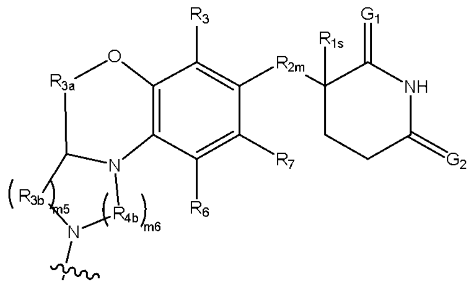,
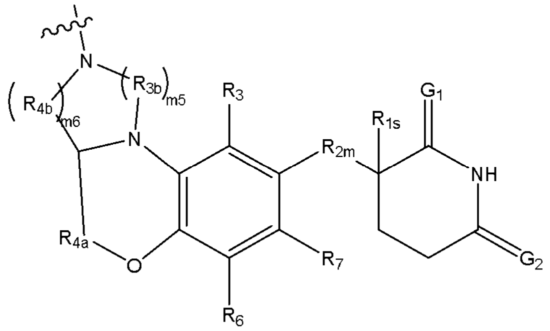,
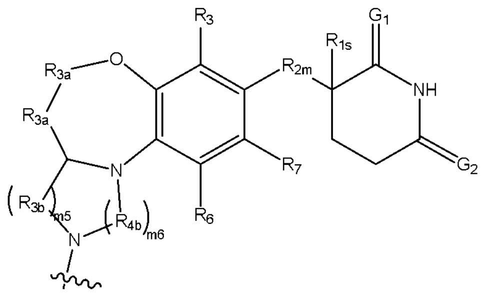,
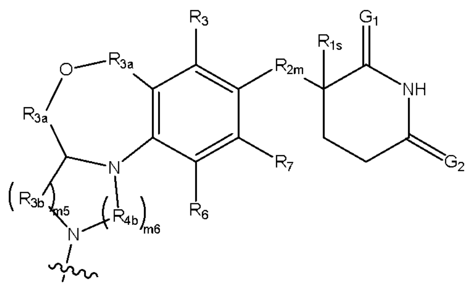,
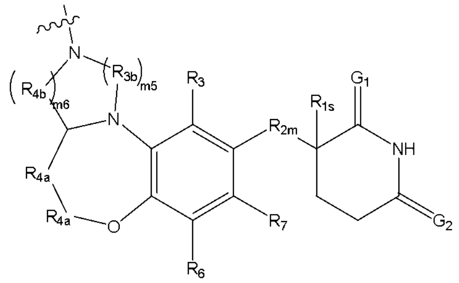,
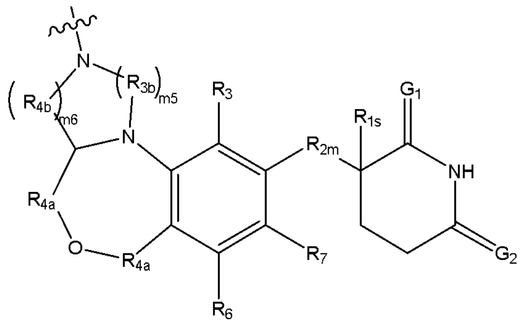,
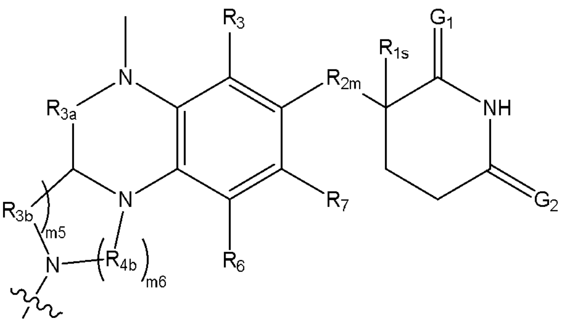;

$W^{11}$, $W^1$, $W^2$ , $R^{3a}$, $R^{3b}$, $R^{3c}$, $R^{3d}$, $R^N$, $R^{3a1}$, $R^{3b1}$, $R^{3c1}$, $R^{3d1}$, $R^d$, $R^e$, $R^f$, $R^g$, $R^D$, $R^E$, $R^F$, $R^G$, $R^J$, $R^K$, $R^L$, $R^Q$, $R^W$, $R^M$, $R^{d1}$, $R^{e1}$, $R^{f1}$ , $R^{g1}$, $R^{D1}$, $R^{E1}$, $R^{F1}$, $R^{G1}$, $W^{31}$, $W^{41}$ , $W^3$, $W^4$, $W^5$, $W^6$, m1, m2, m3, m4, m5, m6 , m7, m8 , m13, m14 , m15, m16 , m17, m18, n1, n2, $R^{N1}$, $R^{NT}$, $R^t$, $R^T$, $R^{t1}$, $R^{T1}$, $G_1$, $G_2$, $R_{1s}$, $R_{2m}$, $R_3$, $R_4$, $R_5$, $R_6$, $R_7$, $R_{3a}$, $R_{4a}$, $R_{3b}$, $R_{4b}$, $R_{3A}$, $R_{4A}$, $R_{3B}$, and $R_{4B}$ are as defined in any one of claims 1, 2-6, 7, or 8.

**10.** The compound according to any one of claims 7 to 9, wherein the CLM is selected from the following structures:

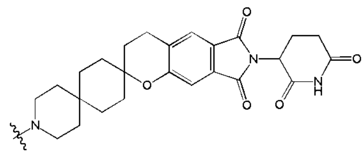,
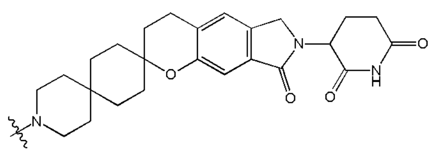

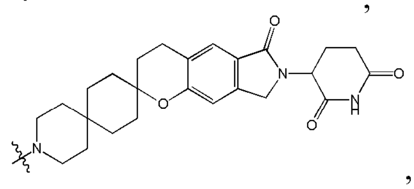,
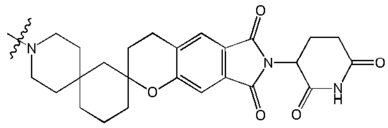

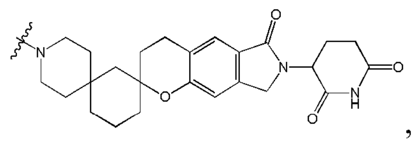,
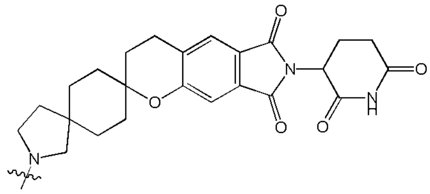

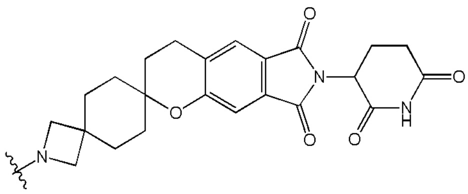,
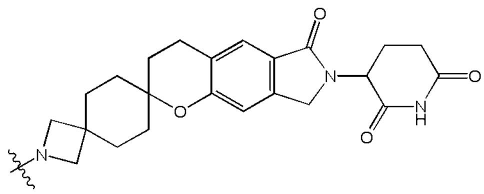

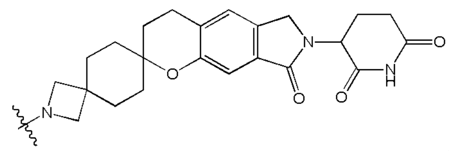,
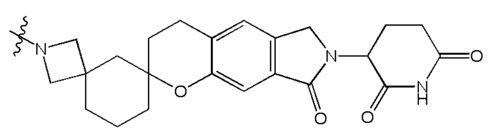

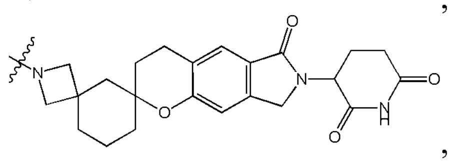,
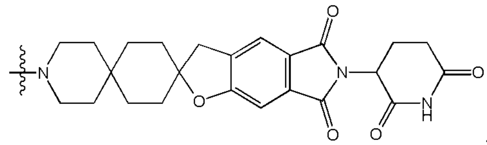,

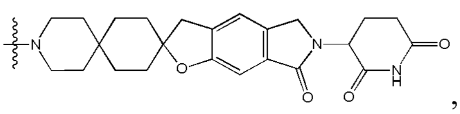,
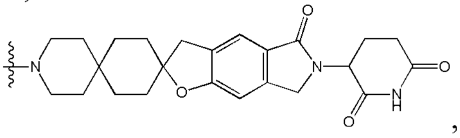,

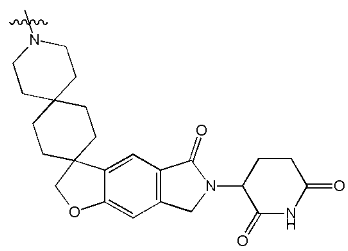,
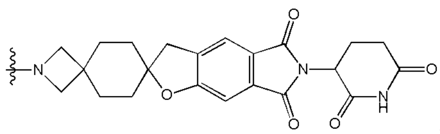,

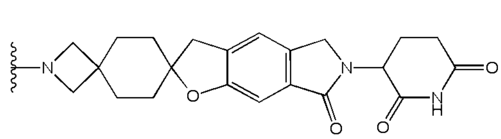,
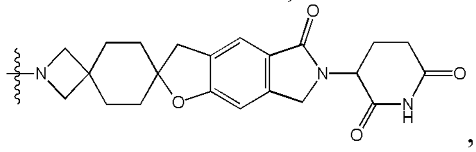,

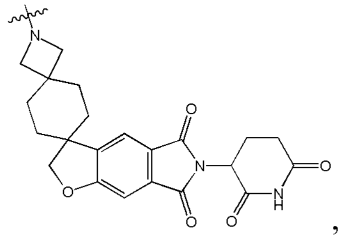,
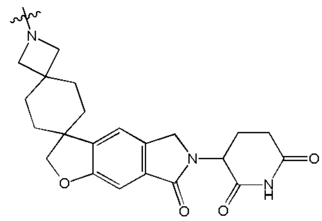,
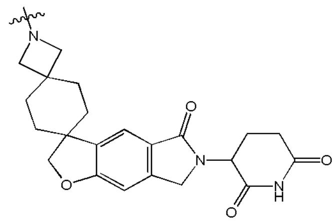

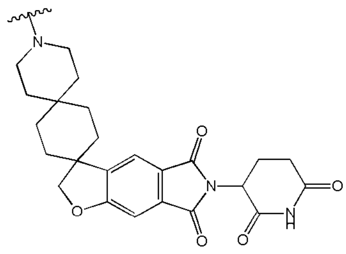
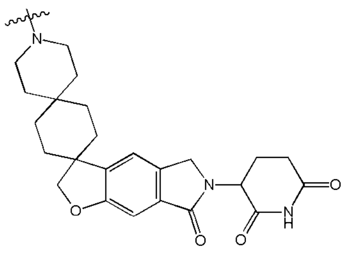
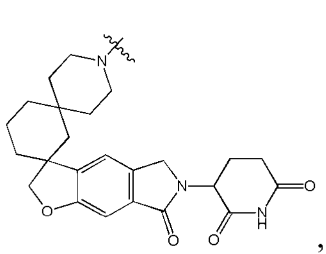,

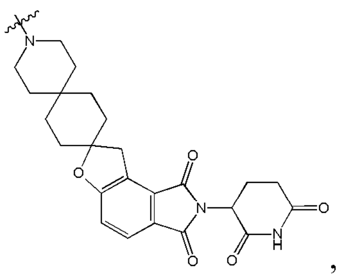,
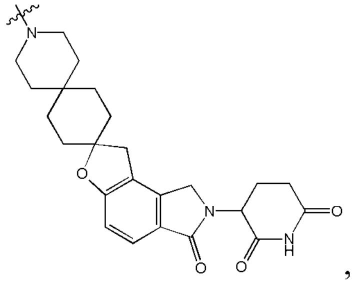,
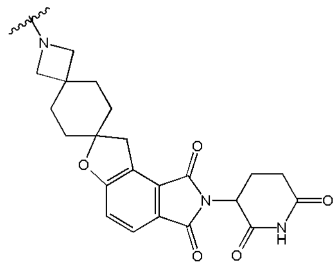

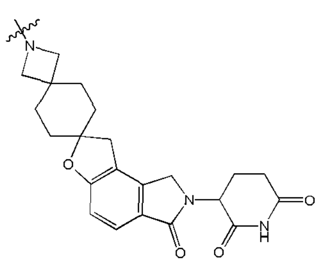
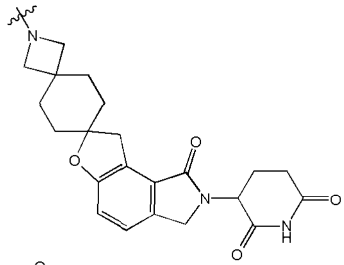
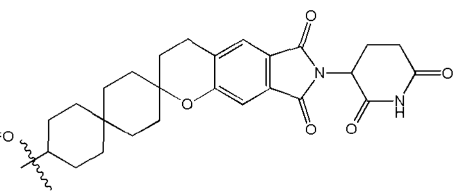

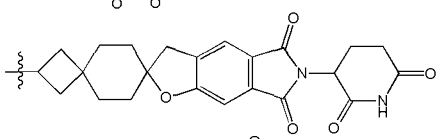
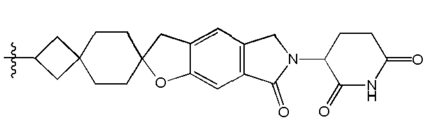

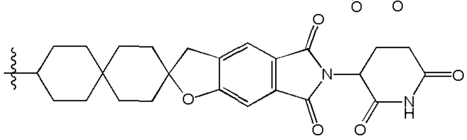
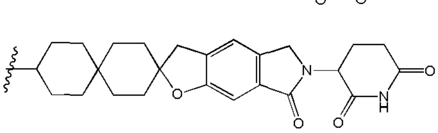

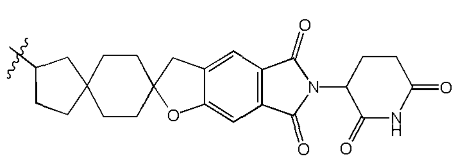
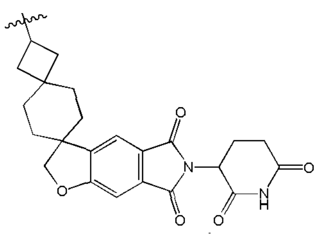
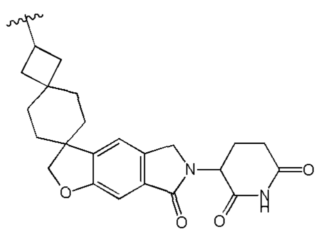

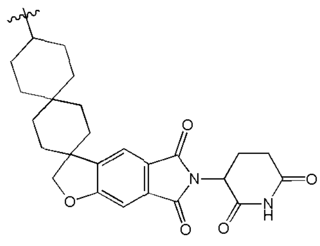
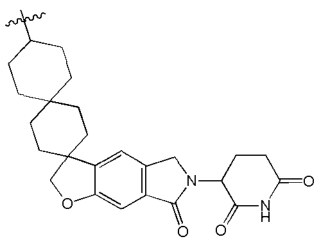
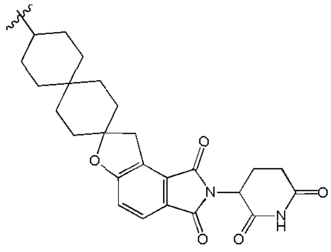

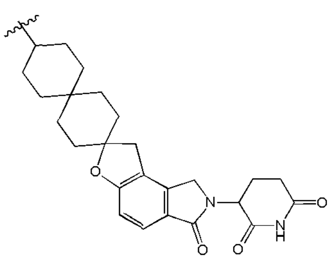
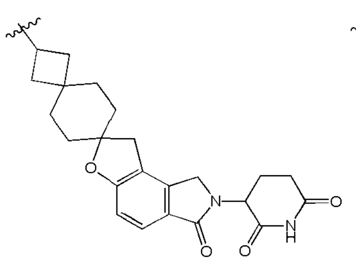
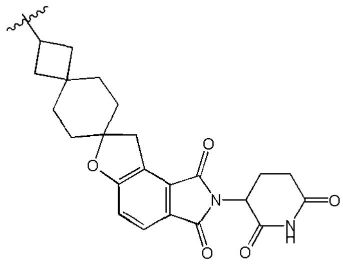

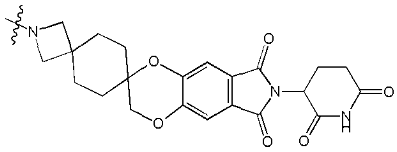
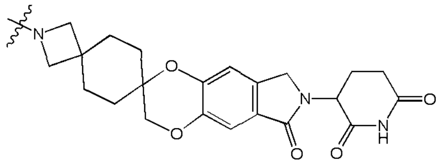

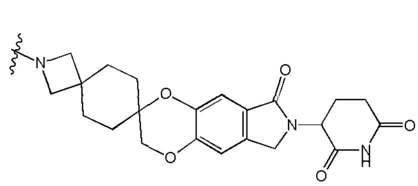
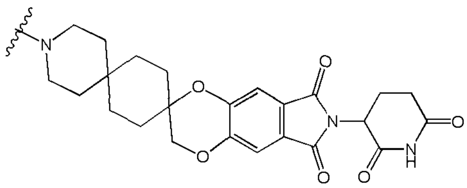

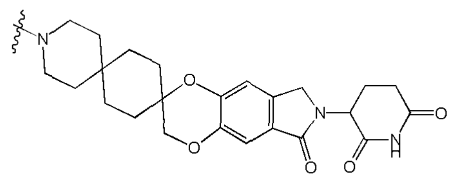

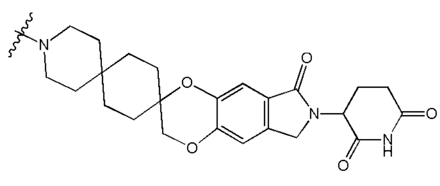
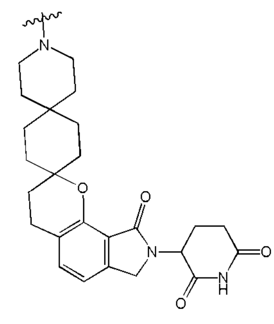
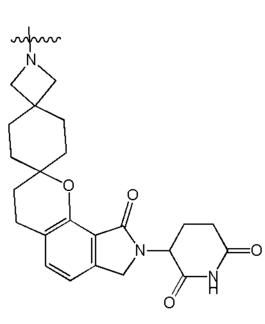

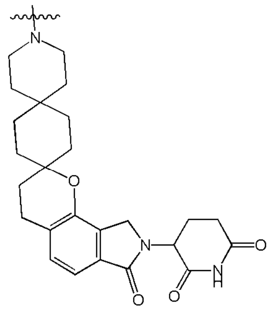
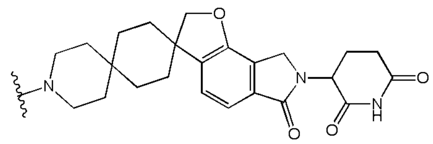
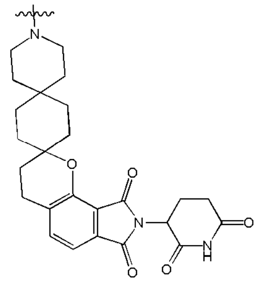
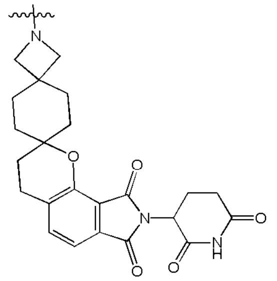
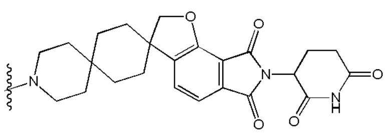
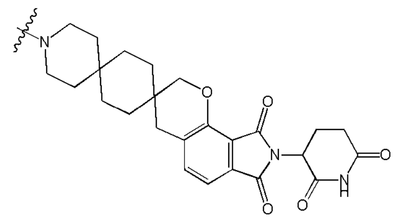
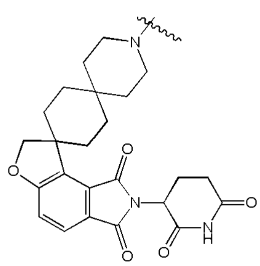
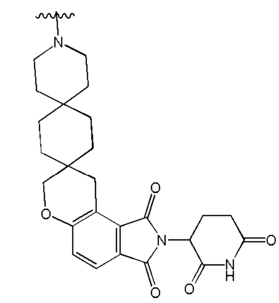
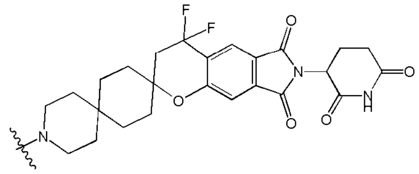
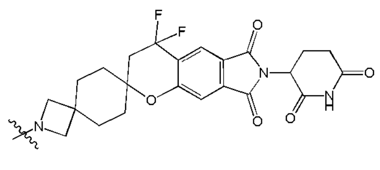
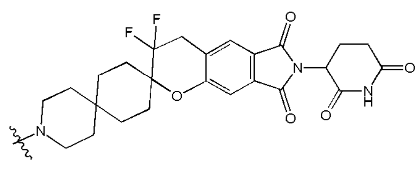
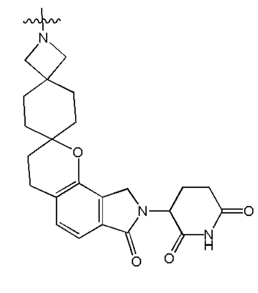
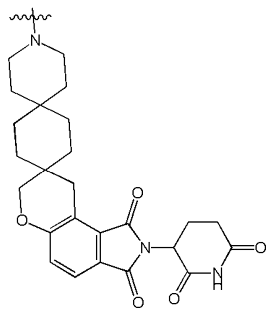
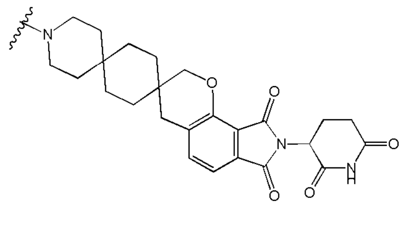
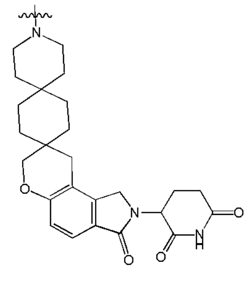

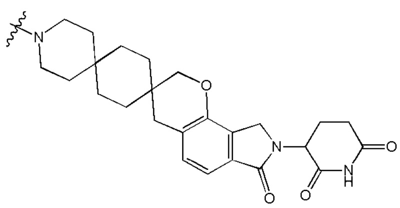
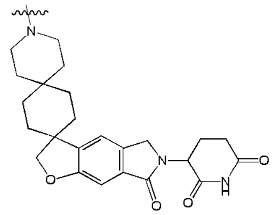
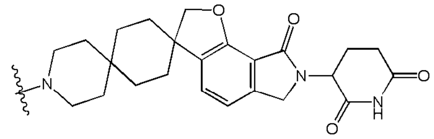

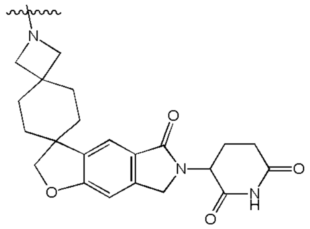
,
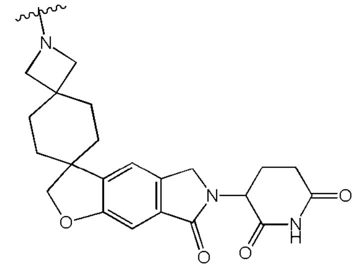
,

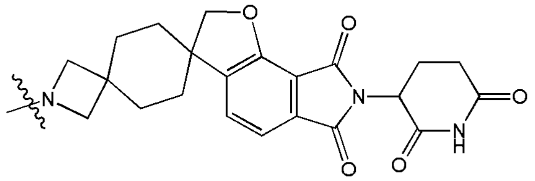
,
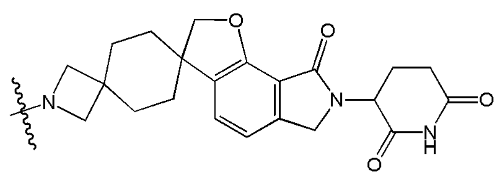

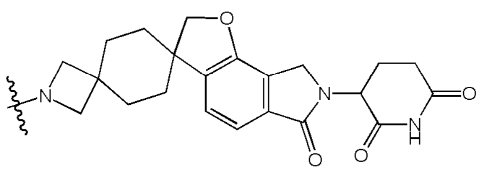
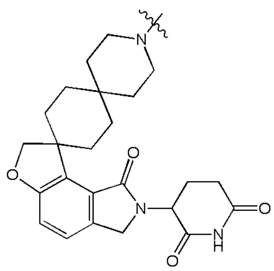
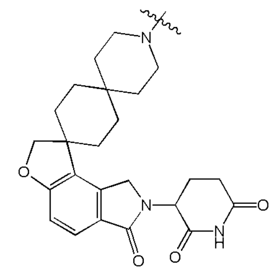

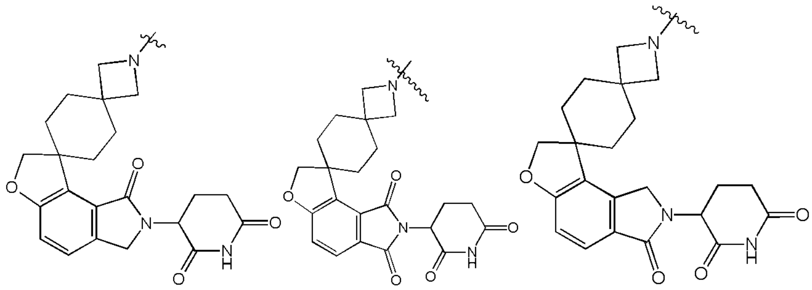
,
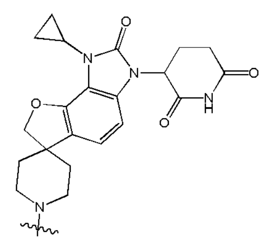

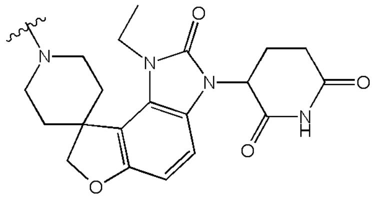
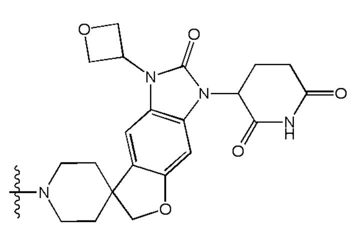
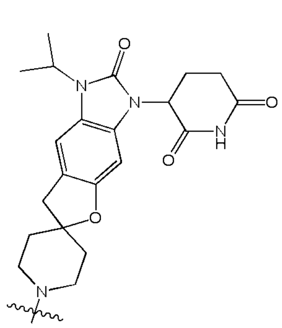

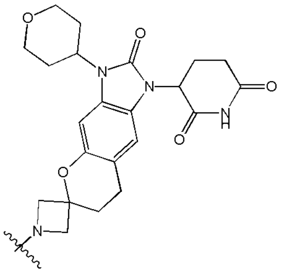
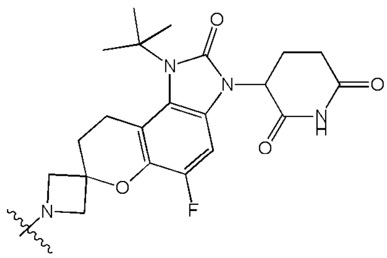
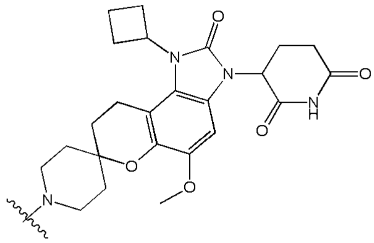

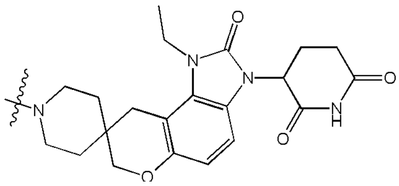
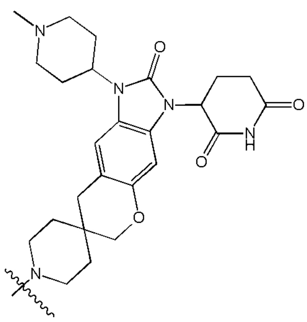
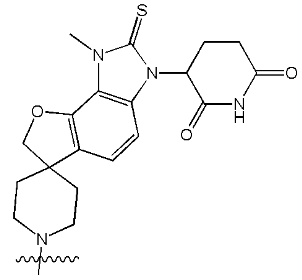

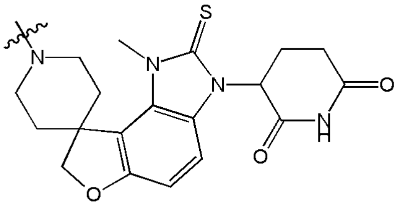
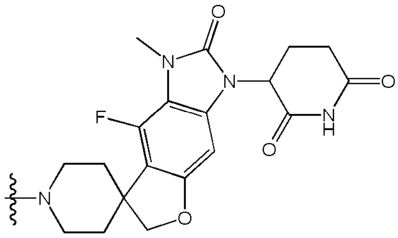
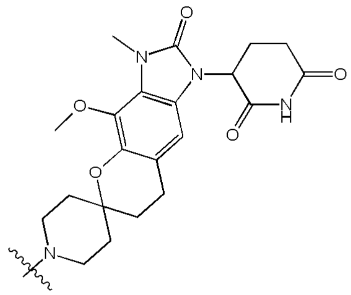

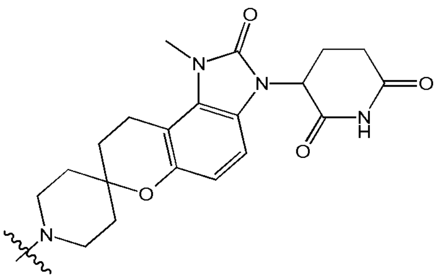
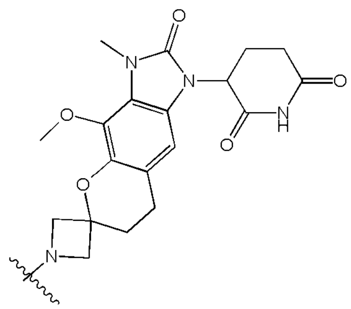

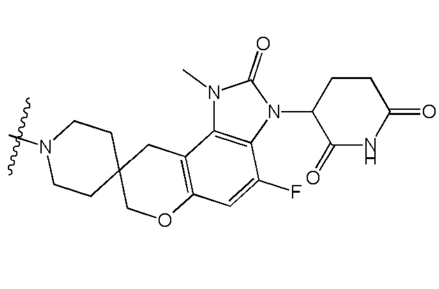
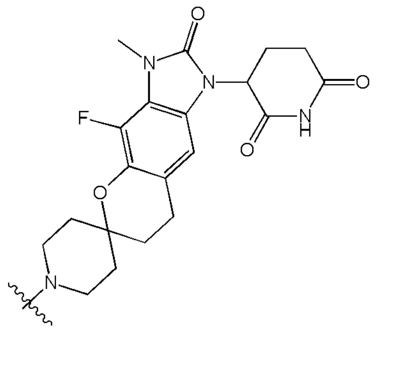

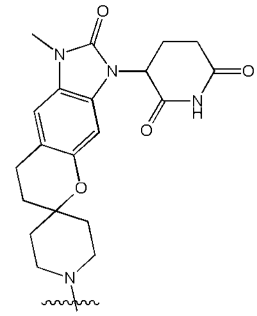,
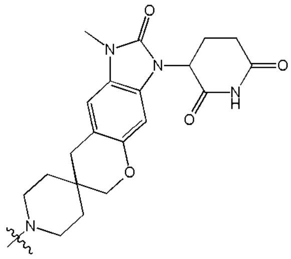,
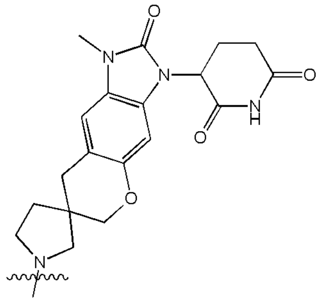,
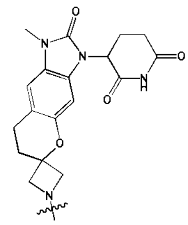,
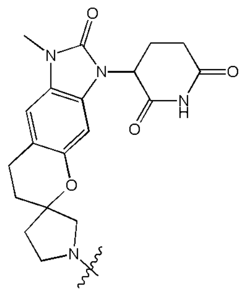,
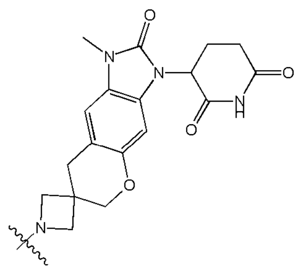,
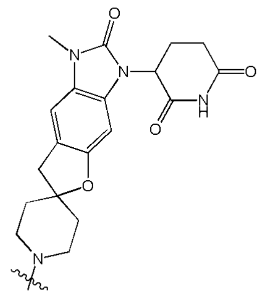,
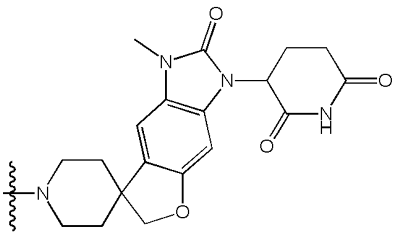,
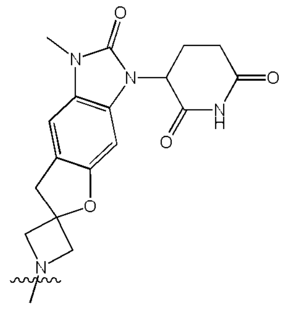,
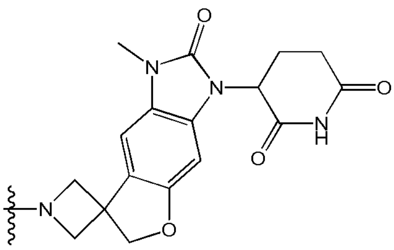,

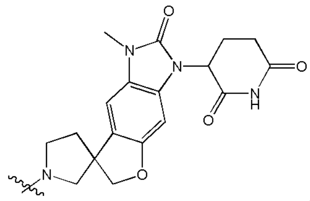,
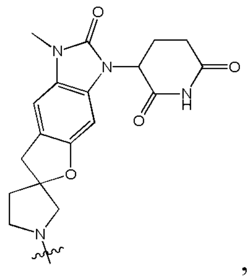,

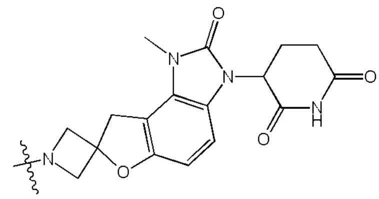,
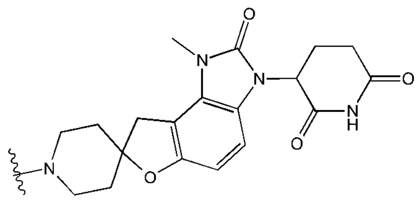,
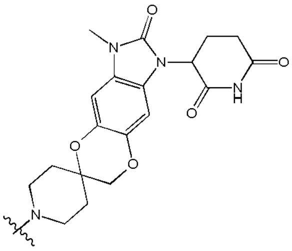,

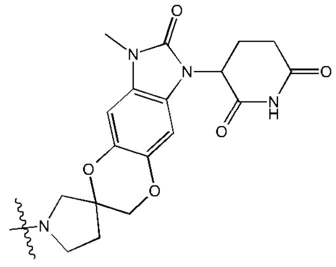,
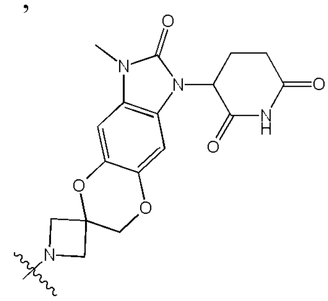,
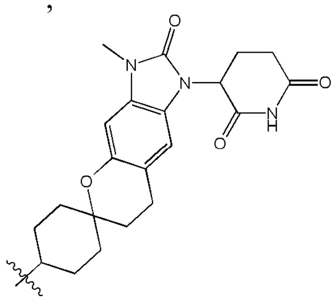,

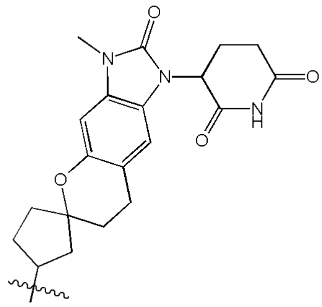,
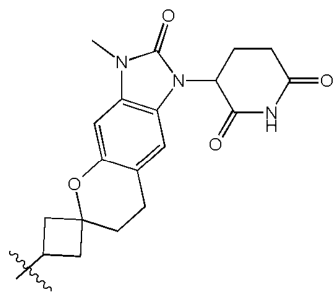,
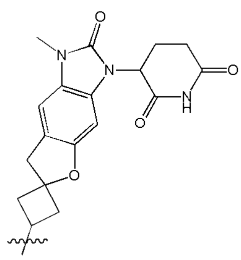,

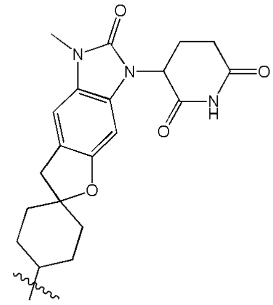,
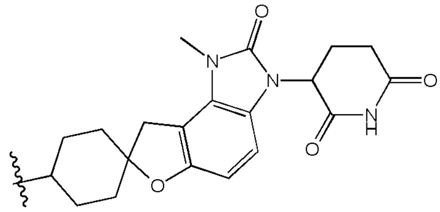,
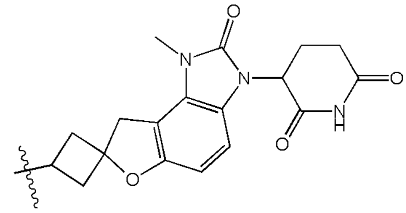,

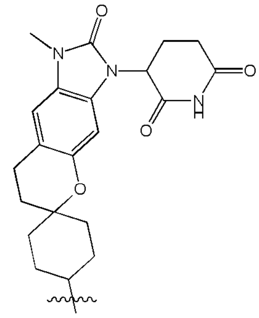,
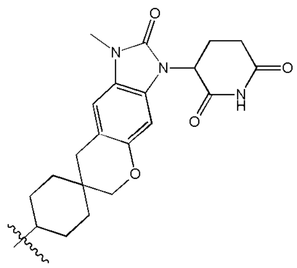,
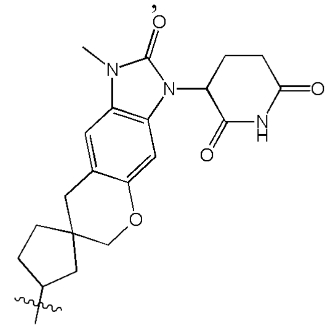,
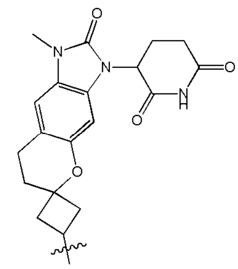,

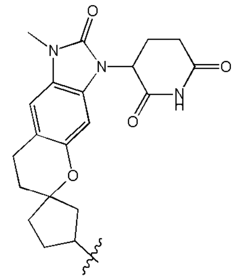,
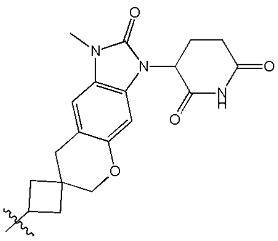,
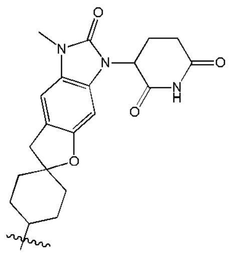,
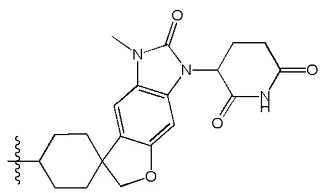,

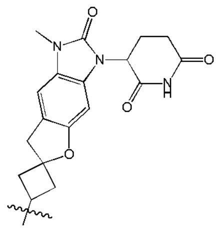,
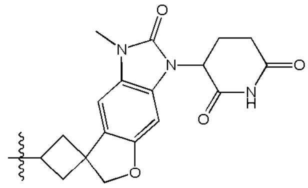,
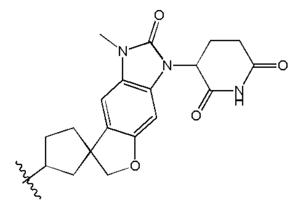,
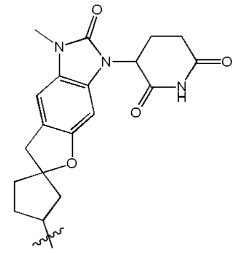,

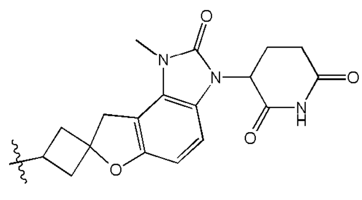,
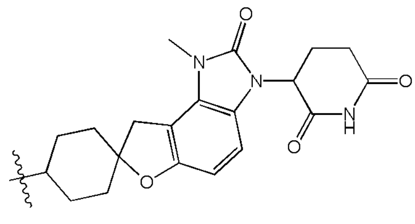,
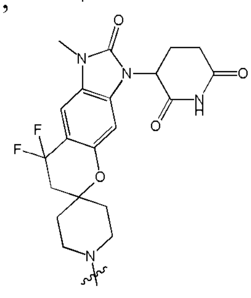,

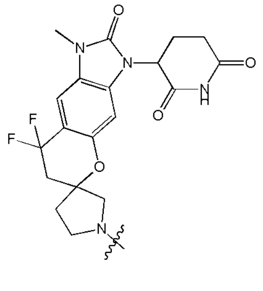,
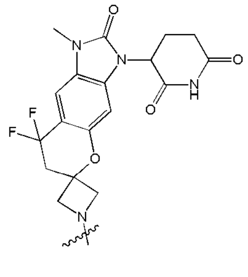,
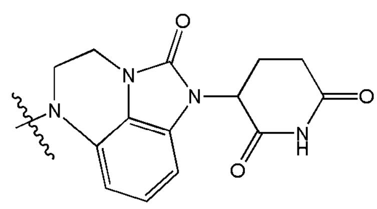,

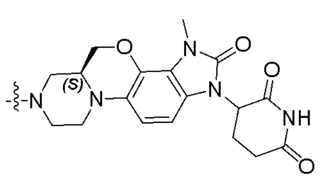 和 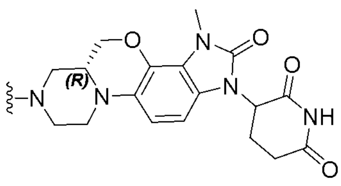 ;

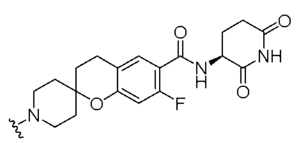

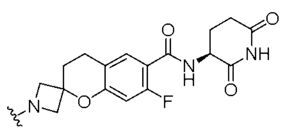

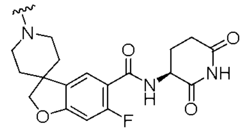

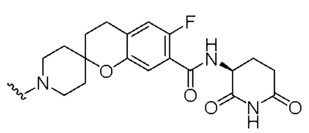

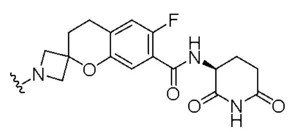

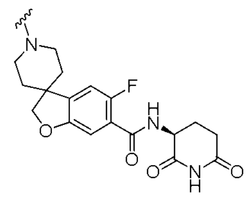

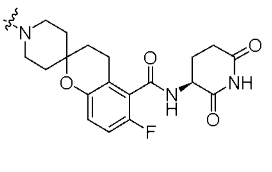

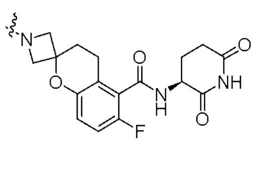

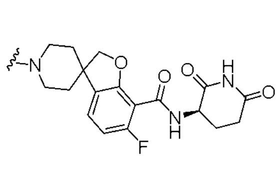

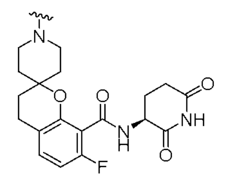

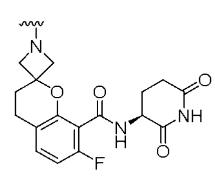

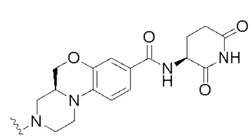

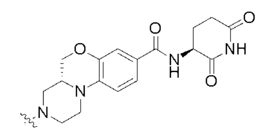

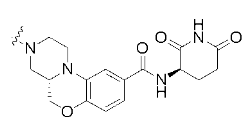

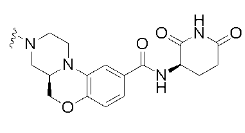

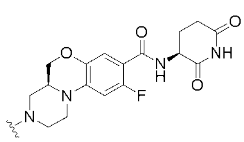

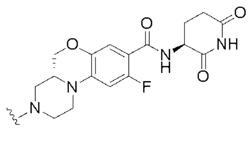

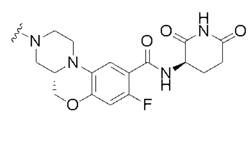

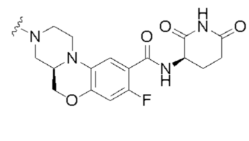

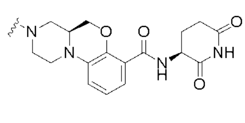

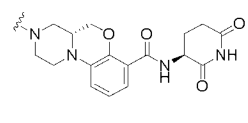

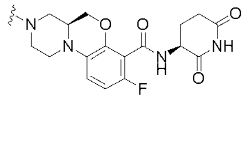

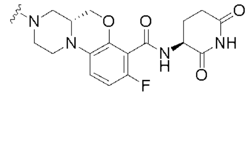

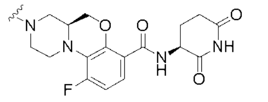
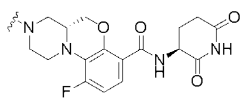

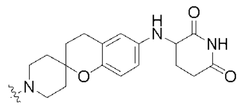
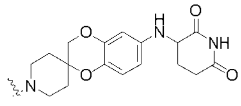
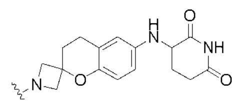
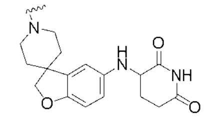

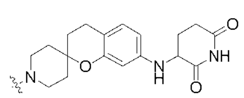
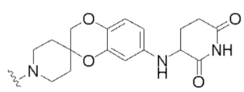
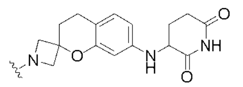
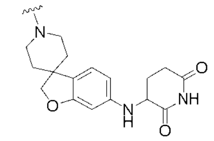

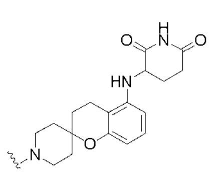
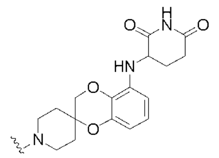
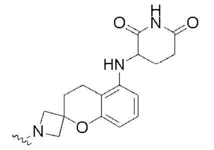
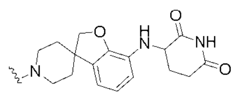

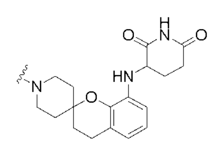
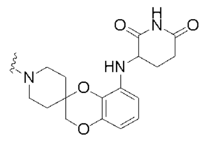
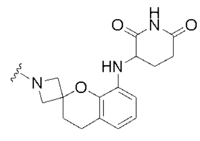

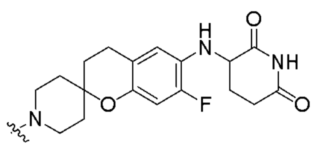
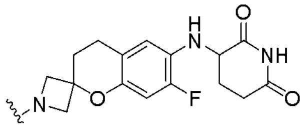
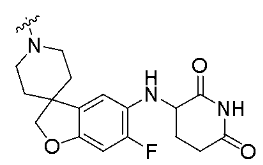

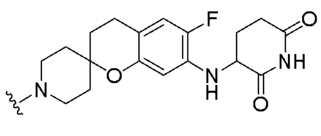
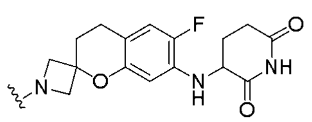
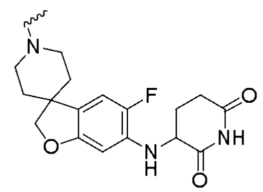

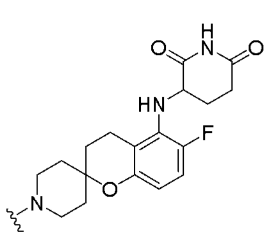
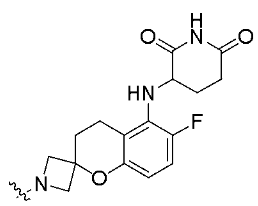
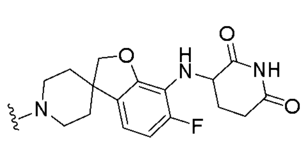

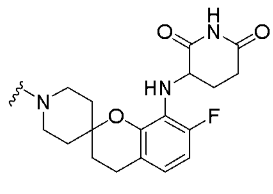
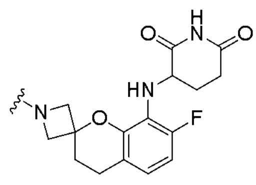

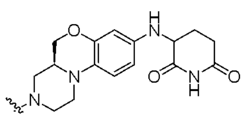
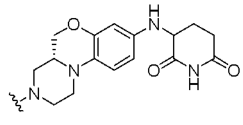
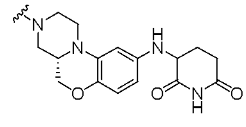
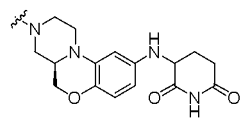

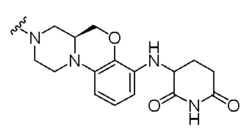
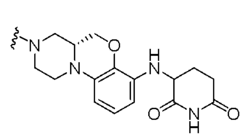
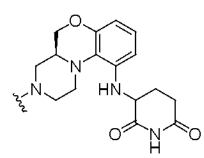
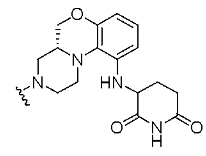

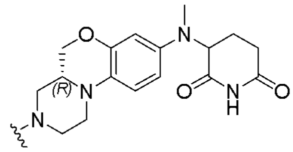
,
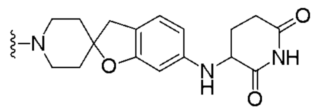
,
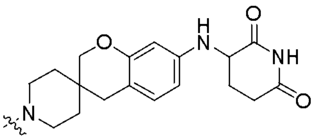

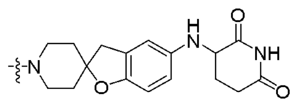
,
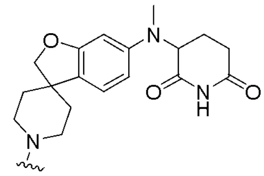
,

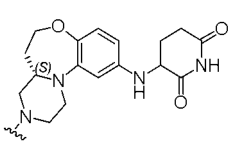
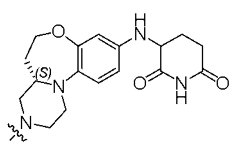
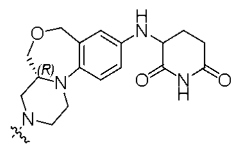
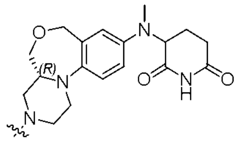

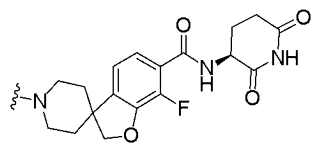
,
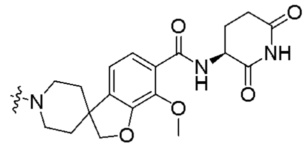
,
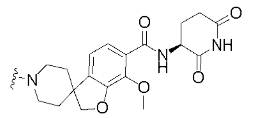
,

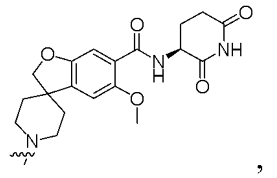
,

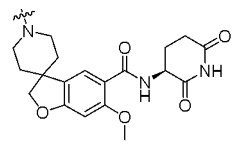
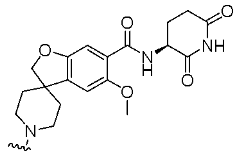
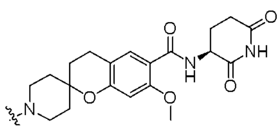
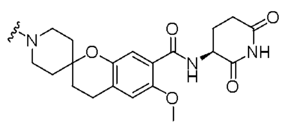

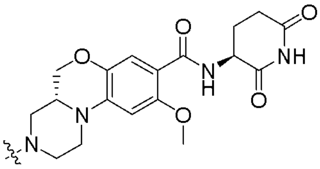
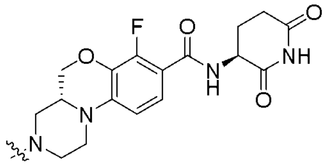
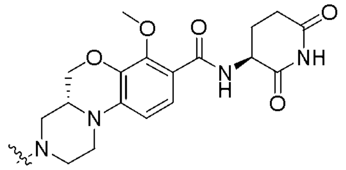

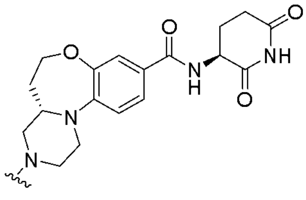

,

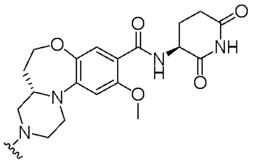

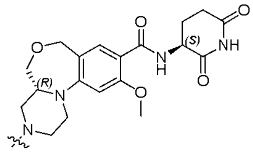

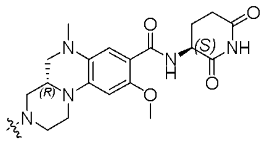

and

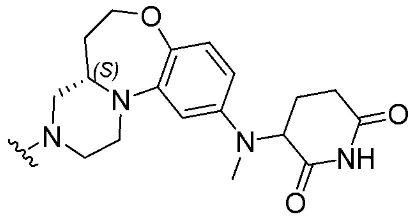

.

**11.** The compound according to any one of claims 7 to 10, wherein the L is a bond or $-(B^L)_q-$;

each occurrence of $B^L$ is identical or different and is independently selected from $CR^{L1}R^{L2}$, O, S, SO, $SO_2$, $NR^{L3}$, $SO_2NR^{L3}$, $SONR^{L3}$, $CONR^{L3}$, $NR^{L3}CONR^{L4}$, $NR^{L3}SO_2NR^{L4}$ CO, $CR^{L1} = CR^{L2}$, C≡C, $SiR^{L1}R^{L2}$, $P(O)R^{L1}$, $P(O)OR^{L1}$, $NR^{L3}C(=NCN)NR^{L4}$, $NR^{L3}C(=NCN)$, $NR^{L3}C(= CNO_2)NR^{L4}$, cycloalkylene, heterocyclylene, arylene, or heteroarylene, wherein the cycloalkylene, heterocyclylene, arylene, and heteroarylene are optionally substituted by 0 to 6 $R^{L1}$ and /or $R^{L2}$;

each occurrence of $R^{L1}$, $R^{L2}$, $R^{L3}$, and $R^{L4}$ is independently selected from H, halogen, $C_{1-8}$ alkyl, O-$C_{1-8}$ alkyl, S-$C_{1-8}$ alkyl, NH-$C_{1-8}$ alkyl, N($C_{1-8}$ alkyl)$_2$, $C_{3-11}$ cycloalkyl, aryl, heteroaryl, $C_{3-11}$ heterocyclyl, O-$C_{3-8}$ cycloalkyl, O-$C_{3-11}$ heterocyclyl, O-aryl, O-heteroaryl, S-$C_{3-8}$ cycloalkyl, NH-$C_{3-8}$ cycloalkyl, N($C_{3-8}$ cycloalkyl)$_2$, N($C_{3-8}$ cycloalkyl)($C_{1-8}$ alkyl), NH-$C_{3-8}$ heterocyclyl, N($C_{3-8}$ heterocyclyl)$_2$, N($C_{3-8}$ heterocyclyl)($C_{1-8}$ alkyl), NH-aryl, N(aryl)($C_{1-8}$ alkyl), NH-heteroaryl, N(heteroaryl)($C_{1-8}$ alkyl), OH, $NH_2$, SH, $SO_2P(O)$(O-$C_{1-8}$ alkyl)($C_{1-8}$ alkyl), P(O)(O-$C_{1-8}$ alkyl)$_2$, C≡C-$C_{1-8}$ alkyl, C≡CH, CH = CH-($C_{1-8}$ alkyl), C($C_{1-8}$ alkyl) = CH-($C_{1-8}$ alkyl), C($C_{1-8}$ alkyl) =C($C_{1-8}$ alkyl)$_2$, $Si(OH)_3$, Si($C_{1-8}$ alkyl)$_3$, Si(OH)($C_{1-8}$ alkyl)$_2$, CO-$C_{1-8}$ alkyl, $CO_2H$, CN, $CF_3$, $CHF_2$, $CH_2F$, $NO_2$, $SF_5$, $SO_2NH$-$C_{1-8}$ alkyl, $SO_2N$($C_{1-8}$ alkyl)$_2$, SONH-$C_{1-8}$ alkyl, SON($C_{1-8}$ alkyl)$_2$, CONH-$C_{1-8}$ alkyl, CON($C_{1-8}$ alkyl)$_2$, N($C_{1-8}$ alkyl)CONH($C_{1-8}$ alkyl), N($C_{1-8}$ alkyl)CON($C_{1-8}$ alkyl)$_2$, NHCONH($C_{1-8}$ alkyl), NHCON($C_{1-8}$ alkyl)$_2$, $NHCONH_2$, N($C_{1-8}$ alkyl)$SO_2NH$($C_{1-8}$ alkyl), N($C_{1-8}$ alkyl)$SO_2N$($C_{1-8}$ alkyl)$_2$, $NHSO_2NH$($C_{1-8}$ alkyl), NH $SO_2N$($C_{1-8}$ alkyl)$_2$, and NH $SO_2NH_2$, optionally, the $C_{1-8}$ alkyl, $C_{3-11}$ cycloalkyl, $C_{3-11}$ heterocyclyl, $C_{6-10}$ aryl, and $C_{5-10}$ heteroaryl are each independently optionally substituted by one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, halocycloalkyl, haloheteroalkyl, alkylamino, aryl, heteroaryl, haloaryl, and haloheteroaryl; and

q is an integer greater than or equal to 1; preferably, q is 1, 2, 3, 4, 5, 6, 7, 8, 9, 10, 11, 12, 13, 14, 15, 16, 17, 18, 19, or 20.

**12.** The compound according to claim 11, wherein the $B^L$ is selected from one or more of the following structures: -O-, -S-, -SO-, $-SO_2-$, $-CH_2-$, -CO-, -NH-, $-N(CH_3)-$,

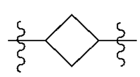

,

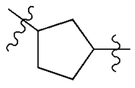, 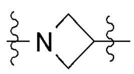, 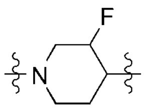, 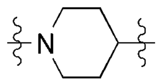, 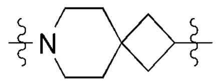, 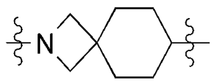,

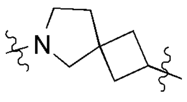, 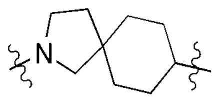, 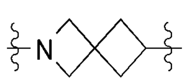, 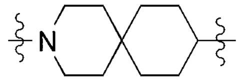, 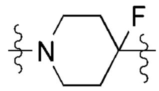,

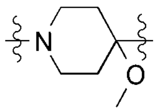, 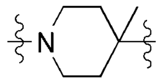, 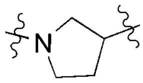, 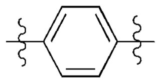, 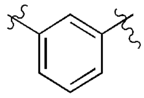, 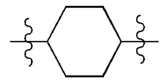,

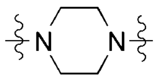, 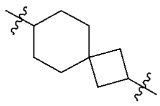, 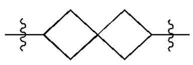, 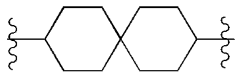, 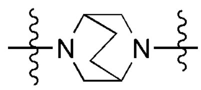,

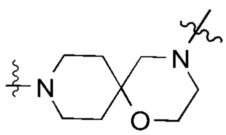, 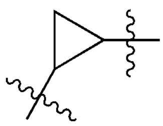, 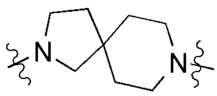, 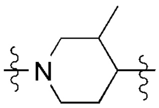, and 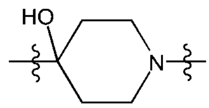;

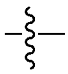

is a point of attachment.

**13.** The compound according to claim 12, wherein the L is selected from the following structures:

a covalent bond, $-(CH_2)_j-$, $-(CH_2)_p-NH-(CH_2)_s-$, $-(CH_2)_y-NH-(CH_2)_j-NH-(CH_2)_s-$, $-(CH_2)_p-CO-(CH_2)_s-$, $-(CH_2)_p-O-(CH_2)_s-$, $-(CH_2)_y-CO-(CH_2)_j-CO-(CH_2)_s-$, $-(CH_2)_y-O-(CH_2)_j-O-(CH_2)_s-$, $-(CH_2)_y-O-(CH_2)_j-CO-(CH_2)_s-$, $-(CH_2)_y-CO-(CH_2)_j-O-(CH_2)_s-$, $-(CH_2)_p-NH-(CH_2)_y-O-(CH_2)_j-CO-(CH_2)_s-$, $-(CH_2)_y-CO-(CH_2)_j-O-(CH_2)_s-NH-(CH_2)_p-$,

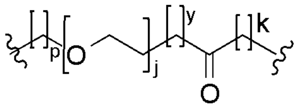, 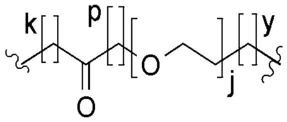, 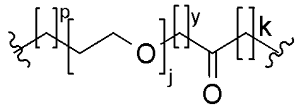,

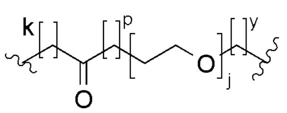, 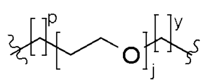, 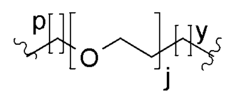,

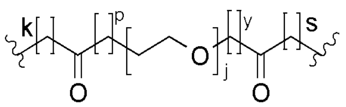, 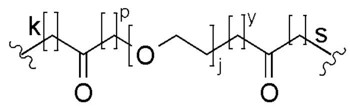, 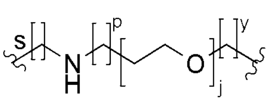,

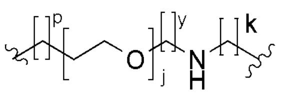, 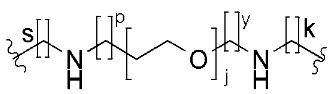, 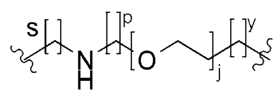,

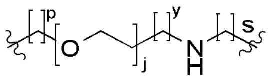, 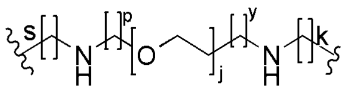, 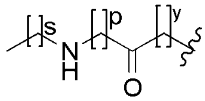, 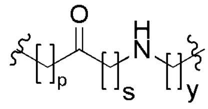,

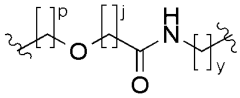, 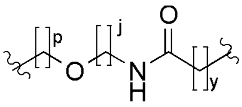, 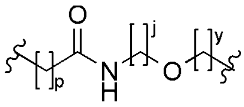, 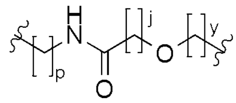,

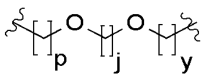, 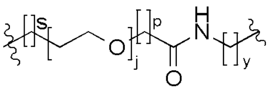, 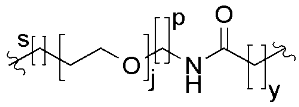,

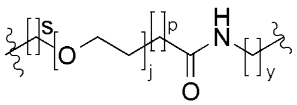, 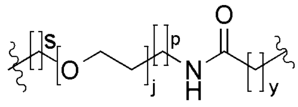, 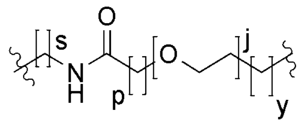,

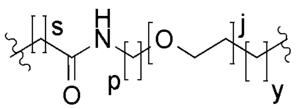, 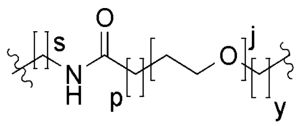, 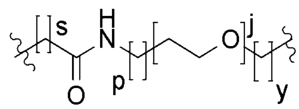,

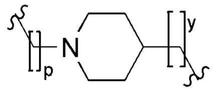, 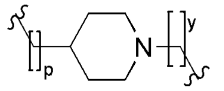, 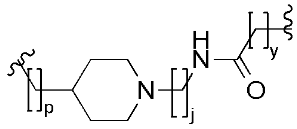, 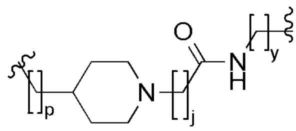,

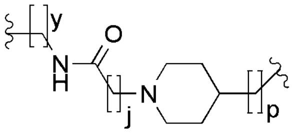, 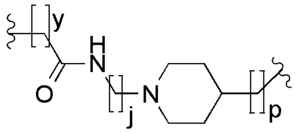, 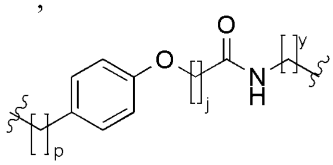,

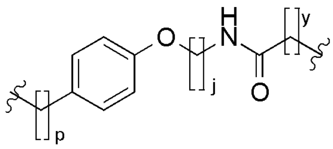, 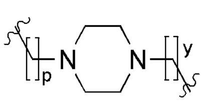, 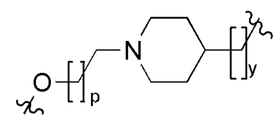,

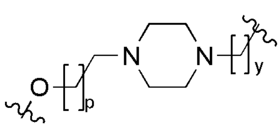, 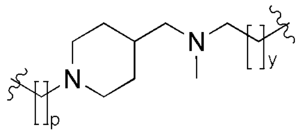, 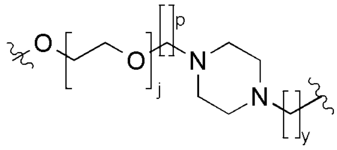,

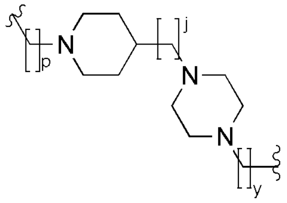, 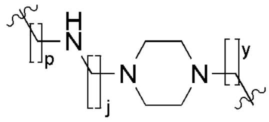, 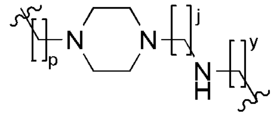,

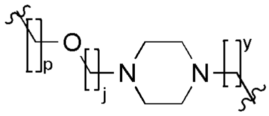 , 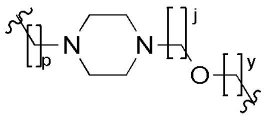 , 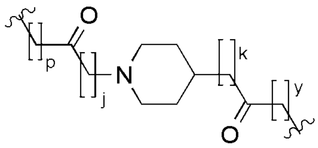 ,

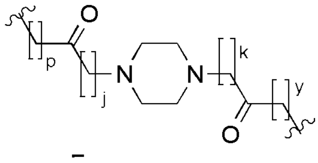 , 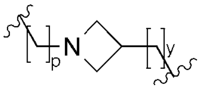 , 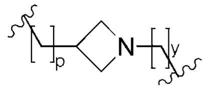 , 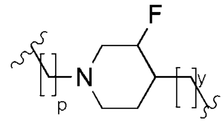 ,

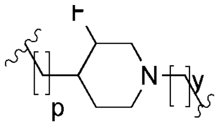 , 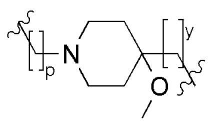 , 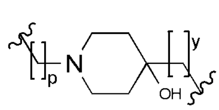 , 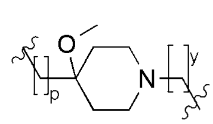 ,

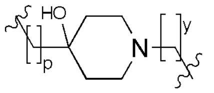 , 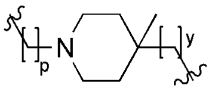 , 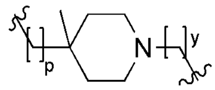 , 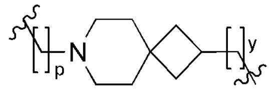 ,

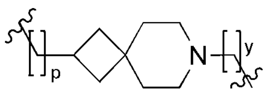 , 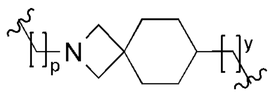 , 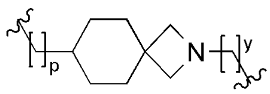 ,

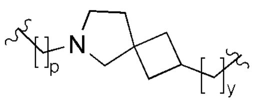 , 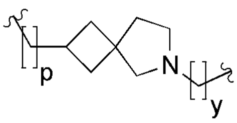 , 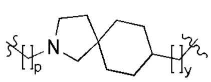 , 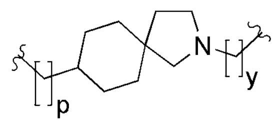 ,

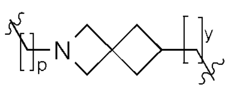 , 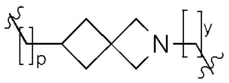 , 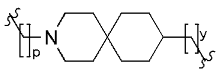 , 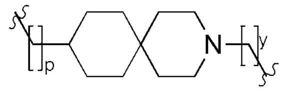 ,

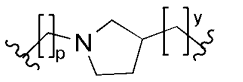 , 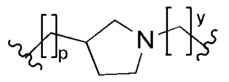 , 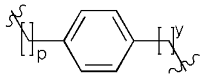 , 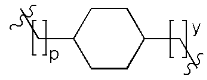 ,

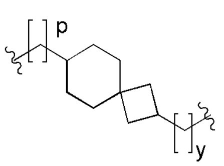 , 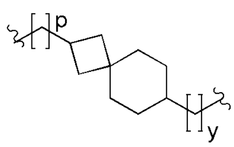 , 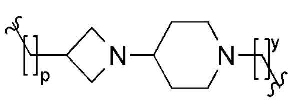 , 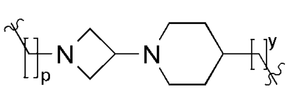 ,

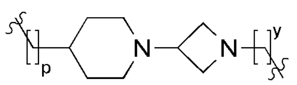 , 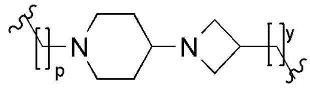 , 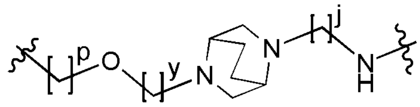 ,

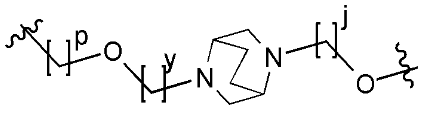, 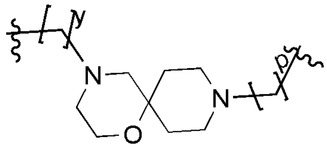, 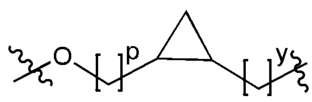,

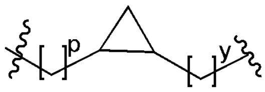, 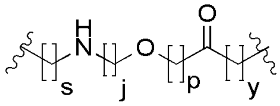, 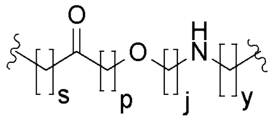, 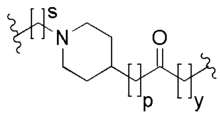,

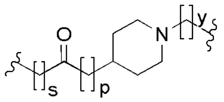, 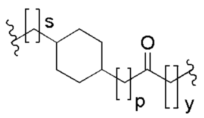, 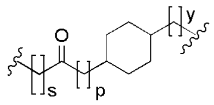, 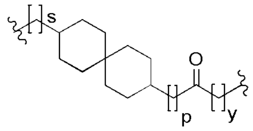,

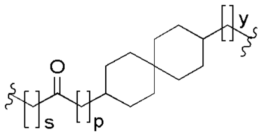, 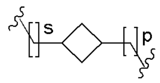, 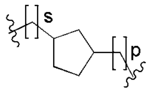, 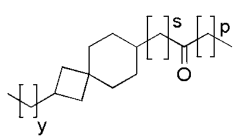,

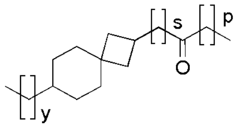, 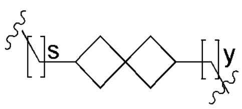, 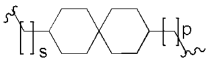, 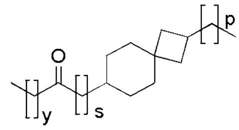,

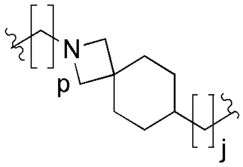, 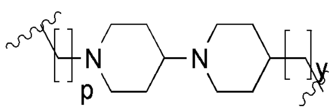, 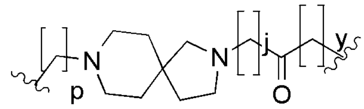,

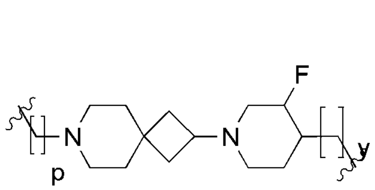, 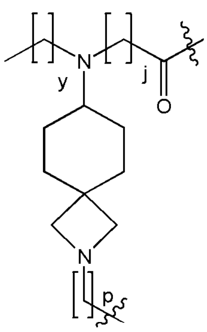, 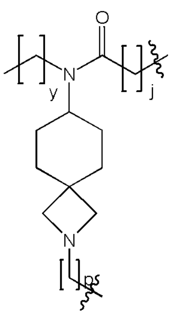, 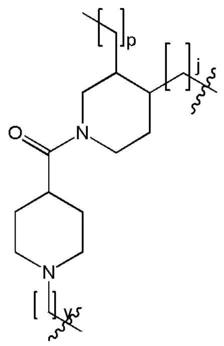,

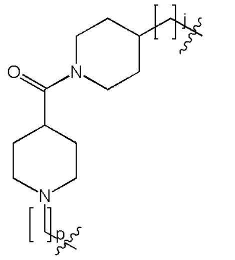, 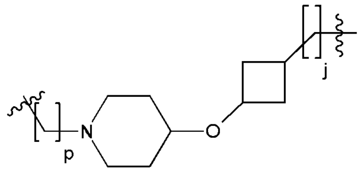, 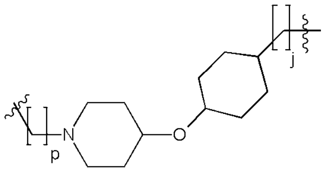,

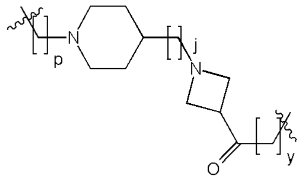, 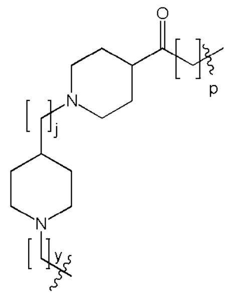, 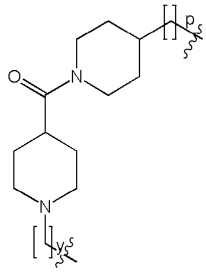, 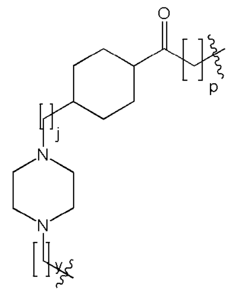,

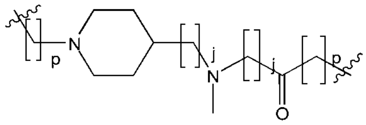, 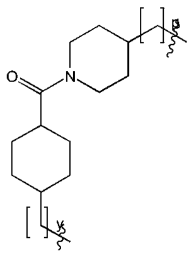, 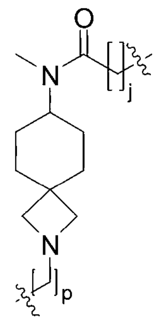, 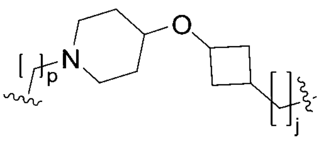,

and

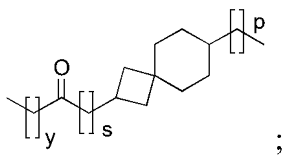;

wherein, j is 1, 2, 3, 4, 5, 6, 7, 8, 9, or 10;
k, s, p, and y are 0, 1, 2, 3, 4, 5, 6, 7, 8, 9, or 10;

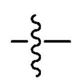

is the point of attachment to the CLM and the PTM;
preferably, the L is selected from the following structures:
a covalent bond, $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-NH-CH_2-$, $-NH-(CH_2)_2-$, $-NH-(CH_2)_3-$, $-NH-(CH_2)_4-$, $-NH-(CH_2)_5-$, $-NH-(CH_2)_6-$, $-NH-(CH_2)_7-$, $-NH-(CH_2)_8-$, $-CO-NH-CH_2-$, $-CO-NH-(CH_2)_2-$, $-CO-NH-(CH_2)_3-$, $-CO-NH-(CH_2)_4-$, $-CO-NH-(CH_2)_5-$, $-CO-NH-(CH_2)_6-$, $-CO-NH-(CH_2)_7-$, $-CO-NH-(CH_2)_8-$, $-CH_2-NH-$, $-(CH_2)_2-NH-$, $-(CH_2)_3-NH-$, $-(CH_2)_4-NH-$, $-(CH_2)_5-NH-$, $-(CH_2)_6-NH-$, $-(CH_2)_7-NH-$, $-(CH_2)_8-NH-$, $-NH-CH_2-NH-$, $-NH-(CH_2)_2-NH-$, $-NH-(CH_2)_3-NH-$, $-NH-(CH_2)_4-NH-$, $-NH-(CH_2)_5-NH-$, $-NH-(CH_2)_6-NH-$, $-NH-(CH_2)_7-NH-$, $-NH-(CH_2)_8-NH-$, $-(CH_2-CH_2-O)-CH_2-CH_2-$, $-(CH_2-CH_2-O)_2-CH_2-CH_2-$, $-(CH_2-CH_2-O)_3-CH_2-CH_2-$, $-NH-(CH_2-CH_2-O)-CH_2-CH_2-$, $-NH-(CH_2-CH_2-O)_2-CH_2-CH_2-$, $-NH-(CH_2-CH_2-O)_3-CH_2-CH_2-$, $-CO-NH-(CH_2-CH_2-O)-CH_2-CH_2-$, $-CO-NH-(CH_2-CH_2-O)_2-CH_2-CH_2-$, $-CO-NH-(CH_2-CH_2-O)_3-CH_2-CH_2-$, $-(CH_2-CH_2-O)-CH_2-CH_2-NH-$, $-(CH_2-CH_2-O)_2-CH_2-CH_2-NH-$, $-(CH_2-CH_2-O)_3-CH_2-CH_2-NH-$, $-NH-(CH_2-CH_2-O)-CH_2-CH_2-NH-$,

$-NH-(CH_2-CH_2-O)_2-CH_2-CH_2-NH-$, $-NH-(CH_2-CH_2-O)_3-CH_2-CH_2-NH-$, $-CO-NH-(CH_2-CH_2-O)-CH_2-CH_2-NH-$, $-CO-NH-(CH_2-CH_2-O)_2-CH_2-CH_2-NH-$, $-CO-NH-(CH_2-CH_2-O)_3-CH_2-CH_2-NH-$, $-CH_2-CH_2-(O-CH_2-CH_2)-$, $-CH_2-CH_2-(O-CH_2-CH_2)_2-$, $-CH_2-CH_2-(O-CH_2-CH_2)_3-$, $-NH-CH_2-CH_2-(O-CH_2-CH_2)-$, $-NH-CH_2-CH_2-(O-CH_2-CH_2)_2-$, $-NH-CH_2-CH_2-(O-CH_2-CH_2)_3-$, $-CO-NH-CH_2-CH_2-(O-CH_2-CH_2)-$, $-CO-NH-CH_2-CH_2-(O-CH_2-CH_2)_2-$, $-CO-NH-CH_2-CH_2-(O-CH_2-CH_2)_3-$, $-CH_2-CH_2-(O-CH_2-CH_2)-NH-$, $-CH_2-CH_2-(O-CH_2-CH_2)_2-NH-$, $-CH_2-CH_2-(O-CH_2-CH_2)_3-NH-$, $-NH-CH_2-CH_2-(O-CH_2-CH_2)-NH-$, $-NH-CH_2-CH_2-(O-CH_2-CH_2)_2-NH-$, $-NH-CH_2-CH_2-(O-CH_2-CH_2)_3-NH-$, $-NH-CH_2-CH_2-O-CH_2-CH_2-CO-$, $-CO-CH_2-CH_2-O-CH_2-CH_2-NH-$, $-NH-(CH_2)_4-CO-$, $-NH-(CH_2)_5-CO-$, $-NH-(CH_2)_6-CO-$, $-CO-(CH_2)_4-NH-$, $-CO-(CH_2)_5-NH-$, $-CO-(CH_2)_6-NH-$, $-NH-(CH_2-CH_2-O)-(CH_2)_3-$, $-NH-(CH_2-CH_2-O)-(CH_2)_4-$, $-NH-(CH_2-CH_2-O)-(CH_2)_5-$, $-NH-(CH_2-CH_2-O)-(CH_2)_6-$, $-(CH_2)_3-(O-CH_2-CH_2)-NH-$, $-(CH_2)_4-(O-CH_2-CH_2)-NH-$, $-(CH_2)_5-(O-CH_2-CH_2)-NH-$, $-(CH_2)_6-(O-CH_2-CH_2)-NH-$, $-CH_2-CH_2-O-(CH_2)_2-CO-$, $-CH_2-CH_2-O-(CH_2)_3-CO-$, $-CH_2-CH_2-O-(CH_2)_4-CO-$, $-CO-(CH_2)_2-O-CH_2-CH_2-$, $-CO-(CH_2)_3-O-CH_2-CH_2-$, $-CO-(CH_2)_4-O-CH_2-CH_2-$, $-CO-(CH_2)_2-$, $-CO-(CH_2)_3-$, $-CO-(CH_2)_4-$, $-CO-(CH_2)_5-$, $-CO-(CH_2)_6-$, $-(CH_2)_2-CO-$, $-(CH_2)_3-CO-$, $-(CH_2)_4-CO-$, $-(CH_2)_5-CO-$, $-(CH_2)_6-CO-$, $-CO-(CH_2)_2-CO-$, $-CO-(CH_2)_3-CO-$, $-CO-(CH_2)_4-CO-$, $-CO-(CH_2)_5-CO-$, $-CO-(CH_2)_6-CO-$, $-CH_2-CO-CH_2-$, $-CH_2-CO-(CH_2)_2-$, $-CH_2-CO-(CH_2)_3-$, $-CH_2-CO-(CH_2)_4-$, $-(CH_2)_2-CO-CH_2-$, $-(CH_2)_2-CO-(CH_2)_2-$, $-(CH_2)_2-CO-(CH_2)_3-$, $-(CH_2)_2-CO-(CH_2)_4-$, $-(CH_2)_3-CO-CH_2-$, $-(CH_2)_3-CO-(CH_2)_2-$, $-(CH_2)_3-CO-(CH_2)_3-$, $-(CH_2)_3-CO-(CH_2)_4-$, $-(CH_2)_4-CO-CH_2-$, $-(CH_2)_4-CO-(CH_2)_2-$, $-(CH_2)_4-CO-(CH_2)_3-$, $-(CH_2)_4-CO-(CH_2)_4-$, $-CH_2-O-CH_2-$, $-CH_2-O-(CH_2)_2-$, $-CH_2-O-(CH_2)_3-$, $-CH_2-O-(CH_2)_4-$, $-(CH_2)_2-O-CH_2-$, $-(CH_2)_2-O-(CH_2)_2-$, $-(CH_2)_2-O-(CH_2)_3-$, $-(CH_2)_2-O-(CH_2)_4-$, $-(CH_2)_3-O-CH_2-$, $-(CH_2)_3-O-(CH_3)_2-$, $-(CH_2)_3-O-(CH_2)_3-$, $-(CH_2)_3-O-(CH_2)_4-$, $-(CH_2)_4-O-CH_2-$, $-(CH_2)_4-O-(CH_2)_2-$, $-(CH_2)_4-O-(CH_2)_3-$, $-(CH_2)_4-O-(CH_2)_4-$,

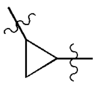, 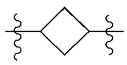, 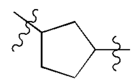, 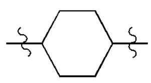, 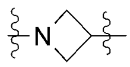, 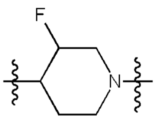,

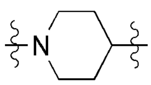, 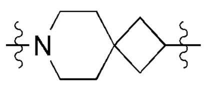, 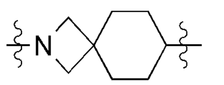, 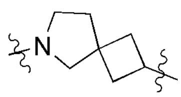, 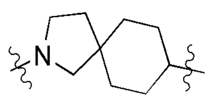,

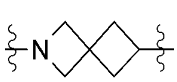, 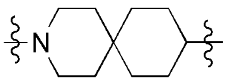, 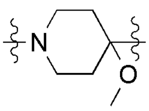, 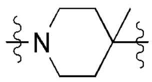, 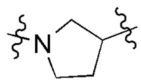,

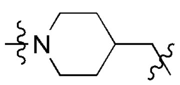, 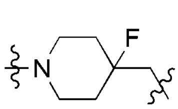, 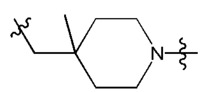, 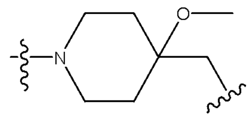,

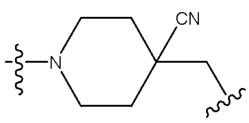, 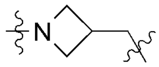, 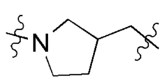, 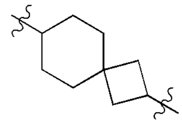, 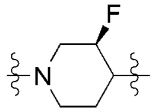,

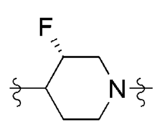, 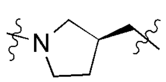, 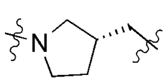, 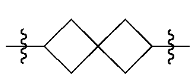, 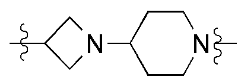,

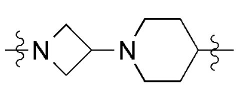, 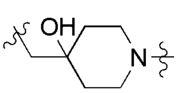, 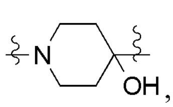, 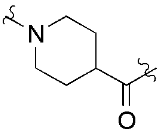, 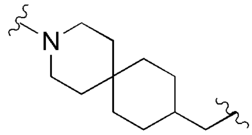,

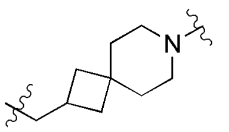 , 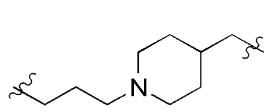 , 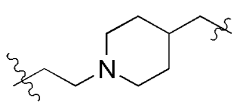 , 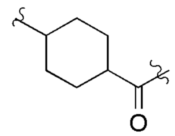 ,

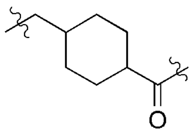 , 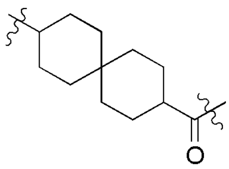 , 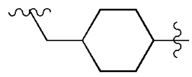 , 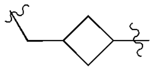 , 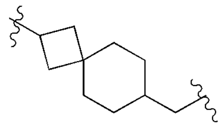 ,

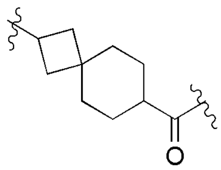 , 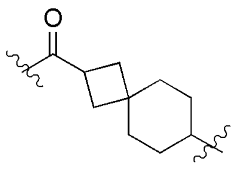 , 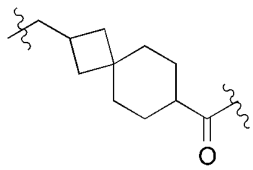 , 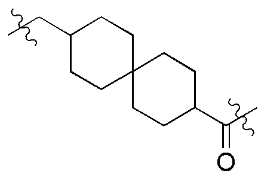 ,

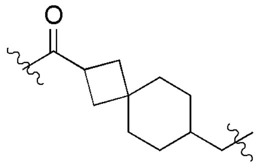 , 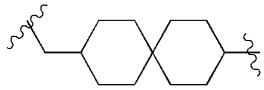 , 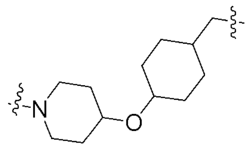 , 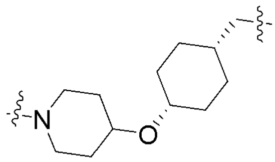 ,

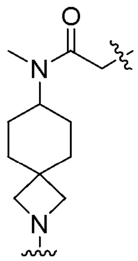 , 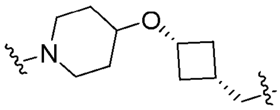 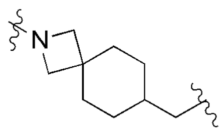 , 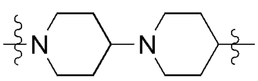 ,

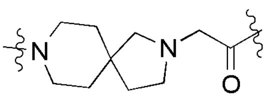 , 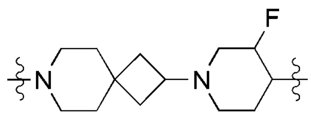 , 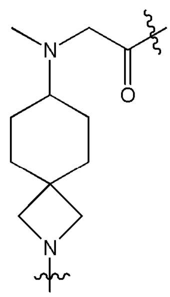 , 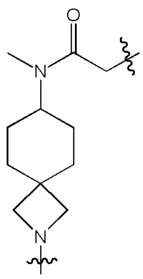 ,

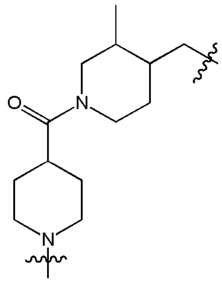, 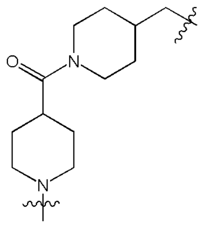, 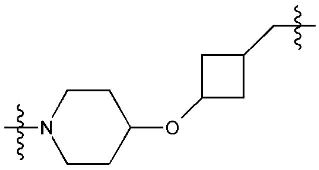, 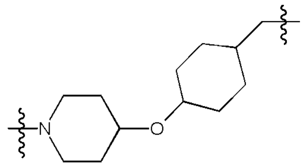,

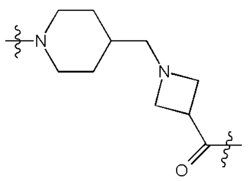, 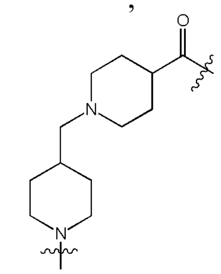, 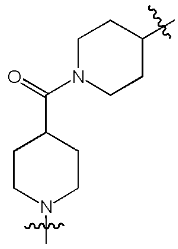, 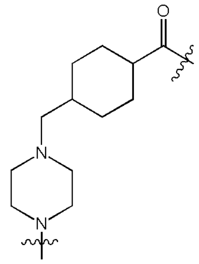,

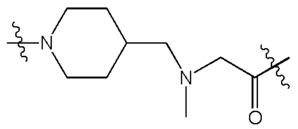, 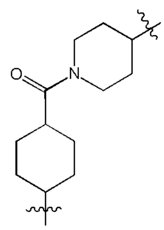, and 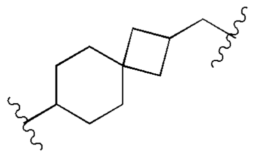.

**14.** The compound according to any one of claims 7 to 13, wherein the PTM is a moiety that binds to a target protein or polypeptide, wherein the target protein is selected from: structural proteins, receptors, enzymes, cell surface proteins; proteins related to cellular integration functions, including those involved in catalytic activity, aromatase activity, motor activity, helicase activity, metabolic processes, antioxidant activity, proteolysis, biosynthesis; proteins having kinase activity, oxidoreductase activity, transferase activity, hydrolase activity, lyase activity, isomerase activity, ligase activity, enzyme regulatory activity, signal transduction activity, structural molecular activity, binding activity, receptor activity, cell motility, membrane fusion, cell communication, regulation of biological processes, development, cell differentiation, response to stimulus; behavioral proteins; cell adhesion proteins; proteins involved in cell necrosis; and proteins involved in transport, including proteins with protein transport activity, nuclear transport activity, ion transport activity, channel transport activity, carrier activity, permease activity, secretory activity, electron transport activity, pathogenesis, chaperone regulator activity, nucleic acid binding activity, transcription regulator activity, activity in extracellular organization and biogenesis, and translation regulator activity.

**15.** The compound according to any one of claims 7 to 14, wherein the PTM is a moiety that binds to a target protein or polypeptide, wherein the target protein is selected from B7.1 and B7, TNFR2, NADPH oxidase, Bcll/Bax and other partners in the apoptosis pathway, C5a receptor, HMG-CoA reductase, PDE V phosphodiesterase type, PDEIV phosphodiesterase type 4, PDEI I, PDEI II, PDE III, squalene epoxidase, CXCR1, CXCR2, nitric oxide (NO) synthase, cyclooxygenase 1, cyclooxygenase 2, 5HT receptor, dopamine receptor, G proteins (i.e., Gq), histamine receptor, 5-lipoxygenase, tryptase serine protease, thymidylate synthase, purine nucleoside phosphorylase, GAPDH trypanosome, glycogen phosphorylase, carbonic anhydrase, chemokine receptor, JAW STAT, RXR and analogs thereof, HIV1 protease, HIV1 integrase, influenza neuraminidase, hepatitis B reverse transcriptase, sodium channel, multidrug-resistant bacteria, protein P-glycoprotein, tyrosine kinase, CD23, CD124, tyrosine kinase p56lck, CD4, CD5, IL-2 receptor, IL-1 receptor, TNF-$\alpha$R, ICAM1, $Ca^{2+}$ channel, VCAM, VLA-4 integrin, selectin, CD40/CD40L, inosine monophosphate dehydrogenase, p38 MAP kinase, Ras/Raf/MEK/ERK pathway, interleukin-1 converting enzyme, caspase, HCV, NS3 protease, HCV NS3 RNA helicase, glycinamide ribonucleotide formyltransferase, rhinovirus, 3C protease, herpes simplex virus-I (HSV-I), protease, cytomegalovirus (CMV) protease, poly(ADP-ribose) polymerase, cyclin-dependent kinase 4/6, vascular endothelial growth factor, oxytocin receptor, microsomal transfer protein inhibitor, bile acid transporter inhibitor, 5$\alpha$ reductase inhibitor, angiotensin II, glycine receptor, norepinephrine reuptake receptor, endothelin receptor, neuropeptide Y and receptor, adenosine receptor, adenosine kinase and AMP deaminase, purinergic receptors (P2Y 1, P2Y2, P2Y4, P2Y6, P2X1-7), farnesyltransferase, geranylgeranyltransferase, TrkA receptor for NGF, β-amyloid, tyrosine kinase Flk-II KDR, vitronectin receptor, integrin receptor, Her-2/neu, telomerase inhibition, cytosolic phospholipase A2, EGF receptor tyrosine kinase, ecdysone 20-monooxygenase, ion channel of the GABA-gated chloride channel, acetylcholinesterase, voltage-sensitive sodium channel protein, calcium release channel and chloride channel, acetyl-CoA carboxylase, adenylosuccinate synthase, protoporphyrinogen oxidase, enolpyruvylshikimate phosphate synthase, MYC protein, androgen receptor, estrogen receptor, interleukin-1 receptor-associated kinase 4 (IRAK4), Aurora kinase A, Bcr-Abl protein, epidermal growth factor receptor, anaplastic lymphoma kinase, Bruton's tyrosine kinase, BRM/BRG1 protein, bromodomain-containing protein 4.

**16.** The compound according to any one of claims 7 to 15, wherein the PTM is a moiety of the following compounds: a Hsp90 inhibitor, a kinase inhibitor, a phosphatase inhibitor, a MDM2 inhibitor, a compound that binds to a protein comprising the human BET bromodomain, a HDAC inhibitor, a human lysine methyltransferase inhibitor, a compound that binds to RAF receptor, a compound that binds to FKBP, an angiogenesis inhibitor, an immunosuppressive compound, an compound that binds to aryl hydrocarbon receptor, a compound that binds to androgen receptor, a compound that binds to estrogen receptor, a compound that binds to IRAK4, a compound that binds to thyroid hormone receptor, a compound that binds to HIV protease, a compound that binds to HIV integrase, a compound that binds to HCV protease, a compound that binds to acyl protein thioesterase 1 and/or 2, a compound that binds to c-MYC protein, a compound that binds to laser kinase A, a compound that binds to Bcr-Abl protein, a compound that binds to bromodomain protein 4 (BRD4), a compound that binds to anaplastic lymphoma kinase (ALK), a compound that binds to Bruton's tyrosine kinase (BTK), a compound that binds to cyclin-dependent kinase 4/6 (CDK4/6), a compound that binds to SMARCA2/4 (BRM/BRG1),or a compound that binds to epidermal growth factor receptor..

**17.** The compound according to claim 16, the PTM is a moiety that binds to an estrogen receptor; preferably, the PTM is selected from the following structures:

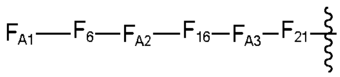 and 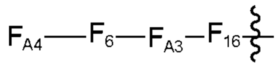 ;

wherein, each occurrence of $F_6$, $F_{16}$, and $F_{21}$ is independently selected from one, or a combination of multiple, selected from a single bond, NH, SO, S, O, $SO_2$, alkylene, haloalkylene, heteroalkylene, alkyleneoxy, heteroalkyleneoxy, alkenylene, alkynylene, C(=O), OC(=O), C(=O)O, C(=O)NH, and NHC(=O); wherein the alkylene, alkyleneoxy, and alkenylene are optionally substituted by 0, 1, 2, 3, 4, 5, or 6 Rc; preferably, each occurrence of $F_6$, $F_{16}$, and $F_{21}$ is independently selected from a single bond, NH, SO, S, O, $SO_2$, C(=O), OC(=O), and NHC(=O); $F_{A1}$ and $F_{A3}$ are each independently 6-10 membered aryl, or 6-10 membered heteroaryl; the aryl or heteroaryl is optionally substituted by 0, 1, 2, 3, 4, 5, or 6 $R_{da}$; preferably, $F_{A1}$ is phenyl, and is optionally substituted by 0, 1, 2, 3, 4, 5, or 6 $R_{da}$; $F_{A3}$ is phenyl, or 6 membered heteroaryl containing 1, 2, or 3 heteroatoms selected from N, O, and P, and is optionally substituted by 0, 1, 2, 3, 4, 5, or 6 $R_{da}$;

$F_{A2}$ is 3-15 membered cycloalkylene, 5-15 membered spirocycloalkylene, 3-15 membered heterocycloalkylene, or 5-15 membered spiroheterocycloalkylene, and is optionally substituted with 0, 1, 2, 3, 4, 5, or 6 $R_{ca}$; preferably, FA2 is 4-6 membered cycloalkylene, 7-11 membered spirocycloalkylene, 4-6 membered heterocycloalkylene, 7-11 membered spiroheterocycloalkylene, and is optionally substituted with 0, 1, 2, 3, 4, 5, or 6 $R_{ca}$; preferably, $F_{A2}$ is 4, 5, or 6 membered cycloalkylene, 10 membered spiroheterocycloalkylene containing 1, 2, or 3 nitrogen atoms, and is optionally substituted with 0, 1, 2, 3, 4, 5, or 6 $R_{ca}$;

$F_{A4}$ is a 6-10 membered aryl-fused 7-15 membered heterospirocyclic group, optionally substituted with 0, 1, 2, 3, 4, 5, or 6 $R_{da}$ substituents; preferably, $F_{A4}$ is a phenyl-fused 7-15 membered heterospirocyclic group containing 1, 2, 3, or 4 heteroatoms selected from N, O, S, optionally substituted with 0, 1, 2, 3, 4, 5, or 6 $R_{da}$ substituents;

each occurrence of $R_{da}$ is independently selected from H, halogen, $C_{1-6}$ alkyl, $C_{1-6}$ haloalkyl, $C_{1-6}$ heteroalkyl, $C_{1-6}$ alkoxy, $C_{1-6}$ heteroalkoxy, $C_{3-7}$ cycloalkyl, $C_{3-7}$ heterocyclyl, $C_{6-8}$ aryl, $C_{5-8}$ heteroaryl, OH, $NH_2$, CN, and $NO_2$; preferably, each occurrence of $R_{da}$ is independently selected from H, F, Cl, Br, I, $CH_3$, $OCH_3$, $CF_3$, OH, $NH_2$, CN, and $NO_2$;

each occurrence of $R_{ca}$ is independently selected from H, halogen, $C_{1-6}$ alkyl, $C_{1-6}$ haloalkyl, $C_{1-6}$ heteroalkyl, $C_{1-6}$ alkoxy, $C_{1-6}$ heteroalkoxy, $C_{3-7}$ cycloalkyl, $C_{3-7}$ heterocyclyl, $C_{6-8}$ aryl, $C_{5-8}$ heteroaryl, oxo (=O), thioxo (=S), OH, $NH_2$, CN, and $NO_2$; preferably, $R_{ca}$ is H, F, Cl, Br, I, $CH_3$, $OCH_3$, $CF_3$, OH, $NH_2$, CN, and $NO_2$;

preferably, the PTM is selected from the following structures:

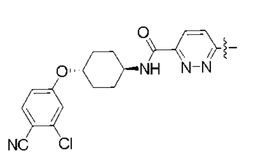, 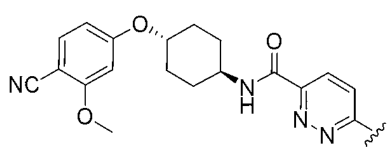, 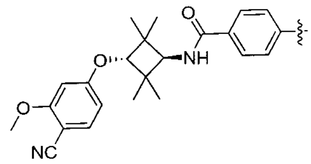,

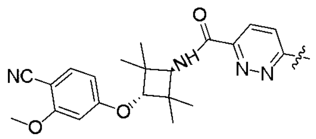, 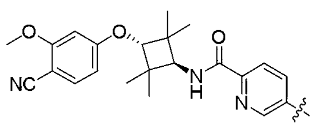, 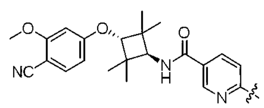,

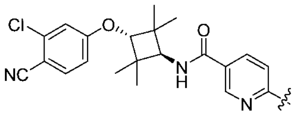, 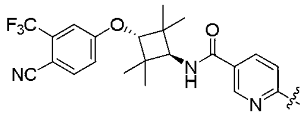, 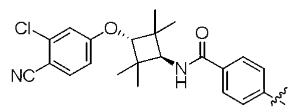,

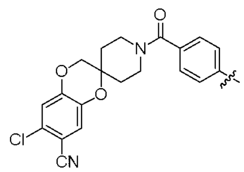, 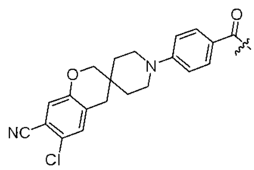, 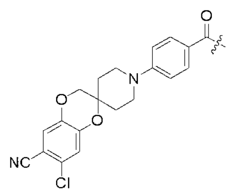, 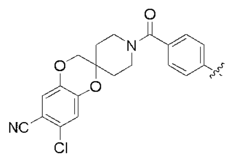

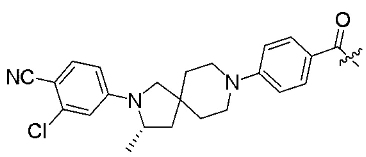 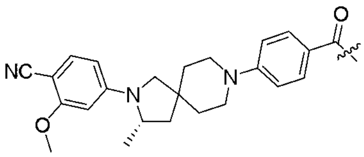, and 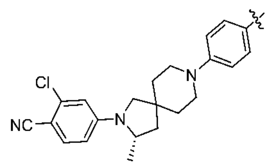;

preferably, the PTM is selected from the following structures:

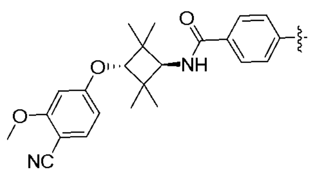,

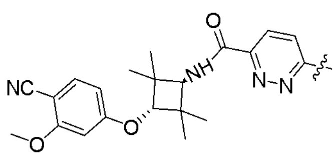, 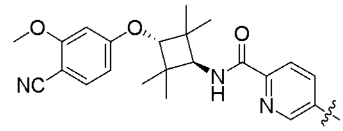,

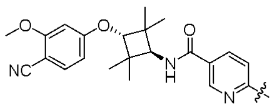, 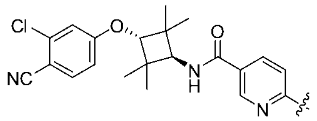, 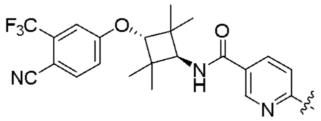,

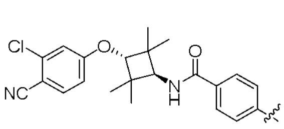, 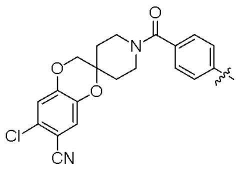, 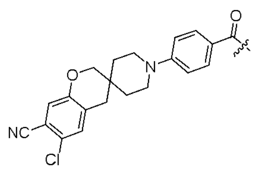, 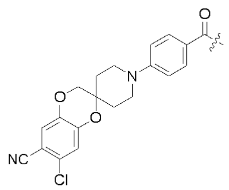,

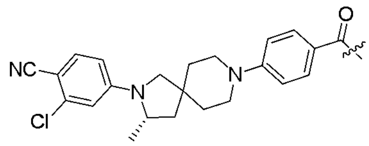, 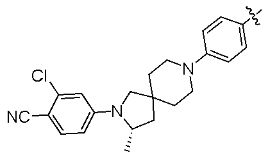, and 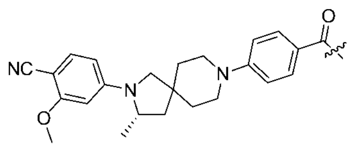.

**18.** The compound according to claim 17, wherein the compound is selected from the following structures:

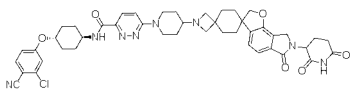
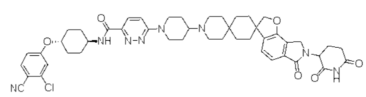
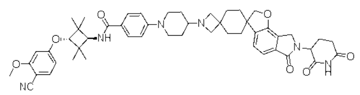
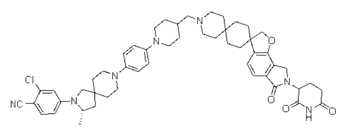
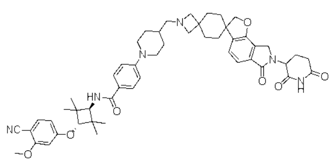
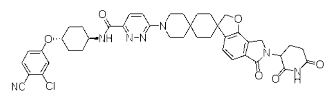
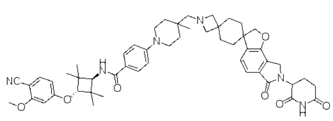
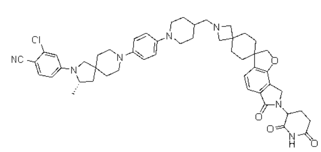
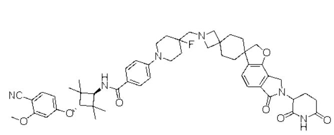
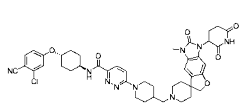
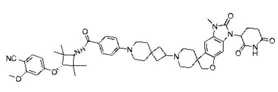
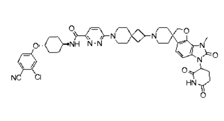
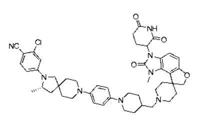
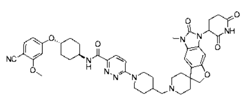
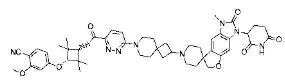
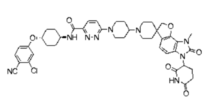

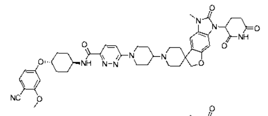
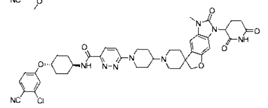
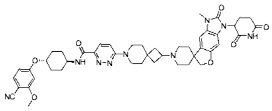
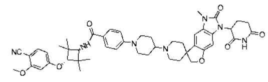
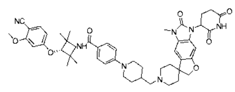
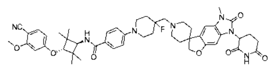
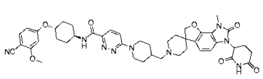
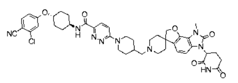
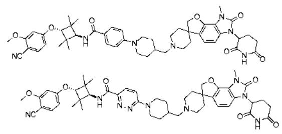
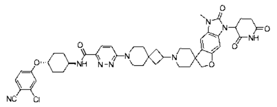
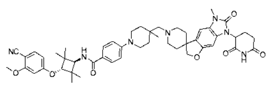
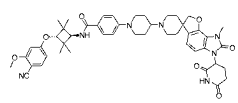
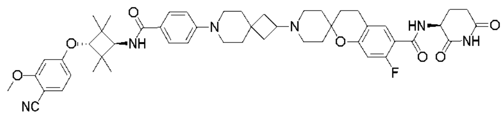
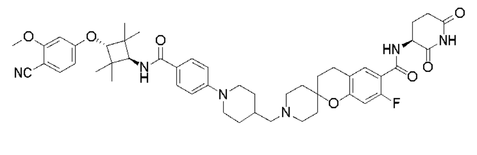
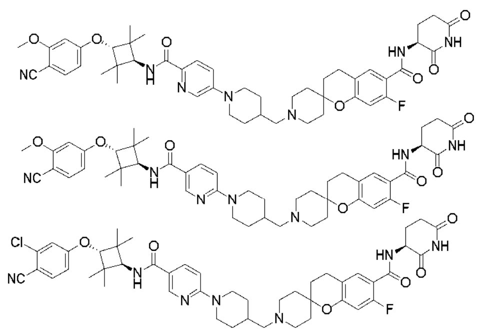
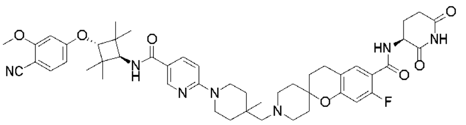
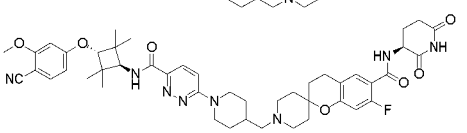
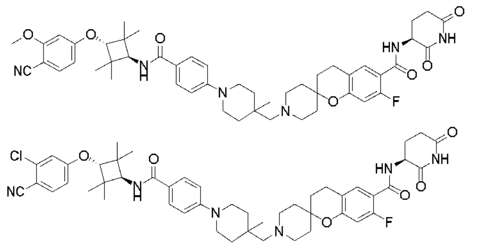
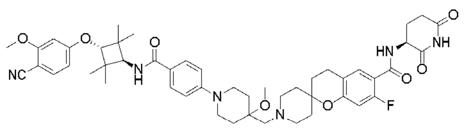
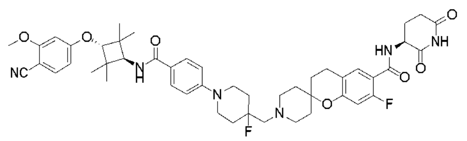

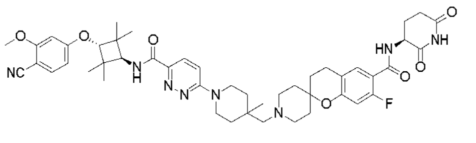

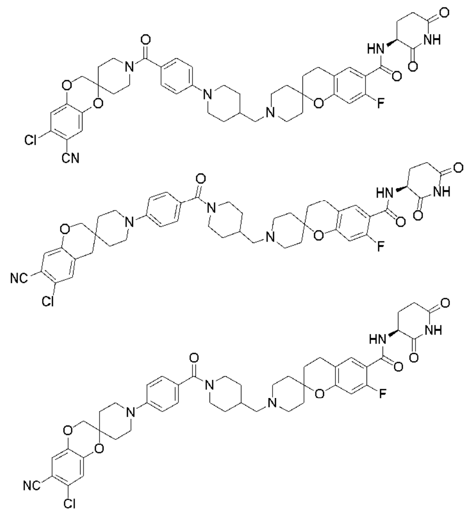

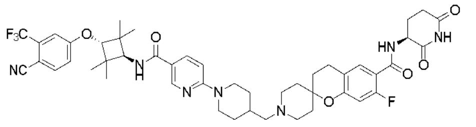

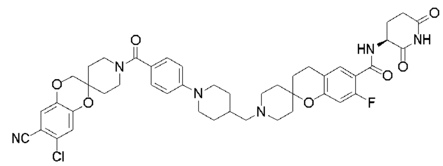

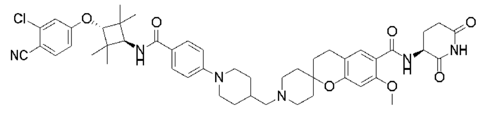

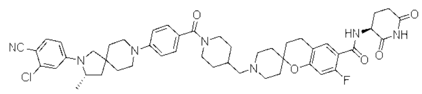

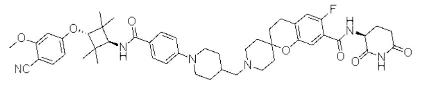

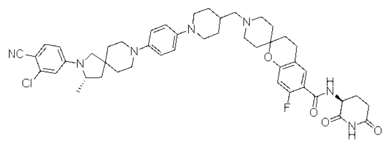

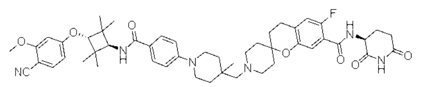

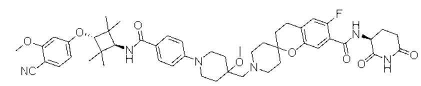

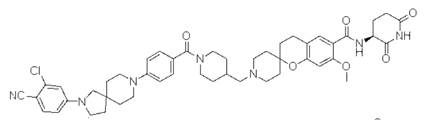

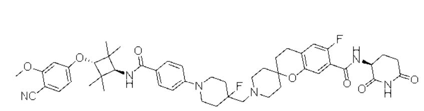

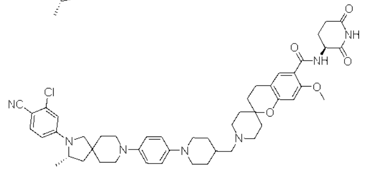

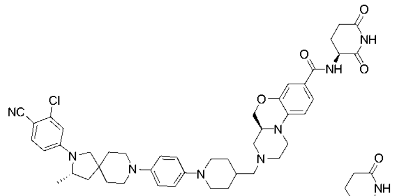

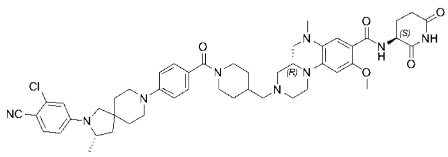

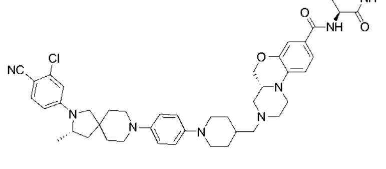

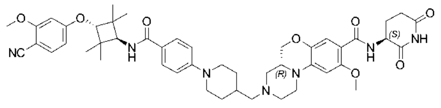

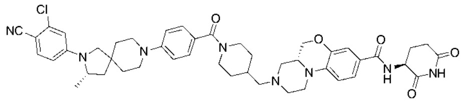

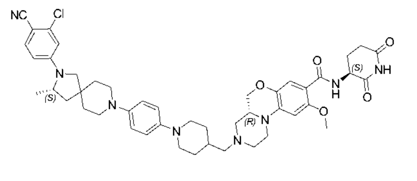

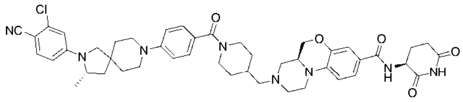

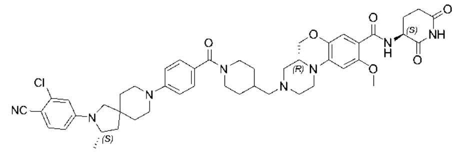

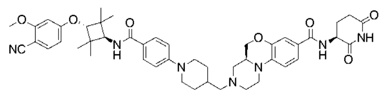

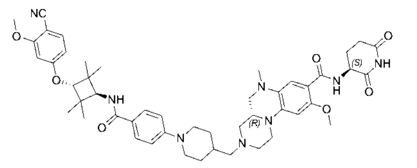

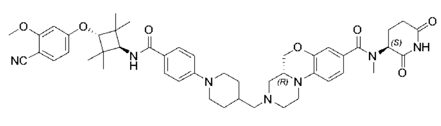

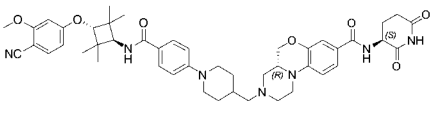

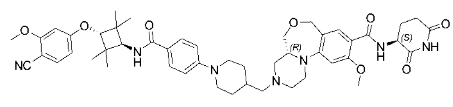

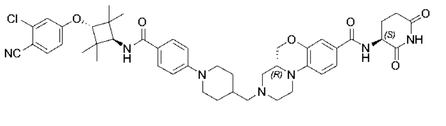

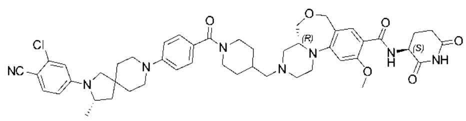

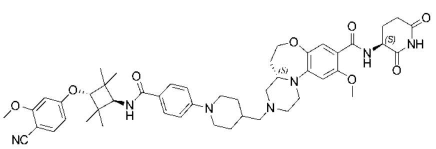

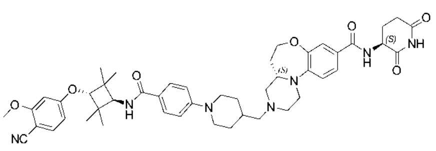

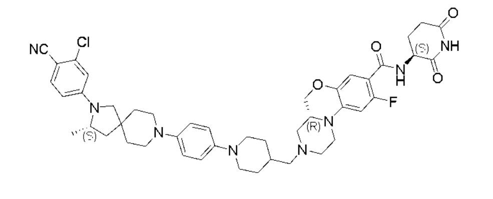

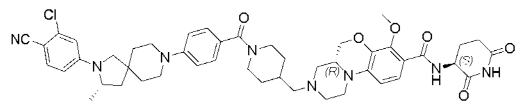

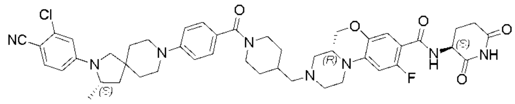

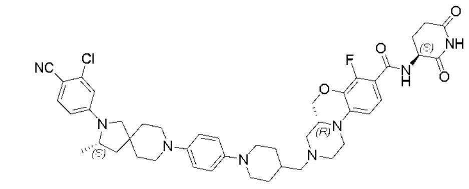

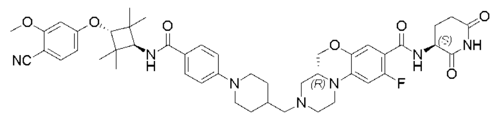

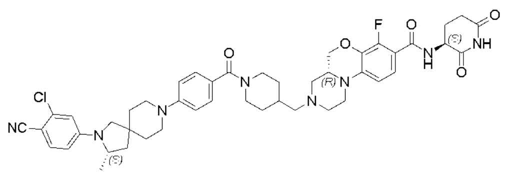

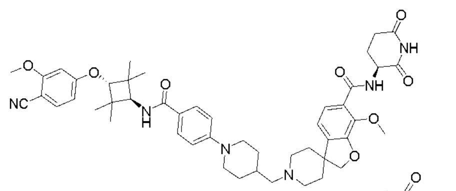

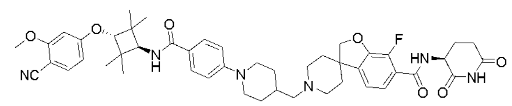

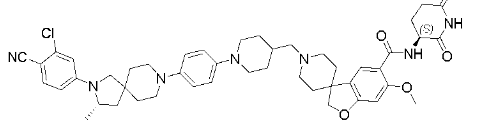

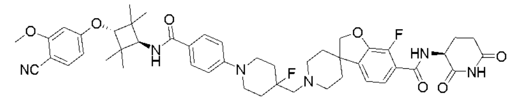

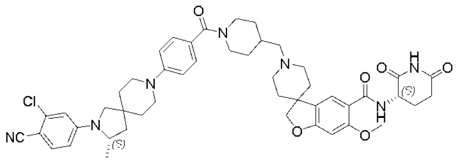

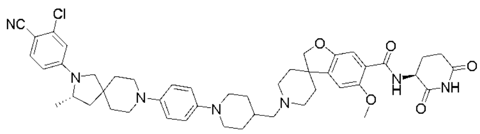

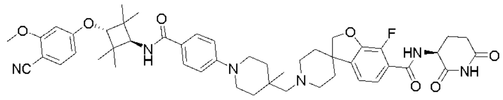

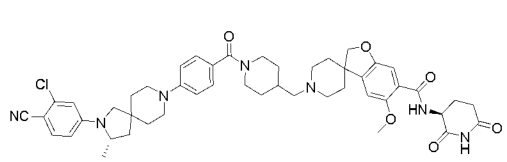

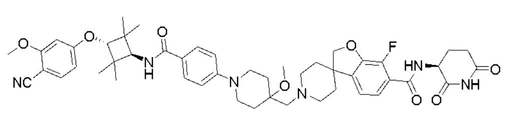

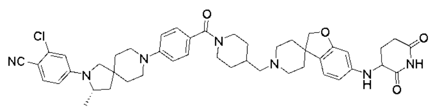

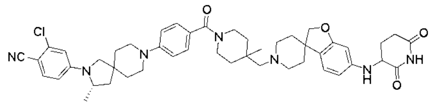

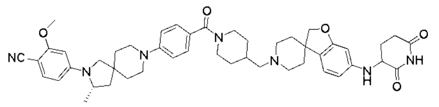

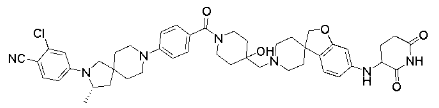

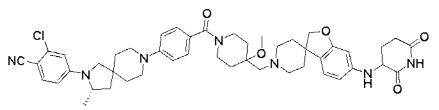

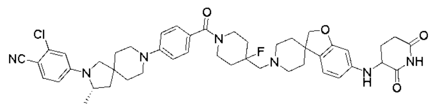

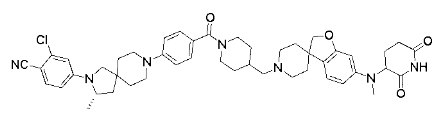

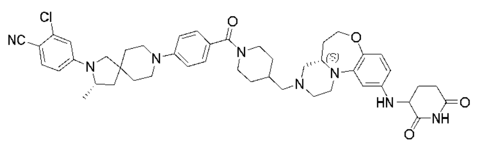

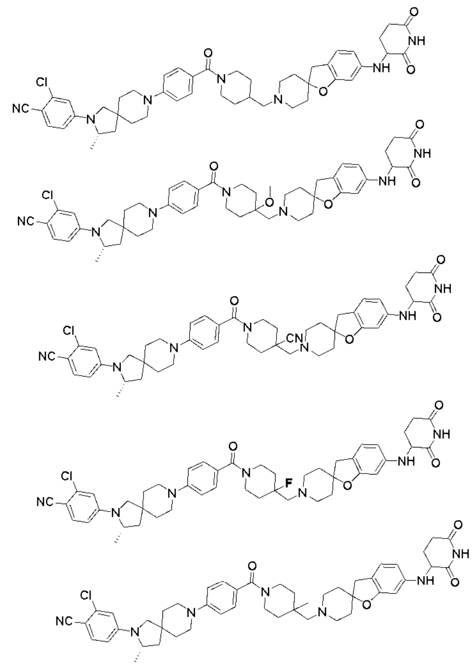

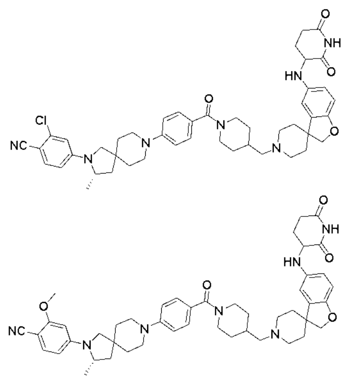

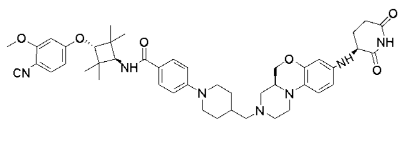

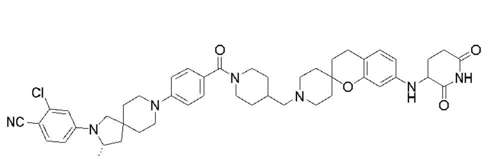

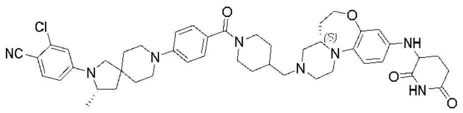

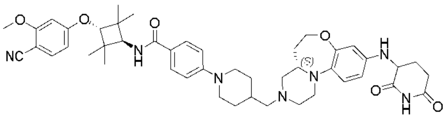

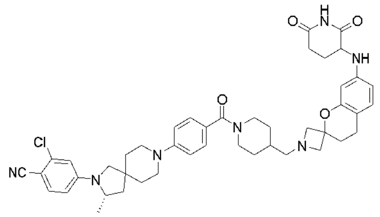

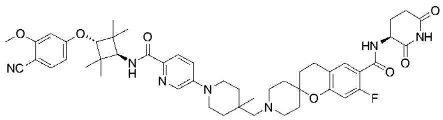

,

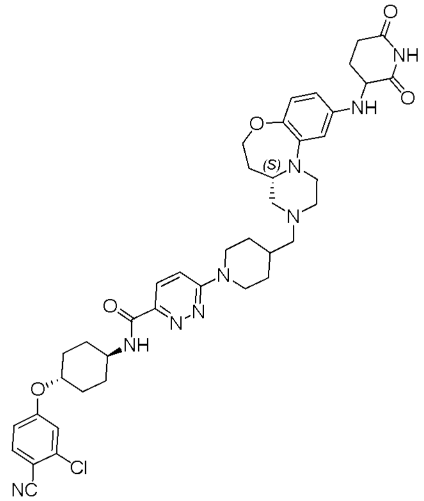

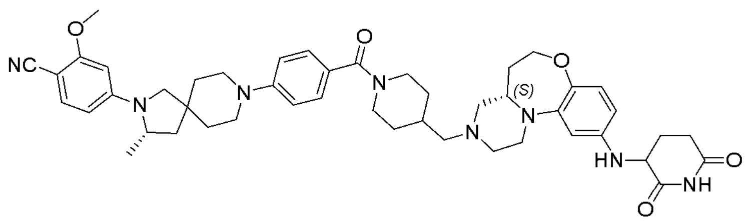

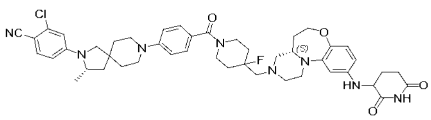

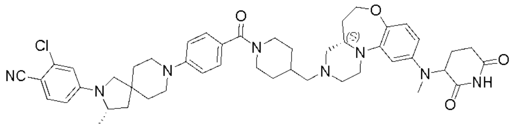

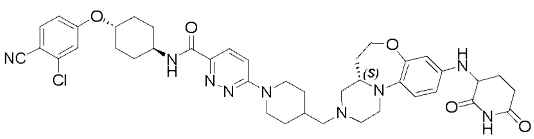

and

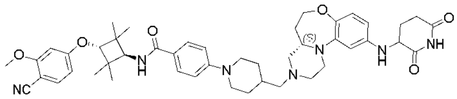

.

**19.** The compound according to claim 16, wherein the PTM is a moiety that binds to an estrogen receptor; preferably, the PTM is selected from the following structure:

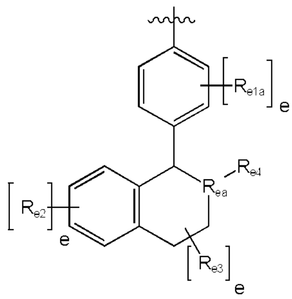;

$R_{ea}$ is selected from $CR_{e1a}$ and N;
each occurrence of $R_{e1a}$, $R_{e2}$, and $R_{e3}$ is independently selected from H, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, and amino, preferably, each occurrence of $R_{e1a}$, and $R_{e2}$ is independently selected from hydroxyl, H, F, Cl, Br, I, and $C_1$-$C_3$ alkyl;
each occurrence of e is independently selected from 0, 1, 2, 3, and 4;
$R_{e4}$ is selected from 6-10 membered aryl, 6-10 membered heteroaryl, $C_1$-$C_6$ alkyl, $C_1$-$C_6$ haloalkyl, the 6-10 membered aryl, or 6-10 membered heteroaryl is optionally substituted with 0, 1, 2, 3, 4, or 5 $R_{e1a}$; preferably, $R_{e4}$ is selected from phenyl and difluoroethyl, the phenyl is optionally substituted with 0, 1, 2, 3, 4, or 5 $R_{e1a}$;
preferably, the PTM is selected from the following structures:

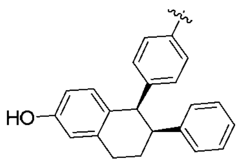 and 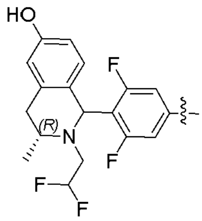.

**20.** The compound according to claim 19, wherein the structure is selected from:

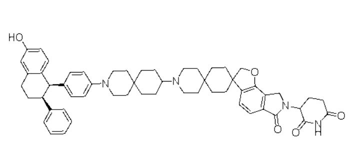, 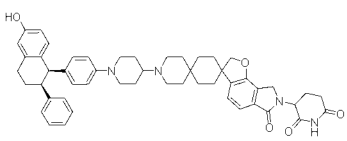

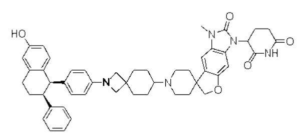

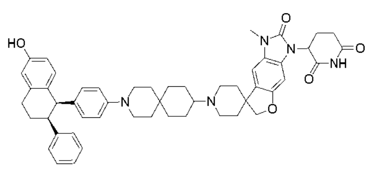

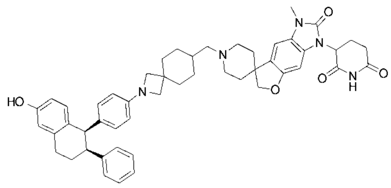

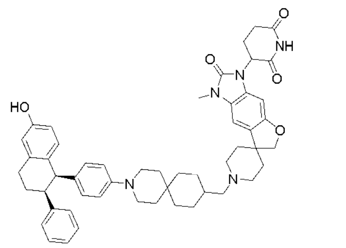

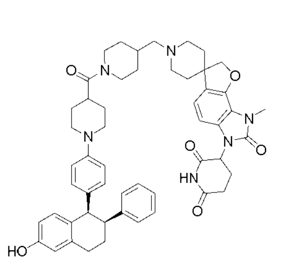

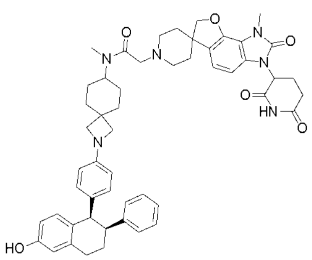

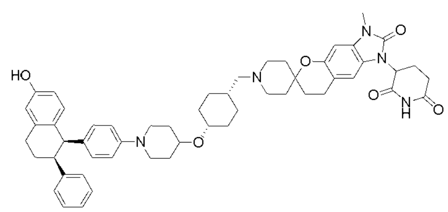

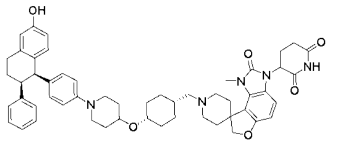
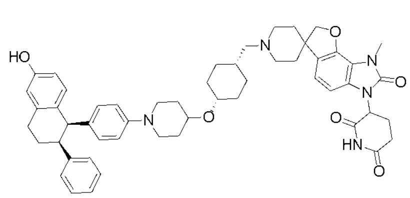
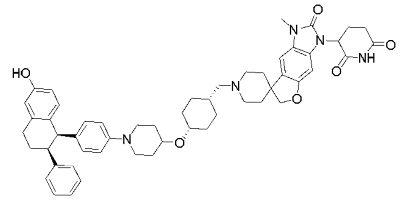

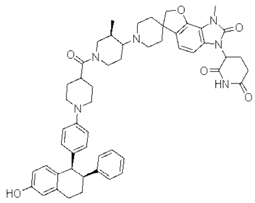
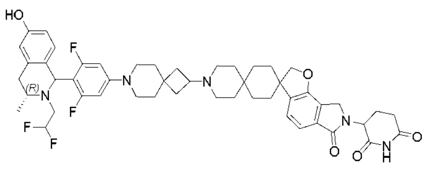
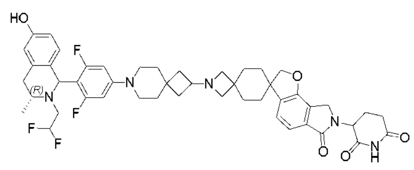

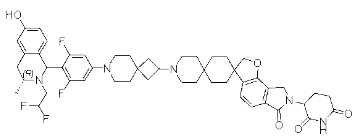
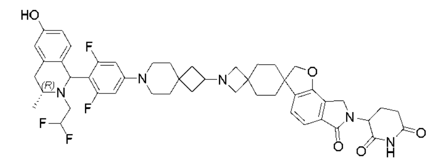
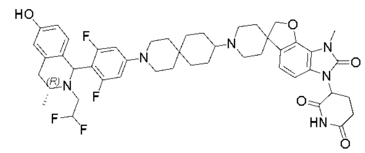

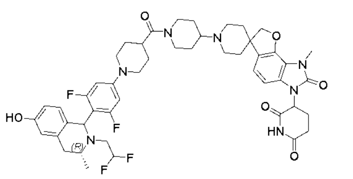
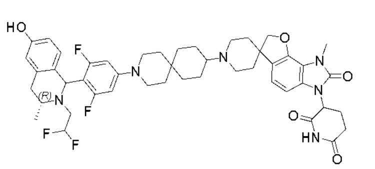
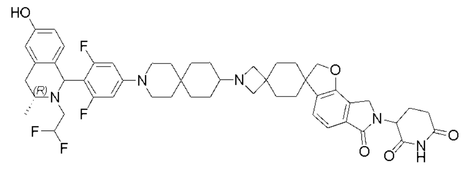

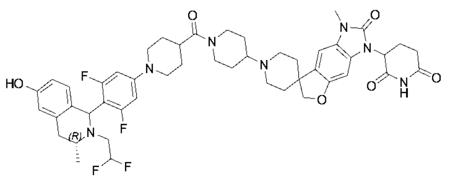
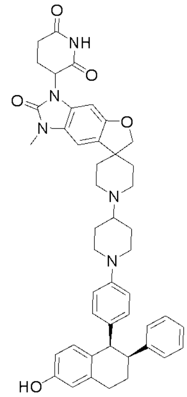
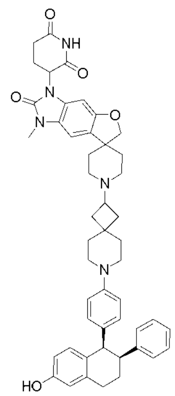
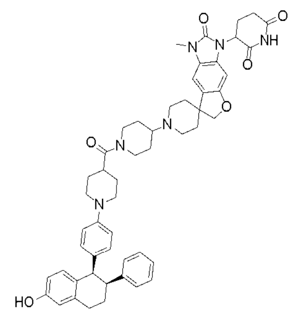

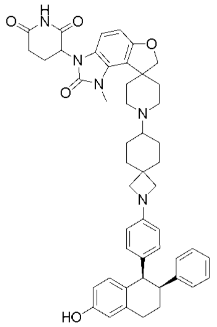
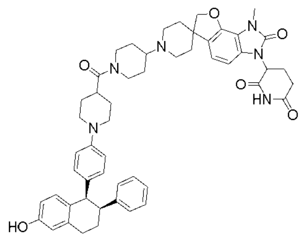
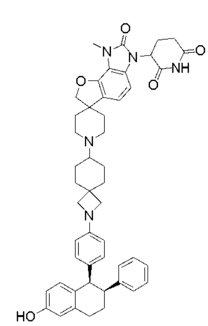
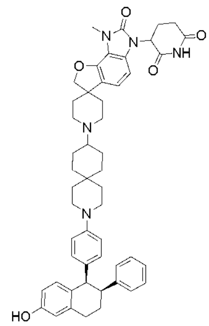
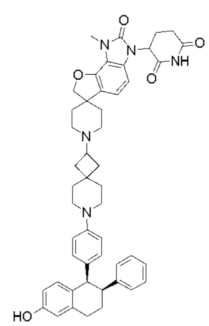

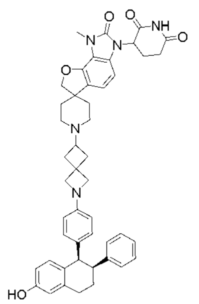
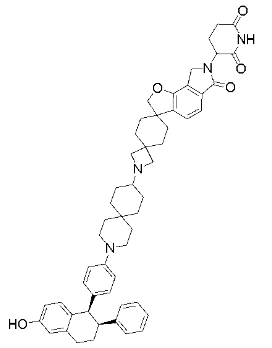
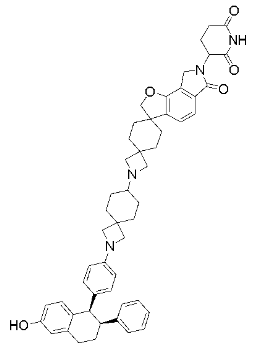
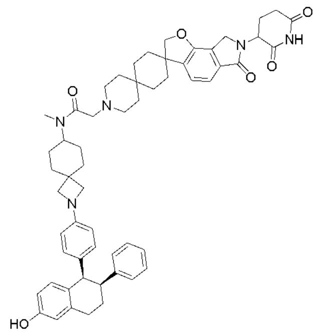
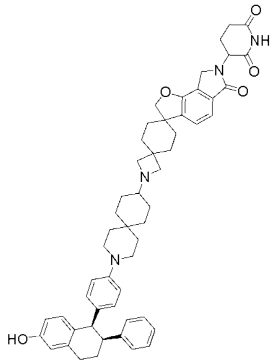
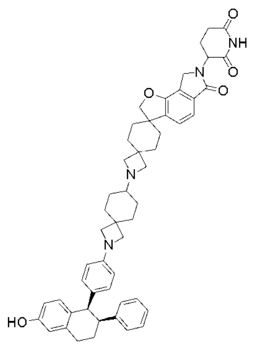
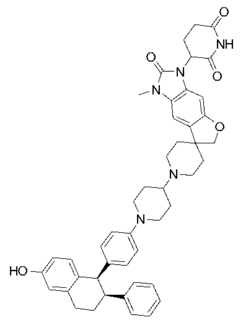
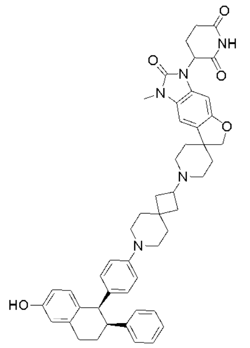
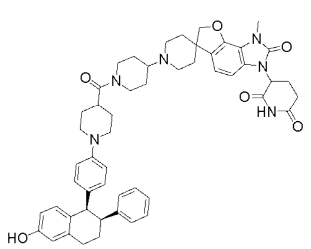
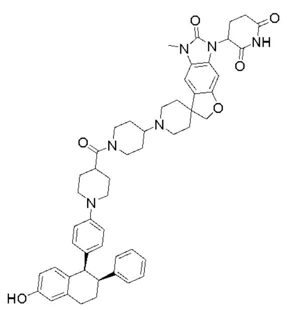
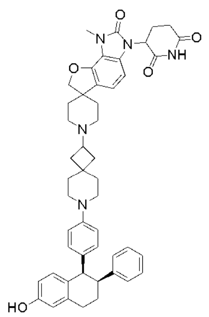
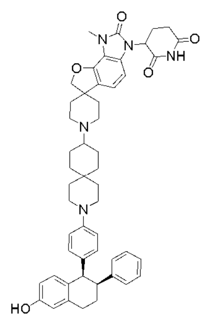
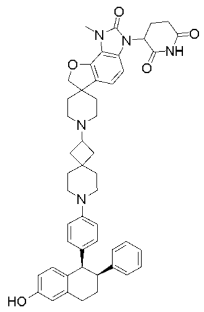
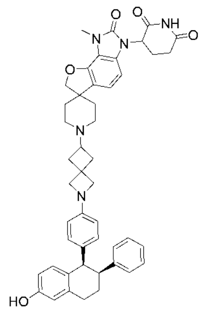
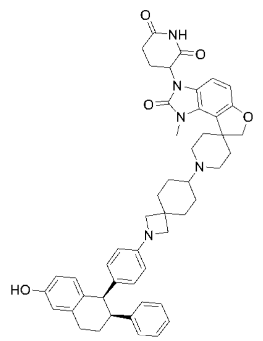
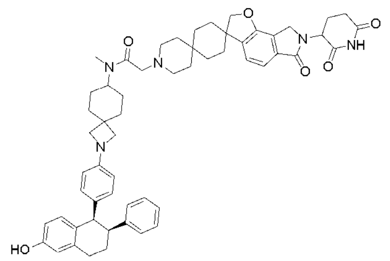
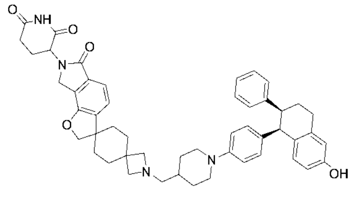
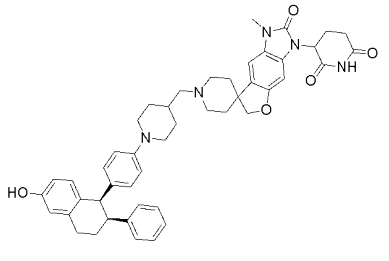
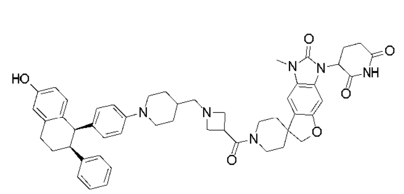

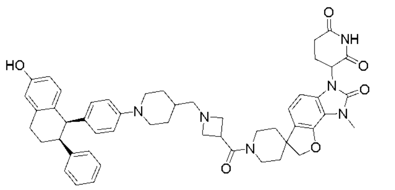
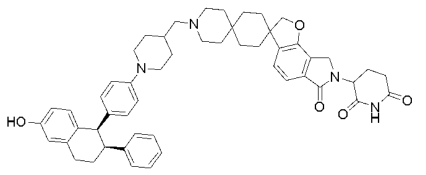
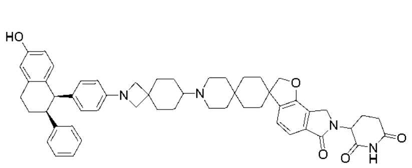
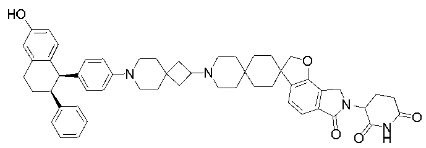
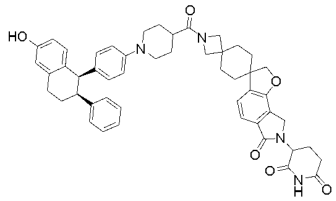
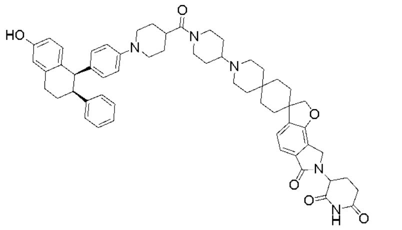
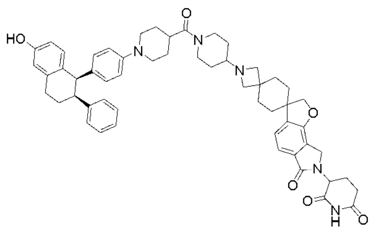
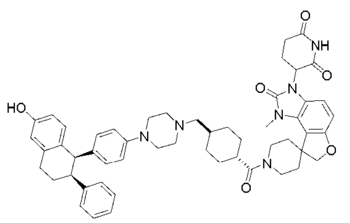
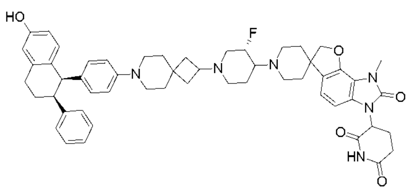
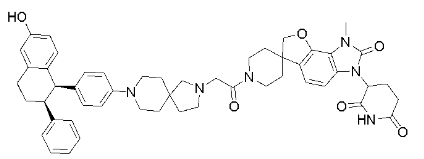
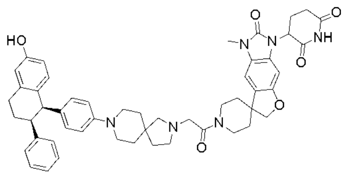
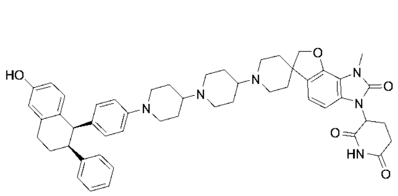
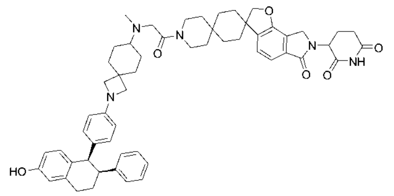
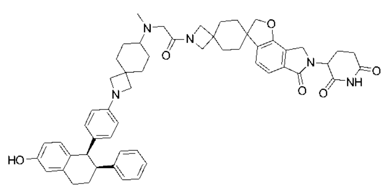
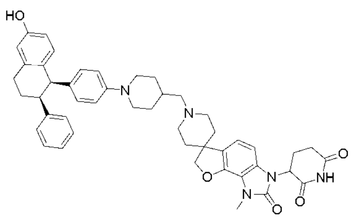
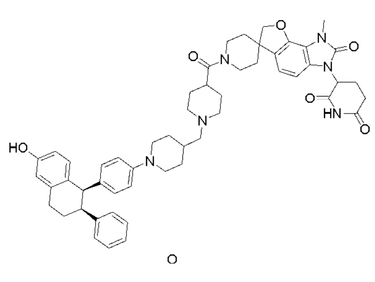
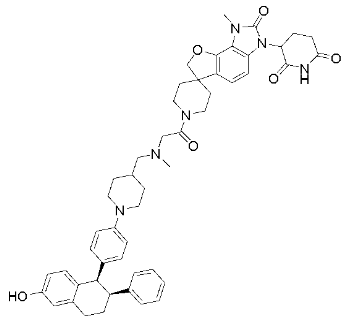

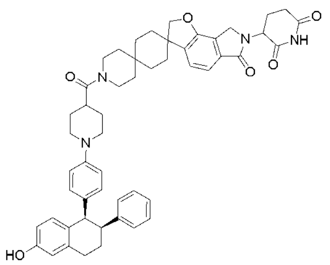
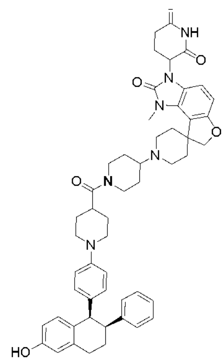
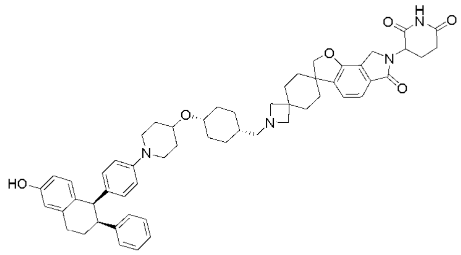

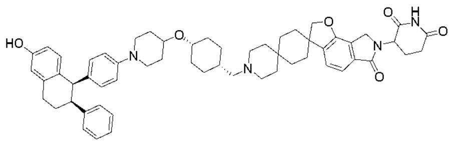
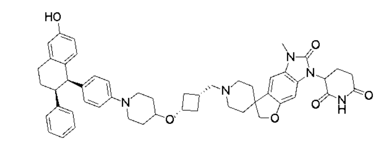
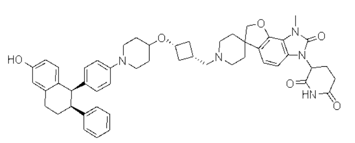

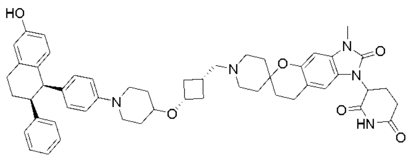
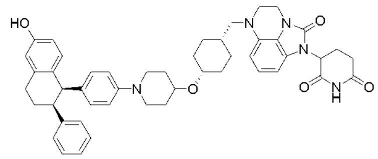
,
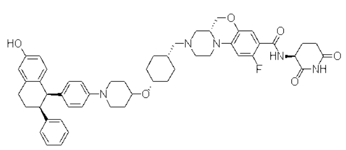

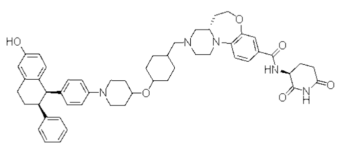
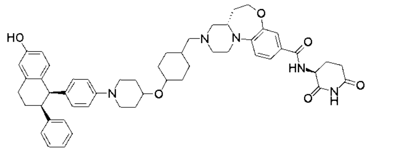
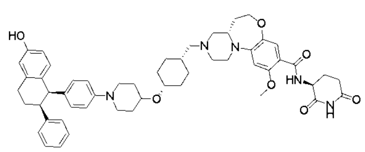

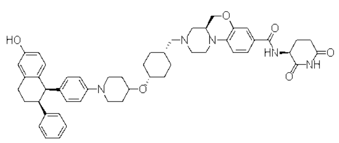
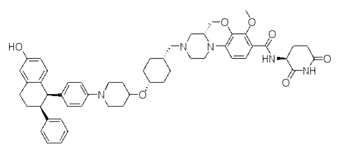
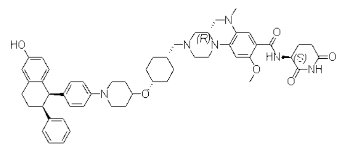

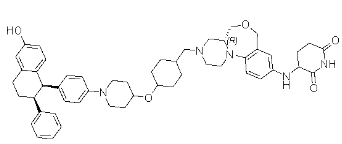
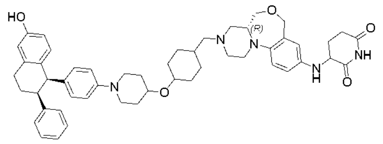
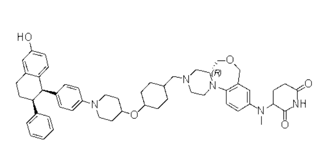

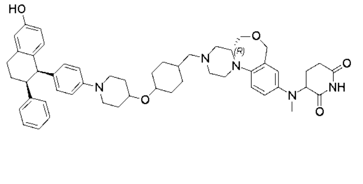
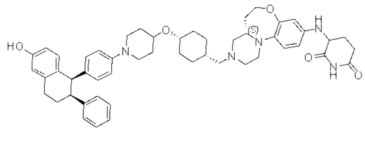
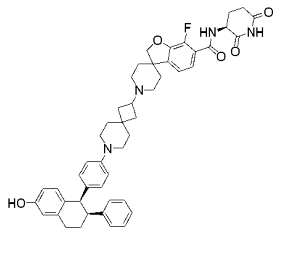

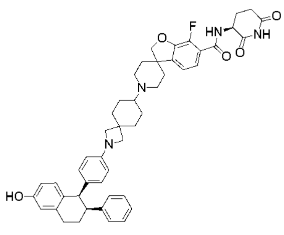
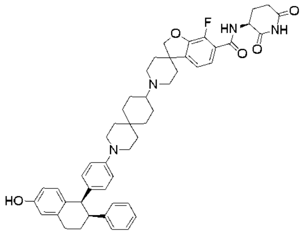
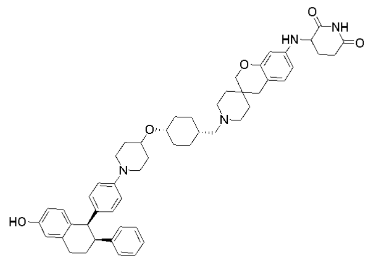

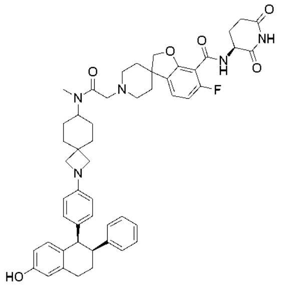
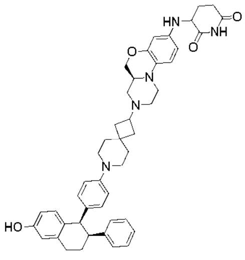
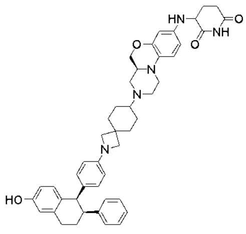
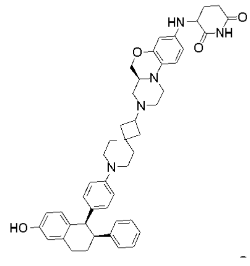

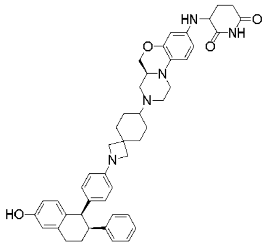
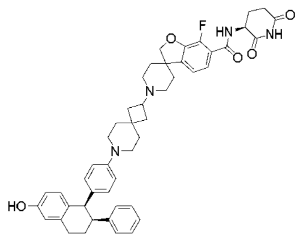
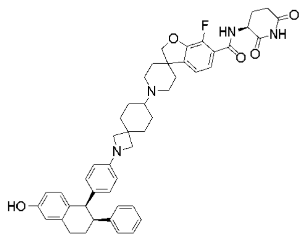
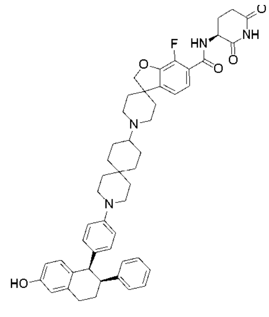

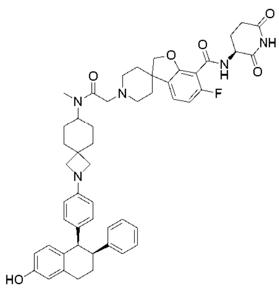
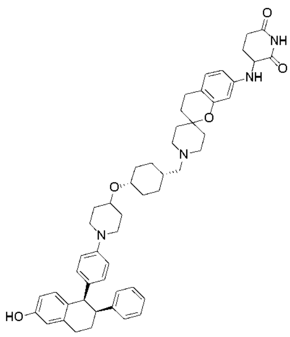
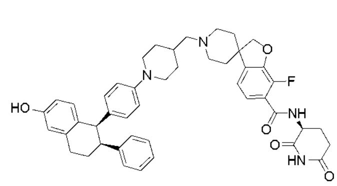

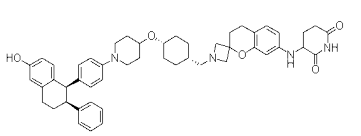
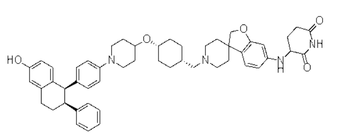
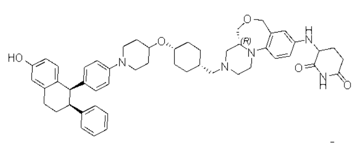

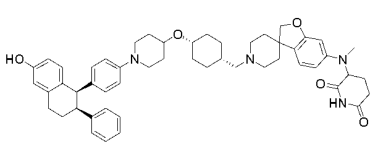
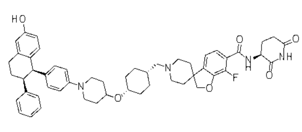
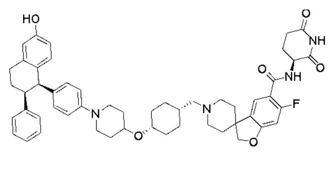

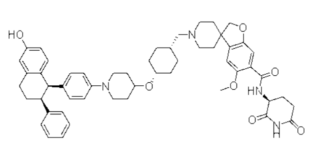 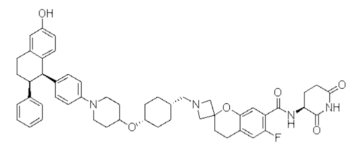 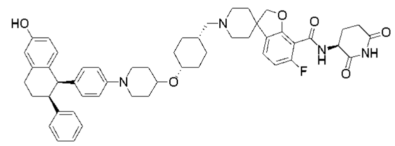

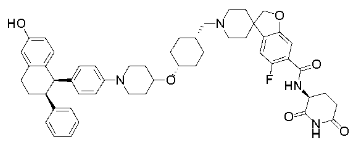 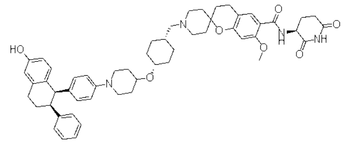 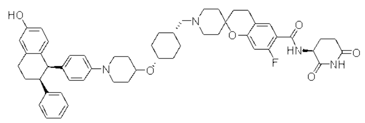

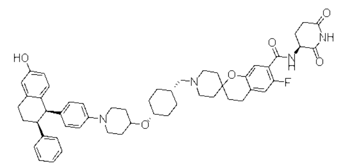 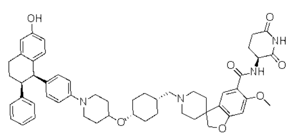 and 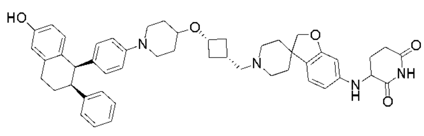 .

**21.** The compound according to claim 16, wherein the PTM is a moiety that binds to SMARCA2/4; preferably, the PTM is selected from the following structure:

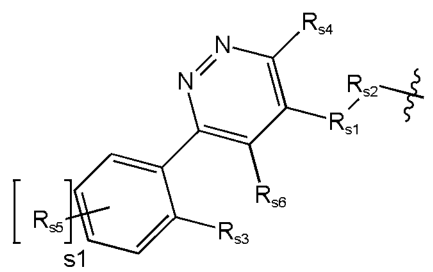

$R_{s1}$ is selected from one, or a combination of multiple, selected from a single bond, NH, SO, S, O, $SO_2$, alkylene, haloalkylene, heteroalkylene, alkyleneoxy, heteroalkyleneoxy, alkenylene, alkynylene, C(=O), OC(=O), C(=O)O, C(=O)NH, and NHC(=O); preferably, $R_{s1}$ is selected from a single bond, $C_{2-6}$ alkenylene, and $C_{2-6}$ alkynylene;
$R_{s2}$ is selected from 6-10 membered arylene, 6-10 membered heteroarylene, 3-15 membered cycloalkylene, 3-15 membered heterocyclylene, and 5-15 membered spirocycloalkylene, wherein the 6-10 membered arylene, 6-10 membered heteroarylene, 3-15 membered cycloalkylene, and 5-15 membered spirocycloalkylene are each optionally substituted with 0, 1, 2, 3, 4, 5, or 6 $R_{s5}$; preferably, $R_{s2}$ is selected from 5-8 membered heteroarylene containing 1, 2, or 3 heteroatoms selected from N, O, or S, 4-6 membered heterocyclylene containing 1, 2, or 3 heteroatoms selected from N, O, or S, and 7-11 membered spirocycloalkylene, wherein the 5-8 membered heteroarylene containing 1, 2, or 3 heteroatoms selected from N, O, or S, the 4-6 membered heterocyclylene containing 1, 2, or 3 heteroatoms selected from N, O, or S, and the 7-11 membered spirocycloalkylene are each optionally substituted with 0, 1, 2, 3, 4, 5, or 6 $R_{s5}$; preferably, $R_{s2}$ is selected from pyrazolylene, 4-membered heterocyclylene containing one nitrogen atom, and 8-membered spirocycloalkylene;
each occurrence of $R_{s3}$, $R_{s4}$, $R_{s5}$, and $R_{s6}$ is independently selected from H, halogen, $C_{1-6}$ alkyl, $C_{1-6}$ haloalkyl, $C_{1-6}$ heteroalkyl, $C_{1-6}$ alkoxy, $C_{1-6}$ heteroalkoxy, $C_{3-7}$ cycloalkyl, $C_{3-7}$ heterocyclyl, $C_{6-8}$ aryl, $C_{5-8}$ heteroaryl, OH, $NH_2$, CN, and $NO_2$; preferably, each occurrence of $R_{s3}$, $R_{s4}$, $R_{s5}$, and $R_{s6}$ is independently selected from H, F, Cl, Br, I, $CH_3$, $OCH_3$, $CF_3$, OH, $NH_2$, CN, and $NO_2$; preferably, $R_{s3}$ is OH, $R_{s4}$ is $NH_2$; and
s1 is selected from 0, 1, 2, 3, and 4;
preferably, the PTM is selected from:

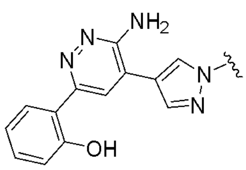 , 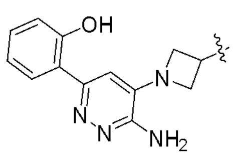 , and 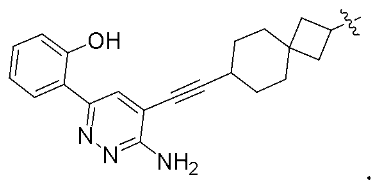 .

**22.** The compound according to claim 21, wherein the structure is selected from:

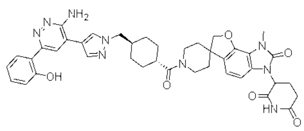
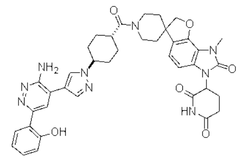
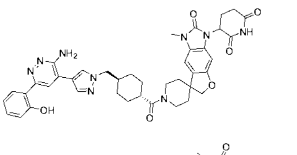
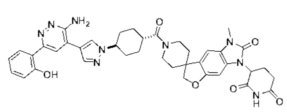
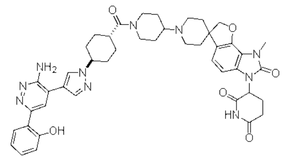
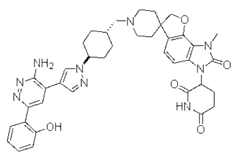
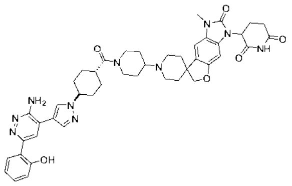
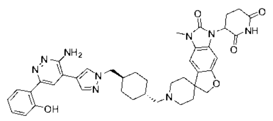
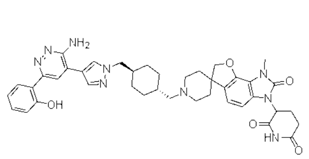
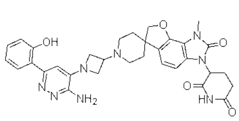
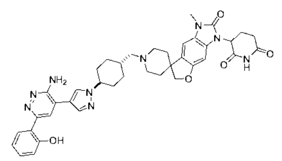
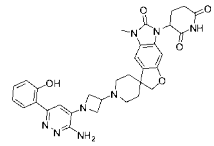
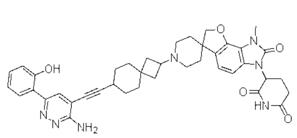
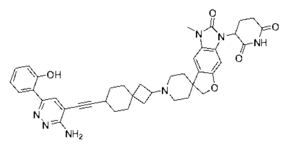
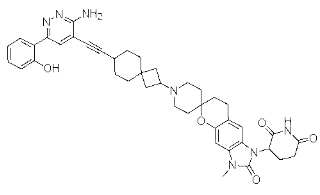
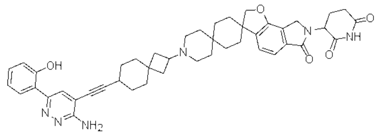
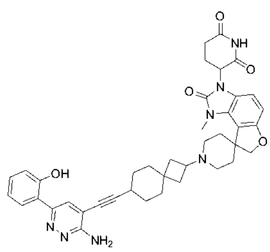

,

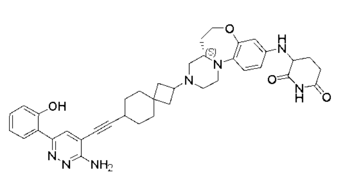
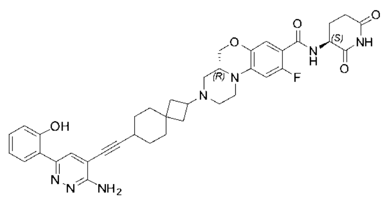
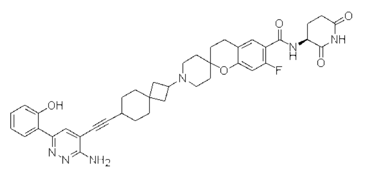
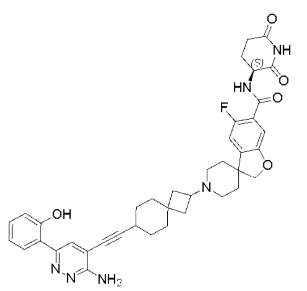
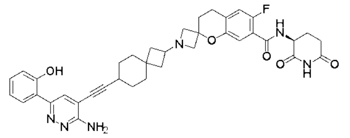
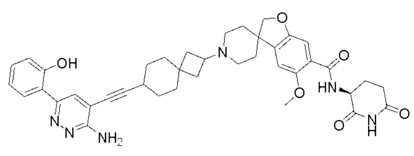
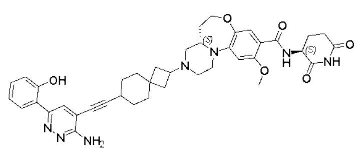
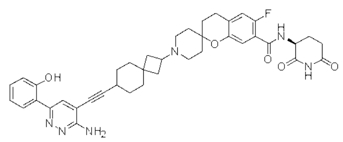
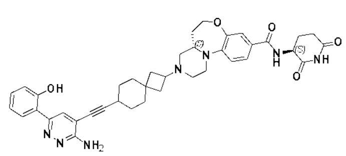

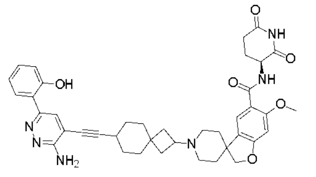
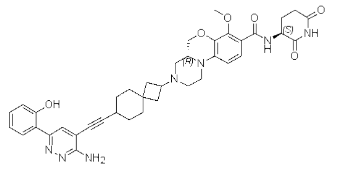
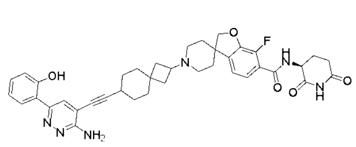
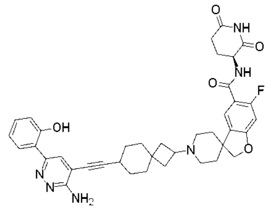
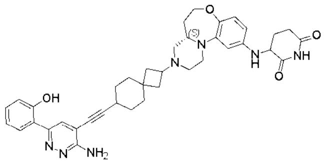
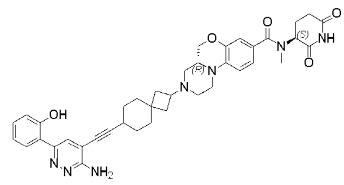
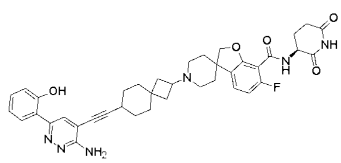
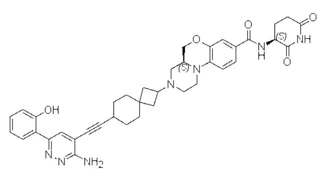
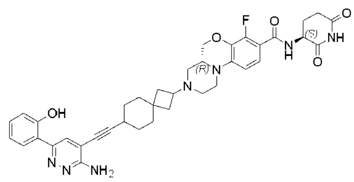
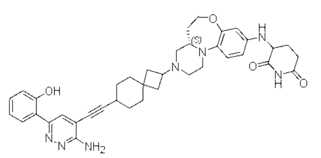
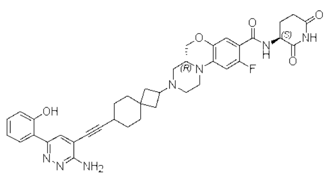
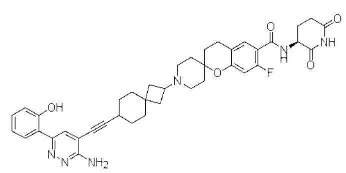
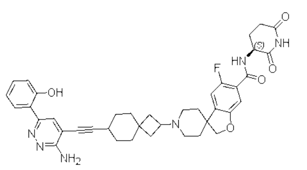
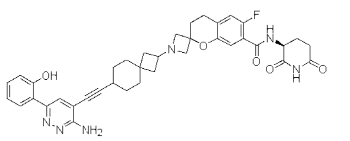
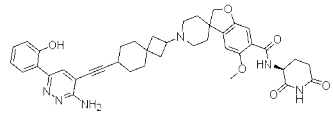
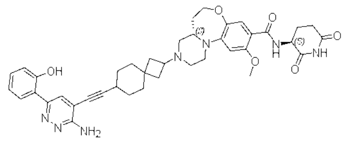
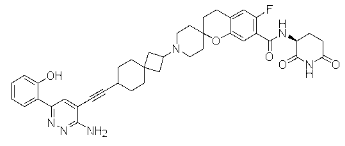
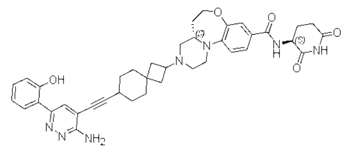
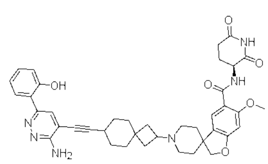
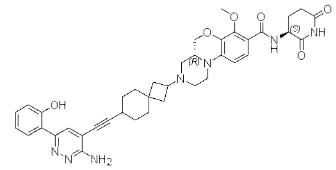
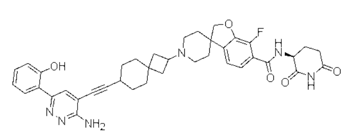
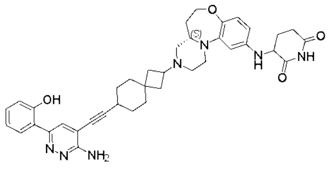
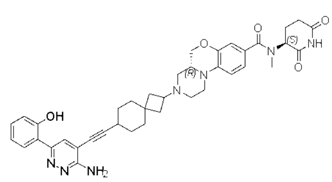
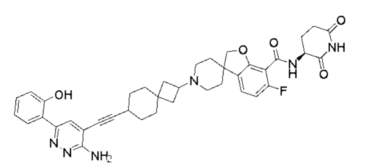

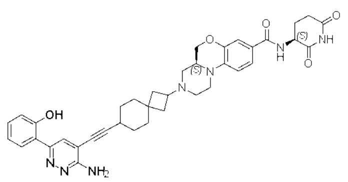

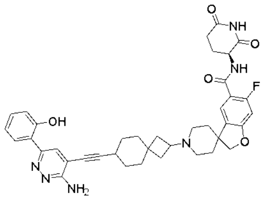

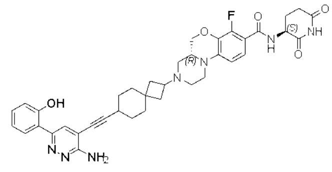

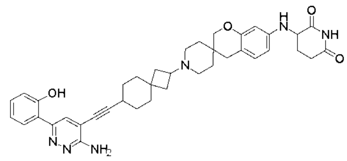

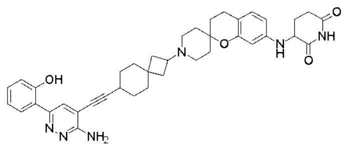

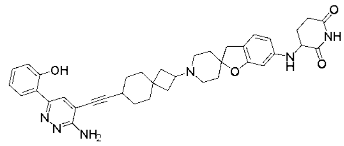

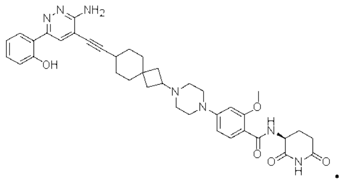

and

**23.** A pharmaceutical composition, wherein the pharmaceutical composition comprises the compound according to any one of claims 1-22 and a pharmaceutically acceptable excipient.

**24.** Use of the compound according to any one of claims 7-22, or the pharmaceutical composition according to claim 23, in the manufacture of a medicament for treating or preventing a condition treated by degradation of a target protein bound to a target protein ligand, wherein the target protein is preferably cyclin-dependent kinase 4/6, androgen receptor, estrogen receptor, Aurora kinase A, IRAK4, SMARCA2/4, Bcr-Abl protein, epidermal growth factor receptor, anaplastic lymphoma kinase, Bruton's tyrosine kinase, BRM/BRG1 protein, or bromodomain-containing protein 4.

**25.** Use of the compound according to any one of claims 7-22, or the pharmaceutical composition according to claim 23, in the manufacture of a medicament for treating or preventing a condition treated by binding to a cereblon protein in vivo.

**26.** Use of the compound according to any one of claims 7-22, or the pharmaceutical composition according to claim 23, in treating or preventing a condition associated with accumulation and/or aggregation of a target protein, wherein the target protein is preferably cyclin-dependent kinase 4/6, androgen receptor, estrogen receptor, Aurora kinase A, Bcr-Abl protein, IRAK4, SMARCA2/4, epidermal growth factor receptor, anaplastic lymphoma kinase, Bruton's tyrosine kinase, BRM/BRG1 protein, or bromodomain-containing protein 4.

**27.** The compound according to any one of claims 7-22, or the pharmaceutical composition according to claim 23, for use in treating or preventing a condition treated by degradation of a target protein bound to a target protein ligand, wherein the target protein is preferably cyclin-dependent kinase 4/6, androgen receptor, estrogen receptor, Aurora kinase A, Bcr-Abl protein, IRAK4, SMARCA2/4, epidermal growth factor receptor, anaplastic lymphoma kinase, Bruton's tyrosine kinase, BRM/BRG1 protein, or bromodomain-containing protein 4.

**28.** The compound according to any one of claims 18-22, or the pharmaceutical composition according to claim 23, for use in treating or preventing a condition treated by binding to a cereblon protein in vivo.

**29.** The compound according to any one of claims 7-22, or the pharmaceutical composition according to claim 23, for use in treating or preventing a condition associated with accumulation and/or aggregation of a target protein, wherein the target protein is preferably cyclin-dependent kinase 4/6, androgen receptor, estrogen receptor, Aurora kinase A, Bcr-Abl protein, IRAK4, SMARCA2/4, epidermal growth factor receptor, anaplastic lymphoma kinase, Bruton's tyrosine kinase, BRM/BRG1 protein, or bromodomain-containing protein 4.

**30.** A method for treating or preventing a condition treated by degradation of a target protein bound to a target protein ligand, comprising administering a therapeutically effective amount of the compound according to any one of claims 7-22, or the pharmaceutical composition according to claim 23, to a subject in need thereof, wherein the target protein

is preferably cyclin-dependent kinase 4/6, androgen receptor, estrogen receptor, Aurora kinase A, IRAK4, SMARCA2/4, Bcr-Abl protein, epidermal growth factor receptor, anaplastic lymphoma kinase, Bruton's tyrosine kinase, BRM/BRG1 protein, or bromodomain-containing protein 4.

**31.** A method for treating or preventing a condition treated by binding to a cereblon protein in vivo, comprising administering a therapeutically effective amount of the compound according to any one of claims 7-22, or the pharmaceutical composition according to claim 23, to a subject in need thereof.

**32.** A method for treating or preventing a condition associated with accumulation and/or aggregation of a target protein, comprising administering a therapeutically effective amount of the compound according to any one of claims 7-22, or the pharmaceutical composition according to claim 23, to a subject in need thereof, wherein the target protein is preferably cyclin-dependent kinase 4/6, androgen receptor, estrogen receptor, Aurora kinase A, Bcr-Abl protein, IRAK4, SMARCA2/4, epidermal growth factor receptor, anaplastic lymphoma kinase, Bruton's tyrosine kinase, BRM/BRG1 protein, or bromodomain-containing protein 4.

**33.** The method according to any one of claims 30 to 32, wherein the condition is a tumor or cancer.

**34.** A method for inducing degradation of a target protein in a cell, the method comprising administering an effective amount of the compound according to any one of claims 7-22, or the pharmaceutical composition according to claim 23, wherein the target protein is preferably cyclin-dependent kinase 4/6, androgen receptor, estrogen receptor, Aurora kinase A, Bcr-Abl protein, IRAK4, SMARCA2/4, epidermal growth factor receptor, anaplastic lymphoma kinase, Bruton's tyrosine kinase, BRM/BRG1 protein, or bromodomain-containing protein 4.

# INTERNATIONAL SEARCH REPORT

International application No.
**PCT/CN2024/095621**

## A. CLASSIFICATION OF SUBJECT MATTER

C07D 491/20(2006.01)i; C07D498/04(2006.01)i; C07D471/04(2006.01)i; C07D471/14(2006.01)i; C07D471/10(2006.01)i; C07D487/10(2006.01)i; C07D471/20(2006.01)i; C07D498/14(2006.01)i; C07D487/04(2006.01)i; A61K31/435(2006.01)i; A61K31/438(2006.01)i; A61K31/437(2006.01)i; A61K31/407(2006.01)i; A61P35/00(2006.01)i

According to International Patent Classification (IPC) or to both national classification and IPC

## B. FIELDS SEARCHED

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNABS; WPABS; CNTXT; CJFD; VEN; WOTXT; USTXT; ENTXT; CNKI; 万方, WANFANG; 超星读秀, CHAOXING DUXIU; ISI-Web of Science; STN-registry; STN-caplus: 甘李药业, 徐福明, 陈文敏, 周冠, 刘晓兵, 丁永正, 李晓光, 小脑蛋白, E3泛素连接酶, 泛素酶, 蛋白降解靶向嵌合体, 癌症, 肿瘤, Cereblon, CRBN, PROTAC, PROTACs, CLM-L-PTM, Cancer, Tumor, 根据式I限定结构进行的子结构检索, substructure search according to formula I

## C. DOCUMENTS CONSIDERED TO BE RELEVANT

| Category* | Citation of document, with indication, where appropriate, of the relevant passages | Relevant to claim No. |
|---|---|---|
| PX | CN 118005655 A (JIANGSU HENGRUI MEDICINE CO., LTD. et al.) 10 May 2024 (2024-05-10)<br>claim 16 | 1-6 |
| PX | WO 2024015340 A1 (UNIV MICHIGAN et al.) 18 January 2024 (2024-01-18)<br>claims 1, 2 and 32-40, and description, paragraphs [0277]-[0289], and tables 1, 2, E2 and E3 | 1-29, 33, 34 |
| PX | WO 2024015346 A1 (ONCOPIA THERAPEUTICS, INC. d.b.a PROTEOVANT THERAPEUTICS, INC. et al.) 18 January 2024 (2024-01-18)<br>claims 1 and 31-38, and description, tables 1 and E1 | 1-6, 23-29, 33, 34 |
| PX | WO 2024015409 A1 (UNIV MICHIGAN et al.) 18 January 2024 (2024-01-18)<br>claims 1-76 | 1-29, 33, 34 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **26 August 2024** | **04 September 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/095621**

**C. DOCUMENTS CONSIDERED TO BE RELEVANT**

| Category* | Citation of document, with indication, where appropriate, of the relevant passages | Relevant to claim No. |
|---|---|---|
| PX | WO 2024015406 A1 (UNIV MICHIGAN et al.) 18 January 2024 (2024-01-18) claims 1-75 | 1-29, 33, 34 |
| PX | WO 2024015412 A1 (UNIV MICHIGAN et al.) 18 January 2024 (2024-01-18) claims 1-67 | 1-29, 33, 34 |
| PX | WO 2023186069 A1 (CSPC ZHONGQI PHARMACEUTICAL TECHNOLOGY (SHIJIAZHUANG) CO., LTD.) 05 October 2023 (2023-10-05) claims 1-53 | 1-29, 33, 34 |
| PX | WO 2023183607 A1 (UNIV MICHIGAN et al.) 28 September 2023 (2023-09-28) claims 1-33 | 1-29, 33, 34 |
| PX | CN 117050058 A (SICHUAN HAISCO PHARMACEUTICAL CO., LTD.) 14 November 2023 (2023-11-14) claims 1-13 | 1-29, 33, 34 |
| PA | WO 2023143589 A1 (GAN & LEE PHARMACEUTICALS CO., LTD.) 03 August 2023 (2023-08-03) claims 1 and 9, and description, embodiments 1-28, page 1, lines 22 and 23, and table 1 | 1-29, 33, 34 |
| PA | WO 2024146617 A1 (GAN & LEE PHARMACEUTICALS CO., LTD.) 11 July 2024 (2024-07-11) claims 1-22 | 1-29, 33, 34 |
| A | WO 2022187423 A1 (UNIV MICHIGAN) 09 September 2022 (2022-09-09) claims 1 and 22, and description, table 1 | 1-29, 33, 34 |
| A | CN 115348872 A (ORIONIS BIOSCIENCES, INC.) 15 November 2022 (2022-11-15) entire document | 1-29, 33, 34 |
| A | CN 114641337 A (THE REGENTS OF THE UNIVERSITY OF MICHIGAN) 17 June 2022 (2022-06-17) entire document | 1-29, 33, 34 |
| A | WO 2021143822 A1 (JIANGSU HENGRUI MEDICINE CO., LTD.) 22 July 2021 (2021-07-22) entire document | 1-29, 33, 34 |
| A | WO 2022081928 A1 (C4 THERAPEUTICS INC.) 21 April 2022 (2022-04-21) entire document | 1-29, 33, 34 |
| A | WO 2022187588 A1 (UNIV MICHIGAN et al.) 09 September 2022 (2022-09-09) entire document | 1-29, 33, 34 |
| A | WO 2023023531 A1 (BIOTHERYX INC.) 23 February 2023 (2023-02-23) entire document | 1-29, 33, 34 |

Form PCT/ISA/210 (second sheet) (July 2022)

**INTERNATIONAL SEARCH REPORT**

International application No.
**PCT/CN2024/095621**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **30-34**
because they relate to subject matter not required to be searched by this Authority, namely:

Claims 30-34 relate to a treatment method for a living human or animal body, which falls within subject matter excluded from the international search, and thus said claims do not comply with PCT Rule 39.1(iv).

With regard to claims 33 and 34, a search is carried out on the basis of the technical subject matter of the use of the compound or composition in the preparation of a corresponding drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/095621**

| Patent document cited in search report | Publication date (day/month/year) | Patent family member(s) | Publication date (day/month/year) |
|---|---|---|---|
| CN 118005655 A | 10 May 2024 | None | |
| WO 2024015340 A1 | 18 January 2024 | None | |
| WO 2024015346 A1 | 18 January 2024 | None | |
| WO 2024015409 A1 | 18 January 2024 | None | |
| WO 2024015406 A1 | 18 January 2024 | None | |
| WO 2024015412 A1 | 18 January 2024 | None | |
| WO 2023186069 A1 | 05 October 2023 | CN 117157298 A | 01 December 2023 |
| WO 2023183607 A1 | 28 September 2023 | None | |
| CN 117050058 A | 14 November 2023 | None | |
| WO 2023143589 A1 | 03 August 2023 | None | |
| WO 2024146617 A1 | 11 July 2024 | None | |
| WO 2022187423 A1 | 09 September 2022 | US 2024166647 A1 | 23 May 2024 |
| CN 115348872 A | 15 November 2022 | WO 2021126974 A1 | 24 June 2021 |
| | | JP 2023508891 A | 06 March 2023 |
| | | AU 2020408333 A1 | 07 July 2022 |
| | | CA 3162266 A1 | 24 June 2021 |
| | | US 2023099031 A1 | 30 March 2023 |
| | | EP 4076530 A1 | 26 October 2022 |
| | | EP 4076530 A4 | 10 April 2024 |
| | | HK 40082590 A0 | 09 June 2023 |
| CN 114641337 A | 17 June 2022 | CA 3151824 A1 | 04 March 2021 |
| | | WO 2021041664 A1 | 04 March 2021 |
| | | AU 2020336381 A1 | 03 March 2022 |
| | | EP 4021580 A1 | 06 July 2022 |
| | | US 2022388978 A1 | 08 December 2022 |
| | | JP 2022545735 A | 28 October 2022 |
| WO 2021143822 A1 | 22 July 2021 | TW 202140448 A | 01 November 2021 |
| WO 2022081928 A1 | 21 April 2022 | EP 4228625 A1 | 23 August 2023 |
| | | IL 302037 A | 01 June 2023 |
| | | KR 20230107570 A | 17 July 2023 |
| | | JP 2023545507 A | 30 October 2023 |
| | | US 2023372496 A1 | 23 November 2023 |
| | | AU 2021361044 A1 | 08 June 2023 |
| | | CA 3194343 A1 | 21 April 2022 |
| | | CN 116723839 A | 08 September 2023 |
| WO 2022187588 A1 | 09 September 2022 | US 2024190880 A1 | 13 June 2024 |
| WO 2023023531 A1 | 23 February 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **YANG L** ; **TU W** ; **HUANG L** ; **MIAO B** ; **KANESHIGE A** ; **JIANG W** ; **LENG L** ; **WANG M** ; **WEN B** ; **SUN D**. Discovery of SMD-3040 as a Potent and Selective SMARCA2 PROTAC Degrader with Strong in vivo Antitumor Activity. *J Med Chem.*, 10 August 2023, vol. 66 (15), 10761-10781